# EUROPEAN PATENT SPECIFICATION

(11) **EP 3 908 601 B1**
(45) Date of publication and mention of the grant of the patent: **24.12.2025**
(21) Application number: 20739226.7
(22) Date of filing: 10.01.2020
(51) Int. Cl.: C07K 14/705, C07K 14/54, A61K 35/17, A61K 31/497, A61K 31/53, A61K 45/06, A61K 39/00, A61P 35/00, C07K 16/28

(54) **COMPOSITIONS FOR TREATING CANCER**
ZUSAMMENSETZUNGEN ZUR BEHANDLUNG VON KREBS
COMPOSITIONS POUR LE TRAITEMENT DU CANCER

(30) Priority: 11.01.2019 US 201962791591 P; 13.08.2019 US 201962886235 P; 15.11.2019 US 201962936223 P; 11.12.2019 US 201962946631 P
(43) Date of publication of application: 17.11.2021
(73) Proprietor: Omeros Corporation, Seattle, WA 98119 (US)
(72) Inventor: CICIRELLI, Michael, Seattle, WA 98119 (US); CUTSHALL, Neil, S., Seattle, WA 98119 (US); DEMOPULOS, Gregory, A., Seattle, WA 98119 (US); GAITANARIS, George, A., Seattle, WA 98119 (US); GAVIN, Marc, A., Seattle, WA 98119 (US); GRAGEROV, Alexander, Seattle, WA 98119 (US); LITTLE, Thomas, L., Seattle, WA 98119 (US); ONRUST, Rene, Seattle, WA 98119 (US)
(74) Representative: Avidity IP
(86) International application number: PCT/US2020/013183
(87) International publication number: WO 2020/146795

(56) References cited:
- WO-A1-2016/209021
- WO-A2-2009/120899
- WO-A2-2009/120899
- US-A1- 2011 027 295
- US-A1- 2011 081 354
- US-A1- 2015 266 933
- US-A1- 2015 361 119
- US-A1- 2017 146 542
- US-A1- 2018 221 472
- US-A1- 2020 276 190
- MICHAEL J BARNES ET AL: "Lysophosphatidylserine suppression of T-cell activation via GPR174 requires G[alpha]s proteins", IMMUNOLOGY AND CELL BIOLOGY, CARLTON, AU, vol. 96, no. 4, 25 March 2018 (2018-03-25), pages 439 - 445, XP071704575, ISSN: 0818-9641, DOI: 10.1111/IMCB.12025
- VIJAYAN DIPTI ET AL: "Targeting immunosuppressive adenosine in cancer", NATURE REVIEWS CANCER, NATURE PUB. GROUP, LONDON, vol. 17, no. 12, 1 December 2017 (2017-12-01), pages 709 - 724, XP036992754, ISSN: 1474-175X, [retrieved on 20171201], DOI: 10.1038/NRC.2017.86
- ROBERTD LEONE ET AL: "Targeting adenosine for cancer immunotherapy", JOURNAL FOR IMMUNOTHERAPY OF CANCER, BIOMED CENTRAL LTD, LONDON, UK, vol. 6, no. 1, 18 June 2018 (2018-06-18), pages 1 - 9, XP021257599, DOI: 10.1186/S40425-018-0360-8
- SAITOH MASARU ET AL: "Adenosine induces apoptosis in the human gastric cancer cells via an intrinsic pathway relevant to activation of AMP-activated protein kinase", BIOCHEMICAL PHARMACOLOGY, vol. 67, no. 10, 1 May 2004 (2004-05-01), US, pages 2005 - 2011, XP055957648, ISSN: 0006-2952, DOI: 10.1016/j.bcp.2004.01.020
- GAVIN M A ET AL: "Phosphatidylserine Suppresses T Cells Through GPR174, and Co-Inhibition of Adenosine Receptors and GPR174 Synergistically Enhances Th1 Cytokine Production", ANNALS OF ONCOLOGY - ABSTRACT BOOK OF ESMO IMMUNO-ONCOLOGY CONGRESS 2019, 12 January 2019 (2019-01-12), XP055957862, DOI: 10.1093/annonc/mdz451.017
- GAVIN M A ET AL: "Abstract B45: Phosphatidylserine suppresses T cells through GPR174, and co-inhibition of adenosine receptors and GPR174 synergistically enhances T cell responses | Cancer Immunology Research | American Association for Cancer Research", CANCER IMMUNOL RES (2020) 8 (3_SUPPLEMENT): B45, 1 March 2020 (2020-03-01), pages 1 - 5, XP055957869, Retrieved from the Internet <URL:https://aacrjournals.org/cancerimmunolres/article/8/3_Supplement/B45/469813/Abstract-B45-Phosphatidylserine-suppresses-T-cells> [retrieved on 20220905], DOI: 10.1158/2326-6074.TUMIMM19-B45
- ANONYMOUS: "OMEROS’PROPRIETARY ORPHAN GPCR PROGRAM DELIVERS NEW TARGET AND APPROACH IN CANCER IMMUNOTHERAPY", OMEROSCORPORATION, 5 December 2016 (2016-12-05), pages 1 - 4, XP055724443, Retrieved from the Internet <URL:https://investor.omeros.com/news-releases/news-release-details/omeros-proprietary-orphan-gpcr-program-delivers-new-target-and#> [retrieved on 20200501]
- BARNES ET AL.: "Lysophosphatidylserine suppression of T- cell activation via GPR174 requires Gas proteins", IMMUNOLOGY & CELL BIOLOGY, vol. 96, no. 4, 25 March 2018 (2018-03-25) - April 2018 (2018-04-01), pages 439 - 445, XP055724444

## Description

### CROSS-REFERENCE TO RELATED APPLICATIONS

This application claims the benefit of U.S. Provisional Application Number 62/791,591, filed January 11, 2019, U.S. Provisional Application Number 62/886,235, filed August 13, 2019, U.S. Provisional Application Number 62/936,223, filed November 15, 2019, and U.S. Provisional Application Number 62/946,631, filed December 11, 2019.

### STATEMENT REGARDING SEQUENCE LISTING

The sequence listing associated with this application is provided in text format in lieu of a paper copy. The name of the text file containing the sequence listing is OG_1_0294_US_Sequence_Listing_20200107_ST25.txt. The file is 11 KB; was created on January 7, 2020; and is being submitted via EFS-Web with the filing of the specification.

### TECHNICAL FIELD

This disclosure is directed to compounds for use in treating cancer by administering to a subject a therapeutically effective amount of an inhibitor of G protein-coupled receptor 174 (GPR174)-mediated signaling, or a combination of an inhibitor of GPR174-mediated signaling and an inhibitor of ATP-Adenosine-A2aR-or A2bR-mediated signaling (such as an A2aR antagonist, and/or an A2bR antagonist, or a combination of an inhibitor of GPR174-mediated signaling and an inhibitor of an enzymatic pathway involved in the production of adenosine, such as CD38, CD39 and/or CD73 (such as a CD73 inhibitor and/or a CD38 inhibitor and/or a CD39 inhibitor), or an inhibitor of GPR174-mediated signaling and a regulatory T cell (Treg) attenuating agent, thereby stimulating an immune response in a mammalian subject in need thereof, such as a subject suffering from cancer.

### BACKGROUND

Mammalian G protein-coupled receptors (GPCRs) constitute a superfamily of diverse proteins with hundreds of members. GPCRs act as receptors for a multitude of different signals. Sensory GPCRs (spur) are receptors for sensory signals of external origin that are sensed as light of different colors, odors, pheromones, or tastes. Most other GPCRs respond to endogenous signals, such as peptides, lipids, neurotransmitters, or nucleotides. GPCRs falling in the latter group are involved in numerous physiological processes, including the regulation of neuronal excitability, metabolism, reproduction, development, cell division, hormonal homeostasis, and behavior, and are differentially expressed in many cell types in the body.

Ligands have been identified for some GPCRs. GPCRs that do not yet have an identified endogenous or cognate ligand are known as orphan GPCRs. As of 2019, approximately 120 GPCRs were considered to be orphan GPCRs, including GPR174 (see "Orphan and other 7TM receptors, IUPHAR/BPS Guide to Pharmacology," accessed on 6/8/2019; Alexander S. et al., Br. J. Pharmacol. 174 Suppl 1:S17-S129, 2017). Of all currently marketed drugs, 30-40% are modulators of specific GPCRs. Of about 240 non-orphan GPCRs, more than half are targeted by drugs on the market or currently in clinical development (see Hauser A.S. et al., Nature Reviews Drug Discovery 16(12):829-842, 2017).

Development of a drug to target a specific GPCR generally requires knowledge of the endogenous ligand or a surrogate ligand, *i.e.,* a non-endogenous molecule that binds and functionally interacts with the specific GPCR, which can serve as a screening control and/or as the basis for medicinal chemistry to develop synthetic drug molecules that functionally interact with that specific GPCR. Given the potential use of orphan GPCRs as targets for new drugs, identification of ligands or surrogate ligands for orphan GPCRs would hasten the development of new drugs for those receptors. However, despite significant effort to identify such ligands, a large number of orphan receptors remain (see, *e.g.,* Levoye et al., Drug Discov. Today 13(1-2):52-8, 2008). Conventional GPCR screening campaigns typically rely on screening for drug discovery using both biochemical and functional assays, such as assays measuring Ca⁺⁺ signaling as a response to GPCR activation. However, these conventional approaches rely on the presence of the endogenous ligand or a surrogate ligand. When used with orphan GPCRs, these assays can only identify agonists to GPCRs. With conventional assay technology, neither inverse agonists nor antagonists can be identified until an agonist is available. Importantly, many of the FDA approved drugs targeting GPCRs are functional inhibitors (*e.g.,* partial agonists, inverse agonists, partial inverse agonists or antagonists). Thus, conventional approaches miss detecting this common and critical class of GPCR ligands.

GPR174, also known as FKSG79 and GPCR17, is expressed in a relatively small number of tissues including spleen, thymus, bone marrow and lymph node (Regard et al., Cell 135:561-71, 2008; Chu et al., J Med Genet 50:479-85, 2013; Sugita et al., Biochem Biophys Res Commun 430:190-5, 2013. Within the lymphoid tissues, GPR174 is expressed to high levels in naive B and T cells, especially in regulatory T cells (Tregs) (Barnes et al., J Exp Med 212:1011-20, 2015). WO 2009/120899 discloses methods and compositions for use in the diagnosis, prognosis, and treatment of human hematopoietic cancers with GPR174 specifically binding antibodies. Vijayan et al., Nature Reviews Cancer 17:709-724, 2017 discloses the role of adenosine signaling in regulating tumor immunity; and Leone et al., J Immunother Cancer, 6:1-9, 2018, discloses adenosine receptor inhibitors in the treatment of primary tumors and metastases and Saitoh et al., BIOCHEMICAL PHARMACOLOGY, vol. 67, no. 10, 1 May 2004 (2004-05-01), pages 2005-2011, discloses adenosine induction of apoptosis in human gastric cancer cells.

As described herein, GPR174 activity has been associated with cancer. Given the role that GPR174 appears to play in cancer, it is desirable to identify inhibitors of this receptor. There is a need in the art for effective cancer treatments. The compositions provided herein address these and other deficiencies in the art.

### BRIEF SUMMARY

The present invention is as set out in the claims. Other disclosures herein not falling within the claims are for the assistance of the skilled person in understanding and practicing the disclosed invention and are not part of the invention. For the avoidance of doubt, it is noted that the present invention does not extend to methods of treatment of the human or animal body. Any references in the description to methods of treatment refer to compounds, pharmaceutical compositions, and medicaments of the present disclosure for use in a method of treatment of the human or animal body by therapy.

The present inventors have identified chemical compounds that functionally interact with GPR174 and inhibit one or more GPR174-mediated signaling pathways and, additionally, have characterized the GPR174 receptor signaling profile, which includes the Gs signaling pathway. The inventors have demonstrated that representative GPR174 inhibitory compounds are capable of reducing the fraction of highly suppressive regulatory T cells or "TRegs" (FoxP3+Helios+) in human peripheral blood mononuclear cells (PBMCs), as described in Examples 6, 8, and 9. The inventors have further determined that GPR174 inhibition stimulates IL-2 production in a dose-dependent manner in PBMCs, as described in Examples 6-11. The inventors have also determined that GPR174 inhibition reduces immune-cell associated programmed death-ligand 1 (PD-L1) expression, cytotoxic T-lymphocyte-associated antigen 4 (CTLA4) expression, T cell immunoreceptor with Ig and ITIM domains (TIGIT) expression and amphiregulin (AREG) expression, as described in Example 13 and 14. Therefore, the GPR174 inhibitory compounds can be used as drugs for use in stimulating an immune response in a mammalian subject and can form the basis for additional therapeutic agents. Based on these discoveries, the present disclosure is related to *in vivo* and *in vitro* methods for inhibiting the GPR174 receptor and thereby stimulating an immune response, particularly in a subject suffering from a non-malignant neoplasm or a malignant neoplasm *(i.e.,* cancer).

As further described herein, the inventors have also demonstrated that the combined inhibition of GPR174 and inhibition of the Adenosine 2a Receptor (A2aR) and/or the inhibition of the Adenosine 2b receptor (A2bR) results in synergistic induction of Th1 cytokine production (*e.g.,* IFN-γ, IL-2, TNF and GM-CSF), in human PBMCs, as described in Examples 15, 18-23, 27, and 29.

As further described herein, the inventors have also demonstrated that murine colon carcinoma tumor growth and melanoma tumor growth was reduced in GPR174-deficient mice treated with an anti-GITR antibody (*i.e.,* a Treg attenuating agent) as compared to wild-type mice, as described in Examples 24 and 25.

As further described herein, the inventors have determined that cancer cell-derived exosomes stimulate GPR174 and inhibition of GPR174 in the presence of cancer cell-derived exosomes enhances Th1 cytokine levels, thereby stimulating and/or amplifying the immune system as described in Example 26.

In summary, as demonstrated herein, GPR174 is an immune system-restricted Gαs-coupled GPCR and PS exposed on liposomes and cellular membranes stimulates GPR174, supporting a model of active GPR174-mediated immune suppression in the tumor microenvironment. Under conditions where both PS/lysoPS and adenosine are present, inhibition of both axes is necessary for effective restoration of T cell function. As further demonstrated herein, GPR174-deficiency enhances anti-tumor immune responses in mice.

Therefore, in one aspect, the present disclosure provides methods and compositions for stimulating and/or amplifying an immune response (*e.g.* for treating cancer) by administering to a subject suffering from, or at risk of developing cancer or cancer metastasis, a therapeutically effective amount of an inhibitor of GPR174-mediated signaling as a single agent or optionally in combination with an inhibitor of ATP-Adenosine-A2aR-or A2bR-mediated signaling (such as an A2aR antagonist, an A2bR antagonist and/or a CD73 inhibitor and/or a CD38 inhibitor and/or a CD39 inhibitor, and/or a Treg attenuating agent, or a combination thereof), thereby stimulating and/or amplifying an immune response in a mammalian subject in need thereof. In some cases, the methods comprise administering to a subject suffering from cancer an inhibitor of GPR174 and an A2aR antagonist. In some cases, the methods comprise administering to a subject suffering from cancer an inhibitor of GPR174 and an A2bR antagonist. In some cases, the methods comprise administering to a subject suffering from cancer an inhibitor of GPR174 and an A2aR/A2bR antagonist. In some cases, the methods comprise administering to a subject suffering from cancer an inhibitor of GPR174 and a CD73 inhibitor. In some cases, the methods comprise administering to a subject suffering from cancer an inhibitor of GPR174 and a CD38 inhibitor. In some cases, the methods comprise administering to a subject suffering from cancer an inhibitor of GPR174 and a CD39 inhibitor. In some cases, the methods comprise administering to a subject suffering from cancer an inhibitor of GPR174 and a Treg attenuating agent. In some cases, the methods comprise administering to a subject suffering from cancer an inhibitor of GPR174 and an A2aR antagonist and optionally at least one or more of an A2bR antagonist, a CD73 inhibitor, a CD38 inhibitor, a CD39 inhibitor and a Treg attenuating agent. In some cases, the A2aR antagonist is a small molecule. In some cases, the A2aR antagonist is an antibody. In some cases, the A2aR antagonist is a peptide- or a nucleotide/nucleic acid-based molecule. In some cases, the A2bR antagonist is a small molecule. In some cases, the A2bR antagonist is an antibody. In some cases, the A2bR antagonist is a peptide- or a nucleotide/nucleic acid-based molecule. In some cases, the CD38 inhibitor is a small molecule. In some cases, the CD38 inhibitor is an antibody. In some cases, the CD38 antagonist is a peptide- or a nucleotide/nucleic acid-based molecule. In some cases, the CD39 inhibitor is a small molecule. In some cases, the CD39 inhibitor is an antibody. In some cases, the CD39 antagonist is a peptide- or a nucleotide/nucleic acid-based molecule. In some cases, the CD73 inhibitor is a small molecule. In some cases, the CD73 inhibitor is an antibody. In some cases, the CD73 antagonist is a peptide- or a nucleotide/nucleic acid-based molecule. In some cases, the Treg attenuating agent is a small molecule. In some cases, the Treg attenuating agent is an antibody. In some cases, the Treg attenuating agent is a peptide or a nucleotide/nucleic acid-based molecule. In some cases, the GPR174 inhibitor is a small molecule. In some cases, the GPR174 inhibitor is an antibody. In some cases of the method, the GPR174 inhibitor, and at least one or more of the A2aR antagonist, the A2bR antagonist, the CD38 inhibitor, the CD39 inhibitor, the CD73 inhibitor and the Treg attenuating agent are administered simultaneously (*e.g.,* co-administered separately or together), or sequentially in any order, provided the effects of the first administered inhibitor remain present at the time of the second (and optionally third) administered inhibitor or attenuating agent.

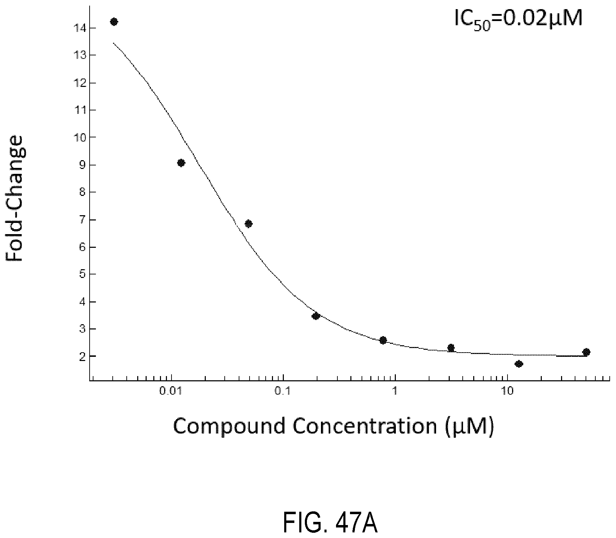

As further described herein in Examples 16 and 17, the inventors have also discovered that phosphatidylserine (PS) is an agonist for GPR174-mediated Gs signaling. Example 17 provides experimental results demonstrating that apoptotic cells stimulate GPR174 Gs signaling pathway in cells expressing GPR174. As further described in Example 16 and shown in FIGURES 47A-47F, PS-mediated GPR174 Gs signaling is inhibited by representative GPR174 inhibitory compounds 6, 10, 11, 20, 23 and 30 which belong to different chemical classes (*i.e.,* Groups I, II and IV). This data demonstrates that compounds 6, 10, 11, 20, 23, and 30 act as GPR174 antagonists and inhibit PS liposome-mediated cAMP signaling. Thus, PS liposome signaling through GPR174 is inhibited by multiple GPR174 inhibitory small molecule compounds with diverse chemical structures. Accordingly, in one case, the present disclosure provides a method of inhibiting PS-mediated activation of GPR174 Gs signaling in an immune cell expressing GPR174 comprising contacting said immune cell with an inhibitor of GPR174 Gs signaling either as a single agent or in combination with at least one of an A2aR antagonist, an A2bR antagonist, a CD38 inhibitor, a CD39 inhibitor, a CD73 inhibitor and/or a Treg attenuating agent. In one case, the present disclosure provides a method of stimulating T-cell mediated immunity in a subject suffering from cancer comprising administering a GPR174 inhibitor either as a single agent or in combination with at least one of an A2aR antagonist, an A2bR antagonist, a CD38 inhibitor, a CD39 inhibitor, a CD73 inhibitor, and/or a Treg attenuating agent to said subject in an amount effective to stimulate T-cell mediated immunity as described herein.

In another case, the disclosure provides a method of increasing an anti-tumor immune response in a subject that is currently undergoing, or has undergone treatment with at least one of an A2aR antagonist, an A2bR antagonist, a CD38 inhibitor, a CD39 inhibitor, a CD73 inhibitor and/or a Treg attenuating agent, comprising administering an effective amount of a GPR174 inhibitor to stimulate an enhanced anti-tumor immune response in the subject, provided that the use of a CD73 inhibitor is preferably avoided during early tumor formation or in the setting of metastasis due to the fact that it might be preferable to have CD73 remain active in such settings.

In some cases, provided herein is a method of stimulating an immune response in a mammalian patient in need thereof comprising administering to the mammalian patient a small molecule inhibitor of GPR174 signaling, alone or in combination with an inhibitor of ATP-Adenosine-A2aR-or ATP-Adenosine-A2bR-mediated signaling (such as an A2aR antagonist, an A2bR antagonist, and/or a CD73 inhibitor and/or a CD38 inhibitor and/or a CD39 inhibitor) and/or a Treg attenuating agent. In some cases, the GPR174 inhibitor inhibits GPR174-mediated signaling through the Gs alpha subunit of the heterotrimeric G protein that stimulates the 3',5'-cyclic adenosine monophosphate (cAMP)-dependent pathway. The cAMP-dependent pathway (also referred to as Gs signaling or Gs pathway) involves synthesis of the second messenger cAMP, which in turn activates the cAMP-dependent protein kinase (Protein Kinase A or PKA).

In one case, the disclosure provides a pharmaceutical composition comprising a combination of an inhibitor of GPR174 signaling and a least one or more of an adenosine A2A receptor antagonist, an adenosine A2B receptor antagonist, a CD73 inhibitor and/or a CD38 inhibitor and/or a CD39 inhibitor and a pharmaceutically acceptable carrier.

In one case, the disclosure provides a pharmaceutical composition comprising a combination of an inhibitor of GPR174 signaling and a Treg attenuating agent and a pharmaceutically acceptable carrier.

In some cases, the GPR174 inhibitor is a small molecule inhibitor that is an inverse agonist of GPR174 signaling.

In some cases, the small molecule inhibitor is an antagonist of GPR174 signaling.

In some cases, the small molecule inhibitor inhibits phosphatidylserine (PS), or lysophosphatidylserine (LysoPS), or pepducin dependent activation of GPR174 signaling in a cell expressing GPR174 by at least 25%.

In some cases, the stimulated immune response comprises a T-cell-mediated immune response.

In some cases, the T-cell mediated immune response comprises suppression of T-Reg activity, differentiation, growth and/or proliferation. In some cases, the T-cell mediated immune response comprises stimulation of Teffector activity, differentiation, growth and/or proliferation. In some cases, the T-cell mediated immune response comprises stimulation of Th1 cell activity, differentiation, growth and/or proliferation. In some cases, the T-cell mediated immune response comprises suppression of Th17 cell activity, differentiation, growth and/or proliferation. In one case, the T-cell mediated immune response comprises preferential growth and activation of Teffector cells over those of Tregulatory cells, resulting in a relative decrease in Tregulatory cell frequency, abundance and/or function.

In some cases, the stimulated immune response comprises a reduction in immune-cell or cancer-cell associated programmed death-ligand 1 (PD-L1) expression or cytotoxic T-lymphocyte-associated antigen 4 (CTLA4) expression or T cell immunoreceptor with Ig and ITIM domains (TIGIT) expression or amphiregulin (AREG) expression.

In some cases, the stimulated immune response comprises an NK-cell mediated immune response. In some cases, at least a portion of the T-cells or the NK-cells express GPR174. In some cases, the NK-cell mediated immune response comprises stimulation of NK-cell function, activity, differentiation, growth and/or proliferation.

In some cases, the stimulated immune response and/or amplified immune response comprises an increase in Th1 cytokine production in the presence of cancer cell-derived exosomes.

In some cases, the patient is suffering from, or harboring a neoplasm not recognized by the body as self. In some cases, the neoplasm is a non-malignant neoplasm, such as a non-malignant tumor. In some cases, the neoplasm is a malignant tumor. In some cases, the neoplasm (either malignant or non-malignant tumor) comprises a T cell infiltrate. In some cases, tumor cells are destroyed by the immune response stimulated by the GPR174 inhibitory compound either as a single agent, or in combination with at least one of an A2aR antagonist, an A2bR antagonist, a CD38 inhibitor, a CD39 inhibitor, a CD73 inhibitor and/or a Treg attenuating agent.

In some cases, the patient is a cancer patient. In some cases, the mammalian patient is a human. In some cases, the cancer is a solid tumor. In some cases, the cancer is a blood cancer. In some cases, the cancer is as disclosed herein. In some cases, at least a portion of the cancer cells express GPR174. In some cases, the GPR174 has 90% or greater sequence identity to SEQ ID NO: 1.

In some cases, T-cell activity, differentiation, proliferation, and/or growth is stimulated in a population of peripheral blood mononuclear cells (PBMCs) contacted with the GPR174 small molecule inhibitor either as a single agent, or in combination with at least one of an A2aR antagonist, an A2bR antagonist, a CD38 inhibitor, a CD39 inhibitor, a CD73 inhibitor and/or a Treg attenuating agent as compared to a control population of PBMCs not contacted with the GPR174 small molecule inhibitor.

In some cases, the fraction of FoxP3⁺Helios+ T-cells (regulatory T-cells) is decreased by at least 20% in a population of PBMCs contacted with the GPR174 small molecule inhibitor either as a single agent, or in combination with at least one of an A2aR antagonist, an A2bR antagonist, a CD38 inhibitor, a CD39 inhibitor, a CD73 inhibitor, and/or a Treg attenuating agent as compared to a control population of PBMCs not contacted with the small molecule GPR174 inhibitor.

In some cases, the cAMP level is decreased by at least 20%, at least 50%, or at least 75% in a cell expressing GPR174 contacted with the small molecule inhibitor of GPR174 signaling either as a single agent, or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the GPR174 small molecule inhibitor.

In some cases, the activity of protein kinase A (PKA) is decreased by at least 20% in cells expressing GPR174 contacted with the small molecule inhibitor of GPR174 either as a single agent, or in combination with at least one of an A2aR antagonist, an A2bR antagonist, a CD38 inhibitor, a CD39 inhibitor, a CD73 inhibitor and/or a Treg attenuating agent, as compared to a control cell not contacted with the GPR174 small molecule inhibitor.

In some cases, the PKA activity is decreased by at least 20%, at least 50%, or at least 75% in a cell expressing GPR174 contacted with the small molecule inhibitor of GPR174 signaling either as a single agent, or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the GPR174 small molecule inhibitor.

In some cases, the production of one or more of IL-2, IFN-γ, TNF-α, IL-6, and IL-10 is increased by at least 20% in peripheral blood mononuclear cells (PBMCs) contacted with the small molecule inhibitor of GPR174 as compared to control cells not contacted with the small molecule inhibitor.

In some cases, the production of one or more of IL-2, IFN-γ, TNF-α, and GM-CSF is increased by at least 20% in peripheral blood mononuclear cells (PBMCs) contacted with the small molecule inhibitor of GPR174 in combination with at least one of an A2aR antagonist, an A2bR antagonist, a CD38 inhibitor, a CD39 inhibitor, a CD73 inhibitor, and/or a Treg attenuating agent as compared to control cells not contacted with the small molecule inhibitor of GPR174.

In some cases, the production of IL-17A is decreased by at least 20% in PBMCs contacted with the small molecule inhibitor as compared to control cells not contacted with the small molecule inhibitor.

In some cases, the production of one or more cytokines is decreased by at least 20%, at least 50%, or at least 80% in in a population of PBMCs exposed to a small molecule inhibitor of GPR174 signaling as compared to a population of PBMCs not exposed to the small molecule inhibitor of GPR174 signaling. In some cases, the production of IL-17A is decreased by at least 20%, at least 50%, or at least 80% in a population of PBMCs exposed to a small molecule inhibitor of GPR174 signaling as compared to a population of PBMCs not exposed to the small molecule inhibitor of GPR174 signaling.

In some cases, the production of one or more cytokines is increased by at least 20%, at least 50%, or at least 80% in a population of PBMCs exposed to a small molecule inhibitor of GPR174 signaling as compared to a population of PBMCs not exposed to the small molecule inhibitor of GPR174 signaling. In some cases, the cytokine is selected from IL-2, IFN-γ, TNF, IL-6, and IL-10.

In some cases, the production of one or more cytokines is increased by at least 20%, at least 50%, or at least 80% in a population of PBMCs exposed to a small molecule inhibitor of GPR174 in combination with at least one of an A2aR antagonist, an A2bR antagonist, a CD38 inhibitor, a CD39 inhibitor, a CD73 inhibitor and/or a Treg attenuating agent as compared to control cells not contacted with the small molecule inhibitor of GPR174. In some cases, the cytokine is a T helper type 1 cell (Th1) cytokine selected from IL-2, IFN-γ, TNF, and GM-CSF.

In some cases, the production of one or more cytokines is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the cytokine is selected from IL-2, IFN-γ, TNF, and GM-CSF. In some cases, the production of one or more of IL-2, IFN-γ, TNF, or GM-CSF is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor, and/or a Treg attenuating agent as compared to a population of PBMCs not exposed to the small molecule inhibitor of GPR174 signaling.

In some cases, the production of IL-2 is increased by at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the production of IFN-γ is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the production of TNF-α is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling.

In some cases, the production of GM-CSF is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, CD39 inhibitor, a CD73 inhibitor and/or a Treg attenuating agent as compared to a population of PBMCs not exposed to the small molecule inhibitor of GPR174 signaling.

In some cases, the production of one or more cytokines are not modulated and do not increase or decrease by more than 20% in a population of PBMCs exposed to a small molecule inhibitor of GPR174 signaling as compared to a population of PBMCs not exposed to the small molecule inhibitor of GPR174 signaling. In some cases, the cytokine or cytokines is selected from the group consisting of: IL-4, IL-5, IL-9, IL-13, IL-17F, IL-21, and IL-22.

In some cases, the production of one or more cytokines is decreased by at least 20%, at least 50%, or at least 80% in a cell expressing GPR174 contacted with the small molecule inhibitor as compared to a control cell expressing GPR174 not contacted with the small molecule inhibitor. In some cases, the production of IL-17A is decreased by at least 20%, at least 50%, or at least 80% in a cell expressing GPR174 contacted with the small molecule inhibitor as compared to a control cell expressing GPR174 not contacted with the small molecule inhibitor.

In some cases, the production of one or more cytokines is increased by at least 20%, at least 50%, at least 100%, at least 500%, or at least 1000% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174. In some cases, the production of one or more of IL-2, IFN-γ, TNF, or GM-CSF is increased by at least 20%, at least 50%, at least 100%, at least 500%, or at least 1000% in a cell expressing GPR174 contacted with the small molecule inhibitor of GPR174 as compared to a control cell expressing GPR174 not contacted with the small molecule inhibitor of GPR174. In some cases, the production of IL-2 is increased by at least 100%, at least 500%, or at least 1000% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174. In some cases, the production of IFN-γ is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174. In some cases, the production of TNF-α is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the production of one or more or IL-4, IL-5, IL-9, IL-13, IL-17F, IL-21, and IL-22 are not modulated and do not increase or decrease by more than 20% in a cell expressing GPR174 contacted with a small molecule inhibitor of GPR174 signaling as compared to a control cell expressing GPR174 not contacted with the small molecule inhibitor. In some cases, the patient has one or more tumor(s) which may or may not comprise infiltrating regulatory T-cells.

In some cases, provided herein is a method of inhibiting the level of cAMP in a cell that expresses GPR174, the method comprising contacting the cell with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent.

In some cases, the cAMP level is decreased by at least 20%, at least 50%, or at least 75% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the contacting is *in vivo.* In some cases, the contacting is *in vitro.* In some cases, the contacting is *ex vivo.*

In some cases, the cell is an immune cell. In some cases, the cell is a T-cell or NK-cell. In some cases, the NK-cell or T-cell activity, differentiation, growth and/or proliferation is stimulated. In some cases, the cell is a mammalian cell. In some cases, the cell is a human cell. In some cases, the cell is a thymus, lymph node, spleen, bone marrow, or PMBC cell.

In some cases, the GPR174 has 90% or greater sequence identity, such as 91%, 92%, 93%, 94%, 95%, 96%, 97%, 98%, 99% to SEQ ID NO:1. In some cases, the GPR174 has a sequence set forth as SEQ ID NO:1.

In some cases, provided herein is a method of modulating the level of one or more cytokines in PBMCs, the method comprising contacting the PBMCs with a small-molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent.

In some cases, the contacting is *in vivo.* In some cases, the contacting is *in vitro.* In some cases, the contacting is *ex vivo.*

In some cases, the PBMCs comprise immune cells. In some cases, the immune cells comprise T-cells or NK-cells. In some cases, the activity, differentiation, growth and/or proliferation of the NK-cells or T-cells is stimulated. In some cases, the PBMCs comprise mammalian cells. In some cases, the PBMCs comprise human cells.

In some cases, the GPR174 has 90% or greater sequence identity to SEQ ID NO: 1.

In some cases, the cytokine is selected from IL-2, IFN-γ, TNF, GM-CSF, IL-6, IL-17A, IL-10, IL-4, IL-5, IL-9, IL-12, IL-13, IL-17F, IL-21, and IL-22. In some cases, the level of one or more cytokines is increased. In some cases, the level of one or more of cytokines selected from the group consisting of IL-2, IFN-γ, TNF and GM-CSF is increased. In some cases, the level of one or more cytokines is decreased. In some cases, the level of one or more cytokines is not modulated.

In some cases, the level of one or more cytokines is decreased by at least 20%, at least 50%, or at least 80% in in a population of PBMCs exposed to a small molecule inhibitor of GPR174 signaling as compared to a population of PBMCs not exposed to the small molecule inhibitor of GPR174 signaling. In some cases, the level of IL-17A is decreased by at least 20%, at least 50%, or at least 80% in a population of PBMCs exposed to a small molecule inhibitor of GPR174 signaling as compared to a population of PBMCs not exposed to the small molecule inhibitor of GPR174 signaling.

In some cases, the level of one or more cytokines is increased by at least 20%, at least 50%, or at least 80% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the cytokine is selected from IL-2, IFN-γ, TNF, and GM-CSF.

In some cases, the level of one or more cytokines is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the cytokine is selected from IL-2, IFN-γ, TNF, and GM-CSF. In some cases, the level of one or more of IL-2, IFN-γ, TNF-α, or GM-CSF is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling.

In some cases, the level of IL-2 is increased by at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the level of IFN-γ is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the level of TNF-α is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling.

In some cases, the level of one or more cytokines are not modulated and do not increase or decrease by more than 20% in a population of PBMCs exposed to a small molecule inhibitor of GPR174 signaling as compared to a population of PBMCs not exposed to the small molecule inhibitor of GPR174 signaling. In some cases, the cytokine or cytokines is selected from the group consisting of: IL-4, IL-5, IL-9, IL-13, IL-17F, IL-21, and IL-22.

In some cases, the level of one or more cytokines is decreased by at least 20%, at least 50%, or at least 80% in a cell expressing GPR174 contacted with the small molecule inhibitor as compared to a control cell expressing GPR174 not contacted with the small molecule inhibitor. In some cases, the level of IL-17A is decreased by at least 20%, at least 50%, or at least 80% in a cell expressing GPR174 contacted with the small molecule inhibitor as compared to a control cell expressing GPR174 not contacted with the small molecule inhibitor.

In some cases, the level of one or more cytokines is increased by at least 20%, at least 50%, at least 100%, at least 500%, or at least 1000% in a cell expressing GPR174 contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174. In some cases, the level of one or more of IL-2, IFN-γ, TNF, or GM-CSF is increased by at least 20%, at least 50%, at least 100%, at least 500%, or at least 1000% in a cell expressing GPR174 contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the level of IL-2 is increased by at least 100%, at least 500%, or at least 1000% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the level of IFN-γ is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the level of TNF (also referred to as "TNF-α") is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the level of one or more or IL-4, IL-5, IL-9, IL-13, IL-17F, IL-21, and IL-22 are not modulated and do not increase or decrease by more than 20% in a cell expressing GPR174 contacted with a small molecule inhibitor of GPR174 signaling as compared to a control cell expressing GPR174 not contacted with the small molecule inhibitor.

In some cases, provided herein is a pharmaceutical composition comprising a small molecule inhibitor of GPR174 signaling and a pharmaceutically acceptable excipient.

In some cases, provided herein is a pharmaceutical composition comprising a small molecule inhibitor of GPR174 signaling in combination with at least one of an A2aR antagonist, an A2bR antagonist, a CD38 inhibitor, a CD39 inhibitor and/or a CD73 inhibitor and a pharmaceutically acceptable excipient.

In some cases, provided herein is a pharmaceutical composition comprising a small molecule inhibitor of GPR174 signaling in combination with an A2aR antagonist and a pharmaceutically acceptable excipient.

In some cases, provided herein is a pharmaceutical composition comprising a small molecule inhibitor of GPR174 signaling in combination with an A2bR antagonist and a pharmaceutically acceptable excipient.

In some cases, provided herein is a pharmaceutical composition comprising a small molecule inhibitor of GPR174 signaling in combination with an A2aR antagonist and an A2bR antagonist and a pharmaceutically acceptable excipient.

In some cases, provided herein is a pharmaceutical composition comprising a small molecule inhibitor of GPR174 signaling in combination with a Treg attenuating agent and a pharmaceutically acceptable excipient.

In some cases, provided herein is a pharmaceutical composition comprising a small molecule inhibitor of GPR174 and at least one of (i) an A2aR antagonist and/or an A2bR antagonist; and (ii) at least one of a CD73 inhibitor and/or a CD38 inhibitor and/or a CD39 inhibitor. In some cases, the pharmaceutical composition further comprises a Treg attenuating agent.

In some cases, the small molecule inhibitor of GPR174 does not comprise an alkyl chain comprising 10 or more C atoms. In some cases, the small molecule inhibitor of GPR174 is not pepducin, LysoPS, PS, or a compound disclosed in US Pub. No. 2015/0361119. In some cases, the small molecule inhibitor has a molecular weight from about 50 Da to about 2500 Da. In some cases, the small molecule inhibitor has a molecular weight from about 50 to 800 Da, such as from about 100 Da to about 800 Da.

In some cases, the small molecule inhibitor alters the cellular distribution of a recombinant GPR174 polypeptide modified to include a nuclear localization signal as compared to the cellular distribution of the recombinant GPR174 polypeptide in the absence of the inhibitor. In some cases, the small molecule inhibitor competitively binds to GPR174 as compared to any one of compounds 1-59. In some cases, the small molecule inhibitor has an EC₅₀ for GPR174 of less than 15 µM as determined by a cellular redistribution assay (CRA). CRA is described in detail herein under "Screening Methods." In some cases, the small molecule inhibitor binds competitively to GPR174 as compared to any one of compounds 1-59 and results in an altered apparent binding affinity of the compound for GPR174 when a cell expressing GPR174 is contacted with the compound and the small molecule inhibitor at the same time as compared to the binding affinity of compound for GPR174 alone. In some cases, the small molecule inhibitor causes a difference in the inhibitory activity of any one of reference compounds 1-59 in a GPR174-mediated signaling assay when tested in the presence of said reference compound as compared to the inhibitory activity of the reference compound alone. In some cases, the small molecule inhibitor binds allosterically to GPR174 as compared to any one of compounds 1-59. In some cases, the small molecule inhibitor is specific for GPR174 as compared to one or more GPCRs in a reference panel of GPCRs as disclosed herein. In some cases, the small molecule inhibitor is specific for GPR174 relative to P2Y10. In some cases, the small molecule inhibitor is specific for GPR174 relative to GPR34. In some cases, the small molecule inhibitor inhibits the Gs signaling pathway.

In some cases, T-cell activity, differentiation, proliferation, and/or growth is stimulated in a population of peripheral blood mononuclear cells (PBMCs) contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control population of PBMCs not contacted with the small molecule inhibitor of GPR174.

In some cases, the subpopulation of regulatory T-cells is decreased in a population of PBMCs contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control population of PBMCs not contacted with the small molecule inhibitor of GPR174.

In some cases, the fraction of FoxP3⁺Helios+ T-cells (regulatory T-cells) is decreased by at least 20% in a population of PBMCs contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control population of PBMCs not contacted with the small molecule inhibitor of GPR174.

In some cases, the level of cAMP is decreased by at least 20% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the cAMP level is decreased by at least 20%, at least 50%, or at least 75% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the activity of PKA is decreased by at least 20% in cells expressing GPR174 contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174.

In some cases, the PKA activity is decreased by at least 20%, at least 50%, or at least 75% in a cell expressing GPR174 contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the level of one or more cytokines is modulated in PBMCs contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to control cells not contacted with the small molecule inhibitor of GPR174.

In some cases, the level of one or more of IL-2, IFN-γ, TNF, and GM-CSF is increased by at least 20% in PBMCs contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to control cells not contacted with the small molecule inhibitor of GPR174.

In some cases, the level of IL-17A is decreased by at least 20% in PBMCs contacted with the small molecule inhibitor as compared to control cells not contacted with the small molecule inhibitor.

In some cases, the level of one or more cytokines is decreased by at least 20%, at least 50%, or at least 80% in in a population of PBMCs exposed to a small molecule inhibitor of GPR174 signaling as compared to a population of PBMCs not exposed to the small molecule inhibitor of GPR174 signaling. In some cases, the level of IL-17A is decreased by at least 20%, at least 50%, or at least 80% in a population of PBMCs exposed to a small molecule inhibitor of GPR174 signaling as compared to a population of PBMCs not exposed to the small molecule inhibitor of GPR174 signaling.

In some cases, the level of one or more cytokines is increased by at least 20%, at least 50%, or at least 80% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the cytokine is selected from IL-2, IFN-γ, TNF and GM-CSF.

In some cases, the level of one or more cytokines is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the cytokine is selected from IL-2, IFN-γ, TNF, and GM-CSF. In some cases, the level of one or more of IL-2, IFN-γ, TNF, or GM-CSF is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling.

In some cases, the level of IL-2 is increased by at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the level of IFN-γ is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling. In some cases, the level of TNF-α is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a population of PBMCs not exposed to the small molecule inhibitor of GPR174 signaling.

In some cases, the level of one or more cytokines are not modulated and do not increase or decrease by more than 20% in a population of PBMCs exposed to a small molecule inhibitor of GPR174 signaling as compared to a population of PBMCs not exposed to the small molecule inhibitor of GPR174 signaling. In some cases, the cytokine or cytokines is selected from the group consisting of: IL-4, IL-5, IL-9, IL-13, IL-17F, IL-21, and IL-22.

In some cases, the level of one or more cytokines is decreased by at least 20%, at least 50%, or at least 80% in an immune cell contacted with the small molecule inhibitor as compared to a control cell expressing GPR174 not contacted with the small molecule inhibitor. In some cases, the level of IL-17A is decreased by at least 20%, at least 50%, or at least 80% in an immune cell contacted with the small molecule inhibitor as compared to a control cell not contacted with the small molecule inhibitor

In some cases, the level of one or more cytokines is increased by at least 20%, at least 50%, at least 100%, at least 500%, or at least 1000% in an immune cell contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174. In some cases, the level of one or more of IL-2, IFN-γ, TNF, or GM-CSF is increased by at least 20%, at least 50%, at least 100%, at least 500%, or at least 1000% in an immune cell contacted with the small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174.

In some cases, the level of IL-2 is increased by at least 100%, at least 500%, or at least 1000% in an immune cell contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174. In some cases, the level of IFN-γ is increased by at least 50%, at least 100%, or at least 500% in an immune cell contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174. In some cases, the level of TNF-α is increased by at least 50%, at least 100%, or at least 500% in an immune cell contacted with a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell not contacted with the small molecule inhibitor of GPR174.

In some cases, the level of one or more or IL-4, IL-5, IL-9, IL-13, IL-17F, IL-21, and IL-22 are not modulated and do not increase or decrease by more than 20% in an immune cell contacted with a small molecule inhibitor of GPR174 signaling as compared to a control cell not contacted with the small molecule inhibitor.

In some cases, the small molecule inhibitor reduces ligand-induced receptor internalization of GPR174 in cells expressing GPR174 as compared to control cells not contacted with the small molecule inhibitor.

In some cases, the receptor internalization is reduced by at least 10%, at least 20%, at least 50%, or at least 80% in a cell expressing GPR174 contacted with a small molecule inhibitor of GPR174 signaling as compared to a control cell expressing GPR174 not contacted with the small molecule inhibitor. In some cases, the receptor internalization is reduced by 1-99% in a cell expressing GPR174 contacted with a small molecule inhibitor of GPR174 signaling as compared to a control cell expressing GPR174 not contacted with the small molecule inhibitor. In some cases, the receptor internalization is reduced by 1-20%, 20-40%, 40-60%, 60-80%, or 80-99% in a cell expressing GPR174 contacted with a small molecule inhibitor of GPR174 signaling as compared to a control cell expressing GPR174 not contacted with the small molecule inhibitor.

In some cases, the β-arrestin recruitment is reduced by at least 10%, at least 20%, at least 50%, or at least 80% in a cell expressing GPR174 contacted with a small molecule inhibitor of GPR174 signaling as compared to a control cell expressing GPR174 not contacted with the small molecule inhibitor. In some cases, the β-arrestin recruitment is reduced by 1-99% in a cell expressing GPR174 contacted with a small molecule inhibitor of GPR174 signaling as compared to a control cell expressing GPR174 not contacted with the small molecule inhibitor. In some cases, the β-arrestin recruitment is reduced by 1-20%, by 20-40%, 40-60%, 60-80%, or by 80-99% in a cell expressing GPR174 contacted with a small molecule inhibitor of GPR174 signaling as compared to a control cell expressing GPR174 not contacted with the small molecule inhibitor.

In some cases, provided herein is a composition comprising a small molecule inhibitor of GPR174 either as a single agent or in combination with at least one of an A2aR antagonist, an A2bR antagonist, a CD38 inhibitor, a CD39 inhibitor, a CD73 inhibitor and/or a Treg attenuating agent and a cell expressing GPR174.

In some cases, the cell is a mammalian cell. In some cases, the cell is a human cell. In some case, the cell is an immune cell. In some cases, the cell is a T cell. In some cases, the T cell is a naive T cell. In some cases, the T cell is a regulatory T cell. In some cases, the T cell is an effector T cell. In some cases, the cell is a thymus, lymph node, spleen, bone marrow, or PBMC cell. In some cases, the level of cAMP is decreased by at least 20% in the composition as compared to a control cell expressing GPR174 not contacted with the small molecule inhibitor of GPR174. In some cases, the activity of PKA is decreased by at least 20% in the composition as compared to a control cell expressing GPR174 not contacted with the small molecule inhibitor of GPR174. In some cases, the level of one or more cytokines is modulated in the composition as compared to a control cell expressing GPR174 not contacted with the small molecule inhibitor of GPR174.

In some cases, the cAMP level is decreased by at least 20%, at least 50%, or at least 75% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, the PKA activity is decreased by at least 20%, at least 50%, or at least 75% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent as compared to a control cell expressing GPR174 not contacted with the small molecule inhibitor of GPR174.

In some cases, provided herein is a composition comprising a small molecule inhibitor of GPR174 signaling either as a single agent or in combination with at least one of an A2aR antagonist, an A2bR antagonist, a CD38 inhibitor, a CD39 inhibitor, a CD73 inhibitor and/or a Treg attenuating agent and a population of cells, wherein at least a portion of the population of cells express GPR174, and wherein at least a portion of the population of cells expressing GPR174 comprises T-cells.

In some cases, wherein the population of cells comprise cancer cells. In some cases, the population of T-cells comprises regulatory T cells. In some cases, the population of T-cells comprises effector T cells.

In another aspect, the disclosure features a method of inhibiting a GPR174-mediated signaling pathway (*e.g.,* a Gs signaling pathway) in a cell. The method includes the step of contacting a cell with an isolated compound predetermined to functionally interact with GPR174 either as a single agent or in combination with at least one of an A2aR antagonist, an A2bR antagonist, a CD38 inhibitor, a CD39 inhibitor, a CD73 inhibitor and/or a Treg attenuating agent, where the cell includes a GPR174-mediated signaling pathway and expresses GPR174 (*e.g.,* a GPR174 polypeptide) and the isolated compound inhibits the ability of the GPR174 to modulate the GPR174-mediated signaling pathway, thereby inhibiting the GPR174-mediated signaling pathway in the cell. In certain cases, the compound is capable of functionally interacting with human GPR174 having the sequence set forth as SEQ ID NO:1, or a naturally occurring variant of GPR174 having at least 90% identity to SEQ ID NO: 1. In particular cases, the isolated compound is capable of altering the cellular distribution of a recombinant GPR174 polypeptide (*e.g.,* human GPR174 having the sequence of SEQ ID NO: 1) modified to alter cellular distribution as compared to the cellular distribution of the recombinant GPR174 polypeptide in the absence of the compound. In some cases, the cell is contacted in vivo in a subject suffering from a disease such as cancer.

In a related aspect, the disclosure features a method of inhibiting a GPR174-mediated signaling pathway in a cell by (i) providing an isolated compound predetermined to functionally interact with human GPR174 and to inhibit a GPR174-mediated signaling pathway in cells expressing GPR174; and (ii) contacting a cell expressing GPR174 with said isolated compound either as a single agent or in combination with at least one of an A2aR antagonist, an A2bR antagonist, a CD38 inhibitor, a CD39 inhibitor, a CD73 inhibitor and/or a Treg attenuating agent, thereby inhibiting a GPR174-mediated signaling pathway in the cell. In some cases, the cell expresses human GPR174 having the sequence set forth as SEQ ID NO:1 or a naturally occurring variant of GPR174 having at least 90% identity to SEQ ID NO:1 (such as at least 91%, 92%, 93%, 94%, 95%, 96%, 97%, 98% or 99%) identity to SEQ ID NO:1 and to inhibit a GPR174-mediated signaling pathway in cells expressing GPR174. In some cases, the method comprises contacting a cell expressing human GPR174 with an isolated compound predetermined to functionally interact with human GPR174 having the sequence set forth as SEQ ID NO:1 or a naturally occurring variant of GPR174 having at least 90% (such as at least 91%, 92%, 93%, 94%, 95%, 96%, 97%, 98% or 99%) identity to SEQ ID NO:1 and to inhibit a GPR174-mediated signaling pathway in cells expressing GPR174. In some cases, step (i) comprises performing at least one assay to measure functional interaction of said compound with GPR174 prior to contacting a cell in accordance with step (ii).

In either aspect, the compound optionally competitively binds the same region of GPR174 as any one of compounds 1-59, provided below.

In another aspect, the disclosure features a method of using a small molecule chemical compound for inhibiting a GPR174-mediated signaling pathway in a cell, said method comprising the steps of
(a) providing a small molecule chemical compound that functionally interacts with GPR174 and inhibits a GPR174-mediated Gs signaling pathway in cells expressing GPR174, wherein the compound is characterized by at least one of the following criteria:
   (i) said compound has a structure selected from the group consisting of Formula I, II, III, IV, V and VI (or (I), (II), (III), (IV), (V), (Va), or (VI)); or
   (ii) said compound changes the binding affinity of any one of reference compounds 1-59 (as set forth in Table 1) to GPR174; or
   (iii) said compound causes a difference in the inhibitory activity of any one of reference compounds 1-59 (as set forth in Table 1), in a GPR174-mediated signaling pathway assay when tested in the presence of the reference compound as compared to the inhibitory activity of the reference compound alone; and
(b) contacting a cell expressing GPR174 that includes a GPR174-mediated Gs signaling pathway with the compound according to step (a) either as a single agent or in combination with at least one of an A2aR antagonist, an A2bR antagonist, a CD38 inhibitor, a CD39 inhibitor, a CD73 inhibitor and/or a Treg attenuating agent, thereby inhibiting a GPR174-mediated signaling pathway in the cell.

In another aspect, the disclosure features a method of using a small molecule chemical compound for inhibiting a GPR174-mediated signaling pathway in a cell, said method comprising the steps of
(a) providing a small molecule chemical compound that functionally interacts with GPR174 and inhibits a GPR174-mediated Gs signaling pathway in cells expressing GPR174, wherein the compound is characterized by at least one of the following criteria:
   (i) said compound has a structure selected from the group consisting of Formula I, II, III, IV, V and VI (or (I), (II), (III), (IV), (V), (Va), or (VI)); or
   (ii) said compound changes the apparent binding affinity of any one of reference compounds 1-59 (as set forth in Table 1) for GPR174; or
   (iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone; and
(b) contacting a cell expressing GPR174 that includes a GPR174-mediated Gs signaling pathway with the compound according to step (a) either as a single agent or in combination with at least one of an A2aR antagonist, an A2bR antagonist, a CD38 inhibitor, a CD39 inhibitor, a CD73 inhibitor and/or a Treg attenuating agent, thereby inhibiting a GPR174-mediated signaling pathway in the cell.

In some cases, the compound changes the binding affinity of any one of the compounds described herein (*e.g*., any one of reference compounds 1-59 of Table 1). In certain cases, the compound decreases (*i.e.,* inhibits) the binding affinity of any one of reference compounds 1-59 to GPR174. In particular cases, the compound competitively decreases (*i.e.,* competitively inhibits) the binding affinity of any one of reference compounds 1-59 to GPR174. In alternate cases, the compound increases the binding affinity of any one of reference compounds 1-59 to GPR174. In one case, said compound has a structure selected from the group consisting of Formula I-VI. In one case, said compound inhibits lysophosphatidylserine (LysoPS)-dependent activation of the GPR174 receptor in said cell. In one case, said compound inhibits lysophosphatidylserine (LysoPS)-dependent activation of the GPR174 receptor in said cell.

In one case, said compound inhibits phosphatidylserine (PS)-dependent activation of the GPR174 receptor in said cell. As described herein in Examples 16 and 17, the inventors have discovered that phosphatidylserine (PS) is an agonist for GPR174-mediated Gs signaling. Example 17 provides experimental results demonstrating that apoptotic cells stimulate GPR174 Gs signaling pathway in cells expressing GPR174. As further described in Example 16 and shown in FIGURES 47A-47F, PS-mediated GPR174 Gs signaling is inhibited by representative GPR174 inhibitory compounds 6, 10, 11, 20, 23 and 30 which belong to different chemical classes (*i.e.,* Groups I, II and IV). This data demonstrates that compounds 6, 10, 11, 20, 23, and 30 act as GPR174 antagonists and inhibit PS liposome-mediated cAMP signaling. Thus, PS liposome signaling through GPR174 is inhibited by multiple GPR174 inhibitory small molecule compounds with diverse chemical structures.

As described herein, extracellular PS is highly enriched in the tumor microenvironment and is found on the surface of tumor cells as well as endothelial cells of blood vessels permeating solid tumors. Furthermore, apoptotic neutrophils and activated platelets, both of which expose PS, are also recruited to solid tumors (see A.K and Rao D.A., Blood 120:4667-4668, 2012; Schlesinger, M., Journal of Hematology and Oncology (11) 125, 2018; Treffers L.W. et al., Immunological Reviews vol 273: 312-328, 2016; and Gregory A. D. and Houghton A. M., Cancer Research Vol 71 (7): 2411-6, 2011). Thus, high concentrations of PS are considered a major source of tumor-mediated immunosuppression and may play a role in resistance to cancer immunotherapies such as checkpoint inhibitors.

Accordingly, the experimental results described in Examples 16 and 17 provide support for a method of stimulating T-cell mediated immunity in a subject suffering from cancer comprising administering a GPR174 inhibitor either as a single agent or in combination with at least one of an A2aR antagonist, an A2bR antagonist, a CD38 inhibitor, a CD39 inhibitor, a CD73 inhibitor and/or a Treg attenuating agent to said subject in an amount effective to stimulate T-cell mediated immunity as described herein.

As further described herein, the inventors have determined that cancer cell-derived exosomes stimulate GPR174 and inhibition of GPR174 in the presence of cancer cell-derived exosomes enhances Th1 cytokine levels to a greater extent than in the presence of PS liposomes, as described in Example 26. Accordingly, in one case, a method is provided for stimulating and/or amplifying an immune response in a mammalian subject suffering from, or at risk of developing cancer or cancer metastasis, comprising:
(a) determining the presence of cancer cell-derived exosome(s) expressing phosphatidylserine (PS) in a sample obtained from said mammalian subject; and
(b) administering a therapeutically effective amount of a GPR174 inhibitor to said subject.

In some cases, the GPR174 inhibitor is a small molecule that inhibits PS-mediated immune suppression. In some cases, the sample is a tissue, cell or cell extract, or a fluid selected from the group consisting of blood, serum, plasma, sputum, urine, saliva and tears. In some cases, step (a) comprises contacting said sample or exosome extract obtained from said sample with a PS binding agent and thereby determining the presence of said cancer cell-derived exosome. As exosome surface membranes reflect the plasma membrane of their parent cells, exosomes from tumor cells are characterized by having phosphatidylserine (PS) on their surface, as opposed to exosomes from normal cells. Exemplary PS-binding agents include, for example an anti-PS antibody or any other PS-binding agent such as, for example, PKC, protein S, factor VII, factor III and other known PS-binding agents. The PS binding agent may be labeled with a detectable agent, such as a radioactive isotope, a colorimetric label, a fluorescent label, a magnetic resonance label, an enzyme, an affinity ligand or a luminescent label. Exosomes may be extracted from a sample obtained from a subject by various means such as, for example, selective precipitation, affinity purification or by differential centrifugation according to known methods (see, *e.g.,* Zhang Y. et al., Mol Cell 39:133-144, 2010). In some cases, the step (a) comprises determining a quantitative amount of cancer cell-derived exosomes in a sample obtained from a subject. In some cases, the method comprises determining whether or not the subject has an amount of cancer cell-derived exosomes above a threshold indicative of the presence of cancer (*i.e.* one or more tumors). In general, normal tumor-free individuals have undetectable levels of PS-exosomes in an unconcentrated sample. In contrast, patients with tumors will typically exhibit values above 100 pg/50 µL of sample in an unconcentrated sample. Thus, values greater than 50 pg/50 µL plasma (or serum) are indicative of a malignancy (*i.e.,* cancer or one or more tumors).

It is known that PS-expressing exosomes are released from tumor cells and circulating PS-positive tumor exosomes can be detected in biological fluids such as blood and urine. In some cases, said cancer cell-derived exosome(s) expressing PS is from a lung cancer cell, a pancreatic cancer cell, an ovarian cancer cell, a breast cancer cell, a colon cancer cell, a renal cancer cell, a liver cancer cell, a skin cancer cell, a brain cancer cell, a head and neck cancer cell or a thyroid cancer cell, or any other cancer cell type disclosed herein. In some cases, the method further comprises administering to said subject at least one of an A2aR antagonist, an A2bR antagonist, a CD38 inhibitor, a CD39 inhibitor, a CD73 inhibitor and/or a Treg attenuating agent.

In some cases, the GPR174 inhibitory compound changes the apparent binding affinity of any one of the compounds described herein (e.g., any one of reference compounds 1-59 of Table 1) for GPR174. In certain cases, the compound decreases the apparent binding affinity of any one of reference compounds 1-59 for GPR174. In particular cases, the compound competitively binds to GPR174 and decreases the apparent binding affinity of any one of reference compounds 1-59 for GPR174. In alternate cases, the compound allosterically binds to GPR174 and decreases the apparent binding affinity of any one of reference compounds 1-59 for GPR174. In alternate cases, the compound increases the apparent binding affinity of any one of reference compounds 1-59 for GPR174. In one case, said compound has a structure selected from the group consisting of Formula I-VI. In one case, said compound inhibits lysophosphatidylserine (LysoPS)-dependent activation of the GPR174 receptor in said cell. In one case, said compound inhibits PS-dependent activation of the GPR174 receptor in said cell.

The cell expressing GPR174 may be a eukaryotic cell, such as a mammalian cell (*e.g.,* a human cell). The cell may be in a mammal (*e.g.,* a human, non-human primate, rodent, canine, feline, equine, swine or bovine). In some cases, the cell is contacted *in vitro.* In some cases, the cell is in a mammal and is contacted *in vivo.* In some cases, the cell expressing GPR174 is an immune cell, such as a T cell. In particular cases, the mammal (*e.g.,* human) may be suffering from, or at risk for developing cancer.

In another aspect, the disclosure features a compound for use in treating cancer, characterized in that the compound interacts with GPR174 to inhibit a GPR174-mediated signaling pathway (*e.g*., a Gs signaling pathway) wherein the GPR174 inhibitory compound is used for treating cancer either as a single agent or in combination with at least one of an A2aR antagonist, an A2bR antagonist, a CD38 inhibitor, a CD39 inhibitor, a CD73 inhibitor and/or a Treg attenuating agent. In certain cases, this GPR174 inhibitory compound is characterized in that it also exhibits the ability to alter the cellular distribution of a recombinant GPR174 polypeptide modified to include a nuclear localization signal as compared to the cellular distribution of the recombinant GPR174 polypeptide in the absence of the compound. In some cases, the method comprises contacting a cancer cell expressing GPR174 and/or an immune cell expressing GPR174 with an isolated compound predetermined to functionally interact with GPR174 and inhibit a GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent. In some cases, the method comprises contacting an immune cell expressing GPR174, such as a T cell with a compound that inhibits a GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent. In some cases, the method comprises performing at least one assay to measure the functional interaction of said compound with GPR174 prior to administrating said compound to the subject. In another aspect, the disclosure features a compound for use in treating a tumor in a patient suffering from cancer, characterized in that the compound interacts with GPR174 to inhibit a GPR174-mediated signaling pathway (*e.g.,* a Gs signaling pathway) either as a single agent or in combination with at least one of an A2aR antagonist, an A2bR antagonist, a CD38 inhibitor, a CD39 inhibitor, a CD73 inhibitor and/or a Treg attenuating agent. In certain cases, this GPR174 inhibitory compound is characterized in that it also exhibits the ability to alter the cellular distribution of a recombinant GPR174 polypeptide modified to include a nuclear localization signal as compared to the cellular distribution of the recombinant GPR174 polypeptide in the absence of the compound. In some cases, the tumor comprises regulatory T cell (Treg) infiltration. In some cases, the tumor comprising Treg infiltration is a solid organ tumor. In some cases, the tumor is in a subject suffering from a cancer selected from the group consisting of breast, lung (such as small-cell lung cancer or non-small cell lung cancer), colorectal, cervical, renal, ovarian, melanoma, pancreatic, hepatocellular, gastric, glioblastoma, glioma, bladder, myeloma (such as multiple myeloma), prostate, thyroid, testicular, and esophageal cancer. In some cases, the method comprises performing at least one assay to measure the functional interaction of said compound with GPR174 prior to administrating said compound to the subject

In another aspect, the disclosure features a method of treating or preventing cancer in a subject in need thereof. The method involves administering to the subject a compound that binds to and inhibits the GPR174 receptor, thereby inhibiting a GPR174-mediated signaling pathway, wherein inhibition of the GPR174-mediated signaling pathway with a GPR174 inhibitory agent, optionally in combination with at least one of an A2aR antagonist, an A2bR antagonist, a CD38 inhibitor, a CD39 inhibitor, a CD73 inhibitor and/or a Treg attenuating agent, results in treatment or prevention of the cancer. In some cases, the method comprises administrating to the subject an isolated compound predetermined to functionally interact with GPR174 and to inhibit a GPR174-mediated signaling pathway wherein inhibition of the signaling pathway results in the treatment of the cancer. In some cases, said GPR174 inhibitory compound is characterized by at least one of the following criteria:
(i) said compound has a structure selected from the group consisting of Formula I-VI;
(ii) said compound changes the binding affinity of any one of reference compounds 1-59 (as set forth in Table 1) to GPR174; and/or
(iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone. In some cases, said compound inhibits LysoPS-dependent activation of the GPR174 receptor in said subject. In some cases, said compound inhibits PS-dependent activation of the GPR174 receptor in said subject. In some cases, said GPR174 inhibitory compound is characterized by at least one of the following criteria:
   (i) said compound has a structure selected from the group consisting of Formula I-VI;
   (ii) said compound changes the apparent binding affinity of any one of reference compounds 1-59 (as set forth in Table 1) for GPR174; and/or
   (iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone. In some cases, said compound inhibits LysoPS-dependent or PS-dependent activation of the GPR174 receptor in said subject. In another aspect, the disclosure features a method of stimulating T cell-mediated immunity in a subject suffering from cancer comprising administering to the subject a small molecule compound predetermined to inhibit a GPR174-mediated signaling pathway either as a single agent or in combination with at least one of an A2aR antagonist, an A2bR antagonist, a CD38 inhibitor, a CD39 inhibitor and/or a CD73 inhibitor. In some cases, the compound inhibits GPR174-mediated signaling in a regulatory T cell, thereby inhibiting the growth and/or proliferation and/or inhibiting the activity of the regulatory T cell. In some cases, the compound inhibits GPR174-mediated signaling in an effector T cell, thereby stimulating the growth and/or proliferation and/or cytotoxic activity of the effector T cell. In some cases, the subject is suffering from a tumor comprising regulatory T cells and inhibition of GPR174 inhibits and/or suppresses growth and/or proliferation of regulatory T cells. In some cases of any of the above aspects, the cell is in a mammal, and said mammal is suffering from, or at risk for developing a cancer.

In some cases, the subject is suffering from or harboring a neoplasm not recognized by the body as self. In some cases, the neoplasm is a non-malignant neoplasm. In some cases, the neoplasm is a malignant neoplasm.

In some cases, the subject is suffering from, or is harboring, a malignant neoplasm *(i.e.,* cancer), selected from the group consisting of: acoustic neuroma, anal cancer (including carcinoma in situ), squamous cell carcinoma, adrenal tumor (including adenoma, hyperaldosteronism, adrenalcortical cancer), Cushing's syndrome, benign paraganglioma, appendix cancer (including pseudomyxoma peritonei, carcinoid tumors, non-carcinoid appendix tumors), bile duct cancer (including intrahepatic bile duct cancer, extrahepatic bile duct cancer, perihilar bile duct cancer, distal bile duct cancer), gallbladder cancer, bone cancer (including chondrosarcoma, osteosarcoma, malignant fibrous histiocytoma, fibrosarcoma, chordoma), brain tumor (including craniopharyngioma, dermoid cysts, epidermoid tumors, glioma, astrocytoma, low-grade astrocytoma, anaplastic astrocytoma, ependymoma, glioblastoma, oligodendrogliomas, hemangioblastoma, pineal gland tumors, pituitary tumors, sarcoma, chordoma), breast cancer (including lobular carcinoma, triple negative breast cancer, recurrent breast cancer, brain metastases), bladder cancer (including transitional cell bladder cancer, squamous cell carcinoma, adenocarcinoma), cancers of unknown urimary (CUP), (including adenocarcinoma, poorly differentiated carcinoma, squamous cell carcinoma, poorly differentiated malignant neoplasm, neuroendocrine carcinoma), cervical cancer (including squamous cell carcinoma, adenocarcinoma, mixed carcinoma), carcinoid tumors, childhood germ cell tumors (including yolk sac tumors, teratoma, embryonal carcinoma, polyembryoma, germinoma), childhood brain tumors, (including ependymoma, craniopharyngioma, chordoma, pleomorphic xanthoastrocytoma, meningioma, primitive neuroectodermal tumors, ganglioglioma, pineoblastoma, germ cell tumors, mixed glial and neuronal tumors, astrocytoma, choroid plexus tumors), childhood leukemias (including lymphoblastic leukemia, myeloid leukemia), childhood hematology disorders (including Fanconi anemia, Diamond-Blackfan anemia, aplastic anemia, Shwachman-Diamond syndrome, Kostmann's syndrome, Neutropenia, Thrombocytopenia, Hemoglobinopathies, erythrocytosis, histioctic disorders, iron overload, clotting and bleeding disorders), childhood liver cancers (including hepatoblastoma, hepatocellular carcinoma), childhood lymphomas (including Hodgkin's lymphoma, Non-Hodgkin's lymphoma, Burkitt's lymphoma, lymphoblastic lymphoma, large cell lymphoma), childhood osteosarcomas; childhood melanomas; childhood soft tissue sarcomas, colon cancer (including adenocarcinoma, hereditary nonpolyposis colorectal cancer syndrome, familial adenomatous polyposis), desmoplastic Small Round Cell Tumors (DSRCT); esophageal cancers (including adenocarcinoma, squamous cell carcinoma), Ewing's sarcomas (including Ewing's Sarcoma of the bone, extraosseous Ewing tumor, peripheral primitive neuroectodermal tumors), Eye cancers (including uveal melanoma, basal cell carcinoma, squamous cell carcinoma, melanoma of the eyelid, melanoma of the conjunctiva, sebaceous carcinoma, merkel cell carcinoma, mucosa-associated lymphoid tissue lymphoma, orbital lymphoma, orbital sarcoma, orbital and optic nerve meningioma, metastic orbital tumors, lacrimal gland lymphoma, adenoid cystic carcinoma, pleomorphic adenoma, transitional cell carcinoma, lacrimal sac lymphoma); Fallopian tube cancers (including endometrioid adenocarcinoma, serous adenocarcinoma, leiomyosarcoma, transitional cell fallopian tube cancer); Hodgkin's lymphomas (including classical Hodgkin's lymphoma, nodular sclerosing Hodgkin's lymphoma, lymphocyte-rich classical Hodgkin's lymphoma, mixed cellularity Hodgkin's lymphoma, lymphocyte depletion Hodgkin's lymphoma, lymphocyte-predominant Hodgkin's lymphoma), Implant-Associated Anaplastic Large Cell Lymphomas (ALCL); Inflammatory Breast Cancers (IBC); Kidney cancers (including renal cell carcinoma, urothelial cancer of the kidney, pelvis and ureter); Leukemias, (including acute lymphocyte leukemia, acute myeloid leukemia, chronic lymphoblastic leukemia, chronic myeloid leukemia), Liver cancers (including hepatocellular carcinoma, fibrolamellar hepatocellular carcinoma, angiosarcoma, hepatoblastoma, hemangiosarcoma), Lung cancers (including non-small cell lung cancer, adenocarcinoma, squamous cell carcinoma, large cell carcinoma, small cell lung cancer, carcinoid tumor, salivary gland carcinoma, lung metastases, sarcoma); Medulloblastomas; Melanomas (including cutaneous melanoma, superficial spreading melanoma, nodular melanoma, lentigo maligna melanoma, acral lentiginous melanoma, ocular melanoma, mucosal melanoma); Mesotheliomas (including sarcomatoid mesothelioma, biphasic mesothelioma), Multiple Endocrine Neoplasias (MEN), (including multiple endocrine neoplasia type 1, multiple endocrine neoplasia type 2); Multiple Myelomas; Myelodysplastic syndromes (MDS) (including refractory anemia, refractory cytopenia with multilineage dysplasia, refractory anemia with ringed sideroblasts, refractory anemia with excess blasts, refractory cytopenia with multilineage dysplasia and ringed sideroblasts); Myeloproliferative disorders (MPD), (including polycythemia vera, primary myelofibrosis, essential thrombocythemia, systemic mastocytosis, hypereosinophilic syndrome); Neuroblastomas; Neurofibromatosis (including neurofibromatosis type 1, neurofibromatosis type 2, schwannomatosis); Non-Hodgkin's Lymphomas (including b-cell lymphoma, t-cell lymphoma, NK-cell lymphoma, mucosa-associated lymphoid tissue lymphoma, follicular lymphoma, mantle cell lymphoma, diffuse large cell lymphoma, primary mediastinal large cell lymphoma, anaplastic large cell lymphoma, burkitt's lymphoma, lymphoblastic lymphoma, marginal zone lymphoma); Oral cancers (including squamous cell carcinoma); Ovarian cancers (including epithelial ovarian cancer, germ cell ovarian cancer, stromal ovarian cancer, primary peritoneal ovarian cancer); Pancreatic cancers (including islet cell carcinoma, sarcoma, lymphoma, pseudopapillary neoplasms, ampullary cancer, pancreatoblastoma, adenocarcinoma); Parathyroid diseases (including hyperparathyroidism, hypoparathyroidism, parathyroid cancer), Penile cancers (including squamous cell carcinoma, kaposi sarcoma, adenocarcinoma, melanoma, basal cell carcinoma); Pituitary tumors (including non-functioning tumors, functioning tumors, pituitary cancer), Prostate cancers (including adenocarcinoma, prostatic intraepithelial neoplasia), Rectal cancers (including adenocarcinoma), Retinoblastomas (including unilateral retinoblastoma, bilateral retinoblastoma, PNET retinoblastoma), Skin cancers (including basal cell carcinoma, squamous cell carcinoma, actinic (solar) keratosis); Skull base tumors (including meningioma, pituitary adenoma, acoustic neuroma, glomus tumors, squamous cell carcinoma, basal cell carcinoma, adenoid cystic carcinoma, adenocarcinoma, chondrosarcoma, rhabdomyosarcoma, osteosarcoma, esthesioblastoma, neuroendocrine carcinoma, mucosal melanoma), Soft tissue sarcomas; Spinal tumors (including intramedullary spinal tumors, intradural extramedullary spinal tumors, extradural spinal tumors, osteoblastoma, enchondroma, aneurysmal bone cysts, giant cell tumors, hangioma, eosinophilic granuloma, osteosarcoma, chordoma, chondrosarcoma, plasmacytoma); Stomach cancers (including lymphoma, gastrointestinal stromal tumors, carcinoid tumors); Testicular cancers (including germ cell tumors, nonseminoma, seminoma, embryonal carcinoma, yolk sac tumors, teratoma, sertoli cell tumors, choriocarcinoma, stromal tumors, leydig cell tumors); Throat cancers (including squamous cell carcinoma); Thyroid cancers (including papillary thyroid cancer, follicular thyroid cancer, hurthle cell carcinoma, medullary thyroid cancer, anaplastic thyroid cancer); Uterine cancers (including endometrioid adenocarcinoma, uterine carcinosarcoma, uterine sarcoma); Vaginal cancers (including squamous cell carcinoma, adenocarcinoma, melanoma, sarcoma); Vulvar cancers (including squamous cell carcinoma, adenocarcinoma, melanoma, sarcoma); von Hippel Lindau Diseases; Waldenstrom's Macroglobulinemias; and Wilms' Tumors.

In some cases, the subject is suffering from, or is harboring, a malignant or non-malignant neoplasm as set forth in paragraphs 1-10 below:
Connective tissue, (including adult fibrous tissue, embryonic (myxomatous) fibrous tissue, fat, cartilage, bone, notochord):
   1a. benign neoplasms of the connective tissue include: fibroma, myxoma, lipoma, chondroma, osteoma and fibrous histiocytoma.
   1b. malignant neoplasms of the connective tissue include: fibrosarcoma, myxosarcoma, liposarcoma, chondrosarcoma, osteosarcoma, chordoma, malignant fibrosis histiocytoma,
Endothelium and Mesothelium (including blood vessels, lymph vessels and mesothelium):
   2a. benign neoplasms of the endothelium and mesothelium include: hemangioma, hemangiopericytoma and lymphangioma.
   2b. malignant neoplasms of the endothelium and mesothelium include: hemangiosarcoma, angiosarcoma, lymphangiosarcoma and mesothelioma.
Blood and Lymphoid cells (including hematopoietic cells and lymphoid tissue,
   3a. benign neoplasms of the blood and lymphoid cells include: preleukemias, myeloproliferative disorders and plasmacytosis.
   3b. malignant neoplasms of the blood and lymphoid cells include: plasmacytoma, multiple myeloma, Hodgkin lymphoma and Non-Hodgkin lymphoma.
Muscle (including smooth muscle and striated muscle):
   4a. benign neoplasms of the muscle include: leiomyoma and rhabdomyoma.
   4b. malignant neoplasms of the muscle include: leiomyosarcoma and rhabdomyosarcoma.
Epithelial tissues (including stratified squamouse, glandular epithelium (including liver, kidney and bile duct), transitional epithelium, placenta and testis):
   5a. benign neoplasms of epithelial tissues include: papilloma, seborrheic keratosis and skin adnexal tumors, adenoma (including hepatic adenoma, renal tubular adenoma and bile duct adenoma), transitional cell papilloma and hydatidiform mole.
   5b. malignant neoplasms of epithelial tissues include: squamous cell carcinoma; epidermoid carcinoma malignant skin adnexal tumors, adenocarcinoma, hepatoma, hepatocellular carcinoma, renal cell carcinoma, hypernephroma, cholangiocarcinoma, transitional cell carcinoma, choriocarcinoma, seminoma and embryonal cell carcinoma.
Neural tissues (including glial cells, nerve cells, meninges and nerve sheath):
   6a. benign neoplasms of neural tissues include: ganglioneuroma, meningioma, schwannoma, neurilemmoma, and neurofibroma.
   6b. malignant neoplasms of neural tissues include: glioma (grades I-III), anaplastic, glioblastoma multiforme (grade IV), neuroblastoma, medulloblastoma, malignant meningioma, malignant schwannoma, neurofibrosarcoma.
Amine Precursor Uptake and Decarboxylation (APUD) tissues (including pituitary, parathyroid, thyroid (C cells), bronchial lining (Kultschitzky cells), adrenalmedulla, pheochromocytoma, pancreas, stomach and intestines, carotid body and chemo-receptor system):
   7a. benign neoplasms of APUD tissues include: basophilic adenoma, eosinophilic adenoma, chromophobe adenoma, parathyroid adenoma, C cell hyperplasia, pheochromocytoma, Islet celladenoma, insulinoma, gastrinoma, carcinoid, chemodectoma and paraganglioma.
   7b. malignant neoplasms of APUD tissues include: parathyroid carcinoma, medullary carcinoma of thyroid, bronchial carcinoid, oat cell carcinoma, malignant pheochromocytoma, Islet cell carcinoma, malignant carcinoid, malignantcarcinoid, and malignant paraganglioma.
Other Neural Crest-derived cells (including pigment-producing cells in skin, eyes, and occasional other sites, Schwann cells of peripheral nervous system and Merkel cells in squamous epithelium):
   8a. benign neoplasms of neural crest-derived cells include: nevus, schwannoma and neurilemmoma.
   8b. malignant neoplasms of neural crest-derived cells include: melanoma, malignant schwannoma and Merkel cell neoplasm.
Tumors (including breast tissue and renal anlage tissue)
   9a. benign neoplasms of breast tissue include fibroadenoma.
   9b. malignant neoplasms of breast tissue include cystosarcoma phylloids; malignant neoplasms of renal anlage include Wilms tumor.
Ovary and Testis (including multipotential cells, germ cells, connective tissue stroma and epithelial tissue)
   10a. Malignant neoplasms of ovary and testis include: seminoma (dysgerminoma in women), choriocarcinoma, embryonal carcinoma, endodermal sinus tumor, and teratocarcinoma and Sertoli-Leydig cell tumors.

In some cases, the subject is suffering from, or is harboring a non-malignant tumor selected from the group consisting of adenoma (including liver, adrenal, pituitary, thyroid and colon and gastric polyps), astrocytoma, craniopharyngioma, desmoid tumor, enchondroma, fibromas or fibroids of any organ, such as uterine fibroma, ganglioneuroma, hemagioma, lipoma, lipoblastoma, hibernoma, lymphanglioma, meningioma, myoma (including leiomyomas and rhabdomyomas), nevi or moles, neuroma, neurofibroma, schwannoma, osteochondroma and papilloma (including skin, cervix, breast duct and conjunctiva).

In some cases, the subject is a cancer patient, wherein the cancer is selected from the group consisting of breast cancer, melanoma, colon cancer, urological cancer, lung cancer, small-cell and non-small-cell lung cancer, relapsed or refractory malignancies, non-Hodgkin and Hodgkin lymphomas, lymphoma, follicular lymphoma, lymphocytic lymphoma, CNS lymphoma, T-cell lymphoma, AIDS-related lymphoma, acute lymphoblastic leukemia, gastrointestinal cancers, liver cancer, hepatocellular carcinoma, ovarian cancer, pancreatic cancer, bile duct cancer, prostate cancer, renal carcinoma, bladder cancer, colorectal cancer, multiple myeloma, mesothelioma, cervical cancer, vaginal cancer, anal cancer, oropharyngeal cancer, myelogenous leukemia, chronic myeloid leukemia, gastric cancer, nasopharyngeal carcinoma, head and neck carcinoma, glioblastoma, gliosarcoma, squamous cell brain cancer, malignant glioma, diffuse pontine gliomas, esophageal cancer, thyroid cancer, astrocytoma, thoracic cancer, endometrial cancer, cutaneous cell carcinoma, leukemia, acinar cell carcinoma, adenocarcinoma, bronchioloalveolar carcinoma, cholangiocarcinoma, chordoma, giant cell carcinoma, intestinal carcinoma, major salivary gland carcinoma, malignant odontogenic neoplasm, malignant peripheral nerve sheath tumor, skin cancer, testicular cancer, germ cell tumor, neuroendocrine carcinoma, parathyroid carcinoma, pituitary gland carcinoma, placental choriocarcinoma, scrotal cancer, tracheal carcinoma, transitional cell carcinoma, cancer of the uterus, vulvar cancer, kidney cancer, rectum cancer, fallopian tube carcinoma, peritoneal carcinoma, epithelial cancer, pleural mesothelioma, sarcomatoid carcinoma, synovial sarcoma, nephroblastoma, neuroblastoma, adult acute myeloid leukemia, myelodysplastic/myeloproliferative neoplasm, embryonal carcinoma, Kaposi sarcoma, bone cancer, uterine cancer, stomach cancer, carcinoma of the endometrium, cancer of the small intestine, cancer of the endocrine system, cancer of the paragland, cancer of the adrenal gland, sarcoma of soft tissue, cancer of the urethra, cancer of the penis, cancer of the ureter, carcinoma of the pelvis, neoplasm of the central nervous system, primary tumor angiogenesis, spinal axis tumor, epidermoid cancer, environmentally induced cancers including those induced by asbestos, adenosarcoma, adenosquamous carcinoma, adrenocortical carcinoma, astrocytic tumors, basal cell carcinoma, chondosarcoma, Ewing's sarcoma, gallbladder cancer, hypopharyngeal cancer, intraocular melanoma, laryngeal cancer, leiomyosarcoma, lip and oral cavity cancer, malignant mesothelial tumors, malignant thymoma, medulloblastoma, medulloepithelioma, Merkel cell carcinoma, mucoepidermoid carcinoma, myelodysplastic syndrome, nasal cavity and paranasal sinus cancer, osteosarcoma, pulmonary blastoma, pineal and supratentorial primitive neuroectodermal tumors, plasma cell neoplasm, retinoblastoma, rhabdomyosarcoma, sarcoma, neuroectodermal tumors and Wilm's tumor.

In some cases, the subject is a cancer patient suffering from, or harboring a solid tumor. In some cases, the cancer patient has a solid tumor that is infiltrated with lymphocyte cells that express GPR174.

In some cases, the subject has one or more tumors infiltrated with regulatory T cells, such as, for example, breast, lung (such as small-cell lung cancer or non-small cell lung cancer), colorectal, cervical, renal, ovarian, melanoma, pancreatic, hepatocellular, gastric, glioblastoma, glioma, bladder, myeloma (such as multiple myeloma), prostate, thyroid, testicular, and esophageal cancer.

In some cases, the method comprises administering a GPR174 inhibitory compound as a single agent. In some cases, the method comprises administering a GPR174 inhibitory compound in combination with at least one of an A2aR antagonist, an A2bR antagonist, a CD38 inhibitor, a CD39 inhibitor, a CD73 inhibitor and/or a Treg attenuating agent. In some cases, the method comprises administering a GPR174 inhibitory compound in combination with an A2aR antagonist. In some cases, the method comprises administering a GPR174 inhibitory compound in combination with an A2bR antagonist. In some cases, the method comprises administering a GPR174 inhibitory compound in combination with a Treg attenuating agent, such as, for example, an agent that binds to one or more of GITR, CTLA-4, CD25, LAG3, TIGIT, NRP1, TGF-β, CCR2, CCR4, CCR8, TNFR2, and/or EZH2 and attenuates, depletes or otherwise impairs the tumor suppressor function of Treg cells. In some cases, the Treg attenuating agent is a small molecule. In some cases, the Treg attenuating agent is selected from the group consisting of an anti-GITR, anti-CTLA-4, anti-CD25, anti-LAG3, anti-TIGIT, anti-NRP1, anti-TGF-β, anti-CCR2, anti-CCR4, anti-CCR8, anti-TNFR2, and/or anti-EZH2 antibody. Exemplary Treg attenuating agents for use in the compositions and methods of the disclosure are provided in Example 25.

In some cases, the method further includes administering one or more additional therapeutic agents. In certain cases, the cancer cell is further contacted with a chemotherapeutic agent.

In another aspect, the disclosure features a pharmaceutical composition including a combination of a GPR174 inhibitor selected from any of the compounds described below (e.g., those of Formulas I-VI and the compounds 1-59 of Table 1) and an inhibitor of ATP-Adenosine-A2aR-A2bR-mediated signaling (such as an A2aR antagonist, an A2bR antagonist and/or a CD73 inhibitor and/or a CD38 inhibitor and/or a CD39 inhibitor and/or a Treg attenuating agent) and a pharmaceutically acceptable carrier. The pharmaceutical composition may be in unit dosage form. In particular cases, the compound may be formulated for oral, intravenous, intraperitoneal, intramuscular, topical, rectal, cutaneous, subcutaneous, nasal, skin *(e.g.,* as a transdermal patch), intracerebroventricular, intraparenchymal, intrathecal, inhalational, intracranial or ocular administration. The pharmaceutical compositions may be used to carry out the methods disclosed herein.

In another aspect, the disclosure features compounds of Formula I that have activity as GPR174 inhibitors. In some cases, the compounds of Formula I have a structure in accordance with Formula IA or Formula IB or Formula IC as described herein. In some cases, the disclosure features pharmaceutical compositions comprising a compound of Formula I, such as a compound in accordance with Formula IA or Formula IB or Formula IC as described herein. Compounds of Formula I are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features compounds of Formula II that have activity as GPR174 inhibitors. In some cases, the compounds of Formula II have a structure in accordance with Formula IIA, Formula IIB, Formula IIC, Formula IID or Formula IIE as described herein. In some cases, the disclosure features pharmaceutical compositions comprising a compound of Formula II, such as a compound in accordance with Formula IIA, Formula IIB, Formula IIC, Formula IID or Formula IIE as described herein. Compounds of Formula II are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features compounds of Formula III that have activity as GPR174 inhibitors. In some cases, the compounds of Formula III have a structure in accordance with Formula IIIA, Formula IIIB, Formula IIIC, Formula IIID, Formula IIIE, or Formula IIIF as described herein. In some cases, the disclosure features pharmaceutical compositions comprising a compound of Formula III, such as a compound in accordance with Formula IIIA, Formula IIIB, Formula IIIC, Formula IIID, Formula IIIE or Formula IIIF as described herein. Compounds of Formula III are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features compounds of Formula IV that have activity as GPR174 inhibitors. In some cases, the compounds of Formula IV have a structure in accordance with Formula IVA, Formula IVB, Formula IVC, Formula IVD, or Formula IVE as described herein. In some cases, the disclosure features pharmaceutical compositions comprising a compound of Formula IV, such as a compound in accordance with Formula IVA, Formula IVB, Formula IVC, Formula IVD or Formula IVE as described herein. Compounds of Formula IV are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features compounds of Formula V that have activity as GPR174 inhibitors. In some cases, the disclosure features pharmaceutical compositions comprising a compound of Formula V as described herein. Compounds of Formula V are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features compounds of Formula Va that have activity as GPR174 inhibitors. In some cases, the disclosure features pharmaceutical compositions comprising a compound of Formula Va as described herein. Compounds of Formula Va are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor, and/or a Treg attenuating agent for treating or preventing cancer as described herein.

In another aspect, the disclosure features compounds of Formula VI that have activity as GPR174 inhibitors. In some cases, the disclosure features pharmaceutical compositions comprising a compound of Formula VI as described herein. Compounds of Formula VI are useful in the methods of inhibiting at least one GPR174-mediated signaling pathway either as a single agent or in combination with at least one of an A2aR antagonist, an A2bR antagonist, a CD38 inhibitor, a CD39 inhibitor, a CD73 inhibitor and/or a Treg attenuating agent for treating or preventing cancer as described herein.

In another aspect, the disclosure features a method of preparing a pharmaceutical composition including a compound that is an inhibitor of a GPR174-mediated signaling pathway, the compound having been contacted *in vitro* with a mammalian cell expressing GPR174 and determined to inhibit a GPR174-mediated signaling pathway, wherein the ability of the compound to inhibit said GPR174-mediated signaling pathway is indicative of being a GPR174 inhibitor, the method comprising formulating the GPR174 inhibitory compound with a pharmaceutically acceptable carrier either as a single agent or in combination with at least one of an A2aR antagonist, an A2bR antagonist, a CD38 inhibitor, a CD39 inhibitor, a CD73 inhibitor and/or a Treg attenuating agent, wherein said GPR174 inhibitory compound is characterized by at least one of the following criteria:
(i) said compound has a structure selected from the group consisting of Formula I-VI;
(ii) said compound changes the binding affinity of any one of reference compounds 1-59 (as set forth in Table 1) to GPR174; and/or
(iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone.

In another aspect, the disclosure features a method of preparing a pharmaceutical composition including a compound that is an inhibitor of a GPR174-mediated signaling pathway, the compound having been contacted *in vitro* with a mammalian cell expressing GPR174 and determined to inhibit a GPR174-mediated signaling pathway, wherein the ability of the compound to inhibit said GPR174-mediated signaling pathway is indicative of being a GPR174 inhibitor, the method comprising formulating the GPR174 inhibitory compound with a pharmaceutically acceptable carrier, either as a single agent or in combination with at least one of an A2aR antagonist, an A2bR antagonist, a CD38 inhibitor, a CD39 inhibitor, a CD73 inhibitor and/or a Treg attenuating agent wherein said GPR174 inhibitory compound is characterized by at least one of the following criteria:
(i) said compound has a structure selected from the group consisting of Formula I-VI;
(ii) said compound changes the apparent binding affinity of any one of reference compounds 1-59 (as set forth in Table 1) to GPR174; and/or
(iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone.

In another case, the disclosure features a GPR174-interacting compound comprising a structure according to any of Formulas I-VI disclosed herein and a detectable moiety. In some cases, the GPR174-interacting compound is characterized by at least one of the following criteria:
(i) said compound has a structure selected from the group consisting of Formula I-VI;
(ii) said compound changes the binding affinity of any one of reference compounds 1-59 (as set forth in Table 1) to GPR174; and/or
(iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone. In one case, the GPR174-interacting compound comprising a detectable moiety comprises a structure according to any of compounds 1-59 disclosed in Table 1. In particular cases, the detectable moiety is at least one of a radioisotope, a bioluminescent tag, a chemiluminescent tag, or a photo-affinity label. In one case, the detectable moiety is a radioisotope.

In another case, the disclosure features a GPR174-interacting compound comprising a structure according to any of Formulas I-VI disclosed herein and a detectable moiety. In some cases, the GPR174-interacting compound is characterized by at least one of the following criteria:
(i) said compound has a structure selected from the group consisting of Formula I-VI;
(ii) said compound changes the apparent binding affinity of any one of reference compounds 1-59 (as set forth in Table 1) to GPR174; and/or
(iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone. In one case, the GPR174-interacting compound comprising a detectable moiety comprises a structure according to any of compounds 1-59 disclosed in Table 1. In particular cases, the detectable moiety is at least one of a radioisotope, a bioluminescent tag, a chemiluminescent tag, or a photo-affinity label. In one case, the detectable moiety is a radioisotope.

In any of the above aspects, the GPR174 inhibitory compound may be a synthetic compound, a semi-synthetic compound (*e.g.,* a chemically modified steroid derived from a natural source), or a natural product. In certain cases, the compound is not an endogenous ligand of GPR174 (*e.g.,* is a surrogate ligand). In various cases, the compound is a functional inhibitor of a GPR174-mediated signaling pathway (*e.g.,* an antagonist, partial agonist, inverse agonist, partial inverse agonist, or negative allosteric modulator). In some cases, the compound is an antagonist of GPR174 and inhibits at least one GPR174-mediated signaling pathway in the presence of an agonist or partial agonist. In some cases, the compound is an inverse agonist. In specific cases, the compound decreases GPR174-mediated signaling pathway activation at least 2/3, 1/2 ,1/5, 1/10, 1/50, or 1/100 of the basal activity at a concentration less than 40, 25, 10, 5, 1, 0.5, 0.25, 0.1, or 0.5 µM (*e.g.,* from 0.01 µM to 0.5 µM, from 0.01 µM to 0.5 µM, from 0.01 µM to 5 µM, from 0.01 µM to 10 µM, from 0.01 µM to 25 µM, or from 0.01 µM to 40 µM). In some cases, the compound is an inverse agonist and decreases at least one GPR174-mediated signaling pathway by at least 1/2 in comparison to basal activity at a concentration of less than 10 µM (*e.g.,* from 0.01 µM to 10 µM).

The GPR174 inhibitory compound may be a polypeptide, an antibody, a non-peptide compound, an expression inhibitor (*e.g*., GPR174 antisense nucleic acid molecules such as antisense RNA, antisense DNA or antisense oligonucleotides, GPR174 ribozymes or GPR174 RNAi molecules) that inhibits GPR174 expression, or a small molecule (*e.g.,* a small organic or organometallic molecule).

In some cases, the compound was not in development by any entity prior to January 11, 2019. In some cases, the compound is not a regulatory agency-approved compound. In some cases, the compound is a compound that was not approved prior to January 11, 2019 by the United States Food and Drug Administration or other regulatory authority for use in a disease associated with GPR174. In some cases, the compound was not in development by any entity prior to January 11, 2019 for use in a disease associated with GPR174. In some cases, the compound was not approved prior to January 11, 2019 by the United States Food and Drug Administration or equivalent regulatory authority as targeting GPR174. In particular cases, the compound is not an antibody or a receptor-binding fragment thereof. In particular cases, the compound is not a pepducin, a cell-penetrating membrane-tethered peptide (see, *e.g.,* Covic L. et al., PNAS 99(2):643-8 (2002)).

In particular cases of any aspect, the GPR174 inhibitory isolated compound is not LysoPS or PS.

In certain cases, the GPR174 inhibitory compound is capable of functionally interacting with human GPR174 having the sequence of SEQ ID NO: 1 and/or altering the cellular distribution of a recombinant GPR174 polypeptide (e.g., human GPR174 having the sequence of SEQ ID NO: 1) modified to include a nuclear localization signal as compared to the cellular distribution of the recombinant GPR174 polypeptide in the absence of the compound.

The compound may be specific for GPR174, *e.g.,* as compared to a reference panel of GPCRs.

In particular cases of any aspect, the isolated compound selectively binds to GPR174 and does not bind to P2Y10.

In particular cases of any aspect, the isolated compound selectively binds to GPR174 and does not bind to GPR34.

In particular cases of any aspect, the isolated compound functionally interacts with GPR174 and does not bind to P2Y10.

In particular cases of any aspect, the isolated compound functionally interacts with GPR174 and does not bind to GPR34.

In particular cases of any aspect, the isolated compound is not disclosed in US2015/0361119.

In addition, the methods and compositions of any of the aspects of the disclosure may employ any compound having a structure according to the formulas described below, such as the exemplary compounds in Table 1, or a pharmaceutically acceptable salt thereof.

In some cases, the GPR174 inhibitory compound has a structure according to the following formula (I): 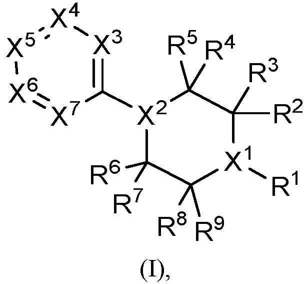
or a stereoisomer thereof, or a pharmaceutically acceptable salt thereof, wherein,
X¹ is N or CR¹⁰;
X² is N or CR¹¹;
X³ is N or CR¹²;
X⁴ is N or CR¹³;
X⁵ is N or CR¹⁴;
X⁶ is N or CR¹⁵;
X⁷ is N or CR¹⁶;
each of R¹, R², R³, R⁴, R⁵, R⁶, R⁷, R⁸, and R⁹ is, independently, H, hydroxy, thiol, optionally substituted amino, optionally substituted amido, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl; or
R² and R³ combine to form =O, =S, or =NR¹⁷; or
R⁴ and R⁵ combine to form =O, =S, or =NR¹⁷; or
R⁶ and R⁷ combine to form =O, =S, or =NR¹⁷; or
R⁸ and R⁹ combine to form =O, =S, or =NR¹⁷;
each of R¹⁰, R¹¹, R¹², R¹³, R¹⁴, R¹⁵, and R¹⁶ is, independently, H, hydroxy, halogen, thiol, optionally substituted amino, optionally substituted amido, cyano, nitro, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl; or
one of:
   (i) R¹² and R¹³, together with the atoms to which each is attached, combine to form an optionally substituted 5-, 6-, or 7-member ring;
   (ii) R¹³ and R¹⁴, together with the atoms to which each is attached, combine to form an optionally substituted 5-, 6-, or 7-member ring;
   (iii) R¹⁴ and R¹⁵, together with the atoms to which each is attached, combine to form an optionally substituted 5-, 6-, or 7-member ring; and
   (iv) R¹⁵ and R¹⁶, together with the atoms to which each is attached, combine to form an optionally substituted 5-, 6-, or 7-member ring;
      and
R¹⁷ is H, hydroxyl, cyano, optionally substituted amino, optionally substituted amido, optionally substituted carboxamide, optionally substituted C₁-C₆ alkyl, optionally substituted C₂-C₆ alkenyl, optionally substituted C₂-C₆ alkynyl, optionally substituted C₁-C₆ alkanoyl, optionally substituted C₇-C₁₁ aryloyl, optionally substituted C₂-C₁₀ heterocyclyloyl, optionally substituted C₂-C₁₀ heteroaryloyl, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
wherein three or fewer of X³, X⁴, X⁵, X⁶, and X⁷ are N; and
at least one of X¹ and X² is N.

In some cases of formula (I), X¹ is N. In certain cases of formula (I), X² is N. In particular cases of formula (I), X³ is CR¹². In other cases of formula (I), X⁴ is CR¹³. In yet other cases of formula (I), X⁵ is CR¹⁴. In still other cases of formula (I), X⁶ is CR¹⁵. In certain other cases of formula (I), X⁷ is CR¹⁶.

In particular cases of formula (I), the isolated compound has the structure according to formula (IA):

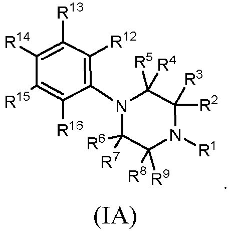

In certain cases of formula (I) or (IA), R² is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In some cases of formula (I) or (IA), R² is H or optionally substituted C₁-C₆ alkyl. In other cases of formula (I) or (IA), R² is H.

In yet other cases of formula (I) or (IA), R³ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In still other cases of formula (I) or (IA), R³ is H or optionally substituted C₁-C₆ alkyl. In particular cases of formula (I) or (IA), R³ is H.

In some cases of formula (I) or (IA), R⁴ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (I) or (IA), R⁴ is H or optionally substituted C₁-C₆ alkyl. In particular cases of formula (I) or (IA), R⁴ is H.

In other cases of formula (I) or (IA), R⁵ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In yet other cases of formula (I) or (IA), R⁵ is H or optionally substituted C₁-C₆ alkyl. In still other cases of formula (I) or (IA), R⁵ is H.

In particular cases of formula (I) or (IA), R⁶ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (I) or (IA), R⁶ is H or optionally substituted C₁-C₆ alkyl. In some cases of formula (I) or (IA), R⁶ is H.

In certain cases of formula (I) or (IA), R⁷ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (I) or (IA), R⁷ is H or optionally substituted C₁-C₆ alkyl. In still other cases of formula (I) or (IA), R⁷ is H.

In some cases of formula (I) or (IA), R⁸ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (I) or (IA), R⁸ is H or optionally substituted C₁-C₆ alkyl. In particular cases of formula (I) or (IA), R⁸ is H.

In other cases of formula (I) or (IA), R⁹ is H, optionally substituted C₁-C₆ alkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In yet other cases of formula (I) or (IA), R⁹ is H or optionally substituted C₁-C₆ alkyl. In still other cases of formula (I) or (IA), R⁹ is H.

In particular cases of formula (I) or (IA), R¹³ is H, hydroxy, optionally substituted amino, optionally substituted C₁-C₆ alkyl, optionally substituted C₁-C₆ heteroalkyl, optionally substituted C₃-C₁₀ cycloalkyl, optionally substituted C₁-C₆ alkoxy, optionally substituted C₆-C₁₀ aryloxy, optionally substituted C₁-C₉ heteroaryloxy, optionally substituted C₆-C₁₀ aryl, optionally substituted C₁-C₉ heteroaryl, or optionally substituted C₁-C₉ heterocyclyl. In certain cases of formula (I) or (IA), R¹³ is H or optionally substituted C₁-C₆ alkyl. In other cases of formula (I) or (IA), R¹³ is H.

In certain cases of formula (I) or (IA), R¹⁶ is H, halogen, cyano, nitro, optionally substituted C₁-C₆ alkyl, optionally substituted C₃-C₁₀ cycloalkyl, optionally substituted C₆-C₁₀ aryl, optionally substituted C₁-C₉ heteroaryl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, or optionally substituted C₁-C₉ heterocyclyl. In other cases of formula (I) or (IA), R¹⁶ is H or optionally substituted C₁-C₆ alkyl. In yet other cases of formula (I) or (IA), R¹⁶ is H.

In some cases of formula (I), the compound has the structure according to formula (IB):

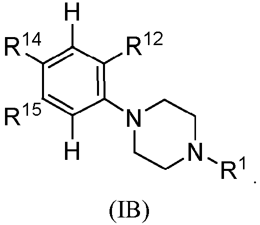

In certain cases of formula (I), (IA), or (IB), R¹² is H, halogen, cyano, nitro, optionally substituted C₁-C₆ alkyl, optionally substituted C₁-C₆ alkoxy, optionally substituted C₁-C₆ alkanoyl, optionally substituted C₇-C₁₁ aryloyl, optionally substituted C₂-C₁₀ heterocyclyloyl, optionally substituted C₂-C₁₀ heteroaryloyl, optionally substituted C₁-C₆ alkylsulfonyl, optionally substituted C₆-C₁₀ arylsulfonyl, optionally substituted C₁-C₉ heteroarylsulfonyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₉ heteroaryl, or optionally substituted C₁-C₉ heterocyclyl. In particular cases of formula (I), (IA), or (IB), R¹² is H, halogen, cyano, nitro, optionally substituted C₁-C₆ alkyl, optionally substituted C₁-C₆ alkoxy, optionally substituted C₁-C₆ alkanoyl, optionally substituted C₁-C₆ alkylsulfonyl, hydroxycarbonyl, optionally substituted ester, optionally substituted carboxamide, optionally substituted C₁-C₆ alkanoyloxy, optionally substituted C₁-C₉ heteroaryl, or optionally substituted C₁-C₉ heterocyclyl. In other cases of formula (I), (IA), or (IB), R¹² is H, halogen, cyano, nitro, optionally substituted C₁-C₆ alkyl, optionally substituted ester, optionally substituted carboxamide, optionally substituted C₁-C₆ alkanoyloxy, or optionally substituted C₁-C₉ heteroaryl. In yet other cases of formula (I), (IA), or (IB), R¹² is H, halogen, nitro, optionally substituted ester, or optionally substituted C₁-C₆ alkanoyloxy. In still other cases of formula (I), (IA), or (IB), R¹² is halogen *(e.g.,* R¹² is fluorine). In certain cases of formula (I), (IA), or (IB), R¹² is nitro.

In particular cases of formula (I), (IA), or (IB), R¹⁴ is H, halogen, cyano, nitro, optionally substituted C₁-C₆ alkyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkanoyl, optionally substituted C₇-C₁₁ aryloyl, optionally substituted C₂-C₁₀ heterocyclyloyl, optionally substituted C₂-C₁₀ heteroaryloyl, optionally substituted C₁-C₆ alkylsulfonyl, optionally substituted C₆-C₁₀ arylsulfonyl, optionally substituted C₁-C₉ heteroarylsulfinyl, optionally substituted C₆-C₁₀ aryl, optionally substituted C₁-C₉ heteroaryl, or optionally substituted C₁-C₉ heterocyclyl. In some cases of formula (I), (IA), or (IB), R¹⁴ is H, halogen, cyano, optionally substituted C₁-C₆ alkyl, optionally substituted ester, optionally substituted carboxamide, optionally substituted C₁-C₆ alkanoyloxy, optionally substituted C₁-C₆ alkanoyl, optionally substituted C₇-C₁₁ aryloyl, optionally substituted C₂-C₁₀ heterocyclyloyl, optionally substituted C₂-C₁₀ heteroaryloyl, optionally substituted C₁-C₆ alkylsulfonyl, optionally substituted C₆-C₁₀ arylsulfonyl, or optionally substituted C₁-C₉ heteroaryl. In other cases of formula (I), (IA), or (IB), R¹⁴ is H, halogen, optionally substituted C₁-C₆ alkyl, optionally substituted ester, optionally substituted carboxamide, optionally substituted C₁-C₆ alkanoyloxy, optionally substituted C₁-C₆ alkanoyl. In yet other cases of formula (I), (IA), or (IB), R¹⁴ is halogen, optionally substituted C₁-C₆ alkyl, or optionally substituted C₁-C₆ alkanoyl. In still other cases of formula (I), (IA), or (IB), R¹⁴ is halogen (*e.g.,* R¹⁴ is fluorine). In some cases of formula (I), (IA), or (IB), R¹⁴ is optionally substituted C₁-C₆ alkanoyl. In particular cases of formula (I), (IA), or (IB), R¹⁴ is optionally substituted C₂-C₄ alkanoyl. In certain cases of formula (I), (IA), or (IB), R¹⁴ is unsubstituted C₂-C₄ alkanoyl.

In some cases of formula (I), (IA), or (IB), R¹⁵ is H, optionally substituted amino, optionally substituted amido, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (I), (IA), or (IB), R¹⁵ is H, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₁-C₆ heteroalkyl, optionally substituted C₃-C₁₀ cycloalkyl, optionally substituted C₆-C₁₀ aryl, optionally substituted C₁-C₉ heteroaryl, or optionally substituted C₁-C₉ heterocyclyl. In particular cases of formula (I), (IA), or (IB), R¹⁵ is H or optionally substituted C₁-C₉ heterocyclyl. In other cases of formula (I), (IA), or (IB), R¹⁵ is H. In yet other cases of formula (I), (IA), or (IB), R¹⁵ is optionally substituted C₁-C₉ heterocyclyl (e.g., R¹⁵ is piperidinyl, methyl-substituted piperidinyl or benzpiperidinyl).

In some cases of Formula (IB), R¹ is selected from the group consisting of C₁₋C₆ alkanoyl, C₆-C₁₀ aryl, C₇-C₁₁ aryloyl, C₂-C₁₀ heteroaryloyl, C₂-C₇ alkoxycarbonyl, and C₆-C₁₀ arylsulfonyl, wherein R¹ is optionally substituted;
R¹² is H, nitro, or halogen;
R¹⁴ is C₁-C₆ alkanoyl or halogen; and
R¹⁵ is H or optionally substituted C₁-C₉ heterocyclyl.

In some cases of Formula (IB), R¹ is selected from an optionally substituted group consisting of C₁₋C₆-alkanoyl, C₇-C₁₁ aryloyl, C₂-C₁₀ heteroaryloyl, C₂-C₇ alkoxycarbonyl, and C₆-C₁₀ arylsulfonyl.

In some cases of formula (IB), R¹² is nitro, and R¹⁴ is fluoro.

In some cases of formula (IB), R¹⁵ is optionally substituted piperidin-1-yl or optionally substituted azepan-1-yl. In some cases of formula (I), (IA), or (IB), the compound has the structure according to formula (IC): 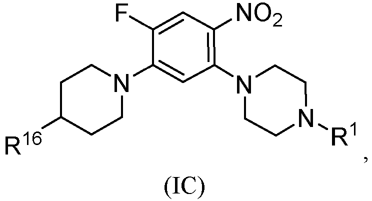 wherein R¹⁶ is H or C₁-C₆ alkyl.

In some cases of formula (IC), R¹⁶ is H or methyl.

In particular cases of formula (I), (IA), (IB), or (IC), R¹ is H, optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted ester, optionally substituted carboxamide, optionally substituted C₁-C₆ alkylsulfonyl, substituted C₆-C₁₀ arylsulfonyl, optionally substituted C₁-C₉ heteroarylsulfonyl, optionally substituted C₁-C₉ heterocyclylsulfonyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (I), (IA), (IB), or (IC), R¹ is optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted ester, optionally substituted carboxamide, optionally substituted C₁-C₆ alkylsulfonyl, substituted C₆-C₁₀ arylsulfonyl, optionally substituted C₁-C₉ heteroarylsulfonyl, optionally substituted C₁-C₉ heterocyclylsulfonyl, optionally substituted C₆-C₁₀ aryl C₁-C₆ alkyl, optionally substituted C₁-C₉ heteroaryl C₁-C₆ alkyl, or optionally substituted C₁-C₉ heterocyclyl C₁-C₆ alkyl. In particular cases of formula (I), (IA), (IB), or (IC), R¹ is optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted ester, optionally substituted carboxamide, optionally substituted C₁-C₆ alkylsulfonyl, substituted C₆-C₁₀ arylsulfonyl, optionally substituted C₁-C₉ heteroarylsulfonyl, optionally substituted C₆-C₁₀ aryl C₁-C₆ alkyl, or optionally substituted C₁-C₉ heteroaryl C₁-C₆ alkyl. In some cases of formula (I), (IA), (IB), or (IC), R¹ is optionally substituted C₁-C₆ alkyl, optionally substituted C₂-C₆ alkanoyl, optionally substituted C₇-C₁₁ aryloyl, optionally substituted ester, optionally substituted C₆-C₁₀ arylsulfonyl, or optionally substituted C₆-C₁₀ aryl C₁-C₆ alkyl. In other cases of formula (I), (IA), (IB), or (IC), R¹ is optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted C₆-C₁₀ arylsulfonyl, or optionally substituted ester. In yet other cases of formula (I), (IA), (IB), or (IC), R¹ is optionally substituted C₂-C₇ alkoxycarbonyl (*e.g*., methyloxycarbonyl or ethyloxycarbonyl). In still other cases of formula (I), (IA), (IB), or (IC), R¹ is optionally substituted C₂-C₆ alkanoyl (*e.g.,* R¹ is acetyl, propanoyl, n-butanoyl, isobutanoyl, or t-pentanoyl). In some cases of formula (I), (IA), (IB), or (IC), R¹ is optionally substituted C₇-C₁₁ aryloyl (*e.g.,* R¹ is 4-fluorobenzoyl or benzoyl). In other cases of formula (I), (IA), (IB), or (IC), R¹ is optionally substituted C₂-C₁₀ heteroaryloyl (e.g., R¹ is 2-thiophenecarbonyl). In certain cases of formula (I), (IA), (IB), or (IC), R¹ is optionally substituted C₆-C₁₀ arylsulfonyl (e.g., R¹ is *p*-tolylsulfonyl or phenylsulfonyl). In other cases of formula (I), (IA), (IB), or (IC), R¹ is optionally substituted C₁-C₆ alkyl, (*e.g.,* R¹ is ethyl or methyl).

In particular cases of formula (I), (IA), (IB), or (1C), the isolated compound is compound 1, 2, 3, 4, 5, 6, 7, 8, 9, 10, 11, 12, 13, 14, 15, 16, 17, 18, or 53:

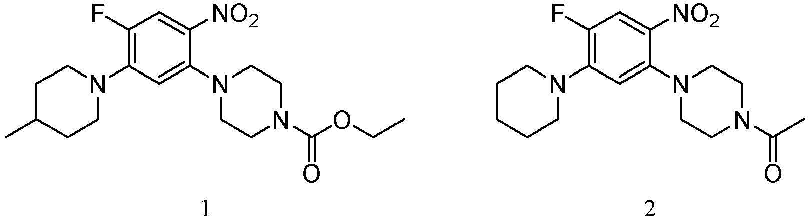

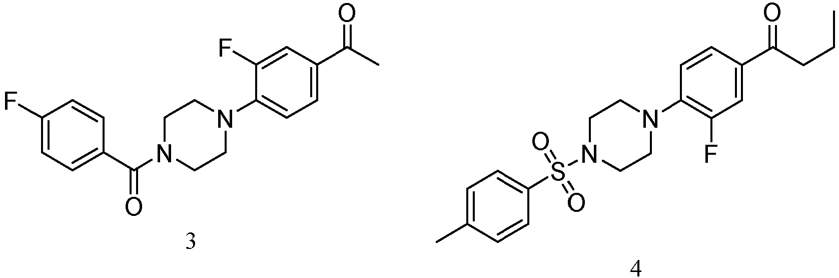

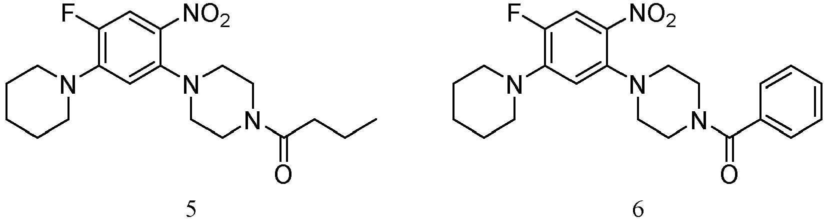

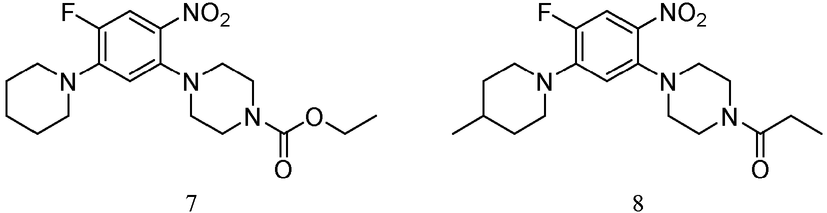

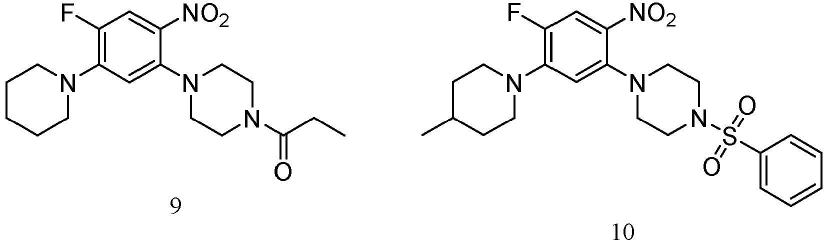

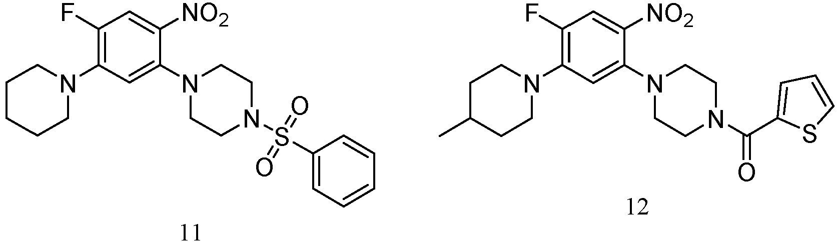

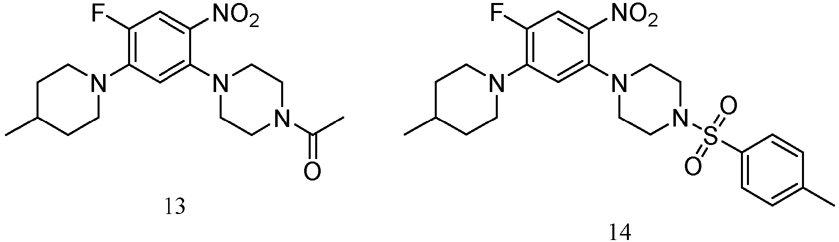

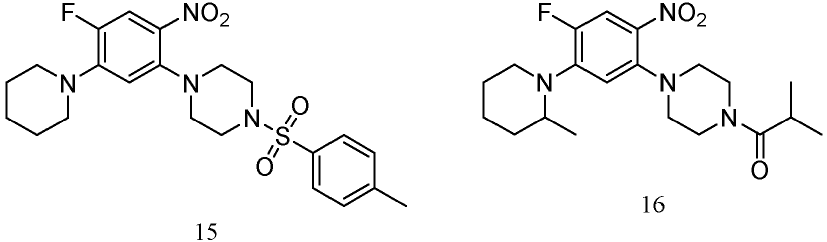

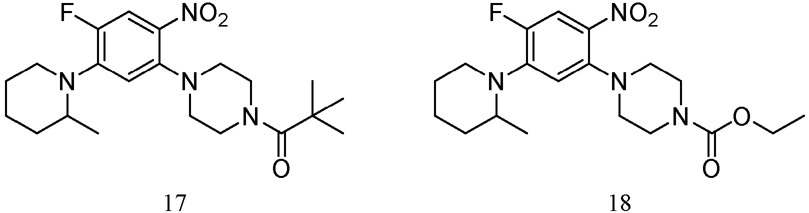

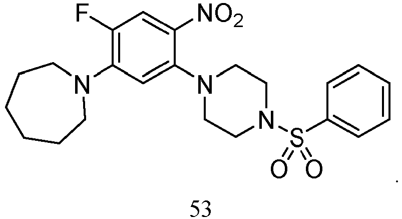

In a particular case of formula (IB), the isolated compound is compound 53:

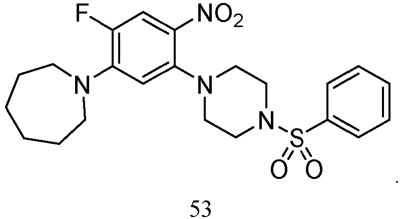

In some cases, the GPR174 inhibitory compound has a structure according to formula (II): 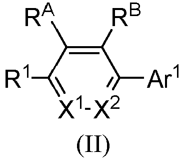
or a stereoisomer thereof, or a pharmaceutically acceptable salt thereof, wherein,
X¹ is N or CR²;
X² is N or CR³;
R^{A} and R^{B}, together with the atoms to which is attached combine to form an optionally substituted 5-membered ring, optionally substituted 6-membered ring, or optionally substituted 7-membered ring;
R¹ is H, halo, hydroxy, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted sulfamoyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl; and
Ar¹ is optionally substituted C₆-C₁₀ aryl, optionally substituted C₁-C₉ heteroaryl, or optionally substituted C₁-C₉ heterocyclyl.

In some cases of formula (II), R^{A} and R^{B}, together with the atoms to which each is attached, combine to form an optionally substituted carbocyclic ring. In certain cases of formula (II), R^{A} and R^{B}, together with the atoms to which each is attached, combine to form an optionally substituted heterocyclic ring. In particular cases of formula (II), R^{A} and R^{B}, together with the atoms to which each is attached, combine to form an optionally substituted 6-membered ring. In other cases of formula (II), R^{A} and R^{B}, together with the atoms to which each is attached, combine to form an optionally substituted non-aromatic ring. In yet other cases of formula (II), R^{A} and R^{B}, together with the atoms to which each is attached, combine to form an optionally substituted aromatic ring.

In still other cases of formula (II), the isolated compound has a structure according to formula (IIA): 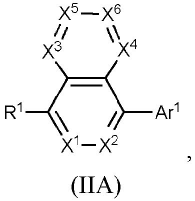 wherein
X³ is N, CR⁴;
X⁴ is N, CR⁵;
X⁵ is N, CR⁶;
X⁶ is N, CR⁷, or absent; and
each of R⁴, R⁵, R⁶, and R⁷ is, independently, H, halo, hydroxy, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted sulfamoyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
wherein
three or fewer of X¹, X², X³, X⁴, X⁵, and X⁶ are N.

In particular cases of formula (IIA), X³ is CR⁴.

In still other cases of formula (IIA), X⁴ is CR⁵.

In other cases of formula (IIA), X⁵ is CR⁶. In yet other cases of formula (IIA), X⁶ is CR⁷.

In some cases of formula (II) or (IIA), X¹ is N. In certain cases of formula (II) or (IIA), X² is N.

In still other cases of formula (II), the isolated compound has a structure of formula (IIB):

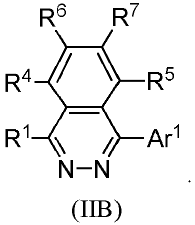

In some cases of formula (IIA) or (IIB), R⁴ is H, optionally substituted amino, halo, optionally substituted amido, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfonyl, optionally substituted C₆-C₁₀ arylsulfonyl, optionally substituted C₁-C₉ heteroarylsulfonyl, optionally substituted C₁-C₉ heterocyclylsulfonyl, optionally substituted sulfamoyl, optionally substituted C₁-C₆ thioalkyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (IIA) or (IIB), R⁴ is H, optionally substituted amino, halo, optionally substituted amido, cyano, optionally substituted C₁-C₆ alkyl, optionally substituted C₂-C₇ alkoxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfonyl, optionally substituted C₆-C₁₀ arylsulfonyl, optionally substituted sulfamoyl, optionally substituted C₁-C₆ heteroalkyl, optionally substituted C₆-C₁₀ aryl, optionally substituted C₁-C₉ heteroaryl, or optionally substituted C₁-C₉ heterocyclyl. In particular cases of formula (IIA) or (IIB), R⁴ is H, halo, optionally substituted C₁-C₆ alkyl, optionally substituted C₁-C₆ alkylsulfonyl, optionally substituted carboxamide, or optionally substituted sulfamoyl. In other cases of formula (IIA) or (IIB), R⁴ is H.

In yet other cases of formula (IIA) or (IIB), R⁵ is H, optionally substituted amino, halo, optionally substituted amido, optionally substituted carboxamide, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted C₁-C₆ alkylsulfonyl, optionally substituted C₆-C₁₀ arylsulfonyl, optionally substituted C₁-C₉ heteroarylsulfonyl, optionally substituted C₁-C₉ heterocyclylsulfonyl, optionally substituted C₁-C₆ thioalkyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In still other cases of formula (IIA) or (IIB), R⁵ is H, optionally substituted amino, halo, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₁-C₆ heteroalkyl, optionally substituted C₆-C₁₀ aryl, optionally substituted C₁-C₉ heteroaryl, or optionally substituted C₁-C₉ heterocyclyl. In some cases of formula (IIA) or (IIB), R⁵ is H, optionally substituted amino, halo, optionally substituted C₁-C₆ alkyl, optionally substituted C₆-C₁₀ aryloxy, optionally substituted C₁-C₉ heteroaryloxy, or optionally substituted C₆-C₁₀ aryl. In particular cases of formula (IIA) or (IIB), R⁵ is H.

In certain cases of formula (IIA) or (IIB), R⁶ is H, optionally substituted amino, halo, optionally substituted amido, optionally substituted carboxamide, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted C₁-C₆ alkylsulfonyl, optionally substituted C₆-C₁₀ arylsulfonyl, optionally substituted C₁-C₉ heteroarylsulfonyl, optionally substituted C₁-C₉ heterocyclylsulfonyl, optionally substituted C₁-C₆ thioalkyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (IIA) or (IIB), R⁶ is H, optionally substituted amino, halo, optionally substituted amido, optionally substituted carboxamide, optionally substituted C₁-C₆ alkyl, optionally substituted C₂-C₇ alkoxycarbonyl, optionally substituted C₁-C₆ alkoxy, optionally substituted C₆-C₁₀ aryloxy, optionally substituted C₁-C₉ heteroaryloxy, optionally substituted C₁-C₆ heteroalkyl, optionally substituted C₆-C₁₀ aryl, optionally substituted C₁-C₉ heteroaryl, or optionally substituted C₁-C₉ heterocyclyl. In yet other cases of formula (IIA) or (IIB), R⁶ is H, optionally substituted amino, optionally substituted amido, halo, or optionally substituted C₁-C₆ alkyl. In still other cases of formula (IIA) or (IIB), R⁶ is H.

In some cases of formula (IIA) or (IIB), R⁷ is H, optionally substituted amino, halo, optionally substituted amido, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfonyl, optionally substituted C₆-C₁₀ arylsulfonyl, optionally substituted C₁-C₉ heteroarylsulfonyl, optionally substituted C₁-C₉ heterocyclylsulfonyl, optionally substituted sulfamoyl, optionally substituted C₁-C₆ thioalkyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (IIA) or (IIB), R⁷ is H, optionally substituted amino, halo, optionally substituted amido, cyano, optionally substituted C₁-C₆ alkyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfonyl, optionally substituted C₆-C₁₀ arylsulfonyl, optionally substituted sulfamoyl, optionally substituted C₁-C₆ heteroalkyl, optionally substituted C₆-C₁₀ aryl, optionally substituted C₁-C₉ heteroaryl, or optionally substituted C₁-C₉ heterocyclyl. In particular cases of formula (IIA) or (IIB), R⁷ is H, halo, optionally substituted C₁-C₆ alkyl, optionally substituted C₁-C₆ alkylsulfonyl, optionally substituted carboxamide, or optionally substituted sulfamoyl. In other cases of formula (IIA) or (IIB), R⁷ is H.

In yet other cases of formula (II), the isolated compound has the structure according to formula (IIC):

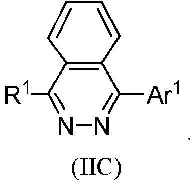

In some cases of formula (II), (IIA), (IIB), or (IIC), Ar¹ is optionally substituted C₆-C₁₀ aryl. In other cases of formula (II), (IIA), (IIB), or (IIC), Ar¹ is optionally substituted C₆ aryl.

In yet other cases of formula (II), the isolated compound has the structure according to formula (IID): 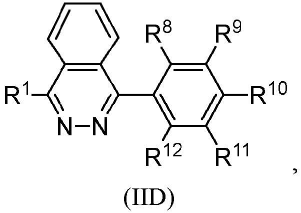 wherein
each of R⁸, R⁹, R¹⁰, R¹¹, and R¹² is, independently, H, halo, hydroxy, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted sulfamoyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
or any two of adjacent R⁸, R⁹, R¹⁰, R¹¹, and R¹², together with the two adjacent carbon atoms to which they are attached, form a 5, 6, or 7-memebered optionally substituted carbocyclic or heterocyclic ring.

In certain cases of formula (IID), R⁸ is H, halo, or optionally substituted C₁-C₆ alkyl. In other cases of formula (IID), R⁸ is H.

In particular cases of formula (IID), R¹¹ is H, halo, or optionally substituted C₁-C₆ alkyl. In other cases of formula (IID), R¹¹ is H.

In some cases of formula (IID), R¹² is H, halo, or optionally substituted C₁-C₆ alkyl. In other cases of formula (IID), R¹² is H.

In certain cases of formula (IID), R⁹ is H, optionally substituted amino, halo, optionally substituted amido, optionally substituted carboxamide, cyano, nitro, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₉ heterocyclylsulfonyl, optionally substituted sulfamoyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (IID), R⁹ is H, optionally substituted amido, halo, cyano, optionally substituted C₁-C₆ alkyl, optionally substituted C₂-C₇ alkoxycarbonyl, optionally substituted C₁-C₆ alkylsulfonyl, optionally substituted C₆-C₁₀ arylsulfonyl, optionally substituted C₁-C₉ heteroarylsulfonyl, optionally substituted C₁-C₉ heterocyclylsulfonyl, optionally substituted sulfamoyl, optionally substituted C₁-C₆ heteroalkyl, optionally substituted C₆-C₁₀ aryl, optionally substituted C₁-C₉ heteroaryl, or optionally substituted C₁-C₉ heterocyclyl. In yet other cases of formula (IID), R⁹ is H, optionally substituted carboxamide, halo, optionally substituted C₁-C₆ alkylsulfonyl, optionally substituted C₁-C₉ heterocyclylsulfonyl, or optionally substituted sulfamoyl. In still other cases of formula of formula (IID), R⁹ is optionally substituted sulfamoyl (e.g., unsubstituted sulfamoyl).

In certain cases of formula (IID), R¹⁰ is H, halo, cyano, optionally substituted C₁-C₆ alkyl, optionally substituted C₂-C₆ alkenyl, optionally substituted C₂-C₆ alkynyl, optionally substituted C₂-C₆ alkanoyl, hydroxycarbonyl, optionally substituted C₂-C₇ alkoxycarbonyl, optionally substituted carboxamide, optionally substituted C₁-C₆ thioalkyl, optionally substituted C₁-C₆ heteroalkyl, optionally substituted C₃-C₁₀ cycloalkyl, optionally substituted C₆-C₁₀ aryl C₁-C₆ alkyl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In yet other cases of formula (IID), R¹⁰ is H, halo, optionally substituted C₁-C₆ alkyl, optionally substituted C₁-C₆ heteroalkyl, optionally substituted C₃-C₁₀ cycloalkyl, optionally substituted C₆-C₁₀ aryl C₁-C₆ alkyl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In still other cases of formula (IID), R¹⁰ is optionally substituted C₁-C₆ alkyl, optionally substituted C₁-C₆ heteroalkyl, or optionally substituted C₃-C₁₀ cycloalkyl. In some cases of formula of formula (IID), R¹⁰ is optionally substituted C₁-C₆ alkyl (e.g., methyl).

In particular cases of formula (II), (IIA), (IIB), (IIC), or (IID), R¹ is H, hydroxy, optionally substituted amino, halo, thiol, optionally substituted amido, optionally substituted carboxamide, cyano, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In certain cases of formula (IID), R¹⁰ is H, optionally substituted amino, optionally substituted amido, optionally substituted C₁-C₆ alkyl, optionally substituted C₁-C₆ alkoxy, optionally substituted C₆-C₁₀ aryloxy, optionally substituted C₁-C₉ heteroaryloxy, optionally substituted C₁-C₆ heteroalkyl, optionally substituted C₆-C₁₀ aryl C₁-C₆ alkyl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In some cases of formula (II), (IIA), (IIB), (IIC), or (IID), R¹ is H, optionally substituted amino, optionally substituted C₆-C₁₀ aryloxy, optionally substituted C₁-C₉ heteroaryloxy, optionally substituted C₆-C₁₀ aryl, optionally substituted C₁-C₉ heteroaryl, or optionally substituted C₁-C₉ heterocyclyl. In other cases of formula (II), (IIA), (IIB), (IIC), or (IID), R¹ is optionally substituted amino. In yet other cases of formula (IID), R¹⁰ is substituted amino, wherein at least one substituent is phenyl. In still other cases of formula (II), (IIA), (IIB), (IIC), or (IID), R¹ is substituted amino, wherein at least one substituent is *o*-tolyl.

In some cases of formula (II), the compound of formula (II) has a structure of formula (IIE): 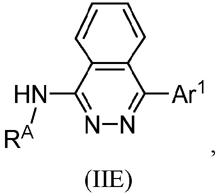 wherein
R^{A} is an optionally substituted phenyl, and
Ar¹ is an optionally substituted phenyl.

In some cases of formula (IIE), R^{A} is phenyl or 2-methylphenyl.

In some cases of formula (IIE), Ar¹ is 3-aminosulfonyl-4-methylphenyl.

In some cases of formula (II), (IIA), (IIB), or (IIC), the isolated compound is compound 19 or 20:

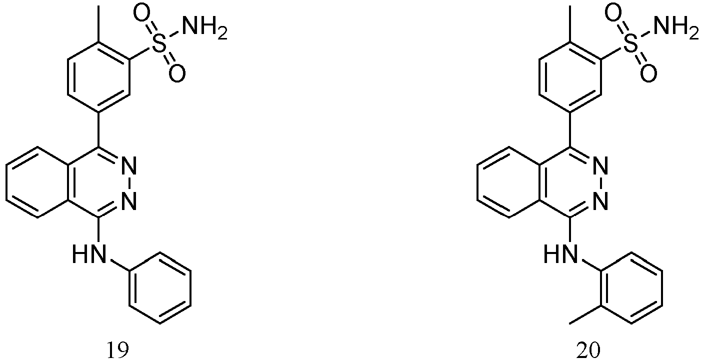

In some cases, the GPR174 inhibitory compound has a structure according to formula (III): 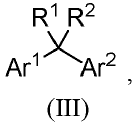
or a stereoisomer thereof, or a tautomer, or a pharmaceutically acceptable salt thereof, wherein
each of R¹ and R² is, independently, H, halo, cyano, optionally substituted C₁-C₆ alkyl, or optionally substituted C₁-C₆ heteroalkyl; and
each of Ar¹ and Ar² is, independently, optionally substituted C₆-C₁₀ aryl or optionally substituted C₁-C₉ heteroaryl.

In certain cases of formula (III), Ar¹ is optionally substituted C₆-C₁₀ aryl. In other cases of formula (III), Ar¹ is optionally substituted C₆ aryl, *e*.*g*., optionally substituted phenyl.

In certain cases of formula (III), Ar² is optionally substituted C₆-C₁₀ aryl. In other cases of formula (III), Ar² is optionally substituted C₆ aryl, *e.g*., optionally substituted phenyl.

In yet other cases of formula (III), the isolated compound has the structure according to formula (IIIA): 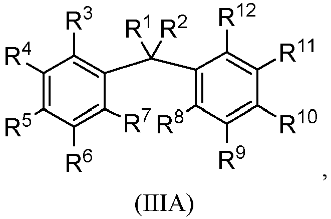 wherein
each of R³, R⁴, R⁵, R⁶, R⁷, R⁸, R⁹, R¹⁰, R¹¹, and R¹² is, independently, H, halo, hydroxy, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₂-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₂-C₆ alkanoylamino, optionally substituted C₇-C₁₁ aryloylamino, optionally substituted C₂-C₁₀ heteroaryloylamino, optionally substituted C₂-C₁₀ heterocyclyloylamino, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted sulfamoyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl.

In particular cases of formula (III) or (IIIA), R¹ is H, halo, or optionally substituted C₁-C₆ alkyl. In other cases of formula (III) or (IIIA), R¹ is H, halo, or methyl. In yet other cases of formula (III) or (IIIA), R¹ is H.

In certain cases of formula (III) or (IIIA), R² is H, halo, or optionally substituted C₁-C₆ alkyl. In other cases of formula (III) or (IIIA), R² is H, halo, or methyl. In yet other cases of formula (III) or (IIIA), R² is H.

In some cases of formula (III) or (IIIA), the isolated compound has the structure according to formula (IIIB):

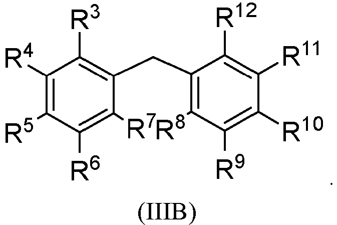

In particular cases of formula (III), (IIIA), or (IIIB), R³ is H, halo, or optionally substituted C₁-C₆ alkyl. In other cases of formula (III), (IIIA), or (IIIB), R³ is H.

In certain cases of formula (III), (IIIA), or (IIIB), R⁴ is H, halo, or optionally substituted C₁-C₆ alkyl. In other cases of formula (III), (IIIA), or (IIIB), R⁴ is H.

In some cases of formula (III), (IIIA), or (IIIB), R⁷ is H, halo, or optionally substituted C₁-C₆ alkyl. In other cases of formula (III), (IIIA), or (IIIB), R⁷ is H.

In other cases of formula (III), (IIIA), or (IIIB), the isolated compound has the structure according to formula (IIIC):

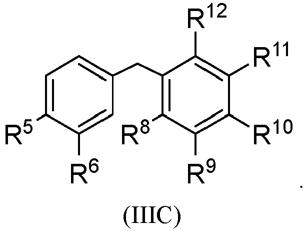

In particular cases of formula (III), (IIIA), (IIIB), or (IIIC), R¹¹ is H, halo, or optionally substituted C₁-C₆ alkyl. In other cases of formula (III), (IIIA), (IIIB), or (IIIC), R¹¹ is H.

In certain cases of formula (III), (IIIA), (IIIB), or (IIIC), R¹² is H, halo, or optionally substituted C₁-C₆ alkyl. In other cases of formula (III), (IIIA), (IIIB), or (IIIC), R¹² is H.

In some cases of formula (III), (IIIA), (IIIB), or (IIIC), R⁸ is H, halo, or optionally substituted C₁-C₆ alkyl. In other cases of formula (III), (IIIA), (IIIB), or (IIIC), R⁸ is H.

In other cases of formula (III), (IIIA), (IIIB), or (IIIC), the isolated compound has the structure according to formula (IIID):

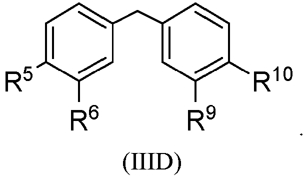

In some cases of formula (III), (IIIA), (IIIB), (IIIC), or (IIID), R⁵ is H, halo, cyano, optionally substituted amino, optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted C₂-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₂-C₆ alkanoylamino, optionally substituted C₇-C₁₁ aryloylamino, optionally substituted C₂-C₁₀ heteroaryloylamino, optionally substituted C₂-C₁₀ heterocyclyloylamino, hydroxycarbonyl, optionally substituted carboxamide, optionally substituted C₁-C₆ alkylsulfonyl, substituted C₆-C₁₀ arylsulfonyl, optionally substituted C₁-C₉ heteroarylsulfonyl, optionally substituted C₁-C₉ heterocyclylsulfonyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (III), (IIIA), (IIIB), (IIIC), or (IIID), R⁵ is optionally substituted amino, optionally substituted C₂-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₂-C₆ alkanoylamino, optionally substituted C₇-C₁₁ aryloylamino, optionally substituted C₂-C₁₀ heteroaryloylamino, optionally substituted C₂-C₁₀ heterocyclyloylamino, hydroxycarbonyl, or optionally substituted carboxamide. In yet other cases of formula (III), (IIIA), (IIIB), (IIIC), or (IIID), R⁵ is optionally substituted amino, optionally substituted C₂-C₆ alkanoylamino, optionally substituted C₇-C₁₁ aryloylamino, optionally substituted C₂-C₁₀ heteroaryloylamino, or optionally substituted C₂-C₁₀ heterocyclyloylamino.

In particular cases of formula (III), (IIIA), (IIIB), (IIIC), or (IIID), R¹⁰ is H, halo, cyano, optionally substituted amino, optionally substituted C₁-C₆ alkyl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted C₂-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₂-C₆ alkanoylamino, optionally substituted C₇-C₁₁ aryloylamino, optionally substituted C₂-C₁₀ heteroaryloylamino, optionally substituted C₂-C₁₀ heterocyclyloylamino, hydroxycarbonyl, optionally substituted carboxamide, optionally substituted C₁-C₆ alkylsulfonyl, substituted C₆-C₁₀ arylsulfonyl, optionally substituted C₁-C₉ heteroarylsulfonyl, optionally substituted C₁-C₉ heterocyclylsulfonyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (III), (IIIA), (IIIB), (IIIC), or (IIID), R¹⁰ is optionally substituted amino, optionally substituted C₂-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₂-C₆ alkanoylamino, optionally substituted C₇-C₁₁ aryloylamino, optionally substituted C₂-C₁₀ heteroaryloylamino, optionally substituted C₂-C₁₀ heterocyclyloylamino, hydroxycarbonyl, or optionally substituted carboxamide. In yet other cases of formula (III), (IIIA), (IIIB), (IIIC), or (IIID), R¹⁰ is optionally substituted amino, optionally substituted C₂-C₆ alkanoylamino, optionally substituted C₇-C₁₁ aryloylamino, optionally substituted C₂-C₁₀ heteroaryloylamino, or optionally substituted C₂-C₁₀ heterocyclyloylamino.

In certain cases of formula (III), (IIIA), (IIIB), (IIIC), or (IIID), the isolated compound has the structure according to formula (IIIE): 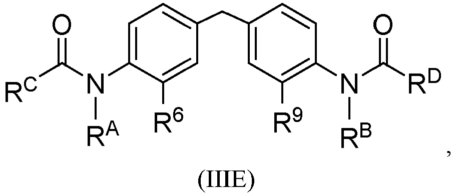 wherein
each of R^{A} and R^{B} is, independently, H or optionally substituted C₁-C₆ alkyl; and
each of R^{C} and R^{D} is, independently, H, optionally substituted C₁-C₆ alkyl, optionally substituted C₃-C₁₀ cycloalkyl, optionally substituted C₆-C₁₀ aryl, optionally substituted C₁-C₉ heteroaryl, or optionally substituted C₁-C₉ heterocyclyl.

In some cases of formula (III), (IIIA), (IIIB), (IIIC), (IIID), or (IIIE), R^{A} is H. In other cases of formula (III), (IIIA), (IIIB), (IIIC), (IIID), or (IIIE), R^{B} is H.

In certain cases of formula (III), (IIIA), (IIIB), (IIIC), (IIID), or (IIIE), R^{C} is optionally substituted C₆-C₁₀ aryl, optionally substituted C₁-C₉ heteroaryl. In other cases of formula (III), (IIIA), (IIIB), (IIIC), (IIID), or (IIIE), R^{C} is optionally substituted C₄ heteroaryl, e.g., thiophen-2-yl.

In other cases of formula (III), (IIIA), (IIIB), (IIIC), (IIID), or (IIIE), R^{D} is optionally substituted C₆-C₁₀ aryl, optionally substituted C₁-C₉ heteroaryl. In still other cases of formula (III), (IIIA), (IIIB), (IIIC), (IIID), or (IIIE), R^{D} is optionally substituted C₄ heteroaryl, e.g., thiophen-2-yl.

In particular cases of formula (III), (IIIA), (IIIB), (IIIC), (IIID), or (IIIE), R⁶ is H, halo, optionally substituted C₁-C₆ alkyl, optionally substituted C₂-C₆ alkenyl, optionally substituted C₂-C₆ alkynyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (III), (IIIA), (IIIB), (IIIC), (IIID), or (IIIE), R⁶ is H, halo, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₁₀ cycloalkyl. In other cases of formula (III), (IIIA), (IIIB), (IIIC), (IIID), or (IIIE), R⁶ is H, halo, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₁₀ cycloalkyl. In still other cases of formula (III), (IIIA), (IIIB), (IIIC), (IIID), or (IIIE), R⁶ is H or optionally substituted C₁-C₆ alkyl. In some cases of formula (III), (IIIA), (IIIB), (IIIC), or (IIID), R⁶ is H. In other cases of formula (III), (IIIA), (IIIB), (IIIC), (IIID), or (IIIE), R⁶ is C₁-C₆ alkyl, e.g., methyl.

In certain cases of formula (III), (IIIA), (IIIB), (IIIC), (IIID), or (IIIE), R⁹ is H, halo, optionally substituted C₁-C₆ alkyl, optionally substituted C₂-C₆ alkenyl, optionally substituted C₂-C₆ alkynyl, optionally substituted C₁-C₆ heteroalkyl, optionally substituted C₃-C₁₀ cycloalkyl, optionally substituted C₆-C₁₀ aryl, optionally substituted C₆-C₁₀ aryl C₁-C₆ alkyl, optionally substituted C₁-C₉ heteroaryl, optionally substituted C₁-C₉ heteroaryl C₁-C₆ alkyl, optionally substituted C₁-C₉ heterocyclyl, or optionally substituted C₁-C₉ heterocyclyl C₁-C₆ alkyl. In other cases of formula (III), (IIIA), (IIIB), (IIIC), (IIID), or (IIIE), R⁹ is H, halo, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₁₀ cycloalkyl. In other cases of formula (III), (IIIA), (IIIB), (IIIC), (IIID), or (IIIE), R⁹ is H, halo, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₁₀ cycloalkyl. In still other cases of formula (III), (IIIA), (IIIB), (IIIC), (IIID), or (IIIE), R⁹ is H or optionally substituted C₁-C₆ alkyl. In some cases of formula (III), (IIIA), (IIIB), (IIIC), (IIID), or (IIIE), R⁹ is H. In other cases of formula (III), (IIIA), (IIIB), (IIIC), (IIID), or (IIIE), R⁹ is C₁-C₆ alkyl, e.g., methyl.

In some cases, the compound of formula (III) has a structure of formula (IIIF): 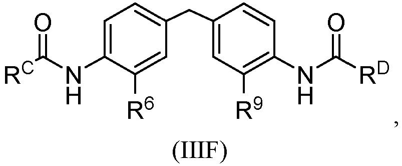 wherein
each of R^{C} and R^{D} is independently optionally substituted C₁-C₉ heteroaryl; and
each of R⁶ and R⁹ is independently optionally substituted C₁-C₆ alkyl.

In some cases of formula (IIIF), each of R^{C} and R^{D} is independently unsubstituted C₁-C₉ heteroaryl; and each of R⁶ and R⁹ is independently unsubstituted C₁-C₆ alkyl.

In some cases of formula (IIIF), each of R^{C} and R^{D} is thien-2-yl.

In some cases of formula (IIIF), each of R⁶ and R⁹ is methyl.

In some cases of formula (III), (IIIA), (IIIB), (IIIC), (IIID), or (IIIE), the isolated compound is compound 21:

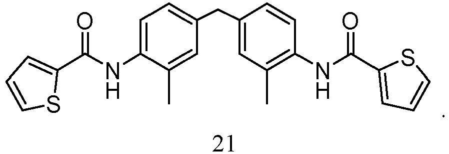

In some cases, the GPR174 inhibitory compound has a structure according to formula (IV): 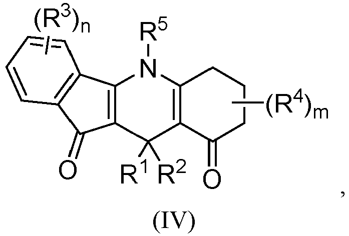
or a stereoisomer thereof, or a tautomer thereof, or a pharmaceutically acceptable salt thereof, wherein
each of R¹ and R² is, independently, H, hydroxy, halo, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
each of R³ and R⁴ is, independently, H, hydroxy, halo, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfonyl, optionally substituted C₆-C₁₀ arylthio, optionally substituted C₆-C₁₀ arylsulfonyl, optionally substituted C₁-C₉ heteroarylthio, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
R⁵ is H, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted C₁-C₆ alkyloxycarbonyl, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl.
n is 0, 1, 2, 3, or 4; and
m is 0, 1, 2, 3, 4, 5, or 6.

In some cases of formula (IV), m is 0.

In other cases of formula (IV), the isolated compound has a structure according to formula (IVA):

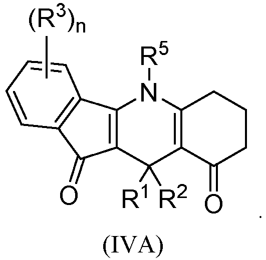

In particular cases of formula (IV) or (IVA), R¹ is H, halo, optionally substituted amino, optionally substituted amido, thiol, cyano, or optionally substituted C₁-C₆ alkyl. In other cases of formula (IV) or (IVA), R¹ is H, halo, or optionally substituted C₁-C₆ alkyl. In yet other cases of formula (IV) or (IVA), R¹ is H.

In certain cases of formula (IV) or (IVA), the isolated compound has a structure according to formula (IVB):

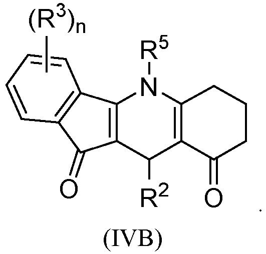

In some cases of formula (IV), (IVA), or (IVB), R⁵ is H, optionally substituted C₁-C₆ alkyl, optionally substituted C₂-C₆ alkenyl, optionally substituted C₂-C₆ alkynyl, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted C₁-C₆ alkyloxycarbonyl, optionally substituted C₁-C₆ alkylsulfonyl, or optionally substituted C₆-C₁₀ arylsulfonyl. In other cases of formula (IV), (IVA), or (IVB), R⁵ is H, optionally substituted C₁-C₆ alkyl, optionally substituted C₂-C₆ alkanoyl, optionally substituted C₇-C₁₁ aryloyl, or optionally substituted C₁-C₆ alkyloxycarbonyl. In yet other cases of formula (IV), (IVA), or (IVB), R⁵ is H, optionally substituted C₁-C₆ alkyl, or optionally substituted C₂-C₆ alkanoyl. In still other cases of formula (IV), (IVA), or (IVB), R⁵ is H.

In certain cases of formula (IV), (IVA), or (IVB), the isolated compound has a structure according to formula (IVC):

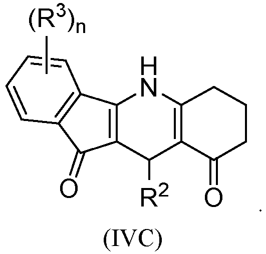

In particular cases of formula (IV), (IVA), (IVB), or (IVC), R² is H, optionally substituted C₁-C₆ alkyl, optionally substituted C₂-C₆ alkenyl, optionally substituted C₂-C₆ alkynyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl. In other cases of formula (IV), (IVA), (IVB), or (IVC), R² is optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₁-C₉ heteroaryl, or optionally substituted C₁-C₉ heterocyclyl. In yet other cases of formula (IV), (IVA), (IVB), or (IVC), R² is optionally substituted C₆-C₁₀ aryl, optionally substituted C₁-C₉ heteroaryl, or optionally substituted C₁-C₉ heterocyclyl. In still other cases of formula (IV), (IVA), (IVB), or (IVC), R² is optionally substituted C₆-C₁₀ aryl or optionally substituted C₁-C₉ heteroaryl. In other cases of formula (IV), (IVA), (IVB), or (IVC), R² is optionally substituted pyridyl (e.g., 2-pyridyl, 3-pyridyl, or 4-pyridyl). In certain cases of formula (IV), (IVA), (IVB), or (IVC), R² is optionally substituted phenyl.

In some cases of formula (IV), (IVA), (IVB), or (IVC), the isolated compound has a structure according to formula (IVD): 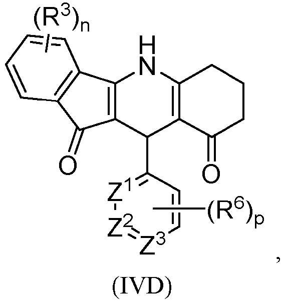
wherein R⁶ at each occurrence is independently, halo, optionally substituted C₁-C₆ alkyl, optionally substituted C₂-C₆ alkenyl, optionally substituted C₆-C₁₀ aryl, optionally substituted C₂-C₆ heteroaryl, optionally substituted C₂-C₆ heterocyclyl, optionally substituted C₂-C₆ alkynyl, optionally substituted amino, optionally substituted amido, thiol, cyano, nitro, C₁-C₆ alkylsulfonyl, hydroxycarbonyl, optionally substituted C₂-C₇ alkoxycarbonyl, optionally substituted C₆-C₁₀ aryloxy, or optionally substituted C₂-C₆ heteroaryloxy;
Z¹ is C or N;
Z² is C or N;
Z³ is N or C; and
p is 0, 1, 2, 3, 4, or 5.

In some cases of formula (IVD), Z¹ is C, Z² is C, and Z³ is N. In other cases of formula (IVD), Z¹ is C, Z² is N, and Z³ is C. In certain cases of formula (IVD), Z¹ is N, Z² is C, and Z³ is C. In certain other cases of formula (IVD), Z¹ is C, Z² is C, and Z³ is C.

In some cases of formula (IV), (IVA), (IVB), or (IVC), the isolated compound has a structure according to formula (IVD): 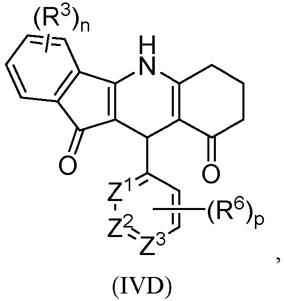
wherein R⁶ at each occurrence is independently, halo, optionally substituted C₁-C₆ alkyl, optionally substituted C₂-C₆ alkenyl, optionally substituted C₆-C₁₀ aryl, optionally substituted C₂-C₆ heteroaryl, optionally substituted C₂-C₆ heterocyclyl, optionally substituted C₂-C₆ alkynyl,optionally substituted amino, optionally substituted amido, thiol, cyano, nitro, C₁-C₆ alkylsulfonyl, hydroxycarbonyl, optionally substituted C₂-C₇ alkoxycarbonyl, optionally substituted C₂-C₆ alkoxy, optionally substituted C₂-C₆ alkenoxy, optionally substituted C₆-C₁₀ aryloxy, or optionally substituted C₂-C₆ heteroaryloxy;
Z¹ is CH or N;
Z² is CH or N;
Z³ is N or CH; and
p is 0, 1, 2, 3, 4, or 5.

In some cases of formula (IVD), Z¹ is C, Z² is C, and Z³ is N. In other cases of formula (IVD), Z¹ is C, Z² is N, and Z³ is CH. In certain cases of formula (IVD), Z¹ is N, Z² is CH, and Z³ is CH. In certain other cases of formula (IVD), Z¹ is CH, Z² is CH, and Z³ is CH.

In some cases of formula (IVD), p is 0. In other cases of formula (IVD), p is 1. In certain cases of formula (IVD), p is 2. In some cases of formula (IVD), p is 1, and R⁶ is in the p- or m-position.

In certain cases of formula (IVD), R⁶ is methoxy, methyl, hydroxyl, ethoxy, ethyl, optionally substituted phenoxy, optionally substituted cyclopentyloxy, t-butoxy, allyoxy, isopropyloxy, n-pentyloxy, trifluoromethyloxy, difluoromethyloxy, fluoro, chloro, nitro, 2-hydroxyethyloxy, optionally substituted 1,3,4-oxadiazolyl, or optionally substituted pyrrolidyl.

In particular cases of formula (IV), (IVA), (IVB), (IVC), or (IVD), R³ is H, halo, optionally substituted amino, optionally substituted amido, thiol, cyano, hydroxycarbonyl, optionally substituted C₂-C₇ alkoxycarbonyl, or optionally substituted C₁-C₆ alkyl.

In other cases of formula (IV), (IVA), (IVB), (IVC), or (IVD), n is 0.

In some cases of formula (IV), the compound has a structure of formula (IVE): 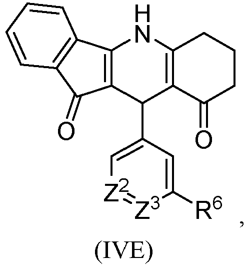 wherein
each of Z² and Z³ is independently CR⁶ or N; and
each of R⁶ is independently H, halogen, hydroxy, nitro, optionally substituted C₁-C₆ alkylsulfonyl, optionally substituted C₁-C₆ alkyl, optionally substituted C₁-C₆ alkoxy, optionally substituted C₄₋C₁₁ cycloalkoxy, optionally substituted C₁-C₆ haloalkoxy, optionally substituted C₂-C₆₋alkenoxy, optionally substituted C₆₋C₁₀ aryloxy, optionally substituted C₁-C₉ heterocyclyl, optionally substituted C₆₋C₁₀ aryl, or optionally substituted C₁-C₉ heteroaryl;
or two adjacent R⁶ groups, taken together with the carbon atoms to which they are attached, form a C₁-C₉ heterocyclyl.

In some cases of formula IV, (IVA), (IVB), or (IVC), the isolated compound is compound 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 54 or 55:

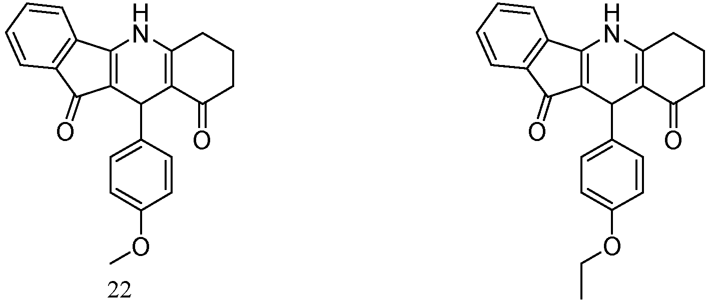

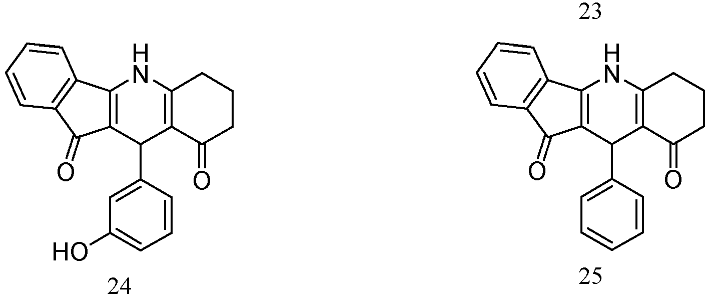

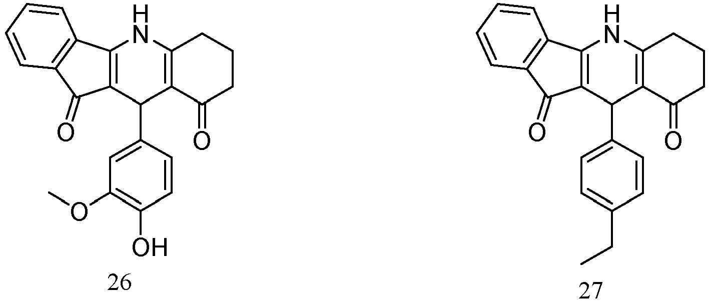

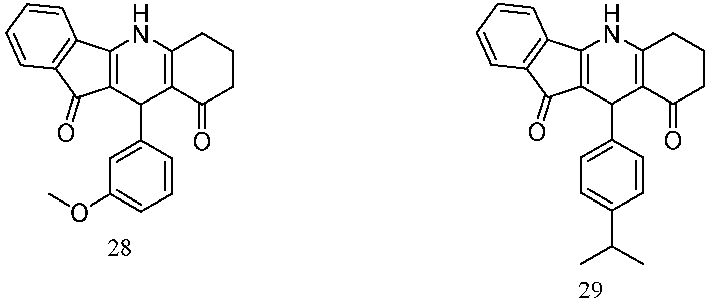

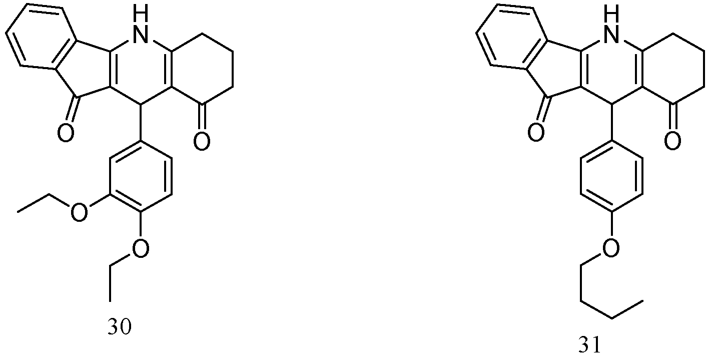

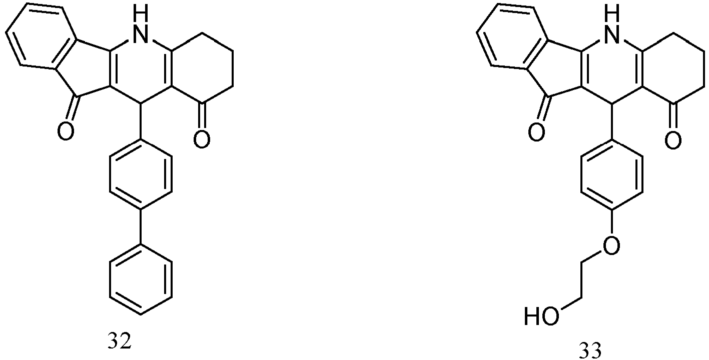

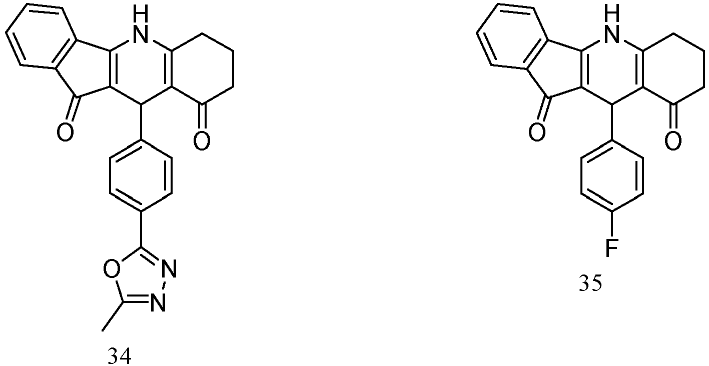

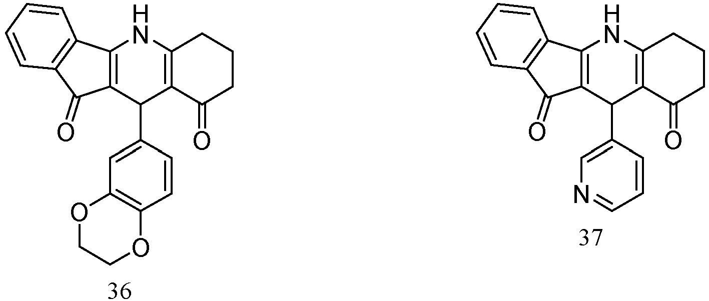

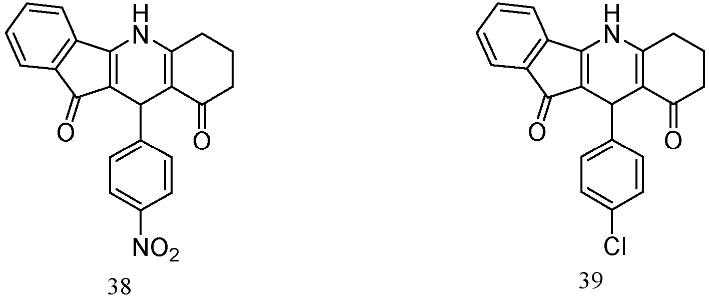

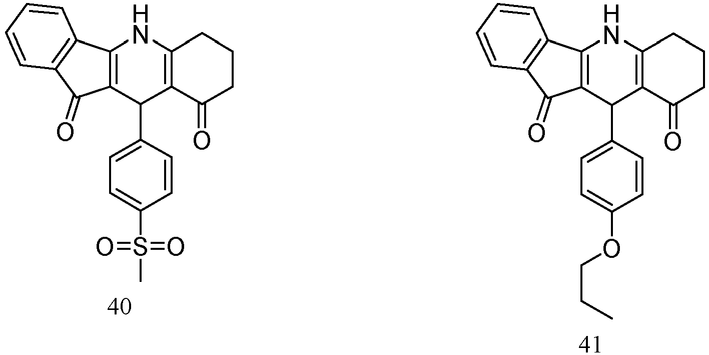

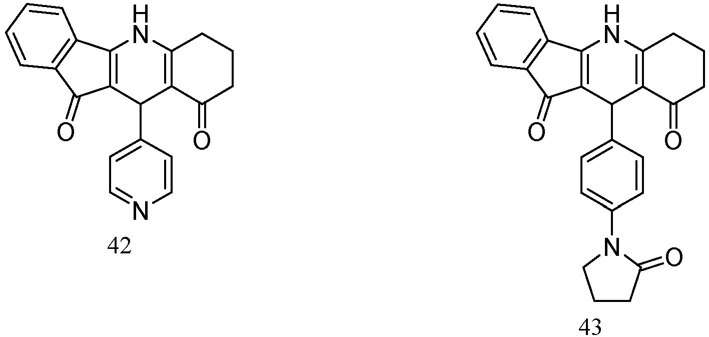

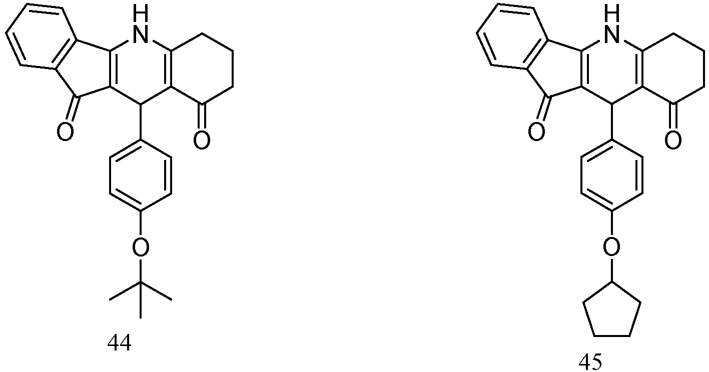

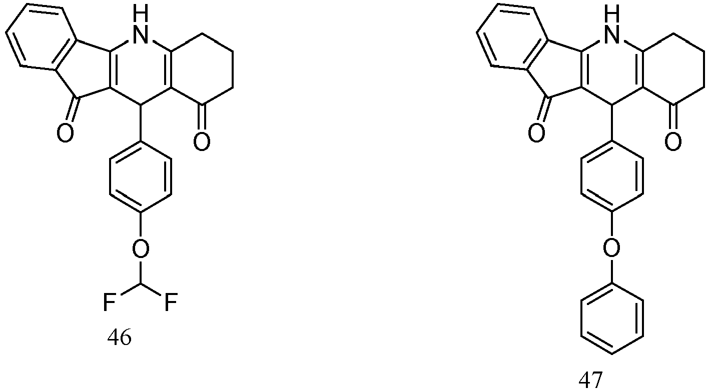

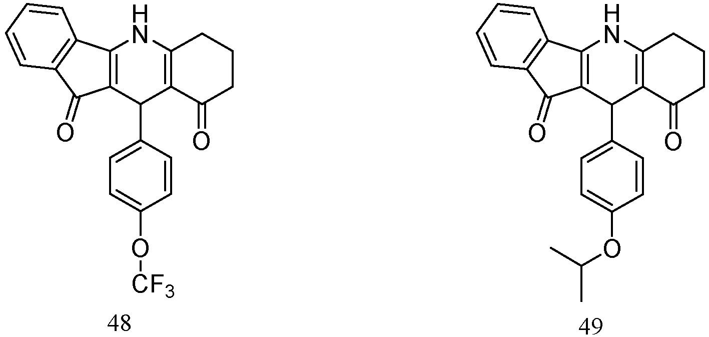

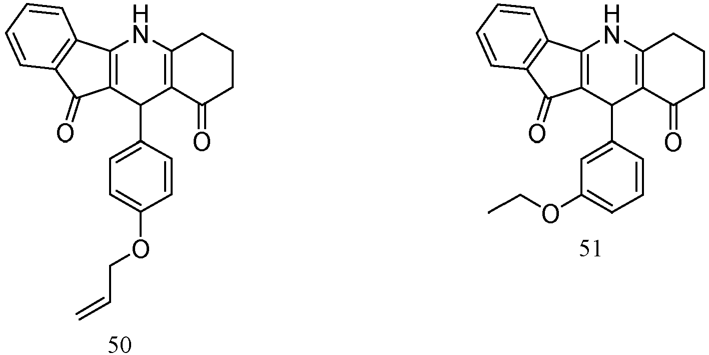

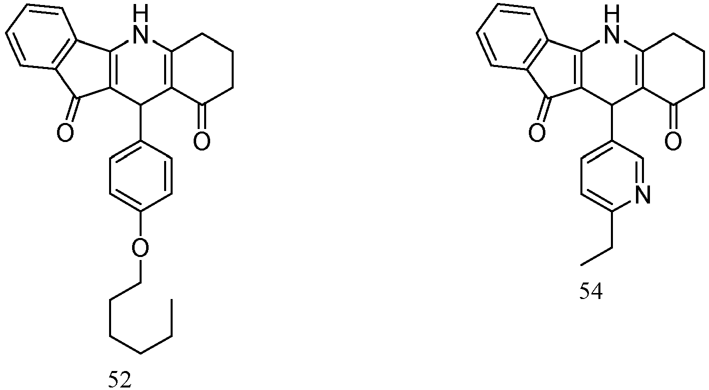

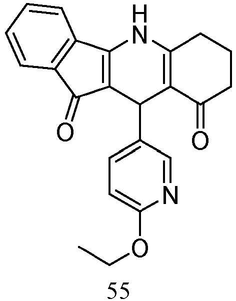

In some cases, the compound of formula (IVE) is compound 54 or 55.

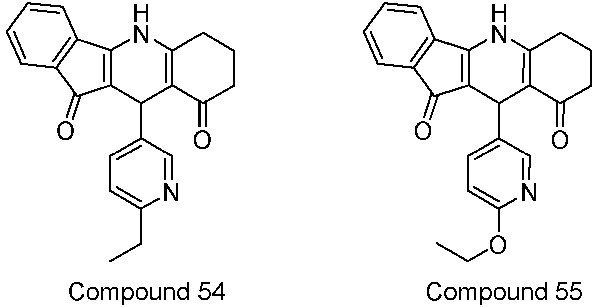

In some cases, the GPR174 inhibitory compound has a structure according to the following formula (V): 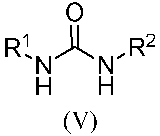
or a stereoisomer thereof, or a tautomer thereof, or a pharmaceutically acceptable salt thereof, wherein
R¹ is phenyl, and
R² is optionally substituted C₆-C₁₀ aryl or optionally substituted C₁-C₉ heteroaryl.

In some cases of formula (V), R² is optionally substituted phenyl.

In some cases of formula (V), R² is phenyl substituted with *para*-C₂-C₆ alkenoxy.

In some cases of formula (V), R² is phenyl substituted with *para*-(2-methylallyl)oxy.
In some cases of formula (V), the compound of formula (V) is compound 56:

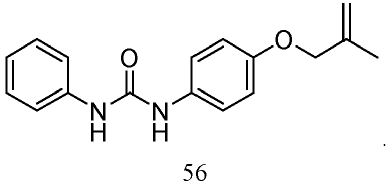

In some cases, the GPR174 inhibitory compound has a structure according to the following formula (Va): 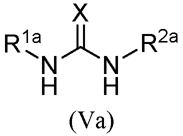
or a stereoisomer thereof, or a tautomer thereof, or a pharmaceutically acceptable salt thereof, wherein
X is O or S;
R^{1a} is an optionally substituted phenyl; and
R^{2a} is an optionally substituted C₆-C₁₀ aryl, an optionally substituted C₃-C₉ heteroaryl or an optionally substituted C₃-C₁₀ heteroarylalkyl.

In some cases, R^{1a} is a substituted phenyl. In some more specific cases, R^{1a} is optionally substituted with halo (*e.g.,* F, Br, Cl, or I). In some cases, R^{1a} has the following structure:

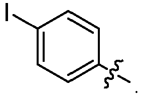

In some cases, R^{2a} is an optionally substituted C₃-C₁₀ heteroarylalkyl. In some cases, R^{2a} is unsubstituted. In some more specific cases, R^{2a} has the following structure:

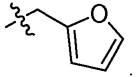

In some cases, X is O. In certain cases, X is S.

In some cases, provided herein is a GPR174 inhibitory compound according to formula (VI): 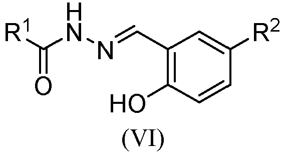
or a stereoisomer thereof, or a tautomer thereof, or a pharmaceutically acceptable salt thereof, wherein
R¹ is an optionally substituted C₁-C₉ heteroaryl, and
R² is halogen.

In some cases of formula (VI), the N=C bond has the (E) configuration.

In some cases of formula (VI), the N=C bond has the (Z) configuration.

In some cases of formula (VI), R¹ is an optionally substituted pyridinyl or an optionally substituted furanyl.

In some cases of formula (VI), R¹ is pyridin-4-yl.

In some cases of formula (VI), R¹ is 2,5-dimethyl-fur-3-yl.

In some cases of formula (VI), R² is halo.

In some cases of formula (VI), R² is chloro or bromo.

In some cases of formula (VI), the compound of formula (VI) is compound 57 or 58: 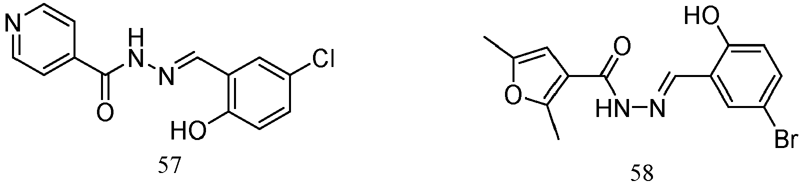 or a stereoisomer thereof, or a tautomer thereof, or a pharmaceutically acceptable salt thereof.

In any of the cases described herein, the compound may be a compound described in Table 1 (*e.g.,* any of compounds 1-59).

In another aspect, the disclosure features a method of identifying a modulatory agent capable of modulating a GPR174-mediated Gs signaling pathway comprising:
(a) contacting a cell with a candidate modulatory agent, wherein said cell expresses GPR174 and includes the GPR174-mediated signaling pathway, and
(b) determining whether the candidate modulatory agent modulates a GPR174-mediated Gs signaling pathway in comparison to a cell contacted with a control or in comparison to a reference standard. In some cases, said candidate modulatory agent is a chemical compound comprising a structure according to any of Formulas I-VI disclosed herein. In certain cases, step (b) further involves performing an assay to detect at least one of the following: transcriptional reporter gene expression, GTPase activity, cAMP level, intracellular messenger level, calcium level, downstream kinase activity, transcriptional activation of downstream genes, change in cell morphology, change in cell growth rate, arachidonic acid release, or extracellular acidification rate.

In some cases, the candidate modulatory agent is selected from the group consisting of a compound, a nucleic acid, a natural extract, and a gas. In certain cases, the compound is a small molecule compound. In particular cases, the chemical compound includes a structure according to any of Formulas I-VI disclosed herein.

In another aspect, the disclosure features a method of identifying a modulatory agent capable of modulating GPR174-mediated Gs signaling pathway activity, comprising:
(a) contacting a cell expressing GPR174 with:
   (i) at least one candidate modulatory agent; and
   (ii) a reference chemical compound known to modulate GPR174-mediated Gs signaling pathway activity;
(b) determining the GPR174-mediated signaling pathway activity level in the cell contacted in accordance with step (a); and
(c) comparing the GPR174-mediated signaling pathway activity level in a cell contacted with the reference chemical compound only with the GPR174-mediated signaling pathway activity determined in step (b); wherein a difference in GPR174-mediated signaling activity between the cell containing the candidate modulatory agent in the presence of the reference compound and the cell contacted with only the reference compound indicates that the candidate modulatory agent is capable of modulating GPR174 activity.

In some cases, said reference chemical compound known to modulate GPR174-mediated Gs signaling pathway activity comprises a structure according to any of Formulas I-VI disclosed herein. In certain cases, step (b) further involves performing an assay to detect at least one of the following: transcriptional reporter gene expression, GTPase activity, cAMP level, intracellular messenger level, calcium level, downstream kinase activity, transcriptional activation of downstream genes, change in cell morphology, change in cell growth rate, arachidonic acid release, or extracellular acidification rate. In some cases, the candidate modulatory agent is selected from the group consisting of a compound, a nucleic acid, a natural extract, and a gas. In certain cases, the compound is a chemical compound. In particular cases, the small molecule compound includes a structure according to any of Formulas I-VI.

In another aspect, the disclosure features a method of identifying a modulatory agent capable of modulating GPR174-mediated signaling pathway activity. The method involves: (a) contacting a cell expressing GPR174 with (i) at least one candidate modulatory agent and (ii) a reference chemical compound known to modulate GPR174-mediated signaling pathway activity; (b) determining the GPR174-mediated signaling pathway activity level in the cell contacted in accordance with step (a); and (c) comparing the GPR174-mediated signaling pathway activity level in a cell contacted with the reference chemical compound only with the GPR174-mediated signaling pathway activity determined in step (b); in which a difference in GPR174-mediated signaling activity between the cell containing the candidate modulatory agent in the presence of the reference compound and the cell contacted with only the reference compound indicates that the candidate modulatory agent is capable of modulating GPR174 activity. In some cases, the reference chemical compound includes a structure according to any one of Formulas I-VI (*e.g.,* any of compounds 1-59).

In certain cases, step (b) further involves performing an assay to detect at least one of the following: transcriptional reporter gene expression, GTPase activity, cAMP level, intracellular messenger level, calcium level, downstream kinase activity, transcriptional activation of downstream genes, change in cell morphology, change in cell growth rate, arachidonic acid release, or extracellular acidification rate.

In some cases, the candidate modulatory agent is selected from the group consisting of a compound, a nucleic acid, a natural extract, and a gas. In certain cases, the compound is a small molecule chemical compound.

In another aspect, the disclosure features a method of using a small molecule chemical compound for inhibiting a GPR174-mediated signaling pathway in a cell. The method involves the following steps:
(a) providing a small molecule chemical compound that functionally interacts with GPR174 and inhibits a GPR174-mediated Gs signaling pathway in cells expressing GPR174, wherein the compound is characterized by at least one of the following criteria:
   (i) the compound has a structure selected from the group consisting of Formula I, II, III, IV, V and VI (or (I), (II), (III), (IV), (V), (Va), or (VI)); or
   (ii) the compound changes the binding affinity of any one of reference compounds 1-59 (as set forth in Table 1) to GPR174; or
   (iii) the compound causes a difference in the inhibitory activity of any one of reference compounds 1-59 (as set forth in Table 1) in a GPR174-mediated signaling pathway assay when tested in the presence of the reference compound as compared to the inhibitory activity of the reference compound alone; and
(b) contacting a cell expressing GPR174 that includes a GPR174-mediated signaling pathway with the compound according to step (a), thereby inhibiting a GPR174-mediated signaling pathway in the cell.

In another aspect, the disclosure features a method of using a small molecule chemical compound for inhibiting a GPR174-mediated signaling pathway in a cell. The method involves the following steps:
(a) providing a small molecule chemical compound that functionally interacts with GPR174 and inhibits a GPR174-mediated Gs signaling pathway in cells expressing GPR174, wherein the compound is characterized by at least one of the following criteria:
   (i) the compound has a structure selected from the group consisting of Formula I, II, III, IV, V and VI (or (I), (II), (III), (IV), (V), (Va), or (VI)); or
   (ii) the compound changes the apparent binding affinity of any one of reference compounds 1-59 (as set forth in Table 1) to GPR174; or
   (iii) said compound causes a difference in the inhibitory activity of any one of reference compounds 1-59 (as set forth in Table 1), in a GPR174-mediated signaling pathway assay when tested in the presence of said reference compound as compared to the inhibitory activity of the reference compound alone; and
(b) contacting a cell expressing GPR174 that includes a GPR174-mediated signaling pathway with the compound according to step (a), thereby inhibiting a GPR174-mediated signaling pathway in the cell.

In some cases, the compound changes the binding affinity of any one of reference compounds 1-59 (as set forth in Table 1) to GPR174. In certain cases, the compound decreases (*i.e.,* inhibits) the binding affinity of any one of reference compounds 1-59 to GPR174. In particular cases, the compound competitively decreases (*i.e.,* competitively inhibits) the binding affinity of any one of reference compounds 1-59 to GPR174. In other cases, the compound increases the binding affinity of any one of reference compounds 1-59 (as set forth in Table 1) to GPR174.

In some cases, the compound changes the apparent binding affinity of any one of reference compounds 1-59 (as set forth in Table 1) for GPR174. In certain cases, the compound decreases the apparent binding affinity of any one of reference compounds 1-59 for GPR174. In particular cases, the compound competitively binds to GPR174 and decreases the apparent binding affinity of any one of reference compounds 1-59 for GPR174. In alternate cases, the compound allosterically binds to GPR174 and decreases the apparent binding affinity of any one of reference compounds 1-59 for GPR174. In other cases, the compound increases the binding affinity of any one of reference compounds 1-59 (as set forth in Table 1) for GPR174.

In certain cases of any of the above aspects of the disclosure, the GPR174-mediated signaling pathway is a Gs pathway.

In some cases of any of the above aspects, the cell is in a mammalian subject in need of treatment for any condition, disease or disorder described herein.

Inhibition of ATP-Adenosine-A2aR- and/or A2bR-mediated signaling (such as an A2aR antagonist, and/or an A2bR antagonist and/or a CD73 inhibitor and/or a CD38 inhibitor and/or a CD39 inhibitor)

Adenosine signaling, via the A2A receptor and/or the A2B receptor pathway in particular, has been identified as a promising approach to cancer immunotherapy. Leone et al., "Targeting adenosine for cancer immunotherapy", J. ImmunoTher. Cancer., 2018, 6:57. Allard et al., " The ectonucleotidases CD39 and CD73: novel checkpoint inhibitor targets", Immunol. Rev., 2017, 276(1), 121-144 (see also Table 15 below). Adenosine is an immunosuppressive metabolite produced at high levels within the tumor microenvironment. Adenosine signaling has emerged as a key metabolic pathway that regulates tumor immunity. In particular, adenosine signaling through the A2A receptor expressed on immune cells potently dampens immune responses in inflamed tissues. Adenosine is produced under hypoxic conditions in inflamed and malignant tissues via conversion of ATP mediated by the enzymes CD73 (NT5E, 5'-nucleotidase (5'-NT) or ecto-5'-nucleotidase) and CD39 (ectonucleoside triphosphate diphosphohydrolase-1, NTPDase1). Adenosine may also be produced from NAD+ by an axis centered on the NAD+-metabolizing CD38 generating adenosine diphosphate ribose (ADPR). Adenosine generated through a CD38-mediated pathway has been shown to correlate with progression of human myeloma (Horenstein et al., Mol Med 22:694-704, 2016). Adenosine generation and signaling, particularly through the A2A receptor, plays a role in resolution of inflammation in response to tissue injury, dampening the immune response. This coupling of wound healing and immunosuppression, however, represents a mechanism of cancer immune evasion. Accordingly, inhibition of the hypoxia-CD38-CD39-CD73-A2AR pathway has been identified as a promising target for cancer immunotherapy.

### Inhibitors of the ATP-adenosine A2A and/or A2B receptor pathway

Inhibitors of the ATP-adenosine A2A receptor pathway and/or inhibitors of the ATP-adenosine A2B receptor pathway that can be used in combination with GPR174 inhibitors in the compositions, methods and uses described herein include adenosine A2A receptor antagonists, A2B receptor antagonists and inhibitors of enzymes that degrade ATP to adenosine such as inhibitors of CD73 and inhibitors of CD38 and inhibitors of inhibitors of CD39, and combinations of such agents.

### Adenosine A2A receptor antagonists

Adenosine A2A receptor antagonists include the compounds ATL-444, AZD4635, caffeine, ciforadenant (CPI-444; V81444), CPI-445, EOS100850, MK-3814, istradefylline (KW-6002), MSX-3, PBF-509 (NIR178), preladenant (SCH-420,814), SCH-58261, SCH-412,348, SCH-442,416, ST-1535, ST-4206, theophylline, tozadenant (SYN115), VER-6623, VER-6947, VER-7835, vipadenant (BIIB-014) and ZM-241,385, whose structures are given below.

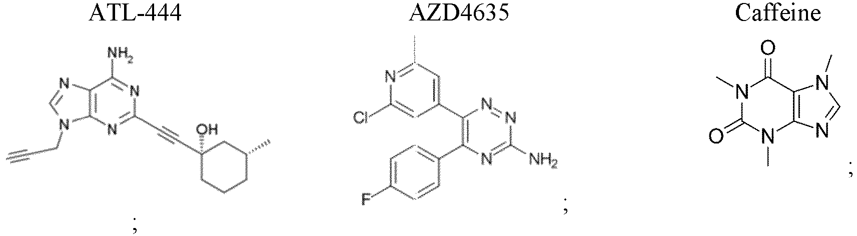

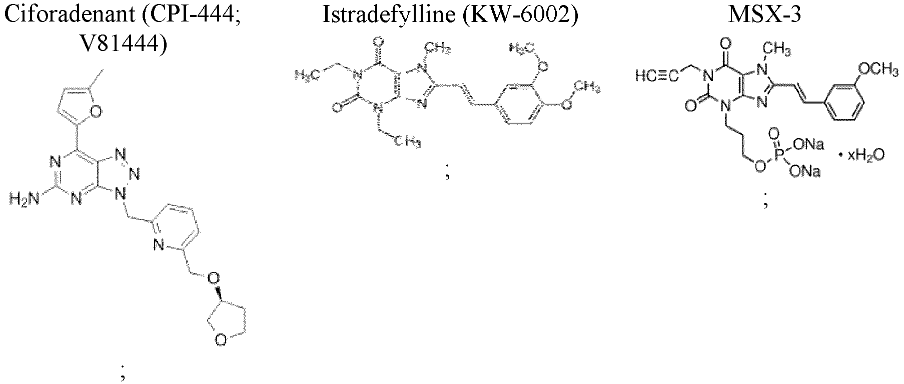

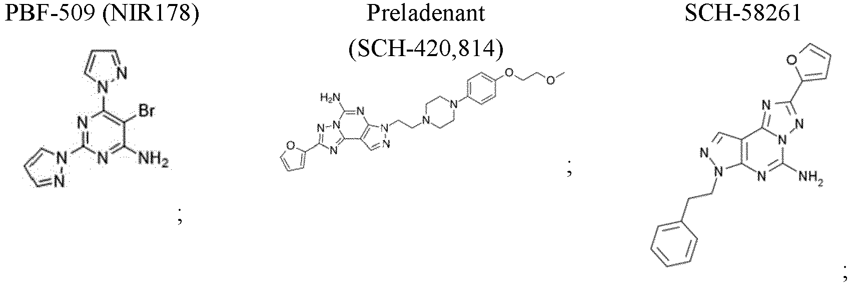

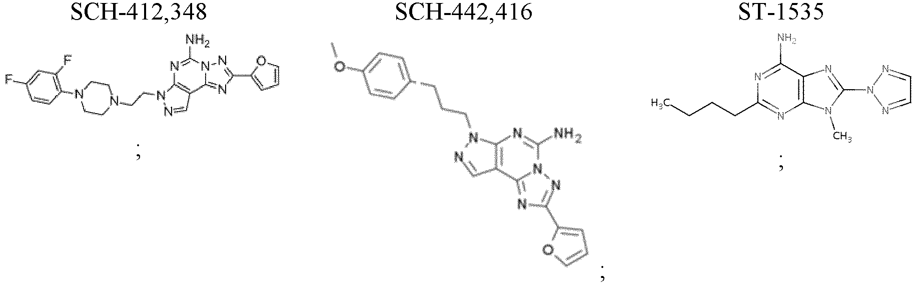

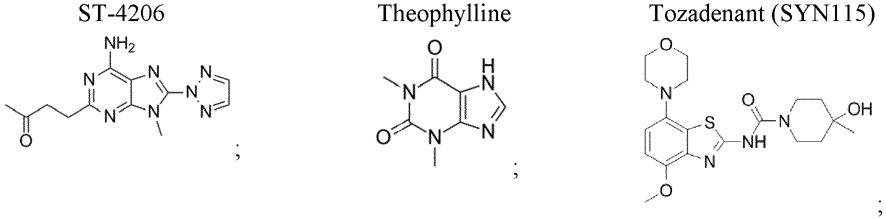

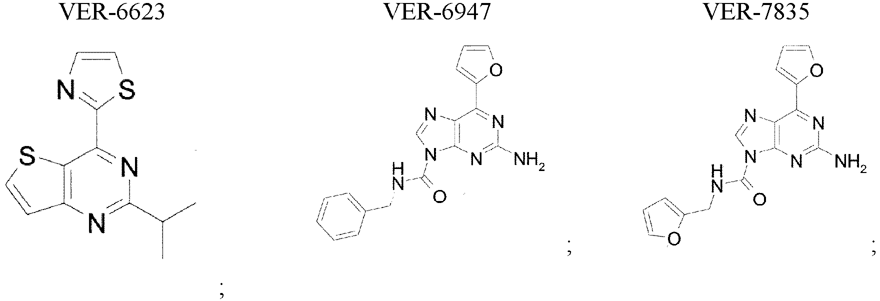

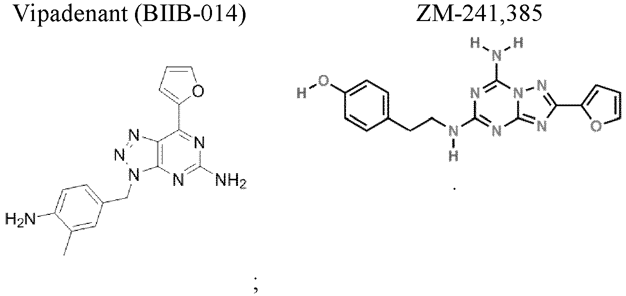

Further A2A receptor antagonists are described in Preti, et al., "History and Perspectives of A2A Adenosine Receptor Antagonists as Potential Therapeutic Agents", Med. Res. Rev., 2015, 35(4), 790-848, and Congreve et al., "Targeting adenosine A2A receptor antagonism for treatment of cancer", Exp. Opin. Drug Discov., 2018, 13(11), 997-1003.

Other A2A receptor antagonists are described in the following international patent applications: PCT pub. Nos. WO 2019/007140; WO 2018/166493; WO 2018/161910; WO 2018/130184; WO 2018/059531; WO 2017/136375; WO2 017/112917; WO 2017/008205; WO 2017/011214; WO 2016/200717; WO 2016/126570; WO 2016/087429; WO 2016/081290; WO 2015/020565; WO 2014/105664 ; WO 2014/105666; WO 2014/101120 ; WO 2014/101113; WO 2013/156614, WO 2013/058681; WO 2012/129381; WO 2012/112962; WO 2012/061787; WO 2012/060844; WO 2012/038980; WO 2011/061527; WO 2011/060207; WO 2011/053507; WO 2010/040003; WO 2010/037122; WO 2009/055308; WO 2009/050198; WO 2008/055711; WO 2007/047293; WO 2007/038212; WO 2006/137527; WO 2006/129626; WO 2006/124770; WO 2006/083949; WO 2012/03898; WO 2011/06152; WO 2011/06020; WO 2011/05350; WO 2010/04000; WO 2010/03712; WO 2009/055308; WO 2009/050198; WO 2008/055711; WO 2007/047293; WO 2007/038212; WO 2006/137527; WO 2006/129626; WO 2006/124770; and WO 2006/083949.

### Adenosine A2B receptor antagonists

Adenosine A2B receptor antagonists include the compounds MRS-1754, GS-6201, ISAM-140, PSB-0788, PSB-1115 and PSB-603 whose structures are given below:

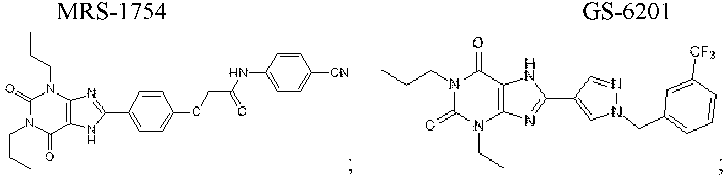

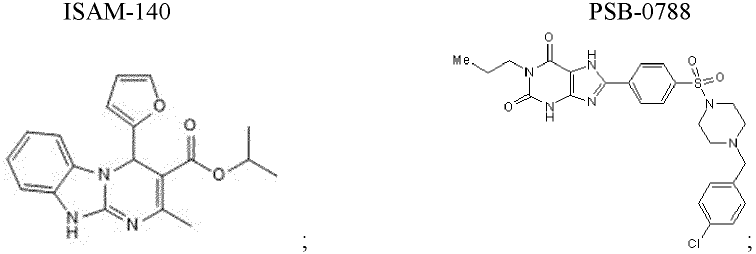

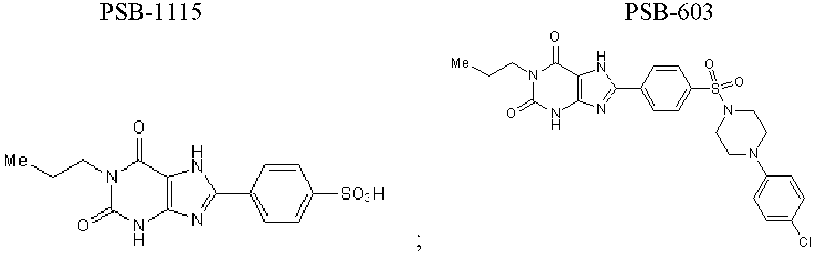

Further A2B receptor antagonists include ATL-801, CVT-6883, MRS-1706, OSIP-339,391, PSB-1901, PBF-1129 and additional A2B receptor antagonists as described in Vigano S. et al., Frontiers in Immunology vol 10:925, 2019; Volpini R. et al., Journal of Med Chem 45(15):3271-9, 2002; Volpini R. et al., Current Pharmaceutical Design 8(25):2285-98, 2002; Baraldi P.G. et al., Journal of Med Chem 47(6):1434-47, 2004; Cacciari B. et al., Mini Reviews in Med Chem 5(12):1053-60, 2005; Baraldi P.G. et al., Current Med Chem 13(28):3467-82, 2006; Beukers M.W. et al., Medicinal Research Reviews, 26(5):667-98, 2006; Elzein E. et al., Bioorganic & Medicinal Chemistry Letters 16(2):302-6, 2006; Carotti A. et al., Journal of Med Chem 49(1):282-99, 2006; Tabrizi M.A. et al., Bioorganic & Medicinal Chemistry 16(5):2419-30, 2008; Stefanachi A. et al., Bioorganic & Medicinal Chemistry 16(6):2852-69, 2008 and Jiang et al., Journal of Med Chem 62(8):4032-4055, 2019 as well as compound 38 described in Stefanachi A. et al., Bioorganic & Medicinal Chemistry 16(22):9780-9, 2008.

Other A2B receptor antagonists are described in the following international patent applications, PCT Pub. Nos WO 1995/011681; WO 1999/042093; WO 2000/049051; WO 2000/073307; WO 2009/157938; WO 2011/005871; WO 2012/112964; WO 2007/149277; WO 2019/123482; WO 2007/134958; WO 2005/051951; WO 2003/042214; WO 2007/039297; WO 2005/040155; WO 2005/042534; WO 2004/106337; WO 2001/016134; WO 2008/027585; WO 2003/053366; WO 2003/053361; WO 2005/070926; WO 2008/080461; WO 2016/150901; WO 2005/051951; WO 2003/042214; WO 2003/063800; and WO 2016/164838. CD73 (NT5E, 5'-nucleotidase (5'-NT) or ecto-5'-nucleotidase) Inhibitors

CD73 inhibitors can include CD73 antibodies, nucleotides (*e*.*g*., inhibitory RNA) that inhibit CD73 expression as well as chemical (*e*.*g*., small molecule) inhibitors.

CD73 antibodies including Oleclumab, are described in the following international patent applications: PCT Pub. Nos. WO 2018/237157; WO 2018/237173; WO 2018/215535; WO 2018/187484; WO 2018/137598; WO 2018/013611; WO 2017/152085; WO 2017/118613; WO 2017/100670; WO 2017/064043; WO 2016/131950; WO 2016/081748; WO 2016/075176; WO 2016/075099; and WO 2016/055609.

Nucleotides that inhibit CD73 expression are described in the following international patent applications: PCT Pub. No. WO 2018/065627.

CD73 inhibitors include N-benzyl-α,β-methyleneadenosine 5'-diphosphate sodium salt and α,β-methyleneadenosine 5'-diphosphate sodium salt (PSB 12379), BMS-986179, MEDI9447, CPI-006 and NZV930. CD73 inhibitors are also described in the following international patent applications: PCT Pub. Nos WO 2018/208727; WO 2018/208980; WO 2018/187512; WO 2018/183635; WO 2018/110555; WO 2018/094148; WO 2018/067424; WO 2017/153952; WO 2017/120508; WO 2017/098421; WO 2015/164573; WO 2015/049447; and WO 2007/135195.

It is noted that while CD73 inhibitors are useful in the compositions and methods described herein for the treatment of cancer, the use of a CD73 inhibitor is preferably avoided during early tumor formation and in the setting of metastasis due to the fact that CD73-derived adenosine could be helpful in preventing metastasis (may act as a barrier in the vascular endothelium), therefore it might be preferable to have CD73 remain active in such settings.

### CD38 (cyclic ADP ribose hydrolase) Inhibitors

CD38 is an ectoenzyme that catalyzes the synthesis and hydrolysis of cyclic ADP-ribose (cADPR) from NAD+ to ADP-ribose in addition to synthesis of NAADP from NADP+.^{[}

CD38 inhibitors can include CD38 antibodies, nucleotides (*e*.*g*., inhibitory RNA) that inhibit CD38 expression as well as chemical (*e*.*g*., small molecule) inhibitors.

CD38 antibodies, including Daratumumab, and other CD38 inhibitors are described in the following publications and international patent applications; Xia C, et al, Drugs of Today. 52 (10): 551-560, 2016; Escande C. et al., Diabetes 62(4):1084-1093, 2013; US Patent No. 9,840,496; US Pub. Nos 2018/85383; 2017/0260164; PCT Pub. Nos. WO 2019/140410; WO 20190/74973; WO 2019/0904931; WO 2019/087151; WO 2019/074973; WO 2019/034753; WO 2019/034752; WO 2019/020643; WO 2018/224685; WO 2018/224683; WO 2018/224682; WO 2016/087975; and WO 2013/002879

### CD39 (Ectonucleoside triphosphate diphosphohydrolase-1, NTPDase1) Inhibitors

CD39 inhibitors can include CD39 antibodies, nucleotides (*e*.*g*., inhibitory RNA) that inhibit CD39 expression as well as chemical (*e.g*., small molecule) inhibitors.

CD39 antibodies, including TTX-030, are described in the following international patent applications: PCT Pub. Nos. WO 2018/167267; WO 2018/065552; WO 2017/191300; WO 2017/157948; WO 2017/089334; WO 2017/064043; WO 2016/073845; WO 2012/085132; and WO 2009/095478.

Nucleotides that inhibit CD39 expression are described in the following international patent applications: PCT Pub. No. WO 2018/065622.

CD39 inhibitors include sodium metatungstate (POM 1); 6-N,N-diethyl-D-β,γ-dibromomethylene ATP trisodium salt (ARL-67156); 1-amino-4-(1-naphthyl)aminoanthraquinone-2-sulfonic acid sodium salt (PSB 06126), 1-amino-4-(4-chlorophenyl)aminoanthraquinone-2-sulfonic acid sodium salt (PSB069) and IPH52. CD39 inhibitors are also described in the following international patent applications: PCT Pub. No. WO 2007/135195.

Other features and advantages of the disclosure will be apparent from the following Detailed Description, the drawings, and the claims.

### BRIEF DESCRIPTION OF THE DRAWINGS

FIGURE 1A depicts the amino acid sequence of human GPR174 (NP_115942.1), set forth as SEQ ID NO:1.
FIGURE 1B depicts the cDNA encoding human GPR174 (NM_032553.1), set forth as SEQ ID NO:2.
FIGURE 1C depicts the amino acid sequence of mouse GPR174 (NP_001028423.1), set forth as SEQ ID NO:3.
FIGURE 1D depicts the amino acid sequence of rat GPR174 (NP_001100408.1), set forth as SEQ ID NO:4.
FIGURE 2 is a graph showing that the rank-ordering of the reported potency of a variety of known agonists to the Muscarinic acetylcholine receptor M1 (CHRM1) receptor is preserved in the cellular redistribution assay (CRA).
FIGURE 3 is a schematic diagram showing the algorithm used to determine the G-protein signaling profile for an orphan GPCR.
FIGURE 4A is a graph showing the effect of various compounds on signaling of the beta-2 adrenergic receptor (ADBR2).
FIGURE 4B is a graph showing the effects of ADBR2 over-expression on the CRE-luc reporter, as determined by transient transfection of increasing amounts (ng) of DNA encoding ADBR2.
FIGURE 4C is a graph showing the effects of ADBR2 over-expression on the NFAT-luc reporter, as determined by transient transfection of increasing amounts (ng) of DNA encoding ADBR2.
FIGURE 4D is a graph demonstrating that the compound ICI 118551, an inverse agonist of ADBR2, specifically inhibits CRE-luc reporter activity in cells transiently transfected with ADBR2.
FIGURE 4E is a graph demonstrating that the compound ICI 118551 does not inhibit the NFAT-luc reporter activity in cells transiently transfected with ADBR2.
FIGURE 5A is a graph illustrating the dose-response curves of representative compounds, including compounds 1, 2, 3, 4, and 20 against GPR174 in the Cellular Redistribution Assay (CRA) assay.
FIGURE 5B is a graph illustrating the dose-response curve of representative compounds, including compound 1, 2, 3, 4, and 20 against CHRM1 in the CRA assay. Also shown is the dose-response curve of the CHRM1-interacting compound pirenzepine.
FIGURES 6A-6B are graphs showing that compound 1 (Group I) inhibited the Gs pathway in the presence of GPR174 (FIGURE 6A), but not in the presence of ADBR2 (FIGURE 6B).
FIGURES 7A-7B are graphs showing that compound 2 (Group I) inhibited the Gs pathway in the presence of GPR174 (FIGURE 7A), but not in the presence of ADBR2 (FIGURE 7B).
FIGURES 8A-8B are graphs showing that compound 4 (Group I) did not modulate the Gs pathway in the presence of GPR174 (FIGURE 8A) and did not modulate the Gs pathway in the presence of ADBR2 (FIGURE 8B).
FIGURES 9A-9B are graphs showing that compound 6 (Group I) inhibited the Gs pathway in the presence of GPR174 (FIGURE 9A), but not in the presence of ADBR2 (FIGURE 9B).
FIGURES 10A-10B are graphs showing that compound 7 (Group I) inhibited the Gs pathway in the presence of GPR174 (FIGURE 10A), but not in the presence of ADBR2 (FIGURE 10B).
FIGURES 11A-11B are graphs showing that compound 10 (Group I) inhibited the Gs pathway in the presence of GPR174 (FIGURE 11A), but not in the presence of ADBR2 (FIGURE 11B).
FIGURES 12A-12B are graphs showing that compound 11 (Group I) inhibited the Gs pathway in the presence of GPR174 (FIGURE 12A), but not in the presence of ADBR2 (FIGURE 12B).
FIGURES 13A-13B are graphs showing that compound 19 (Group II) did not modulate the Gs pathway in the presence of GPR174 (FIGURE 13A) and did not modulate the Gs pathway in the presence of ADBR2 (FIGURE 13B).
FIGURES 14A-14B are graphs showing that compound 21 (Group III) did not modulate the Gs pathway in the presence of GPR174 (FIGURE 14A) and did not modulate the Gs pathway in the presence of ADBR2 (FIGURE 14B).
FIGURES 15A-15B are graphs showing that compound 22 (Group IV) inhibited the Gs pathway in the presence of GPR174 (FIGURE 15A), but not in the presence of ADBR2 (FIGURE 15B).
FIGURE 16A-16B are graphs showing that compound 23 (Group IV) inhibited the Gs pathway in the presence of GPR174 (FIGURE 16A), but not in the presence of ADBR2 (FIGURE 16B).
FIGURE 17A-17B are graphs showing that compound 31 (Group IV) inhibited the Gs pathway in the presence of GPR174 (FIGURE 17A), but not in the presence of ADBR2 (FIGURE 17B).
FIGURES 18A-18B are graphs showing that compound 33 (Group IV) inhibited the Gs pathway in the presence of GPR174 (FIGURE 18A), but not in the presence of ADBR2 (FIGURE 18B).
FIGURES 19A-19B are graphs showing that compound 36 (Group IV) inhibited the Gs pathway in the presence of GPR174 (FIGURE 19A), but not in the presence of ADBR2 (FIGURE 19B).
FIGURES 20A-20B are graphs showing that compound 42 (Group IV) inhibited the Gs pathway in the presence of GPR174 (FIGURE 20A), but not in the presence of ADBR2 (FIGURE 20B).
FIGURES 21A-21B are graphs showing that compound 4 (Group I) does not modulate the Gs pathway in the presence of GPR174 but does compete with the GPR174 agonist LysoPS (FIGURE 21A), and neither compound 4 nor LysoPS modulate Gs signaling in the presence of ADBR (FIGURE 21B).
FIGURE 22 graphically illustrates the relative transcript abundance of GPR174 in human tissues, as measured by qPCR, as described in Example 5.
FIGURE 23 graphically illustrates the relative transcript abundance of GPR174 in human lymphoid cells, as measured by qPCR, as described in Example 5.
FIGURE 24A graphically illustrates the percentage of FoxP3⁺Helios⁻ cells in the CD4⁺ cell population at day 3 post-stimulation in human peripheral blood mononuclear cells (PBMC) cultures treated with vehicle, compound 10, or interleukin-2 (IL-2) (*p*=0.03 for compound 10 vs vehicle; *p*=0.003 for IL-2 vs vehicle), as described in Example 6.
FIGURE 24B graphically illustrates the percentage of FoxP3⁺Helios⁺ cells in the CD4⁺ cell population at day 7 post-stimulation in PBMC cultures treated with vehicle, compound 10, or IL-2 (*p*=0.01 for compound 10 vs. vehicle; *p*=0.01 for IL-2 vs vehicle), as described in Example 6.
FIGURE 25 graphically illustrates the amount of IL-2 in culture supernatants (fold over vehicle levels) on day 2 post-stimulation in PBMC cultures treated with vehicle or compound 10 (1 µM, 3 µM or 10 µM), as described in Example 6.
FIGURE 26 graphically illustrates the amount of IFN-γ in culture supernatants (fold over vehicle levels) on day 2 post-stimulation in PBMC cultures treated with vehicle or compound 10 (1 µM, 3 µM or 10 µM), as described in Example 6.
FIGURE 27 graphically illustrates the amount of IL-2 (pg/mL) in culture supernatants 48 hours after stimulation in splenocyte cultures from WT or GPR174 KO mice treated with vehicle or compound 10 (10 µM), as described in Example 7.
FIGURE 28 graphically illustrates the percentage of FoxP3⁺Helios⁺ cells in the CD4⁺ cell population at day 7 post-stimulation in human PBMC cultures from a single donor treated with vehicle or compound 10 (3 µM, 10 µM or 30 µM) (n=3, **p*<0.05; ***p*<0.01), as described in Example 8.
FIGURE 29 graphically illustrates the amount of IL-2 (pg/mL) in culture supernatants on day 3 post-stimulation in human PBMC cultures from a single donor treated with vehicle or GPR174 inhibitory compound 10 (3 µM, 10 µM, 30 µM, or 60 µM), as described in Example 8.
FIGURE 30 graphically illustrates the fraction of vehicle of FoxP3⁺Helios⁺ cells in the CD4⁺ cell population at day 7 post-stimulation in human PBMC cultures from eight different donors treated with vehicle or compound 10 (30 µM), as described in Example 9.
FIGURE 31 graphically illustrates the amount of IL-2 (fold over IL-2 produced by vehicle) in culture supernatants on day 2 post-stimulation in human PBMC cultures from eight different donors treated with vehicle or compound 10 (30 µM), as described in Example 9.

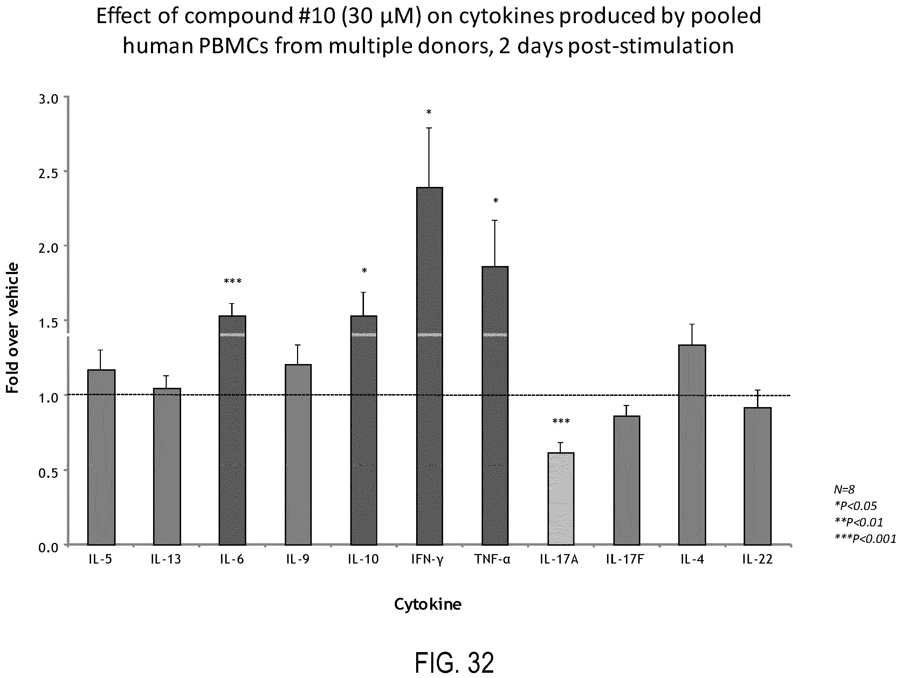

FIGURE 32 graphically illustrates the amount of various cytokines (fold over vehicle produced cytokine) in culture supernatants on day 2 post-stimulation from human PBMCs obtained from eight donors treated with vehicle or compound 10 (30 µM), wherein a statistically significant fold increase was observed for interleukin-6 (IL-6) (****p*<0.001), interleukin-10 (IL-10) (**p*<0.05), interferon gamma (IFN-γ) (**p*<0.05) and tumor necrosis factor (TNF-α)(**p*<0.05) as compared to vehicle control and wherein a statistically significant fold decrease was observed for interleukin 17 (IL-17A) (****p*<0.001) as compared to vehicle control, as described in Example 9.
FIGURE 33 graphically illustrates the fold induction by compound 10 (30 µM) on a panel of cytokines in co-cultured mouse C57BL/6+DBA1 splenocytes at day 6 after mixing, as compared to the cytokine levels measured in co-cultured C57BL/6+DBA1 splenocytes on day 6 in the absence of compound 10, as described in Example 10.
FIGURE 34A shows the CD4⁺ (46.2%), CD8⁺ (17.1%) and non-T cell (33.9%) populations present in a representative PBMC culture from a single donor, four hours after stimulation with anti-CD3 and anti-CD28 antibodies in the presence of compound 10 (10 µM), as described in Example 11.
FIGURE 34B graphically illustrates the fraction of cells with intracellular IL-2 in the CD4⁺ T cell population treated with compound 10 (10 µM) shown in FIGURE 34A, as compared to vehicle control, as described in Example 11.
FIGURE 34C graphically illustrates the fraction of cells with intracellular IL-2 in the CD8⁺ T cell population treated with compound 10 (10 µM) shown in FIGURE 34A, as compared to vehicle control, as described in Example 11.
FIGURE 35A shows the CD4⁺ (31.8%), CD8⁺ (27.5%) and non-T cell (33.1%) populations present in a representative PBMC culture from a single donor, two days after stimulation with anti-CD3 and anti-CD28 antibodies, as described in Example 11.
FIGURE 35B graphically illustrates the fraction of cells with intracellular IL-2 staining in the CD4⁺ T cell population in PBMC cultures treated with compound 10 (0, 0.1, 0.3, 1.0, or 3.0 µM), a representative of which is shown in FIGURE 35A, as described in Example 11.
FIGURE 35C graphically illustrates the fraction of cells with intracellular IL-2 staining in the CD8⁺ T cell population in PBMC cultures treated with compound 10 (0, 0.1, 0.3, 1.0, or 3.0), a representative of which is shown in FIGURE 35A, as described in Example 11.
FIGURE 35D graphically illustrates the fraction of cells with intracellular IL-2 staining in non-T cell populations in PBMC cultures treated with compound 10 (0, 0.1, 0.3, 1.0, or 3.0 µM), a representative of which is shown in FIGURE 35A, as described in Example 11.
FIGURE 35E graphically illustrates the fraction of cells with intracellular IL-10 staining in the CD4⁺ T cell population in PBMC cultures treated with compound 10 (0, 0.1, 0.3, 1.0, or 3.0 µM), a representative of which is shown in FIGURE 35A, as described in Example 11.
FIGURE 35F graphically illustrates the fraction of cells with intracellular IL-10 staining in the CD8⁺ T cell population in PBMC cultures treated with compound 10 (0, 0.1, 0.3, 1.0, or 3.0 µM), a representative of which is shown in FIGURE 35A, as described in Example 11.
FIGURE 35G graphically illustrates the fraction of cells with intracellular IL-10 staining in non-T cell populations in PBMC cultures treated with compound 10 (0, 0.1, 0.3, 1.0, or 3.0 µM), a representative of which is shown in FIGURE 35A, as described in Example 11.
FIGURE 35H graphically illustrates the fraction of cells with intracellular IFN-γ staining in the CD4⁺ T cell population in PBMC cultures treated with compound 10 (0, 0.1, 0.3, 1.0, or 3.0 µM), a representative of which is shown in FIGURE 35A, as described in Example 11.
FIGURE 35I graphically illustrates the fraction of cells with intracellular IFN-γ staining in the CD8⁺ T cell population in PBMC cultures treated with compound 10 (0, 0.1, 0.3, 1.0, or 3.0 µM), a representative of which is shown in FIGURE 35A, as described in Example 11.
FIGURE 35J graphically illustrates the fraction of cells with intracellular IFN-γ staining in non-T cell populations in PBMC cultures treated with compound 10 (0, 0.1, 0.3, 1.0, or 3.0 µM), a representative of which is shown in FIGURE 35A, as described in Example 11.
FIGURE 35K graphically illustrates the fraction of cells with intracellular TNF-α staining in the CD4⁺ T cell population in PBMC cultures treated with compound 10 (0, 0.1, 0.3, 1.0, 3.0, or 10 µM), a representative of which is shown in FIGURE 35A, as described in Example 11.
FIGURE 35L graphically illustrates the fraction of cells with intracellular TNF-α staining in the CD8⁺ T cell population in PBMC cultures treated with compound 10 (0, 0.1, 0.3, 1.0, 3.0, or 10 µM), a representative of which is shown in FIGURE 35A, as described in Example 11.
FIGURE 35M graphically illustrates the fraction of cells with intracellular TNF-α staining in the non-T cell populations in PBMC cultures treated with compound 10 (0, 0.1, 0.3, 1.0, 3.0, or 10 µM), a representative of which is shown in FIGURE 35A, as described in Example 11.
FIGURE 36A shows additional analysis of the non-T cell population from FIGURE 35A sorted into CD56⁺CD16⁻ (2.27%), CD56⁺CD16⁺ (11.8%) and non-NK (78.5%) populations present in the non-T cell population in a representative PBMC culture 2 days after stimulation with anti-CD3 and anti-CD28 antibodies, as described in Example 11.
FIGURE 36B graphically illustrates the fraction of cells with intracellular IFN-γ staining in non-T-CD56⁺CD16⁺ cell populations in PBMC cultures treated with compound 10 (0, 0.1, 0.3, 1.0, or 3.0 µM), a representative of which is shown in FIGURE 36A, as described in Example 11.
FIGURE 36C graphically illustrates the fraction of cells with intracellular IFN-γ staining in non-T-CD56⁺CD16⁻ cell populations in PBMC cultures treated with compound 10 (0, 0.1, 0.3, 1.0, or 3.0 µM), a representative of which is shown in FIGURE 36A, as described in Example 11.
FIGURE 36D graphically illustrates the fraction of cells with intracellular TNF-α staining in non-T-CD56⁺CD16⁺ cell populations in PBMC cultures treated with compound 10 (0, 0.1, 0.3, 1.0, 3.0, or 10 µM), a representative of which is shown in FIGURE 36A, as described in Example 11.
FIGURE 36E graphically illustrates the fraction of cells with intracellular TNF-α staining in non-T-CD56⁺CD16⁻ cell populations in PBMC cultures treated with compound 10 (0, 0.1, 0.3, 1.0, 3.0, or 10 µM), a representative of which is shown in FIGURE 36A, as described in Example 11.
FIGURE 37 graphically illustrates the amount of dextramer stained cells (antigen-specific T cells) as a percent of total CD8⁺ cells in splenocyte cultures obtained from WT and GPR174 KO mice three weeks after immunization with 1000 HAU influenza virus A/PR/8/34, and cultured for 5 days in the presence of NP antigenic peptide, wherein splenocytes obtained from naive WT mice are included as a control, as described in Example 12.
FIGURE 38 graphically illustrates the amount of dextramer stained cells (antigen-specific T cells) as a percent of total CD8⁺ cells in splenocyte cultures obtained from WT and GPR174 KO mice immunized and boosted three weeks later with 1000 HAU (hemagglutinin units) of influenza virus A/PR/8/34, wherein the splenocytes were analyzed 11 days after boost, and wherein splenoctyes obtained from naive WT mice are included as a control, as described in Example 12.
FIGURE 39A graphically illustrates the fraction of naive Regulatory T cells (Treg) with CTLA-4 positive staining (percentage of naive Treg cells (CD45RA+FOXP3+) in PBMC cultures treated with GPR174 inhibitory compound 10 (0, 0.19, 0.38, 0.75, 1.5, 3.0 or 6.0 µM) or equivalent amounts of vehicle control (DMSO), at one day post-stimulation, as described in Example 13;
FIGURE 39B graphically illustrates the fraction of non-regulatory T cells (non-Treg) with CTLA4 positive staining (percentage of naive non-Treg cells (CD45RA+FOXP3-) in PBMC cultures treated with GPR174 inhibitory compound 10 (0, 0.19, 0.38, 0.75, 1.5, 3.0 or 6.0 µM) or equivalent amounts of vehicle control (DMSO), at one day post-stimulation, as described in Example 13;
FIGURE 40A graphically illustrates the fraction of memory Treg with CTLA4 positive staining (percentage of memory Treg (CD45RA-FOXP3+) in PBMC cultures treated with GPR174 inhibitory compound 10 (0, 0.19, 0.38, 0.75, 1.5, 3.0, or 6.0 µM) or equivalent amounts of vehicle control (DMSO), at one day post-stimulation, as described in Example 13;
FIGURE 40B graphically illustrates the fraction of memory non-Treg cells with CTLA4 positive staining (percentage of memory non-Treg (CD45RA-FOXP3-) in PBMC cultures treated with GPR174 inhibitory compound 10 (0, 0.19, 0.38, 0.75, 1.5, 3.0, or 6.0 µM) or equivalent amounts of vehicle control (DMSO), at one day post-stimulation, as described in Example 13;
FIGURE 41 graphically illustrates the fraction of human CD4+ T cells with PD-L1 positive staining in PBMC cultures treated with GPR174 inhibitory compound 10 (0, 1.25 or 5µM) at one day post-stimulation, showing that PD-L1 expression is reduced in CD4+ T cells treated with compound 10 in a dose-dependent manner as compared to vehicle control treated cells, as described in Example 14;
FIGURE 42 graphically illustrates the fraction of human CD8+ T cells with TGIT positive staining in PBMC cultures treated with GPR174 inhibitory compound 10 (0, 1.25 or 5 µM) at one day post-stimulation, showing that TGIT expression is reduced in CD8+ T cells treated with compound 10 in a dose-dependent manner as compared to vehicle control treated cells, as described in Example 14; and
FIGURE 43 graphically illustrates the fraction of human CD4+ T cells with AREG positive staining in PBMC cultures treated with GPR174 inhibitory compound 10 (0, 1.25 or 5 µM) at one day post-stimulation, showing that AREG expression is reduced in CD4+ T cells treated with compound 10 in a dose-dependent manner as compared to vehicle control treated cells, as described in Example 14;
FIGURE 44 graphically illustrates the amount of IFN-γ in culture supernatants 24 hours post-stimulation in human PBMC cultures from a single donor cultured in the presence of GPR174 inhibitory compound 10 (3 µM); A2aRinhibitory compound (ZM241385, 1 µM or 10 µM); a combination of compound 10 (3 µM) plus 1 µM ZM241385; a combination of compound 10 (3 µM) plus 10 µM ZM241385; or vehicle control (DMSO), demonstrating that the combined inhibition of GPR174 and A2aR results in synergistic induction of IFN-γ production, as described in Example 15.
FIGURE 45A graphically illustrates the fold induction of Gs-signaling activity in HEK293 cells expressing wild-type GPR174 and cAMP biosensor expressing plasmid pGlo22F in the presence of increasing concentrations of phosphatidylserine liposomes (PS) or lysophosphatidylserine (Lyso-PS), as described in Example 16.
FIGURE 45B graphically illustrates the fold induction of Gs-signaling activity in HEK293 cells expressing mutant GPR174-v38 and cAMP biosensor expressing plasmid pGlo22F in the presence of increasing concentrations of phosphatidylserine liposomes (PS) or lysophosphatidylserine (Lyso-PS), as described in Example 16.
FIGURE 46 graphically illustrates the level of GPR174 Gs signaling activity in HEK293 cells expressing GPR174 and cAMP biosensor expressing plasmid pGlo22F (shown as the ratio of luminescence after phospholipid addition to the pre-read luminescence value) in the presence of PS, phosphatidylcholine (PC), phosphatidylethanolamine (PE), and phosphatidylinositol (PI) liposomes, as described in Example 16.
FIGURE 47A graphically illustrates the inhibition of PS-induced GPR174 Gs signaling by the GPR174 inhibitory compound 10 (Group I) in a dose-responsive manner, with an apparent IC₅₀ of about 20-40 nM, as described in Example 16.
FIGURE 47B graphically illustrates the inhibition of PS-induced GPR174 Gs signaling by the GPR174 inhibitory compound 6 (Group I) in a dose-responsive manner, with an apparent IC₅₀ of about 0.1 µM, as described in Example 16.
FIGURE 47C graphically illustrates the inhibition of PS-induced GPR174 Gs signaling by the GPR174 inhibitory compound 11 (Group I) in a dose-responsive manner, with an apparent IC₅₀ of about 0.1 µM, as described in Example 16.
FIGURE 47D graphically illustrates the inhibition of PS-induced GPR174 Gs signaling by the GPR174 inhibitory compound 20 (Group II) in a dose-responsive manner, with an apparent IC₅₀ of about 3.0 µM, as described in Example 16.
FIGURE 47E graphically illustrates the inhibition of PS-induced GPR174 Gs signaling by the GPR174 inhibitory compound 30 (Group IV) in a dose-responsive manner, with an apparent IC₅₀ of about 1.3 µM, as described in Example 16.
FIGURE 47F graphically illustrates the inhibition of PS-induced GPR174 Gs signaling by the GPR174 inhibitory compound 23 (Group IV) in a dose-responsive manner, with an apparent IC₅₀ of about 0.15 µM, as described in Example 16.
FIGURE 48A graphically illustrates the ratio of post-addition to pre-read luminescence of GPR174 and GloSensor expressing HEK293 cells exposed to culture medium, or untreated (non-apoptotic) K562 cells, or apoptotic K562 cells (treated with H₂O₂ for 20 hours), as described in Example 17.
FIGURE 48B graphically illustrates the ratio of post-addition to pre-read luminescence of GPR174 and GloSensor expressing HEK293 cells exposed to culture medium, or freshly isolated neutrophils, or apoptotic neutrophils (treated with anti-Fas antibodies), as described in Example 17.
FIGURE 48C graphically illustrates the ratio of post-addition to pre-read luminescence of GPR174 and GloSensor expressing HEK293 cells exposed to culture supernatant (Sup) or platelets (Pl), as described in Example 17.
FIGURE 49A graphically illustrates the amount of IFN-γ (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO), as described in Example 18.
FIGURE 49B graphically illustrates the amount of IL-2 (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO), as described in Example 18.
FIGURE 49C graphically illustrates the amount of TNF (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO), as described in Example 18.
FIGURE 50A graphically illustrates the amount of IFN-γ (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO), as described in Example 18.
FIGURE 50B graphically illustrates the amount of IL-2 (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO), as described in Example 18.
FIGURE 50C graphically illustrates the amount of TNF (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO), as described in Example 18.
FIGURE 51A graphically illustrates the amount of IFN-γ (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM); A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO), as described in Example 18.
FIGURE 51B graphically illustrates the amount of IL-2 (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM); A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO), as described in Example 18.
FIGURE 51C graphically illustrates the amount of TNF (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM); A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO), as described in Example 18.
FIGURE 52A graphically illustrates the fold change from vehicle in the amount of IFN-γ in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO), as described in Example 19.
FIGURE 52B graphically illustrates the fold change from vehicle in the amount of IL-2 in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO), as described in Example 19.
FIGURE 52C graphically illustrates the fold change from vehicle in the amount of TNF in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO), as described in Example 19.
FIGURE 52D graphically illustrates the fold change from vehicle in the amount of GM-CSF in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO), as described in Example 19.
FIGURE 53A graphically illustrates the amount of IFN-γ (pg/mL) in culture supernatants 24 hours post-stimulation of human PBMC cultures from a single donor (donor 3) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 53B graphically illustrates the amount of IL-2 (pg/mL) in culture supernatants 24 hours post-stimulation of human PBMC cultures from a single donor (donor 3) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 53C graphically illustrates the amount of TNF (pg/mL) in culture supernatants 24 hours post-stimulation of human PBMC cultures from a single donor (donor 3) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 54A graphically illustrates the amount of IFN-γ (pg/mL) in culture supernatants 24 hours post-stimulation of human PBMC cultures from a single donor (donor 6) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 54B graphically illustrates the amount of IL-2 (pg/mL) in culture supernatants 24 hours post-stimulation of human PBMC cultures from a single donor (donor 6) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 54C graphically illustrates the amount of TNF (pg/mL) in culture supernatants 24 hours post-stimulation of human PBMC cultures from a single donor (donor 6) cultured in the presence of GPR174 inhibitory compound 10 (1 µM); ZM, 0.1 µM); PBF (0.1 µM), SCH (0.2 µM), MRS (1 µM), Adenosine deaminase (ADA, 0.75 µg/mL), alone or in combination with compound 10, or vehicle control, as described in Example 20.
FIGURE 55A graphically illustrates the amount of IFN-γ (pg/mL) in supernatants 24 hours post-stimulation of human PBMC (donor 7) in the presence of GPR174 inhibitory compounds 6, 10, 11 or 20, each in the presence or absence of ZM, as described in Example 21.
FIGURE 55B graphically illustrates the amount of IL-2 (pg/mL) in supernatants 24 hours post-stimulation of human PBMC (donor 7) in the presence of GPR174 inhibitory compounds 6, 10, 11 or 20, each in the presence or absence of ZM, as described in Example 21.
FIGURE 55C graphically illustrates the amount of TNF (pg/mL) in supernatants 24 hours post-stimulation of human PBMC (donor 7) in the presence of GPR174 inhibitory compounds 6, 10, 11 or 20, each in the presence or absence of ZM, as described in Example 21.
FIGURE 55D graphically illustrates the amount of GM-CSF (pg/mL) in supernatants 24 hours post-stimulation of human PBMC (donor 7) in the presence of GPR174 inhibitory compounds 6, 10, 11 or 20, each in the presence or absence of ZM, as described in Example 21.
FIGURE 56A graphically illustrates the fold change from vehicle in the amount of IFN-γ in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1 µM) plus 0.2 µM ZM plus 2 µM MRS, or vehicle control (DMSO), as described in Example 22.
FIGURE 56B graphically illustrates the fold change from vehicle in the amount of IL-2 in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1 µM) plus 0.2 µM ZM plus 2 µM MRS, or vehicle control (DMSO), as described in Example 22.
FIGURE 56C graphically illustrates the fold change from vehicle in the amount of TNF in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1 µM) plus 0.2 µM ZM plus 2 µM MRS, or vehicle control (DMSO), as described in Example 22.
FIGURE 57 graphically illustrates the fold reduction in AREG+ cells in the presence of vehicle; GPR174 inhibitory compound 10 (1 µM); ZM-241385 (0.2 µM); or the combination of compound 10 (1 µM) and ZM (0.2 µM), as described in Example 23.
FIGURE 58 is a schematic diagram illustrating how both GPR174 and Adenosine receptors respond to products of cell stress and death, as described in Example 23.
FIGURE 59A graphically illustrates the growth of CT26 tumors in individual wild-type (WT) mice inoculated with CT26.cl25 murine colon carcinoma cells on day 0 and treated with anti-GITR antibody on day 7 and 9, as described in Example 24;
FIGURE 59B graphically illustrates the growth of CT26 tumors in individual GPR174-KO mice inoculated with CT26.cl25 murine colon carcinoma cells on day 0 and treated with anti-GITR antibody on day 7 and 9; as described in Example 24;
FIGURE 60 graphically illustrates the percent survival of tumor-bearing WT and GPR174-KO mice inoculated with CT26.cl25 murine colon carcinoma cells on day 0 and treated with anti-GITR antibody on day 7 and 9, demonstrating that GPR174-KO mice had a higher percent survival *(i.e.,* were euthanized significantly later) than WT mice (*p* = 0.03, log-rank test), as described in Example 24;
FIGURE 61A graphically illustrates the growth of CT26 tumors in individual WT mice inoculated with CT26.cl25 murine colon carcinoma cells on day 0 and treated with anti-GITR antibody on day 7, 9, and 14, as described in Example 24;
FIGURE 61B graphically illustrates the growth of tumors in individual GPR174-KO mice inoculated with CT26.cl25 murine colon carcinoma cells on day 0 and treated with anti-GITR antibody on day 7, 9, and 14; as described in Example 24;
FIGURE 62 graphically illustrates the percent survival of tumor-bearing WT and GPR174-KO mice inoculated with CT26.cl25 murine colon carcinoma cells on day 0 and treated with anti-GITR antibody on days 7, 9 and 14, demonstrating that GPR174-KO mice had a higher percent survival (*i.e.,* were euthanized significantly later) than WT mice, as described in Example 24;
FIGURE 63A graphically illustrates the growth of B16F10-Kb melanoma tumors in individual WT mice inoculated with B16F10-Kb cells on day 0 and treated with anti-GITR antibody on day 4 and 14, as described in Example 25;
FIGURE 63B graphically illustrates the growth of B16F10-Kb melanoma tumors in individual GPR174-KO mice inoculated with B16F 10-Kb cells on day 0 and treated with anti-GITR antibody on day 4 and 14, as described in Example 25;
FIGURE 63C graphically illustrates the average tumor volume of B16F10-Kb melanoma tumors in WT and GPR174-KO mice inoculated with B16F10-Kb cells on day 0 and treated with anti-GITR antibody on day 4 and 14, demonstrating that average tumor sizes were significantly smaller in GPR174 KO mice on days 14 (wherein "*" indicates p=0.01) and 16 (wherein "***" indicates: p=0.00005), as described in Example 25;
FIGURE 64 graphically illustrates the percent survival of B16F10-Kb melanoma tumor-bearing WT and GPR174-KO mice inoculated with B16F10-Bb cells on day 0 and treated with anti-GITR antibody on day 4 and 14, demonstrating that GPR174-KO mice had a higher percent survival (*i.e*., were euthanized significantly later) than WT mice (p = 0.006, log-rank test), as described in Example 25;
FIGURE 65 graphically illustrates IL-2 concentrations in cultures of stimulated human T cells in the presence or absence of PS liposomes (PSL) or a GPR174 inhibitor (compound 10, 3 µM), as described in Example 26;
FIGURE 66A graphically illustrates the concentration of IL-2 in cultures of stimulated human T cells in the presence or absence of PS liposomes, tumor exosomes, or a GPR174 inhibitory compound (compound 10, 0.5 µM), as described in Example 26;
FIGURE 66B graphically illustrates the concentration of IFN-γ in cultures of stimulated human T cells in the presence or absence of PS liposomes, tumor exosomes, or a GPR174 inhibitory compound (compound 10, 0.5 µM), as described in Example 26;
FIGURE 66C graphically illustrates the concentration of TNF in cultures of stimulated human T cells in the presence or absence of PS liposomes, tumor exosomes, or a GPR174 inhibitory compound (compound 10, 0.5 µM), as described in Example 26;
FIGURE 67 graphically illustrates the IL-2 levels (fold-change from vehicle) in cultures of stimulated WT or GPR174-KO mouse T cells in the presence or absence of PS liposomes (PSL) or a GPR174 inhibitor (compound 10, 1 µM), as described in Example 26;
FIGURE 68A graphically illustrates the effects of A2a/A2b adenosine receptor antagonist ZM-241385 (0.1 µM), GPR174 inhibitory Compound 10 (1 µM), or both compounds (ZN-241385 plus compound 10) on IL-2 levels in supernatants of cultured splenocytes isolated from n=3 WT mice following 2 days of stimulation with anti-CD3 and anti-CD28 antibodies, as described in Example 27;
FIGURE 68B graphically illustrates the effects of A2a/A2b adenosine receptor antagonist ZM-241385 (0.1 µM), GPR174 inhibitory Compound 10 (1 µM), or both compounds (ZN-241385 plus compound 10) on IL-2 levels in supernatants of cultured splenocytes isolated from n=3 GPR174-KO mice following 2 days of stimulation with anti-CD3 and anti-CD28 antibodies, as described in Example 27;
FIGURE 69 graphically illustrates the effects of A2a/A2b adenosine receptor antagonist ZM-241385 (0.2 µM), GPR174 inhibitory compound 10 (0.3 µM) or GPR174 inhibitory compound 6 (0.3 µM), or ZM-241385 plus compound 10 or ZM-241385 plus compound 6 on IL-2 levels in supernatants of cultured splenocytes isolated from 3 WT mice following 2 days of stimulation with anti-CD3 and anti-CD28 antibodies in the presence of the adenosine receptor agonist NECA (0.1 µM), as described in Example 27;
FIGURE 70A graphically illustrates the effects of A2a/A2b adenosine receptor antagonist ZM-241385 (0.2 µM), GPR174 inhibitory compounds 10 (0.3 µM) or compound 6 (0.3 µM), or ZM-241385 alone or combined with either GPR174 inhibitory compound on IL-2 levels in supernatants of cultured splenocytes isolated from 3 WT mice following 2 days of stimulation with anti-CD3 and anti-CD28 antibodies in the presence of the adenosine receptor agonist NECA (0.1 µM), as described in Example 27;
FIGURE 70B graphically illustrates the effects of A2a/A2b adenosine receptor antagonist ZM-241385 (0.2 µM), GPR174 inhibitory compounds 10 (0.3 µM) or compound 6 (0.3 µM), or ZM-241385 alone or combined with either GPR174 inhibitory compound on IL-2 levels in supernatants of cultured splenocytes isolated from 3 GPR174-KO mice following 2 days of stimulation with anti-CD3 and anti-CD28 antibodies in the presence of the adenosine receptor agonist NECA (0.1 µM), as described in Example 27;
FIGURE 71 graphically illustrates the levels of IL-2 in supernatants of human PBMC stimulated with anti-CD3 and anti-CD28 in the presence or absence of GPR174 inhibitory compound 10 (1µM); A2aR/A2bR inhibitory compound ZM-241385 (0.1 µM), or CD73 inhibitory compound APCP (10 µM) collected at 24 hours post-stimulation, as described in Example 28;
FIGURE 72A graphically illustrates the concentration of IL-2 in purified human T cells stimulated with CD3/CD28 activator in the presence of PS liposomes (PS, 1µM), adenosine receptor agonist NECA (0.1 µM), or a combination of PS (1µM) plus NECA (0.1 µM), as compared to media only, as described in Example 29;
FIGURE 72B graphically illustrates the concentration of IFN-γ (FIG 72B) in purified human T cells stimulated with CD3/CD28 activator in the presence of PS liposomes (PS, 1µM), adenosine receptor agonist NECA (0.1 µM), or a combination of PS (1µM) plus NECA (0.1 µM), as compared to media only, as described in Example 29; and
FIGURE 72C graphically illustrates the concentration of IFN-γ (FIG 72C) in purified human T cells stimulated with CD3/C28 activator in the presence of PS liposomes (PS, 1µM), adenosine receptor agonist NECA (0.05 µM), or a combination of PS (1 µM) plus NECA (0.05 µM), as compared to media only, as described in Example 29.

### DETAILED DESCRIPTION

As set forth in detail below, the present inventors have identified compounds that functionally interact with GPR174 and are capable of inhibiting one or more GPR174-mediated signaling pathways. We have further characterized the signaling pathways activated by GPR174 and have determined a signaling profile for this receptor that includes the Gs signaling pathway. The inventors have further determined that the combined inhibition of GPR174 and inhibition of the Adenosine 2a Receptor (A2aR) and/or Adenosine 2b Receptor (A2bR), or a combination of a GPR174 inhibitory and an inhibitor of ATP hydrolysis into adenosine by CD38, CD39 and CD73 (such as a CD73 inhibitor and/or a a CD38 inhibitor and/or a CD39 inhibitor) and/or a Treg attenuating agent results in synergistic induction of IFN-γ, IL-2, TNF and GM-CSF production in human PBMCs. The inventors have further discovered that phosphatidylserine (PS) is an agonist for GPR174-mediated Gs signaling and have demonstrated that PS signaling through GPR174 is inhibited by multiple GPR174 inhibitory small molecule compounds with diverse chemical structures. As further demonstrated herein, GPR174-deficiency enhances anti-tumor immune responses in mice.

Based on these discoveries, the present disclosure relates to *in vivo* and *in vitro* methods of inhibiting the GPR174-mediated signaling pathways, either alone, or in combination with the inhibition of ATP-Adenosine-A2aR and/or A2bR- mediated signaling (such as an A2aR antagonist and/or an A2bR antagonist), or a combination of an inhibitor of GPR174-mediated signaling and an inhibitor of ATP hydrolysis into adenosine by CD38, CD39 and CD73 (such as a CD73 inhibitor or a CD38 inhibitor or a CD39 inhibitor) and/or a Treg attenuating agent, and thereby stimulating an immune response in a mammalian subject, particularly in subjects suffering from conditions such as cancer as described herein.

### Definitions

The term "G-protein coupled receptor" or "GPCR," or "GPR" refers to a transmembrane receptor that is capable of transmitting a signal from the outside of a cell to the inside of a cell through a G-protein pathway and/or an arrestin pathway. Hundreds of such receptors are known in the art; see, *e.g.,* Fredriksson et al., Mol. Pharmacol. 63:1256-1272, 2003, and Vassilatis, D.K., Proc Natl Acad Sci USA 100: 4903-4908 (2003). These references have characterized the human and mouse GPCRs based on sequence homology and function. Human GPCRs can be broken down into five classes: secretin, rhodopsin, glutamate, frizzled/Tas2, and adhesion. Alternatively, receptors may be classified by their ligands, *e.g.,* peptide hormones or small molecules (*e*.*g*., biogenic amines). Other classification schemes include the A-F classification, where class A represents receptors related to rhodopsin and the adrenergic receptors, class B, receptors related to the calcitonin and parathyroid hormone receptors, class C, receptors related to the metabotropic receptors, and classes D-F represent receptors found in fungi and archaebacteria.

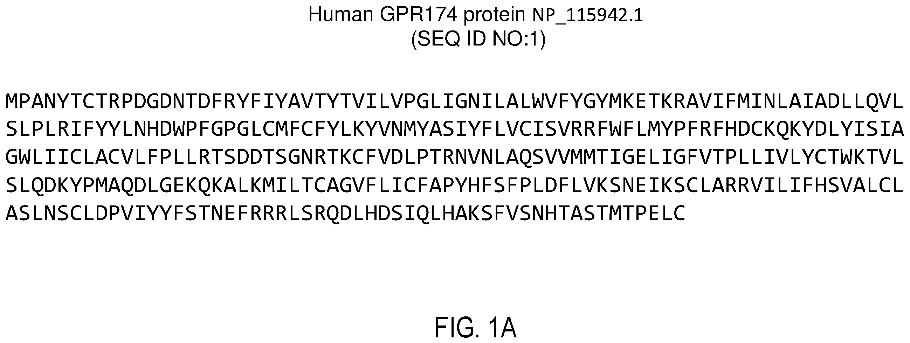

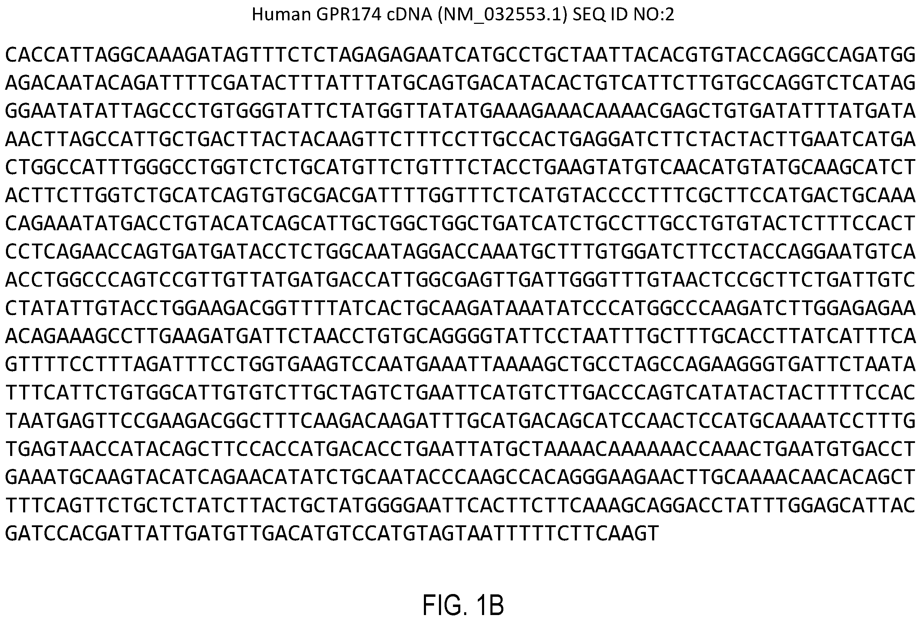

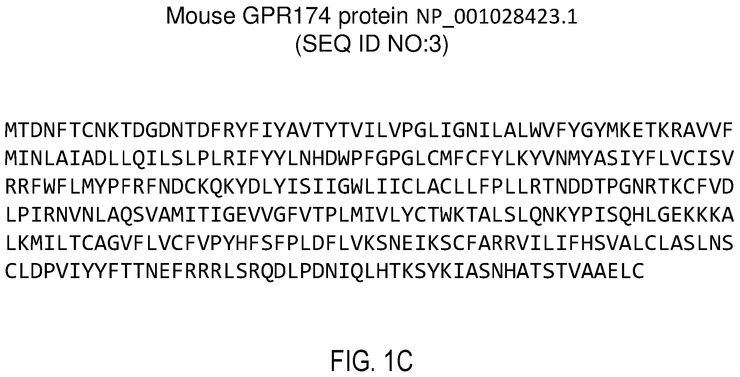

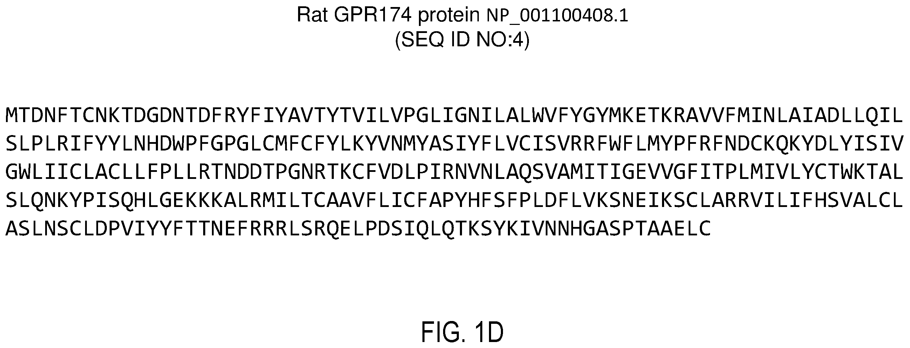

The terms "G-protein coupled receptor 174," "GPR174," "FKSG79, or "GPCR17" refer to any naturally occurring forms of the GPR174 protein, *e.g.,* SEQ ID NO:1 shown in Figure 1, or naturally occurring variants thereof, such as variants having at least 90% identity (such as at least 91%, 92%, 93%, 94%, 95%, 96%, 97%, 98% or 99% identity) to SEQ ID NO: 1. Preferable forms of GPR174 have the ability to signal through at least one G-protein coupled receptor pathway such as Gs.

The term "G-protein" refers to a heterotrimeric protein complex that transmits a signal from an activated GPCR to effector molecule(s) inside the cell such as enzymes and ion channels. G-proteins are made up of Gα, Gβ, and Gγ subunits. Families of Gα subunits include Gq, Gi, Gs, and Gα12/13. G-protein signaling pathways are named for the activated Gα subunit, *i.e.,* Gαs, Gαi, Gαq, and Gα12/13. A heterotrimeric G-protein binds to an activated GPCR protein, that is, a GPCR protein that is bound to a ligand or surrogate ligand. When bound to a GPCR protein, the Gα subunit exchanges bound guanosine diphosphate (GDP) for guanosine-5'-triphosphate (GTP) and dissociates from the Gβ and Gγ subunits, which are typically associated in a heterodimeric complex. Once dissociated, both the Gα-GTP-bound protein and the Gβγ complex can activate signaling pathways. The Gq family includes Gαq, Gα11, Gα14, and Gα15/16. The Gi family includes Gαi1-3, Gαo, Gαt, Gαgust, and Gαz. The Gs family includes Gαs and Gαolf. The G12/13 includes Gα12 and Gα13.

The term "signaling pathway" refers to an intracellular response to a signal. For the purposes of this disclosure, a G-protein signaling pathway refers to an intracellular response that occurs after binding of a ligand, surrogate ligand, or any other functional activator or functional inactivator to a G-protein coupled receptor, *e.g.,* GPR174, and activates a G-protein coupled pathway. G-protein signaling pathways include the Gq, Gi, Gs, G12/13 and βγ pathways. An arrestin signaling pathway refers to an intracellular response that occurs after binding of a ligand, surrogate ligand, or any other functional activator or functional inactivator to a G-protein coupled receptor, *e.g.,* GPR174, and activation of an arrestin-mediated signaling pathway, such as arrestin 1, arrestin 2, arrestin 3, and arrestin 4 signaling pathways.

A "Gq signaling pathway" or "Gαq signaling pathway" refers to an intracellular signaling pathway that is activated by an activated Gq alpha protein. The Gαq protein is activated by exchanging GDP for GTP after binding to an activated GPCR protein. Activation of a Gq signaling pathway typically activates phospholipase C β isoforms (PLCβ), which hydrolyze phosphatidylinositol to generate diacylglycerol (DAG) and inositol 1,4,5-trisphosphate (IP₃). IP₃ is soluble and diffuses through the cytoplasm and interacts with IP₃ receptors on the endoplasmic reticulum, causing the release of calcium and raising the level of intracellular calcium. DAG remains tethered to the inner leaflet of the plasma membrane due to its hydrophobic character, where it recruits protein kinase C (PKC), which becomes activated in conjunction with binding calcium ions. PKC then phosphorylates other proteins to control their function. Downstream effects can also be detected, e.g., expression of a reporter gene regulated by an appropriate transcription factor.

A "Gi signaling pathway" or "Gαi signaling pathway" refers to an intracellular signaling pathway that is activated by an activated Gi alpha protein. The Gαi protein is activated after binding to an activated GPCR protein. Gαi1-3, Gαo, and Gαz inhibit the activity of adenylyl cyclases (AC). The sensory Gi protein, Gαt, activates the cyclic guanosine monophosphate (cGMP)-dependent phosphodiesterase, causing a decrease in intracellular cGMP and Gαgust activates phospholipase C (PLC). All Gᵢ family members, except for Gαz, are sensitive to inhibition by pertussis toxin via adenosine diphosphate (ADP) ribosylation of their α subunit (Siehler, Biotechnology 3:471-83, 2008).

A "Gs signaling pathway" or "Gαs signaling pathway" refers to an intracellular signaling pathway that is activated by an activated Gs alpha protein. The Gαs protein is activated after binding to an activated GPCR protein. The Gs family of G proteins (Gαs and Gαolf) activates AC, which generates the second messenger 3',5'-cyclic adenosine monophosphate (cAMP).

A "G12/13 signaling pathway" or "Gα12/13 signaling pathway" refers to an intracellular signaling pathway that is activated by an activated G12/13 alpha protein. The Gα12/13 protein is activated after binding to a GPCR protein. The G12/13 family of G proteins (Gα12 and Gα13) activates Rho guanine exchange factor (Rho-GEF) proteins. Rho-GEF proteins catalyze the exchange of GDP for GTP to activate RhoA. RhoA, in turn, activates Rho kinase, which further leads to the activation of Activating Transcription Factor 2 (ATF2) and results in cellular responses (Liu et al., Methods Mol. Biol. 237:145-9, 2004).

A "βγ signaling pathway" refers to an intracellular pathway that is activated by a free (*i.e.,* unbound to Gα protein) βγ complex. βγ complexes can, for example, activate phospholipase A2, direct opening of G-protein coupled inward rectifying potassium channels (GIRKs) when bound to muscarinic acetylcholine receptors, activate L-type calcium channels, and initiate the phospholipase C pathway by activating PLC.

An "arrestin signaling pathway" refers to an intracellular signaling pathway that is activated by arrestin 1, arrestin 2, arrestin 3, or arrestin 4. The arrestins have been implicated in signaling through ERK, JNK, p38, Akt, PI3 kinase, and RhoA (DeWire et al., Annu. Rev. Physiol. 69:483-510, 2007).

The term "contacting" is used herein interchangeably with the following: combined with, added to, mixed with, introducing to, passed over, incubated with, flowed over, etc. For purposes of clarity, the phrase "contacting a cell" includes introducing a compound into a mammal (*e*.*g*., orally, into the plasma, or intramuscularly) such that the compound contacts the cells of the mammal *in vivo.*

By the term "GPR174-mediated signaling pathway" is meant a signaling pathway the activity of which is modulated at least in part through GPR174.

As used herein, the phrase "modulates at least one GPR174-mediated signaling pathway" refers to modulating (activating or inhibiting) at least the Gs signaling pathway that is functional in a cell expressing GPR174 by contacting the cell with a compound that functionally interacts with GPR174.

A "modulator" is a compound that functionally interacts with a GPCR and affects the GPCR-mediated signaling activity either by itself or by altering the ability of another compound to affect the GPCR-mediated signaling activity. Modulators include activators and inhibitors of the GPCR-mediated signaling pathway.

An "activator" is a compound that increases GPCR-mediated signaling in a signaling pathway. Activators can activate a receptor directly (*e.g.,* an agonist) or can increase activation of a receptor by another compound (*e.g.,* a positive allosteric modulator). Activators can bind to and increase receptor activity, increase, open, activate, facilitate, enhance activation, sensitize, agonize, drive the conformation of the receptor to the active state, or up-regulate receptor-protein activity. Activators include agonists, partial agonists, and positive allosteric modulators.

An "agonist," which may be a full agonist or a partial agonist, binds to a GPCR, activates the receptor, and initiates a response, typically through activation of a G-protein. An agonist increases receptor activity as compared to the normal baseline level. A compound is deemed to be a receptor agonist if it increases GPCR-mediated signaling pathway activity by at least 20% over an appropriate control (*e*.*g*., background or basal activity) at a concentration of up to 50 µM. A full agonist is always an activator. A partial agonist has a lower intrinsic activity than a full agonist but increases receptor activity as compared to basal activity. A partial agonist may act as a functional inhibitor when in the presence of a full agonist.

A "positive allosteric modulator" or "PAM" is a compound that binds to the GPCR at a site distinct from the ligand-binding site and increases GPCR-mediated signaling activity in response to agonists.

An "inhibitor" is a compound that decreases GPCR-mediated signaling in a signaling pathway. Inhibitors are compounds that functionally interact with a GPCR and partially or totally block activity, decrease, prevent, delay activation, inactivate, antagonize, desensitize, drive the conformation of the receptor to the inactive conformation, block the ability of another compound (*e*.*g*., an endogenous agonist ligand) to interact with the receptor, or otherwise down-regulate the activity of the receptor. Inhibitors can reduce basal activity of the receptor (*e.g.,* an inverse agonist) or can block or reduce activity of another compound (*e.g.,* a partial agonist or antagonist). Inhibitors include antagonists, inverse agonists, partial agonists, partial inverse agonists, and negative allosteric modulators. Inhibitors do not include compounds that act solely by decreasing expression of the receptor nucleic acid or protein.

An "antagonist" is an inhibitor that binds to a GPCR, usually at the same site as a ligand or an agonist. On its own, an antagonist does not activate or inhibit signaling activity via the receptor and does not change receptor activity from basal levels. However, an antagonist is able to inhibit or block activation of a receptor in the presence of an agonist or a ligand or inhibit or block inhibition of a receptor in the presence of an inverse agonist. A compound is deemed to be a receptor antagonist or allosteric modulator if, at a concentration of up to 50 µM, it has activity in a CRA assay, and it increases GPCR-receptor mediated signaling pathway activity by less than a 20% increase over an appropriate control (*e.g.,* background or basal), or decreases GPCR-mediated signaling pathway activity by less than 10% inhibition in comparison to an appropriate control (*e*.*g*., background or basal). Additionally, a compound that inhibits the effect of an agonist or inverse agonist can be considered an antagonist.

An "inverse agonist," which may be a full inverse agonist or a partial inverse agonist, is an inhibitor that binds to a receptor and reduces the basal signaling of the GPCR-mediated signaling pathway. A compound is deemed to be an inverse agonist if, at a concentration of up to 50 µM, it reduces the basal signaling of a GPCR-mediated signaling pathway by at least 10%. A partial inverse agonist has less intrinsic inhibitory activity than a full inverse agonist.

A "negative allosteric modulator" is a compound that binds to a receptor at a site distinct from the ligand binding site and decreases GPCR-mediated signaling activity in response to agonists.

A "partial agonist" can be a functional activator or a functional inhibitor, depending on the presence or absence of a full agonist in a given biological environment. Partial agonists bind the receptor and increase receptor activity as compared to the basal level of activity but have only partial efficacy relative to a full agonist. For example, in the presence of a full agonist, a partial agonist can decrease receptor activation, thereby acting as a functional inhibitor. In the absence of a full agonist, the partial agonist can increase receptor activation, thereby acting as a functional activator.

A "partial inverse agonist" can be a functional activator or a functional inhibitor, depending on the presence or absence of a full inverse agonist in a given biological environment. Partial inverse agonists bind the receptor and decrease receptor activity as compared to the basal level of activity but have only partial efficacy relative to a full inverse agonist. For example, in the presence of a full inverse agonist, a partial inverse agonist can increase receptor activation, thereby acting as a functional activator. In the absence of a full inverse agonist, the partial inverse agonist can decrease receptor activation, thereby acting as a functional inhibitor.

As used herein, the term "basal level of activity" or "basal signaling activity," or baseline activity" refers to the level of GPCR-mediated signaling activity in the absence of a modulator compound. In one case, a basal level of GPR174-mediated signaling pathway activity is established with reference to a specific cell line wherein the cell line is known to have functional GPR174 activity or defective GPR174 activity. In another case, a basal level of GPR174-mediated signaling pathway activity is established with reference to a cell over-expressing GPR174 (*e.g.,* as described in Examples 2, 3, and 4 herein). In some cases, a basal level of GPR174-mediated signaling pathway activity is established with reference to a cell known to interact with an endogenous ligand, or known to contain an endogenous GPR174 ligand, or with reference to a cell known to not contain an endogenous GPR174-ligand, assuming such an endogenous GPR174 ligand is later identified.

A "ligand" is a compound that binds to a receptor and modulates the activity of the receptor.

An "endogenous ligand" is an endogenous entity (e.g., molecule, peptide, or ion) that modulates a receptor in a cell, tissue, or organism of origin.

A "surrogate ligand" is a non-endogenous molecule that binds to and modulates the activity of a receptor. A surrogate ligand may be naturally occurring or may be synthetic. A surrogate ligand can activate or inhibit activity of a receptor. In one case, a surrogate ligand is a small molecule. In a further case, a surrogate ligand is a small organic molecule.

A "biased ligand" is a compound that binds to a GPCR and selectively modulates (more than 2-fold) the activity of one of the GPCR-mediated signaling pathways (*e.g.,* Gq, Gi, Gs, G12/13, βγor an arrestin signaling pathway) as compared to other GPCR-mediated signaling pathway(s).

As used herein, the term "apparent binding affinity" refers to the observed change in binding affinity of a compound for GPR174 in the presence of an additional compound that binds GPR174 compared to the binding affinity observed for the compound for GPR174 without the presence of the additional compound. In some cases, the compound and the additional compound compete for binding at the same site on GPR174. Although the binding affinities of each compound for GPR174 do not change, determining the binding affinity of one compound for GPR174 in the presence of an additional compound that binds GPR174 may result in the measured binding affinity of the compound for GPR174 to decrease. In some cases, the two compounds do not bind at the same site, and the allosteric binding of one compound to GPR174 may result in the binding affinity for GPR174 of the other compound to decrease or increase. This may be due to conformational changes in GPR174 caused by the binding of one of the compounds to GPR174 that affect the binding site of the other compound.

A compound is "specific for" GPR174 if it is capable of modulating (*e*.*g*., inhibiting) GPR174 activity but does not have similar activity at other proteins such as other G-protein coupled receptors, *e.g.,* as compared to a reference panel of proteins (*e.g.,* including GPCRs other than GPR174, and, optionally, also including ion channels and/or other types of transporters). In one example, the reference panel includes the following receptors: muscarinic M1, CCRL2, CMKOR1, GPR3, GPR4, GPR12, GPR17, GPR18, GPR19, GPR20, GPR21, GPR22, GPR25, GPR26, GPR27, GPR31, GPR32, GPR34, GPR37, GPR37L1, GPR39, GPR43, GPR45, GPR48, GPR50, GPR52, GPR61, GPR62, GPR63, GPR65, GPR68, GPR78, GPR80, GPR83, GPR85, GPR87, GPR88, GPR101, GPR132, GPR135, GPR139, GPR141, GPR146, GPR148, GPR149, GPR150, GPR151, GPR152, GPR153, GPR160, GPR161, GPR162, GPR173, GPR182, GPR183, LGR5, LGR6, MAS1, MRGD, MRGE, MRGF, MRGI4, OPN3, OPN4, OPN5, P2Y8, P2Y10, TAAR6, and TAAR8.

In another example, the reference panel includes the following receptors: human adenosine A1; human adenosine A2A; rat adrenergic α1A; rat adrenergic α1B; human adrenergic α2A; human adrenergic β1; human adrenergic β2; rat calcium channel L-type dihydropyridine; human dopamine D1; human dopamine D2; and human G protein-coupled receptor, GPR103. In another example, the reference panel includes the following receptors: rat GABA A; rat glutamate NMDA; human histamine H₁; human muscarinic M₂; human nicotinic acetylcholine α₁; human opiate µ (OP3, MOP); human potassium channel [K_{ATP}]; human potassium channel hERG; human sigma σ₁; rat sigma σ₂; rat sodium channel, site 2; and human transporter, norepinephrine.

In certain cases, a compound that is specific for GPR174 has 2-, 5-, 10-, 25-, 50-, 100-, 500-, 1000-, 5000-, or 10,000-fold greater activity at inhibiting GPR174 activity as compared to other G-protein coupled receptors, *e.g.,* the other GPCRs in one or more of the reference panels described above.

In certain cases, a compound that is specific for GPR174 has 2-, 5-, 10-, 25-, 50-, 100-, 500-, 1000-, 5000-, or 10,000-fold greater activity in a Gs signaling assay that is carried out in in a cell expressing GPR174 as compared to the Gs signaling activity in a cell expressing other GPCRs, *e.g.* the other GPCRs in each of the reference panels described above.

An "agency-approved compound" refers to a compound that was approved for clinical use for human or veterinary purposes prior to November 4, 2016 by the United States Food and Drug Administration (FDA) or similar governmental regulatory agency (*e.g.,* European Medicines Agency (EMA), Health Canada, Japanese Ministry of Health and Welfare).

A compound that is "not approved for use in a disease associated with GPR174" refers to a compound that has not been approved prior to November 4, 2016 for therapeutic use by the FDA or similar governmental regulator agency (*e.g.,* EMA, Health Canada, and Japanese Ministry of Health and Welfare) for an indication that is associated with GPR174 activity (*e.g*., any of those described herein, such as cancer or a nervous system disease or disorder).

By a compound that "is not approved as targeting GPR174" is meant a compound that is not indicated in the pharmacology data submitted to the US FDA or similar governmental regulatory agency (*e.g.,* EMA, Health Canada, and Japanese Ministry of Health and Welfare) as targeting GPR174 prior to November 4, 2016. An indication of such targeting is often found in the label of the approved drug.

The term "compound" or grammatical equivalents as used herein refers to molecules, either naturally occurring or synthetic, *e.g.,* protein; antibody, oligopeptide (*e.g.,* from about 5 to about 25 amino acids in length, such as from about 10 to 20 or 12 to 18 amino acids in length, for example, 12, 15, or 18 amino acids in length); nucleotides (*e*.*g*., inhibitory RNA) that inhibits GPR174 expression, small molecule chemical compound, *e*.*g*., small organic, organometallic, or inorganic molecule; polysaccharide; oligonucleotides; lipid; and fatty acid. The compound can be included in a library of compounds, such as a combinatorial, synthetic, natural, heterocyclic, drug-like, lead-like, organic, inorganic, unrandomized, or randomized library that provides a sufficient range of diversity or it may be a focused or targeted collection of the above compounds. Compounds are optionally linked to a fusion partner, *e*.*g*., targeting compounds, rescue compounds, dimerization compounds, stabilizing compounds, addressable compounds, and other functional moieties. Conventionally, new chemical entities with useful properties are generated by identifying a compound (called a "lead compound") having some desirable property or activity, *e*.*g*., inhibitory activity, creating variants of the lead compound, and evaluating the property and activity of those variant compounds. Often, high-throughput screening ("HTS") methods are employed for such an analysis.

The terms "small molecule," "small organic molecule," and "small inorganic molecule" refer to molecules (either organic, organometallic, or inorganic), organic molecules, and inorganic molecules, respectively, which are either naturally occurring or synthetic and that have a molecular weight of more than about 50 Da and less than about 2500 Da. Small organic (for example) molecules may be less than about 2000 Da, between about 100 Da to about 1000 Da, or between about 100 to about 600 Da, or between about 200 to 500 Da.

The term "a compound predetermined to functionally interact with GPR174" is a compound that has been determined, prior to contacting a cell expressing GPR174 or administering the compound to the subject, to modulate a GPR174-mediated signaling pathway by functionally interacting with GPR174. This predetermination may be made by either
(i) directly acquiring knowledge that the compound functionally interacts with GPR174 by physically demonstrating that the compound functionally interacts with GPR174 by performing an assay measuring functional interaction with GPR174, or by performing an assay measuring GPR174-mediated signaling activity, or
(ii) by indirectly acquiring knowledge that the compound functionally interacts with GPR174 by receiving or obtaining such knowledge or information regarding the results from others physically demonstrating such interaction using an assay measuring functional interaction of the compound with GPR174 or measuring GPR174-mediated signaling activity from another party or source (*e*.*g*., knowledge or information regarding the results from research carried out by others physically demonstrating such interaction using an assay measuring functional interaction of the compound with GPR174 or measuring GPR174-mediated signaling activity, obtained, for example, from publication in a scientific journal, or in a published patent application, or by reading prescribing information, or obtained through private channels (*e*.*g*., personal communication), in each case prior to contacting with the compound a cell expressing GPR174 or administering the compound to the subject. In one case, the compound is predetermined to functionally interact with human GPR174 by directly or indirectly acquiring knowledge that the compound functionally interacts with human GPR174 set forth as SEQ ID NO: 1, or a naturally occurring variant of GPR174 having at least 95% identity to SEQ ID NO: 1.

By "therapeutically effective amount" is meant an amount that produces a desired effect for which it is administered, *e*.*g*., improvement or delay of at least one symptom associated with the disease or condition being treated. The exact dose will depend on the purpose of the treatment and can be ascertained by one skilled in the art using known techniques (see, *e*.*g*., Lieberman, Pharmaceutical Dosage Forms (vols. 1-3, 1992); Lloyd, The Art, Science and Technology of Pharmaceutical Compounding (1999); and Pickar, Dosage Calculations (1999)).

By "substantially pure" or "isolated" is meant a compound (*e.g.,* a polypeptide or conjugate) that has been separated from other chemical components. Typically, the compound is substantially pure when it is at least 30%, by weight, free from other components. In certain cases, the preparation is at least 50%, 60%, 75%, 85%, 90%, 95%, 96%, 97%, 98%, or 99% by weight, free from other components. A purified polypeptide may be obtained, for example, by expression of a recombinant polynucleotide encoding such a polypeptide or by chemically synthesizing the polypeptide. Purity can be measured by any appropriate method, for example, column chromatography, polyacrylamide gel electrophoresis, or by HPLC analysis.

In the context of a naturally occurring compound, the term "isolated" is one which is altered or removed from the natural state (*e*.*g*., through human intervention).

The term "mammal" includes all mammals, including without limitation humans, non-human primates, dogs, cats, horses, sheep, goats, cows, rabbits, pigs, and rodents.

As used herein, and as well understood in the art, "to treat" a disease, disorder, or condition, "treatment" of the disease, disorder, or condition (*e*.*g*., the conditions described herein, such as inflammatory conditions), or "therapy" is an approach for obtaining beneficial or desired results, such as clinical results, and can be performed either for prophylaxis or during the course of clinical pathology. Beneficial or desired results can include, but are not limited to, alleviation or amelioration of one or more symptoms or conditions; diminishment of extent of the disease, disorder, or condition; reduced likelihood of developing the disease, disorder, or condition; stabilized (*i.e.,* not worsening) state of disease, disorder, or condition; preventing the development of or spread of the disease, disorder, or condition; delay or slowing the progress of the disease, disorder, or condition; amelioration or palliation of the disease, disorder, or condition; and remission (whether partial or total), whether detectable or undetectable. "Palliating" a disease, disorder, or condition means that the extent and/or undesirable clinical manifestations of the disease, disorder, or condition are lessened and/or time course of the progression is slowed or lengthened, as compared to the extent or time course in the absence of treatment.

As used herein, the terms "subject" or "patient" refer to any organism to which a compound or composition in accordance with the disclosure may be administered, *e*.*g*., for experimental, diagnostic, prophylactic, and/or therapeutic purposes. A subject to be treated with a compound or composition described here may be one who has been diagnosed by a medical practitioner as having a disease, disorder, or condition, described herein, or one at risk for developing the disease, disorder, or condition described herein. Diagnosis may be performed by any technique or method known in the art. One skilled in the art will understand that a subject may have been diagnosed as having the disease, disorder, or condition, using a standard test or examination or may have been identified, without examination, as one at high risk due to the presence of one or more risk factors. Typical subjects include animals (*e.g*., mammals such as mice, rats, rabbits, non-human primates, and humans).

As used herein, the term "neoplasm" refers to any new and abnormal growth of cells, specifically one in which cell multiplication is uncontrolled and progressive. Neoplasms may be non-malignant (*i.e.* benign) or malignant.

As used herein, the term "tumors" means neoplasms, including solid and liquid *(i.e.,* blood) neoplasms, and benign and malignant neoplasms, including primary and/or metastatic neoplasms.

The term "alkanoyl," as used herein, refers to a group having the structure - C(O)-R, in which R is alkyl. Alkanoyl may be unsubstituted or substituted (*e.g*., optionally substituted alkanoyl) as described for alkyl. The suffix "oyl" may be used to define other groups having the structure -C(O)-R. For example, in alkenoyl group, R is alkenyl; in alknynoyl group, R is alkynyl; in cycloalkanoyl group, R is cycloalkyl; in cycloalkenoyl group, R is cycloalkenyl; and in cycloalkynoyl group, R is cycloalkynyl (all groups are as defined herein). Further, the groups defined with a suffix "oyl" may be further used to define groups having the structure -O-C(O)-R' by adding the suffix "oxy," *e*.*g*., when R' is alkyl, this group is "alkanoyloxy." For example, in alkenoyloxy group, R' is alkenyl; in alknynoyloxy group, R' is alkynyl; in cycloalkanoyloxy group, R' is cycloalkanyl; in cycloalkenoyloxy group, R' is cycloalkenyl; and in cycloalkynoyloxy group, R' is cycloalkynyl (all groups are as defined herein). Each of these groups may be unsubstituted or substituted (*e.g*., optionally substituted) as described for each respective group.

The term "alkenyl," as used herein, refers to a straight-chain or branched-chain monovalent substituent including one or two carbon-carbon double bonds and containing only C and H when unsubstituted. Alkenyl group may contain, unless otherwise specified, 2, 3, 4, 5, or 6 carbon atoms, excluding the carbon atoms of any substituents, if present. Non-limiting examples of alkenyl groups include ethenyl, prop-1-enyl, prop-2-enyl, 1-methylethenyl, but-1-enyl, but-2-enyl, but-3-enyl, 1-methylprop-1-enyl, 2-methylprop-1-enyl, and 1-methylprop-2-enyl. Alkenyl may be unsubstituted or substituted (e.g., optionally substituted alkenyl) as described for alkyl.

The term "alkenylene," as used herein, refers to a straight-chain or branched-chain divalent substituent including one or two carbon-carbon double bonds and containing only C and H when unsubstituted. Alkenylene group may contain, unless otherwise specified, 2, 3, 4, 5, or 6 carbon atoms, excluding the carbon atoms of any substituents, if present. Non-limiting examples of alkenylene groups include ethen-1,1-diyl; ethen-1,2-diyl; prop-1-en-1,1-diyl, prop-2-en-1,1-diyl; prop-1-en-1,2-diyl, prop-1-en-1,3-diyl; prop-2-en-1,1-diyl; prop-2-en-1,2-diyl; but-1-en-1,1-diyl; but-1-en-1,2-diyl; but-1-en-1,3-diyl; but-1-en-1,4-diyl; but-2-en-1,1-diyl; but-2-en-1,2-diyl; but-2-en-1,3-diyl; but-2-en-1,4-diyl; but-2-en-2,3-diyl; but-3-en-1,1-diyl; but-3-en-1,2-diyl; but-3-en-1,3-diyl; but-3-en-2,3-diyl; buta-1,2-dien-1,1-diyl; buta-1,2-dien-1,3-diyl; buta-1,2-dien-1,4-diyl; buta-1,3-dien-1,1-diyl; buta-1,3-dien-1,2-diyl; buta-1,3-dien-1,3-diyl; buta-1,3-dien-1,4-diyl; buta-1,3-dien-2,3-diyl; buta-2,3-dien-1,1-diyl; and buta-2,3-dien-1,2-diyl. Alkenylene may be unsubstituted or substituted (*e.g*., optionally substituted alkenylene) as described for alkylene.

The term "alkoxy" represents a chemical substituent of formula -OR, where R is an optionally substituted alkyl group (*e.g*., optionally substituted C₁-C₆ alkyl group). The substituted alkoxy group can have 1, 2, 3, 4, 5, or 6 substituent groups as defined herein. Similarly, the term "arylalkoxy" represents a chemical substituent of formula -OR, where R is an optionally substituted arylalkyl group. The term "cycloalkoxy" represents a substituent of formula -OR', where R' is an optionally substituted cycloalkyl group as described herein. Similarly, the term "alkenoxy" represents a chemical substituent of formula -OR", where R" is an optionally substituted alkenyl group as described herein.

The term "alkyl," as used herein, refers to a saturated straight-chain or branched-chain monovalent substituent, containing only C and H when unsubstituted. Alkyl group may contain, unless otherwise specified, 1, 2, 3, 4, 5, or 6 carbon atoms, excluding the carbon atoms of any substituents, if present. Non-limiting examples of alkyl group include methyl, ethyl, isobutyl, *tert*-butyl, and the like. Alkyl group may be unsubstituted or substituted (*e.g*., optionally substituted alkyl) with 1, 2, 3, 4, 5, or 6 substituents independently selected from the group consisting of: halo (*e.g.,* F, Cl, Br, or I), CN, NO₂, CF₃, OCF₃, COOR', CONR'₂, OR', SR', SOR', SO₂R', NR'₂, NR'(CO)R', NR'C(O)OR', NR'C(O)NR'₂, NR'SO₂NR'₂, NR'SO₂R', oxo (=O), or oximido (=NOR"), where each R' is, independently, H or an optionally substituted group selected from alkyl, alkenyl, alkynyl, cycloalkyl, cycloalkenyl, cycloalkynyl, heteroalkyl, heteroalkenyl, heteroalkynyl, heteroaryl, and aryl (all as defined herein); and R" is H or an optionally substituted group selected from alkyl, alkenyl, alkynyl, heteroalkyl, heteroalkenyl, heteralkynyl, heteroaryl, and aryl (all as defined herein). Alternatively, a substituted alkyl group may be a perfluoroalkyl group. In certain cases, when at least one of the substituents on alkyl group is oxo, the oxo group is not bonded to the carbon atom bonded to the parent molecular group.

The term "alkylene," as used herein, refers to a saturated straight-chain or branched-chain divalent substituent, containing only C and H when unsubstituted. Alkylene group may contain, unless otherwise specified, 1, 2, 3, 4, 5, or 6 carbon atoms, excluding the carbon atoms of any substituents, if present. Non-limiting examples of alkylene group include methylene, ethane-1,2-diyl, ethane-1,1-diyl, propane-1,3-diyl, propane-1,2-diyl, propane-1,1-diyl, propane-2,2-diyl, butane-1,4-diyl, butane-1,3-diyl, butane-1,2-diyl, butane-1,1-diyl, and butane-2,2-diyl, butane-2,3-diyl. Alkylene group may be unsubstituted or substituted (*e.g*., optionally substituted alkylene) with 1, 2, 3, 4, 5, or 6 substituents independently selected from the group consisting of: halo (*e.g.,* F, Cl, Br, or I), CN, NO₂, CF₃, OCF₃, COOR', CONR'₂, OR', SR', SOR', SO₂R', NR'₂, NR'(CO)R', NR'C(O)OR', NR'C(O)NR'₂, NR'SO₂NR'₂, NR'SO₂R', oxo (=O), or oximido (=NOR"), where each R' is, independently, H or an optionally substituted group selected from alkyl, alkenyl, alkynyl, cycloalkyl, cycloalkenyl, cycloalkynyl, heteroalkyl, heteroalkenyl, heteroalkynyl, heteroaryl, and aryl (all as defined herein); and R" is H or an optionally substituted group selected from alkyl, alkenyl, alkynyl, heteroalkyl, heteroalkenyl, heteralkynyl, heteroaryl, and aryl (all as defined herein). Alternatively, a substituted alkylene group may be a perfluoroalkylene group.

The term "alkylsulfinyl" refers to a group having the structure alkyl-S(O)-, in which alkyl is as described herein. Alkylsulfinyl may be unsubstituted or substituted (*e.g*., optionally substituted alkylsulfinyl) as described for alkyl.

The term "alkylsulfonyl" refers to a group having the structure alkyl-S(O)₂-, in which alkyl is as described herein. Alkylsulfonyl may be unsubstituted or substituted (*e.g*., optionally substituted alkylsulfonyl) as described for alkyl.

The term "alkynyl," as used herein, refers to a straight-chain or branched-chain monovalent substituent including one or two carbon-carbon triple bonds and containing only C and H when unsubstituted. Alkynyl group may contain, unless otherwise specified, 2, 3, 4, 5, or 6 carbon atoms, excluding the carbon atoms of any substituents, if present. Non-limiting examples of alkynyl groups include ethynyl, prop-1-ynyl, prop-2-ynyl, buty-1-nyl, but-2-ynyl, but-3-ynyl, 1-methylprop-2-ynyl, and the like. Alkynyl may be unsubstituted or substituted (*e*.*g*., optionally substituted alkynyl) as described for alkyl.

The term "alkynylene," as used herein, refers to a straight-chain or branched-chain divalent substituent including one or two carbon-carbon triple bonds and containing only C and H when unsubstituted. Alkynylene group may contain, unless otherwise specified, 2, 3, 4, 5, or 6 carbon atoms, excluding the carbon atoms of any substituents, if present. Non-limiting examples of alkenylene groups include ethyn-1,2-diyl; prop-1-yn-1,3-diyl; prop-2-yn-1,1-diyl; but-1-yn-1,3-diyl; but-1-yn-1,4-diyl; but-2-yn-1,1-diyl; but-2-yn-1,4-diyl; but-3-yn-1,1-diyl; but-3-yn-1,2-diyl; but-3-yn-2,2-diyl; and buta-1,3-diyn-1,4-diyl. Alkenylene may be unsubstituted or substituted (*e.g*., optionally substituted alkenylene) as described for alkylene.

The term "amido," as used herein, refers to a group having a structure - N(R^{N1})R^{N2}, in which R^{N1} is -H, -OH, -N(R^{N3})₂, -C(O)R^{N4}, -SO₂OR^{N4}, -SO₂R^{N4}, -SOR^{N4}, alkyl, alkenyl, alkynyl, alkoxy, aryl, arylalkyl, cycloalkyl, cycloalkylalkyl, heterocyclyl (*e.g*., heteroaryl), or heterocyclylalkyl (*e.g*., heteroarylalkyl); R^{N2} is -C(O)R^{N5}, SO₂OR^{N5}, SO₂R^{N5}, or SOR^{N5}; or R^{N1} and R^{N5} combine to form a 5-, 6-, 7-, or 8-membered ring. R^{N3} is H, alkyl, aryl, arylalkyl, cycloalkyl, cycloalkylalkyl, heterocyclyl (*e.g*., heteroaryl), or heterocyclylalkyl (*e.g*., heteroarylalkyl); each of R^{N4} and R^{N5} is, independently, alkyl, alkenyl, alkynyl, alkoxy, aryl, arylalkyl, cycloalkyl, cycloalkylalkyl, heterocyclyl (*e.g*., heteroaryl), or heterocyclylalkyl (*e.g*., heteroarylalkyl). In a preferred case, R^{N1} is H. Amido may be unsubstituted, when R^{N1} is H and the group in R^{N2} is unsubstituted (*e.g.*, R^{N3} is H, unsubstituted alkyl, unsubstituted aryl, unsubstituted arylalkyl, unsubstituted cycloalkyl, unsubstituted cycloalkylalkyl, unsubstituted heterocyclyl (*e.g.*, unsubstituted heteroaryl), or unsubstituted heterocyclylalkyl (*e.g*., unsubstituted heteroarylalkyl); or each of R^{N4} and R^{N5} is unsubstituted alkyl, unsubstituted alkenyl, unsubstituted alkynyl, unsubstituted alkoxy, unsubstituted aryl, unsubstituted arylalkyl, unsubstituted cycloalkyl, unsubstituted cycloalkylalkyl, unsubstituted heterocyclyl (*e.g.,* unsubstituted heteroaryl), or unsubstituted heterocyclylalkyl (*e.g.,* unsubstituted heteroarylalkyl)). Alternatively, amido may be substituted, when at least one of the groups listed under R^{N3}, R^{N4}, or R^{N5} is substituted, and/or when R^{N1} is not H.

The term "amino," as used herein, represents -N(R^{N1})₂, wherein each R^{N1} is, independently, H, OH, NO₂, N(R^{N2})₂, an *N*-protecting group, alkyl, alkenyl, alkynyl, alkoxy, aryl, arylalkyl, cycloalkyl, cycloalkylalkyl, heterocyclyl (*e.g*., heteroaryl), heterocyclylalkyl (*e.g.,* heteroarylalkyl), or two R^{N1} combine to form a heterocyclyl or an N-protecting group, and wherein each R^{N2} is, independently, H, alkyl, or aryl. Amino may be unsubstituted, when each R^{N1} is H, or substituted, when at least one R^{N1} is not H (*e.g.,* optionally substituted amino). In a preferred case, amino is -NH₂ or -NHR^{N1}, wherein R^{N1} is, independently, OH, NO₂, NH₂, NR^{N2}₂, SO₂OR^{N2}, SO₂R^{N2}, SOR^{N2}, alkyl, or aryl, and each R^{N2} can be H, alkyl, or aryl.

The terms "aromatic moiety" and "aryl," as used herein, refer to a carbocyclic monovalent group (monocyclic or fused ring bicyclic), in which the carbocycle satisfies Hückel's rule (4n+2 electrons in a single π system) and has the characteristics of aromatic stabilization relative to a hypothetical molecule not having aromatic stabilization (*e.g.*, benzene as compared to cyclohexatriene). Aryl may contain 6-10 carbons, excluding the carbon atoms of any substituents, if present. Non-limiting examples of monocyclic and fused bicyclic aromatic moieties include phenyl and naphthyl, respectively. Aryl may be unsubstituted or substituted as defined herein. The term "arylene" refers to an aryl group, as described herein, except in that arylene is a divalent substituent. Arylene may be unsubstituted or substituted as defined herein.

The term "arylalkyl," as used herein, represents a chemical substituent (aryl)-(alkylene)-, in which each of aryl and alkylene group is as described herein. Arylalkyl group may be unsubstituted or substituted (*e.g*., optionally substituted C₆-C₁₀ aryl C₁-C₆ alkyl). A non-limiting example of arylalkyl is phenylmethyl, commonly referred to as benzyl. Arylalkenyl (*e.g.,* C₆-C₁₀ aryl C₂-C₆ alkenyl) and arylalkynyl (*e.g.,* C₆-C₁₀ aryl C₂-C₆ alkynyl) are similarly defined as having the general structure of (aryl)-(alkenylene)- and (aryl)-(alkynylene)-, respectively. Arylheteroalkyl, arylheteroalkenyl, and arylheteroalkynyl are similarly defined as having the structure (aryl)-(heteroalkylene)-, (aryl)-(heteroalkenylene)-, and (aryl)-(heteroalkynylene)-, respectively. Similarly, other groups can be defined through the combination of the term defining a group with "alkyl." For example, "heteroarylalkyl" is a chemical substitutent having the general structure (heteroaryl)-(alkylene)-, which may be unsubstituted or substituted (*e.g*., optionally substituted C₁-C₉ heteroaryl C₁-C₆ alkyl) according to the respective definitions of each portion of heteroarylalkyl group. Each of the groups may be unsubstituted or substituted (*e.g*., optionally substituted). The substituents for aryl or heteroaryl portion are those described for aromatic groups. The substitutents for alkyl, heteroalkyl, alkenyl, heteroalkenyl, alkynyl, or heteroalkynyl portion are those described in the respective definitions of these groups.

The term "aryloyl," as used herein, refers to a group having the structure (C₆-C₁₀ aryl)-C(O)-. Aryloyl may be unsubstituted or substituted according to the definition of an aryl group (*e.g*., optionally substituted aryloyl). A typical example of aryloyl group is benzoyl group. Similarly, the term "heteroaryloyl," as used herein, refers to a group having the structure (C₁-C₉ heteroaryl)-C(O)-. Heteroaryloyl may be unsubstituted or substituted (*e.g.*, optionally substituted heteroaryloyl) as described for heteroaryl.

The term "aryloxy," as used herein, refers to a carbocyclic aromatic system linked to another residue through an oxygen atom, *e.g.,* (C₆-C₁₀ aryl)-O-. Aryloxy group may be unsubstituted or substituted (*e.g.*, optionally substituted aryl) as described for the aromatic groups. A typical example of an aryloxy is phenoxy (*e.g.*, optionally substituted phenoxy).

The term "aryloyloxy," as used herein, refers to a group having the structure (C₆-C₁₀ aryl)-C(O)-O-. Aryloyloxy may be unsubstituted or substituted according to the definition of an aryl group (*e.g*., optionally substituted aryloyloxy). A typical example of the aryloyloxy group is benzoate. Similarly, the term "heteroaryloyloxy," as used herein, refers to a group having the structure (C₁-C₉ hetereoaryl)-C(O)-O-. Heteroaryloyloxy may be unsubstituted or substituted (*e.g*., optionally substituted heteroaryloyloxy) as described for heteroaryl.

The term "arylsulfinyl" refers to a group having the structure (C₆-C₁₀ aryl)-S(O)-. Arylsulfinyl group may be unsubstituted or substituted as described herein (*e.g.,* optionally substituted arylsulfinyl). A non-limiting example of arylsulfinyl is phenylsulfinyl.

The term "arylsulfonyl" refers to a group having the structure (C₆-C₁₀ aryl)-S(O)₂-. Arylsulfonyl group may be unsubstituted or substituted as described herein (*e.g.,* optionally substituted arylsulfonyl). A non-limiting example of arylsulfonyl is phenylsulfonyl.

The term "arylthio" refers to a group having the structure (C₆-C₁₀ aryl)-S-. Arylthio group may be unsubstituted or substituted as described herein (*e.g*., optionally substituted arylthio). A non-limiting example of arylthio is phenylthio.

The term "carbocyclic," as used herein, represents an optionally substituted C₃-₁₂ monocyclic, bicyclic, or tricyclic structure in which the rings, which may be aromatic or non-aromatic, are formed by carbon atoms. Carbocyclic structures include cycloalkyl, cycloalkenyl, cycloalkynyl, and aryl groups.

The term "carbonyl," as used herein, refers to a divalent group consisting of a C=O, in which the two valences are on the carbon atom. This term can be used to define other groups having the general structure R-C(O)-. Thus, in alkoxycarbonyl group, R is alkoxy; in aryloxycarbonyl group, R is aryloxy, in aminocarbonyl group, R is amino; in heteroaryloxycarbonyl group, R is heteroaryloxy; in heterocyclyloxycarbonyl group, R is heterocyclyloxy; or in hydroxycarbonyl group, R is hydroxy. Each of the groups may be unsubstituted or substituted in accordance with the definition provided herein. For example, an alkoxycarbonyl group may be unsubstituted or substituted as defined for alkoxy group.

The terms "carboxamide" and "carboxylic acid amide," as used herein, refer to a group having the structure -CONR'R", where each R' and R" is selected, independently, from H, optionally substituted C₁₋₆ alkyl, optionally substituted C₃-C₁₀ cycloalkyl, optionally substituted C₁-C₉ heterocyclyl, optionally substituted C₆-C₁₀ aryl, optionally substituted C₁-C₉ heteroaryl, or R' and R" combine to form an optionally substituted heterocyclyl. Carboxamide may be unsubstituted, when the R' group and the R" group are unsubstituted, or substituted, when at least one of R' and R" is a substituted group as defined herein. Accordingly, optionally substituted carboxamide is a carboxamide that may be unsubstituted or substituted.

The terms "carboxylic acid ester" and "ester," as used herein, refer to a group having the structure -CO₂R', where R' is selected from optionally substituted alkyl, optionally substituted cycloalkyl, optionally substituted heterocyclyl, optionally substituted aryl, or optionally substituted heteroaryl. Ester may be unsubstituted, when the R' group is an unsubstituted group, or substituted, when R' group is a substituted group as defined herein. Accordingly, optionally substituted ester is an ester that may be unsubstituted or substituted.

By "cyano" is meant a group having the structure -CN.

The term "cycloalkenyl," as used herein, refers to a non-aromatic carbocyclic group having one, two, or three carbon-carbon double bonds and having from three to ten carbons (*e.g*., a C₃-C₁₀ cycloalkylene), unless otherwise specified. Non-limiting examples of cycloalkenyl include cycloprop-1-enyl, cycloprop-2-enyl, cyclobut-1-enyl, cyclobut-1-enyl, cyclobut-2-enyl, cyclopent-1-enyl, cyclopent-2-enyl, cyclopent-3-enyl, norbornen-1-yl, norbornen-2-yl, norbornen-5-yl, and norbornen-7-yl. The cycloalkenyl group may be unsubstituted or substituted (*e.g*., optionally substituted cycloalkenyl) as described for cycloalkyl.

The term "cycloalkenylene," as used herein, refers to a divalent non-aromatic carbocyclic group having one, two, or three carbon-carbon double bonds and having from three to ten carbons (*e.g.,* C₃-C₁₀ cycloalkenylene), unless otherwise specified. Non-limiting examples of the cycloalkenylene include cycloprop-1-en-1,2-diyl; cycloprop-2-en-1,1-diyl; cycloprop-2-en-1,2-diyl; cyclobut-1-en-1,2-diyl; cyclobut-1-en-1,3-diyl; cyclobut-1-en-1,4-diyl; cyclobut-2-en-1,1-diyl; cyclobut-2-en-1,4-diyl; cyclopent-1-en-1,2-diyl; cyclopent-1-en-1,3-diyl; cyclopent-1-en-1,4-diyl; cyclopent-1-en-1,5-diyl; cyclopent-2-en-1,1-diyl; cyclopent-2-en-1,4-diyl; cyclopent-2-en-1,5-diyl; cyclopent-3-en-1,1-diyl;cyclopent-1,3-dien-1,2-diyl; cyclopent-1,3-dien-1,3-diyl; cyclopent-1,3-dien-1,4-diyl; cyclopent-1,3-dien-1,5-diyl; cyclopent-1,3-dien-5,5-diyl; norbornadien-1,2-diyl; norbornadien-1,3-diyl; norbornadien-1,4-diyl; norbornadien-1,7-diyl; norbornadien-2,3-diyl; norbornadien-2,5-diyl; norbornadien-2,6-diyl; norbornadien-2,7-diyl; and norbornadien-7,7-diyl. The cycloalkenylene may be unsubstituted or substituted (*e.g*., optionally substituted cycloalkenylene) as described for cycloalkyl.

The term "cycloalkyl," as used herein, refers to a monovalent carbocyclic group having from three to ten carbons (*e.g.,* a C₃-C₁₀ cycloalkyl), unless otherwise specified. Cycloalkyl groups may be monocyclic or bicyclic. Bicyclic cycloalkyl groups may be of bicyclo[p.q.0]alkyl type, in which each of p and q is, independently, 1, 2, 3, 4, 5, 6, or 7, provided that the sum of p and q is 2, 3, 4, 5, 6, 7, or 8. Alternatively, bicyclic cycloalkyl groups may include bridged cycloalkyl structures, *e.g*., bicyclo[p.q.r]alkyl, in which r is 1, 2, or 3, each of p and q is, independently, 1, 2, 3, 4, 5, or 6, provided that the sum of p, q, and r is 3, 4, 5, 6, 7, or 8. The cycloalkyl group may be a spirocyclic group, *e.g.*, spiro[p.q]alkyl, in which each of p and q is, independently, 2, 3, 4, 5, 6, or 7, provided that the sum of p and q is 4, 5, 6, 7, 8, or 9. Non-limiting examples of cycloalkyl include cyclopropyl, cyclobutyl, cyclopentyl, cyclohexyl, cycloheptyl, 1-bicyclo[2.2.1]heptyl, 2-bicyclo[2.2.1]heptyl, 5-bicyclo[2.2.1]heptyl, 7-bicyclo[2.2.1]heptyl, and decalinyl. The cycloalkyl group may be unsubstituted or substituted (*e.g*., optionally substituted cycloalkyl) with 1, 2, 3, 4, 5, or 6 substituents independently selected from the group consisting of: alkyl, alkenyl, alkynyl, heteroalkyl, heteroalkenyl, heteroalkynyl, aryl, arylalkyl, heteroaryl, halo *(e.g.,* F, Cl, Br, or I), CN, NO₂, CF₃, OCF₃, COOR', CONR'₂, OR', SR', SOR', SO₂R', NR'₂, NR'(CO)R',NR'C(O)OR', NR'C(O)NR'₂, NR'SO₂NR'₂, NR'SO₂R', oxo (=O), or oximido (=NOR"), where each R' is, independently, H or an optionally substituted group selected from alkyl, alkenyl, alkynyl, cycloalkyl, cycloalkenyl, cycloalkynyl, heteroalkyl, heteroalkenyl, heteroalkynyl, heteroaryl, and aryl (all as defined herein); and R" is H or an optionally substituted group selected from alkyl, alkenyl, alkynyl, heteroalkyl, heteroalkenyl, heteralkynyl, heteroaryl, and aryl (all as defined herein). Alternatively, a substituted cycloalkyl group may be a perfluorocycloalkyl group.

The term "cycloalkylene," as used herein, refers to a divalent carbocyclic group having from three to ten carbons (*e.g.,* C₃-C₁₀ cycloalkyl), unless otherwise specified. Non-limiting examples of cycloalkylene include cyclopropane-1,1-diyl; cyclopropane-1,2-diyl; cyclobutane-1,1-diyl; cyclobutane-1,2-diyl; cyclobutane-1,3-diyl; bicyclo[2.2.1]hepta-1,2-diyl; bicyclo[2.2.1]hepta-1,3-diyl; bicyclo[2.2.1]hepta-1,4-diyl; bicyclo[2.2.1]hepta-1,7-diyl; bicyclo[2.2.1]hepta-2,2-diyl; bicyclo[2.2.1]hepta-2,3-diyl; bicyclo[2.2.1]hepta-2,7-diyl; decalin-1,2-diyl; decalin-1,3-diyl; decalin-1,4-diyl; decalin-1,5-diyl; decalin-1,6-diyl; decalin-2,2-diyl; decalin-2,3-diyl; decalin-2,4-diyl; and decalin-2,5-diyl. The cycloalkylene group may be unsubstituted or substituted (*e.g*., optionally substituted cycloalkylene) as described for cycloalkyl.

The term "cycloalkynyl," as used herein, refers to a monovalent carbocyclic group having one or two non-contiguous carbon-carbon triple bonds and having from eight to ten carbons (*e.g.,* a C₈-C₁₀ cycloalkyl), unless otherwise specified. Non-limiting examples of cycloalkynyl include cyclooctynyl, cyclononynyl, cyclodecynyl, and cyclodecadiynyl. The cycloalkynyl group may be unsubstituted or substituted (*e.g*., optionally substituted cycloalkynyl) as described for cycloalkyl.

Halo may be any halogen atom, especially F, Cl, Br, or **I,** and more particularly it is fluoro or chloro.

The term "haloalkyl," as used herein, represents an alkyl group, as defined herein, substituted by a halogen group (*i.e.,* F, Cl, Br, or I). A haloalkyl may be substituted with one, two, three, or, in the case of alkyl groups of two carbons or more, four halogens. Haloalkyl groups include perfluoroalkyls. In some cases, the haloalkyl group can be further substituted with 1, 2, 3, or 4 substituent groups as described herein for alkyl groups.

The term "heteroalkenyl," as used herein refers to an alkenyl group in which alkenyl chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkenyl groups includes more than two contiguous oxygen atoms. The heteroalkenyl group may be unsubstituted or substituted (*e.g*., optionally substituted heteroalkenyl). When the heteroalkenyl group is substituted and the substituent is bonded to the heteroatom, the substituent is selected accordingly. The substituent bonded to the heteroatom, valency permitting, is selected from the group consisting of: alkyl, alkanoyl, alkenyl, alkenoyl, alkynyl, alkynoyl, cycloalkyl, cycloalkanoyl, cycloalkenyl, cycloalkenoyl, cycloalkynyl, cycloalkynoyl, aryl, aryloyl, heteroaryl, heteroaryloyl, heterocyclyl, heterocycloyl, amino, aminocarbonyl, alkoxycarbonyl, aryloxycarbonyl, heteroaryloxycarbonyl, and heterocyclyloxycarbonyl. When the heteroalkenyl group is substituted and the substituent is bonded to carbon, the substituent is selected from those described for alkyl, provided that the substituent on the carbon atom bonded to the heteroatom is not halo. In some cases, the heteroalkenyl group has C at the terminus that attaches to other groups. In some cases, the heteroatom is O or N.

The term "heteroalkenylene," as used herein refers to an alkenylene group in which alkenylene chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkenylene groups includes more than two contiguous oxygen atoms. The heteroalkenylene group may be unsubstituted or substituted (*e.g*., optionally substituted heteroalkenylene). When the heteroalkenylene group is substituted and the substituent is bonded to the heteroatom, the substituent is selected accordingly. The substituent bonded to the heteroatom, valency permitting, is selected from the group consisting of: alkyl, alkanoyl, alkenyl, alkenoyl, alkynyl, alkynoyl, cycloalkyl, cycloalkanoyl, cycloalkenyl, cycloalkenoyl, cycloalkynyl, cycloalkynoyl, aryl, aryloyl, heteroaryl, heteroaryloyl, heterocyclyl, heterocycloyl, amino, aminocarbonyl, alkoxycarbonyl, aryloxycarbonyl, heteroaryloxycarbonyl, and heterocyclyloxycarbonyl. When the heteroalkenylene group is substituted and the substituent is bonded to carbon, the substituent is selected from those described for alkyl, provided that the substituent on the carbon atom bonded to the heteroatom is not halo. In some cases, the heteroalkenylene group has C at each terminus that attaches to other groups. In some cases, the heteroatom is O or N.

The term "heteroalkyl," as used herein refers to an alkyl group in which alkyl chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkyl groups includes more than two contiguous oxygen atoms. The heteroalkyl group may be unsubstituted or substituted (*e.g.*, optionally substituted heteroalkyl). When the heteroalkyl group is substituted and the substituent is bonded to the heteroatom, the substituent is selected accordingly. The substituent bonded to the heteroatom, valency permitting, is selected from the group consisting of: alkyl, alkanoyl, alkenyl, alkenoyl, alkynyl, alkynoyl, cycloalkyl, cycloalkanoyl, cycloalkenyl, cycloalkenoyl, cycloalkynyl, cycloalkynoyl, aryl, aryloyl, heteroaryl, heteroaryloyl, heterocyclyl, heterocycloyl, amino, aminocarbonyl, alkoxycarbonyl, aryloxycarbonyl, heteroaryloxycarbonyl, and heterocyclyloxycarbonyl. When the heteroalkyl group is substituted and the substituent is bonded to carbon, the substituent is selected from those described for alkyl, provided that the substituent on the carbon atom bonded to the heteroatom is not halo. In some cases, the heteroalkyl group has C at the terminus that attaches to another group. In some cases, the heteroatom is O or N.

The term "heteroalkylene," as used herein refers to an alkylene group in which alkylene chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkylene groups includes more than two contiguous oxygen atoms. The heteroalkylene group may be unsubstituted or substituted (*e.g.,* optionally substituted heteroalkylene). When the heteroalkylene group is substituted and the substituent is bonded to the heteroatom, the substituent is selected accordingly. The substituent bonded to the heteroatom, valency permitting, is selected from the group consisting of: alkyl, alkanoyl, alkenyl, alkenoyl, alkynyl, alkynoyl, cycloalkyl, cycloalkanoyl, cycloalkenyl, cycloalkenoyl, cycloalkynyl, cycloalkynoyl, aryl, aryloyl, heteroaryl, heteroaryloyl, heterocyclyl, heterocycloyl, amino, aminocarbonyl, alkoxycarbonyl, aryloxycarbonyl, heteroaryloxycarbonyl, and heterocyclyloxycarbonyl. When the heteroalkylene group is substituted and the substituent is bonded to carbon, the substituent is selected from those described for alkylene, provided that the substituent on the carbon atom bonded to the heteroatom is not halo. In some cases, the heteroalkylene group has C at each terminus that attaches to other groups. In some cases, the heteroatom is O or N.

The term "heteroalkynyl," as used herein, refers to an alkynyl group in which alkynyl chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkynyl groups includes more than two contiguous oxygen atoms. The heteroalkynyl group may be unsubstituted or substituted (*e.g*., optionally substituted heteroalkynyl) as described for heteroalkenyl.

The term "heteroalkynylene," as used herein refers to an alkynylene group in which alkynylene chain is interrupted once by one, two, or three heteroatoms; twice, each time, independently, by one, two, or three heteroatoms; three times, each time, independently, by one, two, or three heteroatoms; or four times, each time, independently, by one, two, or three heteroatoms. Each heteroatom is, independently, O, N, or S. None of the heteroalkynylene groups includes more than two contiguous oxygen atoms. The heteroalkynylene group may be unsubstituted or substituted (*e.g*., optionally substituted heteroalkynylene). The heteroalkynylene group may be unsubstituted or substituted (*e.g*., optionally substituted heteroalkynylene) as described for heteroalkenylene.

The terms "heteroaromatic moiety" and "heteroaryl," as used herein, refer to heterocyclic structure (monocyclic or fused bicyclic) satisfying Hückel's rule (4n+2 electrons in a single π system) and thus having the characteristics of aromatic stabilization. Excluding the heteroatoms of any substituents, if present, heteroaryl group contains one, two, three, or four heteroatoms selected from the group consisting of O, S, and N. Heteroaryl group contains 1, 2, 3, 4, 5, 6, 7, 8, or 9 carbon atoms, excluding the carbon atoms of any substituents, if present. The inclusion of a heteroatom permits inclusion of 5-membered rings to be considered aromatic as well as 6-membered rings. Thus, non-limiting examples of heteroaromatic moieties include pyridyl, pyrimidyl, indolyl, benzimidazolyl, benzotriazolyl, isoquinolyl, quinolyl, benzothiazolyl, benzofuranyl, thienyl, furyl, pyrrolyl, thiazolyl, oxazolyl, isoxazolyl, benzoxazolyl, benzoisoxazolyl, and imidazolyl. Because tautomers are theoretically possible, phthalimido is also considered aromatic. Typically, heteroaryl ring systems contain 5-12 ring member atoms. For example, heteroaryl group can be a five- to twelve-membered ring system. In some cases, the heteroaromatic moiety is a 6-membered aromatic ring system containing 1-2 nitrogen atoms. In some cases, heteroaryl group is an optionally substituted pyridyl, indolyl, pyrimidyl, pyridazinyl, benzothiazolyl, benzimidazolyl, pyrazolyl, imidazolyl, isoxazolyl, thiazolyl, benzothiazolyl, or indolyl. In certain cases, the heteroaromatic moiety is pyridyl or pyrimidyl. The term "heteroarylene" refers to a heteroaryl group, as described herein, except in that heteroarylene is a divalent substituent.

The term "heteroarylalkylthio," as used herein, represents a chemical substituent of formula -SR, where R is a heteroarylalkyl group. In some cases, heteroarylalkyl group can be further substituted with 1, 2, 3, or 4 substituent groups as described herein.

The term "heteroarylsulfinyl" refers to a group having the structure heteroaryl-S(O)-, in which heteroaryl is as described herein. Heteroarylsulfinyl group may be unsubstituted or substituted as described herein.

The term "heteroarylsulfonyl" refers to a group having the structure heteroaryl-S(O)₂-, in which heteroaryl is as described herein. Heteroarylsulfonyl group may be unsubstituted or substituted as described herein.

The term "heteroarylthio" refers to a group having the structure heteroaryl-S-, in which heteroaryl is as described herein. Heteroarylthio group may be unsubstituted or substituted as described herein.

The term "heterocyclyl," as used herein represents cyclic heteroalkyl or heteroalkenyl that is, *e.g.,* a 3-, 4-, 5-, 6-, or 7-membered ring, unless otherwise specified. Excluding the heteroatoms of any substituents, if present, heterocyclyl group contains one, two, three, or four heteroatoms selected from the group consisting of O, S, and N. The heterocyclyl group contains, unless otherwise specified, 1, 2, 3, 4, 5, 6, 7, 8, or 9 carbon atoms (*e.g.,* C₁-C₉ heterocyclyl), excluding the carbon atoms of any substituents, if present. Sulfur may be included as divalent sulfur (-S-), tetravalent sulfur (-S(=O)-), or hexavalent sulfur (-S(=O)₂-). The 5-membered ring has zero to two double bonds, and the 6- and 7-membered rings have zero to three double bonds. The term "heterocyclyl" also represents a heterocyclic compound having a bridged multicyclic structure in which one or more carbons and/or heteroatoms bridges two non-adjacent members of a monocyclic ring, *e.g.,* a quinuclidinyl group. The term "heterocyclyl" includes bicyclic, tricyclic, and tetracyclic groups in which any of the above heterocyclic rings is fused to one, two, or three carbocyclic rings, *e.g.,* an aryl ring, a cyclohexane ring, a cyclohexene ring, a cyclopentane ring, a cyclopentene ring, or another monocyclic heterocyclic ring, such as indolyl, quinolyl, isoquinolyl, tetrahydroquinolyl, benzofuryl, benzothienyl and the like. Exemplary heterocycles include pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidiniyl, morpholinyl, thiomorpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, thiazolidinyl, isothiazolyl, isoindazoyl, triazolyl, tetrazolyl, oxadiazolyl, purinyl, thiadiazolyl (*e.g*., 1,3,4-thiadiazole), tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, dithiazolyl, benzofuranyl, benzothienyl and the like. Still other exemplary heterocyclyls include: 2,3,4,5-tetrahydro-2-oxo-oxazolyl; 2,3-dihydro-2-oxo-1H-imidazolyl; 2,3,4,5-tetrahydro-5-oxo-1H-pyrazolyl (*e.g.,* 2,3,4,5-tetrahydro-2-phenyl-5-oxo-1H-pyrazolyl); 2,3,4,5-tetrahydro-2,4-dioxo-1H-imidazolyl (*e.g*., 2,3,4,5-tetrahydro-2,4-dioxo-5-methyl-5-phenyl-1H-imidazolyl); 2,3-dihydro-2-thioxo-1,3,4-oxadiazolyl (*e.g*., 2,3-dihydro-2-thioxo-5-phenyl-1,3,4-oxadiazolyl); 4,5-dihydro-5-oxo-1*H-*triazolyl (*e.g.,* 4,5-dihydro-3-methyl-4-amino 5-oxo-1*H*-triazolyl); 1,2,3,4-tetrahydro-2,4-dioxopyridinyl (*e.g*., 1,2,3,4-tetrahydro-2,4-dioxo-3,3-diethylpyridinyl); 2,6-dioxo-piperidinyl (*e.g.,* 2,6-dioxo-3-ethyl-3-phenylpiperidinyl); 1,6-dihydro-6-oxopyridiminyl; 1,6-dihydro-4-oxopyrimidinyl (*e.g*., 2-(methylthio)-1,6-dihydro-4-oxo-5-methylpyrimidin-1-yl); 1,2,3,4-tetrahydro-2,4-dioxopyrimidinyl (*e.g*., 1,2,3,4-tetrahydro-2,4-dioxo-3-ethylpyrimidinyl); 1,6-dihydro-6-oxo-pyridazinyl (*e.g*., 1,6-dihydro-6-oxo-3-ethylpyridazinyl); 1,6-dihydro-6-oxo-1,2,4-triazinyl (*e.g*., 1,6-dihydro-5-isopropyl-6-oxo-1,2,4-triazinyl); 2,3-dihydro-2-oxo-1*H-*indolyl (*e.g*., 3,3-dimethyl-2,3-dihydro-2-oxo-1*H*-indolyl and 2,3-dihydro-2-oxo-3,3'-spiropropane-1*H*-indol-1-yl); 1,3-dihydro-1-oxo-2*H*-iso-indolyl; 1,3-dihydro-1,3-dioxo-2*H-*iso-indolyl; 1*H*-benzopyrazolyl (*e.g*., 1-(ethoxycarbonyl)- 1*H*-benzopyrazolyl); 2,3-dihydro-2-oxo-1*H*-benzimidazolyl (*e.g.,* 3-ethyl-2,3-dihydro-2-oxo-1*H*-benzimidazolyl); 2,3-dihydro-2-oxo-benzoxazolyl (*e.g*., 5-chloro-2,3-dihydro-2-oxo-benzoxazolyl); 2,3-dihydro-2-oxo-benzoxazolyl; 2-oxo-2H-benzopyranyl; 1,4-benzodioxanyl; 1,3-benzodioxanyl; 2,3-dihydro-3-oxo,4*H*-1,3-benzothiazinyl; 3,4-dihydro-4-oxo-3*H*-quinazolinyl (*e.g.,* 2-methyl-3,4-dihydro-4-oxo-3*H-*quinazolinyl); 1,2,3,4-tetrahydro-2,4-dioxo-3*H*-quinazolyl (*e.g.,* 1-ethyl-1,2,3,4-tetrahydro-2,4-dioxo-3*H*-quinazolyl); 1,2,3,6-tetrahydro-2,6-dioxo-7*H*-purinyl (*e.g*., 1,2,3,6-tetrahydro-1,3-dimethyl-2,6-dioxo-7 *H* -purinyl); 1,2,3,6-tetrahydro-2,6-dioxo-1 *H -* purinyl (*e.g.*, 1,2,3,6-tetrahydro-3,7-dimethyl-2,6-dioxo-1 *H* -purinyl); 2-oxobenz[*c*,*d*]indolyl; 1,1-dioxo-2H-naphth[1,8-*c*,*d*]isothiazolyl; and 1,8-naphthylenedicarboxamido. Heterocyclyl group may be unsubstituted or substituted (*e.g*., optionally substituted heterocyclyl). The term "heterocyclylene" refers to a heterocyclyl group, as described herein, except in that heterocyclylene is a divalent substituent.

The term "heterocyclyloxy," as used herein, refers to a group having the structure (C₁-C₉ heterocyclyl)-O-. Heterocyclyloxy may be unsubstituted or substituted (*e.g*., optionally substituted heterocyclyloxy) according to the definition of heterocyclyl.

The term "heterocyclyloyl," as used herein, refers to a group having the structure (C₁-C₉ heterocyclyl)-C(O). Heterocyclyloyl may be unsubstituted or substituted (*e.g.*, optionally substituted heterocyclyloyl) according to the definition of heterocyclyl.

The term "heterocyclyloyloxy," as used herein, refers to a group having the structure (C₁-C₉ heterocyclyl)-C(O)-O-. Heterocyclyloyloxy may be unsubstituted or substituted (*e.g.,* optionally substituted heterocyclyloyloxy) according to the definition of hetercyclyl.

The term "heterocyclylsulfinyl" refers to a group having the structure heterocyclyl-S(O)-, in which heterocyclyl is as described herein. Heterocyclylsulfinyl group may be unsubstituted or substituted as described herein.

The term "heterocyclylsulfonyl" refers to a group having the structure heterocyclyl-S(O)₂-, in which heterocyclyl is as described herein. Heterocyclylsulfonyl group may be unsubstituted or substituted as described herein.

The term "heterocyclylthio" refers to a group having the structure heterocyclylS-, in which heterocyclyl is as described herein. Heterocyclylthio group may be unsubstituted or substituted as described herein.

The term "hydroxy," as used herein, represents an -OH group.

The term "hydroxyalkyl," as used herein, represents an alkyl group, as defined herein, substituted by one to three hydroxy groups, with the proviso that no more than one hydroxy group may be attached to a single carbon atom of alkyl group, and is exemplified by hydroxymethyl, dihydroxypropyl, and the like.

The term "nitro," as used herein refers to -NO₂ group.

The term "*n*-membered ring," in which *n* is 5, 6, 7, or 8, as used herein, refers to a carbocyclic or heterocyclic structure that may be aromatic or non-aromatic. When the *n-*membered ring is carbocyclic aromatic, it is subject to the definition for aromatic moiety. When the *n*-membered ring is carbocyclic non-aromatic, it is subject to the definition for cycloalkylene. When the *n*-membered ring is heterocyclic aromatic, it is subject to the definition for heteroarylene. When the *n*-membered ring is heterocyclic non-aromatic, it is subject to the definition for heterocyclylene. The *n*-membered ring may be unsubstituted or substituted (*e.g*., optionally substituted *n*-membered ring) according to the respective definition provided herein, unless otherwise specified. In some cases, the *n*-membered ring can be substituted with 1, 2, 3, 4, or 5 substitutents, each substituent being independently selected from the group consisting of H, halo, hydroxy, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀ arylthio, optionally substituted C₆-C₁₀ arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted sulfamoyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, and optionally substituted C₁-C₉ heterocyclyl C₂-C₆ alkynyl.

An "oxo" group is a divalent substituent consisting of oxygen atom, *e.g., =O.*

The term "pharmaceutically acceptable salt," as used herein, represents those salts which are, within the scope of sound medical judgment, suitable for use in contact with the tissues of humans and animals without undue toxicity, irritation, allergic response and the like and are commensurate with a reasonable benefit/risk ratio. Pharmaceutically acceptable salts are well known in the art. For example, S. M. Berge et al. describe pharmaceutically acceptable salts in detail in J. Pharm. Sci. 66:1-19, 1977. The salts can be prepared *in situ* during the final isolation and purification of the compounds of the disclosure or separately by reacting the free base group with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts and the like. Representative alkali or alkaline earth metal salts include sodium, lithium, potassium, calcium, magnesium and the like, as well as nontoxic ammonium, quaternary ammonium, and amine cations, including, but not limited to ammonium, tetramethylammonium, tetraethylammonium, methylammonium, dimethylammonium, trimethylammonium, triethylammonium, ethylammonium, and the like.

The term "protecting group," as used herein, represents a group intended to protect a functional group (*e.g.,* a hydroxy, an amino, or a carbonyl) from participating in one or more undesirable reactions during chemical synthesis (*e.g*., polynucleotide synthesis). The term "*O*-protecting group," as used herein, represents a group intended to protect an oxygen containing (*e.g*., phenol, hydroxyl or carbonyl) group from participating in one or more undesirable reactions during chemical synthesis. The term "*N*-protecting group," as used herein, represents a group intended to protect a nitrogen containing (*e.g*., an amino or hydrazine) group from participating in one or more undesirable reactions during chemical synthesis. Commonly used *O*- and *N*-protecting groups are disclosed in Greene, "Protective Groups in Organic Synthesis," 3rd Edition (John Wiley & Sons, New York, 1999). Exemplary *O*- and *N*-protecting groups include acyl, aryloyl, or carbamyl groups such as formyl, acetyl, propionyl, pivaloyl, *t*-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, *t*-butyldimethylsilyl, tri-*iso*-propylsilyloxymethyl, 4,4'-dimethoxytrityl, isobutyryl, phenoxyacetyl, 4-isopropylpehenoxyacetyl, dimethylformamidino, and 4-nitrobenzoyl.

Exemplary *O*-protecting groups for protecting carbonyl containing groups include, but are not limited to: acetals, acylals, 1,3-dithianes, 1,3-dioxanes, 1,3-dioxolanes, and 1,3-dithiolanes.

Other *O*-protecting groups include, but are not limited to: substituted alkyl, aryl, and aryl-alkylene ethers *(e.g.,* trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; t-butyl ether; p-chlorophenyl, p-methoxyphenyl, p-nitrophenyl, benzyl, p-methoxybenzyl, and nitrobenzyl); silyl ethers (*e.g.,* trimethylsilyl; triethylsilyl; triisopropylsilyl; dimethylisopropylsilyl; t-butyldimethylsilyl; t-butyldiphenylsilyl; tribenzylsilyl; triphenylsilyl; and diphenymethylsilyl); carbonates (*e.g*., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl).

Other *N-*protecting groups include, but are not limited to, chiral auxiliaries such as protected or unprotected D, L or D, L-amino acids such as alanine, leucine, phenylalanine, and the like; sulfonyl-containing groups such as benzenesulfonyl, p-toluenesulfonyl, and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyl oxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, aryl-alkylene groups such as benzyl, triphenylmethyl, benzyloxymethyl, and the like and silyl groups such as trimethylsilyl, and the like. Useful *N*-protecting groups are formyl, acetyl, benzoyl, pivaloyl, t-butylacetyl, alanyl, phenylsulfonyl, benzyl, t-butyloxycarbonyl (Boc), and benzyloxycarbonyl (Cbz).

The term "sulfamoyl," as used herein, refers to a group having the structure - SO₂-N(R^{N1})₂, wherein each R^{N1} is, independently, H, alkyl, alkenyl, alkynyl, aryl, arylalkyl, cycloalkyl, cycloalkylalkyl, heterocyclyl (*e.g.,* heteroaryl), heterocyclylalkyl (*e.g.,* heteroarylalkyl), or two R^{N1} combine to form a heterocyclyl. The sulfamoyl group may be unsubstituted, when each R^{N1} is H, or substituted, when at least one R^{N1} is not H (*e.g.,* optionally substituted sulfamoyl). In a preferred case, sulfamoyl is -SO₂NH₂ or -SO₂NHR^{N1}, wherein R^{N1} is, independently, alkyl, aryl, arylalkyl, cycloalkyl, cycloalkylalkyl, heterocyclyl (*e.g.,* heteroaryl), heterocyclylalkyl (*e.g.,* heteroarylalkyl).

The term "thioalkyl," as used herein, represents a chemical substituent of formula -SR, where R is an alkyl group. In some cases, alkyl group can be further substituted with 1, 2, 3, or 4 substituent groups as described herein.

The term "thiol" represents an -SH group.

Typical optional substituents on aromatic or heteroaromatic groups include independently halo (*e.g.,* F, Cl, Br, or I), optionally substituted alkyl, optionally substituted alkenyl, optionally substituted alkynyl, optionally substituted cycloalkyl, optionally substituted cycloalkenyl, optionally substituted cycloalkynyl, CN, NO₂, CF₃, OCF₃, COOR', CONR'₂, OR', SR', SOR', SO₂R', NR'₂, NR'(CO)R',NR'C(O)OR', NR'C(O)NR'₂, NR'SO₂NR'₂, or NR'SO₂R', wherein each R' is independently H or an optionally substituted group selected from alkyl, alkenyl, alkynyl, cycloalkyl, cycloalkenyl, cycloalkynyl, heteroalkyl, heteroalkenyl, heteroalkynyl, heteroaryl, and aryl (all as defined above); or the substituent may be an optionally substituted group selected from alkyl, alkenyl, alkynyl, cycloalkyl, cycloalkenyl, cycloalkynyl, heteroalkyl, heteroalkenyl, heteroalkynyl, aryl, heteroaryl, O-aryl, O-heteroaryl, and arylalkyl.

Unless otherwise specified, typical optional substituents on non-aromatic groups include independently halo (*e.g.,* F, Cl, Br, or I), CN, NO₂, CF₃, OCF₃, COOR', CONR'₂, OR', SR', SOR', SO₂R', NR'₂, NR'(CO)R', NR'C(O)OR', NR'C(O)NR'₂, NR'SO₂NR'₂, or NR'SO₂R', wherein each R' is independently H or an optionally substituted group selected from alkyl, alkenyl, alkynyl, cycloalkyl, cycloalkenyl, cycloalkynyl, heteroalkyl, heteroalkenyl, heteroalkynyl, heteroaryl, and aryl (all as defined above); or the substituent may be an optionally substituted group selected from alkyl, alkenyl, alkynyl, cycloalkyl, cycloalkenyl, cycloalkynyl, heteroalkyl, heteroalkenyl, heteroalkynyl, aryl, heteroaryl, O-aryl, O-heteroaryl, and arylalkyl. A non-aromatic group may also include a substituent selected from =O and =NOR' where R' is H or an optionally substituted group selected from alkyl, alkenyl, alkynyl, heteroalkyl, heteroalkenyl, heteralkynyl, heteroaryl, and aryl (all as defined above).

In general, a substituent group (*e.g.,* alkyl, alkenyl, alkynyl, or aryl (including all heteroforms defined above) may itself optionally be substituted by additional substituents. The nature of these substituents is similar to those recited with regard to the substituents on the basic structures above. Thus, where an case of a substituent is alkyl, this alkyl may optionally be substituted by the remaining substituents listed as substituents where this makes chemical sense, and where this does not undermine the size limit of alkyl per se; *e.g.,* alkyl substituted by alkyl or by alkenyl would simply extend the upper limit of carbon atoms for these cases, and is not included. However, alkyl substituted by aryl, amino, halo and the like would be included. For example, where a group is substituted, the group may be substituted with 1, 2, 3, 4, 5, or 6 substituents. Optional substituents include, but are not limited to: C1-C6 alkyl or heteroalkyl, C2-C6 alkenyl or heteroalkenyl, C2-C6 alkynyl or heteroalkynyl, halogen; aryl, heteroaryl, azido(-N₃), nitro (-NO₂), cyano (-CN), acyloxy(-OC(=O)R'), acyl (-C(=O)R'), alkoxy (-OR'), amido (-NR'C(=O)R"), carboxamide (*e.g*., -C(=O)NRR'), amino (-NRR'), carboxylic acid (-CO₂H), carboxylic ester (-CO₂R'), carbamoyl (-OC(=O)NR'R" or - NRC(=O)OR'), hydroxy (-OH), isocyano (-NC), sulfonate (-S(=O)₂OR), sulfonamide (-S(=O)₂NRR' or -NRS(=O)₂R'), or sulfonyl (-S(=O)₂R), where each R or R' is selected, independently, from H, C1-C6 alkyl or heteroalkyl, C2-C6 alkenyl or heteroalkenyl, C2-C6 alkynyl or heteroalkynyl, aryl, or heteroaryl. A substituted group may have, for example, 1, 2, 3, 4, 5, 6, 7, 8, or 9 substituents.

In some cases, the disclosure features moieties that are amino acid residues. The amino acid residue may be of a naturally occurring amino acid (*e.g.,* Ala, Arg, Asn, Asp, Cys, Gln, Glu, Gly, His, Ile, Leu, Lys, Met, Phe, Pro, Ser, Thr, Trp, Tyr, or Val), or the amino acid residue may be of a non-naturally occurring amino acid. A "non-naturally occurring amino acid" is an amino acid which is not naturally produced or found in a mammal. Examples of non-naturally occurring amino acids include D-amino acids; an amino acid having an acetylaminomethyl group attached to a sulfur atom of a cysteine; a pegylated amino acid; the omega amino acids of the formula NH₂(CH₂)ₙCOOH wherein n is 2-6, neutral nonpolar amino acids, such as sarcosine, t-butyl alanine, t-butyl glycine, N-methyl isoleucine, and norleucine; phenylglycine; citrulline; methionine sulfoxide; cysteic acid; ornithine; and hydroxyproline.

### GPR174 Inhibitory Antibodies

In certain cases, the GPR174 inhibitory compound is an antibody or antibody fragment that binds to GPR174. In certain cases, the GPR174 antibody or receptor-binding fragment binds to the epitope bound by any of the compounds identified herein. Such antibodies include polyclonal, monoclonal, and recombinant antibodies derived from any antibody-producing vertebrate and may be multi-specific, chimeric, humanized, anti-idiotype, and antibody fragments. Antibody fragments include Fab, Fab', F(ab)₂, F(ab')₂, Fv fragments, scFv fragments and single-chain antibodies.

GPR174 inhibitory antibodies can be produced using any methods known in the art. Polyclonal antibodies, for example, can be prepared by immunizing an animal with a GPR174 polypeptide or an immunogenic portion thereof. GPR174 inhibitory monoclonal antibodies encompasses not only intact monoclonal antibodies and full-length monoclonal antibodies, but also fragments thereof (such as Fab, Fab', F(ab')₂, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. It is not intended to be limited as regards the source of the antibody or the manner in which it is made (*e.g.,* by hybridoma, phage selection, recombinant expression, transgenic animals, etc.). The term includes whole immunoglobulins as well as the fragments etc. described above under the definition of "antibody".

GPR174 inhibitory monoclonal antibodies can be obtained from naive or immunized animals or B-lymphocyte cell lines derived therefrom using the well-known hybridoma technology or by screening populations of cultured or immortalized cells from these animals. The monoclonal antibodies can be prepared directly from selected hybridomas or can be made recombinantly. Humanized monoclonal antibodies are produced by transferring the non-human (*e.g*., mouse) complementarity determining regions (CDR), from the heavy and light variable domains of the mouse immunoglobulin into a human variable domain. Human monoclonal antibodies can also be obtained through the use of transgenic mice that have been engineered to produce specific human antibodies in response to antigenic challenge. Additionally, human monoclonal antibodies can be obtained through the screening of cultured human B lymphocytes or the screening of human antibody libraries displayed in various contexts including phage, yeast, or vertebrate cells. Humanized or fully human antibodies specific to human GPR101 comprised of IgG2 or IgG4 isotypes can be produced by one of several methods known to one of ordinary skilled in the art, as described in Vaughan et al., Nature Biotechnical 16:535-539, 1998.

GPR174 antibodies can be induced using GPR174 polypeptides (*e.g*., full-length human GPR174 set forth as SEQ ID NO:1) or using antigenic GPR174 epitope-bearing peptides (*e.g.,* a portion of the GPR174 polypeptide). Immunogenic peptides may be as small as five amino acid residues. For example, the GPR174 polypeptide including the entire amino acid sequence of SEQ ID NO:1 may be used to induce GPR174 antibodies useful in the method of the disclosure. Particular GPR174 domains known to be involved in receptor-ligand interactions may be expressed as recombinant polypeptides and used as antigens. In addition, peptides comprising a portion of at least 6 amino acids of the GPR174 polypeptide (SEQ ID NO:1) are also useful to induce GPR174 antibodies. The GPR174 peptides and polypeptides used to raise antibodies may be isolated as natural, recombinant, or synthetic polypeptides.

Antigens useful for inducing GPR174 antibodies also include fusion polypeptides, such as fusions of GPR174 or a portion thereof with an immunoglobulin polypeptide or with maltose-binding protein. The polypeptide immunogen may be a full-length molecule or a portion thereof. If the polypeptide portion is hapten-like, such portion may be advantageously joined or linked to a macromolecular carrier (such as keyhole limpet hemocyanin (KLH), bovine serum albumin (BSA), or tetanus toxoid) for immunization.

The GPR174 antigens may also include the polypeptide or a portion thereof presented within a lipid membrane or a detergent. As a G-protein-coupled receptor, GPR174 contains seven hydrophobic transmembrane domains, which render the protein difficult to dissolve in an aqueous environment. Numerous strategies to accommodate the hydrophobic nature of GPR174 may be adopted. For example, such strategies include immunizing an animal or screening an antibody library with hydrophilic subdomains of the receptor in aqueous solutions. Alternatively, immunogens comprising the full-length protein or subdomains containing hydrophobic segments may be solubilized in a detergent or may be presented to the animal or antibody library in the membrane of whole cells engineered to overexpress the protein, or in membrane preparations or exosomes derived from such cells, or in liposomes.

In some cases, the GPR174 inhibitory compound is a GPR174 monoclonal antibody. GPR174 monoclonal antibodies are highly specific, being directed against a single GPR174 epitope. As used herein, the modifier "monoclonal" indicates the character of the antibody as being obtained from a substantially homogenous population of antibodies and is not to be construed as requiring production of the antibody by any particular method. Monoclonal antibodies can be obtained using any technique that provides for the production of antibody molecules by continuous cell lines in culture, such as the hybridoma method described by Kohler et al., Nature 256:495, 1975, or they may be made by recombinant DNA methods (see, *e.g.,* U.S. Patent No. 4,816,567 to Cabilly). Monoclonal antibodies may also be isolated from phage antibody libraries using the techniques described in Clackson et al., Nature 352:624-8, 1991, and Marks et al., J. Mol. Biol. 222:581-97, 1991. Such antibodies can be of any immunoglobulin class including IgG, IgM, IgE, IgA, IgD, and any subclass thereof.

For example, monoclonal antibodies can be obtained by injecting a suitable mammal (*e.g*., a BALB/c mouse) with a composition comprising a GPR174 polypeptide or portion thereof. After a predetermined period of time, splenocytes are removed from the mouse and suspended in a cell culture medium. The splenocytes are then fused with an immortal cell line to form a hybridoma. The formed hybridomas are grown in cell culture and screened for their ability to produce a monoclonal antibody against GPR174. (See also Current Protocols in Immunology, Vol. 1., John Wiley & Sons, pages 2.5.1-2.6.7, 1991.)

A number of *ex vivo* display methods have also been developed for the discovery of human or nonhuman monoclonal antibodies. In such methods, polynucleotide antibody libraries are created by cloning a repertoire of natural or synthetic immunoglobulin heavy and light chains into vectors containing elements for expression in the host, such as a transcription enhancer and promoter, and a polyadenylation signal sequence, as well as a polynucleotide sequence encoding a domain such as a phage surface protein or a transmembrane domain to ensure expression of the antibody on the surface of the host. The antibody library is introduced into host cells by techniques such as transfection, transduction, or site-specific genomic targeting. Strategies such as the aforementioned have been used to display antibody libraries on phage (see, *e.g.,* Clackson et al., Nature 352:624-8, 1991; Marks et al., J Mol Biol 222:581-97, 1991), yeast (see *e.g.,* Barnard et al., J Ind Microbial Biotechnol 37:961-71, 2010), mammalian (see *e.g.,* Bowers et al., PNAS 108:20455-60, 2011) and chicken (see *e.g.,* Yabuki et al. PLoS One 7:e36032, 2012) cells. Monoclonal antibodies can be isolated and purified from hybridoma cultures by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, The Humana Press, Inc., Vol. 10, pages 79-104, 1992).

Once produced, polyclonal, monoclonal, or phage-derived antibodies are first tested for specific GPR174 binding. A variety of assays known to those skilled in the art may be utilized to detect antibodies that specifically bind to GPR174. Exemplary assays include Western blot or immunoprecipitation analysis by standard methods (*e.g.*, as described in Ausubel et al.), immunoelectrophoresis, enzyme-linked immune-sorbent assays, dot blots, inhibition or competition assays, and sandwich assays (as described in Harlow and Land, Antibodies: A Laboratory Manual, Cold Spring Harbor Laboratory Press, 1988). Once antibodies are identified that specifically bind to GPR174, the antibodies are tested for the ability to function as a GPR174 inhibitory compound in one of several assays such as, those described herein.

### Chimeric/humanized antibodies

GPR174 inhibitory monoclonal antibodies useful in the method of the invention include chimeric antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies (U.S. Patent No. 4,816,567, to Cabilly; and Morrison, S.L., et al., Proc. Nat'l Acad. Sci. USA 81:6851-6855, (1984)). One form of a chimeric antibody useful in the disclosure is a humanized monoclonal GPR174 antibody. Humanized forms of non-human (*e*.*g*., murine) antibodies are chimeric antibodies, which contain minimal sequence derived from non-human immunoglobulin. Humanized monoclonal antibodies are produced by transferring the non-human (*e.g.*, mouse) complementarity determining regions (CDR), from the heavy and light variable chains of the mouse immunoglobulin into a human variable domain. Typically, residues of human antibodies are then substituted in the framework regions of the non-human counterparts. Furthermore, humanized antibodies may comprise residues that are not found in the recipient antibody or in the donor antibody. These modifications are made to further refine antibody performance. In general, the humanized antibody will comprise substantially all of at least one, and typically two, variable domains in which all or substantially all of the hypervariable loops correspond to those of a non-human immunoglobulin and all or substantially all of the Fv framework regions are those of a human immunoglobulin sequence. The humanized antibody optionally also will comprise at least a portion of an immunoglobulin constant region (Fc), typically that of a human immunoglobulin. For further details, see Jones, P.T., et al., Nature 321:522-525, (1986); Reichmann, L., et al., Nature 332:323-329, (1988); and Presta, Curr. Op. Struct. Biol. 2:593-596, (1992).

The humanized antibodies useful in the disclosure include human monoclonal antibodies including at least a GPR174 binding CDR3 region. In addition, the Fc portions may be replaced so as to produce IgA or IgM as well as human IgG antibodies. Such humanized antibodies will have particular clinical utility because they will specifically recognize human GPR174 but will not evoke an immune response in humans against the antibody itself. Consequently, they are better suited for *in vivo* administration in humans, especially when repeated or long-term administration is necessary. Techniques for producing humanized monoclonal antibodies are also described, for example, by Jones, P.T., et al., Nature 321:522, (1986); Carter, P., et al., Proc. Nat'l. Acad. Sci. USA 89:4285, (1992); Sandhu, J.S., Crit. Rev. Biotech. 12:437, (1992); Singer, I.I., et al., J. Immun. 150:2844, (1993); Sudhir (ed.), Antibody Engineering Protocols, Humana Press, Inc., (1995); Kelley, "Engineering Therapeutic Antibodies," in Protein Engineering: Principles and Practice, Cleland et al. (eds.), John Wiley & Sons, Inc., pages 399-434, (1996); and by U.S. Patent No. 5,693,762, to Queen, 1997. In addition, there are commercial entities that will synthesize humanized antibodies from specific murine antibody regions, such as Protein Design Labs (Mountain View, CA).

### Recombinant antibodies

GPR174 inhibitory monoclonal antibodies can also be made using recombinant methods. For example, human antibodies can be made using human immunoglobulin expression libraries (available for example, from Stratagene, Corp., La Jolla, CA) to produce fragments of human antibodies (V_{H}, V_{L}, Fv, Factor D, Fab or F(ab')₂). These fragments are then used to construct whole human antibodies using techniques similar to those for producing chimeric antibodies.

### Immunoglobulin fragments

The GPR174 inhibitory compounds useful in the method of the disclosure encompass not only intact immunoglobulin molecules but also the well-known fragments including Fab, Fab', F(ab)₂, F(ab')₂ and Fv fragments, scFv fragments, diabodies, linear antibodies, single-chain antibody molecules and multi-specific (*e.g*., bispecific and trispecific) antibodies formed from antibody fragments. It is well known in the art that only a small portion of an antibody molecule, the paratope, is involved in the binding of the antibody to its epitope (see, *e.g.,* Clark, W.R., The Experimental Foundations of Modern Immunology, Wiley & Sons, Inc., NY, 1986). The pFc' and Fc regions of the antibody are effectors of the classical complement pathway but are not involved in antigen binding. An antibody from which the pFc' region has been enzymatically cleaved, or which has been produced without the pFc' region, is designated an F(ab')₂ fragment and retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, is designated a Fab fragment, and retains one of the antigen binding sites of an intact antibody molecule. Antibody fragments can be obtained by proteolytic hydrolysis, such as by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an Fc fragment directly. These methods are described, for example, U.S. Patent No. 4,331,647 to Goldenberg; Nisonoff, A., et al., Arch. Biochem. Biophys. 89:230, (1960); Porter, R.R., Biochem. J. 73:119, (1959); Edelman, et al., in Methods in Enzymology 1:422, Academic Press, (1967); and by Coligan at pages 2.8.1-2.8.10 and 2.10.-2.10.4.

### Single-chain antibody fragments

Alternatively, one can create single peptide chain binding molecules specific for GPR174 in which the heavy and light chain Fv regions are connected. The Fv fragments may be connected by a peptide linker to form a single-chain antigen binding protein (scFv). These single-chain antigen binding proteins are prepared by constructing a structural gene comprising DNA sequences encoding the V_{H} and V_{L} domains which are connected by an oligonucleotide. The structural gene is inserted into an expression vector, which is subsequently introduced into a host cell, such as *E. coli.* The recombinant host cells synthesize a single polypeptide chain with a linker peptide bridging the two V domains. Methods for producing scFvs are described for example, by Whitlow, et al., "Methods: A Companion to Methods in Enzymology" 2:97, (1991); Bird, et al., Science 242:423, (1988); U.S. Patent No. 4,946,778, to Ladner; Pack, P., et al., Bio/Technology 11: 1271, (1993).

As an illustrative example, a GPR174-specific scFv can be obtained by exposing lymphocytes to GPR174 polypeptide *in vitro* and selecting antibody display libraries in phage or similar vectors (for example, through the use of immobilized or labeled GPR174 protein or peptide). Genes encoding polypeptides having potential GPR174 polypeptide binding domains can be obtained by screening random peptide libraries displayed on phage or on bacteria such as *E. coli.* These random peptide display libraries can be used to screen for peptides which interact with GPR174. Techniques for creating and screening such random peptide display libraries are well known in the art (U.S. Patent No. 5,223,409, to Lardner; U.S. Patent No. 4,946,778, to Ladner; U.S. Patent No. 5,403,484, to Lardner; U.S. Patent No. 5,571,698, to Lardner; and Kay et al., Phage Display of Peptides and Proteins Academic Press, Inc., 1996) and random peptide display libraries and kits for screening such libraries are available commercially, for instance from CLONTECH Laboratories, Inc. (Palo Alto, Calif.), Invitrogen Inc. (San Diego, Calif.), New England Biolabs, Inc. (Ipswich, Mass.), and Pharmacia LKB Biotechnology Inc. (Piscataway, N.J.).

### B. Screening methods

As described herein, the inventors have identified a number of compounds capable of inhibiting GPR174-mediated signaling activity and have identified the G-protein signaling pathways that are modulated by GPR174. Described below are screening methods that can be used to identify such compounds and methods for measuring signal pathway activation.

### 1. CRA

One method for identifying compounds capable of modulating a receptor is using a cellular redistribution assay ("CRA"). Exemplary CRA methods are found in U.S. Patent No. 7,309,576 issued to O'Dowd et al. and in O'Dowd et al., J. Biomol. Screen. 12:175-85, 2007. Briefly, this method involves incorporation of a nuclear localization sequence (NLS) into a transmembrane protein that does not contain an endogenous functional NLS. Incorporation of the NLS alters the cellular localization of the GPCR, such that the majority of the NLS-modified receptor is present in the cell interior. An NLS-modified GPCR, when interacting with a functionally active compound, is mostly localized on the cell surface. Thus, it becomes possible to identify receptor modulators without knowledge of the endogenous ligand or a surrogate ligand.

The inventors have identified a number of compounds that inhibit GPR174 using a CRA assay similar to that described in U.S. Patent No. 7,309,576 and in O'Dowd et al., J. Biomol. Screen. 12:175-85, 2007. These compounds are described in detail below.

### 2. CRA Validation

To demonstrate that compounds identified in the CRA assay are capable of modulating G-protein signaling pathways, the inventors tested compounds identified in the CRA assays that measure signal transduction in several non-orphan receptors. As shown in Example 1, the inventors demonstrated that all of the compounds identified by screening compound libraries against the CHRM1, NPSR, and ADRA1a receptors using the CRA modulated the downstream signaling pathways of these receptors. These results demonstrate that the CRA screening "hits," in general, are relevant to receptor activity.

### 3. Overview of G-protein coupled receptor-regulated signaling pathways

While hundreds of GPCR proteins have been identified, only a small number of heterotrimeric G-proteins are activated by GPCR proteins. Heterotrimeric G-proteins include three subunits, the alpha, beta, and gamma subunits. The nomenclature of G-proteins is determined by the alpha subunit, *e.g.*, Gαq, Gαs, Gαi, Gαz, Gαo, Gα12, Gα13, Gα15, and Gα16. The G-proteins are grouped into families based on homology that also share some signaling characteristics. The G-protein signaling pathways include the Gq, Gs, Gi, and Gα12/13 signaling pathways. In addition to the G-protein signaling pathways, GPCRs can signal through βγ subunit complexes, arrestin 1, arrestin 2, arrestin 3, and arrestin 4.

Numerous assays have been developed to measure GPCR stimulation and the signaling pathways that are activated by GPCRs (Siehler S., Biotechnology 3:471-83, 2008 and references within). There are several generic assay technologies that measure GPCR activation. The binding of guanosine-5'-O-(3'-[³⁵S]thio)-triphosphate ([³⁵S]GTPγS) to Gα subunits has been used extensively to measure receptor activation (Milligan, Trends Pharmocol. Sci. 24:87-90, 2003).

Another common approach to measure receptor activation is the measurement of ligand-induced receptor internalization. GPCRs are typically internalized within 1-2 hours after agonist stimulation through a process of GPCR phosphorylation and the subsequent binding of β-arrestin, which acts as an adaptor to couple the receptor to clathrin-coated pits and targets the receptor for internalization to endosomes (Kahout et al., Mol. Pharmacol. 63:9-18, 2003). Several technologies have been developed that utilize some aspect of receptor internalization to show agonist-induced receptor activation (Siehler, Biotechnology 3:471-83, 2008 and references within). These generic assays are limited to measuring agonist-induced receptor activation and do not address which G-proteins couple to the GPCR and thus the signaling pathways that the GPCR activates to elicit its biological response.

### a. Gq signaling pathway

The G-proteins Gαq, Gα11, Gα14, Gα15/16 (collectively, the Gq family) activate the Gq signaling pathway and are associated with activation of the enzyme PLC, which in turn hydrolyzes the phospholipid PIP₂, releasing two intracellular messengers: DAG and IP₃. IP₃ is soluble and diffuses through the cytoplasm and interacts with IP₃ receptors on the endoplasmic reticulum, causing the release of calcium and raising the level of cytosolic calcium. This increase can be directly measured by intracellular calcium-detecting fluorescent dyes. DAG remains tethered to the inner leaflet of the plasma membrane due to its hydrophobic character, where it recruits protein kinase C (PKC), which becomes activated in conjunction with binding calcium ions. This results in a host of cellular responses through stimulation of calcium-sensitive proteins such as calmodulin (Kawakami et al., J. Biochem. 132:677-82, 2002).

Increased accumulation of IP₃ is associated with activation of Gq-associated receptors. Assays that detect IP₃ accumulation can be utilized to determine if a candidate compound is, *e.g.,* an agonist to a Gq-associated receptor (*i.e.,* such a compound would increase the levels of IP₃). Gq-associated receptors can also be assayed using a transcriptional reporter assay. For example, Gq-dependent PLC causes activation of genes containing Activator Protein 1 (AP1) elements, also known as AP-1 elements; thus, activated Gq-associated receptors will evidence an increase in the expression of such genes. Inverse agonists of a Gq-associated receptor will produce a decrease in such expression, and agonists will produce an increase in such expression. AP1 and other Gq-regulated transcriptional activators such as the serum response element (SRE) and the nuclear factor of activated T-cells response element (NFAT) can be operatively linked to a nucleic acid encoding a conveniently assayed reporter enzyme, such as luciferase, β-galactosidase, alkaline phosphatase, or other detectable output. Assays for such detection are commercially available, *e.g*., from Promega and Stratagene.

### b. Gs signaling pathway

The activated Gs protein (GαS and Gαolf) stimulates the enzyme AC, which catalyzes the conversion of ATP to cAMP. GPCRs that couple to the Gs protein are associated with increased cellular levels of cAMP that can be directly measured. The increase in cAMP activates protein kinase A (PKA) and has other downstream effects. PKA for example, phosphorylates and thus activates transcription of genes regulated by the cAMP response element binding (CREB) proteins (Neves et al., Science 296:1636-39, 2002). Gs activity can be assayed in a transcriptional reporter assay using a CREB element operatively linked to a nucleic acid encoding a conveniently assayed reporter enzyme, such as luciferase, β-galactosidase, or other detectable output. In addition, assays that detect intracellular cAMP levels can be utilized to assess Gs signaling activity. A variety of approaches known in the art for measuring cAMP can be utilized; in some cases a preferred approach relies upon the use of anti-cAMP antibodies in an ELISA-based format.

### c. Gi signaling pathways

The G-proteins G1 (Gαi1, Gαi2, and Gαi3), Gαo, Gαt1, Gαt2, Gαgust, Gαz (collectively, the Gi family) activate the Gi signaling pathway and are associated with inhibition of AC and suppression of cAMP generation. The sensory Gi protein, Gαt, activates the cGMP-dependent phosphodiesterase, causing a decrease in intracellular cGMP and Gαgust activates PLC. Thus, assays that detect intracellular cAMP levels, as described above, can be utilized to assess Gi signaling pathway activity. Some cells have relatively low levels of cAMP, thus, raising the cellular level of cAMP can be helpful to determine a basal level of Gi signaling activity. Those of skill are able to raise cellular cAMP levels by contacting cells with, for example, phorbol esters or forskolin. Alternatively, Gi receptor activity can be assayed using hybrid G-proteins to redirect Gi signaling to either Gq or Gs and using methods described herein. All Gi family members, except for Gαz, are sensitive to inhibition by pertussis toxin via ADP ribosylation of their respective α subunit (Siehler S., Biotechnology 3:471-83 (2008)).

### d. Other G-protein signaling pathways

Gα12/13 proteins are involved in Rho family GTPase signaling (through RhoGEF superfamily) and control cell cytoskeleton remodeling, thus regulating cell migration. Rho-GEF proteins catalyze the exchange of GDP for GTP to activate RhoA. RhoA, in turn, activates Rho kinase, which further leads to the activation of Activating Transcription Factor 2 (ATF2) and results in cellular responses (Liu et al., Methods Mol. Biol. 237:145-9, 2004).

### e. Role of β- and γ-subunits

In addition to the 16 Gα subunits there are five different β- and 12 γ-subunits that have been described (Milligan et al., Br. J. Pharmacol. 147:S46-S55, 2006). The βγ subunits form a heterodimer and were initially considered to be no more than a binding partner for the Gα subunit to suppress spontaneous signaling and to provide a membrane anchor for the Gα subunit. Although the role of the βγ subunits are still emerging, it is clear that the βγ subunits play a larger role in GPCR signaling and can directly activate enzymes and ion channels (Dupre et al., Annu. Rev. Pharmacol. Toxicol. 49:31-56, 2009).

### f. Arrestin signaling pathways

In addition to the G-protein-based signaling pathways, GPCRs can signal through arrestin 1, arrestin 2, arrestin 3, and arrestin 4. In addition to playing a role in terminating G-protein signaling and GPCR internalization, the arrestins have been implicated in signaling through ERK, JNK, p38, Akt, PI3 kinase, and RhoA (DeWire et al., Annu. Rev. Physiol. 69:483-510, 2007).

### 4. Testing of initial CRA hits for signaling activity

The inventors have confirmed that the initial hits identified using their CRA assay for compounds that interact with GPR174 are capable of inhibiting the Gs signaling pathway through GPR174. Accordingly, the inventors have definitely determined that the identified compounds functionally interact with GPR174. In particular (with reference to Table 1), compounds 1, 2, 5-18 and 22-56 were found to inhibit the basal activity of GPR174, demonstrating that, in this system, these compounds are GPR174 inverse agonists. These results are described in detail in Example 4 below. Compounds 3, 19-21 and 57-58 were found to have activity in the CRA and were found to be non-modulators in the GPR174 Gs signaling assay, therefore these compounds are characterized as GPR174 antagonists or allosteric modulators. Compound 4 was found to have activity in the CRA, was found to be a non-modulator in the GPR174 Gs signaling assay and was found to compete with the GPR174 agonist LysoPS, therefore compound 4 is characterized as a GPR174 antagonist. These results are described in detail in Example 4 below.

### C. GPR174

GPCRs have seven transmembrane alpha-helices. GPCRs have an extracellular N-terminus, three extracellular loops, three intracellular loops, and an intracellular C-terminus. The extracellular loops and transmembrane domains are involved in ligand binding, while the transmembrane domains and intracellular loops facilitate signal transduction. See, *e.g.,* Luttrell, Methods Mol. Biol. 332:3-49, 2006. Most GPCR proteins have conserved cysteine residues that form disulfide bridges in the extracellular loops. Many GPCR proteins are glycosylated at their N-terminus. Conserved cysteine residues are also found in the C-terminus and can serve as a site for palmitoylation. See, *e.g.,* Lutrell, *supra.* Structures have been solved for some GPCR proteins and consensus models of GPCR structure are available. See, *e.g.,* Lagerström and Schiöth, Nat. Rev. 7:339-57, 2008.

GPR174, also known as FKSG79 and GPCR17, is an orphan G protein-coupled receptor (GPCR) (Davenport et al., IUPHAR/BPS, Class A Orphans: GPR174, accessed on 11/1/2016). The human GPR174 protein is 333 amino acids, as set forth in SEQ ID NO:1 (NP_115942.1) shown in FIGURE 1A, encoded by SEQ ID NO:2 (NM_032553.1), as shown in FIGURE 1B. The GPR174 protein has seven transmembrane domains. The human GPR174 gene has a single coding exon (Takeda et al., FEBS Lett 520:97-101, 2002) and has been mapped to chromosome Xq21.1 (Sugita et al., Biochem Biophys Res Commun 430:190-195, 2013). The amino acid sequence of murine GPR174 protein (NP_001028423.1) is set forth as SEQ ID NO:3 (shown in FIGURE 1C). The amino acid sequence of rat GPR174 protein (NP_001100408.1) is set forth as SEQ ID NO:4 (shown in FIGURE 1D).

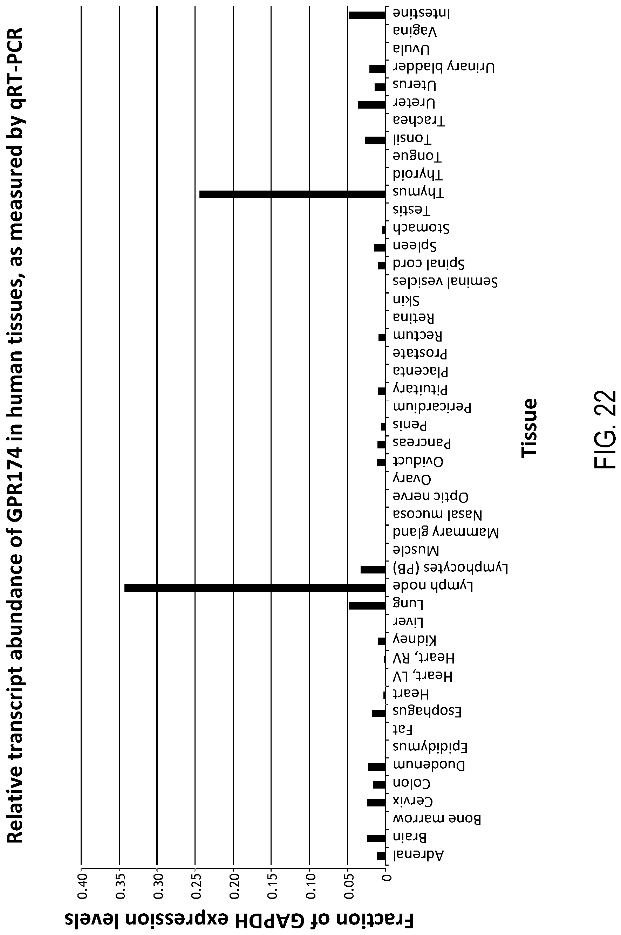

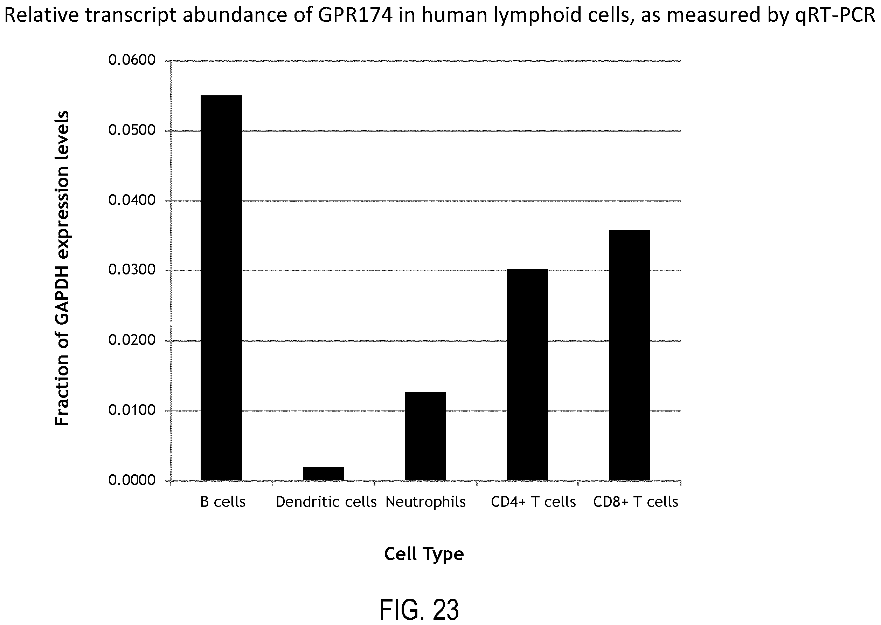

GPR174 is expressed in a relatively small number of tissues. By quantitative real time PCR (qPCR), GPR174 was found to be most abundantly expressed in thymus, lymph nodes, spleen and bone marrow, as shown in FIGURE 22 (see also Regard et al., Cell 135:561-71, 2008; Chu et al., J Med Genet 50:479-85, 2013; Sugita et al., Biochem Biophys Res Commun 430:190-195, 2013). Within the lymphoid tissues, GPR174 is expressed to high levels in naive B and T cells, especially in regulatory T cells, as shown in FIGURE 23 (see also Barnes et al., J Exp Med 212:1011-20, 2015). A recent report has identified lysophosphatidylserine (LysoPS) as a ligand for GPR174 (Inoue et al., Nat Methods 9:1021-9, 2012). LysoPS is a lysophospholipid mediator generated by enzymatic hydrolysis of membrane phospholipid, phosphatidylserine (PS). LysoPS is secreted by cells of the immune system and can induce multiple cellular responses, including T-cell suppression and mast cell degranulation (Makide et al., Prostaglandins Other Lipid Mediat 89:135-9, 2009). The EC₅₀ of LysoPS for GPR174 varies from 80 nM to 520 nM, depending on the assay used (Inoue et al., Nat Methods 9:1021-9, 2012; Uwamizu et al., J Biochem 157(3):151-160, 2015; Ikubo et al., J Med Chem 58(10):4204-19, 2015). In addition, LysoPS is a ligand for two other GPCRs-namely, GPR34 and P2Y10. The EC₅₀ of LysoPS for P2Y10 is 28 nM under conditions that the EC₅₀ for GPR174 is 520 nM. It should be noted that the International Union of Basic and Clinical Pharmacology (IUPHAR) continues to list GPR174 as an orphan GPCR (guidetopharmacology.org/GRAC).

GPR174 has been associated with several autoimmune diseases. The GPR174 gene localizes to the X chromosome, where a cluster of single nucleotide polymorphisms (SNP) near or within the gene has been associated with susceptibility to Grave's disease in both Asian and Caucasian populations (Chu et al., J Med Genet 50:479-85, Szymanski et al., Tissue Antigens 83:41-4, 2014). The strength of the association of the lead SNP, rs3827440, with Grave's disease is similar to that for a particular HLA-DRB1 allele, the strongest known genetic risk factor for the disease (Kula et al., Thyroid 16:447-53, 2006; Barlow et al., Clin Endocrinol (Oxf) 44:73-7, 1996). The same SNP is also significantly associated with autoimmune Addison's disease, another autoimmune endocrinopathy that results from adrenal damage (Napier et al., J Clin Endocrinol Metab 100:E187-90, 2015).

Barnes et al. (J Exp Med 212:1011-20, 2015) have demonstrated that in the mouse GPR174 is expressed in naive B and T cells and is also highly and preferentially expressed in a subset of T cells known as regulatory T cells (Tregs). In male mice lacking GPR174 (Gpr174^{-/Y}), Treg cell numbers were significantly increased in certain tissues, consistent with the observation that CD4+ T cell proliferation and differentiation into Treg cells is suppressed as a consequence of GPR174 signaling (Barnes et al., 2015 supra). In an experimental autoimmune encephalomyelitis model, peak disease severity was significantly reduced in the Gpr174^{-/Y} mice (Barnes et al., 2015 *supra*)*.* Together, these results underscore the importance of GPR174 in modulating Treg cells and suggest a possible therapeutic utility for the use of GPR174 antagonists in suppressing autoimmunity.

A link between GPR174 and certain cancers has been reported. Qin et al., (2011) discovered that GPR174 is expressed in metastatic melanoma, particularly in subcutaneous metastases relative to either lymph node or brain metastases (Qin et al., Pigment Cell Melanoma Res 24:207-18, 2011). Sugita et al., (2013, *supra*)*,* observed that ectopic overexpression of GPR174 in CHO cells resulted in an unusual elongated, spindle-shaped morphology and delayed proliferation, demonstrating that the receptor may affect phenotypic change. Also, modest expression of GPR174 message was noted in lymphoma (Unigene EST Profile database).

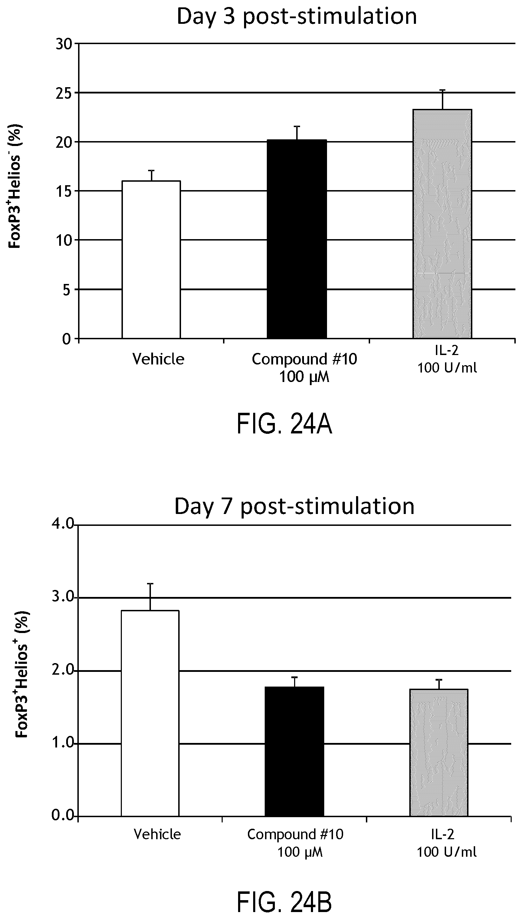

Given the oncogenic impact of intra-tumoral Tregs (Savage et al., 2013, supra), we explored the potential of GPR174 inhibitors to modulate Treg quantity and/or activity. As described in Example 6, and shown in FIGURES 24A, 24B, 25 and 26, the inventors surprisingly determined that in human PBMCs, inhibition of GPR174 enhances the activation of the immune system while also reducing the abundance of Treg cells. This is a surprising finding in view of the mouse data described in Barnes et al. (2015, *supra*)*,* which showed that in male mice lacking GPR174, Treg cell numbers were significantly increased in certain tissues.

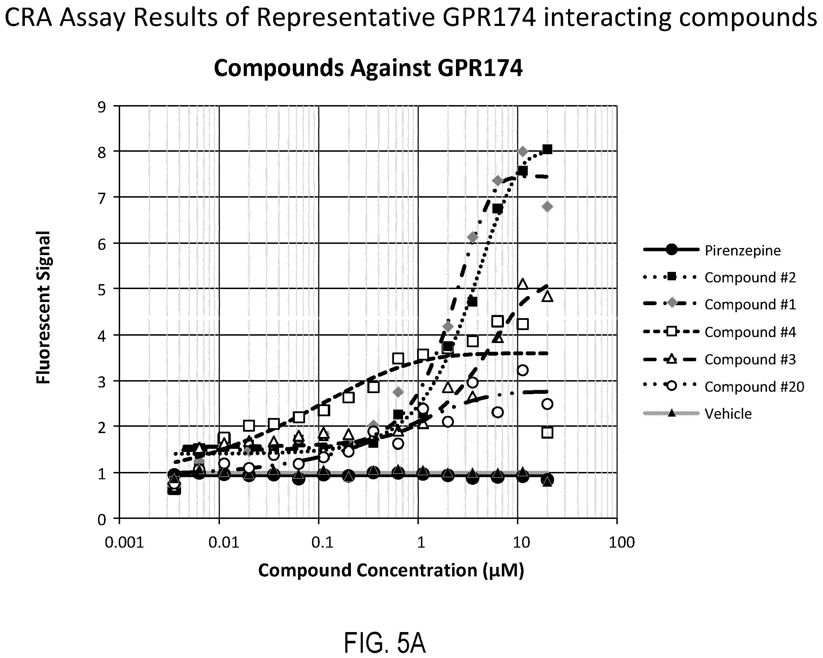

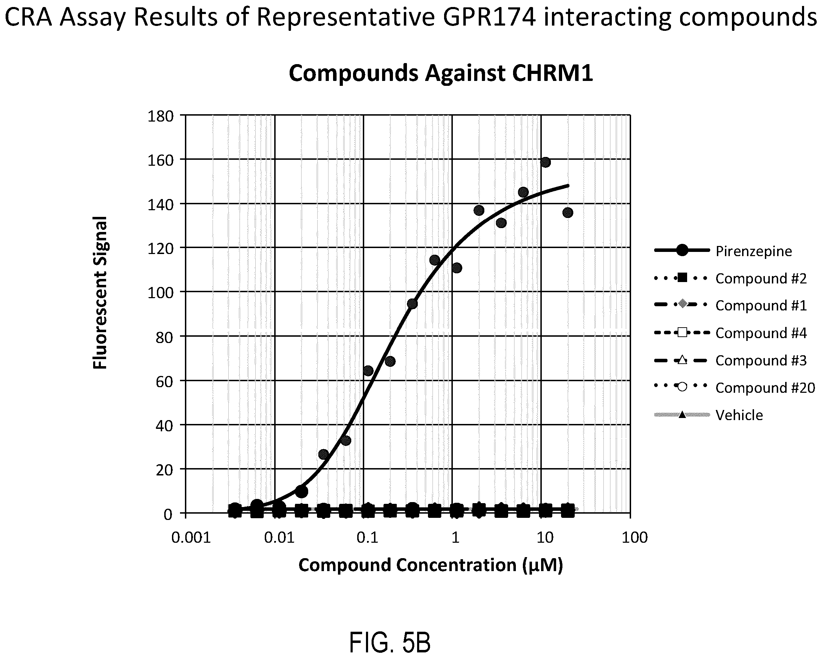

As further described herein in Examples 3 and 4, shown in Table 1 and in FIGURES 5-21, the inventors have determined that GPR174 is a Gs-coupled receptor that either constitutively or through ligand activation is expected to elevate intracellular cAMP levels. cAMP is a key regulator of the immune system (Mosenden and Tasken, Cellular Signaling 23:1009-1016, 2011). cAMP is known to inhibit IL-2 production and T cell receptor (TCR)-mediated signaling, and subsequently inhibit T cell activation (see Vang et al., J Exp Med 193:497-507, 2001; Ruppelt et al., J Immunol 179:5159-5168, 2007). IL-2 is a major cytokine that determines T cell survival and differentiation into effector, memory, and regulatory T cells. GPR174 inhibitors, such as those disclosed herein, are expected to reduce cAMP concentration and therefore promote T cell activation by stimulating TCR signaling and IL-2 secretion, a mechanism that explains the down-regulation of Treg cells and the activation of the effector T cells, thus the overall potentiation of T-cell mediated immunity, a critical component in cancer immunotherapy. A large number of cancers are expected to be more susceptible to a more robust immune system promoted by an agent such as a GPR174 inhibitor that stimulates T-cell mediated immunity (see Jiang et al., Oncoimmunology 5(6):e1163462, 2016; Papaioannou et al., Ann Transl Med 4(14):261, 2016; Park et al., Immune checkpoint inhibitors for cancer treatment, Arch Pharm Res, Oct 21, 2016; Sukari et al., Anticancer Res 36:5593-5606, 2016).

As further described herein in Examples 16 and 17, the inventors have discovered that phosphatidylserine (PS) is an agonist for GPR174-mediated Gs signaling. Example 17 provides experimental results demonstrating that apoptotic cells stimulate GPR174 Gs signaling pathway in cells expressing GPR174. As further described in Example 16 and shown in FIGURES 47A-47F, PS-mediated GPR174 Gs signaling is inhibited by representative GPR174 inhibitory compounds 6, 10, 11, 20, 23 and 30 which belong to different chemical classes (*i.e.,* Groups I, II and IV). This data demonstrates that compounds 6, 10, 11, 20, 23 and 30 act as GPR174 antagonists and inhibit PS liposome-mediated cAMP signaling. Thus, PS liposome signaling through GPR174 is inhibited by multiple GPR174 inhibitory small molecule compounds with diverse chemical structures. As described herein, extracellular PS is highly enriched in the tumor microenvironment and is found on the surface of tumor cells as well as endothelial cells of blood vessels permeating solid tumors. Furthermore, apoptotic neutrophils and activated platelets, both of which expose PS, are also recruited to solid tumors (see A.K and Rao D.A., Blood 120:4667-4668, 2012; Schlesinger, M., Journal of Hematology and Oncology (11) 125, 2018; Treffers L.W. et al., Immunological Reviews vol 273: 312-328, 2016; and Gregory A. D. and Houghton A. M., Cancer Research Vol 71 (7): 2411-6, 2011). Thus, high concentrations of PS are considered a major source of tumor-mediated immunosuppression and may play a role in resistance to cancer immunotherapies such as checkpoint inhibitors.

Accordingly, one case provides a method of treating cancer, the method comprising administering to a patient a therapeutically effective amount of a GPR174 inhibitor that inhibits a GPR174 G-alpha-s signaling thereby stimulating an immune response in the patient. In another more specific case, GPR174 expressed on immune cells is contacted by phosphatidylserine (PS) or lysophosphatidylserine (lysoPS) in the tumor microenvironment or associated lymphoid tissues and wherein said GPR174 inhibitor inhibits PS or lysoPS mediated GPR174 signaling. In certain cases, the cancer comprises live cells, dying cells, or extracellular vesicles having phosphatidylserine (PS) on their surface.

In some cases, the patient is a mammalian patient. In certain specific cases, the method further comprises administering at least one additional agent selected from the group consisting of:
i. an adenosine-A2A (A2A) receptor antagonist;
ii. an adenosine-A2B (A2B) receptor antagonist;
iii. a CD73 inhibitor;
iv. a CD38 inhibitor;
v. a CD39 inhibitor; or
vi. a Treg attenuating agent, wherein the GPR174 inhibitor and the at least one additional agent are administered simultaneously, or sequentially in any order, provided that the effects of the first administered inhibitor or antagonist remain present at the time of the second administered inhibitor or antagonist.

Additionally, in one case, the present disclosure provides a method of stimulating T-cell mediated immunity in a subject suffering from cancer comprising administering a GPR174 inhibitor to said subject in an amount effective to stimulate T-cell mediated immunity. In some cases, the subject is suffering from a cancer selected from the group consisting of: breast cancer, melanoma, colon cancer, urological cancer, lung cancer, small-cell and non-small-cell lung cancer, relapsed or refractory malignancies, non-Hodgkin and Hodgkin lymphomas, lymphoma, follicular lymphoma, lymphocytic lymphoma, CNS lymphoma, T-cell lymphoma, AIDS-related lymphoma, acute lymphoblastic leukemia, gastrointestinal cancers, liver cancer, hepatocellular carcinoma, ovarian cancer, pancreatic cancer, bile duct cancer, prostate cancer, renal carcinoma, bladder cancer, colorectal cancer, multiple myeloma, mesothelioma, cervical cancer, vaginal cancer, anal cancer, oropharyngeal cancer, myelogenous leukemia, chronic myeloid leukemia, gastric cancer, nasopharyngeal carcinoma, head and neck carcinoma, glioblastoma, gliosarcoma, squamous cell brain cancer, malignant glioma, diffuse pontine gliomas, esophageal cancer, thyroid cancer, astrocytoma, thoracic cancer, endometrial cancer, cutaneous cell carcinoma, leukemia, acinar cell carcinoma, adenocarcinoma, bronchioloalveolar carcinoma, cholangiocarcinoma, chordoma, giant cell carcinoma, intestinal carcinoma, major salivary gland carcinoma, malignant odontogenic neoplasm, malignant peripheral nerve sheath tumor, skin cancer, testicular cancer, germ cell tumor, neuroendocrine carcinoma, parathyroid carcinoma, pituitary gland carcinoma, placental choriocarcinoma, scrotal cancer, tracheal carcinoma, transitional cell carcinoma, cancer of the uterus, vulvar cancer, kidney cancer, rectum cancer, fallopian tube carcinoma, peritoneal carcinoma, epithelial cancer, pleural mesothelioma, sarcomatoid carcinoma, synovial sarcoma, nephroblastoma, neuroblastoma, adult acute myeloid leukemia, myelodysplastic/myeloproliferative neoplasm, embryonal carcinoma, Kaposi sarcoma, bone cancer, uterine cancer, stomach cancer, carcinoma of the endometrium, cancer of the small intestine, cancer of the endocrine system, cancer of the paragland, cancer of the adrenal gland, sarcoma of soft tissue, cancer of the urethra, cancer of the penis, cancer of the ureter, carcinoma of the pelvis, neoplasm of the central nervous system, primary tumor angiogenesis, spinal axis tumor, epidermoid cancer, environmentally induced cancers including those induced by asbestos, adenosarcoma, adenosquamous carcinoma, adrenocortical carcinoma, astrocytic tumors, basal cell carcinoma, chondosarcoma, Ewing's sarcoma, gallbladder cancer, hypopharyngeal cancer, intraocular melanoma, laryngeal cancer, leiomyosarcoma, lip and oral cavity cancer, malignant mesothelial tumors, malignant thymoma, medulloblastoma, medulloepithelioma, Merkel cell carcinoma, mucoepidermoid carcinoma, myelodysplastic syndrome, nasal cavity and paranasal sinus cancer, osteosarcoma, pulmonary blastoma, pineal and supratentorial primitive neuroectodermal tumors, plasma cell neoplasm, retinoblastoma, rhabdomyosarcoma, sarcoma, neuroectodermal tumors and Wilm's tumor.

In addition to their immune function, animal experiments have shown that T cells and cytokines also impact CNS functions. T cell deficiency impairs cognition (Kipnis et al., PNAS 101:8180-8185, 2004), an effect mediated by IL-4 produced by meningeal resident T cells (Derecki et al., J Exp Med 207:1067-1080, 2010). IL-4 was also implemented in promoting axonal regrowth after spinal cord or optical nerve injury (Walsh et al., J Clin Invest 125:699-714, 2015). Lymphocyte-deficient mice are less social, and this effect is mediated by IFN-γ produced by T cells residing in choroid plexus (Filano et al., Nature 535:425-429, 2016). Stimulating T cells either through depletion of Tregs (Baruch et al., Nat Commun 6:7967, 2015) or by blocking T cell check-point protein PD-1 (Baruch et al., Nat Med 22:135-137, 2016) reduced amyloid-β plaques in mouse brains and improved learning and memory scores. These data implicate T cells and their respective cytokines in normal functioning of the CNS, in neurodegenerative diseases, and in neural tissue repair after injury. Therefore, GPR174 inhibitory compounds, which affect T cell activity, differentiation and cytokine production, as shown in Examples 3 and 4, have therapeutic potential for treating mental illness and neurodegenerative diseases, such as schizophrenia, Parkinson's disease, autism, mild cognitive impairment, age-related cognitive decline, Alzheimer's disease, frontotemporal dementia, amyotrophic lateral sclerosis, as well as spinal cord and traumatic brain injury.

Natural killer cells (NK cells) play a critical role in the innate immune system, and unlike other cells in the immune system, NK cells can be cytotoxic without pre-stimulation (Mandal et al., Hematol Oncol Stem Cell Ther. (2):47-55, 2015). For example, they can attack tumor cells even when the expression of MHC class I molecules is absent or downregulated. Tumor cells may also be attacked by NK cells when NKG2D ligands on tumor cells are upregulated (Mandal et al., Hematol Oncol Stem Cell Ther. (2):47-55, 2015). However, NK cells can be suppressed by Treg cells, and there is a correlation between an increased frequency of Treg cells and cancer progression (Pedroza-Pacheco, et al., Cell Mol Immunol. 10(3):222-9, 2013). Accordingly, many therapies have sought to suppress Treg cells in order to promote NK cells and improve patient prognosis. NK cells are also vital to the immune response against viruses and other pathogens as well as being implicated in autoimmune processes (Zwimer, et al., Front Immunol. 8: 25, 2017). The function, differentiation, growth and/or proliferation of NK cells can be activated by cytokines such as IL-2, IL-21, IL-12, IL-15, IL-18, IL-10, IFN-alpha, IFN-beta and TGF-β (Wu, et al., Front Immunol. 8: 930, 2017). As such, NK cell function, differentiation, growth and/or proliferation may be affected by GPR174 inhibitory compounds through the effects of GPR174 inhibition on T cell differentiation and/or cytokine production, for example. Thus, GPR174 inhibitory compounds may have additional therapeutic potential for diseases such as cancer.

In accordance with the foregoing, in one aspect, the present disclosure provides a method of treating, preventing, and/or reducing the likelihood of developing cancer in a subject in need of such treatment. The method comprises administering a compound that inhibits a GPR174-mediated signaling pathway to the subject, or to cells obtained from the subject and re-introduced into the subject, wherein inhibition of the GPR174-mediated signaling pathway results in treatment or prevention or reduction in the severity of the disease, disorder, or condition. Examples of indications that can be treated or prevented using methods of the present disclosure are discussed below.

### D. GPR174 Interacting compounds

The inventors have identified the following compounds as functionally interacting with GPR174 receptor using a modified form of the CRA assay described above. Structures of the identified representative compounds are set forth below in Table 1.

**Table 1: Representative compounds**

| Compound No./Formula Group | Structure | CRA EC₅₀ (µM) | Human GPR174 CRE Activity, EC₅₀; Fold decrease in Gs signaling activity |
|---|---|---|---|
| 1 (Group I) | 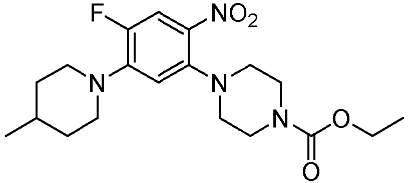 | 2.1, 2.1, 1.5, 0.9 | IA; 0.4, 0.8; 2.9 fold decrease |
| 2 (Group I) | 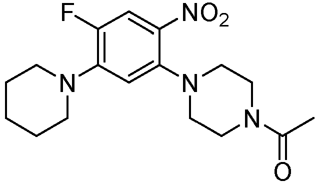 | 3.3, 3.0, 5.1, 0.4 | IA; 0.4, 0.3; 1.7 fold decrease |
| 3 (Group I) | 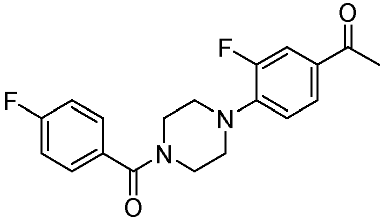 | 4.1. 2.0, >10 | Antagonist or allosteric modulator |
| 4 (Group I) | 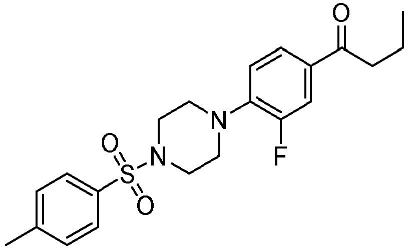 | 0.2, 2.0, >1 | Antagonist |
| 5 (Group I) | 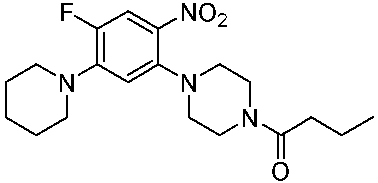 | >1.2 | IA; 0.9, 2.4 fold decrease |
| 6 (Group I) | 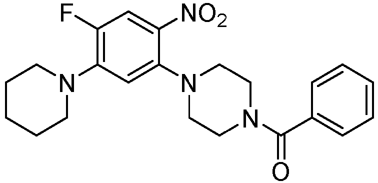 | 1.4, >0.4 | IA; 0.4, 0.3; 3.8 fold decrease |
| 7 (Group I) | 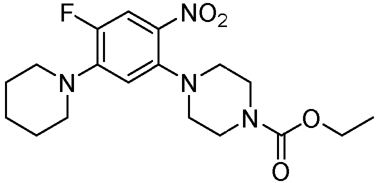 | >0.6, >0.7 | IA; 0.3, 0.5; 2.4 fold decrease |
| 8 (Group I) | 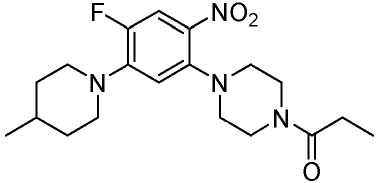 | >0.7, >1.6 | IA; 0.7, 0.4; 3.3 fold decrease |
| 9 (Group I) | 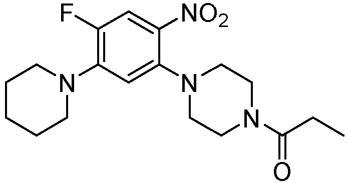 | >2 | IA; 0.7, 0.5; 2.1 fold decrease |
| 10 (Group I) | 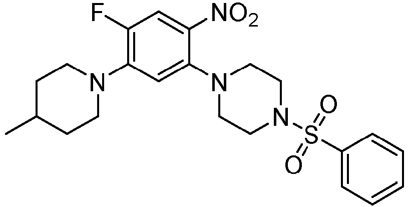 | 0.6, 0.5 | IA, 0.2, 0.5; 5.1 fold decrease |
| 11 (Group I) | 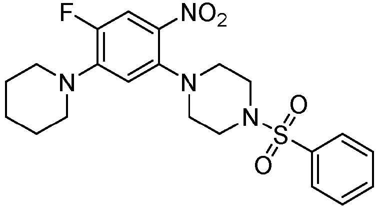 | 1.5, >1.1 | IA, 0.5, 1.3, 0.6; 3.1 fold decrease |
| 12 (Group I) | 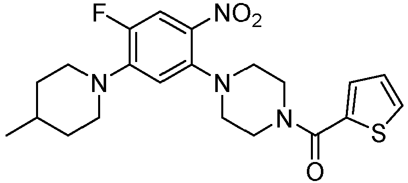 | 0.3 | IA, 0.4, 0.7, 3.9 fold decrease |
| 13 (Group I) | 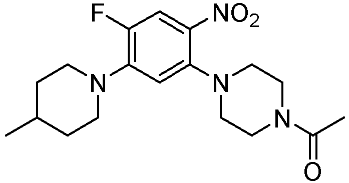 | >2 | IA, 0.8, 1.2; 2.2 fold decrease |
| 14 (Group I) | 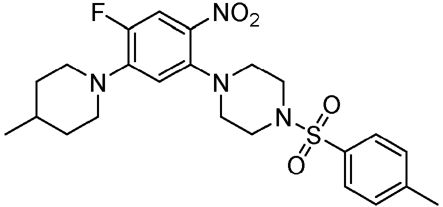 | ND | IA, 1.1; 3 fold decrease |
| 15 (Group I) | 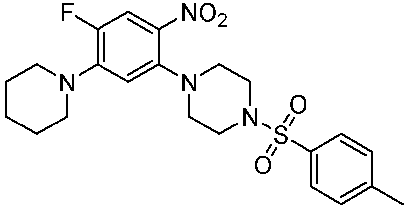 | ND | IA, 1.7, 2.6 fold decrease |
| 16 (Group I) | 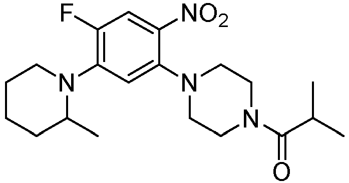 | ND | IA, 0.8, 2.1 fold decrease |
| 17 (Group I) | 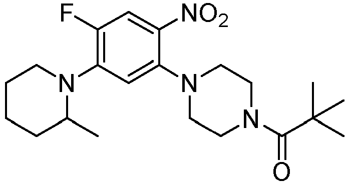 | ND | IA, 3.0, 2.0 fold decrease |
| 18 (Group I) | 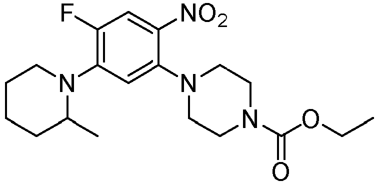 | ND | IA, 1.0; 1.9 fold decrease |
| 53 (Group I) | 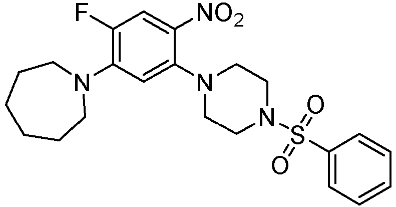 | ND | IA, 1.8; 3.4 decrease |
| 19 (Group II) | 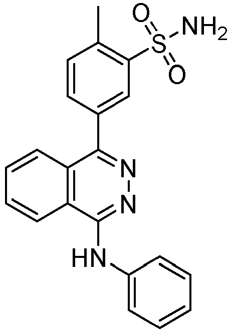 | 1.0, 2.0, 1.6 | Antagonist or allosteric modulator |
| 20 (Group II) | 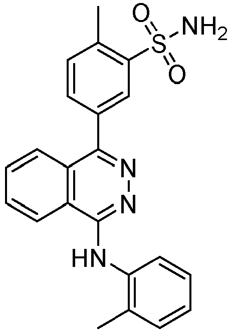 | 0.5. 1.0, 5.0 | Antagonist or allosteric modulator |
| 21 (Group III) | 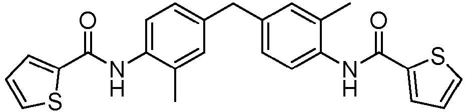 | 2.7, 0.5, >0.5 | Antagonist or allosteric modulator |
| 22 (Group IV) | 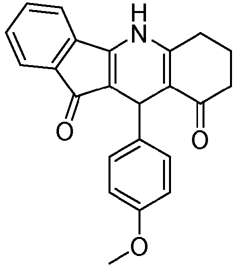 | 6.7, 5.3 | IA, 1.0, 1.5; 10.2 fold decrease |
| 23 (Group IV) | 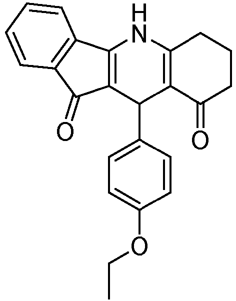 | **>10,** 2.9 | IA, 0.4, 0.9, 1.2, 1.0; 14.1 fold decrease |
| 24 (Group IV) | 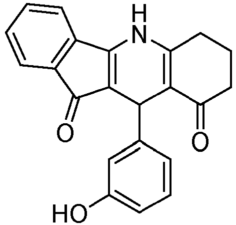 | ND | IA, 4.4; 2.8 fold decrease |
| 25 (Group IV) | 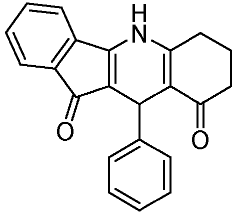 | ND | IA, 4.6, 4.2 fold decrease |
| 26 (Group IV) | 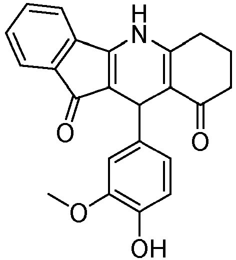 | ND | IA, 3.6, 1.7 fold decrease |
| 27 (Group IV) | 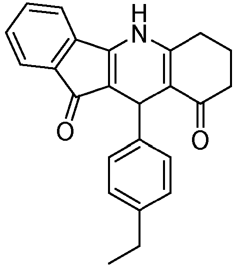 | ND | IA, 0.8, 0.5, 6.2 fold decrease |
| 28 (Group IV) | 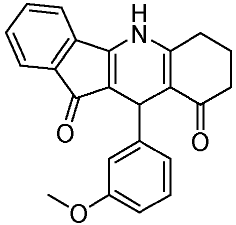 | ND | IA, 3.6, 1.9 fold decrease |
| 29 (Group IV) | 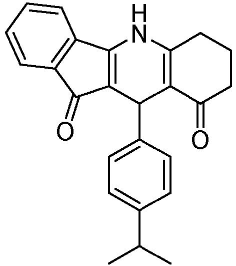 | ND | IA, 1.2, 5.3 fold decrease |
| 30 (Group IV) | 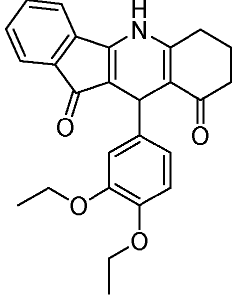 | ND | IA, 3.8, 2.2 fold decrease |
| 31 (Group IV) | 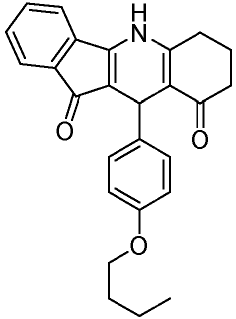 | ND | IA, 1.2, 8.9 fold decrease |
| 32 (Group IV) | 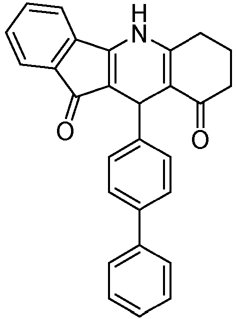 | ND | IA, 2.5, 3.9 fold decrease |
| 33 (Group IV) | 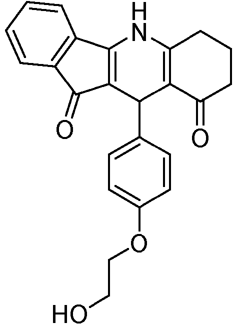 | ND | IA, 4.4, 9.2 fold decrease |
| 34 (Group IV) | 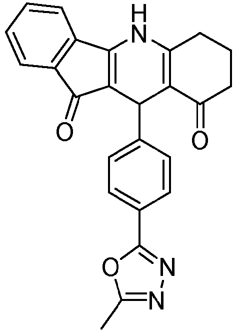 | ND | IA, 2.8, 8.4 fold decrease |
| 35 (Group IV) | 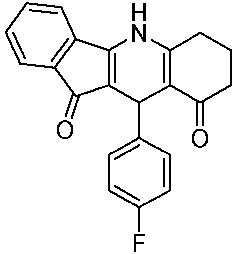 | ND | IA, 4.3, 2.8 fold decrease |
| 36 (Group IV) | 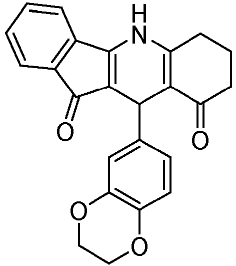 | ND | IA, 2.6, 8.3 fold decrease |
| 37 (Group IV) | 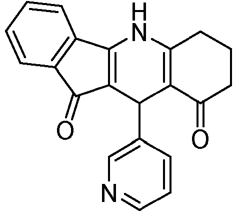 | ND | IA, 5.4, 2.8 fold decrease |
| 38 (Group IV) | 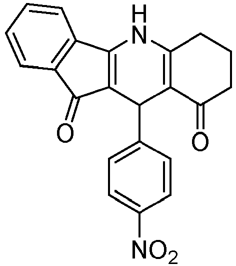 | ND | IA, 2.7, 8.6 fold decrease |
| 39 (Group IV) | 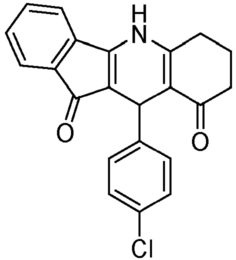 | ND | IA, 3, 7.8 fold decrease |
| 40 (Group IV) | 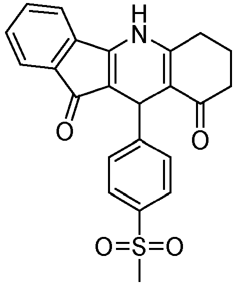 | ND | IA, 3.3, 8.8 fold decrease |
| 41 (Group IV) | 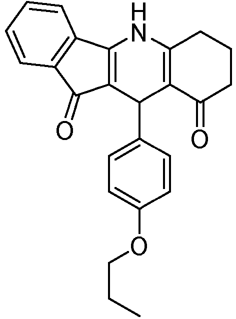 | ND | IA, 1.0; 13.7 fold decrease |
| 42 (Group IV) | 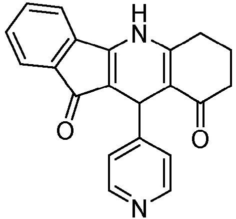 | ND | IA, 6.6, 4.3 fold decrease |
| 43 (Group IV) | 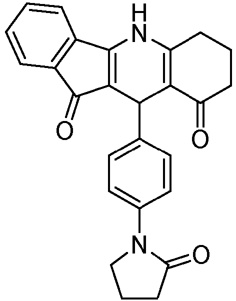 | ND | IA, 5.2, 5.1 fold decrease |
| 44 (Group IV) | 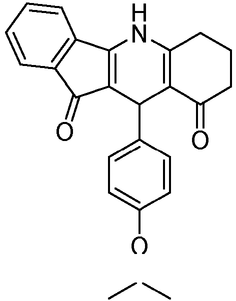 | ND | IA, 1.3, 4.7 fold decrease |
| 45 (Group IV) | 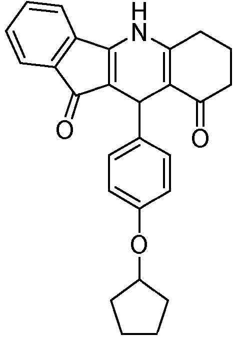 | ND | IA, 1.0; 7.6 fold decrease |
| 46 (Group IV) | 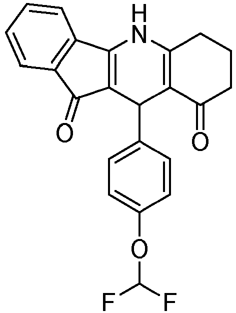 | ND | IA, 1.2, 8.1 fold decrease |
| 47 (Group IV) | 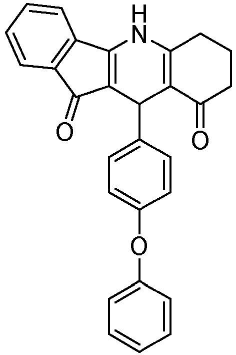 | ND | IA, 1.0, 7.1 fold decrease |
| 48 (Group IV) | 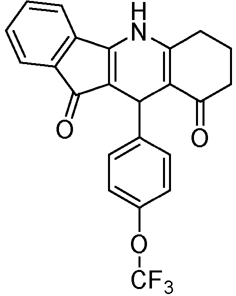 | ND | IA, 1.2, 5 fold decrease |
| 49 (Group IV) | 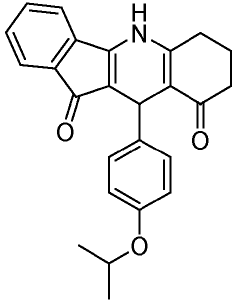 | ND | IA, 0.6; 14.3 fold decrease |
| 50 (Group IV) | 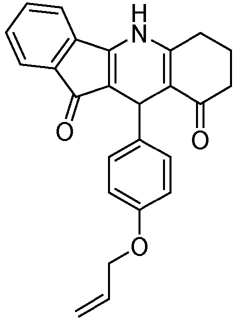 | ND | IA, 1.2, 10.1 fold decrease |
| 51 (Group IV) | 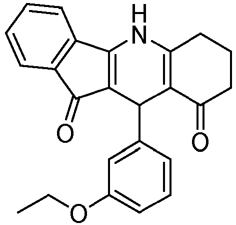 | ND | IA, 1.6, 1.7 fold decrease |
| 52 (Group IV) | 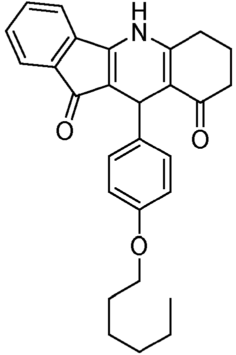 | ND | IA, 0.5, 3.1 fold decrease |
| 54 (Group IV) | 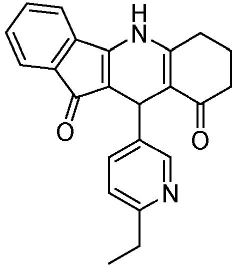 | ND | IA, 2.0, 7 fold decrease |
| 55 (Group IV) | 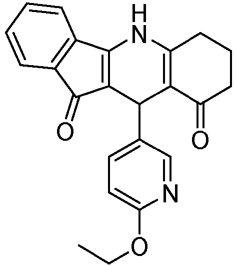 | ND | IA, 0.6, 7 fold decrease |
| 56 (Group V) | 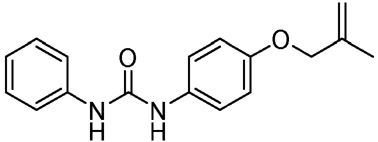 | 0.5 | IA, 0.5, 3 fold decrease |
| 57 (Group VI) | 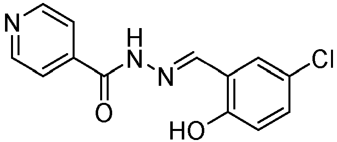 | 5.6 | Antagonist or allosteric modulator |
| 58 (Group VI) | 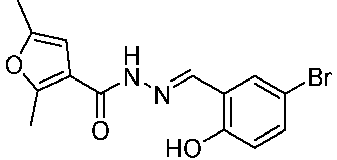 | 3.7 | Antagonist or allosteric modulator |
| 59 (Group Va) | 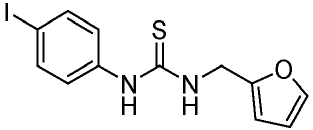 | ND | NM |

| | | | |
|---|---|---|---|
| *For the EC₅₀ values, it is noted that all the compounds included in Table 1 showed at least a minimum activity level of greater than 3 times the average background of the assay when tested at 40 µM. Where present, multiple EC₅₀ values correspond to values obtained in separate experiments. "IA" refers to "inverse agonist." "NM" refers to "non-modulator." Compound 4 is labeled as an antagonist as it was found to compete with the GPR174 agonist LysoPS (see Example 4). | | | |

The GPR174 interacting compounds described herein (*e.g.,* a compound according to any of Formulas I, II, III, IV, V or VI (or (I), (II), (III), (IV), (V), (Va), or (VI)) or any of exemplary Compounds 1-59 of Table 1) can be prepared according to methods known in the art using conventional starting materials. Exemplary syntheses are provided herein.

Compounds of formula (I) can be prepared according to methods known in the art. As shown in Scheme 1A, piperazine derivative A and electrophile B may be subjected to a cross-coupling reaction or a nucleophilic aromatic substitution reaction (*e.g.*, when one or both of X³ and X⁷ is N). The cross-coupling reaction may be a C-C cross-coupling reaction (*e.g.,* Suzuki coupling, Hiyama coupling, Stille coupling, Negishi coupling, Tamao-Kumada coupling, or Murahashi coupling), C-N cross-coupling reaction (*e.g*., Buchwald-Hartwig coupling or Ullmann coupling), C-O cross-coupling reaction, etc. Typical cross-coupling reaction conditions include a reaction between an electrophile (*e.g*., compound B) and a nucleophile (*e.g.,* compound A) in the presence of catalytic quantities of a metal salt, *e.g.,* a palladium, copper, iron, or nickel salt (*e.g.,* PdCl₂, Pd(OAc)₂, CuBr, CuI, (CuOTf)₂·toluene complex, Fe(OTf)₃, FeCl₃, FeBr₃, NiCl₂, or NiBr₂). Optional ligands, *e.g.,* a phosphine (*e.g.,* PPh₃, P(2-furyl)₃, P(*t*-Bu)₃, dppf, dppb, or BINAP), an *N*-heterocyclic carbene (*e.g.,* SIMes or SIPr), or di-pyridine (*e.g*., 2,2'-bipyridyl or 1,10-phenanthroline), may be added to promote the reaction. Alternatively, an organometallic complex, *e.g*., Pd(PPh₃)₄ or (dppf)PdCl₂, may be employed directly with or without additional ligands. Additives, *e.g*., tetrabutylammonium fluoride, LiCl, KOAc, or AgOTf, may be added to minimize dehalogenation or to facilitate the cross-coupling reaction. One of skill in the art would be able to determine an appropriate solvent for the reaction through routine screening. Non-limiting examples of solvents used in cross-coupling reactions are water, ethanol, acetone, tetrahydrofuran, toluene, 1,4-dioxane, and mixtures thereof. For non-limiting examples of conditions and catalysts that can be used in cross-coupling chemistry, see Miyaura et al., "Cross-Coupling Reactions: A Practical Guide" in Topics in Current Chemistry, Springer, 2002; Nicolaou et al., Angew. Chem. Int. Ed., 44:4442-4489, 2005; Maiti et al., Chem. Sci., 2:57-68, 2011. An appropriate base may also be necessary for catalyst turnover, *e.g*., in Suzuki cross-coupling or Buchwald-Hartwig cross-coupling. Typical bases include K₃PO₄, Na₂CO₃, Cs₂CO₃, trialkylamine (*e.g*., Hünig's base or triethylamine), and pyridine or substituted pyridine (*e.g*., lutidine or collidine). The nucleophilic aromatic substitution reaction conditions are well-known in the art and ordinarily involve the use of F or Cl as a leaving group (*LG*). A non-limiting example of nucleophilic aromatic substitution reaction conditions is provided in Petit and Vo-Thanh, 15th International Electronic Conference on Synthetic Organic Chemistry, Section C004, 2011. The product of this transformation may be subjected to deprotection (removal of *PG^{N}*) to afford C, which can be functionalized further to include R¹ that is not H through reductive amination, amidation, cross-coupling, acylation, or sulfonation. Typical amidation conditions include the use of reagents, such as EDC/DMAP, HATU/HOAt, or HBTU/HOAt. One skilled in the art would be aware of reductive amination conditions and appropriate hydride sources (*e.g.*, NaBH(OAc)₃ or NaBH₃CN). Exemplary synthetic procedures for the preparation of compounds of formula (I) can be found in Sythana S. et al, Organic Process Research and Development, (2014), 18, 912-918 and Garino et al., J. Med. Chem. (2006), 49, 4275-4285. Additionally, compounds of formula (I) can be further modified to provide other compounds of formula (I) depicted in Scheme 1B.

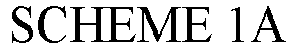

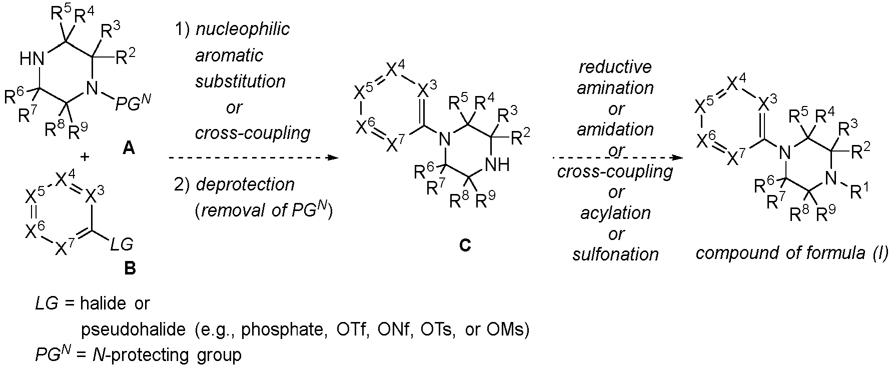

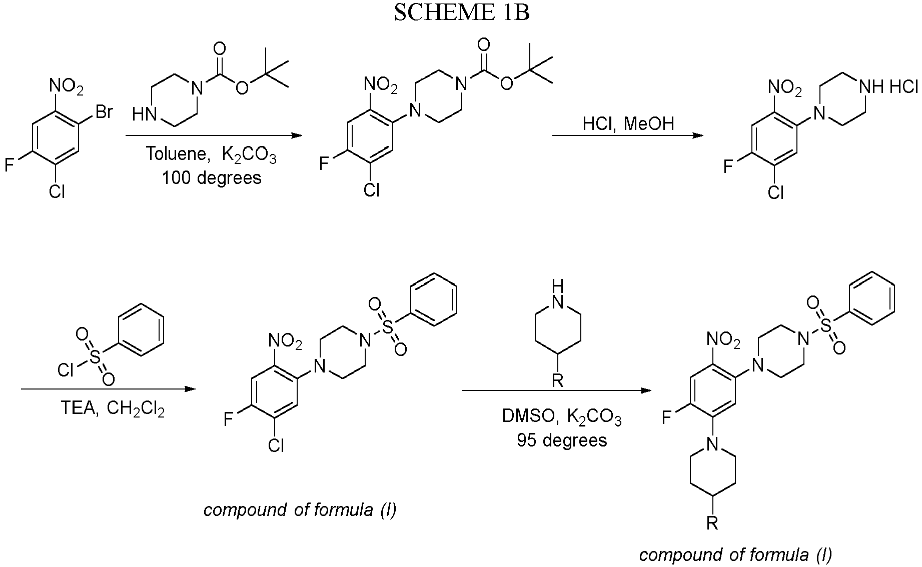

Compounds of formula (II) can be prepared according to methods known in the art. As shown in Scheme 2, cyclic anhydride A can be subjected to alkylation with B or C to furnish compound D. The reaction with B can be carried out under cross-coupling reaction conditions as described herein (*e.g*., when [M] is Cu, Sn, Si, or B). A non-limiting example of the cross-coupling reaction conditions suitable for this transformation is provided in Xin et al., Synthesis, 1970-1978, 2007. When [M] of B is Li, Mg, or Zn (*e.g.,* diaryl-Zn or diheteroaryl-Zn), the reaction between A and B may proceed without a catalyst. One of skill in the art would be able to select appropriate conditions based on the presence or absence of certain substituents (*e.g.,* the presence of carbonyl groups that are not part of the moiety undergoing a reaction would exclude the use of, *e.g.,* Li, Mg, and diaryl- or diheteroaryl-Zn reagents). Alternatively, when D is prepared from cyclic anhydride A and compound C, the appropriate conditions can be Friedel-Crafts reaction conditions (*e.g.\*, in the presence of catalytic or stoichoimetric quantities of oxophilic Lewis Acid (*e.g.,* AlCl₃ or BF₃)). Compound D upon esterification and condensation with hydrazine (*e.g.*, hydrazine hydrate) can afford compound E. The esterification reactions conditions may include Steglich esterification (*e.g.*, EDC/DMAP) or treatment of compound D with *iso*-butyl chloroformate and *N*-methylmorpholine to prepare an intermediate mixed anhydride, which is then reacted with a nucleophile (*e.g.*, alcohol). Alternatively, esterification reaction may involve the use of a mild base (*e.g.,* K₂CO₃) and an electrophile (*e.g.,* Me₂SO₄) in polar aprotic solvent (*e.g.,* acetone). A non-limiting example of the reaction conditions for phthalazine formation from o-acylbenzoic acid ester is provided in Li et al., Molecules, 11:574-582, 2006. Compound E can be subjected to a halogenation reaction (*e.g.*, with PCl₅, POCl₃, PCl₃, or a mixture of two reagents or three reagents) and subsequent nucleophilic aromatic substitution or cross-coupling to introduce R¹, thereby yielding a compound of formula (II). The cross-coupling reaction and nucleophilic aromatic substitution reaction conditions are as described herein.

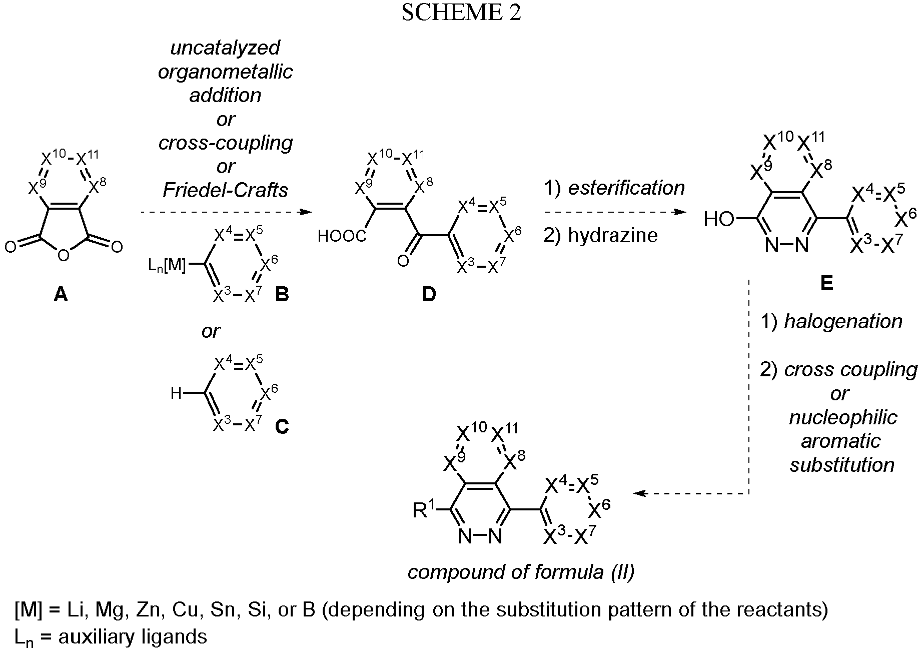

Compounds of formula (III) can be prepared according to methods known in the art. General methods for the preparation of compounds of formula (III) are described in Endo et al., Journal of Organic Chemistry, 77(17): 7223-7231 2012. A non-limiting example of the synthesis route towards a compound of formula (III) is shown in Scheme 3, Route 1.

As shown in Scheme 3, a compound of formula (III) (*e.g*., compound D) can be prepared from simple starting materials, such as aryl halides, preferably, bromides, A and B, through a one-pot sequential Suzuki-Miyaura cross-coupling reaction using diborylmethane C. Compound D can be further modified, for example, by removal of optional protecting groups or modification of substituents in the aryl rings. Alternatively, as shown in Scheme 3, Route 2, compound E can be used as a starting material, as described by Wang et al., Organic & Biomolecular Chemistry, (2015), 13(17), 4925-4930.

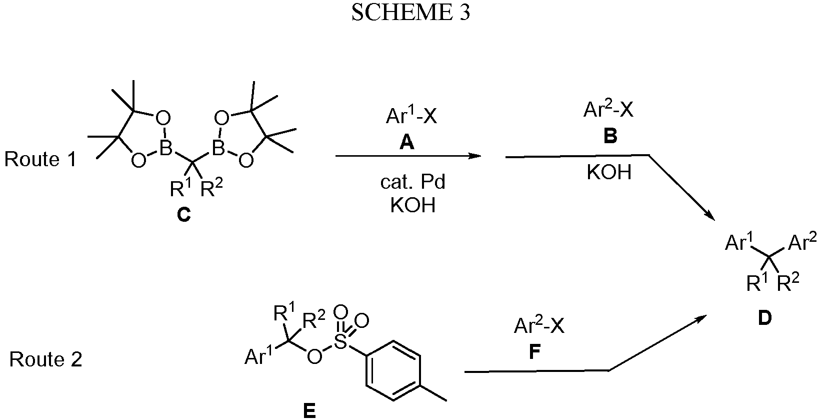

Compounds of formula (IV) can be prepared according to methods known in the art. For example, as shown in Scheme 4, compound A and compound B can be reacted with arylaldehyde C and amine D under appropriate conditions to give compound E (compound of formula (IV)). Non-limiting examples of reactions conditions useful in the synthesis of compound E are provided in Tu et al., Organic & Biomolecular Chemistry, 4(21): 3980-3985; 2006; Kosal et al., Angew. Chem. Int. Ed., 51, 12036-12040, 2012; and Heravi et al., Synthetic Communications, 40(15): 2191-2200; 2010; Chen et al., Journal of Heterocyclic Chemistry, (2007), 44(5), 1201-1205; Abdolmohammadi, Chinese Chemical Letters, 24(4), 318-320; 2013; Heravi et al., Synthetic Communications (2010), 40(15), 2191-2200; Quang et al., Bulletin of the Korean Chemical Society, (2012), 33(4), 1170-1176; Laengle et al., European J. Med. Chem, (2015), 95, 249-266, and Shirini et al., Dyes and Pigments (2013), 97, 19-25.

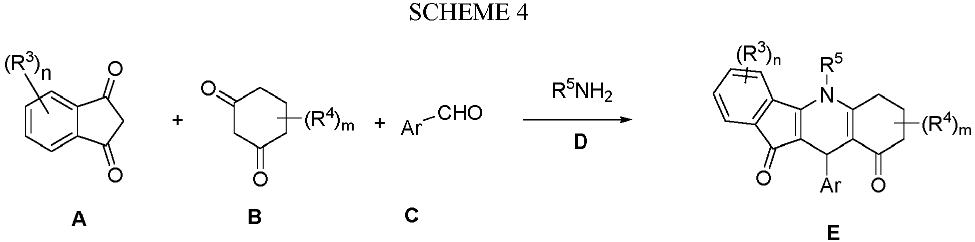

In the reactions described above, it may be necessary to protect reactive functional groups (*e.g*., hydroxy, amino, thio, or carboxy groups) to avoid their unwanted participation in the reactions. The incorporation of such groups, and the methods required to introduce and remove them are known to those skilled in the art (for example, Greene, *supra*). The deprotection step may be the final step in the synthesis such that the removal of protecting groups affords compounds of formula (I), (II), (III), (IV), (V), (Va), or (VI) as disclosed herein. Starting materials used in any of the schemes above can be purchased or prepared by methods described in the chemical literature, or by adaptations thereof, using methods known by those skilled in the art. The order in which the steps are performed can vary depending on the groups introduced and the reagents used, but would be apparent to those skilled in the art.

Compounds of any of formulas (I), (II), (III), (IV), (V), (Va), or (VI) or any of the intermediates described in the schemes above, can be further derivatized by using one or more standard synthetic methods known to those skilled in the art. Such methods can involve substitution, oxidation or reduction reactions. These methods can also be used to obtain or modify compounds of formula (I), (II), (III), (IV), (V), (Va), or (VI) or any preceding intermediates by modifying, introducing or removing appropriate functional groups. Particular substitution approaches include alkylation, arylation, heteroarylation, acylation, thioacylation, halogenation, sulphonylation, nitration, formylation, hydrolysis, and coupling procedures. These procedures can be used to introduce a functional group onto the parent molecule (*e.g.,* the nitration or sulphonylation of aromatic rings) or to couple two molecules together (for example to couple an amine to a carboxylic acid to afford an amide; or to form a carbon-carbon bond between two heterocycles). For example, alcohol or phenol groups can be converted to ether groups by coupling a phenol with an alcohol in a solvent, *e.g*., tetrahydrofuran in the presence of a phosphine (*e.g.,* triphenylphosphine) and a dehydrating agent (*e.g.,* diethyl-, diisopropyl-, or dimethylazodicarboxylate). Alternatively, ether groups can be prepared by deprotonation of an alcohol, using a suitable base (*e.g.*, sodium hydride) followed by the addition of an alkylating agent (*e.g.,* an alkyl halide or an alkylsulphonate).

In another example, a primary or secondary amine can be alkylated using a reductive alkylation process. For example, the amine can be treated with an aldehyde and a borohydride (*e.g.*, sodium triacetoxyborohydride, or sodium cyanoborohydride) in a solvent (*e.g.,* a halogenated hydrocarbon, for example, dichloromethane, or an alcohol, for example, ethanol) and, where necessary, in the presence of an acid (*e.g.,* acetic acid).

In another example, -OH groups may be generated from the corresponding ester, acid, acid chloride or aldehyde by reduction with a suitable reducing agent, *e.g.,* a complex metal hydride, *e.g.,* lithium aluminum hydride in a solvent (*e.g.,* tetrahydrofuran).

In another example, hydroxy groups (including phenolic OH groups) can be converted into leaving groups, *e.g.,* halogen atoms or sulphonyloxy groups (*e.g.,* alkylsulphonyloxy, *e.g.,* trifluoromethylsulphonyloxy, or arylsuphonyl, *e.g., p-*toluenesulphonyloxy) using conditions known to those skilled in the art. For example, an aliphatic alcohol can be reacted with thionyl chloride in a halogenated hydrocarbon (*e.g.*, dichloromethane) to afford the corresponding alkylchloride. A base (*e.g.*, triethylamine) can also be used in the reaction.

In another example, ester groups can be converted to the corresponding carboxylic acid by acid- or base-catalysed hydrolysis depending on the nature of the ester group. Acid catalysed hydrolysis can be achieved by treatment with an organic or inorganic acid (*e.g.,* trifluoroacetic acid in an aqueous solvent, or a mineral acid, *e.g.,* hydrochloric acid in a solvent, *e.g.*, dioxan). Base catalysed hydrolysis can be achieved by treatment with an alkali metal hydroxide (*e.g.,* lithium hydroxide in an aqueous alcohol, *e.g.,* methanol).

In another example, aromatic halogen substituents in the compounds may be subjected to halogen-metal exchange by treatment with a base (*e.g.,* a lithium base, *e.g.,* n-butyl or t-butyl lithium) optionally at a low temperature (*e.g.,* -78°C) in a solvent (*e.g.,* tetrahydrofuran) and the mixture may then quenched with an electrophile to introduce a desired substituent. Thus, for example, a formyl group can be introduced by using dimethylformamide as the electrophile. Aromatic halogen substituents can also be subjected to palladium catalysed reactions to introduce groups, *e.g*., carboxylic acids, esters, cyano, or amino substituents.

In another example, aromatic halogen substituents in the compounds may participate in a range of metal catalyzed reactions to introduce alternative functional groups, *e.g*., amines, amides, ethers, thiols, aryl groups, or heteroaryl groups.

Particular oxidation approaches include dehydrogenations and aromatization, and the addition of oxygen to certain functional groups. For example, aldehyde groups can be prepared by oxidation of the corresponding alcohol using conditions well known to those skilled in the art. For example, an alcohol can be treated with an oxidizing agent (*e.g.,* the Dess-Martin reagent) in a solvent (*e.g.,* a halogenated hydrocarbon, for example dichloromethane). Alternative oxidizing conditions can be used, *e.g*., treatment with oxalyl chloride and an activating amount of dimethylsulphoxide and subsequent quenching by the addition of an amine (*e.g.*, triethylamine). Such a reaction can be carried out in an appropriate solvent (*e.g.,* a halogenated hydrocarbon, for example dichloromethane) and under appropriate conditions (*e.g.,* cooling below room temperature, *e.g.,* to -78°C followed by warming to room temperature). In another example, sulphur atoms can be oxidized to the corresponding sulphoxide or sulphone using an oxidizing agent (*e.g.,* a peroxy acid, *e.g., 3-*chloroperoxybenzoic acid) in an inert solvent (*e.g.,* a halogenated hydrocarbon, *e.g.,* dichloromethane) at around ambient temperature.

Particular reduction approaches include the removal of oxygen atoms from particular functional groups, saturation (or partial saturation) of unsaturated compounds including aromatic rings. For example, primary alcohols can be generated from the corresponding ester or aldehyde by reduction, using a metal hydride (*e.g.*, lithium aluminium hydride or sodium borohydride in a solvent, *e.g*., methanol). Alternatively, -OH groups can be generated from the corresponding carboxylic acid by reduction, using a metal hydride (*e.g.,* lithium aluminium hydride in a solvent, *e.g.*, tetrahydrofuran). In another example, a nitro group may be reduced to an amine by catalytic hydrogenation in the presence of a metal catalyst (*e.g.,* palladium on a solid support, *e.g.,* carbon) in a solvent (*e.g.,* an ether, *e.g.,* tetrahydrofuran, or an alcohol, *e.g.,* methanol), or by chemical reduction using a metal (*e.g.,* tin or iron) in the presence of an acid (*e.g.*, hydrochloric acid). In a further example an amine can be obtained by reduction of a nitrile, *e.g.,* by catalytic hydrogenation in the presence of a metal catalyst (*e.g.,* palladium on a solid support, *e.g.,* carbon), or Raney nickel in a solvent (*e.g.,* tetrahydrofuran) and under suitable conditions (*e.g.,* cooling to below room temperature, *e.g.,* to -78°C, or heating, *e.g.,* to reflux).

### GPR174-interacting reference compounds

As described herein, in various cases, the disclosure features reference compounds that have been definitively predetermined to functionally interact with GPR174, comprise a structure according to Formula I, II, III, IV, V or VI (such as, for example compounds 1-59 in Table 1 or Formula (I), (II), (III), (IV), (V), (Va), or (VI)), and optionally further comprise a detectable moiety, also referred to herein as "reference compounds comprising a detectable moiety." GPR174-interacting reference compounds comprising a detectable moiety, such as, for example, a radioisotope, fluorescent tag, bioluminescent tag, chemiluminescent tag, photo-affinity label, and the like, can be prepared according to methods known in the art using conventional starting materials.

For example, in one case, the GPR174-interacting reference compound is labeled with, or synthesized under suitable conditions to incorporate a molecular tag(s) incorporating a radioisotope such as ¹²⁵I, ¹⁴C, ³H, ¹¹C, ¹⁸F, ^{99m}Tc, or another suitable radiometric label using methods known in the art. See for example, Seevers R. H and Counsell, R.E., "Radioiodination Techniques for Small Organic Molecules, Chem Rev 82:575-590, 1982; Voges R. et al., "Preparation of Compounds Labeled with Tritium and Carbon-14," John Wiley & Sons, 2009; Ametamey, S.M., et al., "Molecular Imaging with PET," Chem Rev 108:1501-1516, 2008; and Cheng Y. et al., J Med Chem, 55:2279-2286, 2012.

In another case, the GPR174-interacting reference compound is labeled with, or synthesized under suitable conditions to incorporate a photo-affinity label using methods known in the art. See for example, Spletstoser J.T. et al., J. Med. Chem. 47:6459-6465, 2004.

In another case, the GPR174-interacting reference compound is labeled with, or synthesized under suitable conditions to incorporate a fluorescent tag such as 6-carboxyflourescein (FAM), Alexa Fluor^{®} (Molecular Probes, Inc), Fluorescein isothiocyanate (FITC), and the like using methods known in the art. See for example, Goncalves, M.S., Chem Rev 109:190-212, 2009.

In another case, the GPR174-interacting reference compound is labeled with a bioluminescent or chemiluminescent tag, such as isoluminol, or acridinium ester, or an enzyme label such as horse radish peroxidase (HRP) or alkaline phosphatase (AP) for further amplified readouts using enhanced chemiluminescence (ECL) substrates, using methods known in the art. See for example, Barton V. et al., J Med Chem 53:4555-4559, 2010.

### E. GPR174 signaling pathway

As disclosed herein, the inventors have discovered that GPR174 modulates the Gs signaling pathway. Assignment of GPR174 to the Gs signaling pathway occurred after determining the basal activity of GPR174 in a panel of transcriptional reporter assays, followed by validation of this assignment in the presence and absence of small-molecule surrogate ligands for GPR174, which were identified in a separate screening assay by their ability to functionally interact with the membrane-bound GPR174 in a CRA. As explained in detail herein, the CRA is based on the observation that substituting an NLS for a particular sequence in a GPCR intracellular port effectively removes the modified and unbound receptor from the cell surface and that this effect is reversed by the presence of a receptor-specific ligand.

Briefly, to determine the basal activity of GPR174 in the Gs signaling pathway, GPR174 was overexpressed in mammalian tissue culture cells that also included an expression cassette with luciferase, a reporter protein, under the transcriptional control of one of five promoters. The promoters were a cAMP response element (CRE), NFAT, AP1, serum response element (SRE), and serum response factor (SRF). The Gs signaling pathway induces transcription from the CRE promoter. The Gq signaling pathway induces transcription from NFAT, AP1, SRE, CRE, and SRF promoters. The Gi signaling pathway can induce transcription from CRE in the presence of Gs chimeric G-proteins or induce transcription of the NFAT AP1, SRE, CRE and SRF promoters with G15, G16, or Gq chimeric G-proteins. The G12/13 signaling pathway induces transcription from the SRE and SRF promoters. Those of skill in the art will recognize that other reporters can be used in the assay, *e.g*., β-galactosidase or other detectable or assayable proteins.

As shown in the Examples below, it was determined that GPR174 modulates the Gs signaling pathway.

### Potency of GPR174 Interacting Compounds

In one case, a GPR174 inhibitor useful in the methods of the disclosure is a compound that inhibits a GPR174-mediated signaling pathway (*e.g.*, a Gs pathway) at least 0.5-fold (such as at least 1.5-fold, at least 2-fold, at least 5-fold, at least 10-fold, at least 50-fold, or at least 100-fold) in comparison to basal activity of the receptor itself at a compound concentration of less than 40 µM (such as less than 25, 10, 5, 1, 0.5, 0.25, 0.1, or less than 0.5 µM).

In some cases, the GPR174 modulator is an inhibitor of a GPR174 -mediated signaling pathway and decreases the activity of at least one GPR174-mediated signaling pathway (*e.g*., a Gs pathway) by at least 10% (such as at least 0.5-fold, at least 2-fold, at least 5-fold, at least 10-fold, at least 50-fold, or at least 100-fold) in comparison to basal activity of the receptor itself at a compound concentration of less than 40 µM (such as less than 25, 10, 5, 1, 0.5, 0.25, 0.1, or less than 0.5 µM).

In some cases, the GPR174 modulator is an inhibitor of a GPR174 -mediated signaling pathway and decreases the activity of at least one GPR174-mediated signaling pathway (*e.g*., a Gs pathway) in the presence of an agonist by at least 20% (such as at least 0.5-fold, at least 2-fold, at least 5-fold, at least 10-fold, at least 50-fold, or at least 100-fold) at an inhibitor concentration of less than 40 µM (such as less than 25, 10, 5, 1, 0.5, 0.25, 0.1, or less than 0.5 µM) in comparison to the activity of the receptor with an agonist and no inhibitor.

In some cases, the GPR174 modulator is an inhibitor of a GPR174 -mediated signaling pathway and decreases the activity of at least one GPR174-mediated signaling pathway (*e.g*., a Gs pathway) in the presence of an activator by at least 20% (such as at least 0.5-fold, at least 2-fold, at least 5-fold, at least 10-fold, at least 50-fold, or at least 100-fold) at an inhibitor concentration of less than 40 µM (such as less than 25, 10, 5, 1, 0.5, 0.25, 0.1, or less than 0.5 µM) in comparison to the activity of the receptor with an activator and no inhibitor.

An exemplary assay suitable for determining the activity of a GPR174 modulator (activator or inhibitor) in comparison to GPR174 basal activity is provided in Examples 3 and 4 herein, which describe the use of reporter assays for measuring GPR174-mediated signaling pathway activity (*e.g*., Gs pathway activity) in the absence (to determine basal activity) or in the presence of a GPR174 modulator compound.

### F. Screening methods for the identification of additional compounds

Now having identified compounds that inhibit GPR174 and the signaling pathways modulated by this receptor in accordance with the present disclosure, additional methods for identifying other GPR174 modulators can be employed.

In the case of orphan GPCRs wherein the natural ligand remains unknown, labeled surrogate ligands may be used as reference compounds as a means of measuring binding to the natural receptors and/or engineered receptor constructs so that competition (*i.e.,* competitive binding) or non-competitive binding with other receptor modulatory agents can be determined. The surrogate ligands (*e.g*., compounds 1-59 provided in Table 1) may bind to the natural binding site (orthosteric site) or they may bind to allosteric binding site(s) on the GPR174 receptor.

Examples of such screening methods for the identification of agents that modulate GPR174 signaling activity are described below.

As used herein, the term "candidate modulatory agent," in the context of the screening methods for identification of agents that modulate GPR174 activity, refers to any modulatory agent, including compounds as defined herein (*i.e.,* including molecules, either naturally occurring or synthetic, *e.g.,* protein; peptide (*e.g.,* from about 5 to about 25 amino acids in length, such as from about 10 to 20 or 12 to 18 amino acids in length, for example, 12, 15, or 18 amino acids in length); antibody, or fragment or mimetic thereof; aptamer, small molecule chemical compound, *e.g.*, small organic, organometallic, or inorganic molecule; polysaccharide; oligonucleotides; lipid; and fatty acid. The compound can be included in a library of compounds, such as a combinatorial, synthetic, natural, heterocyclic, drug-like, lead-like, organic, inorganic, unrandomized, or randomized library that provides a sufficient range of diversity or it may be a focused or targeted collection of the above compounds). Candidate modulatory agents also encompass natural extracts, nucleotides, nucleic acid molecules (including a library of nucleic acid molecules, such as a cDNA library encoding a modulatory agent), nucleotide analogues, nucleosides and nucleoside analogues, and gases (such as, for example, hydrogen sulfide (H₂S), or nitric oxide (NO)).

As used herein, the term "natural extract" denotes any extract that is obtained from a natural source, such as a eukaryotic cell (*e.g.,* a mammalian cell (including a mammalian cell line) or mammalian tissue), bacteria, animal, plant, fruit, tree, and the like.

In accordance with the foregoing, in one aspect the disclosure provides a method of identifying a modulatory agent capable of modulating a GPR174-mediated signaling pathway (*e.g*., a Gs pathway) comprising: (a) contacting a cell expressing GPR174 with (i) at least one candidate modulatory compound and (ii) a reference compound comprising a detectable moiety, wherein the reference compound (*i.e.,* a surrogate ligand) is known to modulate a GPR174-mediated signaling pathway activity, and (b) determining whether the candidate modulatory compound changes (*i.e.,* increases or decreases) the binding affinity of the reference compound to GPR174. In some cases, a candidate modulatory compound that decreases the binding affinity (*i.e.,* inhibits binding) of the reference compound to GPR174 is identified as a modulatory agent that is capable of modulating a GPR174-mediated signaling pathway. In some cases, a candidate compound that competitively decreases the binding affinity (*i.e.* competitively inhibits binding) of the reference compound to GPR174 is identified as a compound that binds to the same region of GPR174 as the reference compound and is capable of modulating a GPR174-mediated signaling pathway. In some cases, a candidate modulatory compound that increases the binding affinity (*i.e.*, enhances binding) of the reference compound to GPR174 is identified as a modulatory agent that acts as an allosteric modulatory agent that is capable of modulating a GPR174-mediated signaling pathway. In some cases, the candidate modulatory agent decreases the binding affinity of the reference compound to GPR174 by at least 2-fold. In some cases, the candidate modulatory agent increases the binding affinity of the reference compound to GPR174 by at least 2-fold. In some cases, the method further comprises performing a signaling assay to measure the effect of the identified compound on the GPR174-mediated signaling pathway, optionally in the presence of a reference compound known to modulate at least one GPR174-mediated signaling pathway.

In accordance with the foregoing, in one aspect the disclosure provides a method of identifying a modulatory agent capable of modulating a GPR174-mediated signaling pathway (*e.g.*, a Gs pathway) comprising: (a) contacting a cell expressing GPR174 with (i) at least one candidate modulatory compound and (ii) a reference compound comprising a detectable moiety, wherein the reference compound (*i.e.,* a surrogate ligand) is known to modulate a GPR174-mediated signaling pathway activity, and (b) determining whether the candidate modulatory compound changes (*i.e.,* increases or decreases) the the apparent binding affinity of the reference compound to GPR174. In some cases, a candidate modulatory compound that decreases the apparent binding affinity of the reference compound to GPR174 is identified as a modulatory agent that is capable of modulating a GPR174-mediated signaling pathway. In some cases, a candidate compound that competitively decreases the apparent binding affinity of the reference compound for GPR174 is identified as a compound that binds to the same region of GPR174 as the reference compound and is capable of modulating a GPR174-mediated signaling pathway. In some cases, a candidate modulatory compound that decreases the binding affinity of the reference compound for GPR174 is identified as a modulatory agent that acts as a negative allosteric modulatory agent that binds to an allosteric site on GPR174. In some cases, a candidate modulatory compound that increases the apparent binding affinity of the reference compound for GPR174 is identified as a modulatory agent that acts as a positive allosteric modulatory agent that is capable of modulating a GPR174-mediated signaling pathway. In some cases, the candidate modulatory agent decreases the apparent binding affinity of the reference compound for GPR174 by at least 2-fold. In some cases, the candidate modulatory agent increases the apparent binding affinity of the reference compound to GPR174 by at least 2-fold. In some cases, the method further comprises performing a signaling assay to measure the effect of the identified compound on the GPR174-mediated signaling pathway, optionally in the presence of a reference compound known to modulate at least one GPR174-mediated signaling pathway. In some cases, a candidate modulatory compound that decreases the apparent activity of GPR174-mediated signaling relative to the reference compound with GPR174 is identified as a modulatory agent that is capable of modulating a GPR174-mediated signaling pathway. In some cases, the candidate modulatory agent decreases the apparent activity of GPR174-mediated signaling with the reference compound by at least 10%. In some cases, a candidate modulatory compound that increases the apparent activity of GPR174-mediated signaling relative to the reference compound with GPR174 is identified as a modulatory agent that is capable of activating a GPR174-mediated signaling pathway. In some cases, the candidate modulatory agent increases the apparent activity of GPR174-mediated signaling with the reference compound by at least 20 %.

In some cases, the disclosure provides a method of identifying a modulatory agent capable of modulating a GPR174-mediated signaling pathway (*e.g*., a Gs pathway) comprising
(a) contacting a cell expressing GPR174 with
   (i) at least one candidate modulatory agent and
   (ii) a reference compound, wherein the reference compound is known to modulate a GPR174-mediated signaling pathway activity and
(b) comparing the GPR174-mediated signaling activity in the cell contacted with the candidate modulatory compound and the reference compound to the GPR174-mediated signaling activity in the cell contacted with a reference compound alone; wherein a difference (*i.e..,* at least a two-fold difference) in the signaling activity in the cell contacted with the combination of the reference compound indicates that the candidate modulatory compound is a compound that modulates GPR174-mediated signaling pathway activity.

In accordance with this aspect of the disclosure, the reference compound known to modulate at least one GPR174-mediated signaling pathway comprises a structure according to any of Formulas I, II, III, IV, V or VI (or (I), (II), (III), (IV), (V), (Va), or (VI)) disclosed herein. In one case, the reference compound known to modulate at least one GPR174-mediated signaling pathway is any of compounds 1-59 provided in Table 1.

The term "reference compound comprising a detectable moiety" refers to a reference compound that is modified to include, or is covalently linked to, any type of detectable moiety suitable for providing a readout of GPR174-bound reference compound in any of a wide variety of different binding assay formats well known by those of skill in the art including, but not limited to, the use of radioisotopes, fluorescence, fluorescence polarization (FP), fluorescence resonance energy transfer (FRET), bioluminescence, bioluminescence resonance energy transfer (BRET), chemiluminescence, photo-affinity label, time-resolved fluorometry (TRF), and the like. For example, in one case, the reference compound is labeled with a molecular tag(s) incorporating a radioisotope such as ¹²⁵I, ¹⁴C, ³H, ¹¹C, ¹⁸F, ^{99m}Tc, or another suitable radiometric label. In another case, the reference compound is labeled with a fluorescent tag such as 6-carboxyflourescein (FAM), Alexa Fluor^{®} (Molecular Probes, Inc), Fluorescein isothiocyanate (FITC), and the like. In another case, the reference compound is labeled with a bioluminescent or chemiluminescent tag, such as isoluminol, or acridinium ester, or an enzyme label such as horse radish peroxidase (HRP) or alkaline phosphatase (AP) for further amplified readouts using enhanced chemiluminescence (ECL) substrates. In yet another case, the reference compound is covalently linked to a high affinity binding molecule such as biotin to make use of various streptavidin-linked tags or enzymes as described above.

In accordance with one case of this aspect of the disclosure, a binding assay involves contacting a cell expressing GPR174, or a cell membrane derived therefrom, with at least one candidate modulatory compound and a reference compound comprising a detectable moiety. In general, cells expressing GPR174 are cultured using standard cell culture techniques to 80-90% confluence and then used to prepare whole cells, broken cell preparations or cell membrane preparations, which are subsequently frozen at -80° C. Alternatively, whole cells or cell preparations may be used fresh, without freezing. Cells or membrane preparations, thawed or used fresh, are incubated with an optimal concentration of reference compound (surrogate ligand) (*e.g.,* 0.01 to 10,000 nM) in the presence or absence of various concentrations of candidate competitor (*i.e.,* candidate modulatory agent), preferably in a multi-well format. After incubation in a suitable binding buffer for a suitable time (*i.e.,* 10 minutes to 2 hours) at a suitable temperature (*i.e.,* ~20-37° C), the cells are transferred to a multi-well filter plate and washed multiple times with cold wash buffer. The bound reference compound comprising the detectable moiety is measured with the appropriate instrument to provide a read-out of the binding affinity. Alternatively, bound reference compound may be separated from free compound by a variety of separation techniques, including, centrifugation, dialysis, resin binding, and various types of chromatography (affinity, thin layer, ion exchange, HPLC, FPLC, etc). When using homogenous assay technologies, separation the wash steps may be omitted. The amount of bound labeled reference compound is quantitated to detect dissociation of the labeled reference compound from GPR174 after incubation with the candidate modulatory agent(s) compared to control wells incubated in the absence of the candidate modulatory agent(s).

An excess of unlabeled reference compound (surrogate ligand) may be used to assess non-specific binding (NSB) in negative control wells (no competitor added) and positive control (no competitor added) wells are used to determine maximum signal from the tagged surrogate ligand. Percent activity is calculated as 100 x (Signal with competitor - NSB) / (Positive Control - NSB) for each competitor concentration used and IC₅₀ values (concentration of competitor yielding 50% activity) are generated using an appropriate curve fitting model, such as a 4- or 5-parameter logistic fit, Hill equation, or other equations based on saturation binding models of the data. In one case, the assays are configured so that the IC₅₀ values reflect dissociation constants for the inhibitory molecules (for example IC₅₀ = Kᵢ(1+[Reference Compound]/K_{D}) where [Reference Compound] << K_{D}). In cases where competitive inhibition is weak and an IC₅₀ value cannot be generated, values are reported as > the highest concentration tested. Compounds are considered "active competitors" when the % activity at the highest concentration tested is significantly less (P ≥ 95%, n=3) than 100% activity. In other cases, such as positive allosteric modulation, binding may be characterized by EC50s (half maximal effective concentrations) exceeding the binding of the reference compound alone due to enhanced affinity and/or accessibility of binding sites. In cases where EC50 values cannot be generated for positive modulators, compounds are considered "active positive modulators" when the % activity at the highest concentration tested is significantly greater (P≥95%, n=3) than 100%.

In one case, the modulatory agents identified are small molecule compounds that competitively bind the same region of GPR174 as the reference compound and display an appropriate dose curve when used to compete off a reference compound (surrogate ligand), with a resulting IC₅₀ <100 µM, or more preferably an IC₅₀ <10 µM.

As described herein, the present inventors have identified chemical compounds (*i.e.,* surrogate ligands) that functionally interact with GPR174 and inhibit one or more GPR174-mediated signaling pathways and, additionally, have characterized the GPR174 receptor signaling profile, which includes the Gs-signaling pathway. Now having identified compounds that modulate GPR174, these newly identified surrogate ligands for GPR174 can be used as reference compounds in the competitive binding assays described herein, using routine methods known by those of skill in the art, to identify additional compounds that bind to the precise region of interest on GPR174 for use in modulating GPR174-mediated signaling. As is well known in the art, binding assays are capable of detecting competitive, as well as non-competitive or uncompetitive inhibitors. These binding assays may also detect allosteric modulators which may stimulate or inhibit binding with additive or synergistic effects.

In accordance with the foregoing, in one case, the disclosure features a method of using a small molecule chemical compound for inhibiting a GPR174-mediated signaling pathway in a cell, said method comprising the steps of: (a) providing a small molecule chemical compound that functionally interacts with GPR174 and inhibits a GPR174-mediated signaling pathway (*e.g.*, a Gs pathway) in cells expressing GPR174, wherein said compound is characterized by at least one of the following criteria:
said compound has a structure selected from the group consisting of Formula I, II, III, IV, V or VI (or (I), (II), (III), (IV), (V), (Va), or (VI)); or
said compound changes the binding affinity of any one of reference compounds 1-59 (as set forth in Table 1) to GPR174; or
said compound causes a difference in the modulatory activity of any one of reference compounds 1-59 (as set forth in Table 1), in a GPR174-mediated signaling pathway assay when tested in the presence of said reference compound as compared to the modulatory activity of the reference compound alone; and
contacting a cell expressing GPR174 that comprises a GPR174-mediated signaling pathway with said compound according to step (a), thereby modulating a GPR174-mediated signaling pathway in said cell.

In some cases, the compound changes (*i.e.,* increases or decreases) the binding affinity of any one of reference compounds 1-59 to GPR174, in accordance with criteria (ii). In some cases, the compound decreases (*i.e.,* inhibits) the binding affinity of any one of reference compounds 1-59 to GPR174. In some cases, the compound competitively inhibits (*i.e.,* decreases the binding affinity) of at least one of reference compounds 1-59 to GPR174. In some cases, the compound increases the binding affinity (enhances binding) of any one of reference compounds 1-59 to GPR174.

In accordance with the foregoing, in one case, the disclosure features a method of using a small molecule chemical compound for inhibiting a GPR174-mediated signaling pathway in a cell, said method comprising the steps of: (a) providing a small molecule chemical compound that functionally interacts with GPR174 and inhibits a GPR174-mediated signaling pathway (*e.g*., a Gs pathway) in cells expressing GPR174, wherein said compound is characterized by at least one of the following criteria:
said compound has a structure selected from the group consisting of Formula I, II, III, IV, V, or VI (or (I), (II), (III), (IV), (V), (Va), or (VI)); or
said compound changes the apparent binding affinity of any one of reference compounds 1-59 (as set forth in Table 1) to GPR174; or
said compound causes a difference in the modulatory activity of any one of reference compounds 1-59 (as set forth in Table 1), in a GPR174-mediated signaling pathway assay when tested in the presence of said reference compound as compared to the modulatory activity of the reference compound alone; and
contacting a cell expressing GPR174 that comprises a GPR174-mediated signaling pathway with said compound according to step (a), thereby modulating a GPR174-mediated signaling pathway in said cell.

In some cases, the compound changes (*i.e.,* increases or decreases) the apparent binding affinity of any one of reference compounds 1-59 to GPR174, in accordance with criteria (ii). In some cases, the compound decreases the apparent binding affinity of any one of reference compounds 1-59 for GPR174. In some cases, the compound competitively binds to GPR174 (*i.e.,* has a higher binding affinity) relative to at least one of reference compounds 1-59 to GPR174. In some cases, the compound noncompetitively or uncompetitively binds to GPR174 (*i.e.,* binds at an allosteric site and decreases the apparent binding affinity) relative to at least one of reference compounds 1-59 to GPR174. In some cases, the compound increases the binding affinity (*i.e.,* enhances binding and is a positive allosteric modulator) of any one of reference compounds 1-59 to GPR174.

In another case, the disclosure features a compound comprising a detectable moiety, wherein the compound comprises a structure according to any of Formulas I, II, III, IV, V or VI (or (I), (II), (III), (IV), (V), (Va), or (VI)) disclosed herein and modulates a GPR174-mediated signaling pathway (*e.g*., a Gs pathway). In one case, the compound comprising a detectable moiety comprises a structure according to any of compounds 1-59 disclosed in Table 1. In one case, the detectable moiety is at least one of a radioisotope(s), a fluorescent tag, a chemiluminescent tag, a bioluminescent tag or a photo-affinity label. In one case, the reference compound comprises a radioisotope selected from the group consisting of ¹²⁵I, ¹⁴C, ³H, ¹¹C, ¹⁸F, and ^{99m}Tc. The compounds having a structure according to any of Formulas I, II, III, IV, V or VI (or (I), (II), (III), (IV), (V), (Va), or (VI)) and further comprising a detectable moiety (*e.g*., compounds 1-59 in Table 1) can be used in methods of identifying one or more modulatory agents capable of modulating a GPR174-mediated signaling pathway, and also can be used as diagnostic probes for detecting the presence or amount of the GPR174 receptor in a test sample *in vitro* or for use as molecular imaging probes *in vivo,* such as, for example, in Positron emission tomography (PET).

In accordance with the foregoing, in one aspect, the disclosure provides a method of identifying a modulatory agent capable of modulating a GPR174-mediated signaling pathway (*e.g.*, a Gs pathway) comprising: (a) contacting a cell with a candidate modulatory agent that is introduced to the cell, wherein said cell expresses GPR174 and comprises the signaling pathway, and (b) determining whether the candidate modulatory agent modulates at least one of a GPR174-mediated signaling pathway in comparison to a cell contacted with a control or in comparison to a reference standard. In one case, step (b) comprises performing an assay to detect at least one of the following: transcriptional reporter gene expression, GTPase activity, cAMP level, intracellular messenger level, calcium level, downstream kinase activity, transcriptional activation of downstream genes, change in cell morphology, change in cell growth rate, arachidonic acid release, or extracellular acidification rate. In one case, said candidate modulatory agent is selected from the group consisting of a compound, a nucleic acid, a natural extract, and a gas. In one case, said candidate modulatory agent is a chemical compound. In one case, said chemical compound comprises a structure according to any of Formulas I, II, III, IV, V or VI (or (I), (II), (III), (IV), (V), (Va), or (VI)) disclosed herein. For example, the methods according to this aspect of the disclosure can be carried out to evaluate the GPR174-modulatory activity of particular chemical compounds comprising a structure according to any of Formulas I, II, III, IV or V (or (I), (II), (III), (IV), (V), (Va), or (VI)) disclosed herein.

In one case, said chemical compound comprises a structure according to any of Formulas (I), (II), (III), (IV), (V), (Va), or (VI) disclosed herein. For example, the methods according to this aspect of the disclosure can be carried out to evaluate the GPR174-modulatory activity of particular chemical compounds comprising a structure according to any of Formulas (I), (II), (III), (IV), (V), (Va), or (VI) disclosed herein.

A candidate modulatory agent can modulate (*i.e.*, inhibit or activate) at least one of a GPR174-mediated pathway (*e.g*., a Gs pathway) in comparison to a cell contacted with a control or in comparison to a reference standard. The Gs signaling pathway activity in the presence of the candidate agent is compared to the activity of a control, which may be a cell contacted with an agent known to be a GPR174 modulatory agent, or a reference value obtained from a cell contacted with an agent known to be a GPR174 modulatory agent. Alternatively, the control may be the Gs signaling pathway activity in a cell in the absence of the candidate agent. The candidate modulatory agent employed in such an assay may either be free in solution, attached to a solid support, borne on a cell surface or located intracellularly, or associated with a portion of the cell.

In another case, the disclosure features a method of identifying a modulatory agent capable of modulating GPR174-mediated signaling pathway activity (*e.g.*, Gs pathway activity), comprising:
(a) contacting a cell expressing GPR174 with:
   (i) at least one candidate modulatory agent; and
   (ii) a reference chemical compound known to modulate GPR174-mediated signaling pathway activity;
(b) determining the GPR174-mediated signaling pathway activity level in the cell contacted in accordance with step (a); and
(c) comparing the GPR174-mediated signaling pathway activity level in a cell contacted with the reference chemical compound only with the GPR174-mediated signaling pathway activity determined in step (b); wherein a difference in GPR174-mediated signaling activity between the cell containing the candidate modulatory agent in the presence of the reference compound and the cell contacted with only the reference compound indicates that the candidate modulatory agent is capable of modulating GPR174 activity. In one case, the reference chemical compound comprises a structure according to any of Formulas (I), (II), (III), (IV), (V), (Va), or (VI) disclosed herein.

In one case, step (b) comprises performing an assay to detect at least one of the following: transcriptional reporter gene expression, GTPase activity, cAMP level, intracellular messenger level, calcium level, downstream kinase activity, transcriptional activation of downstream genes, change in cell morphology, change in cell growth rate, arachidonic acid release, or extracellular acidification rate. In one case, said candidate modulatory agent is selected from the group consisting of a compound, a nucleic acid, a natural extract, and a gas. In one case, said compound is a chemical compound.

The assays for use in accordance with the screening methods of the disclosure can measure the signaling output of a GPCR protein by detecting, *e.g.*, GTPase activity, intracellular messenger levels, downstream kinase activity, or transcriptional activation of downstream genes. The choice of assay will depend on the signaling pathway that is activated by the GPCR protein, and the needs of the user.

In general, samples or assays comprising GPR174 proteins are treated with a candidate modulatory agent, such as a candidate compound. These samples are then compared to control samples not treated with the candidate compound, and optionally treated with a control agent to examine the effect on GPR174 activity, in the case of agonists. Control samples (untreated with, *e.g.*, activators, inhibitors, agonists, inverse agonists, or antagonists) are assigned a relative protein activity value of 100%. Activity of an inhibitor, such as an antagonist or an inverse agonist, is typically determined in the presence of an agonist or ligand, such as the GPR174 agonists disclosed herein, or can be determined in overexpression studies. Inhibition of GPR174 is achieved when the activity value relative to the control is about 80%, preferably about 50%, and more preferably about 25-0%, depending on the conditions being tested. Activation of GPR174 is achieved when the activity value relative to the control (untreated with activators or agonists) is at least 110%, more preferably 150%, more preferably 200-500% (*i.e.,* two- to five-fold higher relative to the control), and more preferably 1000-3000% higher. In some cases, a reference standard is used in the methods, which may be a numerical value (*e.g.* a threshold value) determined by averaging a plurality of control samples (untreated with, *e.g.*, activators, inhibitors, agonists, inverse agonists, or antagonists).

### 1. Transcriptional reporter assay

Assayable reporter proteins, such as luciferase, provide a useful tool for assaying GPCR activity measured by a transcriptional reporter assay. Cells (*e.g.,* HEK293 cells, CHO cells, or COS 7 cells) are transiently co-transfected with both a GPR174 expression construct and a reporter construct that includes a cDNA for the reporter protein downstream from a transcription factor binding site, such as the cAMP-response element (CRE), AP-1, serum response element (SRE), or NFAT or serum response factor (SRF) binding sites. Agonist binding to receptors coupled to the Gs subtype of G-proteins leads to increases in cAMP, thereby activating the CRE transcription factor and resulting in expression of the reporter gene. Agonist binding to receptors coupled to the Gq subtype of G-protein leads to production of diacylglycerol that activates protein kinase C, which leads to the activation of SRE, AP-1 NFAT, SRF, and CRE transcription factors, in turn resulting in expression of the reporter gene. Gi activation can be detected through a CRE reporter system, if necessary, by first incubating cells with forskolin or the like to increase intracellular levels of cAMP. Gi expression can also be detected through the use of chimeric Gq and Gs proteins. G12/13 activation can be detected through the SRF and SRE reporter genes. Expression levels of the reporter protein reflect the activation status of the signaling events. See, *e.g.,* George et al., J. Biomol. Screen. 2:235-40 (1997) and Stratowa et al., Curr. Opin. Biotechnol. 6:574-81 (1995). Comparison is made to cells transfected with a GPR174 expression construct or a control expression construct to determine the extent of inhibition or activation of GPR174 activity.

For assessment of GPR174 activity, cells are transiently co-transfected with both a GPR174 expression construct and a reporter construct that includes a cDNA for the reporter protein downstream from transcription-factor binding sites, *i.e.,* SFE, NFAT, SRE, SRF, or CRE. Comparison is made to cells transfected with a GPR174 expression construct or a control expression construct to determine the extent of inhibition or activation of GPR174 activity.

### 2. Label-free biosensor measurement of GPCR activation

Changes in GPCR activity lead to changes in cell morphology that can be detected using biosensors. Such systems do not require labeling of either the GPCR protein or the test compound. Cell morphology can be detected using either measurement of impedance or optical signals. *See, e.g.,* Fang et al., Comb. Chem. High Throughput Screen. 11:357-68, 2008 and Peters et al., Assay Drug Dev. Technol. 8:219-27, 2010. Impedance measurement systems include, *e.g.,* the CellKey^{™} system (MDS Sciex, South San Francisco, CA). Optical signal measurement is provided by the Epic TM system (Corning, NY) or the BIND^{®} assay system (SRU Biosystems, Inc.). These systems require a ligand or surrogate ligand to detect activation of a specific GPCR protein. Cells that express a GPCR protein of interest, *i.e.,* GPR174, are contacted with a ligand or surrogate ligand and monitored for changes in impedence or optical signal. Comparison is made to cells that are not treated with a ligand or surrogate ligand.

### 3. Labeled ligand or surrogate ligand binding assay

Cells are assayed for their ability to bind specifically to a GPR174 ligand or surrogate ligand, *e.g.,* an inhibitor or activator. Cells that transiently or stably express GPR174 protein are grown to sub-confluence, harvested from flasks in PBS, and pelleted. Cell pellets are homogenized. The homogenate is centrifuged at 47,000 *g* for 15 minutes. The membrane pellet is resuspended in 1 mL tissue. An aliquot of the membrane preparation is used to determine protein concentration. For measurement of saturation binding, cell membranes are incubated with various concentrations of a labeled ligand or surrogate ligand. Non-specific binding is defined by the inclusion of unlabeled ligand or surrogate ligand. After the binding incubation, plates are harvested onto GF/C filters presoaked in 0.3% non-fat dry milk. Filters are dried and counted in a 96-well microplate scintillation counter. K_{d} values are calculated.

### 4. GTPγS binding assay

Because G-protein-coupled receptors such as GPR174 signal through intracellular G-proteins whose activity involves GTP binding and hydrolysis to yield bound GDP, measurement of binding of the non-hydrolyzable GTP analog [³⁵S]-GTPγS in the presence and absence of candidate inhibitors or activators provides another assay for GPR174 activity. See, *e.g.,* Kowal et al., Neuropharmacology 37:179-187 (1998). In one exemplary assay, cells stably transfected with a GPR174 expression vector are grown in 10-cm tissue culture dishes to sub confluence, rinsed once with 5 mL of ice-cold Ca²⁺/Mg²⁺-free phosphate-buffered saline, and scraped into 5 mL of the same buffer. Cells are pelleted by centrifugation (500 g, 5 minutes), resuspended in TEE buffer (25 mM Tris, pH 7.5, 5 mM EDTA, 5 mM EGTA), and frozen in liquid nitrogen. After thawing, the cells are homogenized using a Dounce homogenizer (1 mL TEE per plate of cells), and centrifuged at 1,000 g for 5 minutes to remove nuclei and unbroken cells.

The homogenate supernatant is centrifuged at 20,000 g for 20 minutes to isolate the membrane fraction, and the membrane pellet is washed once with TEE and resuspended in binding buffer (20 mM HEPES, pH 7.5, 150 mM NaCl, 10 mm MgCl₂, 1 mM EDTA). The resuspended membranes can be frozen in liquid nitrogen and stored at -70°C until use. Aliquots of cell membranes prepared as described above and stored at -70°C are thawed, homogenized, and diluted. Final homogenates are incubated with varying concentrations of candidate compounds or guanosine-5'- triphosphate (GTP) for 30 minutes at 30°C and then placed on ice. To each sample, guanosine 5'-0-(3[³⁵S thio)triphosphate (NEN, 1200 Ci/mmol; [³⁵S]-GTPγS) is added to a final concentration of 100-200 pM. Samples are incubated at 30°C for an additional 30 minutes, 1 mL of 10 mM HEPES, pH 7.4, 10 mM MgCl₂, at 4°C is added, and the reaction is stopped by filtration.

Samples are filtered over Whatman GF/B filters and the filters are washed with 20 mL ice-cold 10 mM HEPES, pH 7.4, 10 mM MgCl₂. Filters are counted by liquid scintillation spectroscopy. Nonspecific binding of [³⁵S]-GTPγS is measured in the presence of GTP and subtracted from the total. Comparison is made to untransfected control cells to determine the change in GPR174 activity.

### 5. Intracellular calcium measurement using FLIPR

Changes in intracellular calcium levels are another recognized indicator of G-protein-coupled receptor activity, and such assays can be employed to screen for modulators of GPR174 activity. In one particular example of such an assay, mammalian cells stably transfected with a GPR174 expression vector are plated at a density of 40,000 cells/well in 96-well plates specially designed to discriminate fluorescence signals emanating from the various wells on the plate. The cells are incubated for 60 minutes at 37°C in modified Dulbecco's PBS containing pyruvate and 1 g/L glucose with the addition of 1% fetal bovine serum and a calcium indicator dye, such as Fluo-3^{™} AM, Fluo-4^{™} AM, Calcium Green^{™}-1 AM, or Oregon Green^{™} BAPTA-1 AM. Plates are washed once with modified Dulbecco's PBS without 1% fetal bovine serum and incubated for 10 minutes at 37°C to remove residual dye from the cellular membrane. A calcium response is initiated by the addition of one or more candidate GPR174 inhibitors or activators, calcium ionophore A23187 (positive control), or ATP (positive control). Fluorescence is measured by Molecular Device's FLIPR with an argon laser (excitation 144 at 488 nm). See, *e.g.,* Kuntzweiler et al., Drug Dev. Res. 44:14-20 (1998). Comparison is made to untransfected control cells to determine the change in GPR174 activity.

### 6. Aequorin assays

The protein aequorin provides another method to measure intracellular calcium after activation of the Gq signaling pathway. In the presence of the cofactor coelenterazine, aequorin will emit a measurable luminescence that is proportional to the amount of intracellular (cytoplasmic) free calcium. See, *e.g.,* Cobbold et al. "Aequorin measurements of cytoplasmic free calcium," In: McCormack J. G. and Cobbold P. H., eds., Cellular Calcium: A Practical Approach. Oxford: IRL Press (1991); Stables et al., Anal. Biochem. 252:115-26 (1997); and Haugland, Handbook of Fluorescent Probes and Research Chemicals, Sixth edition. Eugene Ore.: Molecular Probes (1996).

In a typical assay, mammalian cells are transiently co-transfected with both a GPR174 expression construct and a construct that encodes the photoprotein apoaequorin. The cells are cultured for twenty-four hours at 37°C in, *e.g.,* MEM (Gibco/BRL, Gaithersburg, MD) supplemented with 10% fetal bovine serum, 2 mM glutainine, 10 U/mL penicillin and 10 µg/mL streptomycin, at which time the medium is changed to serum-free medium containing coelenterazine (Molecular Probes, Eugene, OR). Cells are incubated for two additional hours at 37°C. Subsequently, cells are detached from the plate washed, and resuspended at 200,000 cells/mL in serum-free MEM.

Inhibitors or activators of GPR174 are prepared in serum-free MEM and dispensed into wells of an opaque 96-well assay plate. Plates are then loaded onto an MLX microtiter plate luminometer (Dynex Technologies, Inc., Chantilly, VA). The instrument is programmed to dispense cell suspensions into each well, one well at a time, and immediately read luminescence for 15 seconds. Dose-response curves for the GPR174 inhibitors or activators are constructed using the area under the curve for each light signal peak. Data are analyzed and EC₅₀ values are obtained. Changes in luminescence caused by the compounds indicate changes in GPR174 activity.

### 7. Arachidonic acid release

The activation of GPCRs also has been observed to potentiate arachidonic acid release in cells, providing yet another useful assay for identification of inhibitors or activators of GPCR activity. See, *e.g.,* Kanterman et al., Mol. Pharmacol. 39:364-9 (1991). For example, CHO cells that are stably transfected with a GPR174 expression vector are plated in 24-well plates at a density of 15,000 cells/well and grown in MEM medium supplemented with 10% fetal bovine serum, 2 mM glutamine, 10 U/mL penicillin and streptomycin for 48 hours at 37°C before use. Cells of each well are labeled by incubation with [³H]-arachidonic acid (Amersham Corp., 210 Ci/mmol) for 2 hours at 37°C. The cells are then washed twice with 1 mL of buffer. Candidate compounds are added in 1 mL of the same buffer, either alone or with ATP and the cells are incubated at 37°C for 30 minutes. Buffer alone and mock-transfected cells are used as controls. Samples (0.5 mL) from each well are counted by liquid scintillation spectroscopy.

### 8. Extracellular acidification rate

In yet another assay, the effects of candidate inhibitors or activators of GPCR activity are assayed by monitoring extracellular changes in pH induced by the test compounds. See, *e.g.,* Dunlop et al., J. Pharmacol. Toxicol. Methods 40:47-55 (1998). In one case, cells transfected with a GPCR expression vector are seeded into 12-mm capsule cups (Molecular Devices Corp.) at 400,000 cells/cup in MEM supplemented with 10% fetal bovine serum, 2 mM L-glutamine, 10 U/mL penicillin, and 10 µg/mL streptomycin. The cells are incubated in this medium at 37°C in 5% CO₂ for 24 hours. Extracellular acidification rates are measured using a Cytosensor microphysiometer (Molecular Devices Corp.). Candidate agonists or other agents are diluted into the running buffer and perfused through a second fluid path. The pH of the running buffer in the sensor chamber is recorded during the cycle from 43-58 seconds, and the pump is re-started at 60 seconds to start the next cycle. The rate of acidification of the running buffer during the recording time is calculated by the Cytosoft program. Changes in the rate of acidification are calculated by subtracting the baseline value (the average of four rate measurements immediately before addition of a candidate compound) from the highest rate measurement obtained after addition of a candidate compound.

### 9. cAMP assays

In one type of assay, levels of cyclic adenosine monophosphate (cAMP) are measured in cells transfected with a GPCR expression vector. The GPCR-expressing cells are exposed to candidate compounds. Protocols for cAMP assays have been described in the literature. See, *e.g.,* Sutherland et al., Circulation 37:279-306, 1968; Frandsen et al., Life Sci. 18:529-41, 1976; Dooley et al., J. Pharmacol. Exp. Ther. 283:735-41, 1997; and George et al., J. Biomol. Screen. 2:235-40, 1997. Additional protocols that describe cAMP assays include: Hill et al., Br. J. Pharmacol. 161(6): 1266-1275, 2010; Smith et al., Appl. Biochem. Biotechnol. 41(3):189-218, 1993; and Berrera et al., Handb. Exp. Pharmacol. (186):285-98, 2008. An exemplary protocol for such an assay, using an Adenylyl Cyclase Activation FlashPlate^{®} Assay from NEN^{®} Life Science Products, is set forth below.

Briefly, the GPCR coding sequence (*e.g.,* a cDNA or intronless genomic DNA) is subcloned into an expression vector and transiently transfected into a host cell line, *e.g.,* Chinese Hamster Ovary (CHO) cells, using known methods. Transfected CHO cells are seeded into 96-well microplates from the FlashPlate^{®} microplates (which are coated with solid scintillant to which antisera to cAMP has been bound). For a control, some wells are seeded with wild-type (untransfected) CHO cells. Other wells in the plate receive various amounts of a cAMP standard solution for use in creating a standard curve.

One or more test compounds (*i.e.,* candidate inhibitors or activators) are added to the cells in each well, with water and/or compound-free medium/diluent serving as a control or controls. After treatment, cAMP is allowed to accumulate in the cells for exactly 15 minutes at room temperature. The assay is terminated by the addition of lysis buffer containing labeled cAMP, and the plate is counted using a Packard Topcount^{®} 96-well microplate scintillation counter. Unlabeled cAMP from the lysed cells (or from standards) and fixed amounts of cAMP compete for antibody bound to the plate. A standard curve is constructed, and cAMP values for the unknowns are obtained by interpolation. Changes in intracellular cAMP levels of cells in response to exposure to a test compound are indicative of GPCR inhibitory or activating activity. Other assays for detecting cAMP levels can be used, *e.g*., detection of labeled cAMP.

Additionally, many other kits to measure cAMP levels are commercially available, *e.g*., the cAMP Direct Immunoassay Detection Kit from Abcam (Cat. No. ab138880), the LANCE^{®} cAMP Detection Kit from Perkin Elmer, the Direct cAMP ELISA kit from Enzo Life Sciences, Inc., and the CatchPoint^{®} cAMP Fluorescent Assay Kit from Molecular Devices.

In some cases, the cAMP level is decreased by at least 20%, at least 50%, or at least 75% in a cell expressing GPR174 contacted with a small molecule inhibitor of GPR174 signaling as compared to a control cell expressing GPR174 not contacted with the small molecule inhibitor.

### 10. Expression of GPR174 protein

Recombinant GPR174 polypeptides, either full-length or fragments thereof, may be produced using standard techniques known in the art. These proteins can then be used in the assays described herein. In particular, such recombinant GPR174 polypeptides are, for example, useful in *in vitro* assays for identifying inhibitors or activators of GPR174. Recombinant production can take place in host cells or in cell-free translation systems, as is known in the art.

Typical fusion expression vectors include pGEX (Pharmacia Biotech Inc; Smith et al., Gene 67:31-40, 1988), pMAL (New England Biolabs, Ipswich, MA), pRIT5 (Pharmacia, Piscataway, N.J.), and pFUSE (Invivogen, San Diego, CA) which fuse glutathione S-transferase (GST), maltose E binding-protein, protein A, or IgG Fc, respectively, to the target recombinant protein.

If a GPR174 polypeptide is to be expressed for use in screening assays, it may be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Recombinant polypeptides (or alternatively, GPR174 polypeptides isolated from an organism) may be targeted to the cell membrane. Membrane-bound GPR174 can be prepared by expressing GPR174 in a suitable cell or cell line, *e.g., Pichia pastoris* cells, oocytes, or COS cells. Membranes containing the recombinant polypeptide may then be isolated from other cellular components by standard methods known in the art.

### 11. Kinase activity assays

In another type of assay, the activity of kinases downstream from GPR174 in a signaling pathway, *e.g*., the Gs signaling pathway, may be measured. Kinases downstream from GPR174 include, but are not limited to PKA, and the activity of one or more of the kinases downstream of GPR174 may be modulated in the presence of a GPR174 inhibitor. For example, the activity of protein kinase A (PKA) can be used to determine changes in the GPR174-mediated Gs signaling pathway as cAMP, the level of which is modulated by Gs signaling, activates PKA. Protocols for PKA assays have been described in the literature. See, *e.g.,* Karege et al., Brain Res. 903(1-2):86-93, 2001. Many kits are also commercially available to measure PKA activity including the cPKA Kinase Activity Assay Kit from Abcam (Cat. No. ab139435), the PKA Colorimetric Activity Kit from Life Technologies Corporation, and the PKA kinase activity kit from Enzo Life Sciences, Inc. These and similar assays can be used, for example, to compare a difference in activity of PKA between cells transfected with a GPCR expression vector and exposed to a small molecule inhibitor of GPR174 signaling and cells transfected with a GPCR expression vector, but not exposed to a small molecule inhibitor of GPR174 signaling.

In some cases, the PKA activity is decreased by at least 20%, at least 50%, or at least 75% in a cell expressing GPR174 contacted with a small molecule inhibitor of GPR174 signaling as compared to a control cell expressing GPR174 not contacted with the small molecule inhibitor.

### 12. Cytokine production assays

The level of cytokines such as IL-2 can be modulated by cAMP levels and Gs signaling. Assays that measure the level of one or more cytokines may be used to determine changes in the GPR174-mediated Gs signaling pathway. The level of one or more cytokines can be measured by an ELISA sandwich immunoassay. For example, IL-2 and IFN-γ can be measured using ELISA kits from ThermoFisher Scientific. The level of one or more cytokines can also be measured using bead-based immunoassays that utilize beads specific for different cytokines, which are differentiated by size and by fluorescent intensities using flow cytometry. Bead-based assay kits are also commercially available, *e.g*., the LEGENDplex^{™} Human Th Cytokine Panel from BioLegend allows simultaneous measurement of IL-2, IL-4, IL-5, IL-6, IL-9, IL-10, IL-12, IL-13, IL-17A, IL-17F, IL-21, IL-22, GM-CSF, IFN-γ and TNF-α. Alternatively, these cytokines can also be detected in supernatants with the MSD (MesoScale) platform.

Intracellular cytokine levels can also be determined. For example, peripheral blood mononuclear cells (PBMCs) can be treated with BD GolgiStop (BD Biosciences) reagent to block vesicular traffic and to cause accumulation of intracellular cytokine-filled secretory vesicles. These cells can then be stained with fluorescent antibodies for cell type-specific surface markers and antibodies against specific cytokines (*e.g.*, those from BioLegend), such as IL-2, IL-10, GM-CSF, IFN-γ and TNF-α. Stained cells can be analyzed by flow cytometry to determine the level of one or more cytokines.

These and similar assays can be used, as another example, to compare a difference in the level of one or more cytokines between a population of PBMCs exposed to a small molecule inhibitor of GPR174 signaling and a population of PBMCs, but not exposed to a small molecule inhibitor of GPR174 signaling.

In some cases, the level of one or more cytokines is decreased by at least 20%, at least 50%, or at least 80% in in a population of PBMCs exposed to a small molecule inhibitor of GPR174 signaling as compared to a population of PBMCs not exposed to the small molecule inhibitor of GPR174 signaling. In some cases, the level of IL-17A is decreased by at least 20%, at least 50%, or at least 80% in a population of PBMCs exposed to a small molecule inhibitor of GPR174 signaling as compared to a population of PBMCs not exposed to the small molecule inhibitor of GPR174 signaling.

In some cases, the level of one or more cytokines is increased by at least 20%, at least 50%, or at least 80% in a population of PBMCs exposed to a small molecule inhibitor of GPR174 signaling either as a single agent or in combination with at least one of an A2aR antagonist, an A2bR antagonist, a CD38 inhibitor, CD39 inhibitor and/or a CD73 inhibitor as compared to a population of PBMCs not exposed to the small molecule inhibitor of GPR174 signaling. In some cases, the cytokine is selected from IL-2, IFN-γ, TNF, and GM-CSF.

In some cases, the level of one or more cytokines is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a population of PBMCs not exposed to the small molecule inhibitor of GPR174 signaling. In some cases, the cytokine is selected from IL-2, IFN-γ, TNF, GM-CSF, IL-6, IL-12, IL-17A, and IL-10. In some cases, the level of one or more of IL-2, IFN-γ, TNF, or GM-CSF is increased by at least 20%, at least 50%, at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a population of PBMCs not exposed to the small molecule inhibitor of GPR174 signaling.

In some cases, the level of IL-2 is increased by at least 100%, at least 500%, or at least 1000% in a population of PBMCs exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a population of PBMCs not exposed to the small molecule inhibitor of GPR174 signaling. In some cases, the level of IFN-γ is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a population of PBMCs not exposed to the small molecule inhibitor of GPR174 signaling. In some cases, the level of TNF-α is increased by at least 50%, at least 100%, or at least 500% in a population of PBMCs exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a population of PBMCs not exposed to the small molecule inhibitor of GPR174 signaling.

In some cases, the level of one or more cytokines are not modulated and do not increase or decrease by more than 20% in a population of PBMCs exposed to a small molecule inhibitor of GPR174 signaling as compared to a population of PBMCs not exposed to the small molecule inhibitor of GPR174 signaling. In some cases, the cytokine or cytokines is selected from the group consisting of: IL-4, IL-5, IL-9, IL-13, IL-17F, IL-21, and IL-22.

Additionally, these and similar assays can be used, for example, to compare a difference in the level of one or more cytokines between cells expressing GPR174 and exposed to a small molecule inhibitor of GPR174 signaling either as a single agent or in combination with at least one of an A2aR antagonist, an A2bR antagonist, a CD38 inhibitor, a CD39 inhibitor and/or a CD73 inhibitor and cells expressing GPR174, but not exposed to a small molecule inhibitor of GPR174 signaling.

In some cases, the production of one or more cytokines is decreased by at least 20%, at least 50%, or at least 80% in a cell expressing GPR174 contacted with the small molecule inhibitor as compared to a control cell expressing GPR174 not contacted with the small molecule inhibitor. In some cases, the production of IL-17A is decreased by at least 20%, at least 50%, or at least 80% in a cell expressing GPR174 contacted with the small molecule inhibitor as compared to a control cell expressing GPR174 not contacted with the small molecule inhibitor

In some cases, the production of one or more cytokines is increased by at least 20%, at least 50%, at least 100%, at least 500%, or at least 1000% in a cell expressing GPR174 contacted with the small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a control cell expressing GPR174 not contacted with the small molecule inhibitor of GPR174. In some cases, the production of one or more of IL-2, IFN-γ, TNF, or GM-CSF is increased by at least 20%, at least 50%, at least 100%, at least 500%, or at least 1000% in a cell expressing GPR174 contacted with the small molecule inhibitor of GPR174 either as a single agent or in combination with at least one of an A2aR antagonist, an A2bR antagonist, a CD38 inhibitor, a CD39 inhibitor and/or a CD73 inhibitor as compared to a control cell expressing GPR174 not contacted with the small molecule inhibitor of GPR174.

In some cases, the production of IL-2 is increased by at least 100%, at least 500%, or at least 1000% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a control cell expressing GPR174 not contacted with the small molecule inhibitor of GPR174. In some cases, the production of IFN-γ is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a control cell expressing GPR174 not contacted with the small molecule inhibitor of GPR174. In some cases, the production of TNF-α is increased by at least 50%, at least 100%, or at least 500% in a cell expressing GPR174 contacted with a small molecule inhibitor of GPR174 signaling either as a single agent or in combination with at least one of an A2aR antagonist, an A2bR antagonist, a CD38 inhibitor, a CD39 inhibitor and/or a CD73 inhibitor as compared to a control cell expressing GPR174 not contacted with the small molecule inhibitor of GPR174.

In some cases, the production of one or more or IL-4, IL-5, IL-9, IL-13, IL-17F, IL-21, and IL-22 are not modulated and do not increase or decrease by more than 20% in a cell expressing GPR174 contacted with a small molecule inhibitor of GPR174 signaling as compared to a control cell expressing GPR174 not contacted with the small molecule inhibitor.

### 13. Receptor internalization assays

GPCR-agonist interaction in a context of a living cell can activate G proteins and their respective downstream signaling cascades as well as recruit β-arrestins that can engage additional signaling and induce receptor internalization (see, *e.g*., Paterson et al. Pharmacol Rev. 69(3): 256-297, 2017). There are many assays that utilize arrestin recruitment and receptor internalization to assess agonist-receptor interaction or antagonist inhibition of agonist-receptor interaction. There are numerous versions of both assays including some that are commercially available. For example, Discoverx provides both β-arrestin engagement and receptor internalization assays, and ThermoFisher Scientific and Molecular Devices supply the Tango GPCR assay and the TransFluor technology, respectively, for quantitation of β-arrestin recruitment.

In some cases, the receptor internalization is reduced by at least 10%, at least 20%, at least 50%, or at least 80% in a cell expressing GPR174 contacted with a small molecule inhibitor of GPR174 signaling as compared to a control cell expressing GPR174 not contacted with the small molecule inhibitor. In some cases, the receptor internalization is reduced by 1-99% in a cell expressing GPR174 contacted with a small molecule inhibitor of GPR174 signaling as compared to a control cell expressing GPR174 not contacted with the small molecule inhibitor. In some cases, the receptor internalization is reduced by 1-20%, by 20-40%, 40-60%, 60-80%, or by 80-99% in a cell expressing GPR174 contacted with a small molecule inhibitor of GPR174 signaling as compared to a control cell expressing GPR174 not contacted with the small molecule inhibitor.

In some cases, the β-arrestin recruitment is reduced by at least 10%, at least 20%, at least 50%, or at least 80% in a cell expressing GPR174 contacted with a small molecule inhibitor of GPR174 signaling as compared to a control cell expressing GPR174 not contacted with the small molecule inhibitor. In some cases, the β-arrestin recruitment is reduced by 1-99% in a cell expressing GPR174 contacted with a small molecule inhibitor of GPR174 signaling as compared to a control cell expressing GPR174 not contacted with the small molecule inhibitor. In some cases, the β-arrestin recruitment is reduced by 1-20%, by 20-40%, 40-60%, 60-80%, or by 80-99% in a cell expressing GPR174 contacted with a small molecule inhibitor of GPR174 signaling as compared to a control cell expressing GPR174 not contacted with the small molecule inhibitor.

### G. Compounds

The compounds used in the present disclosure include small molecules. Such can be identified using screening assays (*e.g*., those described herein). Particular types of compounds, as well as methods of generating such compounds, are described below.

### 1. Small molecules

Additional inhibitors include large or small inorganic, organometallic or organic molecules. In certain cases, the inhibitor is a small organic molecule, or a derivative or analog thereof. Such small molecules preferably have a molecular weight below 2,000 Daltons, *e.g.*, between 200 and 1,000 Daltons or between 400 and 700 Daltons. It is preferred that these small molecules be organic molecules. Examples of small molecules that inhibit GPR174 activity are described above.

In certain cases, an inhibitor includes a protecting group. The term "protecting group" refers to chemical moieties that block at least some reactive moieties and prevent such groups from participating in chemical reactions until the protective group is removed (or "cleaved"). Examples of blocking/protecting groups are described, *e.g.,* in Greene and Wuts, Protective Groups in Organic Synthesis, 3rd Ed., John Wiley & Sons, New York, NY, 1999.

Any of the inhibitors may possess one or more chiral centers, and each center may exist in the R or S configuration. Inhibitors of the present disclosure include all diastereomeric, enantiomeric, and epimeric forms as well as mixtures thereof. Stereoisomers may be obtained, if desired, by methods known in the art as, for example, the separation of stereoisomers by chiral chromatographic columns. Inhibitors further include N-oxides, crystalline forms (also known as polymorphs), and pharmaceutically acceptable salts, as well as active metabolites of any inhibitor or activator. All tautomers are included within the scope of the inhibitors or activators presented herein. In addition, the inhibitors described herein can exist in unsolvated as well as solvated forms with pharmaceutically acceptable solvents such as water, ethanol, and the like. The solvated forms of the inhibitors presented herein are also included within the present disclosure. The inhibitors may include isotopically labeled compounds. Useful isotopes include hydrogen, carbon, nitrogen, oxygen, phosphorous, fluorine, and chlorine, (*e.g.,* ²H, ³H, ¹³C, ¹⁴C, ¹⁵N, ¹⁸O, ¹⁷O, ³¹P, ³²P, ³⁵S, ¹⁸F, and ³⁶Cl). Isotopically-labeled compounds can be prepared by synthesizing a compound using a readily available isotopically-labeled reagent in place of a non-isotopically-labeled reagent.

In a particular case, a small molecule inhibitor binds to GPR174. The small molecule may bind to the extracellular or transmembrane region of GPR174 and interfere with or reduce ligand binding to GPR174, thus acting as an inhibitor. In a further case, a small molecule inhibitor binds to the extracellular region of GPR174 and acts as an antagonist to inhibit GPR174 activity, *i.e.,* signaling the associated G-protein regulated signaling pathway. In another case, the small molecule is a small organic molecule that inhibits GPR174 activity, *i.e.,* signaling the associated G-protein regulated signaling pathway. Other mechanisms of action, such as positive or negative allosteric modulation, are included as well.

### 2. Polypeptides and polynucleotides

In certain cases, methods of the disclosure are practiced using peptide or polypeptide modulators of GPR174. Peptides and polypeptides may be readily synthesized or produced recombinantly using routine methods known and available in the art. For example, polynucleotides can be used as a tool to express a polypeptide in an appropriate cell. Methods well known to those skilled in the art may be used to construct expression vectors containing sequences encoding a polynucleotide or polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F.M. et al. (1989) Current Protocols in Molecular Biology, John Wiley & Sons, New York. N.Y.

### H. Therapeutic methods

Based on our identification of GPR174 receptor inhibitors, coupled with our discovery of the signal transduction pathway of GPR174, methods for inhibiting GPR174 and its associated G-protein regulated signaling pathways are described herein; wherein a GPR174 inhibitory compound is used as a single agent or in combination with at least one of an A2aR antagonist, an A2bR antagonist, a CD38 inhibitor, a CD39 inhibitor and/or a CD73 inhibitor. The methods can be used to increase or decrease signal transduction in a cell *in vitro, ex vivo,* or *in vivo* (*e.g.,* in a mammalian cell such as a human cell). In certain cases, the compound is administered to a subject having a disease that is associated with GPR174 activity to reduce or to improve the symptoms of the disease or the underlying disease pathology. In other cases, the compound is administered to a subject at risk of developing a disease that is associated with GPR174 activity to reduce the risk of developing the disease. In *ex vivo* applications, cells are contacted with the compound outside the subject. These cells are then transplanted back into the subject. Medical conditions that are associated with GPR174 activity or in which GPR174 can play a role in the disorder and/or its treatment include cancer.

As further described herein, the inventors have demonstrated that the combined inhibition of GPR174 and inhibition of the A2aR and/or the A2bR, or a combination of a GPR174 inhibitor and an inhibitor of an enzymatic pathway involved in the production of adenosine, such as CD38, CD39 and CD73 (such as a CD73 inhibitor and/or a CD38 inhibitor and/or a CD39 inhibitor) results in synergistic induction of IFN-γ, IL-2, TNF and GM-CSF production in human PBMCs, therefore, in certain cases, methods are provided for treating cancer by administering to a subject a therapeutically effective amount of a GPR174 inhibitor and an inhibitor of ATP-Adenosine-A2aR-and/or A2bRmediated signaling (such as an A2aR antagonist and/or an A2bR antagonist and/or a CD73 inhibitor and/or a CD38 inhibitor and/or a CD39 inhibitor) thereby stimulating an immune response in said subject.

In some cases, the cancer is selected from the group consisting of breast cancer, melanoma, colon cancer, urological cancer, lung cancer, small cell and non-small cell lung cancer, relapsed or refractory malignancies, non-Hodgkin and Hodgkin lymphoma, lymphoma, follicular lymphoma, lymphocytic lymphoma, CNS lymphoma, T-cell lymphoma, AIDS-related lymphoma, acute lymphoblastic leukemia, gastrointestinal cancers, liver cancer, hepatocellular carcinoma, ovarian cancer, pancreatic cancer, bile duct cancer, prostate cancer, renal carcinoma, bladder cancer, colorectal cancer, multiple myeloma, mesothelioma, cervical cancer, vaginal cancer, anal cancer, oropharyngeal cancer, myelogenous leukemia, gastric cancer, nasopharyngeal carcinoma, head and neck carcinoma, glioblastoma, gliosarcoma, squamous cell brain cancer, malignant glioma, diffuse pontine gliomas, esophageal cancer, thyroid cancer, astrocytoma, thoracic cancer, endometrial cancer, cutaneous cell carcinoma, leukemia, acinar cell carcinoma, adenocarcinoma, bronchioloalveolar carcinoma, cholangiocarcinoma, chordoma, giant cell carcinoma, intestinal carcinoma, major salivary gland carcinoma, malignant odontogenic neoplasm, malignant peripheral nerve sheath tumor, skin cancer, testicular cancer, germ cell tumor, neuroendocrine carcinoma, parathyroid carcinoma, pituitary gland carcinoma, placental choriocarcinoma, scrotal cancer, tracheal carcinoma, transitional cell carcinoma, cancer of the uterus, vulvar cancer, kidney cancer, rectum cancer, fallopian tube carcinoma, peritoneal carcinoma, epithelial cancer, pleural mesothelioma, sarcomatoid carcinoma, synovial sarcoma, nephroblastoma, neuroblastoma, adult acute myeloid leukemia, myelodysplastic/myeloproliferative neoplasm, embryonal carcinoma, Kaposi sarcoma, bone cancer, uterine cancer, stomach cancer, carcinoma of the endometrium, cancer of the small intestine, cancer of the endocrine system, cancer of the paragland, cancer of the adrenal gland, sarcoma of soft tissue, cancer of the urethra, cancer of the penis, cancer of the ureter, carcinoma of the pelvis, neoplasm of the central nervous system, primary tumor angiogenesis, spinal axis tumor, epidermoid cancer, environmentally induced cancers including those induced by asbestos, adenosarcoma, adenosquamous carcinoma, adrenocortical carcinoma, astrocytic tumors, basal cell carcinoma, chondosarcoma, Ewing's sarcoma, gallbladder cancer, hypopharyngeal cancer, intraocular melanoma, laryngeal cancer, leiomyosarcoma, lip and oral cavity cancer, malignant mesothelial tumors, malignant thymoma, medulloblastoma, medulloepithelioma, Merkel cell carcinoma, mucoepidermoid carcinoma, myelodysplastic syndrome, nasal cavity and paranasal sinus cancer, osteosarcoma, pulmonary blastoma, pineal and supratentorial primitive neuroectodermal tumors, plasma cell neoplasm, retinoblastoma, rhabdomyosarcoma, sarcoma, neuroectodermal tumors and Wilm's tumor.

In some cases, the subject suffering from cancer has one or more tumors infiltrated with regulatory T cells, such as, for example, breast, lung (such as small-cell lung cancer or non-small cell lung cancer), colorectal, cervical, renal, ovarian, melanoma, pancreatic, hepatocellular, gastric, glioblastoma, glioma, bladder, myeloma (such as multiple myeloma), prostate, thyroid, testicular, and esophageal cancer.

In some cases, the subject suffering from cancer is resistant to checkpoint inhibitors, such as anti-PD-1 (*e.g.*, Keytruda^{®} and Opdivo^{®}) and anti-CTLA-4 (Yervoy^{®}), and/or to cellular therapies such at CAR-T cells and adoptive T cell therapy. Checkpoint inhibitors are only effective in a minority of patients, and high levels of adenosine-generating molecules have been observed in non-responding patients. Furthermore, overcoming natural immunosuppression in solid tumors represents a major hurdle for cellular therapies. As PS and adenosine are both products of cell stress and death in solid tumors, it is expected that these patients resistant to checkpoint inhibitors and/or cellular therapies would benefit greatly from the combined inhibition of the GPR174 and adenosine pathways.

In some cases, the method further includes administering one or more additional therapeutic agents. In certain cases, the cancer cell is further contacted with a chemotherapeutic agent.

### 2. Administration and dosage

The compounds (in the form of their compositions) are administered to patients by the usual means known in the art, for example, by injection, oral, intravenous, subcutaneous, intraperitoneal, intramuscular, infusion, infiltration, irrigation, intra-articular, inhalation, subcutaneous, topical, rectal, vaginal, cutaneous, nasal, transdermal, or ocular administration and the like. For administration by injection and/or infiltration or infusion, the compositions or formulations according to the disclosure may be suspended or dissolved as known in the art in a vehicle suitable for injection and/or infiltration or infusion. Such vehicles include isotonic saline, buffered or unbuffered and the like. Depending on the intended use, they also may contain other ingredients, including other active ingredients, such as isotonicity agents, sodium chloride, pH modifiers, colorants, preservatives, antibodies, enzymes, antibiotics, antifungals, antivirals, other anti-infective agents, and/or diagnostic aids such as radio-opaque dyes, radiolabeled agents, and the like, as known in the art. However, the compositions of this disclosure may comprise a simple solution or suspension of a compound or a pharmaceutically acceptable salt of a compound, in distilled water or saline.

Alternatively, the therapeutic compounds may be delivered by other means such as intranasally, by inhalation, or in the form of liposomes, nanocapsules, vesicles, and the like. Compositions for intranasal administration usually take the form of drops, sprays containing liquid forms (solutions, suspensions, emulsions, liposomes, etc.) of the active compounds. Administration by inhalation generally involves formation of vapors, mists, dry powders or aerosols, and again may include solutions, suspensions, emulsions and the like containing the active therapeutic agents.

Routes and frequency of administration of the therapeutic compositions described herein, as well as dosage, will vary from individual to individual, and may be readily established using standard techniques. Preferably, between 1 and 100 doses may be administered over a 52-week period. A suitable dose is an amount of a compound that, when administered as described above, is capable of killing or slowing the growth of, cancers or cancer cells.

In general, an appropriate dosage and treatment regimen provides the active compound(s) in an amount sufficient to provide therapeutic and/or prophylactic benefit. In certain cases, the dosage is 0.1-500 mg, for example, about 0.1-50, 0.1-40, 0.1-20, 0.1-10, 0.2-20, 0.3-15, 0.4-10, 0.5-1, 0.5-100, 0.5-50, 0.5-30, 0.5-20, 0.5-10, 0.5-5, 1-50, 1-30, 1-20, 1-10, 1-5, 5-50, 5-20, 5-10, 10-100, 20-200, 30-150, 40-100, 50-100, 50-300, 50-250, 100-300, and 100-250 mg of each of the active ingredient(s). Such a response can be monitored by establishing an improved clinical outcome (*e.g.*, reduced inflammation, inhibition of cancer cell growth, more frequent remissions, complete or partial, longer disease-free survival, decreased morbidity, or an improvement in one or more standard tests or assays known in the art for the assessment of the disease, condition, or disorder status) in treated patients as compared to non-treated patients.

A therapeutic amount of a compound described in this application, means an amount effective to yield the desired therapeutic response, for example, an amount effective to decrease inflammation, or to, or delay the growth of a cancer or to cause a cancer to shrink or not metastasize. If what is administered is not the compound (or compounds), but an enantiomer, prodrug, salt or metabolite of the compound (or compounds), then the term "therapeutically effective amount" means an amount of such material that produces in the patient the same blood concentration of the compound in question that is produced by the administration of a therapeutically effective amount of the compound itself.

Patients that can be treated with a compound described in this application, and the pharmaceutically acceptable salts, prodrugs, enantiomers, and metabolites of such compounds, according to the methods of this disclosure include, for example, patients that have been diagnosed as having any of the diseases or disorders described herein.

Within such methods, pharmaceutical compositions are typically administered to a patient. As used herein, a "patient" or a "subject" refers to any warm-blooded animal, preferably a human. A patient may or may not be afflicted with an inflammatory condition, or an autoimmune disorder, or a cancer. Accordingly, the above pharmaceutical compositions may be used to prevent the development of an inflammatory condition, or an autoimmune disorder, or a cancer, or to treat a patient afflicted with an inflammatory condition, or an autoimmune disorder, or a cancer. An inflammatory condition, or an autoimmune disorder, or a cancer, may be diagnosed using criteria generally accepted in the art. In the case of cancer, pharmaceutical compositions may be administered either prior to or following surgical removal of primary tumors and/or treatment such as administration of radiotherapy or conventional chemotherapeutic drugs, or bone marrow transplantation (autologous, allogeneic, or syngeneic).

The compounds or compositions provided herein may be used alone or in combination with one or more additional therapeutic agents suitable for treatment of a particular indication. For example, a compound or composition of the disclosure may be co-administered to a subject who has, or is at risk for developing, cancer with conventional anticancer therapeutic regimens such as surgery, irradiation, chemotherapy and/or bone marrow transplantation (autologous, syngeneic, allogeneic, or unrelated) or in combination with conventional therapeutic regimens to treat an inflammatory condition, or an autoimmune disorder, or a cancer. Kits for administering the compounds may be prepared containing a composition or formulation of the compound in question, or an enantiomer, prodrug, metabolite, or pharmaceutically acceptable salt of any of these, together with the customary items for administering the therapeutic ingredient.

### 3. Combination therapy

GPR174 inhibitors (including antagonists, inverse agonists, partial agonists, and negative allosteric modulators), can be used in combination an inhibitor of an enzymatic pathway involved in the production of adenosine, and/or an inhibitor of the ATP-Adenosine-A2aR-and/or A2bR-mediated signaling (such as an A2aR antagonist, and/or an A2bR antagonist, and/or a CD73 inhibitor and/or a CD38 inhibitor and/or a CD39 inhibitor), and optionally with one or more additional therapeutic agents (*e.g.*, therapeutic compounds, compositions, treatments, therapies, and/or medical procedures). In such combination therapies, therapeutic agents of the disclosure may be formulated with or administered concurrently with, prior to, or subsequent to, one or more other desired therapies. The particular combination of therapies to employ in a combination regimen will take into account compatibility of the desired therapeutics and/or procedures and the desired therapeutic effect to be achieved. It will also be appreciated that the therapies employed may achieve a desired effect for the same condition, disease, or disorder, or they may achieve different effects *(e.g.,* control of any adverse effects).

In general, for use in treatment, the compounds described herein may be used alone, as mixtures of two or more compounds, or in combination with other agents, compounds, and/or pharmaceuticals. Examples of other agents that can be combined with the compounds described herein include agents that are known to be used for the treatment of inflammatory conditions, autoimmune disorders, or cancers. Another example of a potential agent to combine with the compounds described herein would include agents for the treatment of different yet associated or related symptoms or indications. Depending on the mode of administration, the agents may be formulated into suitable compositions to permit facile delivery. Each component of a combination therapy may be formulated in a variety of ways that are known in the art. For example, the first and second agents of the combination therapy may be formulated together or separately. The compound of the present disclosure and additional agent may be suitably administered to the patient at one time or over a series of treatments.

As described herein, in some cases, the combination therapy may provide "synergy" and prove to be "synergistic," *i.e.,* the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds, agents, and/or treatments are administered or delivered sequentially, *e.g.*, by different injections in separate syringes. In general, during alternation therapy, an effective dosage of each active ingredient is administered sequentially, *i.e.,* serially, whereas in combination therapy, effective dosages of two or more active ingredients are administered together. Suitable dosages for any of the above co-administered agents are those presently used and may be lowered due to the combined action (synergy) of a compound of the present disclosure and other co-administered agents or treatments.

Each compound of a combination therapy, as described herein, may be formulated in a variety of ways that are known in the art. For example, the first and second agents of the combination therapy may be formulated together or separately. The individually or separately formulated agents can be packaged together as a kit. Examples include, but are not limited to, kits that contain, *e.g.*, two pills, a pill and a powder, a suppository and a liquid in a vial, two topical creams, etc. The kit can include optional components that aid in the administration of the unit dose to patients, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, bI-Ver packs, bottles, tubes, and the like. Two or more compounds may be mixed together in a tablet, capsule, or other vehicle, or may be partitioned. In one example, the first compound is contained on the inside of the tablet, and the second compound is on the outside, such that a substantial portion of the second compound is released prior to the release of the first compound.

### 4. Compositions

For administration to a subject, *e.g.,* for treatment of diseases such as cancer, a GPR174 inhibitor is included or formulated into a composition, optionally in combination with at least one of an inhibitor of ATP-Adenosine-A2aR-and/or A2bR-mediated signaling (such as an A2aR antagonist, and/or an A2bR antagonist and/or a CD73 inhibitor and/or a CD38 inhibitor and/or a CD39 inhibitor), wherein the composition includes a pharmaceutical carrier for packing, storage, shipment, and administration. In addition, racemic mixtures, enantiomers, prodrugs of either the racemic mixture or of a stereoisomer, a metabolite of either the racemic mixture or of a stereoisomer, or a salt of any of these, may be included in a formulation or composition including a pharmaceutical carrier. The compositions contain one or more pharmaceutically acceptable carrier(s) and may also contain other therapeutically active ingredients as well as adjuvants and other ingredients that may be found in pharmaceutical compositions.

Thus, compounds of this disclosure can be formulated with a pharmaceutically acceptable carrier for administration to a subject. While any suitable carrier known to those of ordinary skill in the art may be employed in the pharmaceutical compositions of this disclosure, the type of carrier will vary depending on the mode of administration. The pharmaceutical composition is typically formulated such that the compound in question is present in a therapeutically effective amount, *i.e.,* the amount of compound required to achieve the desired effect in terms of treating a subject.

For preparing pharmaceutical compositions, the pharmaceutically acceptable carriers can be either solid or liquid. Solid form preparations include powders, tablets, pills, capsules, cachets, suppositories, and dispersible granules. A solid carrier can be one or more substance(s) that may also act as diluents, flavoring agents, binders, preservatives, tablet disintegrating agents, or an encapsulating material.

In powders, the carrier is a finely divided solid that is in a mixture with the finely divided active component. In tablets, the active component is mixed with the carrier having the necessary binding properties in suitable proportions and compacted in the shape and size desired.

Suitable carriers for the solid compositions of this disclosure include, for instance, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Alternatively, the compositions may be prepared in a form with an encapsulating material as a carrier providing a capsule in which the active component, with or without other carriers, is surrounded by a carrier, which is thus in association with it. Similarly, cachets and lozenges are included. Tablets, powders, capsules, pills, cachets, and lozenges can be used as solid dosage forms suitable for oral administration.

In certain cases, the GPR174 inhibitor either as a single agent or in combination with at least one of an A2aR antagonist, an A2bR antagonist, a CD38 inhibitor, a CD39 inhibitor, a CD73 inhibitor and/or a Treg attenuating agent may be dispersed in one or more pharmaceutical acceptable polymers or waxes and, for example, prepared into solid dosage forms. The GPR174 inhibitor or activator can be dispersed into the one or more pharmaceutical acceptable polymers through various processes. For example, a solvent-based process, a fusion-melt process, a hybrid fusion-solvent process or other dispersion processes can be used to prepare one or more pharmaceutical active drug substances into solid dispersion. Both melting and solvent based techniques define approaches to dissolve one or both of the active ingredient and the polymer.

In one aspect, the solvent-based process uses a solvent, such as water, non-organic solvents, and organic solvents, to dissolve and intimately disperse or dissolve the drug and the one or more pharmaceutical acceptable polymers. The solvent is later removed by evaporation or other ways while the drug/polymer solid dispersion is collected into a solid dosage form. The use of organic solvents may generate hazardous and toxic wastes to the environment. If possible, water is used for water soluble drugs to prepare a dispersion. Other suitable solvents may be, for example, alcohols and acetone for the use of water-insoluble polymers.

The resulting dispersion for preparing the solid dosage forms can be mixed with additional polymers, controlled release agents, binders, lubricant, and/or fillers. For example, the resulting dispersion can be blended with a mixture of polymers, controlled release agents, binders, lubricant, and/or fillers, through granulation before compressing into tablets or other solid dosage forms.

In another aspect, the fusion-melt process involves melting the GPR174 inhibitor and the one or more pharmaceutical acceptable polymers together at temperatures at or above the melting point of either the one or more pharmaceutical acceptable polymers and/or the inhibitor or activator. In the fusion-melt process, the inhibitor or activator and one or more pharmaceutical acceptable polymers can first be blended and melted in a suitable mixer. The molten mixture is then cooled rapidly to provide a congealed mass. Alternatively, the one or more pharmaceutical acceptable polymers can be melted into a molten state before mixing with the inhibitor or activator into a homogeneous state. The melted mixture of the inhibitor or activator and the one or more pharmaceutical acceptable polymers may be congealed by lowering the temperatures and then prepared into pharmaceutical dosage forms, such as a solid dosage form, *e.g*., powder and tablets. For example, the cooled mixture can be subsequently milled to produce a powder form. Alternatively, the cooled mixture can be milled and blended with additional fillers, lubricant, or binders and compressed into tablets.

In still another aspect, the hybrid fusion-solvent process can be used. For example, if there is thermal instability and immiscibility between the inhibitor or activator and the one or more pharmaceutical acceptable polymers, the inhibitor or activator can initially be dissolved in a small quantity of a solvent and added to a molten pharmaceutical acceptable polymer. The solvent is then evaporated to generate a product that is subsequently milled to produce a solid dosage form, such as a powder form, or compressed into tablets.

Liquid form preparations include solutions, suspensions, and emulsions, for example, water or water/propylene glycol solutions or suspensions. Aqueous suspensions suitable for oral use can be made by dispersing the finely divided compound in water with viscous material, such as natural or synthetic gums, resins, methylcellulose, sodium carboxymethylcellulose, and other well-known suspending agents. For parenteral injection, liquid preparations can be formulated in solution in aqueous polyethylene glycol solution. In certain cases, the pharmaceutical compositions are formulated in a stable emulsion formulation *(e.g.,* a water-in-oil emulsion or an oil-in-water emulsion) or an aqueous formulation that preferably comprises one or more surfactants. Suitable surfactants well known to those skilled in the art may be used in such emulsions. In one case, the composition comprising the compound in question is in the form of a micellar dispersion comprising at least one suitable surfactant. The surfactants useful in such micellar dispersions include phospholipids. Examples of phospholipids include: diacyl phosphatidyl glycerols, such as: dimyristoyl phosphatidyl glycerol (DPMG), dipalmitoyl phosphatidyl glycerol (DPPG), and distearoyl phosphatidyl glycerol (DSPG); diacyl phosphatidyl cholines, such as: dimyristoyl phosphatidylcholine (DPMC), dipalmitoyl phosphatidylcholine (DPPC), and distearoyl phosphatidylcholine (DSPC); diacyl phosphatidic acids, such as: dimyristoyl phosphatidic acid (DPMA), dipalmitoyl phosphatidic acid (DPPA), and distearoyl phosphatidic acid (DSPA); and diacyl phosphatidyl ethanolamines such as: dimyristoyl phosphatidyl ethanolamine (DPME), dipalmitoyl phosphatidyl ethanolamine (DPPE), and distearoyl phosphatidyl ethanolamine (DSPE). Other examples include derivatives of ethanolamine (such as phosphatidyl ethanolamine, as mentioned above, or cephalin), serine (such as phosphatidyl serine) and 3'-O-lysyl glycerol (such as 3'-O-lysyl-phosphatidylglycerol).

Also included in compositions for use in this disclosure are solid form preparations that are intended to be converted, shortly before use, to liquid form preparations for oral administration. Such liquid forms include solutions, suspensions, and emulsions. These preparations may contain, in addition to the active compound, colorants, flavors, stabilizers, buffers, artificial and natural sweeteners, dispersants, thickeners, solubilizing agents, and the like.

The compositions of the disclosure may also be in the form of controlled release or sustained release compositions as known in the art, for instance, in matrices of biodegradable or non-biodegradable injectable polymeric microspheres or microcapsules, in liposomes, in emulsions, and the like, including for use as subcutaneous depots.

The pharmaceutical preparation comprising a GPR174 inhibitor either as a single agent or in combination with at least one of an A2aR antagonist, an A2bR antagonist, a CD38 inhibitor, a CD39 inhibitor, a CD73 inhibitor and/or a Treg attenuating agent may be in unit-dosage form. In such form the preparation is subdivided into unit doses containing appropriate quantities of the active component. The unit-dosage form can be a packaged preparation, the package containing discrete quantities of preparation, such as packeted tablets, capsules, and powders in vials or ampoules. Also, the unit-dosage form can be a capsule, tablet, cachet, or lozenge itself, or it can be the appropriate number of any of these in packaged form.

In some cases, the pharmaceutical preparation comprising a GPR174 inhibitor either as a single agent or in combination with at least one of an A2aR antagonist, an A2bR antagonist, a CD38 inhibitor, a CD39 inhibitor, a CD73 inhibitor and/or a Treg attenuating agent may be formulated for topical, subcutaneous, intradermal, subdermal, subcutaneous, or transdermal administration. Topical administration relates to the use of a composition applied to the surface of the skin *(e.g.,* at the site of a wound, lesion or blemish) for exertion of local action. Accordingly, such topical compositions include those pharmaceutical or cosmetic forms in which the composition is applied externally by direct contact with the skin surface to be treated, such as the face, neck, arms, legs, and/or torso. Conventional pharmaceutical or cosmetic forms for this purpose include ointments, liniments, creams, shampoos, lotions, pastes, jellies, sprays, aerosols, and the like, and may further be applied directly or in patches or impregnated dressings, *e.g*., depending on the wound/blemish and skin region to be treated. The term "ointment" embraces formulations (including creams) having oleaginous, watersoluble and emulsion-type bases, *e.g*., petrolatum, lanolin, polyethylene glycols, as well as mixtures of these. In some cases, the pharmaceutical preparation comprising a GPR174 inhibitor for use in promoting wound healing is formulated as a "wound dressing," comprising any of a variety of materials utilized for covering and protecting a wound. Examples include occlusive dressings, adhesive dressings, antiseptic dressings, and protective dressings. In pharmaceutical preparations, a "cream" is a semisolid emulsion of the oil-in-water or water-in-oil type suitable for topical administration. In accordance with the present disclosure, creams and foams used will also be suitable for use with the therapeutic agents herein described.

The following examples are intended to illustrate the present disclosure. Examples disclosed herein not falling within the scope of the appended claims are for the assistance of the skilled person in understanding and practicing the disclosed invention but not part of the claimed invention.

### EXAMPLES

### EXAMPLE 1

### CRA AND CRA VALIDATION

The inventors used a CRA similar to that described in U.S. Patent No. 7,309,576 and O'Dowd et al., J. Biomol. Screen. 12:175-85, 2007 to identify compounds that are capable of interacting with GPR174 using a library of approximately 370,000 chemical entities. Briefly described, the CRA assay described in U.S. Patent No. 7,309,576 involves artificially inserting a NLS into the dopamine D1 receptor (DRD1), which allows the receptor to traffic from the cell membrane to the cell interior, and that the binding of antagonists to the receptor retained the receptor at the cell surface, while removal of the ligand allowed the receptor to continue to internalize from the cell surface. Both antagonists and agonists that selectively bound the receptor retained the receptor on the plasma membrane. GPR174 constructs were generated for use in a CRA-based compound screening assay. From this screen, the inventors initially identified compounds of formula I-VI, as described herein and included in Table 1.

It is further noted that the compounds identified by the inventors as having activity in the CRA assay for GPR174 *(i.e.,* compounds of formula I-VI) were found specifically interact with GPR174 as compared to a reference panel of 68 other GPCRs against which these compounds were also screened. For example, the compounds identified as having activity in the CRA assay for GPR174 were found to have no activity when tested in a CRA assay (for example, in a CRA assay as described in Example 1, when tested at a concentration of 2 µM) for other GPCRs in a reference panel including: muscarinic M1, CCRL2, CMKOR1, GPR3, GPR4, GPR12, GPR15, GPR17, GPR18, GPR19, GPR20, GPR21, GPR22, GPR25, GPR26, GPR27, GPR31, GPR37, GPR37L1, GPR39, GPR43, GPR45, GPR48, GPR50, GPR52, GPR61, GPR62, GPR63, GPR65, GPR68, GPR78, GPR80, GPR83, GPR85, GPR87, GPR88, GPR101, GPR132, GPR135, GPR139, GPR141, GPR146, GPR148, GPR149, GPR150, GPR151, GPR152, GPR153, GPR160, GPR161, GPR162, GPR173, GPR182, GPR183, LGR5, LGR6, MAS1, MRGD, MRGE, MRGF, MRGI4, OPN3, OPN4, OPN5, P2Y8, P2Y10, TAAR6, and TAAR8.

The inventors also sought to validate that the compounds identified using the CRA are modulatory compounds. To this end, the above-described CRA was used to screen a library for compounds that interact with three control non-orphan receptors, and the results of this screen were compared to the results of screening the same library for compound that were identified using a Fluorometric Imaging Plate Reader (FLIPR) assay, a conventional technique commonly used to identify compounds interacting with non-orphan GPCRs signaling through Ca⁺⁺. Three non-orphan GPCRs -- Muscarinic acetylcholine receptor M1 (CHRM1), Adrenergic receptor alpha 1a (ADRA1a), and Neuropeptide S receptor (NPSR) - were screened against a 10,000-compound GPCR-focused library. The outcome of these studies is summarized in Table 2. Thirty interacting compound "hits" were identified using the CRA, and eighty-one hits were identified using the FLIPR for the CHRM1 receptor; 9 CRA hits and 29 FLIPR hits for the NPS receptor were identified; and 110 CRA hits and 110 FLIPR hits for ADRA1a. All of the compounds that were identified in the CRA screen were also FLIPR hits for the corresponding receptor, demonstrating that each one of the CRA hits affects receptor signaling.

**Table 2. Comparison of CRA and FLIPR with three different receptors, each screened against a 10,000-compound GPCR-focused library.**

| GPCR | FLIPR hits | CRA hits | CRA hits effective in FLIPR |
|---|---|---|---|
| CHRM1 | 81 | 30 | 30 |
| NPSR | 29 | 9 | 9 |
| ADRA1a | 110 | 110 | 110 |

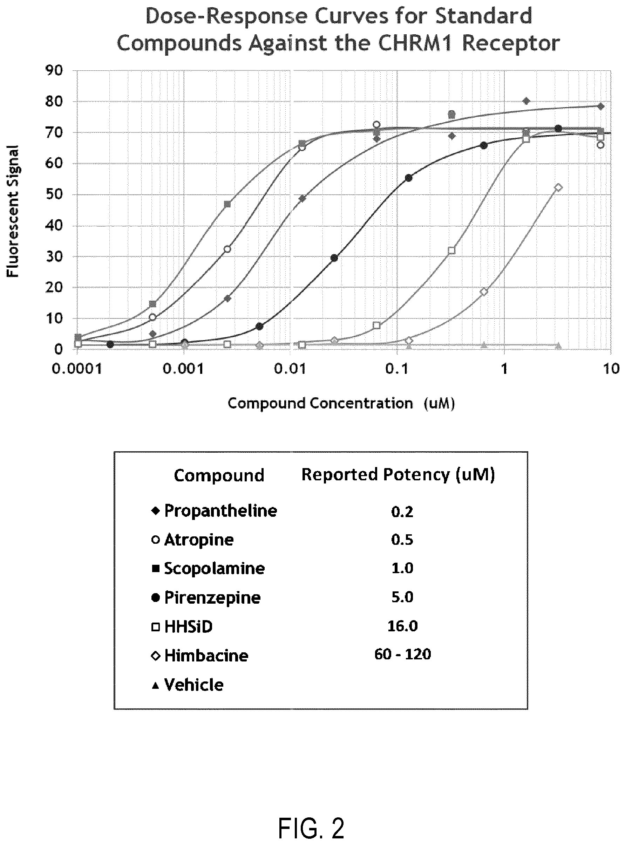

In addition, the CRA can also provide the relative potencies of compounds against the target receptor. This was demonstrated using the CHRM1 receptor and a variety of CHRM1-targeting compounds. As can be seen in Figure 2, the reported compound potencies are in the same rank order as the rank order of the concentration at which a fluorescent signal is observed in the CRA. As shown in Figure 5A, representative compound 1 (Group I), compound 2 (Group I), compound 3 (Group I), compound 4 (Group I), and compound 20 (Group II) identified as hits in the CRA assay as compounds that interact with GPR174 show a dose-response curve against GPR174, whereas the same set of compounds do not show any activity against CHRM1 (Figure 5B).

### EXAMPLE 2

### ASSAYS FOR IDENTIFICATION OF A GPCR SIGNALING PATHWAY

To identify the signaling pathway(s) that an activated GPCR utilizes to elicit its biological response, a multitude of assay technologies have been developed (Siehler, Biotechnology 3:471-83, 2008 and references within). The Gαs family of G proteins stimulates the activity of ACs, whereas the Gαi1-3, Gαo, and Gαz members of the Gαi family of G proteins inhibit the activity of ACs. ACs generate the second messenger cAMP using ATP as a substrate. A number of cAMP assay kits are available that measure the levels of cAMP (Siehler, Biotechnology 3:471-83, 2008 and references within). To detect the activation of Gαs coupled GPCRs, increases in cAMP levels are typically measured 15-60 minutes after agonist stimulation (Williams, Nat. Rev. Drug. Discov. 3:125-35, 2004). To measure the inhibition of ACs by the Gαi family of G proteins, cells are typically treated with forskolin, a nonspecific activator of ACs, to raise levels of intracellular cAMP. The activity of the Gαi family of G proteins is determined by measuring the reduction in cAMP levels of forskolin-stimulated cells (Wang et al., Assay Drug. Dev. Technol. 9:522-31, 2011).

The Gαq family of G proteins (Gαq, Gα11, Gα14, and Gα15/16), activates phospholipase C β isoforms (PLCβ) to generate the second messengers DAG and IP₃. IP₃ is soluble and diffuses through the cytoplasm and interacts with IP₃ receptors on the endoplasmic reticulum, causing the release of calcium and raising the level of intracellular calcium. DAG recruits PKC, which becomes activated in conjunction with binding calcium ions. Cellular assays are available to identify the second messengers IP₃ and calcium. Assays to measure inositol phosphate accumulation typically detect inositol monophosphate (IP₁), because IP₃ and IP₂ are rapidly degraded by cellular phosphatases (Liu et al., Anal. Biochem. 318:91-99, 2003; Trinquet et al., Anal. Biochem. 358:126-135, 2006). Calcium detection has been a well-established method to monitor the activation of Gαq-coupled GPCRs. Calcium responses are transient, and levels of calcium typically revert to baseline values in minutes. Calcium assays rely on the response to Ca²⁺ binding to fluorescent dyes that have been loaded into cells (Merit et al., J. Biomol. Screen. 10:780-7, 2005; Xin et al., J. Biomol. Screen. 12:705-14, 2007).

The G12/13 family of G proteins (Gα12 and Gα13) activates Rho-GEF proteins. Rho-GEF proteins catalyze the exchange of GDP for GTP to activate RhoA. RhoA in turn activates Rho kinase, which further leads to the activation of ATF2. Assays to directly measure Gα12/13 activation of RhoA are limited due to their lack of quantitation, low throughput, and need of specialized imaging instrumentation (Ren et al., Methods Enzymol. 325:264-72, 2000).

In addition to the signaling pathways activated by GPCRs through the activation or inhibition of adenylyl cyclases by Gαs and Gαi, stimulation of PLC-β by Gαq and activation of RhoA kinase by Gα12/13, the mitogen-activated protein kinase (MAPKs) signaling cascade is activated by G proteins (Siehler, Biotechnology 3:471-83, 2008 and references within). The activation of the MAPK signaling cascade can occur through Gαq by Ca²⁺-mediated activation of proline-rich tyrosine kinase 2 (Pyk2) or PKC activation of Raf-1 (Marinissen et al., Trends Pharmacol. Sci. 22:368-76, 2001; Pierce et al., Oncogene 20:1532-9, 2001). In addition to Gαq activation of the MAPK signaling cascade, the βγ subunits released from Gi-coupled GPCRs activate either receptor tyrosine kinases (RTKs) or non-receptor tyrosine kinases (Src), which leads to activation of Ras and the MAPK cascade (Marinissen et al., Trends Pharmacol. Sci. 22:368-76, 2001; Pierce et al., Oncogene 20:1532-9, 2001).

As outlined above, there are numerous assays available to identify the signaling pathway(s) coupled to activated GPCRs. However, these conventional assays rely on an agonist to stimulate the receptor. For orphan GPCRs, receptors whose agonists are not known, these assays are not suitable. Of the approximately 370 human GPCRs that respond to endogenous signals, such as peptides, lipids, neurotransmitters, or nucleotides (Vassilatis et al., Proc. Natl. Acad. Sci. USA 100:4903-8, 2003), it is estimated that >120 GPCRs are orphans (Howard et al., Trends Pharmacol. Sci. 22:132-40, 2001; www.iuphar-db.org). Traditional assays require an agonist to activate the GPCR to measure increases in the levels of second messenger molecules or identify activated signaling components. Although development of reporter assays and the use of receptor over-expression allow for the identification of possible signaling pathways for orphan GPCRs (Bresnick et al., Assay Drug Dev. Technol. 1:239-49, 2003), as described below, it has been observed that GPCR over-expression may lead to false activation of reporter genes. Therefore, in order to obtain an accurate determination of the signaling profile of GPR174, the inventors first determined the signaling pathways in reporter assays affected by the over-expressed GPR174, and then validated these results with compounds that were identified in the CRA assay as interacting with GPR174.

A reporter assay uses a reporter gene that contains a sequence of DNA whose product is synthesized in the response to activation of the signaling cascade under investigation. The DNA sequence contains a promoter, specific for a transcription factor, to control transcription of a reporter gene. The reporter gene ideally has low basal expression, is specific for a signaling pathway, has a large increase in the transcription of the reporter gene upon activation of the signaling pathway of interest, and can be reproducibly measured (Dinger et al., Molecular Biology in Medicinal Chemistry 73-94, 2004; Schenborn et al., Mol. Biotechnol. 13:29-44, 1999). Several reporter genes are commercially available. The firefly luciferase has emerged as a good reporter gene to study signal pathways due to its low endogenous activity, high sensitivity, large dynamic range, and ease of detection (Cheng, Curr. Chem. Genomics 4:84-91, 2010; Dinger et al., Molecular Biology in Medicinal Chemistry 73-94, 2004). Another advantage of firefly luciferase is that it can be measured in the presence of *Renilla* luciferase. The substrate of *Renilla* luciferase, coelenterazine, is distinct from the substrate of firefly luciferase, D-luciferin. Because of the substrate distinction, *Renilla* has been used as an internal control for luciferase-based assay systems of mammalian gene expression (Shifera et al. Anal. Biochem. 396:167-72, 2010). In these assays, *Renilla* luciferase is expressed under the control of a constitutive promoter, and firefly luciferase is expressed under the control of an inducible promoter. This system developed by Promega (Madison, WI, USA) allows for the normalization of results of the firefly luciferase assay measurements, thereby reducing variability that can be caused by experimental differences such as transfection efficiency or toxicity from transfected gene products and from the treatment of the cells with compounds.

To identify GPCR signaling pathways, synthetic promoters containing only a single type of transcription factor binding site are introduced into the reporter gene so that the reporter gene will be activated only by specific signaling components of an activated signaling pathway (Hill et al., Curr. Opin. Pharmacol. 1:526-32, 2001; Cheng, Curr. Chem. Genomics 4:84-91, 2010). Reporter constructs have been developed that contain promoter elements (CRE, SRE, NFAT, AP1, and SRF) linked to firefly luciferase that can be used to identify and distinguish each of the signaling pathways activated by GPCRs (Stratagene, CA and Promega, WI).

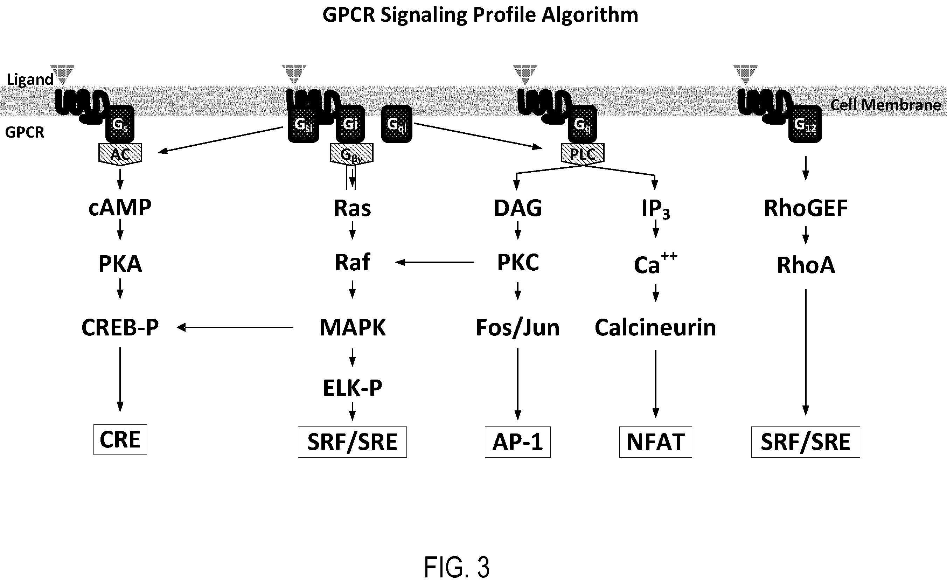

GPCRs that couple to the Gs family of G proteins activate AC to generate cAMP. cAMP activates protein kinase A (PKA) and leads to the phosphorylation of CREB. Phosphorylated CREB binds to the cAMP response element (CRE) and activates CRE-dependent promoters. To identify GPCR activation of the Gs signaling pathway, CRE-dependent promoters direct the expression of the firefly luciferase (Luc) reporter gene. In one example, we used the pCRE-Luc (Stratagene, CA) and pGL4.29 (Promega, WI) CRE-Luc reporter plasmids, which are sensitive to the cAMP levels and result in transcriptional activation and increased synthesis of luciferase when cAMP levels are increased (Table 3). The algorithm is also shown diagrammatically in Figure 3.

**Table 3: Reporter constructs used to study signaling pathways of orphan GPCRs**

| | Gs | G12/13 | Gq | Gi | Gi | Gi | Gi |
|---|---|---|---|---|---|---|---|
| | | | | No chimeric G proteins | Gs chimeras | Gq chimeras | Gα15/16 |
| CRE-Luc | + | | + | | + | + | + |
| AP1-Luc | | | + | | | + | + |
| NFAT-Luc | | | + | | | + | + |
| SRE-Luc | | + | + | | | + | + |
| SRF-Luc | | + | + | | | + | + |

GPCRs that couple to the Gq family of G proteins (Gαq, Gα11, Gα14, and Gα16) activate PLCβ, which hydrolyzes phosphatidylinositol to generate DAG and IP₃. DAG and IP₃ control calcium efflux from the endoplasmic reticulum. In turn, DAG and calcium control the activity of several isoforms of PKC. PKC isoforms activate the FOS and JUN proteins, which in combination bind to and activate the Activator Protein 1 (AP1) response element. Calcium also binds to and activates calcineurin, which dephosphorylates the transcription factor Nuclear Factor of Activated T-cells (NFAT). Dephosphorylated NFAT binds to and activates the NFAT response element. To measure activation of PLCα by Gq-coupled GPCRs, we used the AP1 and NFAT-dependent promoters to direct expression of the Luc reporter gene. Specifically, we used the AP1-Luc reporter construct pAP1-Luc (Stratagene, CA) and the NFAT-Luc reporter constructs pNFAT-Luc and pGL4.30 (Stratagene, CA and Promega, WI). Activation of the AP1-Luc and NFAT-Luc reporter constructs results in the transcriptional activation of firefly luciferase (Stratagene, CA and Promega, WI). In addition to stimulation of the AP1-Luc and NFAT-Luc reporter constructs through activation of the Gq family of G proteins, signaling components from Gq-activated GPCRs can activate the MAPK, RhoA, and Gs signaling pathways. The reporter constructs for the MAPK, RhoA, and Gs signaling pathways are also activated through Gq-coupled GPCRs (Siehler S., Biotechnology 3:471-83, 2008 and references within; Mao et al., J. Biol. Chem. 273:118-27, 1998; Hill et al., Curr. Opin. Pharmacol. 1:526-532, 2001; Paguio et al., Promega, Cell notes 16:22-25, 2006). The reporter constructs for these signaling pathways, pCRE-Luc, pGL4.29, pSRE-Luc, pGL4.33, and pGL4.34 (Stratagene, CA and Promega, WI) are utilized in reporter assays to identify Gq signaling by GPCRs (Table 3).

GPCRs that couple to the Gi family of G proteins (Gαi1-3, Gαo, and Gαz) inhibit AC activity, which suppresses the generation of cAMP (Siehler S., *Biotechnology* 3:471-83, 2008). Due to the difficulty in measuring the inhibition of AC activity, we used chimeric G proteins that redirect Gi-coupled GPCRs to activate either PLC or AC (Coward et al., Anal. Biochem. 270:242-8, 1999). Chimeric G proteins were initially developed following the observation that the carboxyl terminus of the G protein is involved in receptor recognition while the rest of the G protein is involved in effector activation. Due to the ease of measuring the activation of signaling molecules in the Gq and Gs pathways and the difficulty of measuring the activation of Gi-coupled GPCRs, chimeric G proteins were developed for Gq in which the last five amino acids of Gαq were replaced with the last five amino acids from either Gαi or Gαo to form the chimeric G proteins Gqi5 or Gqo5 (Coward et al., Anal. Biochem. 270:242-8, 1999; Molecular Devices, CA). The Gqi5 and Gqo5 chimeric G proteins are able to redirect Gi coupled GPCRs to activate phospholipase C. With the Gq chimeric G proteins, GPCRs that normally inhibit AC can now be measured with reporter constructs that measure Gαq activation. Likewise, chimeric G proteins were prepared for Gs in which the last thirteen carboxyl terminal amino acids of Gαs were replaced with the last thirteen carboxyl terminal amino acids of either Gαt, Gαi, Gαo or Gαz to form the chimeric G proteins Gαs-t, Gαs-i, Gαso and Gαs-z. The Gαs-t, Gαs-i, Gαs-o and Gαs-z chimeric G proteins are able to redirect Gi coupled GPCRs to activate AC. With the Gs chimeric G proteins, GPCRs that normally inhibit AC can now be measured with reporter constructs that measure Gαs activation. Similar to the chimeric G proteins, which are useful tools to couple Gi activated GPCRs to other signaling pathways that are more amenable for measurement, the promiscuous G proteins Gα15 and Gα16 are reported to couple a wide variety of GPCRs to PLCβ (Offermanns et al., J. Biol. Chem. 270:15175-80, 1995). Gα15/16, in addition to the chimeric Gq G proteins, are useful tools to couple Gi GPCRs to the Gq signaling pathway. Activation of Gi coupled GPCRs do not activate the CRE, SRE, NFAT, AP1, or SRF reporter constructs. Although it has been reported Gi-coupled GPCRs activate the MAPK signaling cascade through the βγ subunits, we do not observe activation of the MAPK-dependent reporter construct SRE-Luc or any of the reporter constructs upon Gi GPCR activation (Table 3). When Gi-coupled GPCRs are activated with co-expressed Gs chimeric G protein, the Cre-Luc is stimulated via activation of AC by the chimeric Gs G protein. Likewise, when Gi-coupled GPCRs are activated with co-expressed Gq chimeric G proteins or the promiscuous Gα16 G protein, the CRE, SRE, NFAT, AP1, and SRF reporter constructs are stimulated via activation of PLCβ by the chimeric Gq or Gα16 G proteins (Table 3).

The G12/13 family of G proteins (Gα12 and Gα13) activates the Rho-GEF protein, which catalyzes the exchange of GDP for GTP to activate RhoA GTPase. RhoA, in turn, activates Rho kinase, which further leads to the activation of ATF2. ATF2 binds to the Serum Response Factor (SRF) element and activates SRF and SRE-dependent promoters (Siehler, Biotechnology 3:471-83, 2008 and references within). The reporter constructs for these signaling pathways, pSRE-Luc, pGL4.33, and pGL4.34 (Stratagene, CA and Promega, WI), are utilized in reporter assays to identify Gα12/13 signaling by GPCRs (Table 3).

Orphan GPCRs have no agonist and rely on the basal activity of the orphan GPCR to activate the signaling pathway. GPCRs in their basal state are in equilibrium between active and inactive states (Bond et al., Trends Pharmacol. Sci. 27:92-96, 2006). The extent of the basal activation is dependent on the receptor, with some GPCRs being constitutively active in the absence of an agonist and other GPCRs having very low levels of basal activity (Hebert, Biochem. Cell. Biol. 76:1-11, 1998). Due to the basal activity of GPCRs, GPCRs that are over-expressed in transient transfections may activate their expected signaling pathways in the absence of an activating ligand (Bresnick et al., Assay Drug Dev. Technol. 1:239-249, 2003). Reporter assays may be used as a tool to investigate the signaling pathways of orphan GPCRs due to amplification of the basal activity via induction of the reporter gene. However, as demonstrated below, reporter assays relying solely on transient transfection-based overexpression of a GPCR may be unreliable for the accurate determination of the signaling profile of a GPCR of interest, such as a Class A GPCR *(e.g.,* GPR174).

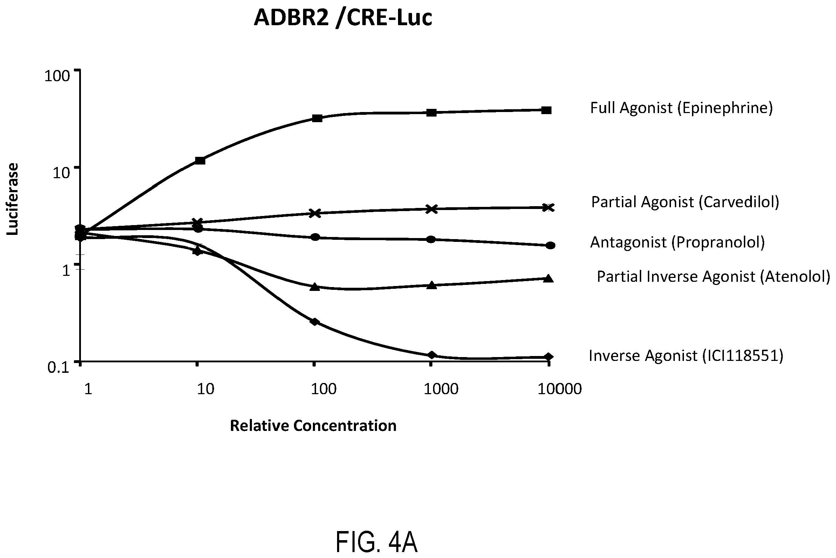

High-throughput screens of compound libraries against GPCRs have identified compounds that interact and modulate GPCR activity. Traditional high throughput screens usually involved assays that displace a radiolabeled ligand or antagonize a functional response of an activator to the GPCR (Xiao, Comb. Chem. High Throughput Screen. 11:195-215, 2008). It is now recognized that compounds that interact with GPCRs modulate the basal activity of the receptor in different ways (Bond et al., Trends Pharmacol. Sci. 27:92-96, 2006). Agonists drive the conformation of the receptor to the active state, inverse agonists drive the conformation of the receptor to the inactive conformation, and antagonists do not affect the basal equilibrium of the receptor. By over-expressing a GPCR in a cell-based system, the signaling pathway of a GPCR can initially be predicted with reporter constructs as described above. Compounds that have been identified in high-throughput assays to interact with a GPCR can be tested to determine the nature of the compound by the modulation of the basal signal predicted by the over-expressed GPCR, and thereby validate the signaling profile of the GPCR that was initially predicted by over-expressing the GPCR in the reporter assay. If the signal is increased the compound is an agonist, if the signal is inhibited the compound is an inverse agonist and if the compound has no effect on the signal, it is an antagonist or allosteric modulator. In our reporter assay system, we have used, among others, the β-2-adrenergic (ADBR2) GPCR and several compounds interacting with this receptor to illustrate the expected effects on signaling (Figure 4A).

The use of compounds interacting with a particular GPCR is essential for the accurate determination of the signaling profile of a receptor. As demonstrated below, GPCR over-expression may lead to false activation of reporter genes. Therefore, only reporter assays affected by both the over-expressed receptor and its corresponding compounds provide an accurate determination of a GPCR signaling profile.

### Experimental Results with ADBR2 Demonstrate the Need to Validate Signaling Pathway Transient Transfection Assay Results with Compound Modulation

β-2-adrenergic (ADBR2), a Class A GPCR, is known to stimulate adenylate cyclase activity through activation of the Gs signaling pathway (Wenzel-Seifert, K. et al, Biochem Pharmacol., 64: 9-20 (2002)). A compound (ICI 118551) known to act as an inverse agonist at ADBR2 has been reported (Hothersall J.D. et al., Br J Pharmacol., 164(2):317-31 (2011)). An experiment was carried out to compare the results of (1) over-expression of ADBR2 in a reporter assay with different reporter constructs and (2) over-expression of ADBR2 in the presence/absence of compound ICI 118551 known to interact with ADBR2 and act as an inverse agonist to determine whether transfection studies alone would accurately identify the signaling pathway of ADBR2.

### Reporter Assays:

The reporter assays were performed by transiently transfecting increasing amounts of ADBR2 construct pCMV6-XL4-ADBR2 (SC107904, Origene, MD), pNFAT-Luc, pGL4.30, pAP1-Luc, pCRE-Luc, pGL4.29 (100 ng), pSRE-Luc, pGL4.33 or pGL4.34 and TK-renilla as internal control (0.3 ng) (Promega) with lipofectamine 2000 (2 µg) (Invitrogen, CA) according to the manufacturer's directions. The DNA/lipofectamine 2000 mix (100 µL) was incubated at room temperature for 30 minutes and then added into 24-well tissue culture plates (Corning, NY) containing 70-90% confluent cultured cells, (HEK293 or CHO cell lines (ATCC, VA)), in 500 µL of media. The cells were further incubated for 4-6 hours at 37^{□}C and then 500-1000 µL of additional media was added to each well of transfected cells. The following day, the media were removed, and the cells lysed. Luciferase and renilla activities were measured with the Dual-luciferase assay system (Promega, WI) according to the manufacturer's directions. Lysis buffer (100 µL) was added to the cells and incubated at room temperature for 20-30 minutes to allow for the cells to lyse. The lysate (10 µL) was measured for luciferase and renilla activities with LARII and Stop&Glo reagent (50 µL each) with a SpectraMax L (Molecular Devices, CA). Data were analyzed with either Excel (Microsoft, WA) or Prism 4.03 (GraphPad, CA).

### Compound Assays:

The compound assays were performed by transiently transfecting the ADBR2 construct pCMV6-XL4-ADBR2 (SC107904, Origene, MD), either pNFAT-Luc, pGL4.30 or pGL4.29 (3000 ng), and TK-renilla as internal control (10 ng; Promega) with lipofectamine 2000 (60 µg) (Invitrogen, CA) according to the manufacturer's directions. The DNA/lipofectamine 2000 mix (3000 µL) was incubated at room temperature for 30 minutes and then added into a 10 cm culture dish (Corning, NY) containing 70-90% confluent cultured cells, (HEK293 or CHO cell lines (ATCC, VA)), in 15 mL of media. The cells were further incubated for 4-6 hours at 37°C and then media were removed and cells were detached with 2 mL trypsin (Life Technologies, CA). Media (26 mL) were added to the detached cells and 120 □L of transfected cells were transferred to each well on a 96-well plate. Increasing concentration of ICI 118551 or pirenzepine in DMSO (1.2µl) was added to the cells after plating into the 96-well plate. The cells were further incubated at 37°C for various times. The media were removed and the cells lysed. Luciferase and renilla activities were measured with the Dual-luciferase assay system (Promega, WI) according to the manufacturer's directions. Lysis buffer (25 µL) was added to the cells and incubated at room temperature for 20-30 minutes to allow for the cells to lyse. The lysate (10 µL) was measured for luciferase and renilla activity with LARII and Stop&Glo reagent (50 µL each) with a SpectraMax L (Molecular Devices, CA). Data were analyzed with either Excel (Microsoft, WA) or Prism 4.03 (GraphPad, CA).

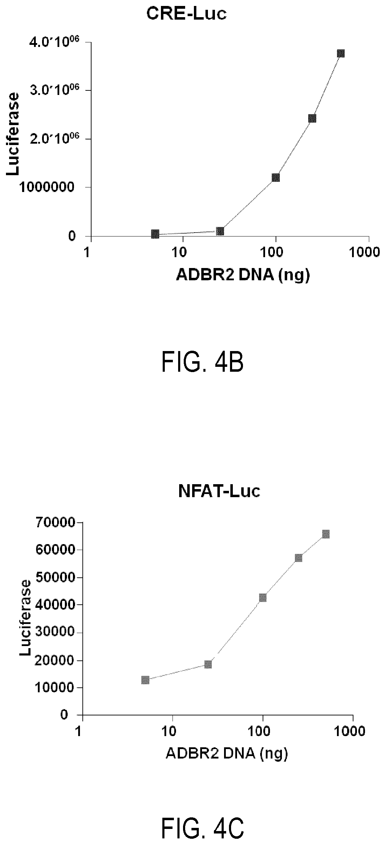

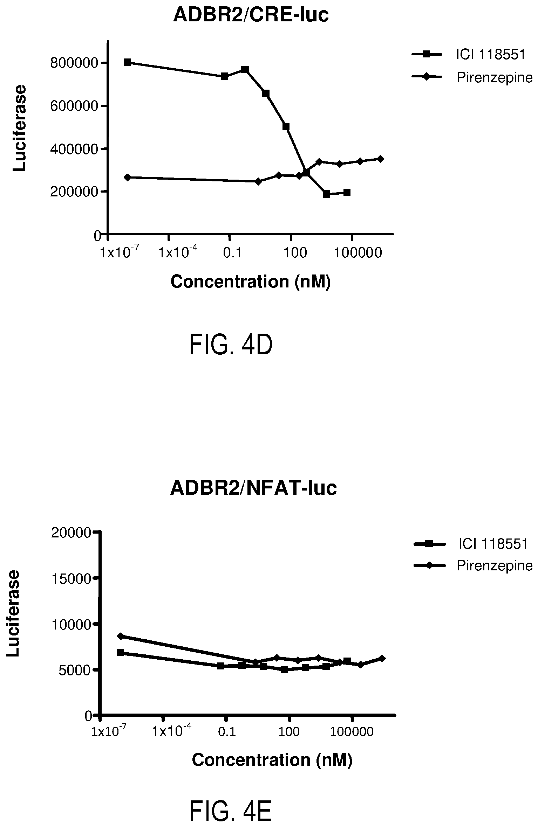

Over-expression of ADBR2 in the reporter assay resulted in strong stimulation of the Stratagene CRE-Luc reporter (pGL4.29), as expected (Figure 4B). However, the NFAT-Luc reporters were also stimulated, although to a lesser extent (Figure 4C). When compound ICI 118551, known to act as an inverse agonist at ADBR2, was added to cells transfected with ADBR2 and CRE-Luc, it was determined that ICI 118551 specifically inhibited the activity of ADBR2 with the CRE-Luc reporter, as compared with a non-specific control compound (pirenzepine), as shown in Figure 4D. In contrast, when compound ICI 118551 was added to cells transfected with ADBR2 and NFAT-luc, it was determined that compound ICI 118551 did not modulate the activity of the NFAT-Luc reporters with ADBR2 over-expression (Figure 4E), indicating the activation of the NFAT-Luc reporters by transiently transfected ADBR2 was non-specific.

This experiment demonstrates that GPCR over-expression may lead to false activation of reporter genes. Therefore, compound modulation of the GPCR is required to validate the accuracy of a signaling pathway that was initially predicted based upon activation of the reporter constructs in an assay relying solely on GPCR overexpression.

### EXAMPLE 3

### IDENTIFICATION OF THE GPR174 SIGNALING PATHWAYS

To determine which signaling pathways GPR174 may activate, GPR174 was tested with the reporter constructs pAP1-Luc, pNFAT-Luc, pGL4.30, pCRE-Luc, pGL4.29, pSRE-Luc, pGL4.33 and pGL4.34 (Stratagene, CA and Promega, WI). The algorithm used is shown in Figure 3.

The reporter assays were performed by transiently transfecting increasing amounts of GPR174 construct pCMV6-XL4-GPR174 (SC104514, Origene, MD), pNFAT-Luc, pGL4.30, pAP1-Luc, pCRE-Luc, pGL4.29 (100ng), pSRE-Luc, pGL4.33 or pGL4.34 and TK-renilla as internal control (0.3 ng, (Promega) with lipofectamine 2000 (2 ug, Invitrogen CA) according to the manufacturer's directions. The DNA/lipofectamine 2000 mix (100 µL) was incubated at room temperature for 30 minutes and then added into 24-well tissue culture plates (Corning, NY) containing 70-90% confluent cultured cells, typically HEK293 or CHO cell lines (ATCC, VA), in 500 µL of media. The cells were further incubated for 4-6 hours at 37°C and then 500-1000 µL of additional media were added to each well of transfected cells. The following day, the media were removed, and the cells lysed. Luciferase and renilla activities were measured with the Dual-luciferase assay system (Promega, WI) according to the manufacturer's directions. Typically, lysis buffer (100 µL) was added to the cells and incubated at room temperature for 20-30 minutes to allow for the cells to lyse. The lysate (10 µL) was measured for luciferase and renilla activities with LARII and Stop&Glo reagent (50 µL each) with a SpectraMax L (Molecular Devices, CA). Data were analyzed with either Excel (Microsoft, WA) or Prism 4.03 (GraphPad, CA).

Over-expression of GPR174 strongly activated the two Cre-luc reporter constructs (Table 4), suggesting that GPR174 activates the Gs signaling pathway to activate adenylate cyclase. The data also indicates that GPR174 does not activate the Gq or G12/13 family of G proteins. As described herein, it has been determined that GPR174 does not activate the Gi signaling pathway.

**Table 4: Fold Stimulation of reporter constructs with GPR174 titration.**

| GPR174 (ng DNA) | S-Cre | S-NFAT | S-Sre | AP-1 | P-Cre | PNFAT | P-Sre | Srf |
|---|---|---|---|---|---|---|---|---|
| 0ng | 1 | 1 | 1 | 1 | 1 | 1 | 1 | 1 |
| 5ng | 40 | 2.1 | 0.9 | 2.0 | 52 | 2.2 | 1.0 | 0.9 |
| 25ng | 52 | 1.8 | 0.8 | 2.6 | 145 | 8.0 | 1.4 | 1.1 |
| 100ng | 93 | 2.2 | 1.0 | 2.6 | 252 | 4.2 | 1.5 | 1.2 |
| 250ng | 102 | 2.4 | 1.1 | 3.3 | 298 | 3.2 | 1.6 | 1.2 |

| | | | | | | | | |
|---|---|---|---|---|---|---|---|---|
| Note: "S" indicates reporter constructs from Stratagene. "P" indicates reporter constructs from Promega | | | | | | | | |

To see if GPR174 may couple to the Gi signaling pathway in addition to the Gs signaling pathway, GPR174 was tested with Gq and Gs chimeric G proteins as follows.

The Gi reporter assays with the Gs chimeric proteins were performed by transiently transfecting increasing amounts of GPR174 construct pCMV6-XL4-GPR174 (SC104514, Origene, MD), varying amounts of either Gαs-t, Gαs-i, Gαs-o or Gαs-z, pCRE-Luc or pGL4.29 (100 ng), and TK-renilla as an internal control (0.3 ng) with lipofectamine 2000 (2 ug) (Invitrogen, CA) according to the manufacturer's specifications.

The DNA/lipofectamine 2000 mix (100 µL) was incubated at room temperature for 30 minutes and then added into 24-well tissue culture plates (Corning, NY) containing 70-90% confluent cultured cells, typically HEK293 or CHO cell lines (ATCC, VA), in 500 µl of media. The cells were further incubated for 4-6 hours at 37°C and then 500-1000µL of additional media were added to each well of transfected cells. The following day the media were removed, and the cells lysed. Luciferase and renilla activities were measured with the Dual-luciferase assay system (Promega, WI) according to the manufacturer's directions. Typically, lysis buffer (100 µL) was added to the cells and incubated at room temperature for 20-30 minutes to allow for the cells to lyse. The lysate (100 µL) was measured for luciferase and renilla activities with LARII and Stop&Glo reagent (50 µL each) with a SpectraMax L (Molecular Devices, CA). Data were analyzed with either Excel (Microsoft, WA) or Prism 4.03 (GraphPad, CA). The Gi reporter assays with the Gq chimeric proteins were performed by transiently transfecting increasing amounts of GPR174 construct pCMV6-XL4-GPR174, varying amounts of either RD-PGQI5 (Gqi5), or RD-PGQO5 (Gqo5) (Molecular Devices, CA), pNFAT-Luc, pGL4.30, pAP1-Luc and TK-renilla as internal control (0.3 ng, Promega) with lipofectamine 2000 (2 µg) (Invitrogen, CA) according to the manufacturer's directions. The DNA/lipofectamine 2000 mix (100 µL) was incubated at room temperature for 30 minutes and then added into 24-well tissue culture plates (Corning, NY) containing 70-90% confluent cultured cells, typically HEK293 or CHO cell lines (ATCC, VA), in 500 µL of media. The cells were further incubated for 4-6 hours at 37°C and then 500-1000 µl of additional media were added to each well of transfected cells. The following day the media were removed, and the cells lysed. Luciferase and renilla activities were measured with the Dual-luciferase assay system (Promega, WI) according to the manufacturer's directions. Typically, lysis buffer (100 µL) was added to the cells and incubated at room temperature for 20-30 minutes to allow for the cells to lyse. The lysate (10 µL) was measured for luciferase and renilla activity with LARII and Stop&Glo reagent (50 µL with a SpectraMax L (Molecular Devices, CA). Data were analyzed with either Excel (Microsoft, WA) or Prism 4.03 (GraphPad, CA).

As shown in Table 5, titration of GPR174 with the Gq chimeric G proteins Gαq-i5 and Gαq-o5, did not activate the AP1, NFAT or SRE reporters significantly. Likewise, titration of GPR174 with the Gs chimeric G proteins, Gαs-t, Gαs-i, Gαs-o or Gαs-z did not further activate the Cre reporter compared with the absence of Gs chimeric G protein (Table 6). These results indicate that GPR174 does not couple to the Gi signaling pathway.

**Table 5: Fold stimulation of reporter constructs with GPR174 titration and either Gqi5 or Gqo5.**

| GPR174 (ng DNA) | AP1-Gqi5 | AP1-Gqo5 | AP1-control | NFAT-Gqi5 | NFAT-Gqo5 | NFAT-control | Sre-Gqi5 | Sre-Gqo5 | SRE-control |
|---|---|---|---|---|---|---|---|---|---|
| 0ng | 1 | 1 | 1 | 1 | 1 | 1 | 1 | 1 | 1 |
| 5ng | 2.1 | 2.2 | 1.9 | 1.7 | 0.8 | 2.6 | 1.3 | 1.6 | 1.1 |
| 25ng | 3.7 | 4.6 | 2.2 | 2.1 | 1.1 | 2.6 | 1.5 | 2.0 | 1.3 |
| 100ng | 3.1 | 4.4 | 1.7 | 2.0 | 1.3 | 2.4 | 2.1 | 2.7 | 1.5 |
| 250ng | 2.6 | 4.3 | 2.1 | 1.6 | 1.1 | 2.3 | 2.6 | 2.7 | 1.6 |

**Table 6: Fold stimulation of the Cre reporter construct with GPR174 titration and either Gs-t, Gs-o, Gs-1, Gs-z or no Gs chimera.**

| GPR174 (ng DNA) | Cre/Gs-t | Cre/Gs-o | Cre/Gs-i | Cre/Gs-z | Cre/No Gs chimera |
|---|---|---|---|---|---|
| 0ng | 1 | 1 | 1 | 1 | 1 |
| 5ng | 8.8 | 5.6 | 101 | 2.2 | 134 |
| 25ng | 13.7 | 19.3 | 184 | 3.7 | 198 |
| 100ng | 16.9 | 22.4 | 263 | 4.9 | 270 |
| 250ng | 19.7 | 20.9 | 259 | 3.7 | 210 |

In summary, the results described in the reporter assays described above indicates that GPR174 stimulates the Gs signaling pathway and does not couple to the Gi, Gq or G12/13 signaling pathways.

### EXAMPLE 4

### FUNCTIONAL INTERACTION OF IDENTIFIED COMPOUNDS

The reporter assays described above in Example 3 indicate that GPR174 stimulates the Gs signaling pathway. Representative compounds from each of the chemical groups I-VI (shown in Table 1) that were determined to interact with GPR174 in the CRA assay were tested in the reporter assay and to determine the specificity of GPR174 signaling and the nature of the interacting GPR174 compound.

### Reporter Assays

Compounds were tested in the reporter system with increasing concentrations of GPR174-interacting compounds. GPR174 construct pCMV6-XL4-GPR174 or ADBR2, reporter of interest, pCRE-Luc or GL4.29 (3000 ng), and TK-renilla as an internal control (10 ng) with lipofectamine 2000 (60 ug) (Invitrogen, CA) according to the manufacturer's directions.

The DNA/lipofectamine 2000 mix (3000 µL) was incubated at room temperature for 30 minutes and then added into a 10 cm culture dish (Corning, NY) containing 70-90% confluent cultured cells, typically HEK293 or CHO cell lines (ATCC, VA), in 15 mL of media. The cells were further incubated for 4-6 hours at 37°C and then media were removed, and cells were detached with 2 mL trypsin (Life Technologies, CA). Media (26 mL) were added to the detached cells and 120 µL of transfected cells were transferred to each well on a 96-well plate. Increasing concentration of each compound in DMSO (1.2 µl) was added to the cells after plating into the 96-well plate. The cells were further incubated at 37°C for various times. The media were removed, and the cells lysed. Luciferase and renilla activities were measured with the Dual-luciferase assay system (Promega, WI) according to the manufacturer's directions. Typically, lysis buffer (25 µL) was added to the cells and incubated at room temperature for 20-30 minutes to allow for the cells to lyse. The lysate (10 µL) was measured for luciferase and renilla activity with LARII and Stop&Glo reagent (50 µL each) with a SpectraMax L (Molecular Devices, CA). Data were analyzed with either Excel (Microsoft, WA) or Prism 4.03 (GraphPad, CA).

Compounds 1-59 that interact with GPR174, as shown in Table 1, were each titrated against GPR174 and the control receptor, ADBR2. ADBR2 is a GPCR, like GPR174, that activates the Gs signaling pathway.

The results of the signaling assay titrating human GPR174 with pCRE-Luc and increasing concentration of compounds shown in Table 1 are provided in Table 1 (EC50 values and fold effect). As shown in Table 1, in Group I, compounds 1, 2, 5-18 and 53 are inverse agonists and compounds 3-4 were found to be non-modulators in the signaling assay and are therefore characterized as either GPR174 antagonists or allosteric modulators. As further described in this Example, it was determined that compound 4 does compete with the GPR174 agonist LysoPS and is therefore confirmed to be a GPR174 antagonist. In Group II, compounds 19 and 20 were both found to be non-modulators in the signaling assay and are therefore characterized as either GPR174 antagonists or allosteric modulators. In Group III, compound 21 was found to be a non-modulator in the signaling assay and is therefore characterized as a GPR174 antagonist or allosteric modulator. In Group IV, compounds 22-55 are inverse agonists. In Group V, compound 56 was found to be an inverse agonist. In Group VI, compounds 57 and 58 were found to be GPR174 antagonists or allosteric modulators.

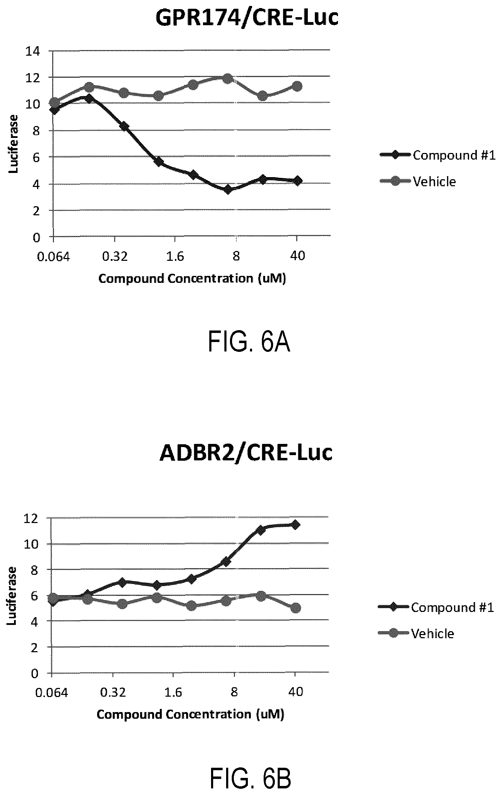

The signaling assay results of representative GPR174 interacting compounds are provided in FIGURES 6-20.

As shown in FIGURE 6A, compound 1 (Group I) inhibited the Gs pathway in the presence of GPR174 but did not inhibit the Gs pathway in the presence of ADBR2 (FIGURE 6B), indicating that compound 1 is a specific inverse agonist of GPR174.

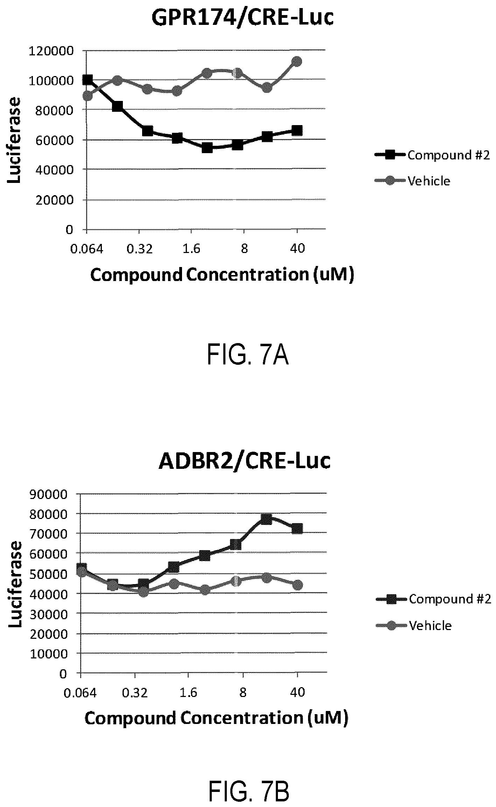

As shown in FIGURE 7A, compound 2 (Group I) inhibited the Gs pathway in the presence of GPR174 but did not inhibit the Gs pathway in the presence of ADBR2 (FIGURE 7B), indicating that compound 2 is a specific inverse agonist of GPR174.

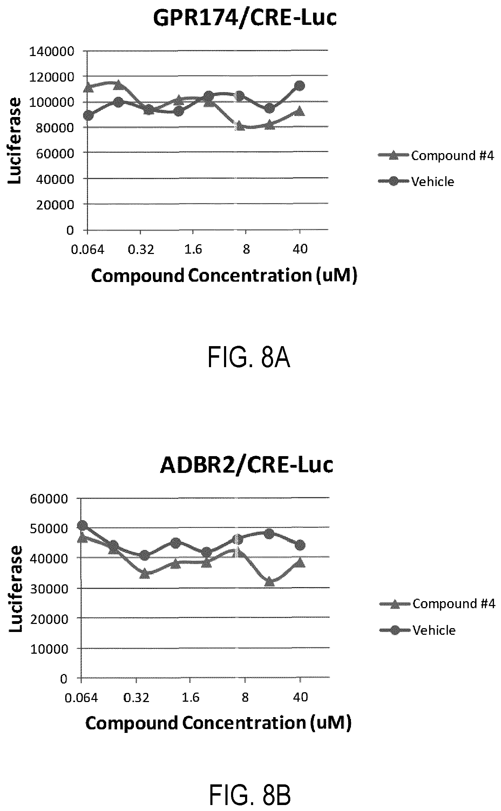

As shown in FIGURE 5A and Table 1, compound 4 shows a dose-response curve against GPR174 in the CRA assay. As shown in FIGURE 8A, compound 4 (Group I) did not modulate the Gs pathway in the presence of GPR174 and did not modulate the Gs pathway in the presence of ADBR2 (FIGURE 8B), therefore compound 4 is characterized as a GPR174 antagonist.

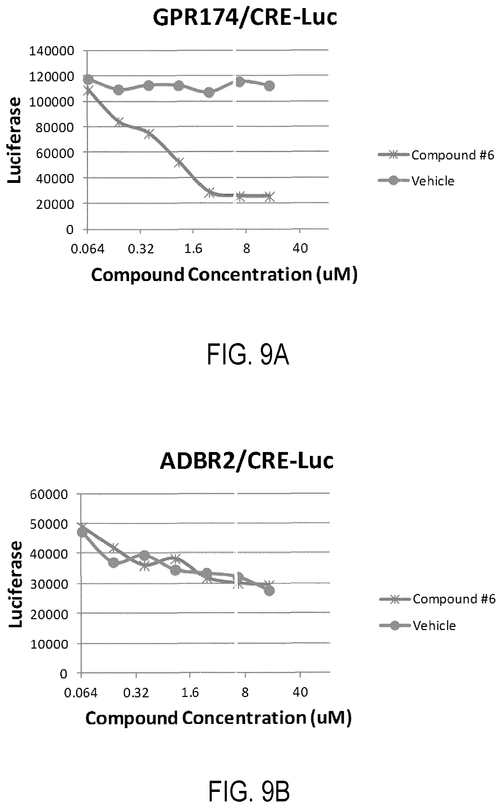

As shown in FIGURE 9A, compound 6 (Group I) inhibited the Gs pathway in the presence of GPR174 but did not inhibit the Gs pathway in the presence of ADBR2 (FIGURE 9B), indicating that compound 6 is a specific inverse agonist of GPR174.

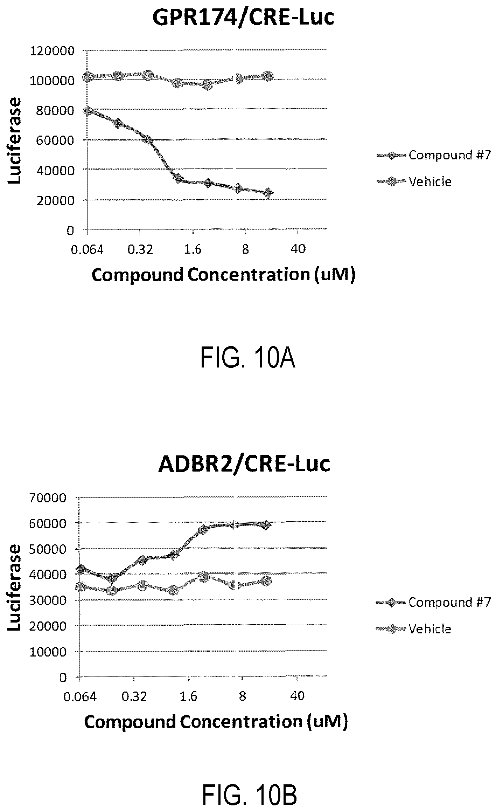

As shown in FIGURE 10A, compound 7 (Group I) inhibited the Gs pathway in the presence of GPR174 but did not inhibit the Gs pathway in the presence of ADBR2 (FIGURE 10B), indicating that compound 7 is a specific inverse agonist of GPR174.

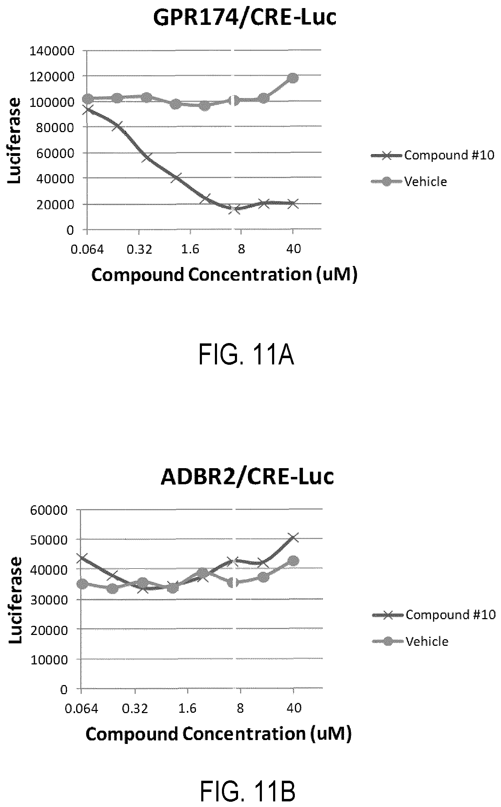

As shown in FIGURE 11A, compound 10 (Group I) inhibited the Gs pathway in the presence of GPR174 but did not inhibit the Gs pathway in the presence of ADBR2 (FIGURE 11B), indicating that compound 10 is a specific inverse agonist of GPR174.

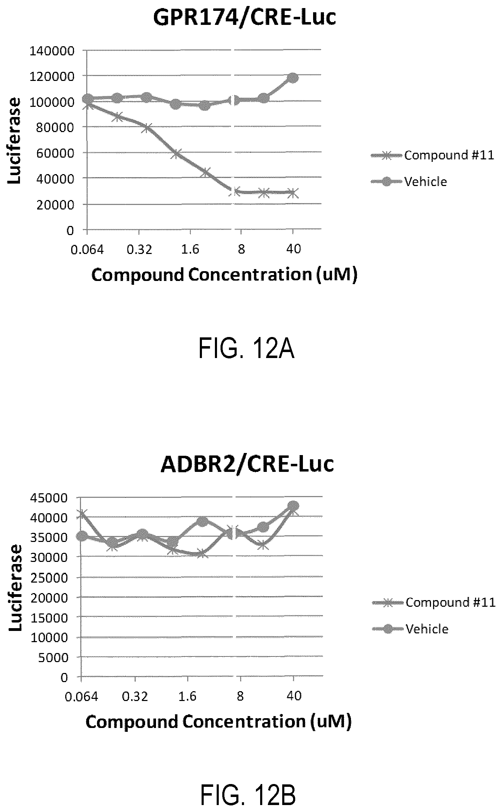

As shown in FIGURE 12A, compound 11 (Group I) inhibited the Gs pathway in the presence of GPR174 but did not inhibit the Gs pathway in the presence of ADBR2 (FIGURE 12B), indicating that compound 11 is a specific inverse agonist of GPR174.

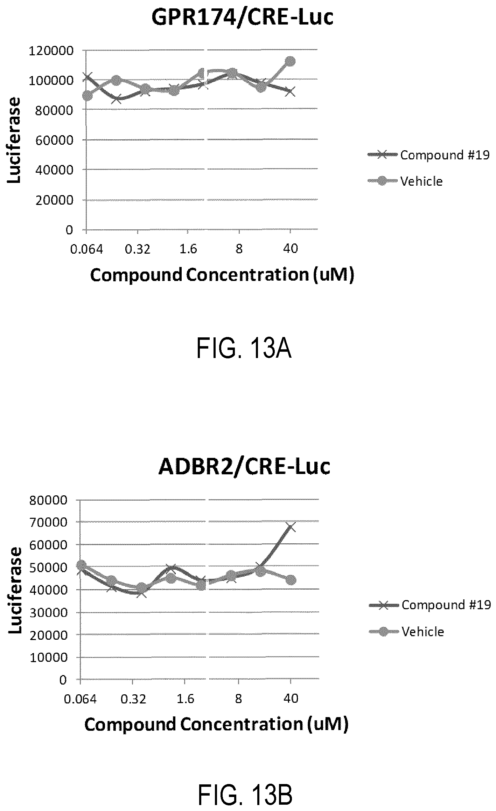

As shown in Table 1, compound 19 has activity in the CRA assay. As shown in FIGURE 13A, compound 19 (Group II) did not modulate the Gs pathway in the presence of GPR174 and did not modulate the Gs pathway in the presence of ADBR2 (FIGURE 13B), therefore compound 19 is characterized as a GPR174 antagonist or allosteric modulator.

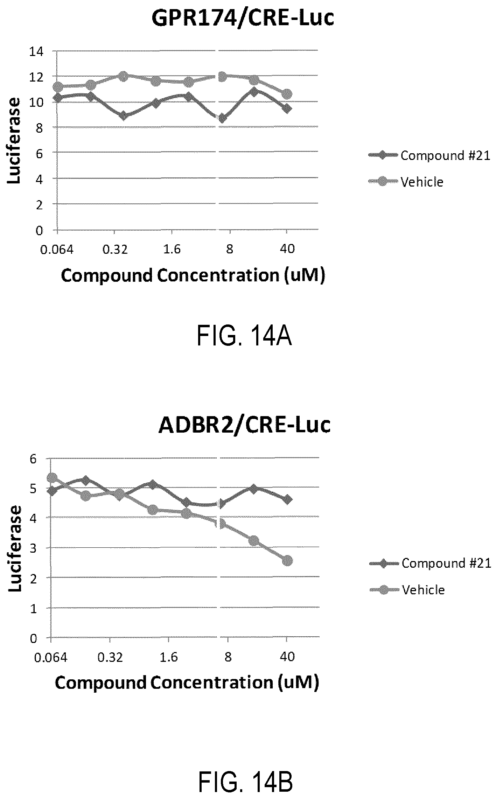

As shown in Table 1, compound 21 has activity in the CRA assay. As shown in FIGURE 14A, compound 21 (Group III) did not modulate the Gs pathway in the presence of GPR174 and did not modulate the Gs pathway in the presence of ADBR2 (FIGURE 14B), therefore compound 21 is characterized as a GPR174 antagonist or allosteric modulator.

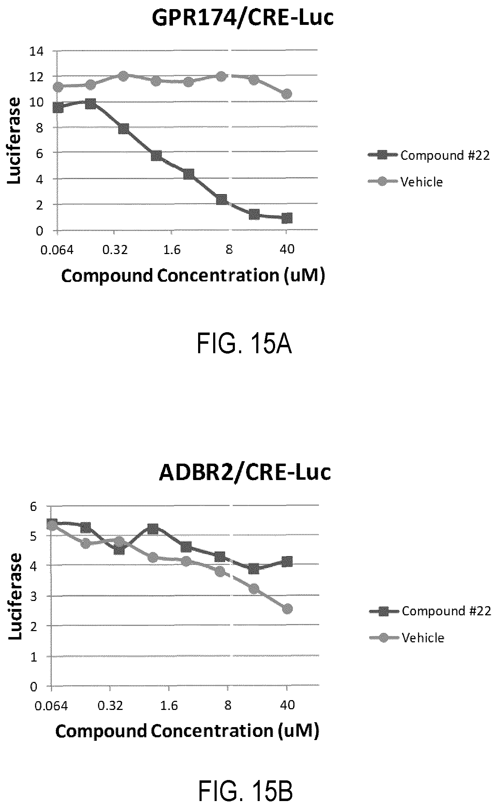

As shown in FIGURE 15A, compound 22 (Group IV) inhibited the Gs pathway in the presence of GPR174 but did not inhibit the Gs pathway in the presence of ADBR2 (FIGURE 15B), indicating that compound 22 is a specific inverse agonist of GPR174.

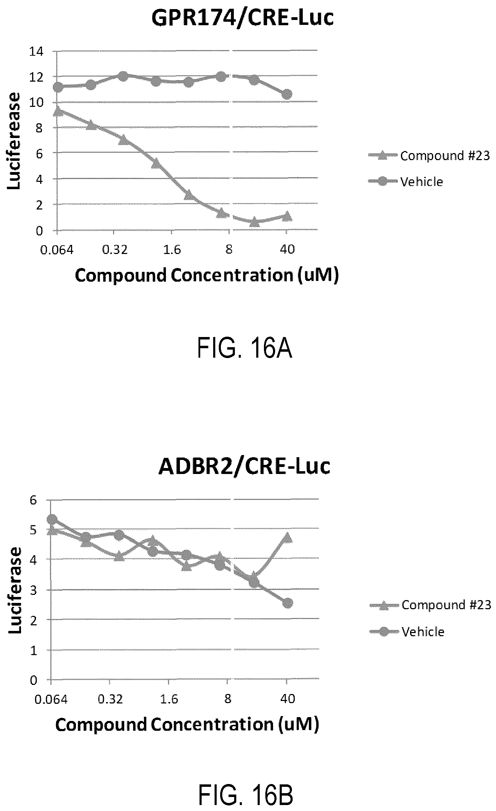

As shown in FIGURE 16A, compound 23 (Group IV) inhibited the Gs pathway in the presence of GPR174 but did not inhibit the Gs pathway in the presence of ADBR2 (FIGURE 16B), indicating that compound 23 is a specific inverse agonist of GPR174.

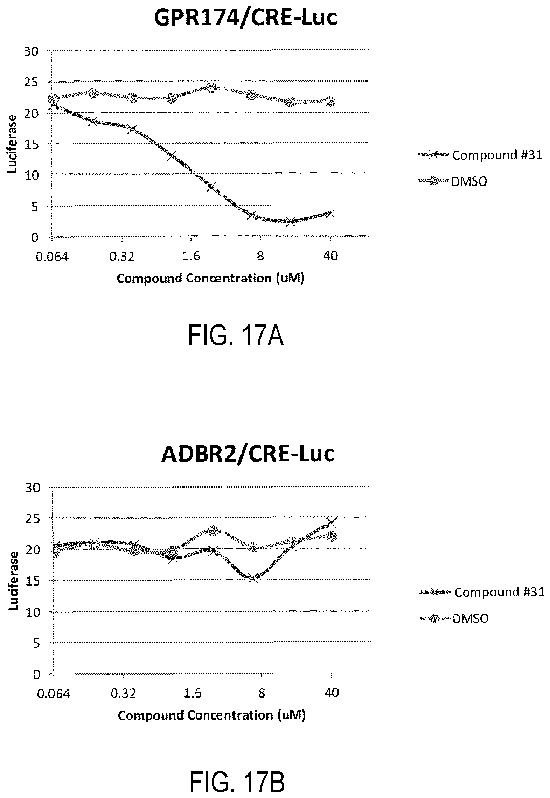

As shown in FIGURE 17A, compound 31 (Group IV) inhibited the Gs pathway in the presence of GPR174 but did not inhibit the Gs pathway in the presence of ADBR2 (FIGURE 17B), indicating that compound 31 is a specific inverse agonist of GPR174.

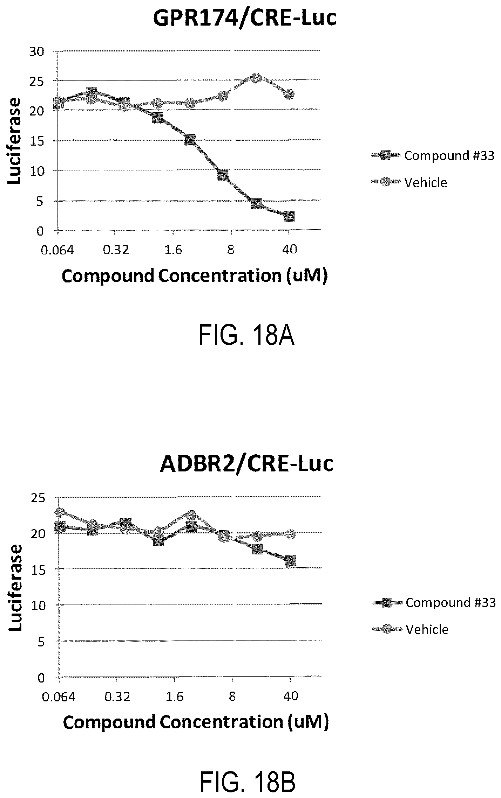

As shown in FIGURE 18A, compound 33 (Group IV) inhibited the Gs pathway in the presence of GPR174 but did not inhibit the Gs pathway in the presence of ADBR2 (FIGURE 18B), indicating that compound 33 is a specific inverse agonist of GPR174.

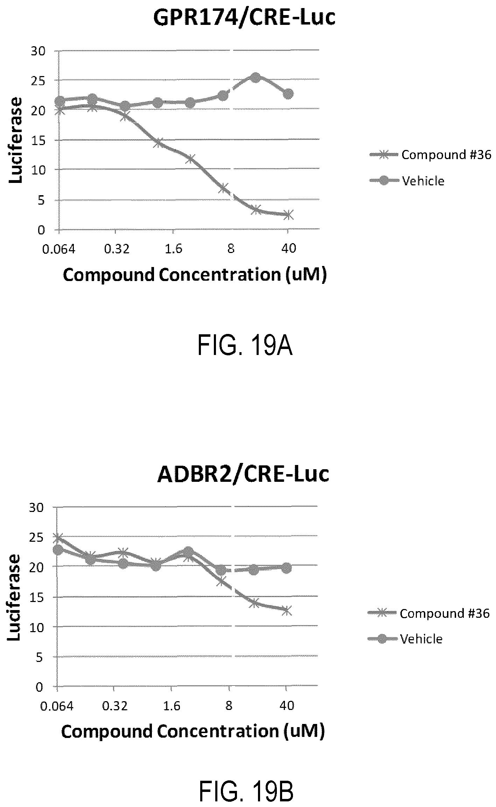

As shown in FIGURE 19A, compound 36 (Group IV) inhibited the Gs pathway in the presence of GPR174 but did not inhibit the Gs pathway in the presence of ADBR2 (FIGURE 19B), indicating that compound 36 is a specific inverse agonist of GPR174.

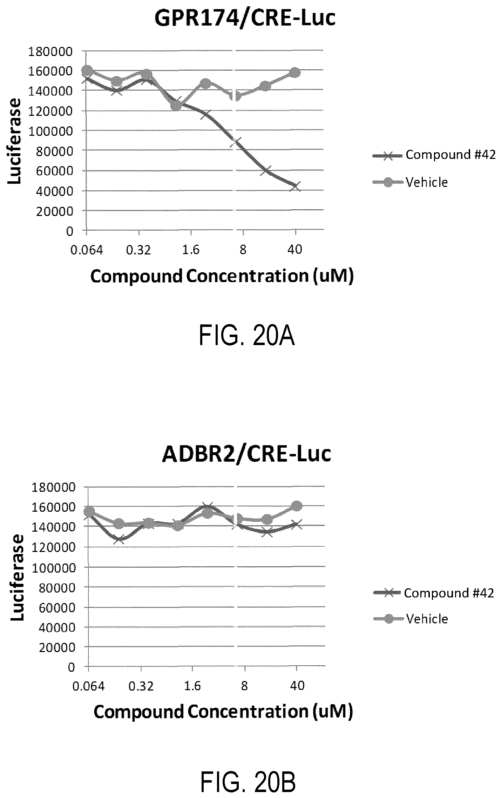

As shown in FIGURE 20A, compound 42 (Group IV) inhibited the Gs pathway in the presence of GPR174 but did not inhibit the Gs pathway in the presence of ADBR2 (FIGURE 20B), indicating that compound 42 is a specific inverse agonist of GPR174.

Compound 4 is confirmed to be an antagonist of GPR174

In order to confirm that GPR174 interacting compound 4 is an antagonist of GPR174-mediated Gs signaling, a GloSensor assay was carried out with increasing concentrations of compound 4 in the presence or absence of a fixed concentration (1 µM) of the GPR174 agonist LysoPS as follows.

The GloSensor Assay was performed by transiently transfecting the GPR174 construct, pCMV6-XL4-GPR174 or ADBR2, pGlo22 (300ng) (Promega) with lipofectamine 2000 (60 µg) (Invitrogen, CA) according to the manufacturer's directions. The DNA/lipofectamine 2000 mix (3000 µL) was incubated at room temperature for 30 minutes and then added into a 10 cm culture dish (Corning, NY) containing 70-90% confluent cultured cells, typically HEK293 or CHO cell lines (ATCC, VA), in 15 mL of media. The cells were further incubated for 4-6 hours at 37°C and then media were removed, and cells were detached with 2 mL trypsin (Life Technologies, CA). Media (26 mL) was added to the detached cells and 100 µL of transfected cells were transferred to each well on a 96-well plate. The cells were further incubated at 37°C overnight. The following day, the media were removed and 100µl of equilibration media (Promega) was added to each well to the manufacturer's directions. The cells were then incubated at room temperature for 2 hours and the cell plates were pre-read with a SpectraMax L (Molecular Devices, CA). After the pre-read, increasing concentrations of each compound in DMSO (1.0 µl) was added to the cells. The cell plates containing the compound were read immediately after the compound was added and at various times up to 30 minutes after the addition of the compound with a SpectraMax L (Molecular Devices, CA). Data were analyzed with either Excel (Microsoft, WA) or Prism 4.03 (GraphPad, CA).

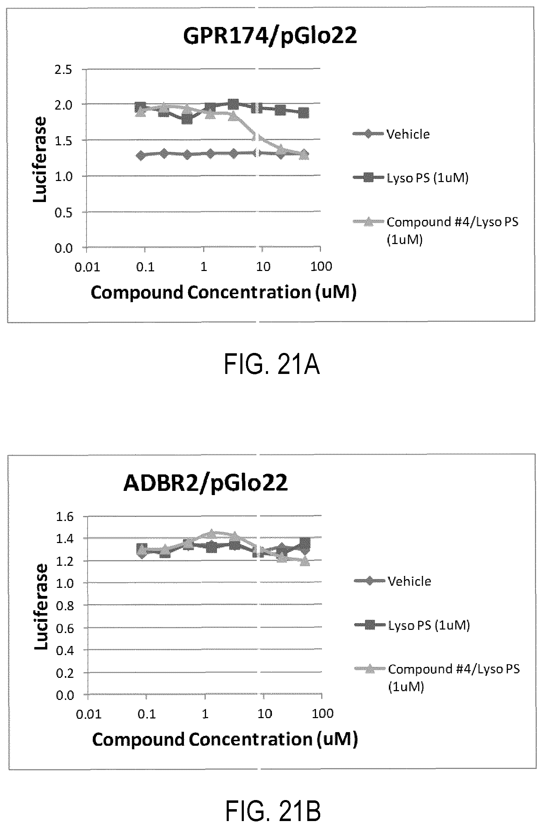

As shown in FIGURE 21A, compound 4 does not modulate the Gs pathway in the presence of GPR174 but does compete with the GPR174 agonist LysoPS. As shown in FIGURE 21B, neither compound 4 nor LysoPS modulate Gs signaling in the presence of ADBR, thereby confirming that compound 4 is a specific antagonist for GPR174.

In view of these results, compounds 1, 2, 5-18 and 22-55 are characterized as GPR174 inverse agonists. Compounds 3, 19-21 and 57-58 are characterized as GPR174 antagonists or allosteric modulators. Compound 4 is characterized as a GPR174 antagonist. The modulation of GPR174 signaling observed in the CRE-Luc reporter assays demonstrates that GPR174 signals through Gs. As described in this example, the inventors have definitively determined that compounds that functionally interact with GPR174 modulate GPR174 Gs signaling.

### EXAMPLE 5

### GPR174 EXPRESSION PROFILE IN HUMAN TISSUES BY RT-PCR

The expression profile of GPR174 was analyzed in human tissues as follows.

The human normal cDNA array was purchased from OriGene (Cat #HMRT103). Quantitative PCR (qPCR) was performed on each cDNA sample in quadruplicates with GAPDH (normalization control) and GPR174 specific primers. FIGURE 22 graphically illustrates the relative transcript abundance of GPR174 in human tissues, as measured by qPCR. As shown in FIGURE 22, GPR174 is strongly expressed in variety of tissues including the thymus, lymph nodes, spleen and bone marrow.

In order to determine the expression profile of GPR174 in human lymphoid cells, human neutrophils, dendritic cells, B cells, CD4+ T cells and CD8+ T cells were purchased from Astarte Biologics. RNA was isolated using PureLink RNA Micro Scale Kit (Life Technologies). cDNA was produced from total RNA using SuperScript III First-Strand Synthesis Kit (Life Technologies) with random priming. Quantitative PCR (qPCR) was performed on each cDNA sample in quadruplicates with GAPDH (normalization control) and GPR174 specific primers. FIGURE 23 graphically illustrates the relative transcript abundance of GPR174 in human lymphoid cells, as measured by qPCR. As shown in FIGURE 23, within the human lymphoid tissues, GPR174 is expressed at high levels in naive B and T cells, and especially in regulatory T cells (see Barnes et al., 2015, *supra*).

### EXAMPLE 6

This Example demonstrates that representative GPR174 inhibitory compound 10 reduces the fraction of highly suppressive T-Regs (FoxP3⁺Helios⁺) in cultured human PBMCs

The following experiments were carried out to analyze whether representative GPR174 inhibitory compound 10 (Group I) is capable of modulating regulatory T cell (Treg) behavior.

An experiment was carried out with human peripheral blood mononuclear cells (PBMCs) to test Treg formation and survival using methods described in Gavin et al., Proc Natl Acad Sci 103:6659-664, 2006.

Briefly described, human PBMCs, obtained from a volunteer donor, were adjusted to a density of 2 × 10⁶ cells/mL, stimulated with 100 ng/mL of anti-CD3 and 100 ng/mL of anti-CD28 antibodies, and treated with 100 µM of GPR174 inverse agonist compound 10 (shown in Table 1), or 100 U/mL of IL-2. A fraction of the cells was taken for staining on day 3 post-stimulation, while the rest of the cells were split 1:4 with fresh medium and used for staining on day 7 post-stimulation. To determine the fraction of natural Treg (nTreg) cells (FoxP3⁺Helios⁺), PBMCs were washed, stained with fluorescently labeled anti-CD4 antibodies, fixed, permeabilized and stained with a mixture of fluorescent anti-FoxP3 and anti-Helios antibodies. Labeled cells were analyzed by flow cytometry for populations stained for these three markers. Each experiment was performed in triplicate and was repeated five times.

The effect of compound 10 on IL-2 and IFN-γ production in human PBMCs was determined as follows. PBMCs from a single donor were adjusted to a density of 2 × 10⁶ cells/mL, stimulated with 100 ng/mL of anti-CD3 and 100 ng/mL of anti-CD28 antibodies and treated with 1 µM, 3 µM, or 10 µM of compound 10. Supernatant from these cells was taken on day 2 post-stimulation and the levels of IL-2 and IFN-γ were determined by ELISA assay using Ready-SET-Go ELISA kits from Affimetrix Ebioscience according to the manufacturer's instructions.

FIGURE 24A graphically illustrates the percentage of FoxP3+Helios- cells in the CD4+ cell population at day 3 post-stimulation in PBMC cultures treated with vehicle, compound 10, or IL-2 (*p*=0.03 for compound 10 vs vehicle; *p*=0.003 for IL-2 vs vehicle). FIGURE 24B graphically illustrates the percentage of FoxP3⁺Helios⁺ cells in the CD4+ cell population at day 7 post-stimulation in PBMC cultures treated with vehicle, compound 10, or IL-2 (*p*=0.01 for compound 10 vs. vehicle; *p*=0.01 for IL-2 vs vehicle). As shown in FIGURE 24A, the GPR174 inverse agonist compound 10 significantly increased the fraction of conventional (FoxP3+Helios-) T cells; while at day 7, compound 10 significantly reduced the abundance of double-positive (FoxP3⁺Helios⁺) nTreg cells (FIGURE 24B).

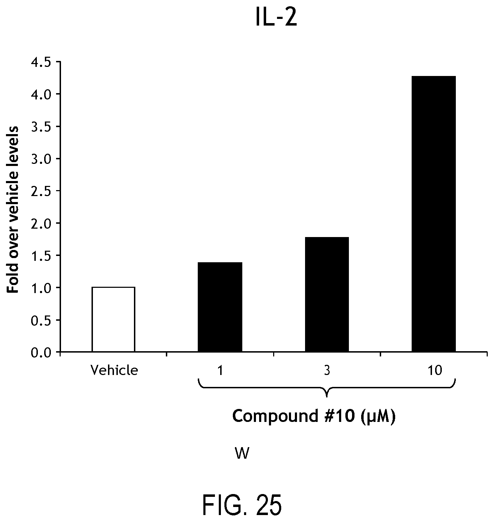

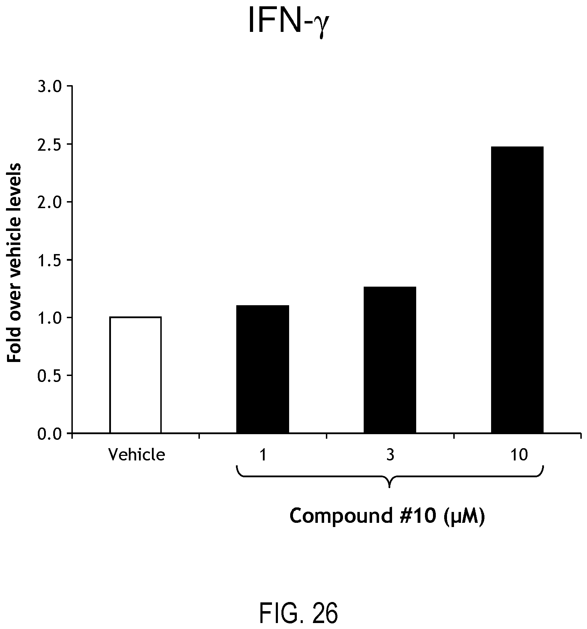

FIGURE 25 graphically illustrates the amount of IL-2 in culture supernatants (fold over vehicle levels) on day 2 post-stimulation in PBMC cultures treated with vehicle or compound 10 (1 µM, 3 µM or 10 µM). FIGURE 26 graphically illustrates the amount of IFN-γ in culture supernatants (fold over vehicle levels) on day 2 post-stimulation in PBMC cultures treated with vehicle or compound 10 (1 µM, 3 µM or 10 µM). As shown in FIGURE 25, compound 10 increased IL-2 levels in a dose-dependent manner and at a concentration as low as 1 µM. Similar effects showing increased IL-2 levels were observed in an experiment with PBMC cultures after treatment with compound 6 (data not shown). Similarly, as shown in FIGURE 26, compound 10 increased interferon gamma (IFN-γ) in a dose-dependent manner.

As described in this Example, cell-based assays were carried out with human PBMCs to test Treg formation and survival along the lines of observations made by Gavin et al. (PNAS 103:6659-6665, 2006). To mimic T cell activation by antigen-presenting cells, PBMCs were stimulated with anti-CD3 and anti-CD28 antibodies, and test articles (either GPR174-targeting compound 10; IL-2; or vehicle control) were added immediately thereafter. Cells were collected 3 and 7 days later, fixed, stained with fluorescent antibodies against helper T cell marker CD4 and Treg transcription factors forkhead box P3 (FoxP3) and Helios.

Following T cell activation, FoxP3 is transiently upregulated in conventional (*i.e.,* effector T cells) (FoxP3⁻Helios⁻) T cells, generating a FoxP3⁺ Helios- population on day 3 that is reduced or absent by day 7. In contrast, the pre-existing natural Treg (nTreg) population, defined as FoxP3⁺Helios⁺, persists during this time frame and still represents 1-5% of CD4⁺ T cells on day 7. Because nTreg cells do not proliferate as rapidly as conventional T cells following T cell activation, the relative abundance of nTreg can decline during this time frame depending on the strength of T cell stimulation.

As shown in FIGURE 24A, the GPR174 inverse agonist compound 10 significantly increased the fraction of conventional (FoxP3⁺Helios⁻) T cells; while at day 7, compound 10 significantly reduced the abundance of double-positive (FoxP3⁺Helios⁺) nTreg cells (FIGURE 24B). These results indicate that in human PBMCs, inhibition of GPR174 enhances the activation of the immune system while also reducing the abundance of nTreg cells. This is a surprising finding in view of the mouse data described in Barnes et al. (2015, *supra*), which showed that in male mice lacking GPR174, Treg cell numbers were significantly increased in certain tissues. As shown in this Example, in human cells GPR174 inhibition has the opposite effect on Tregs compared to GPR174 inhibition in mouse cells. It is noted that differences in effector and regulatory T cell subsets of human versus those of mice have also been described for another GPCR, GPR15 (see Nguyen et al., Nat Immunol 16:207-213, 2015).

To further evaluate the properties of GPR174 inhibition in human cells, we examined the levels of select cytokines produced in response to treatment of PBMCs with increasing concentrations of compound 10. As shown in FIGURE 25, compound 10 increased IL-2 levels in a dose-dependent manner and at a concentration as low as 1 µM. Similarly, as shown in FIGURE 26, compound 10 increased interferon gamma (IFN-γ) in a dose-dependent manner.

These results indicate the GPR174 inhibition potentiates the activity of effector T cells, a cell population important for the treatment of inflammatory conditions and cancer (Ramsay et al., British Journal of Haematology 162:313-325, 2013). In addition, the results indicate that GPR174 inhibition reduces the abundance of Tregs. The reduction of immunosuppressive Tregs should also be useful in cancer immunotherapy, as it is well established that tumor microenvironment is selectively enriched in Tregs that, in turn, contribute to the failure of immune surveillance in detection of these tumors (see, *e.g.,* Takeuchi and Nishikawa, Roles of regulatory T cells in cancer immunity, International Immunol 28:401-409, 2016). The reduction of Treg cells should improve the outcomes of cancer treatments. Tumors employ multiple ways to evade immune surveillance. One of these strategies is by creating an immunosuppressive microenvironment by promoting Treg development through induction of TGF-β, or other means. Tregs have been found in a variety of cancers and might expand specifically in response to tumor antigens. In mouse models, reduction or elimination of Tregs increases the bodies resistance to cancer (Klages et al., Cancer Res 70:7788-7799, 2010; Li et al., 2010; Teng et al., Cancer Res 70:7800-7809, 2010; Bos et al., J Exp Med, 210:2435-2466, 2013). High frequency of Treg cells in solid organ tumors most often correlates with poor patient outcomes. Meta-analysis of multiple studies revealed that Treg infiltration in tumors correlates with poor prognosis for overall survival in breast and lung cancer, and also in other cancers such as colorectal, cervical, renal, ovarian, melanoma, pancreatic, hepatocellular and gastric (Shang et al., Sci Rep 5:15179-15187, 2015).

Clinical efficacy after immune checkpoint blockade is associated with the somatic mutational burden in the tumor cells (Rizvi et al., Science 348:124-128, 2015; Snyder et al., N. Engl. J. Med. 371:2189-2199, 2014; Van Allen et al., Science 350: 207-211, 2015; Le et al., N. Engl. J. Med. 372:2509-2520, 2015). That is, clinical benefit of such therapy is limited to those patients whose cancer cells are harboring mutation-derived neoantigens and are being recognized as "non-self" by the immune system (Matsushita et al., Nature 482:400-404, 2012; Gubin et al., Nature 515:577-581, 2014). Tregs engaged in self-tolerance favorably control the activation of T cell responses to cancer antigens that are derived from self-constituents (shared antigens) but are less suppressive to T cells recognizing foreign antigens (Maeda et al., Science 346:1536-1540, 2014). Therefore, it is anticipated that integration of approaches for reducing the suppressive activity and/or number of Tregs with approaches for blocking immune checkpoint molecules will broaden the therapeutic spectrum of cancer immunotherapy to cancer patients who have a lower number of neoantigens.

### EXAMPLE 7

This Example describes an analysis of the effect of GPR174 inhibitory compound 10 on IL-2 production in mouse splenocytes obtained from wild-type or GPR174 knockout mice.

GPR174 knockout (KO) mice (referred to also as "FKSG79") were generated as described in Gragerov A. et al., PNAS vol 104(36):14406-14411, 2007. Splenocyte cultures were generated from four GPR174 KO mice and four wild-type (WT) mice. Single-cell suspension of individual mouse spleens were pelleted by centrifugation, resuspended in RBC lysis buffer to remove erythrocytes, spun down again and resuspended in T cell medium (RPMI-1640, 10% FBS, 6 mM L-Glutamine, 12.5 mM HEPES, 50 µM 2-mercaptoethanol, Pen/Strep). Thus, prepared splenocytes were plated into 96-well plates at a density of 3 × 10⁶ cells/mL, 250 µL/well and stimulated with 100 ng/mL of anti-CD3 antibodies and 100 ng/mL of anti-CD28. Cells were treated with either vehicle (0.1% DMSO) or GPR174 inhibitory Compound 10 (10 µM). 48 hours later, cell supernatants were analyzed for cytokine levels using LEGENDplex mouse Th1 cytokine detection kit (BioLegend). Four GPR174 KO and four WT spleens were analyzed, each in triplicate.

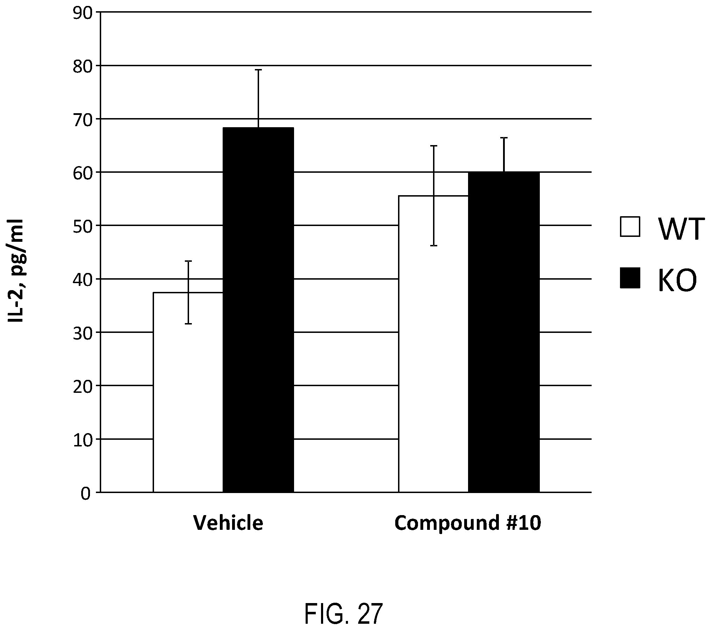

FIGURE 27 graphically illustrates the amount of IL-2 (pg/mL) in culture supernatants 48 hours after stimulation in splenocyte cultures from WT or GPR174 KO mice treated with vehicle or compound 10 (10 µM). As shown in FIGURE 27, the level of IL-2 is increased by treatment with GPR174 inhibitory compound 10 in WT, but not GPR174 KO splenocytes, where IL-2 is high without the treatment. The *p*-value for the difference between KO and WT in vehicle-treated splenocytes is *p*=0.02. There is no significant difference between KO and WT in the compound 10-treated group, or between compound 10 treated cells and vehicle-treated KO cells.

These results indicate that the effect of compound 10 on IL-2 production (described in Example 6 and shown in FIGURE 25) is mediated by GPR174 inhibition.

### EXAMPLE 8

This Example demonstrates that representative GPR174 inhibitory compound 10 reduces the fraction of immunosuppressive T-Regs (FoxP3⁺Helios⁺) and stimulates IL-2 production in a dose-dependent manner in cultured human peripheral blood mononuclear cells (PBMCs) obtained from a single donor.

An experiment was carried out with human peripheral blood mononuclear cells using methods similar to those described above in Example 6, with the difference being that the effect of GPR174 inhibitory compound 10 was analyzed at a range of concentrations (3 µM to 60 µM).

Briefly, human PBMCs, obtained from a volunteer donor, were adjusted to a density of 2 × 10⁶ cells/mL, stimulated with 100 ng/mL of anti-CD3 and 100 ng/mL of anti-CD28 antibodies, and treated with 3 µM, 10 µM or 30 µM of GPR174 inverse agonist compound 10 (shown in Table 1), or vehicle control. A fraction of the cells was taken for staining on day 3 post-stimulation, while the rest of the cells were split 1:4 with fresh medium and used for staining on day 7 post-stimulation. To determine the fraction of natural Treg (nTreg) cells (FoxP3⁺Helios⁺), PBMCs were washed, stained with fluorescently labeled anti-CD4 antibodies, fixed, permeabilized and stained with a mixture of fluorescent anti-FoxP3 and anti-Helios antibodies. Labeled cells were analyzed by flow cytometry for populations stained for these three markers. Each experiment was performed in triplicate and was repeated three times.

The effect of compound 10 on IL-2 production in human PBMCs was determined as follows. PBMCs from a single donor were adjusted to a density of 2 × 10⁶ cells/mL, stimulated with 100 ng/mL of anti-CD3 and 100 ng/mL of anti-CD28 antibodies and treated with 3 µM, 10 µM or 30 µM of compound 10. Supernatant from these cells was taken on day 3 post-stimulation and the levels of IL-2 were determined by ELISA assay using Ready-SET-Go^{™} ELISA kits from Affimetrix Ebioscience and LEGENDplex^{™} bead-based immunoassays from BioLegend according to the manufacturer's instructions.

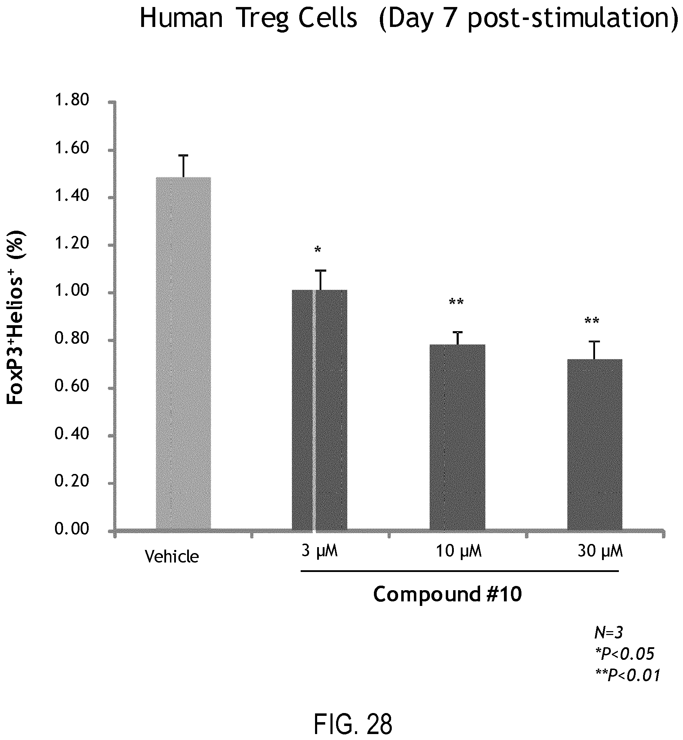

FIGURE 28 graphically illustrates the percentage of FoxP3⁺Helios⁺ cells in the CD4⁺ cell population at day 7 post-stimulation in PBMC cultures treated with vehicle or compound 10 (3 µM, 10 µM or 30 µM) (n=3, **p<*0.05; ***p<*0.01). As shown in FIGURE 28, at day 7 post-stimulation, the GPR174 inverse agonist compound 10 significantly reduced the abundance of double-positive (FoxP3⁺Helios⁺) nTreg cells in a dose-dependent manner, a result consistent with the results described in Example 6.

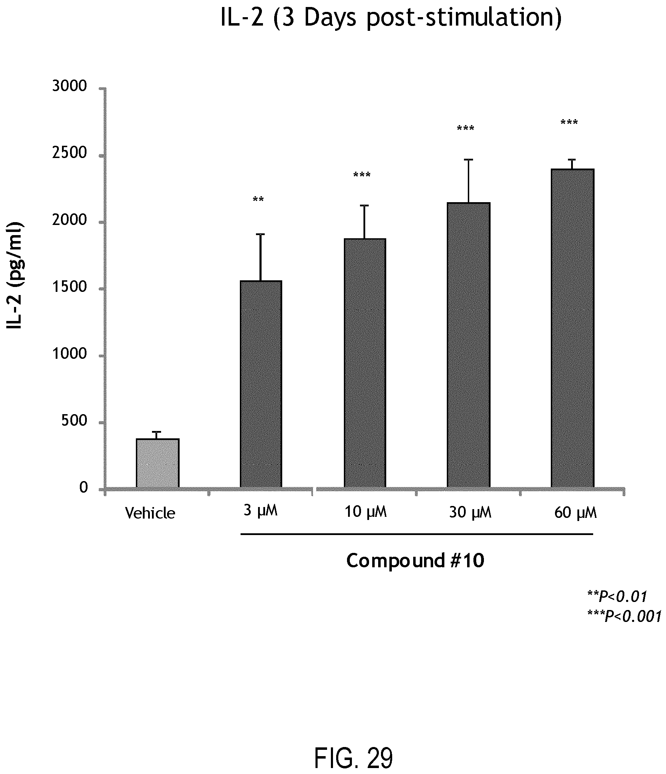

FIGURE 29 graphically illustrates the amount of IL-2 (pg/mL) in culture supernatants on day 3 post-stimulation in PBMC cultures from an individual volunteer, the PBMC were treated with vehicle or GPR174 inhibitory compound 10 (3 µM, 10 µM, 30 µM or 60 µM). As shown in FIGURE 29, compound 10 increased IL-2 levels in culture supernatants in a dose-dependent manner, which is consistent with the results described in Example 6. IL-2 is a major cytokine that determines T cell survival and differentiation into effector, memory, and regulatory T cells.

### EXAMPLE 9

This Example describes the analysis of the effect of representative GPR174 inhibitory compound 10 on Tregs, IL-2 and other cytokines in PBMC obtained from multiple donors.

To validate and further extend the results described in Examples 6 and 8, the effect of representative GPR174 inhibitory compound 10 on Tregs, IL-2 and other cytokines were analyzed in cultured human PBMC obtained from multiple volunteer donors.

Briefly, human PBMC, obtained from eight volunteer donors, were adjusted to a density of 2 × 10⁶ cells/mL, stimulated with 100 ng/mL of anti-CD3 and 100 ng/mL of anti-CD28 antibodies, and treated with 30 µM of GPR174 inverse agonist compound 10 (shown in Table 1). A fraction of the cells was taken for staining on day 2 post-stimulation, while the rest of the cells were split 1:4 with fresh medium and used for staining on day 7 post-stimulation. To determine the fraction of natural Treg (nTreg) cells (FoxP3⁺Helios⁺), PBMC were washed, stained with fluorescently labeled anti-CD4 antibodies, fixed, permeabilized and stained with a mixture of fluorescent anti-FoxP3 and anti-Helios antibodies. Labeled cells were analyzed by flow cytometry for populations stained for these three markers. Each experiment was performed in triplicate and was repeated five times.

The effect of compound 10 on IL-2 production in human PBMC from each donor was determined as follows. PBMC from each donor were adjusted to a density of 2 × 10⁶ cells/mL, stimulated with 100 ng/mL of anti-CD3 and 100 ng/mL of anti-CD28 antibodies and treated with 30 µM compound 10. Supernatant from these cells was taken on day 2 post-stimulation and the levels of IL-2 were determined by ELISA assay using LEGENDplex^{™} bead-based immunoassays from BioLegend (San Diego, CA) according to the manufacturer's instructions.

The effect of compound 10 on the production of a panel of additional cytokines (interleukin 5 (IL-5), interleukin 13 (IL-13), interleukin 6 (IL-6), interleukin 9 (IL-9), interleukin 10 (IL-10), interferon gamma (IFN-γ), tumor necrosis factor alpha (TNF-α), interleukin 17A (IL-17A), interleukin 17F (IL-17F), interleukin 4 (IL-4) and interleukin 22 (IL-22)) in pooled human PBMC obtained from eight donors was determined as follows. PBMC from eight donors were pooled, then adjusted to a density of 2 × 10⁶ cells/mL, stimulated with 100 ng/mL of anti-CD3 and 100 ng/mL of anti-CD28 antibodies and treated with 30 µM compound 10. Supernatant from these cells was taken on day 2 post-stimulation and the levels of IL-5, IL-13, IL-6, IL-9, IL-10, IFN-γ, TNF-α, IL-17A, IL-17F, IL-4 and IL-22 were determined by ELISA using LEGENDplex^{™} bead-based immunoassays from BioLegend (San Diego, CA) according to the manufacturer's instructions.

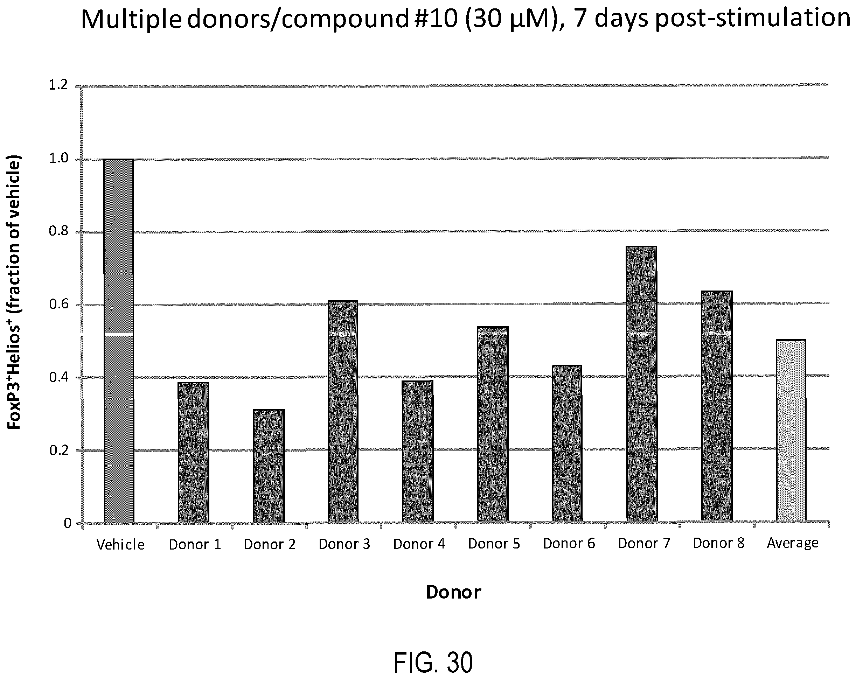

FIGURE 30 graphically illustrates the fraction of vehicle of FoxP3⁺Helios⁺ cells in the CD4⁺ cell population at day 7 post-stimulation in human PBMC cultures from eight different donors treated with vehicle or compound 10 (30 µM). As shown in FIGURE 30, at day 7 post-stimulation, the GPR174 inverse agonist compound 10 significantly reduced the abundance of double-positive (FoxP3⁺Helios⁺) nTreg cells in PBMC cultures from all eight donors, a result consistent with the results described in Examples 6 and 8.

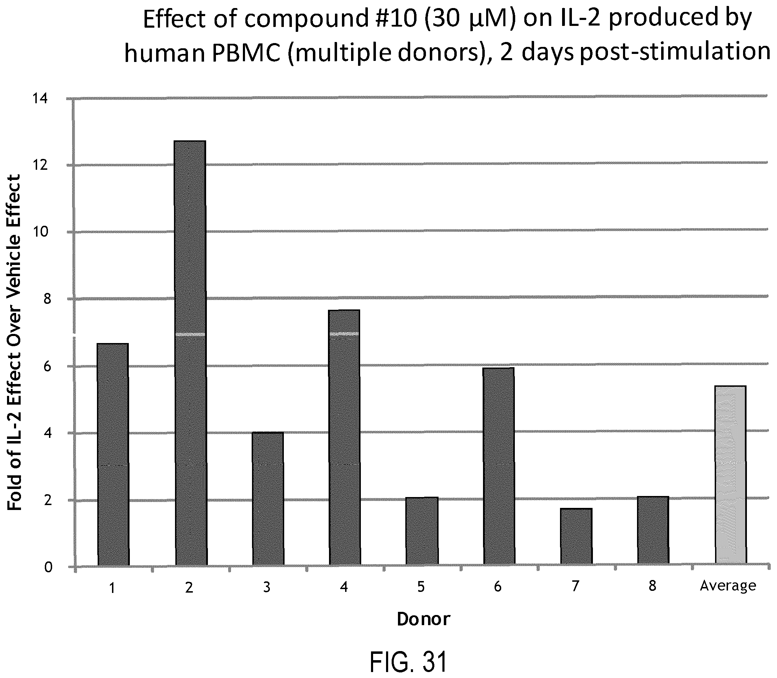

FIGURE 31 graphically illustrates the amount of IL-2 (fold over vehicle) in culture supernatants on day 2 post-stimulation in human PBMC cultures from eight different donors treated with vehicle or compound 10 (30 µM). As shown in FIGURE 31, compound 10 increased IL-2 levels in each of the PBMC cultures from eight donors, with an average of about 5-fold, which is consistent with the results described in Examples 6 and 8.

FIGURE 32 graphically illustrates the amount of various cytokines (fold over vehicle) in culture supernatants on day 2 post-stimulation from human PBMC obtained from eight donors treated with vehicle of compound 10 (30 µM). As shown in FIGURE 32, a statistically significant fold increase was observed for IL-6 (****p*<0.001), IL-10 (**p*<0.05), IFN-γ (*p<0.05) and TNF-α (*p<0.05) as compared to vehicle control. As further shown in FIGURE 32, a statistically significant fold decrease was observed for IL-17A (***p<0.001) as compared to vehicle control.

It is noted that IFN-γ induction was also observed in non-human primate, dog and rat, but not in mouse PBMCs.

The results obtained in this Example validate and further extend the results described in Examples 6 and 8 and show that a representative GPR174 inhibitor, compound 10, significantly reduced the population of Tregs in each of the human PBMC cultures from eight different donors. It is further demonstrated in pooled human PBMCs obtained from eight donors that GPR174 inhibition increases, with statistical significance, the levels of cytokines IL-2 and IFN-γ, as well as IL-6, IL-10 and TNF-α. The increase in this subset of cytokines (IL-2, IFN-γ, IL-6, IL-10 and TNF-α) indicates that GPR174 inhibition activates the Th1 subset of T cells that mainly generate responses against intracellular infectious agents.

### EXAMPLE 10

This Example describes an analysis of the effect of representative GPR174 inhibitory compound 10 on cytokines produced in mixed culture splenocytes obtained from different mouse strains.

In order to stimulate T cells in mice, splenocytes obtained from two different mouse strains, C57BL/6 and DBA1, were mixed in culture.

An initial experiment was carried out to determine the time course of cytokine production of the co-cultured splenocytes in which cytokine production (IFN-γ, IL-5, TNF-α, IL-2, IL-6, IL-4, IL-10, IL-9, IL-17A, IL-17F, IL-21, IL-22 and IL-13) was measured in the co-culture supernatant at day 2, day 4, and day 6 after mixing in the absence of compound 10. The results are shown below in Table 7.

**Table 7: C57BL/6 + DBA1 splenocyte co-culture**

| cytokine | Day 2 (pg/mL) | Day 4 (pg/mL) | Day 6 (pg/mL) |
|---|---|---|---|
| IFN-γ | 871 | 36030 | 8648 |
| IL-5 | 18 | 18 | 20 |
| TNF-α | 15 | 96 | 46 |
| IL-2 | 263 | 1079 | 504 |
| IL-6 | 14 | 369 | 106 |
| IL-4 | 11 | 30 | 13 |
| IL-10 | 22 | 93 | 50 |
| IL-9 | 25 | 78 | 78 |
| IL-17A | 18 | 161 | 49 |
| IL-17F | 16 | 11 | 7 |
| IL-21 | 26 | 26 | 26 |
| IL-22 | 24 | 1098 | 288 |
| IL-13 | 18 | 33 | 32 |

As shown above in Table 7, peak levels for each cytokine measured in the C57BL/6 + DBA1 splenocyte co-culture were observed at day 4 and maintained at day 6 after mixing.

The effect of compound 10 on production of cytokines (IFN-γ, IL-5, TNF-α, IL-2, IL-6, IL-4, IL-10, IL-9, IL17A, IL17F, IL-21, IL-22 and IL-13) in mouse splenocyte cocultures was determined as follows. Splenocytes obtained from two different mouse strains, C57BL/6 and DBA1, were mixed in culture in the presence or absence of 30 µM compound 10. Cytokine production of IFN-γ, IL-5, TNF-α, IL-2, IL-6, IL-4, IL-10, IL-9, IL-17A, IL-17F, IL-21, IL-22 and IL-13 was measured in the co-culture supernatant at day 4 and day 6 after mixing. Cytokine levels were determined by LEGENDplex^{™} bead-based immunoassays from BioLegend (San Diego, CA) according to the manufacturer's instructions.

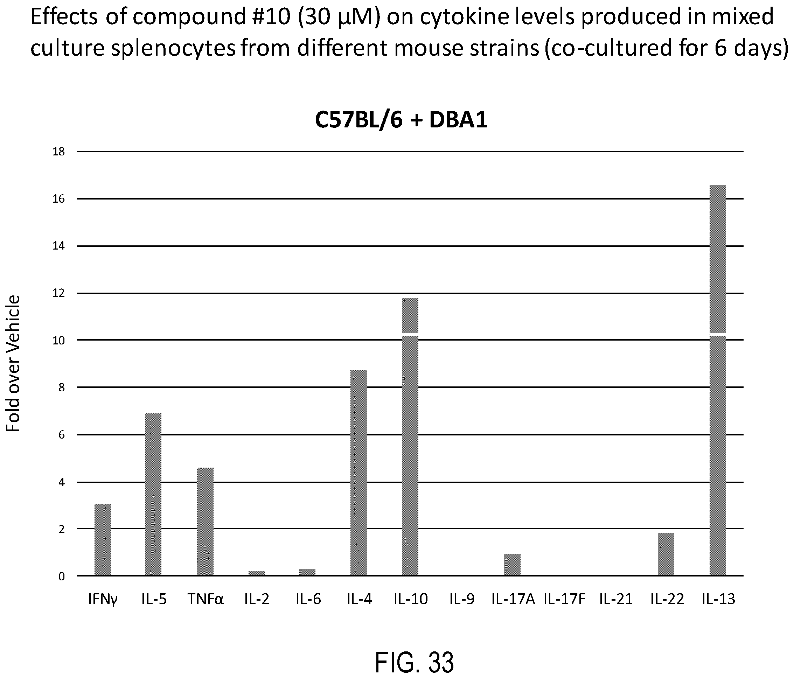

FIGURE 33 graphically illustrates the fold induction by compound 10 (30 µM) on a panel of cytokines in co-cultured mouse C57BL/6+DBA1 splenocytes at day 6 after mixing, as compared to the cytokine levels measured in co-cultured C57BL/6+DBA1 splenocytes on day 6 in the absence of compound 10. These results demonstrate that compound 10 causes a strong alteration in quantities of cytokines produced during splenocyte allogeneic stimulation, indicating that inhibition of GPR174 significantly modulates immune response. It is noted that the apparent difference between the sets of cytokines induced in human PBMCs (as described in Example 9) as compared to mouse splenocytes is not necessarily meaningful, as it is hard to compare responses of a relatively naive immune system of animals bred in a practically sterile and protected facility to those of human volunteers that have been exposed to multiple antigens.

### EXAMPLE 11

This Example describes a study that was carried out to determine what cell types in the human PBMC cultures are responsible for the cytokine production induced by representative GPR174 inhibitory compound 10.

In a first experiment, human PBMCs from two different donors were incubated overnight in the presence or absence of compound 10 (10 µM). The PBMC cultures were stimulated with anti-CD3 and anti-CD28 antibodies and vesicular transport was simultaneously blocked with GolgiStop^{™}. The cells were fixed four hours later and stained with antibodies for cell type markers (FITC-anti-CD3 and PerCP-Cy5.5-anti-CD4) and IL-2 (PE-anti-IL-2). The cells were analyzed by flow cytometry. The fraction of cells with intracellular IL-2 (PE-labeled) was then determined in the CD4⁺ and CD8⁺ T cell populations, as well as in non-T cells.

In a second experiment, human PBMCs from two different donors were stimulated with anti-CD3 and anti-CD28 antibodies and incubated in the absence or presence of increasing concentrations of compound 10 (0, 0.1, 0.3, 1.0, or 3.0 µM). The vesicular transport was blocked two days later with GolgiStop^{™}. The cells were fixed four hours later and stained with antibodies for cell type markers (FITC-anti-CD3, PerCP-Cy5.5-anti-CD4, PE-anti-CD56 and Pacific Blue-anti-CD16) and either of the cytokines IL-2, IL-10 and IFN-γ (anticytokine antibodies were all APC-labeled). The cells were analyzed by flow cytometry. The fraction of cells with intracellular IL-2, IFN-γ, IL-10 and TNF-α was then determined in different cellular populations.

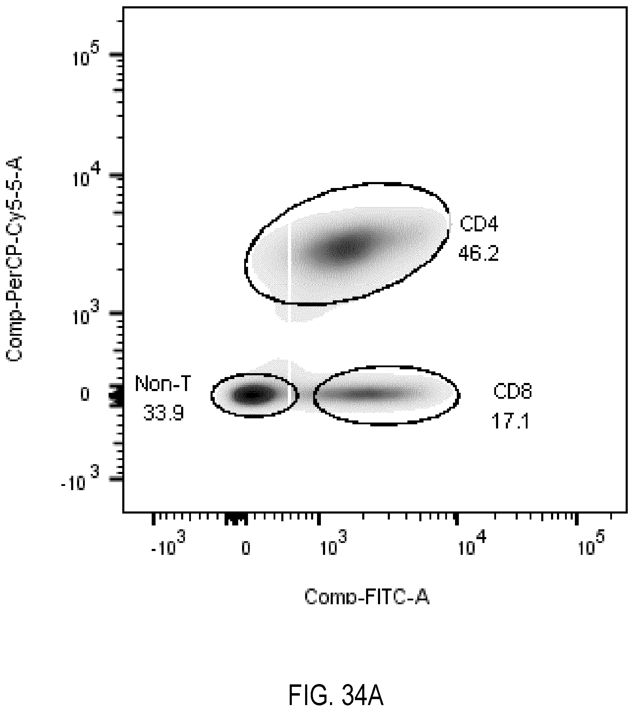

The results of the first experiment for one of the two donors are shown in FIGURES 34A-34C. Similar results were also observed from the second donor (data not shown).

FIGURE 34A shows the CD4⁺ (46.2%), CD8⁺ (17.1%) and non-T cell (33.9%) populations present in a representative PBMC culture four hours after stimulation with anti-CD3 and anti-CD28 antibodies in the presence of compound 10 (10 µM).

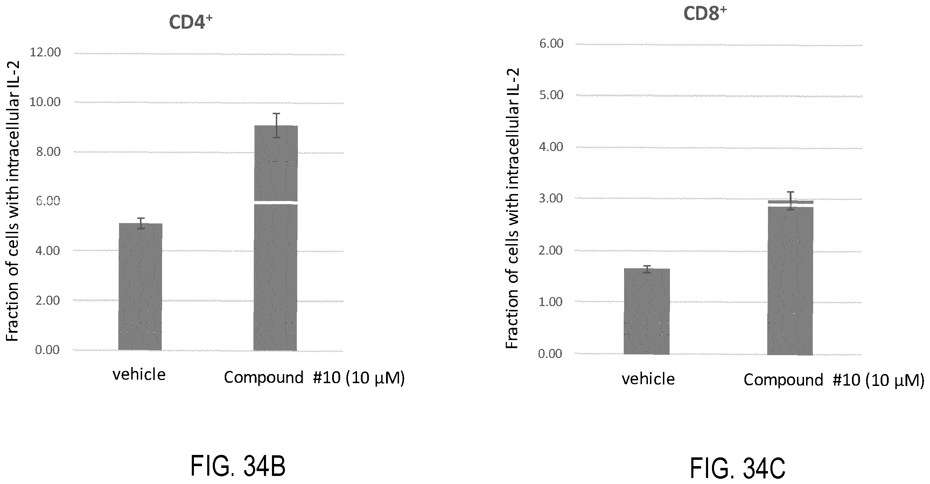

FIGURE 34B graphically illustrates the fraction of cells with intracellular IL-2 staining in the CD4⁺ T cell population shown in FIGURE 34A, which has been treated with compound 10 (10 µM), as compared to vehicle control; and

FIGURE 34C graphically illustrates the fraction of cells with intracellular IL-2 staining in the CD8⁺ T cell population shown in FIGURE 34A, which has been treated with compound 10 (10 µM), as compared to vehicle control.

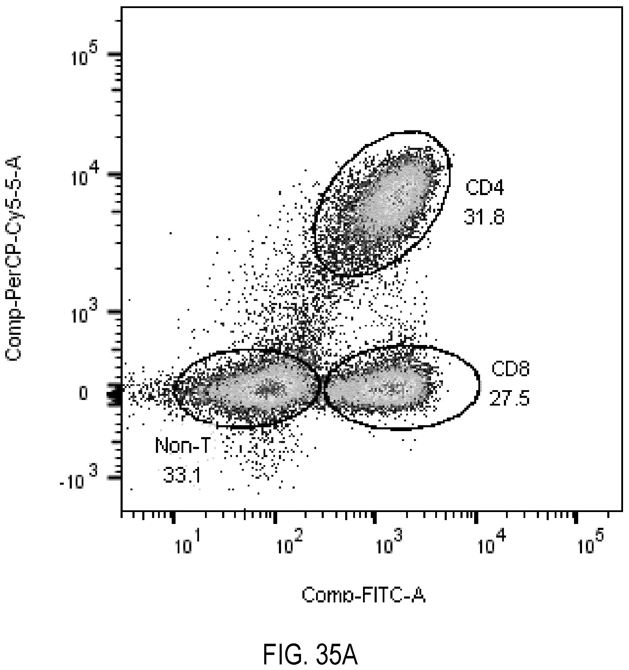

The results of the second experiment for one of the two donors are shown in FIGURES 35A-35M. Similar results were also observed from the second donor (data not shown).

FIGURE 35A shows the CD4⁺ (31.8%), CD8⁺ (27.5%) and non-T cell (33.1%) populations present in a representative PBMC culture 2 days after stimulation with anti-CD3 and anti-CD28 antibodies in the presence of compound 10.

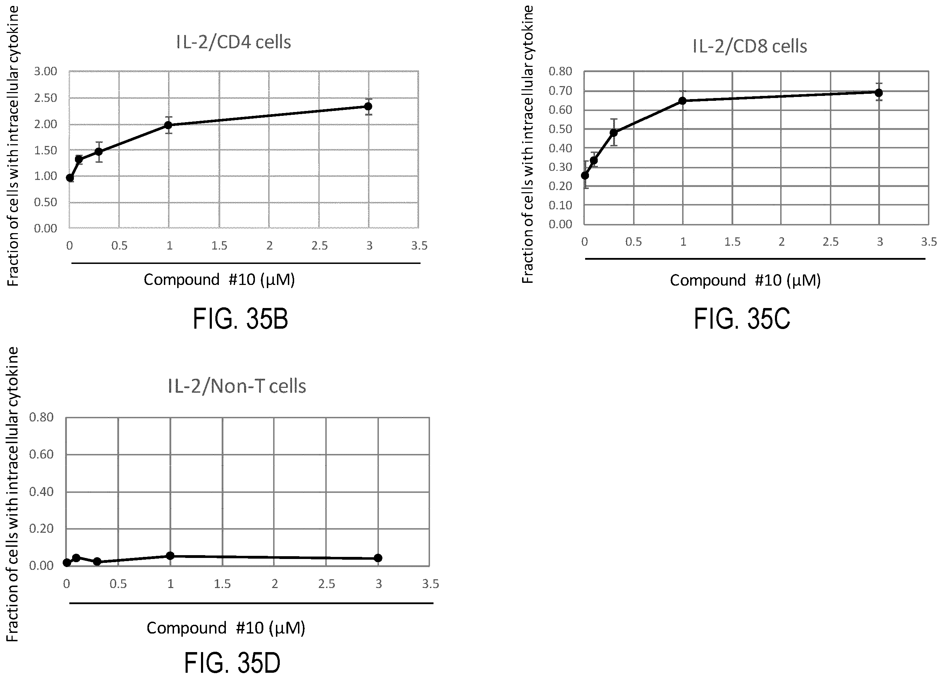

FIGURE 35B graphically illustrates the fraction of cells with intracellular IL-2 staining in the CD4⁺ T cell population in PBMC cultures treated with compound 10 (0, 0.1, 0.3, 1.0 or 3.0 µM), a representative of which is shown in FIGURE 35A.

FIGURE 35C graphically illustrates the fraction of cells with intracellular IL-2 staining in the CD8⁺ T cell population in PBMC cultures treated with compound 10 (0, 0.1, 0.3, 1.0 or 3.0 µM), a representative of which is shown in FIGURE 35A.

FIGURE 35D graphically illustrates the fraction of cells with intracellular IL-2 staining in non-T cell populations in PBMC cultures treated with compound 10 (0, 0.1, 0.3, 1.0 or 3.0 µM), a representative of which is shown in FIGURE 35A.

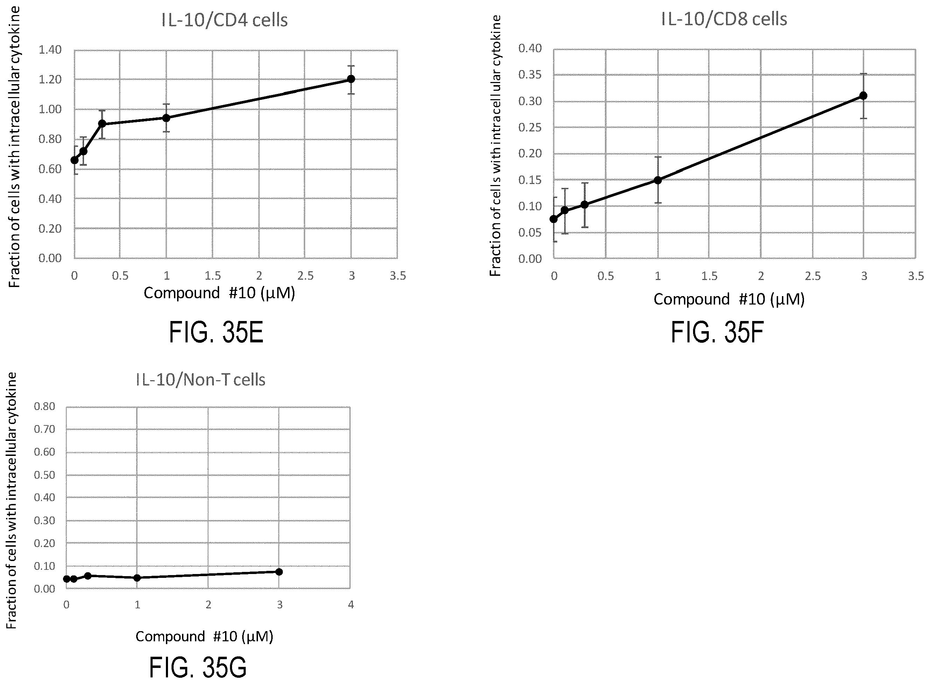

FIGURE 35E graphically illustrates the fraction of cells with intracellular IL-10 staining in the CD4⁺ T cell population in PBMC cultures treated with compound 10 (0, 0.1, 0.3, 1.0 or 3.0 µM), a representative of which is shown in FIGURE 35A.

FIGURE 35F graphically illustrates the fraction of cells with intracellular IL-10 staining in the CD8⁺ T cell populations in PBMC cultures treated with compound 10 (0, 0.1, 0.3, 1.0 or 3.0 µM), a representative of which is shown in FIGURE 35A; and

FIGURE 35G graphically illustrates the fraction of cells with intracellular IL-10 staining in non-T cell populations in PBMC cultures treated with compound 10 (0, 0.1, 0.3, 1.0 or 3.0 µM), a representative of which is shown in FIGURE 35A.

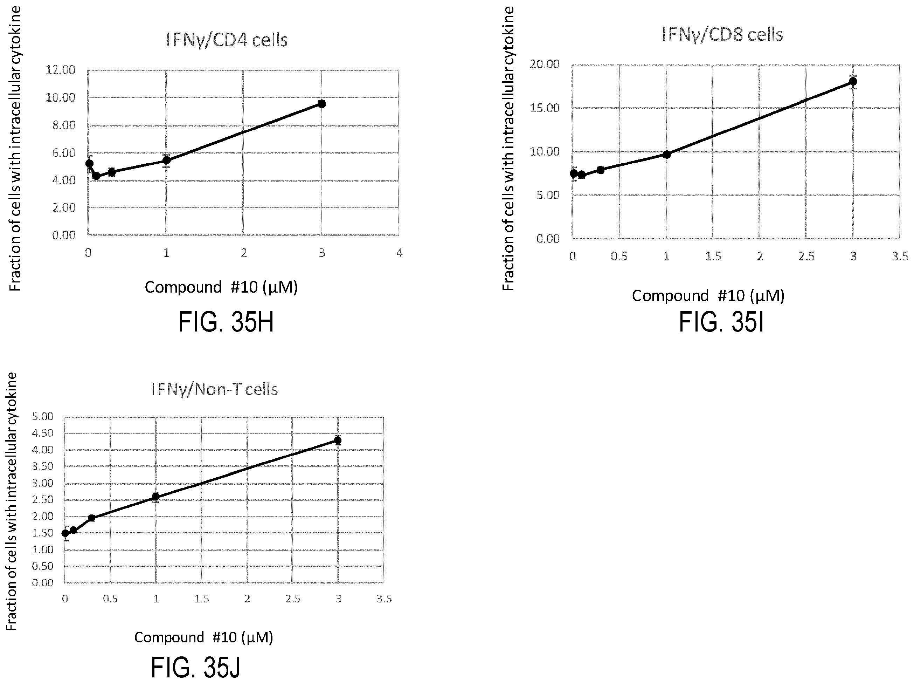

FIGURE 35H graphically illustrates the fraction of cells with intracellular IFN-γ staining in the CD4⁺ T cell population in PBMC cultures treated with compound 10 (0, 0.1, 0.3, 1.0 or 3.0 µM), a representative of which is shown in FIGURE 35A.

FIGURE 35I graphically illustrates the fraction of cells with intracellular IFN-γ staining in the CD8⁺ T cell population in PBMC cultures treated with compound 10 (0, 0.1, 0.3, 1.0 or 3.0 µM), a representative of which is shown in FIGURE 35A.

FIGURE 35J graphically illustrates the fraction of cells with intracellular IFN-γ staining in non-T cell populations in PBMC cultures treated with compound 10 (0, 0.1, 0.3, 1.0 or 3.0 µM), a representative of which is shown in FIGURE 35A.

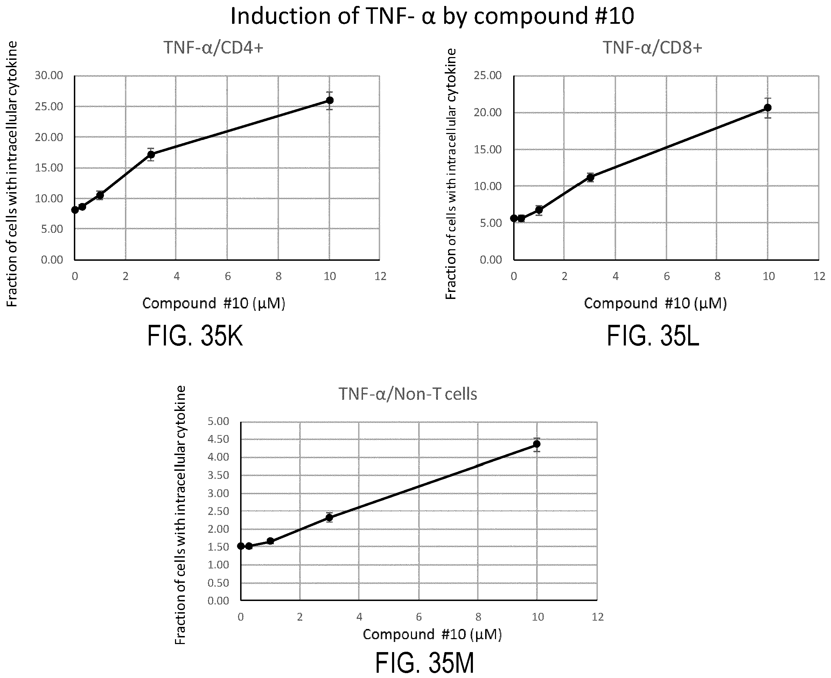

FIGURE 35K graphically illustrates the fraction of cells with intracellular TNF-α staining in the CD4⁺ T cell population in PBMC cultures treated with compound 10 (0, 0.1, 0.3, 1.0, 3.0 or 10 µM), a representative of which is shown in FIGURE 35A.

FIGURE 35L graphically illustrates the fraction of cells with intracellular TNF-α staining in the CD8⁺ T cell population in PBMC cultures treated with compound 10 (0, 0.1, 0.3, 1.0, 3.0 or 10 µM), a representative of which is shown in FIGURE 35A.

FIGURE 35M graphically illustrates the fraction of cells with intracellular TNF-α staining in non-T cell populations in PBMC cultures treated with compound 10 (0, 0.1, 0.3, 1.0, 3.0 or 10 µM), a representative of which is shown in FIGURE 35A.

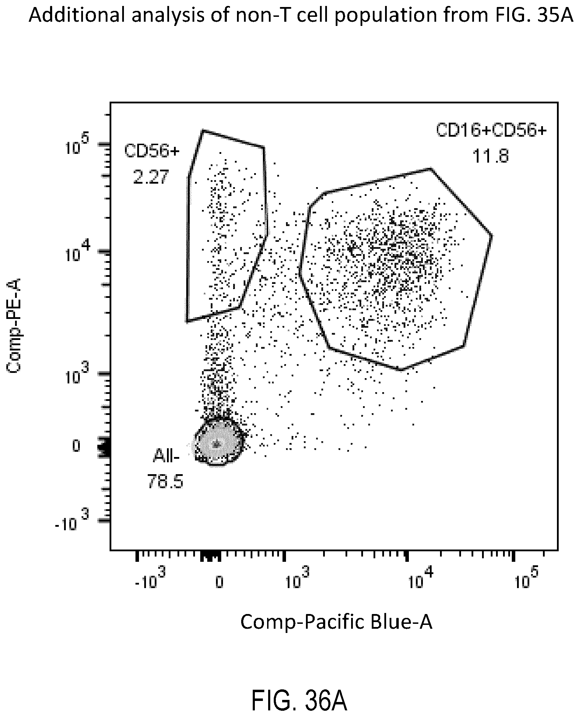

The results of the additional characterization of IFNγ and TNF-α expression in non-T cells (from FIGURES 35A, 35J and 35M) are shown in in FIGURES 36A-36E. These results were generated from a single donor. Similar results were also observed from the second donor (data not shown).

FIGURE 36A shows the non-T cell population from FIGURE 35A sorted into CD56⁺CD16⁻ (2.27%), CD56⁺CD16⁺ (11.8%) and non-NK (78.5%) populations present in the non-T cell population in a representative PBMC culture two days after stimulation with anti-CD3 and anti-CD28 antibodies.

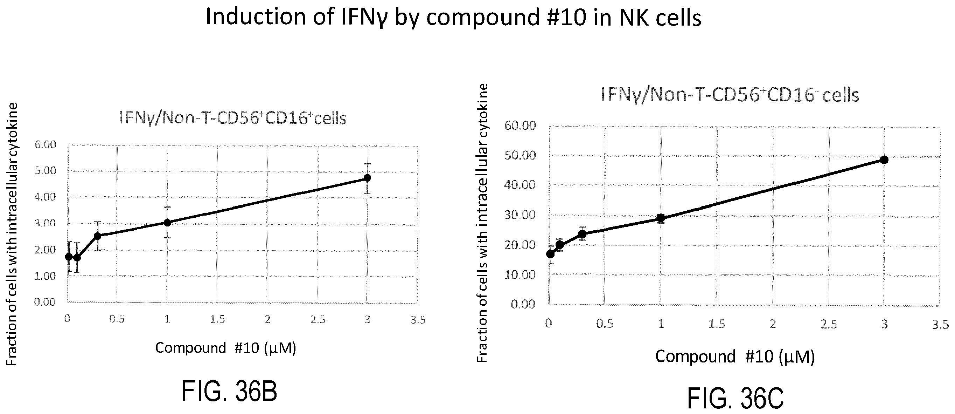

FIGURE 36B graphically illustrates the fraction of cells with intracellular IFN-γ staining in non-T-CD56⁺CD16⁺ cell populations in PBMC cultures treated with compound 10 (0, 0.1, 0.3, 1.0 or 3.0 µM), a representative of which is shown in FIGURE 36A, and

FIGURE 36C graphically illustrates the fraction of cells with intracellular IFN-γ staining in non-T-CD56⁺CD16⁻ cell populations in PBMC cultures treated with compound 10 (0, 0.1, 0.3, 1.0 or 3.0 µM), a representative of which is shown in FIGURE 36A.

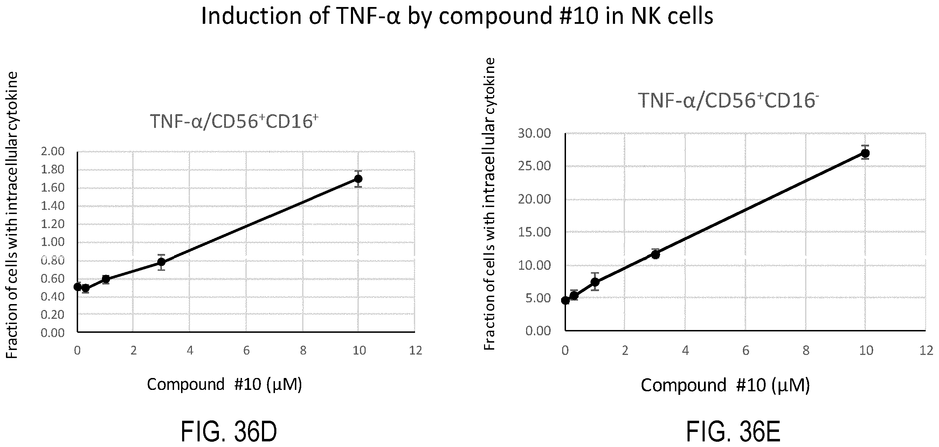

FIGURE 36D graphically illustrates the fraction of cells with intracellular TNF-α staining in non-T-CD56⁺CD16⁺ cell populations in PBMC cultures treated with compound 10 (0, 0.1, 0.3, 1.0, 3.0 or 10 µM), a representative of which is shown in FIGURE 36A, and

FIGURE 36E graphically illustrates the fraction of cells with intracellular TNF-α staining in non-T-CD56⁺CD16⁻ cell populations in PBMC cultures treated with compound 10 (0, 0.1, 0.3, 1.0, 3.0 or 10 µM), a representative of which is shown in FIGURE 36A.

The results shown in FIGURES 35A-M and FIGURES 36A-E identify cell types affected by inhibition of GPR174 with compound 10: IL-2 and IL-10 production is induced specifically in T cells, both CD4 and CD8 positive, while IFN-γ and TNF-α production is enhanced in CD4 and CD8 positive T cells, as well as in NK cells. These data specify the mechanism of immune stimulation by GPR174 inhibition.

### EXAMPLE 12

This Example describes a study that was carried out to assess *in vivo* consequences of the absence of GPR174 activity by comparing the effect of immunization with influenza virus on the level of flu antigen-specific CD8⁺ T cells in wild-type and GPR174 (KO) knockout mice.

Animal exposure to the mouse-adapted influenza virus HIN1 A/PR/8/34 is a widely used model to assess the immune response to an acute infection (see Allen I.C., Mouse Models of Innate Immunity: Methods and Protocols, Methods in Molecular Biology, vol 1031: pp 177-188 (2013); Castiglioni P. et al., J Immunol 180:4956-64, 2008; Zak O., Sande MA (eds), Handbook of Animal Models of Infection, Academic Press: pp981-986, 1999). Accordingly, as described in this Example, a study was carried out to assess *in vivo* consequences of the absence of GPR174 activity by comparing the effect of immunization with influenza virus on the level of flu antigen-specific CD8⁺ T cells in wild-type and GPR174 (KO) knockout mice.

In a first experiment, wild-type (WT) and GPR174 KO mice (generated as described in Example 7) were immunized by intraperitoneal (ip) injection with 1000 HAU (hemagglutinin units) of influenza virus A/PR/8/34. Three weeks later, splenocytes were obtained from two naïve, four immunized WT and four immunized GPR174 KO mice and placed in the presence of 2 µM of known influenza nucleoprotein (NP) antigenic peptide "ASNENMETM" (SEQ ID NO:5) to induce preferential proliferation of CD8⁺ T cells recognizing this antigen in a complex with MHC class I presenting molecules. Five days later, the quantity of the CD8⁺ cells displaying T cell receptor (TCR) that recognizes the MHC-influenza peptide complex was evaluated by staining the splenocytes with anti-CD8 antibodies and MHC-ASNENMETM dextramers (Immudex, Denmark). Fluorescently labeled MHC dextramers are reagents that carry multiple MHC-peptide complexes, and thus have the ability to interact simultaneously with multiple TCRs on a single T cell, allowing for a stable interaction between the reagent and the T cell, and as a result are useful for the detection and quantitation of antigen-specific T cells.

In a second experiment, four WT and four GPR174 KO mice were immunized by ip injection with 1000 HAU of influenza virus A/PR/8/34. Three weeks post immunization these mice were boosted by a second injection of 1000 HAU of influenza virus A/PR/8/34, and their splenocytes were analyzed 11 days later, along with the splenocytes from two naive animals, by dextramer staining without prior *in vitro* incubation.

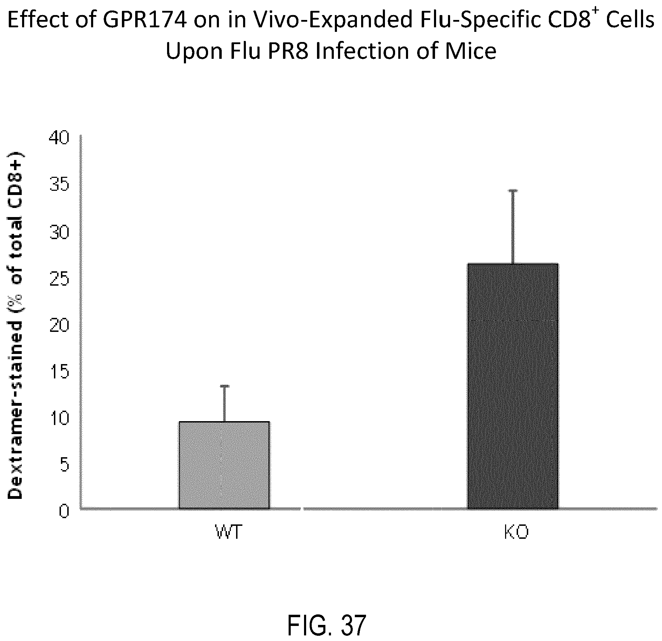

The results of the first experiment are shown in FIGURE 37.

FIGURE 37 graphically illustrates the amount of dextramer stained cells (antigen-specific T cells) as a percent of total CD8⁺ cells in splenocyte cultures obtained from WT and GPR174 KO mice three weeks after immunization with influenza virus A/PR/8/34, and cultured for five days in the presence of NP antigenic peptide. Splenocytes obtained from control naive WT mice are also shown in FIGURE 37. As shown in FIGURE 37, the splenocyte culture from GPR174 KO mice has a higher percentage of dextramer stained CD8⁺ cells (antigen-specific T cells) as compared to the percentage of dextramer stained CD8⁺ cells in splenoctye cultures obtained from naive and WT mice.

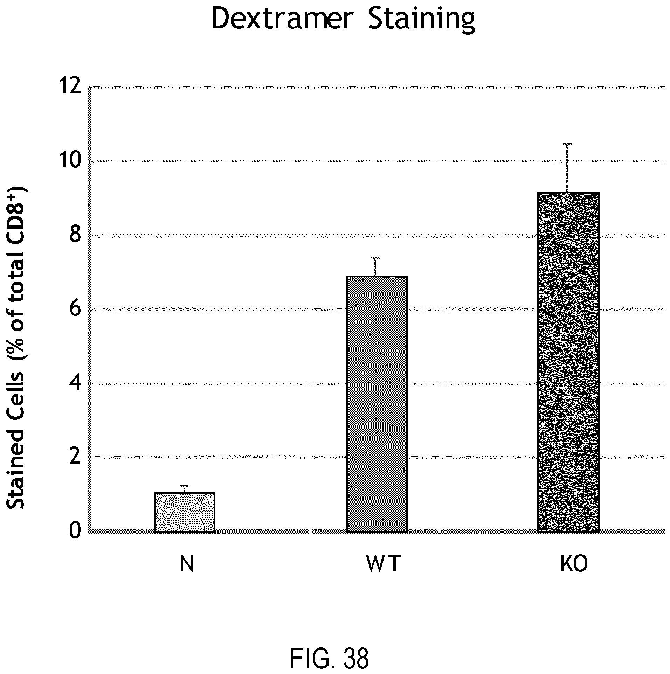

The results of the second experiment are shown in FIGURE 38. FIGURE 38 graphically illustrates the amount of dextramer stained cells (antigen-specific T cells) as a percent of total CD8⁺ cells in splenocytes obtained from WT and GPR174 KO mice that were immunized and boosted three weeks later with 1000 HAU of influenza virus A/PR/8/34, wherein the splenocytes were analyzed 11 days after boost. Splenocytes obtained from control naive (N) WT mice are also shown in FIGURE 38. As shown in FIGURE 38, the splenoctyes from GPR174 KO mice have a higher percentage of dextramer stained CD8⁺ cells (antigen-specific T cells) as compared to the amount of dextramer stained CD8⁺ cells in splenocytes obtained from WT and naïve mice.

In summary, the results in this Example demonstrate that in both experimental settings, *i.e.,* with or without prior *in vitro* incubation of antigen-specific T cells, there is a trend towards higher numbers of the flu antigen-specific CD8⁺ T cells in splenocytes from GPR174 KO mice as compared to WT mice, indicating that GPR174 limits immune response and inhibition of GPR174 is immunostimulatory.

### EXAMPLE 13

This Example describes a study that was carried out to assess the effect of representative GPR174 inhibitory compound 10 on cytotoxic T-lymphocyte-associated antigen 4 (CTLA4) expression on human naïve T cells and human memory T cells.

Background/Rationale: CTLA4 is expressed by T cells activated through the T cell receptor and CD28. CTLA4 is homologous to the T-cell co-stimulatory protein, CD28, and both molecules bind to CD80 and CD86, also called B7-1 and B7-2 respectively, on antigen-presenting cells. CTLA-4 binds CD80 and CD86 with greater affinity and avidity than CD28 thus enabling it to outcompete CD28 for its ligands. CTLA4 is also found in regulatory T cells and contributes to their inhibitory function. Blocking or inhibiting CTLA4 serves as a means of reversing immune system tolerance to tumors and thereby providing an immunotherapy strategy for patients with cancer (see *e.g.,* Baumeister S.H. et al., Ann Rev Immunol 34:539-73, 2016). This approach is called immune checkpoint blockade therapy.

PBMC cells from a single donor were cultured overnight with additional irradiated antigen-presenting cells (APC) in the presence of various concentrations of GPR174 inhibitory compound 10 (0.019 to 6 µM) or equivalent concentrations of vehicle (DMSO). Anti-CD3 (OKT3) was then added to the cultures for an additional 24 hours. The cells were analyzed by flow cytometry for expression of FOXP3, CTLA-4, CD4, CD3, CD45RA and CD25.

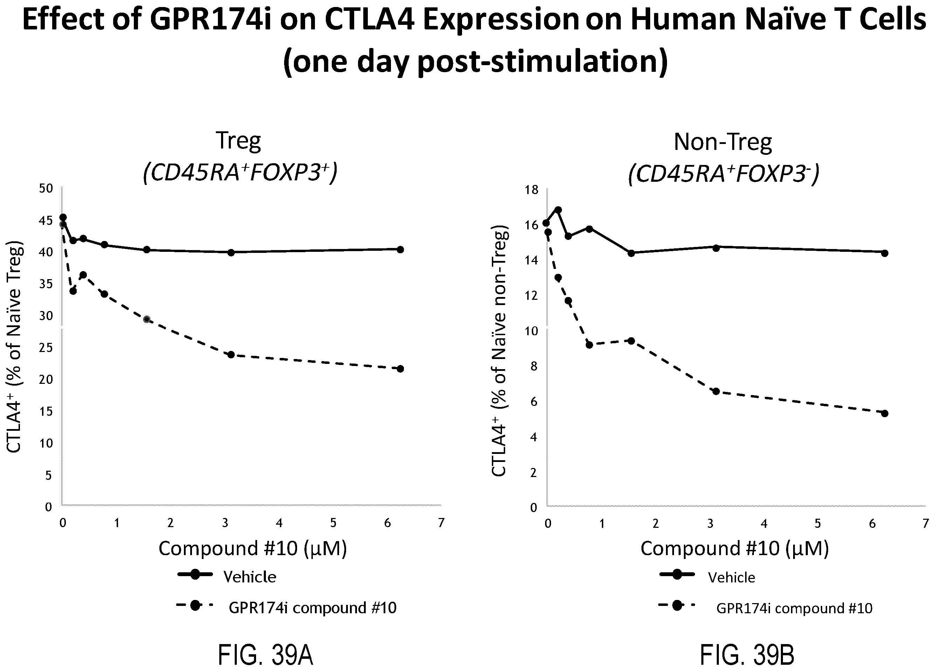

FIGURE 39A graphically illustrates the fraction of naive Regulatory T cells (Treg) with CTLA-4 positive staining (percentage of naive Treg cells (CD45RA+FOXP3+) in PBMC cultures treated with compound 10 (0, 0.19, 0.38, 0.75, 1.5, 3.0 or 6.0 µM), at one day post-stimulation, showing that CTLA4 expression is reduced in naive Treg cells treated with GPR174 inhibitory compound 10 in a dose-dependent manner as compared to vehicle control treated cells.

FIGURE 39B graphically illustrates the fraction of non-regulatory T cells (non-Treg) with CTLA4 positive staining (percentage of naive non-Treg cells (CD45RA+FOXP3-) in PBMC cultures treated with compound 10 (0, 0.19, 0.38, 0.75, 1.5, 3.0 or 6.0 µM) at one day post-stimulation, showing that CTLA4 expression is reduced in non-Treg cells treated with GPR174 inhibitory compound 10 in a dose-dependent manner as compared to vehicle control treated cells.

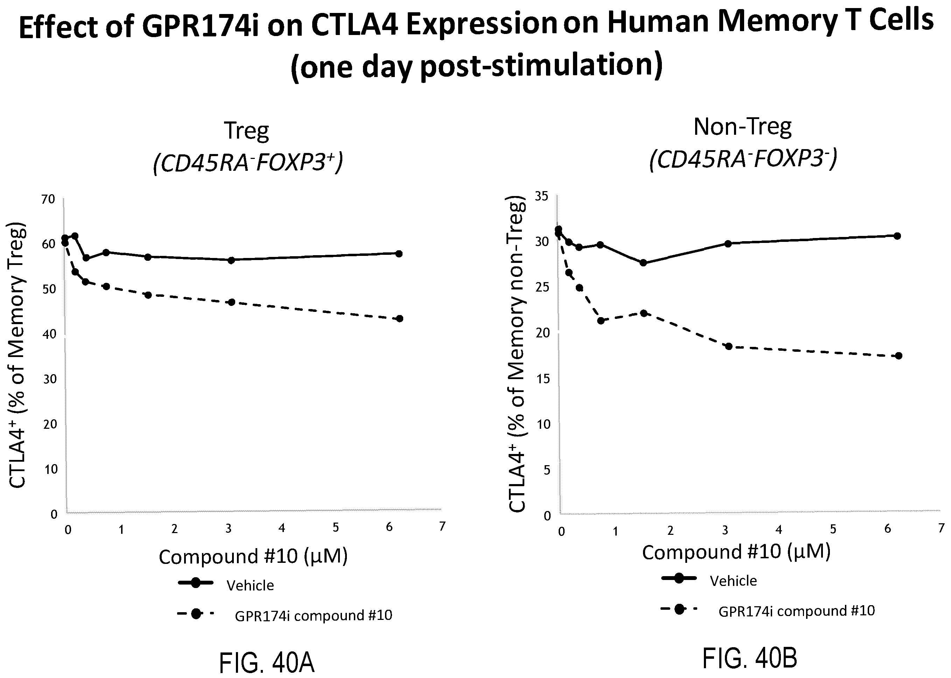

FIGURE 40A graphically illustrates the fraction of memory Treg with CTLA4 positive staining (percentage of memory Treg (CD45RA-FOXP3+) in PBMC cultures treated with GPR174 inhibitory compound 10 (0, 0.19, 0.38, 0.75, 1.5, 3.0 or 6.0 µM) at one day post-stimulation, showing that CTLA4 expression is reduced in memory Treg cells treated with compound 10 in a dose-dependent manner as compared to vehicle control treated cells.

FIGURE 40B graphically illustrates the fraction of memory non-Treg cells with CTLA4 positive staining (percentage of memory non-Treg (CD45RA-FOXP3-) in PBMC cultures treated with GPR174 inhibitory compound 10 (0, 0.19, 0.38, 0.75, 1.5, 3.0 or 6.0 µM) at one day post-stimulation, showing that CTLA4 expression is reduced in memory non-Treg cells treated with compound 10 in a dose-dependent manner as compared to vehicle control treated cells.

In summary, the results in this Example indicate that inhibition of GPR174 not only activates immune response in T cells stimulated by engaging TCR and its co-stimulatory protein CD28, but also prevents induction of immune checkpoint CTLA-4 that normally limits T cell activation.

### EXAMPLE 14

This Example describes a study that was carried out to assess the effect of representative GPR174 inhibitory compound 10 on the expression of PD-L1, TIGIT and other T cell expressed immune checkpoint molecules on human T cells.

Programmed death-ligand 1 (PD-L1) also known as CD274 or B7-H1 is a transmembrane protein that has a role in suppressing the immune system. The binding of PD-L1 to PD-1 or B7.1 transmits an inhibitory signal that reduces the proliferation of T cells and can also induce apoptosis. It appears that upregulation of PD-L1 allows cancers to evade the host immune system. Many PD-L1 inhibitors are in development as immuno-oncology therapies and are showing good results (see *e.g.,* Baumeister S.H. et al., Ann Rev Immunol 34:539-73, 2016; Teng F. et al., Cancer Lett. 414:166-173, 2017). Clinically available examples include Durvalumab, atezolizumab, and avelumab.

T cell immunoreceptor with Ig and ITIM domains (TIGIT) is expressed by activated cytotoxic T cells and regulatory T cells and has also been shown to be upregulated on T cells in multiple cancer models. The ligands CD155 and CD112 are found on dendritic cells and macrophages and are also highly expressed in several types of cancer. TIGIT expression is highly correlated with the expression of other coinhibitory molecules, including PD-1. Studies have revealed TIGIT as a unique and potentially complementary target to other inhibitory immune checkpoints. First, in addition to directly inhibiting cytotoxic T-cell activity, TIGIT can foster an immunosuppressive microenvironment through its impact on other immune cells. For example, by binding to CD155 on the surface of dendritic cells, TIGIT increases the secretion of the immunosuppressive cytokine interleukin-10, and engagement of TIGIT on regulatory T cells enhances their immunosuppressive functions (see *e.g.,* Blake S.J. et al., Clin Cancer Res 22(21):5183-5188, 2016; Grogan J. et al. J Immunother Cancer 4(suppl 1):P209, 2016; Kurtulis S. et al., J Clin Invest 125(11):4053-4062, 2015; Lozano E. et al., J Immunol 188(8):3869-3875, 2012).

Recent studies suggest that Treg cells can participate in tissue repair in a manner separable from their immunosuppressive capacity. Using transplantable models of lung tumors in mice, it was found that amphiregulin (AREG), a member of the epidermal growth factor family, was prominently up-regulated in intratumoral Treg cells. Furthermore, T cell-restricted amphiregulin deficiency resulted in markedly delayed lung tumor progression. This observed deterrence in tumor progression was not associated with detectable changes in T cell immune responsiveness or Treg and effector T cell numbers. These observations suggest a novel "nonimmune" modality for intratumoral Treg and effector T cells in promoting tumor growth through the production of factors normally involved in tissue repair and maintenance (see *e.g.,* Green J.A. et al., J Exp Med Oct 16 2017, doi:10.1084/jem.20170356. Epub ahead of print).

PBMC cells from a single donor mixed with additional irradiated antigen-presenting cells (APC) and anti-CD3 antibodies (UCHT1) were incubated in the presence of various concentrations of GPR174 inhibitory compound 10 (1.25 to 5 µM) or equivalent concentrations of vehicle (DMSO). The cells were analyzed by flow cytometry for expression of CD4, CD8, PD-L1, TIGIT and AREG.

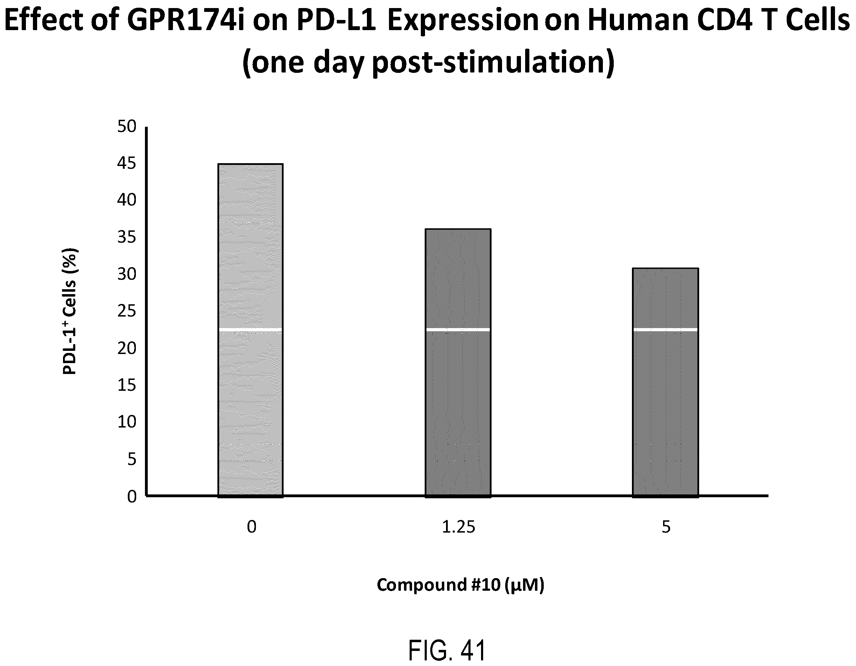

FIGURE 41 graphically illustrates the fraction of human CD4+ T cells with PD-L1 positive staining in PBMC cultures treated with GPR174 inhibitory compound 10 (0, 1.25 or 5µM) at one day post-stimulation, showing that PD-L1 expression is reduced in CD4+ T cells treated with compound 10 in a dose-dependent manner as compared to vehicle control treated cells.

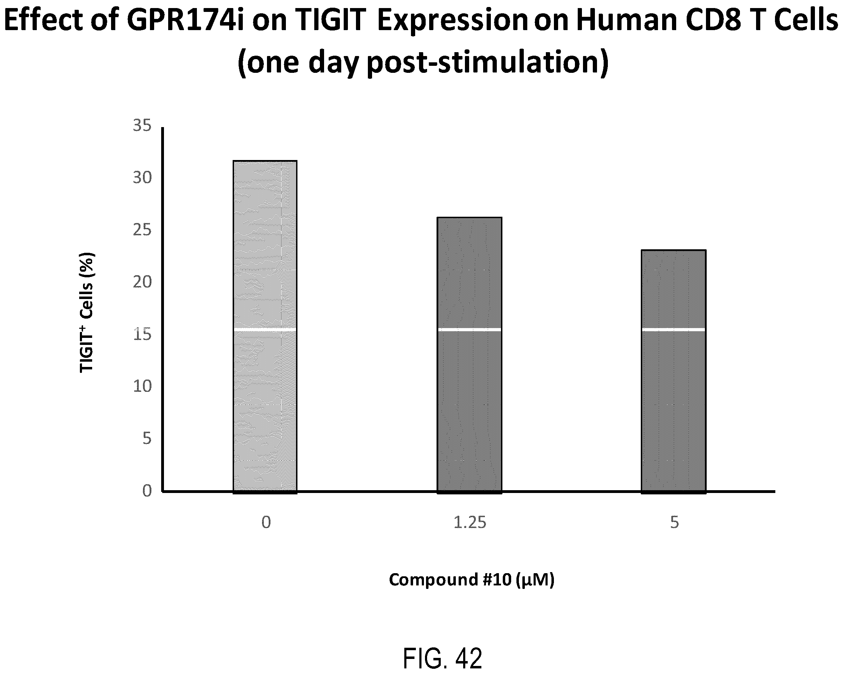

FIGURE 42 graphically illustrates the fraction of human CD8+ T cells with TGIT positive staining in PBMC cultures treated with GPR174 inhibitory compound 10 (0, 1.25 or 5µM) at one day post-stimulation, showing that TGIT expression is reduced in CD8+ T cells treated with compound 10 in a dose-dependent manner as compared to vehicle control treated cells.

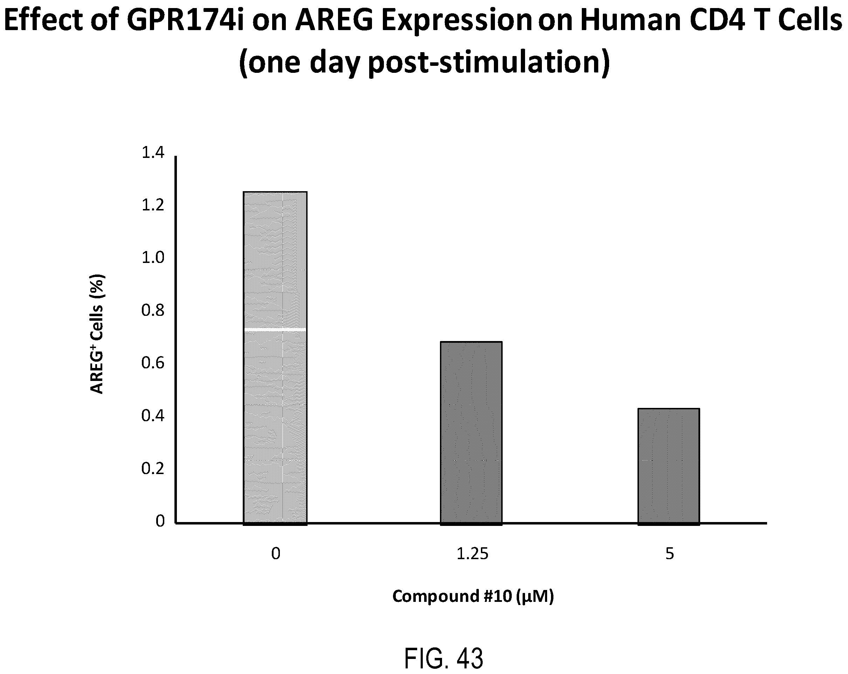

FIGURE 43 graphically illustrates the fraction of human CD4+ T cells with AREG positive staining in PBMC cultures treated with GPR174 inhibitory compound 10 (0, 1.25 or 5µM) at one day post-stimulation, showing that AREG expression is reduced in CD4+ T cells treated with compound 10 in a dose-dependent manner as compared to vehicle control treated cells.

As described above in Example 13, it was demonstrated that GPR174 inhibitory compound 10 prevents induction of immune checkpoint CTLA-4 that normally limits T cell activation. As shown in this Example, the GPR174 inhibitory compound 10 decreased expression of two additional immune checkpoint molecules, PD-L1 and TIGIT. These results demonstrate that a GPR174 inhibitory compound is capable of down-regulating several molecules responsible for tumor immune tolerance which should further increase T cell mobilization against malignant cells. In addition, T cell-produced tumor-promoting growth factor amphiregulin (AREG) is also downregulated after treatment with the GPR174 inhibitory compound, which is also expected to limit tumor growth.

### EXAMPLE 15

This Example demonstrates that the combination of a GPR174 inhibitor and an Adenosine 2a Receptor (A2aR) inhibitor results in a synergistic induction of IFN-γ production in human PBMCs.

Human PBMCs, obtained from a human volunteer donor, were dispensed at density of 1 × 10⁶ cells/well in a 96-well flat-bottom plate and were stimulated in triplicate with 1 µg/mL anti-CD3 antibody (UCHT1) and 0.1 µg/mL anti-CD28 antibody (CD28.2) in the presence of:
GPR174 inhibitory compound 10 (3 µM);
A2aR inhibitory compound (ZM241385, 1 µM or 10 µM);
a combination of compound 10 (3 µM) plus 1 µM ZM241385;
a combination of compound 10 (3 µM) plus 10 µM ZM241385; or
vehicle control (DMSO).

Supernatants from these cells were collected at 24 hours post-stimulation and the levels of IFN-γ were determined by ELISA assay. Statistical analysis was carried out using one-way ANOVA with Tukey's post-hoc multiple comparison's correction.

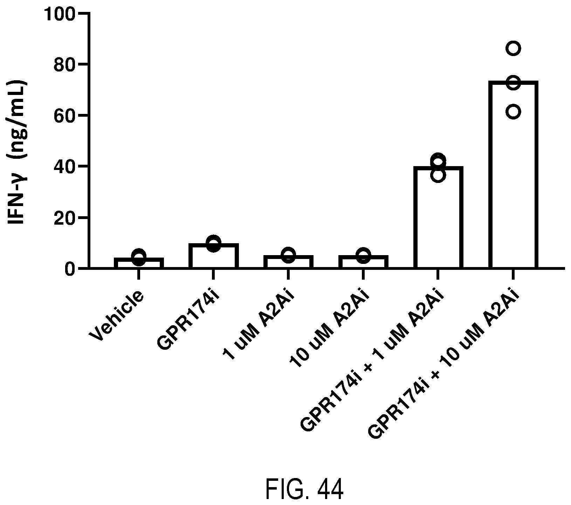

FIGURE 44 graphically illustrates the amount of IFN-γ in culture supernatants 24 hours post-stimulation in human PBMC cultures from a single donor cultured in the presence of GPR174 inhibitory compound 10 (3 µM); A2aR inhibitory compound (ZM241385, 1 µM or 10 µM);a combination of compound 10 (3 µM) plus 1 µM ZM241385; a combination of compound 10 (3 µM) plus 10 µM ZM241385; or vehicle control (DMSO).

As shown in FIGURE 44, a 2.3-fold increase was observed for GPR174i (3 µM compound 10); a 1.2-fold increase was observed for A2Ai (ZM241385, 1 µM, and 10 µM); a 9.3-fold increase was observed for the combination of GPR174i (3 µM compound 10) plus A2Ai (ZM241385, 1 µM); and a 17.1-fold increase was observed for the combination of GPR174i (3 µM compound 10) plus A2Ai (ZM241385, 10 µM). As shown in Table 8 below, statistical analysis (one-way ANOVA with Tukey's post-hoc multiple comparisons correction) indicates that only the combination of inhibitors resulted in a significant increase in IFN-γ production in comparison to each inhibitor alone or vehicle control.

**Table 8: Statistical Analysis for the results shown in FIGURE 44**

| Turkey's multiple comparisons test | Summary of significance | Adjusted *p* value |
|---|---|---|
| Vehicle vs. GPR174i | ns | 0.7772 |
| Vehicle vs. A2Ai (1 µM) | ns | 0.9999 |
| Vehicle vs. A2Ai (10 µM) | ns | >0.9999 |
| Vehicle vs. GPR174i + A2Ai (1 µM) | **** | <0.0001 |
| Vehicle vs. GPR174i + A2Ai (10 µM) | **** | <0.0001 |
| | | |
| GPR174i vs. A2Ai (1 µM) | ns | 0.8851 |
| GPR174i vs. A2Ai (10 µM) | ns | 0.8745 |
| GPR174i vs. GPR174i +A2Ai (1 µM) | *** | 0.0002 |
| GPR174i vs. GPR174i + A2Ai (10 µM) | **** | <0.0001 |
| | | |
| A2Ai (1 µM) vs. A2Ai (10 µM) | ns | >0.9999 |
| A2Ai (1 µM) vs. GPR174i + A2Ai (1 µM) | **** | <0.0001 |
| A2Ai (1 µM) vs. GPR174i + A2Ai (10 µM) | **** | <0.0001 |
| | | |
| A2Ai (10 µM) vs. GPR174i + A2Ai (1 µM) | **** | <0.0001 |
| A2Ai (10 µM) vs. GPR174i + A2Ai (10 µM) | **** | <0.0001 |
| | | |
| GPR174i + A2Ai (1 µM) vs. GPR174i + A2Ai (10 µM) | **** | <0.0001 |

| | | |
|---|---|---|
| "ns" = not significant | | |

The results shown in FIGURE 44 and Table 8 demonstrate that the combined inhibition of GPR174 and A2aR promotes the synergistic induction of IFN-γ production. As IFN-γ is a central mediator of anti-tumor immune responses, this result indicates that combined GPR174 inhibition and A2aR inhibition significantly amplifies the type of immune cell function - known as T helper 1, or Th1, that is important for effective cancer immunotherapy. Importantly, both GPR174 and A2aR activate the GαS/cyclic AMP signaling pathway, which is well characterized as a suppressive checkpoint for Th1 immune responses. Furthermore, A2aR antagonists are currently under clinical development as cancer immunotherapy checkpoint inhibitors, as are inhibitors of CD38, CD39 and CD73, enzymes that convert ATP to the A2aR ligand adenosine (see Table 15 below). Cellular stress and cell death in the tumor microenvironment lead to increased concentrations of both adenosine and the GPR174 ligand lysophosphatidylserine (lysoPS), a metabolite of the cell membrane lipid phosphotidylserine (PS). Thus, immune cells in and around tumor tissue can be restrained by the combined activities of adenosine, PS, and lysophosphatidylserine, and effective liberation of the Th1 responses from this suppression appears to require the combined inhibition of both GPR174 and the CD38/CD39/CD73/A2aR/A2bR axis. Therefore, the results provided in this Example indicate that for cyclic AMP signaling to be reduced to levels that permit robust Th1 immunity, both GPR174 and adenosine signaling must be inhibited.

### EXAMPLE 16

This Example describes the discovery that phosphatidylserine (PS) is an agonist for GPR174-mediated Gs signaling.

As described herein, a recent report has identified lysophosphatidylserine (LysoPS) as a ligand for GPR174 (Inoue et al., Nat Methods 9:1021-9, 2012). LysoPS is a lysophospholipid mediator generated by enzymatic hydrolysis of membrane phospholipid, phosphatidylserine (PS). LysoPS is secreted by cells of the immune system and can induce multiple cellular responses, including T-cell suppression and mast cell degranulation (Makide et al., Prostaglandins Other Lipid Mediat 89:135-9, 2009).

As described in this Example, the inventors have determined that phosphatidylserine (PS) is also an agonist of GPR174. PS is a phospholipid and is a component of the cell membrane. PS is actively held facing the cytosolid (inner) side of the cell membrane by the enzyme flippase. However, when a cell undergoes apoptosis, PS is no longer restricted to the cytosolic side by flippase. Instead, scramblase catalyzes the rapid exchange of PSs between the two sides of the cell membrane. When PS flips to the extracellular (outer) surface of the cell, it acts as a signal for macrophages to engulf these cells (Verhoven B. et al., J of Exp Med. 182(5):1597-601, 1995.

Several transmembrane receptors-largely expressed by phagocytic cells such as monocytes, macrophages and dendritic cells-bind PS or PS/protein complexes and mediate efferocytosis. These include the TAM family of receptor tyrosine kinases (Tyro3, Axl, MerTK), which recognize PS complexed to GAS6 or PROS1 serum proteins; TIM (T cell immunoglobulin- and mucin-domain-containing molecule) proteins, CD300, and BAI1, which directly interact with PS; and MFG-E8, which bridges PS to integrins (see Arandjelovic S and Ravichandran K, Nature Immunology 16:907-917, 2015; Lemke G and Burstyn-Cohen T., Ann NY Acad Sci 1209:23-29, 2010; Morizono K and Chen I.S.Y., Journal of Virology, Vol 88 (8):4275-4290, 2014; and Park, S. and Kin, I., Experimental & Molecular Medicine 49 e331, 2017). The signals these receptors deliver to cells actively recognizing PS and mediating efferocytosis are largely immunosuppressive.

In addition to apoptotic cells, extracellular PS is highly enriched in the tumor microenvironment and is found on the surface of tumor cells as well as endothelial cells of blood vessels permeating solid tumors. Furthermore, apoptotic neutrophils and activated platelets, both of which expose PS, are also recruited to solid tumors (see A.K and Rao D.A., Blood 120:4667-4668, 2012; Schlesinger, M., Journal of Hematology and Oncology (11) 125, 2018; Treffers L.W. et al., Immunological Reviews vol 273: 312-328, 2016; and Gregory A. D. and Houghton A. M., Cancer Research Vol 71 (7): 2411-6, 2011). PS is also found in extracellular vesicles (EV) derived from tumors and apoptotic cells, including microvesicles, exosomes and exomeres (see *e.g.,* Zhang et al., Cell Reports 27:940-954, 2019 and Raposo G and Stoorvogel W., Jour Cell Biol 200(4):373, 2013). Strikingly, proteins that bind PS are highly concentrated in solid tumors in mouse models, as detected by whole animal imaging (Li R. et al., Mol Cancer Ther 17(1): 169-82, 2018; Desai T.J. et al., Oncotarget 7:30678-30690, 2016; and Birge R. B. et al., Cell Death & Differentiation 23: 962-978, 2016). Thus, high concentrations of PS are considered a major source of tumor-mediated immunosuppression and may play a role in resistance to cancer immunotherapies such as checkpoint inhibitors.

Because lysoPS and PS are very similar in structure, yet differ in terms of aqueous solubility, we addressed in this Example whether liposomes generated from PS would agonize GPR174 to the same extent as lysoPS. The ability for PS-exposing membranes to agonize GPR174 would indicate that the enzymes required to hydrolyze PS into lysoPS would not be required for the abundant PS in tumors to act on GPR174-expressing cells.

Comparison of PS and LysoPS Gs signaling in HEK293 cells transfected with wild-type GPR174 or mutant GPR174

Unilamellar liposomes of phosphatidylserine (PS) and other phospholipids were prepared by hydration in PBS and extrusion at 50-54°C through a 0.1 micron filter using a mini-extruder (Avanti). Lysophosphatidylserine (Lyso-PS) is soluble.

HEK293 cells were grown in 6-well plates and transfected using Lipofectamine 2000 (Thermo Fisher Scientific) with 300 ng/well of cAMP biosensor expressing plasmid pGlo22F (Promega) and 2 ng of a plasmid expressing wild-type GPR174 or 2 ng of a plasmid expressing a GPR174 mutant with lowered basal activity, referred to as "GPR174-v38."

5-6 hours after transfection, the cells were trypsinized and plated at a density of 5000 cells/well into 96-well plates and incubated overnight. The next day, the culture medium was replaced with 100 µL/well of serum free medium X-vivo15 (Lonza) with 2% GloSensor Assay reagent (Promega). The plates were incubated for 1 hour at room temperature and measured in a luminometer to obtain the pre-compound values. Phosphatidylserine liposomes (PS) at a concentration range of 10 nM to 10,000 nM or lysophosphatidylserine (Lyso-PS) at a concentration range of 10 nM to 10,000 nM were added to the wells and incubated for 15 minutes at room temperature. The plates were measured in a luminometer to obtain the post-compound addition measurement. The ratio of post-compound addition to pre-compound addition measurements *(i.e.,* the extent of Glosensor signal induction by an agonist) was determined.

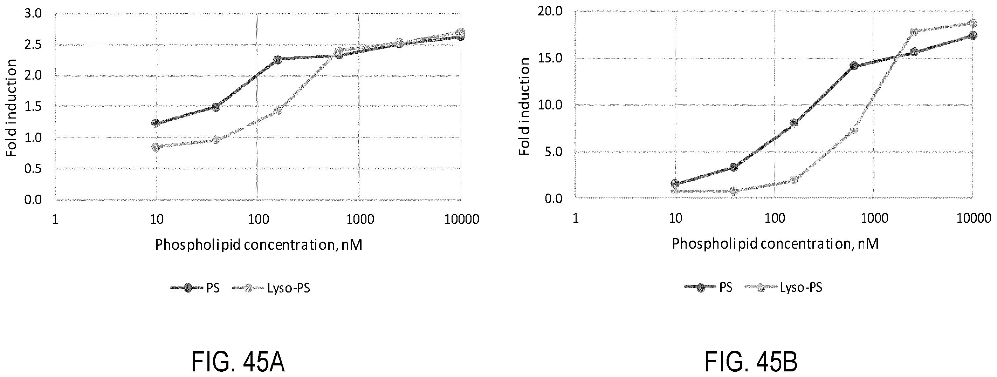

FIGURE 45A graphically illustrates the fold induction of Gs-signaling activity in HEK293 cells expressing wild-type GPR174 and cAMP biosensor expressing plasmid pGlo22F in the presence of increasing concentrations of phosphatidylserine liposomes (PS) or lysophosphatidylserine (Lyso-PS). As shown in FIGURE 45A, both PS and Lyso-PS stimulate GPR174 Gs signaling in a concentration-dependent manner (PS with an EC₅₀ = 83 nM). It is worth noting that the apparent potency of PS is 4- to 5-fold higher than that of Lyso-PS (*i.e,,* the same signal is achieved at a lower concentration of PS), making it unlikely that liposomeinduced signal could be explained by decomposition of PS into Lyso-PS during the signaling experiment. Further in this regard, it is noted that direct LC-MS measurement of the amounts of PS and Lyso-PS in our preparations demonstrated the presence of only small amounts of Lyso-PS (3%) in our PS preparation, which did not increase following incubation of PS with cells and medium in a signaling experiment (data not shown).

FIGURE 45B graphically illustrates, with an increased signal window as compared to FIGURE 45A, the fold induction of Gs-signaling activity in HEK293 cells expressing mutant GPR174-v38 and cAMP biosensor expressing plasmid pGlo22F in the presence of increasing concentrations of phosphatidylserine liposomes (PS) or lysophosphatidylserine (Lyso-PS). As shown in FIGURE 45B, both PS and Lyso-PS stimulate GPR174 Gs signaling in a concentration-dependent manner. The signaling window for GPR174-v38 is superior to the wild type GPR174 receptor (GPR174-WT), but the lipid doseresponses are somewhat right-shifted, indicating lower affinities for both PS and Lyso-PS. For this mutant version of GPR174, similar to the results observed with GPR174-WT, PS liposomes serve as a more potent agonist than Lyso-PS.

### Analysis of additional phospholipids for the ability to function as GPR174 agonists

Additional phospholipids: phosphatidylcholine (PC), phosphatidylethanolamine (PE), phosphatidylinositol (PI) and phosphatidylserine (PS): were prepared as unilamellar liposomes by hydration in PBS and extrusion at 50-54°C through a 0.1 micron filter using a mini-extruder (Avanti) and were analyzed in comparison to PS for the ability to function as GPR174 agonists. All lipids were used at the final concentration of 4 µM. The signaling experiment was conducted as described above for FIGURE 45, but instead of transient transfection, a pool of HEK293 cells stably transfected with WT GPR174 and GloSensor cAMP biosensor was used.

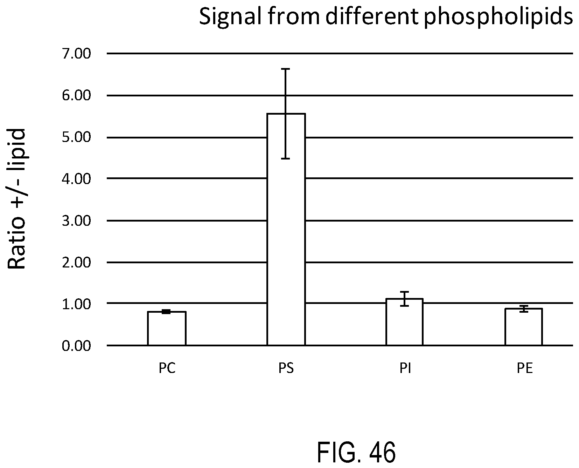

FIGURE 46 graphically illustrates the level of GPR174 Gs signaling activity in HEK293 cells expressing GPR174 and cAMP biosensor expressing plasmid pGlo22F (shown as the ratio of luminescence after phospholipid addition to the pre-read luminescence value) in the presence of phosphatidylserine (PS), phosphatidylcholine (PC), phosphatidylethanolamine (PE), and phosphatidylinositol (PI) liposomes.

As shown in FIGURE 46, GPR174 Gs signaling was stimulated in the presence of PS, whereas phosphatidylcholine (PC), phosphatidylethanolamine (PE), and phosphatidylinositol (PI) liposomes were completely inactive. Therefore, it is demonstrated that GPR174 Gs signaling is LysoPS and PS-specific.

### Analysis of PS-mediated GPR174 signaling in the presence of representative GPR174 inhibitory compounds

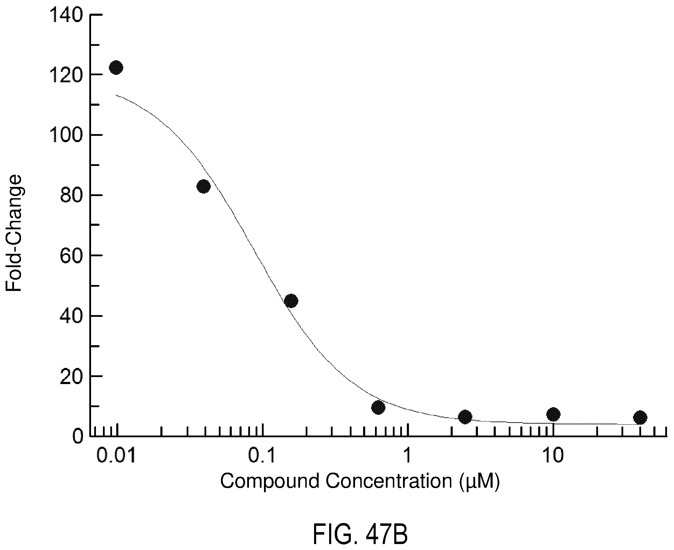

The signaling experiment was conducted as described for FIGURE 45, but instead of transient transfection a pool of HEK293 cells stably transfected with either WT GPR174 (for compound 10, as shown in FIGURE 47A) or GPR174-v38 for the rest of the tested compounds, as shown in FIGURES 47B-47F)) and GloSensor cAMP biosensor was used. A dilution series of each of the GPR174 inhibitory compounds 10 (Group I), 6 (Group I), 11 (Group I), 20 (Group II), 23 (Group IV), and 30 (Group IV) were prepared in DMSO. After the plates were pre-read in a luminometer, 1 µL of the compound dilution was added per well (with control wells receiving 1 µL of DMSO) followed by addition of PS liposomes to the final concentration of 1 µM. Plates were read in a luminometer after 15 minutes of incubation. The ratio of luminescence after PS addition to the pre-read luminescence value was used as a measure of receptor-mediated signaling.

FIGURE 47A graphically illustrates the inhibition of PS-induced GPR174 Gs signaling by the GPR174 inhibitory compound 10 (Group I) in a dose-responsive manner, with an apparent IC₅₀ of about 20-40 nM. This data demonstrates that compound 10 is a relatively potent antagonist of PS-mediated GPR174 Gs signaling. At concentrations of 1-2 µM, compound 10 achieves almost complete inhibition of the PS signaling through the GPR174 receptor.

FIGURE 47B graphically illustrates the inhibition of PS-induced GPR174 Gs signaling by the GPR174 inhibitory compound 6 (Group I) in a dose-responsive manner, with an apparent IC₅₀ of about 0.1 µM.

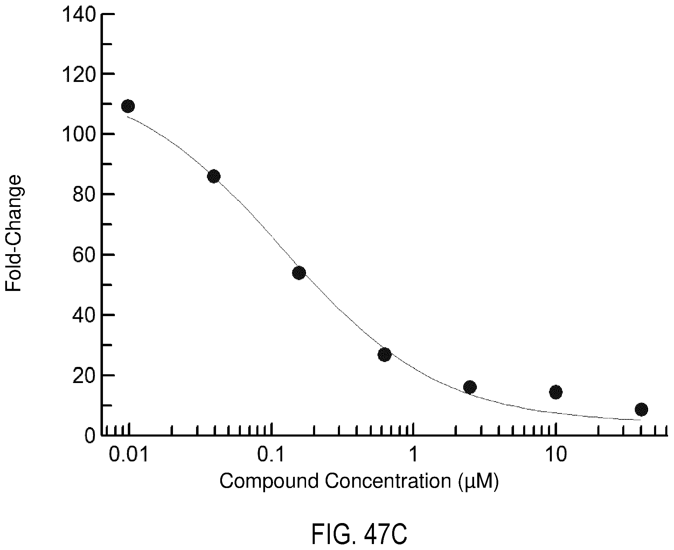

FIGURE 47C graphically illustrates the inhibition of PS-induced GPR174 Gs signaling by the GPR174 inhibitory compound 11 (Group I) in a dose-responsive manner, with an apparent IC₅₀ of about 0.1 µM.

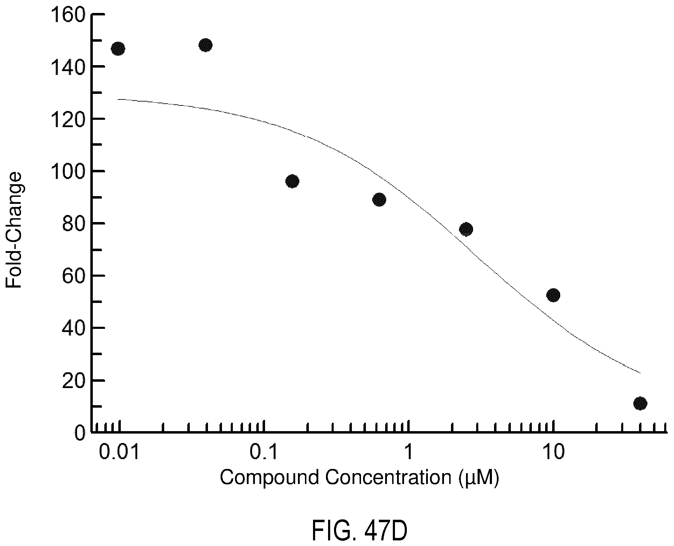

FIGURE 47D graphically illustrates the inhibition of PS-induced GPR174 Gs signaling by the GPR174 inhibitory compound 20 (Group II) in a dose-responsive manner, with an apparent IC₅₀ of about 3.0 µM.

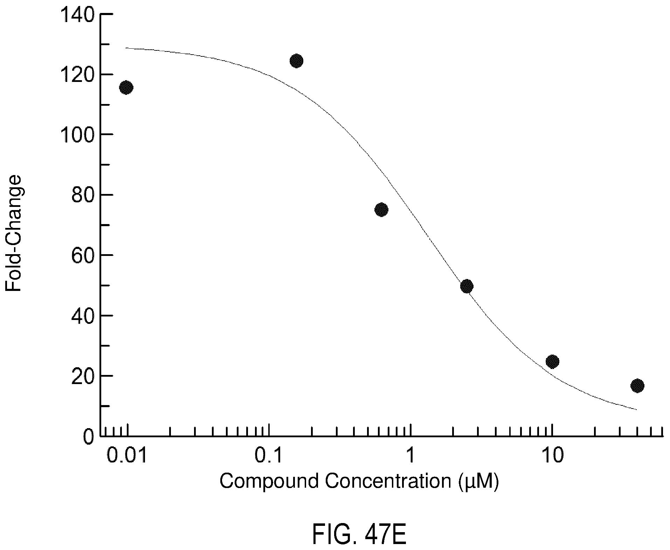

FIGURE 47E graphically illustrates the inhibition of PS-induced GPR174 Gs signaling by the GPR174 inhibitory compound 30 (Group IV) in a dose-responsive manner, with an apparent IC₅₀ of about 1.3 µM.

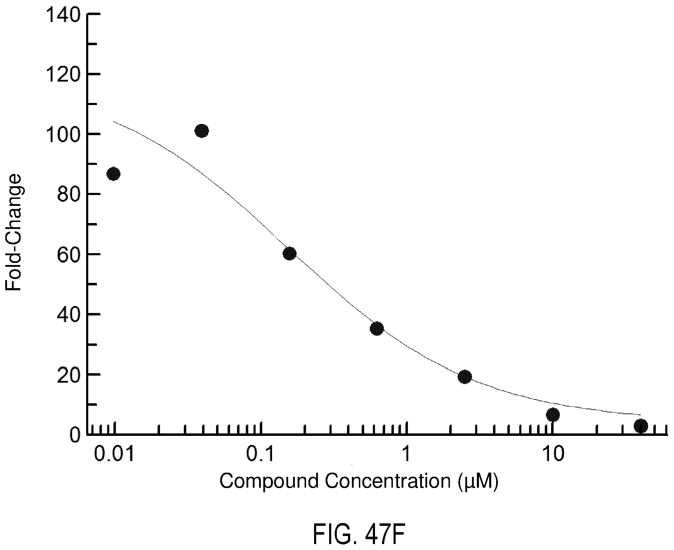

FIGURE 47F graphically illustrates the inhibition of PS-induced GPR174 Gs signaling by the GPR174 inhibitory compound 23 (Group IV) in a dose-responsive manner, with an apparent IC₅₀ of about 0.15 µM.

It was also determined (data not shown) that inhibition of PS-induced GPR174 Gs signaling by GPR174 inhibitory compound 59 (Group Va) occurred in a dose-responsive manner, with an apparent IC₅₀ of about 10 µM.

As shown in FIGURES 47A-47F, PS-mediated GPR174 Gs signaling is inhibited by representative GPR174 inhibitory compounds 6, 10, 11, 20, 23, and 30 which belong to different chemical classes (*i.e.,* Groups I, II and IV). These data demonstrate that compounds 6, 10, 11, 20, 23, and 30 act as GPR174 antagonists and inhibit PS liposomemediated cAMP signaling. Thus, PS liposome signaling through GPR174 is inhibited by multiple GPR174 inhibitory small-molecule compounds with diverse chemical structures.

These data demonstrate that PS is a potent and specific GPR174 agonist and that compound 10 is a relatively potent GPR174 inhibitor. The ability of PS to directly stimulate GPR174 has many implications for cancer immunotherapy. PS is exposed on solid tumors, apoptotic cells, including tumor cells and neutrophils, extracellular vesicles from tumor and other cells (exosomes), live tumor cells, activated T-cells and B-cells, activated platelets, monocytes and macrophages and vascular endothelium. As PS is thought to typically act on immune cells of the myeloid lineage through efferocytosis receptors (described above), GPR174 is predominantly expressed on lymphocytes, including T cells, B cells and NK cells, raising the possibility that PS can directly modulate adaptive immune responses. GPR174 signals through the Gs-cAMP pathway, which suppresses Th1 and NK cell function (Zidek K., Eur Cytokine Netw 10(3):319-28, 1999; and Serezani C. H. et al., American Journal of Respiratory Cell and Molecular Biology Vol 39(2): 127-132, 2008); thus, inhibition of GPR174 should augment anti-tumor T cell and NK cell responses.

We also demonstrate in this Example that 1-2 µM of compound 10 achieves almost complete inhibition of the PS signaling through GPR174, thus these concentrations of the inhibitor are suitable for functional studies to explore GPR174 and PS modulation of T-cell responses.

### EXAMPLE 17

### GPR174 SIGNALING IN HEK293 CELLS AFTER EXPOSURE TO APOPTOTIC K562 CELLS

This Example describes experimental results demonstrating that apoptotic cells stimulate GPR174 Gs signaling pathway in cells expressing GPR174.

As described in Example 16, the inventors have discovered that PS is a naturally occurring agonist of the GPR174 Gs signaling pathway. As introduced in Example 16, PS can be exposed on many cell types in the tumor microenvironment; however, in Example 16 we only made use of PS-containing liposomes to stimulate GPR174. In this Example, we explored whether PS exposed on cells would also agonize GPR174 in our signaling assay. For this, we employed 1) a cell line undergoing apoptosis, 2) apoptotic primary neutrophils, and 3) activated platelets.

Apoptosis was induced in K562 cells by treating overnight with 0.8 mM H₂O₂. The presence of a high percentage of apoptotic cells after treatment with H₂O₂ was confirmed by flow cytometry following annexin V staining (data not shown).

HEK293 cells stably transfected with GPR174 wild-type and GloSensor expressing plasmids were plated in 96-well plates at a density of 5000 cells/well and were probed with 50 µL/well of a 10 × 10⁶/mL suspension of untreated K562 cells, or K562 cells treated overnight with 0.8 mM H₂O₂, or cell medium. The change in luminescence was measured 15 minutes later. HEK293 cells stably transfected with GloSensor-expressing plasmid only did not respond to apoptotic cells (data not shown).

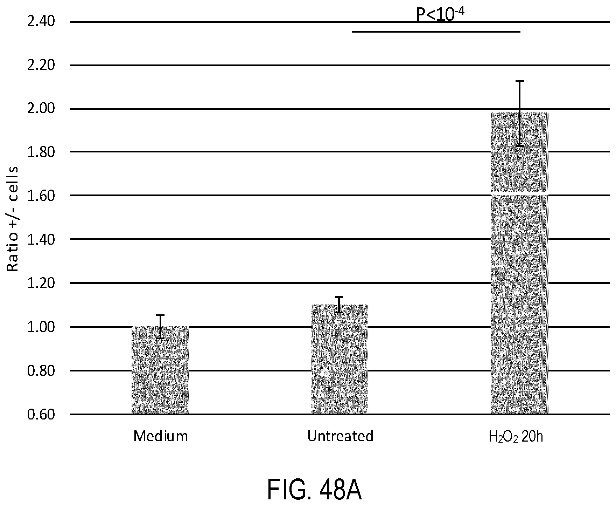

FIGURE 48A graphically illustrates the ratio of post-addition to pre-read luminescence of GPR174 and GloSensor-expressing HEK293 cells exposed to culture medium, or untreated (non-apoptotic) K562 cells, or apoptotic K562 cells (treated with H₂O₂ for 20 hours). As shown in FIGURE 48A, GPR174 signaling in HEK293 cells exposed to apoptotic K562 cells was significantly higher than in HEK293 cells exposed to untreated K562 cells (p<10⁻⁴) or HEK293 cells exposed to medium only.

GPR174 signaling in HEK293 cells after exposure to apoptotic neutrophils

Apoptosis was induced in donor blood neutrophils by incubating overnight with agonist anti-Fas antibodies (Fisher Scientific). A high percentage of apoptotic cells after treatment with anti-Fas antibodies was confirmed by flow cytometry following annexin V staining (data not shown).

HEK293 cells stably transfected with GPR174 wild-type and GloSensor-expressing plasmids were plated in 96-well plates at a density of 5000 cells/well and were probed with 50 µL/well of a 10 × 10⁶ cell/mL suspension of freshly isolated from donor neutrophils, or neutrophils incubated overnight with anti-Fas antibodies, or cell medium.

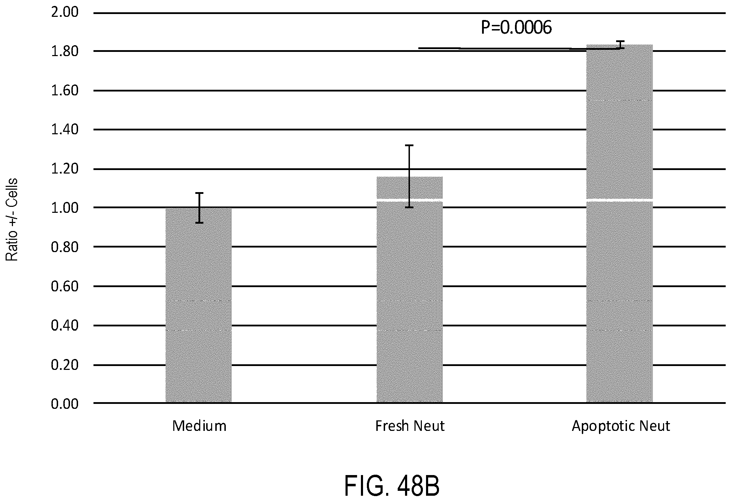

FIGURE 48B graphically illustrates the ratio of post-addition to pre-read luminescence of GPR174 and GloSensor-expressing HEK293 cells exposed to culture medium, or freshly isolated neutrophils, or apoptotic neutrophils (treated with anti-Fas antibodies). As shown in FIGURE 48B, GPR174 signaling in HEK293 cells exposed to apoptotic neutrophils was significantly higher than in HEK293 cells exposed to freshly isolated neutrophils (p=0.0006) or HEK293 cells exposed to medium only.

GPR174 signaling in HEK293 cells after exposure to activated platelets

Donor blood platelets were isolated by differential centrifugation. A high percentage of platelets isolated in this way were activated as confirmed flow cytometry following annexin V staining (data not shown).

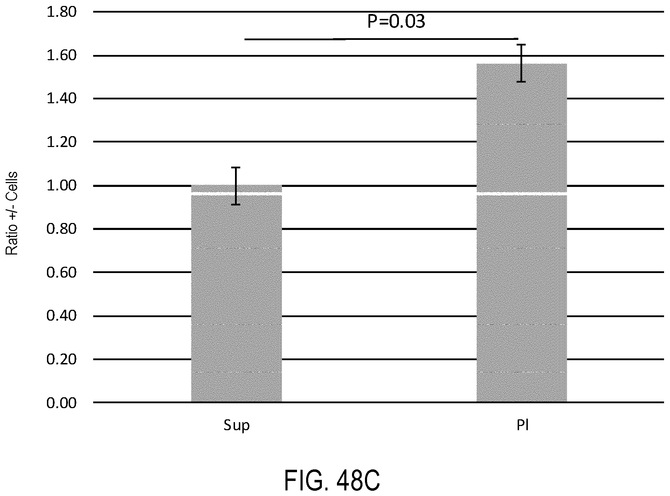

HEK293 cells stably transfected with GPR174 wild type and GloSensor expressing plasmids were plated in 96-well plates at a density of 5000 cells/well and were probed with 50 µL/well of a 5 × 10⁸ cells/mL platelets (referred to as "Pl" in FIGURE 48C) or supernatants from the platelets (referred to as "Sup" in FIGURE 48C).

FIGURE 48C graphically illustrates the ratio of post-addition to pre-read luminescence of GPR174 and GloSensor-expressing HEK293 cells exposed to culture supernatant (Sup) or platelets (Pl). As shown in FIGURE 48C, GPR174 signaling in HEK293 cells exposed to activated platelets was significantly higher than in HEK293 cells exposed to culture supernatants (p = 0.03) or HEK293 cells.

In agreement with the ability of PS liposomes to act as a potent GPR174 agonist, the data in this Example demonstrate that PS exposed at the cell surface- either as a result of apoptosis, as in the case of K562 cells and neutrophils, or as a result of platelet activationserves as a GPR174 agonist. These effects were observed at high cell concentration, suggesting cell-cell contact was important for this type of signal transduction. The discovery that PS is a potent natural ligand for GPR174 and that PS stimulates an immunosuppressive GPCR, namely GPR174 on T lymphocytes represents a significant advancement in our understanding of how PS may influence tumor immunity. As GPR174 is a Gs-coupled receptor and cAMP is known to be an immunosuppressive signal, we believe that the ability of lymphocyte-expressed GPR174 to respond to apoptosis-induced cell surface PS-exposure contributes to the ability of apoptotic cells to suppress the adaptive immune system. It is likely that some types of tumors and viral infections exploit this regulatory feature to evade immune surveillance by providing a PS-rich surface environment. Therefore, it is believed that GPR174 inhibitory compounds have therapeutic utility for inhibiting PS- and Lyso-PS-mediated GPR174 signaling and thereby avoiding immunosuppression that is typically induced in the presence of apoptotic cells and/or tumors.

### EXAMPLE 18

This Example describes experimental results further demonstrating that a GPR174 inhibitory compound in combination with various Adenosine 2a Receptor (A2aR) inhibitors synergistically induce Th1 cytokines in human PBMCs.

As described in Example 15, it was determined that the combination of a representative GPR174 inhibitor (compound 10) and the Adenosine 2a Receptor (A2aR) inhibitor ZM-241385 resulted in a synergistic induction of IFN-γ production in human PBMCs.

To extend our initial finding, further experiments were carried out to confirm that synergistic cytokine induction is observable with a representative GPR174 inhibitory (compound 10) combined with various A2aR inhibitors.

### A2aR inhibitor SCH-58261 ("SCH")

Human PBMCs, obtained from a human volunteer donor (donor 1), were dispensed in X-vivo15 media (Lonza) supplemented with Glutamax, penicillin and streptomycin, at density of 1 × 10⁶ cells/well in a 96-well flat-bottom plate and were stimulated with 1 µg/mL anti-CD3 antibody (UCHT1) and 0.1 µg/mL anti-CD28 antibody (CD28.2) in the presence of:
GPR174 inhibitory compound 10 (2 µM);
A2aR inhibitory compound (SCH-58261, 0.2 µM, 0.6 µM or 2 µM);
GPR174 inhibitory compound 10 (2 µM) plus A2aR inhibitory compound (SCH-58261, 0.2 µM, 0.6 µM or 2 µM); or
vehicle control (DMSO).

Supernatants from these cells were collected at 24 hours post-stimulation and the levels of IFN-γ, IL-2 and TNF were determined by ELISA assays.

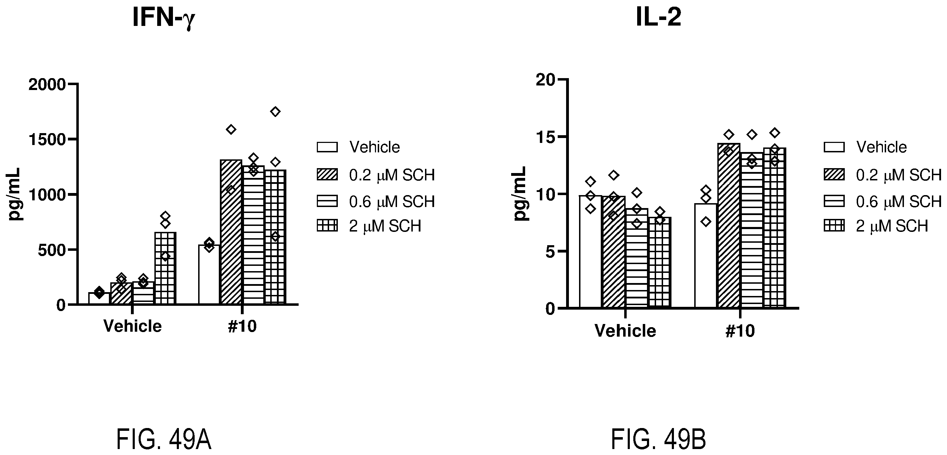

FIGURE 49A graphically illustrates the amount of IFN-γ (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO).

As shown in FIGURE 49A, and summarized in Table 9, the lower concentrations of A2aR inhibitor (0.2 µM and 0.6 µM of SCH-58261) increased IFN-γ levels by less than 2-fold and GPR174 inhibitory compound 10 (2 µM) increased IFN-γ levels by 4.9-fold. In contrast, the combination of the inhibitors increased IFN-γ levels by 11- to 12-fold, indicating synergistic activity between the A2aRi and the GPR174i because these values exceeded the sum of the increases obtained with each inhibitor alone.

FIGURE 49B graphically illustrates the amount of IL-2 (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO).

As shown in FIGURE 49B, and summarized in Table 9, all concentrations of A2aR inhibitor (0.2 µM, 0.6 µM and 2 µM of SCH-58261) as well as GPR174 inhibitor (2 µM of compound 10) had no effect on IL-2 accumulation. In contrast, the combination of the inhibitors significantly increased IL-2 levels by 1.4- to 1.5-fold, indicating synergistic activity between the A2aRi and the GPR174i because these values exceeded the sum of the increases obtained with each inhibitor alone.

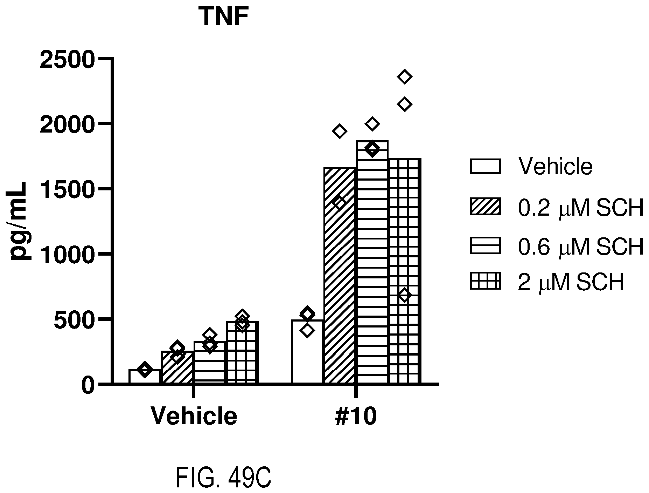

FIGURE 49C graphically illustrates the amount of TNF (pg/mL) in culture supernatants 24 hours post-stimulation in human PBMC cultures from a single donor (donor 1) cultured in the presence of GPR174 inhibitory compound 10 (2 µM); A2aR inhibitory compound (SCH-58261, 0.2 µM, 0.6 µM or 2 µM); a combination of compound 10 (2 µM) plus 0.2 µM SCH-58261; a combination of compound 10 (2 µM) plus 0.6 µM SCH-58261 or a combination of compound 10 (2 µM) plus 2 µM SCH-58261; or vehicle control (DMSO).

As shown in FIGURE 49C and summarized in Table 9, the lower concentrations of A2aR inhibitor (0.2 µM and 0.6 µM of SCH-58261) increased TNF levels by less than 3-fold and GPR174 inhibitor (2 µM of compound 10) increased TNF levels by 4.2-fold. In contrast, the combination of A2aRi and GPR174i increased TNF levels by 14- to 16-fold, indicating synergistic activity between the two inhibitors because these values exceeded the sum of the increases obtained with each inhibitor alone.

**Table 9: Summary of Results from FIGURES 49A-C**

| | | Vehicle¹ | 0.2 µM SCH¹ | 0.6 µM SCH¹ | 2 µM SCH¹ |
|---|---|---|---|---|---|
| IFN-γ | Vehicle | 1.0 | 1.8 | 1.9* | 5.9* |
| | 2 µM Cmp 10 | 4.9* | 11.8* | 11.3* | 11.0* |
| IL-2 | Vehicle | 1.0 | 1.0 | 0.9 | 0.8 |
| | 2 µM Cmp 10 | 0.9 | 1.5* | 1.4* | 1.4* |
| TNF | Vehicle | 1.0 | 2.2* | 2.8* | 4.1* |
| | 2 µM Cmp 10 | 4.2* | 14.2* | 15.9* | 14.7* |

| | | | | | |
|---|---|---|---|---|---|
| ¹Fold-change values for GPR174 inhibitory compound 10 and A2aR inhibitory compound SCH-58261 at the indicated concentrations, relative to vehicle control. *, p<0.05, t-test, two-tailed distribution, two-sample equal variance. A2aR inhibitor ZM-241385 ("ZM") and A2aR inhibitor PBF-509 ("PBF") | | | | | |

Human PBMCs, obtained from two individual human volunteer donors (donor 2 and donor 3), were dispensed at density of 1 × 10⁶ cells/well in a 96-well flat-bottom plate and were stimulated with 1 µg/mL anti-CD3 antibody (UCHT1) and 0.1 µg/mL anti-CD28 antibody (CD28.2) in the presence of:
GPR174 inhibitory compound 10 (1 µM);
A2aR inhibitory compound (ZM-241385, 0.2 µM);
A2aR inhibitory compound (PBF-509, 0.1 µM or 0.2 µM)
GPR174 inhibitory compound 10 (1 µM) plus A2aR inhibitory compound (ZM-241385, 0.2 µM); or
GPR174 inhibitory compound 10 (1 µM) plus A2aR inhibitory compound (PBF-509, 0.1 µM or 0.2 µM); or
vehicle control (DMSO).

Supernatants from these cells were collected at 24 hours post-stimulation and the levels of IFN-γ, IL-2 and TNF were determined by ELISA assays.

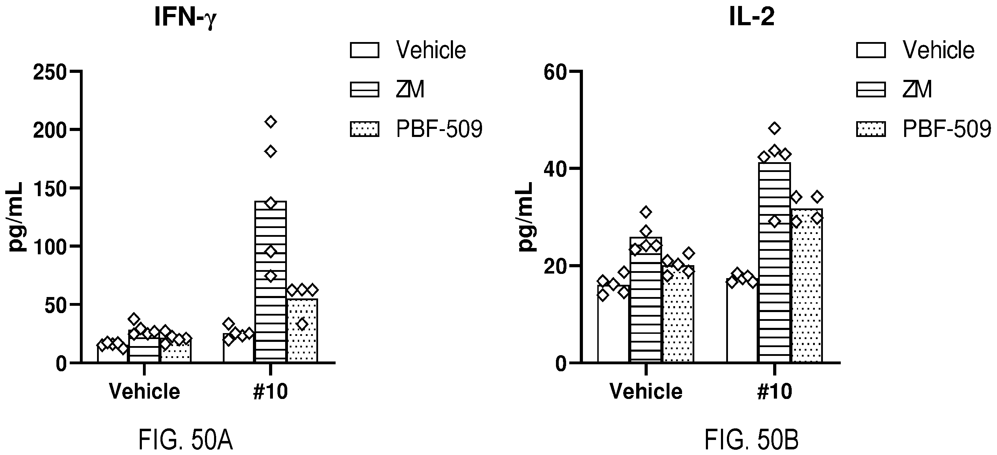

FIGURE 50A graphically illustrates the amount of IFN-γ (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO).

As shown in FIGURE 50A, and summarized in Table 10, a high degree of IFN-γ induction was observed with ZM or PBF-509 in the presence of compound 10 (8.8- and 3.5-fold, respectively). In contrast, ZM or PBF-509 alone resulted in IFN-γ induction of 1.8-fold and 1.4-fold respectively. Compound 10 alone induced IFN-γ 1.6-fold. Thus, the values obtained with the combination of an A2aR inhibitory compound (*e.g.,* ZM or PBF-509) and a GPR174 inhibitory compound (*e.g.*, compound 10) demonstrated synergistic activity as they exceeded the sum of the increases obtained with each inhibitor alone.

FIGURE 50B graphically illustrates the amount of IL-2 (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO).

As shown in FIGURE 50B and summarized in Table 10, a high degree of IL-2 induction was observed with ZM or PBF-509 in the presence of compound 10 (2.6- and 2.0-fold, respectively). In contrast, ZM or PBF-509 alone resulted in IL-2 induction of 1.6- and 1.3-fold, respectively. Compound 10 alone induced IL-2 1.1-fold. Thus, the values obtained with the combination of an A2aR inhibitory compound *(e.g.,* ZM or PBF-509) and a GPR174 inhibitory compound (e.g., compound 10) demonstrated synergistic activity as they exceeded the sum of the increases obtained with each inhibitor alone.

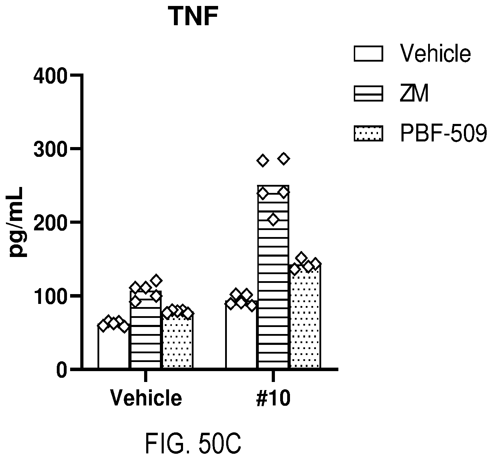

FIGURE 50C graphically illustrates the amount of TNF (pg/mL) in culture supernatants 24 hours post-stimulation in human PBMC cultures from a single donor (donor 2) cultured in the presence of GPR174 inhibitory compound 10 (1 µM); A2aR inhibitory compound (ZM, 0.2 µM); A2aR inhibitory compound (PBF-509 0.1 µM), a combination of compound 10 (1 µM) plus 0.2 µM ZM; a combination of compound 10 (1 µM) plus 0.1 µM PBF-509; or vehicle control (DMSO).

As shown in FIGURE 50C and summarized in Table 10, a high degree of TNF induction was observed with ZM or PBF-509 in the presence of compound 10 (4.0- and 2.3-fold, respectively). In contrast, ZM or PBF-509 alone resulted in TNF induction of 1.7- and 1.3-fold, respectively. Compound 10 alone induced TNF 1.5-fold. Thus, the values obtained with the combination of an A2aR inhibitory compound *(e.g.,* ZM or PBF-509) and a GPR174 inhibitory compound (e.g., compound 10) demonstrated synergistic activity as they exceeded the sum of the increases obtained with each inhibitor alone.

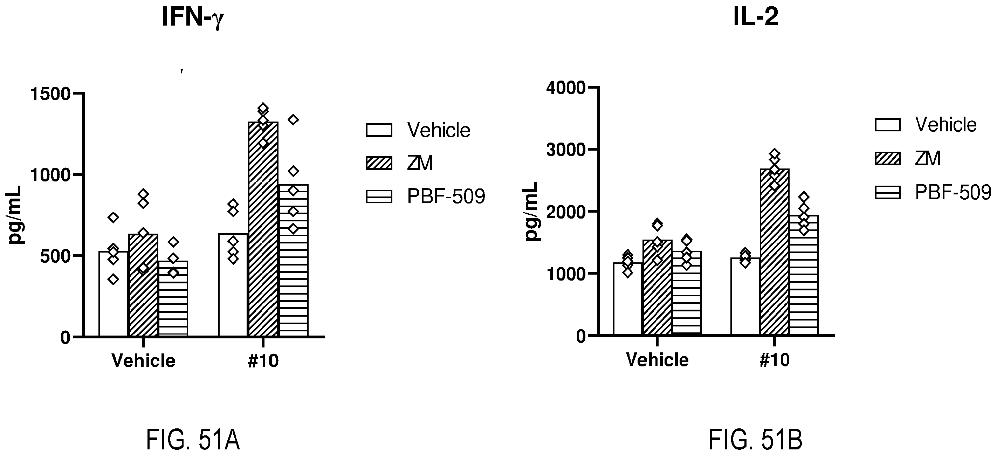

FIGURE 51A graphically illustrates the amount of IFN-γ (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM); A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO).

As shown in FIGURE 51A and summarized in Table 10, significant induction of IFN-γ was observed with ZM or PBF-509 in the presence of compound 10 (2.5- and 1.8-fold, respectively). In contrast, ZM or PBF-509 alone resulted in IFN-γ induction of 1.3- and 1.2-fold, respectively. Compound 10 alone induced IFN-γ 1.2-fold. Thus, the values obtained with the combination of an A2aR inhibitory compound (*e.g.,* ZM or PBF-509) and a GPR174 inhibitory compound (*e.g*., compound 10) demonstrated synergistic activity as they exceeded the sum of the increases obtained with each inhibitor alone.

FIGURE 51B graphically illustrates the amount of IL-2 (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM);A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO).

As shown in FIGURE 51B and summarized in Table 10, a high degree of IL-2 induction was observed with ZM or PBF-509 in the presence of compound 10 (2.3- and 1.6-fold, respectively). In contrast, ZM or PBF-509 alone resulted in IL-2 induction of 1.3- and 1.2-fold, respectively. Compound 10 alone induced IL-2 1.1-fold. Thus, the values obtained with the combination of an A2aR inhibitory compound (*e.g.,* ZM or PBF) and a GPR174 inhibitory compound (*e.g*., compound 10) demonstrated synergistic activity as they exceeded the sum of the increases obtained with each inhibitor alone.

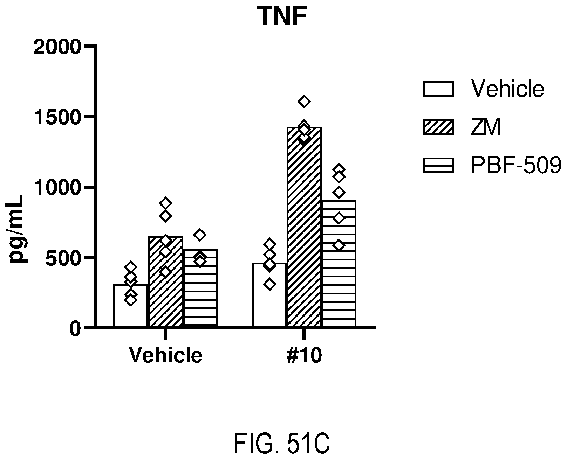

FIGURE 51C graphically illustrates the amount of TNF (pg/mL) in culture supernatants 24 hours post-stimulation in human PBMC cultures from a single donor (donor 3) cultured in the presence of GPR174 inhibitory compound 10 (1 µM); A2aR inhibitory compound (ZM, 0.2 µM); A2aR inhibitory compound (PBF-509 0.2 µM), a combination of compound 10 (1 µM) plus 0.2 µM ZM; a combination of compound 10 (1 µM) plus 0.2 µM PBF-509; or vehicle control (DMSO).

As shown in FIGURE 51C and summarized in Table 10, a high degree of TNF induction was observed with ZM or PBF-509 in the presence of compound 10 (4.6- and 2.9-fold, respectively). In contrast, ZM or PBF-509 alone resulted in TNF induction of 2.1- and 1.8-fold, respectively. Compound 10 alone induced TNF 1.5-fold. Thus, the values obtained with the combination of an A2aR inhibitory compound (*e.g.,* ZM or PBF-509) and a GPR174 inhibitory compound (*e.g*., compound 10) demonstrated synergistic activity as they exceeded the sum of the increases obtained with each inhibitor alone.

**Table 10: Summary of Results Shown in FIGURES 50A-C and 51A-C**

| Donor | Cytokine | | Vehicle¹ | ZM¹ | PBF¹ |
|---|---|---|---|---|---|
| 2 | IFN-γ | vehicle | 1.0 | 1.8* | 1.4* |
| 2 | IFN-γ | Cmpd 10 | 1.6* | 8.8* | 3.5* |
| 2 | IL-2 | vehicle | 1.0 | 1.6* | 1.3* |
| 2 | IL-2 | Cmpd 10 | 1.1 | 2.6* | 2.0* |
| 2 | TNF | vehicle | 1.0 | 1.7* | 1.3* |
| 2 | TNF | Cmpd 10 | 1.5* | 4.0* | 2.3* |
| | | | | | |
| 3 | IFN-γ | vehicle | 1.0 | 1.2 | 0.9 |
| 3 | IFN-γ | Cmpd 10 | 1.2 | 2.5* | 1.8* |
| 3 | IL-2 | vehicle | 1.0 | 1.3* | 1.2 |
| 3 | IL-2 | Cmpd 10 | 1.1 | 2.3* | 1.6* |
| 3 | TNF | vehicle | 1.0 | 2.1* | 1.8* |
| 3 | TNF | Cmpd 10 | 1.5* | 4.6* | 2.9* |

| | | | | | |
|---|---|---|---|---|---|
| ¹Fold-change values for GPR174 inhibitory compound 10 and A2aR inhibitory compounds ZM-241385 and PBF-509, relative to vehicle control. *, p<0.05, t-test, two-tailed distribution, two-sample equal variance. | | | | | |

As described in Example 15, it was determined that the combination of a representative GPR174 inhibitor (compound 10) and the Adenosine 2a Receptor (A2aR) inhibitor ZM-241385 resulted in a synergistic induction of IFN-γ production in human PBMCs. Using the same PBMC stimulation system as described in Example 15, as demonstrated in this Example we have now observed synergistic IFN-γ induction with three A2aR inhibitors: ZM-241385, SCH-58261 and PBF-509 in combination with GPR174 inhibitory compound 10, as shown in FIGURES 49A, 50A and 51A. In all cases, the A2aR inhibitors exhibited greater IFN-γ induction in the presence of GPR174 inhibitory compound 10, and similarly, compound 10 was most effective when combined with an A2aR inhibitor. As further shown in FIGURES 49B, 50B and 51B, we also observed synergistic IL-2 induction with three A2aR inhibitors: ZM-241385, SCH-58261 and PBF-509 in combination with compound 10. As shown in FIGURE 49C, 50C and 51C, synergistic TNF induction with three A2aR inhibitors: ZM-241385, SCH-58261 and PBF-509 in combination with compound 10, was also observed.

These findings solidify our initial finding that inhibition of the A2aR and GPR174 results in cooperative enhancement of T-cell activation and cytokine production. As the cytokines elevated by this activity, IFN-γ, IL-2 and TNF, are characteristic of Th1 cells, these findings lead to the conclusion that the combined inhibition of the GPCRs A2aR and GPR174 will enhance an anti-tumor immune response.

It should be noted that the A2aR inhibitors tested belong to four distinct chemical classes and that none of them affects GPR174 signaling when tested in signaling assays (data not shown). Similarly, the GPR174 inhibitors do not affect A2aR signaling when assessed in signaling assays (data not shown). These data demonstrate that in all cases the observed effects are on target.

Our initial finding utilized 1 µM and 10 µM ZM-241385 doses, which should inhibit both A2aR and A2bR. In contrast, the experiments presented in this Example demonstrate similar activity with two additional A2aR inhibitory compounds possessing greater A2aR specificity over A2bR (see Table 12 in Example 20).

In conclusion, the synergistic cytokine induction observed in the presence of A2aR-selective doses of PBF-509 (0.1 µM) and SCH-58261 (0.2 µM) in combination with GPR174 inhibitory compound 10 indicate that inhibition of A2aR alone in the presence of a GPR174 inhibitory compound (e.g., compound 10) can amplify Th1 responses and thereby enhance anti-tumor responses.

### EXAMPLE 19

This Example describes an extended evaluation of synergistic Th1 cytokine induction in which a total of 44 individual tests of A2aR inhibitor ZM-241385 and GPR174 inhibitory compound 10 were analyzed alone and together in PBMCs isolated from 12 healthy donors.

### Background

As described above in Examples 15 and 18, synergistic IFN-γ induction was observed in PBMCs isolated from several different donors with three different A2aR inhibitors: ZM-241385, SCH-58261 and PBF-509 when each was combined with GPR174i (compound 10) as compared to cytokine induction in the presence of each inhibitor alone.

This Example describes an extended evaluation of synergistic IFN-γ induction in which a total of 44 individual tests of ZM-241385 and compound 10 were analyzed alone and together in PBMCs isolated from 12 healthy donors.

### Methods

Human PBMCs, obtained from twelve individual human volunteer donors were dispensed at density of 1 × 10⁶ cells/well in a 96-well flat-bottom plate and were stimulated in triplicate with 1 µg/mL anti-CD3 antibody (UCHT1) and 0.1 µg/mL anti-CD28 antibody (CD28.2) in the presence of:
GPR174 inhibitory compound 10 (1 µM, 2 µM);
A2aR inhibitory compound (ZM-241385, 0.2 µM, 1 µM);
GPR174 inhibitory compound 10 (1 µM, 2 µM) plus A2aR inhibitory compound (ZM-241385, 0.2 µM, 1 µM); or Vehicle control (DMSO).

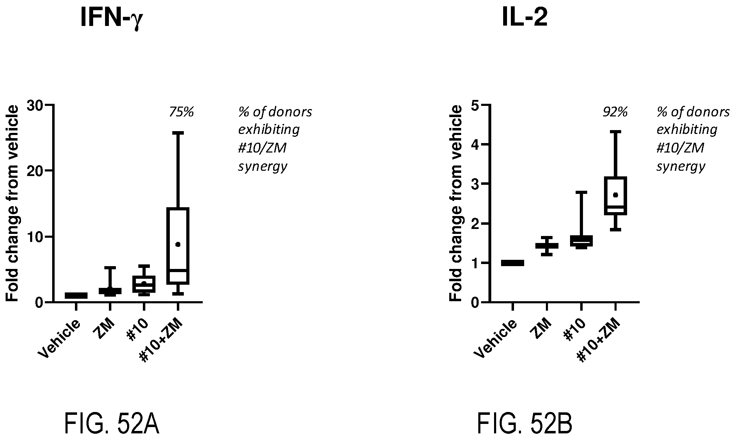

A total of 44 experiments using PBMC from 12 healthy donors were tested (9 donors for GM-CSF), with each donor tested in 1 to 8 separate experiments, depending on the donor (1 to 4 experiments each for GM-CSF). For donors tested multiple times, the mean of replicate experiments was used for the displayed data, such that each donor is represented with a single value. Cytokines were detected in supernatants with the Legendplex (Biolegend) or the MSD (MesoScale) platforms. The median (line), mean (dot) and range (error bars) of the aggregate data are shown in FIGURES 52A-52E. The percentage of donors exhibiting synergistic activity of compound 10 and ZM together (defined as a cytokine increase greater than the sum of increases observed with each compound alone) are shown in each plot. One-way ANOVA with Tukey's post-hoc multiple comparisons correction was performed to identify significant differences (FC, mean fold change; ****, p<0.0001; ***, p<0.001; **, p<0.01; *, p<0.05, ns, not significant).

### Results

FIGURE 52A graphically illustrates the fold change from vehicle in the amount of IFN-γ in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO).

As shown in FIGURE 52A and summarized in Table 11, a high degree of IFN-γ induction was observed with ZM in the presence of compound 10 (8.8-fold average, 25-fold maximum). In contrast, ZM alone resulted in an average IFN-γ induction of 2.1-fold. By itself, compound 10 was slightly more effective than ZM, with an average IFN-γ induction of 2.8-fold (p=0.06) as shown in Table 11. Among the individual donors, 75% (9/12) exhibited synergistic induction of IFN-γ, as the values obtained with both inhibitors exceeded the sum of the increases obtained with each inhibitor alone.

FIGURE 52B graphically illustrates the fold change from vehicle in the amount of IL-2 in culture supernatants 24 hours post-stimulation in human PBMC cultures from 12 donors cultured in the presence of GPR174 inhibitory compound 10 (1-2 µM); A2aR inhibitory compound (ZM, 0.2-1 µM);a combination of compound 10 (1-2 µM) plus 0.2-1 µM ZM; or vehicle control (DMSO).

As shown in FIGURE 52B and summarized in Table 11, a high degree of IL-2 induction was observed with ZM in the presence of compound 10 (2.7-fold average, 4.3-fold maximum). In contrast, ZM alone induced IL-2 1.4-fold on average. Compound 10 by itself was slightly more effective than ZM, inducing IL-2 resulted in a 1.7-fold on average. Among the individual donors, 92% (11/12) exhibited synergistic induction of IL-2, as the values obtained with both inhibitors *(i.e.,* A2aRi and GPR174i) exceeded the sum of the increases obtained with each inhibitor alone.

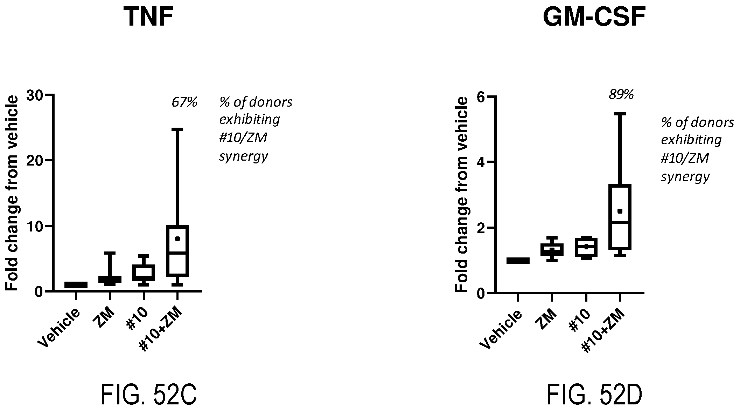

FIGURE 52C graphically illustrates the fold change from vehicle in the amount of TNF in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO).

As shown in FIGURE 52C and summarized in Table 11, on average, a high degree of TNF induction was observed with ZM in the presence of compound 10 (8.0-fold average, 25-fold maximum). In contrast, ZM alone induced TNF 2.1-fold on average. Compound 10 by itself was slightly more effective than ZM, inducing TNF 2.7-fold on average. Among the individual donors, 67% (8/12) exhibited synergistic induction of TNF, as the values obtained with both inhibitors *(i.e.,* A2aRi and GPR174i) exceeded the sum of the increases obtained with each inhibitor alone.

FIGURE 52D graphically illustrates the fold change from vehicle in the amount of GM-CSF in culture supernatants 24 hours post-stimulation in human PBMC cultures from 12 donors cultured in the presence of GPR174 inhibitory compound 10 (1-2 µM); A2aR inhibitory compound (ZM, 0.2-1 µM); a combination of compound 10 (1-2 µM) plus 0.2-1 µM ZM; or vehicle control (DMSO).

As shown in FIGURE 52D and summarized in Table 11, on average, a high degree of granulocyte-macrophage colony stimulating factor (GM-CSF) induction was observed with ZM in the presence of compound 10 (2.5-fold average, 5.5-fold maximum). In contrast, ZM alone induced GM-CSF 1.3-fold. Compound 10 by itself was slightly more effective than ZM, inducing GM-CSF 1.4-fold on average. Among the individual donors, 89% (8/9) exhibited synergistic induction of GM-CSF, as the values obtained with both inhibitors (*i.e.*, A2aRi and GPR174i) exceeded the sum of the increases obtained with each inhibitor alone.

**Table 11: Summary of Results shown in FIGURES 52A-52D**

| | IFN-γ | | IL-2 | | TNF | | GM-CSF | |
|---|---|---|---|---|---|---|---|---|
| | FC¹ | P² | FC | P | FC | P | FC | P |
| Vehicle vs ZM | 2.1 | 0.0412 | 1.4 | <0.0001 | 2.1 | 0.0559 | 1.3 | 0.0116 |
| Vehicle vs Cmpd 10 | 2.8 | 0.0048 | 1.7 | 0.0004 | 2.7 | 0.0074 | 1.4 | 0.0076 |
| Vehicle vs Cmpd 10+ZM | 8.8 | 0.0322 | 2.7 | <0.0001 | 8.0 | 0.0365 | 2.5 | 0.0502 |
| ZM vs Cmpd 10 | 1.3 | 0.0625 | 1.2 | 0.2186 | 1.3 | 0.1131 | 1.1 | 0.6792 |
| ZM vs Cmpd 10+ZM | 4.2 | 0.0376 | 1.9 | 0.0003 | 3.8 | 0.0451 | 1.9 | 0.0858 |
| Cmpd 10 vs Cmpd 10+ZM | 3.1 | 0.0549 | 1.6 | <0.0001 | 3.0 | 0.0610 | 1.8 | 0.1049 |

| | | | | | | | | |
|---|---|---|---|---|---|---|---|---|
| ¹Fold-change for the indicated compound comparison ²p-value for Tukey's multiple comparisons test | | | | | | | | |

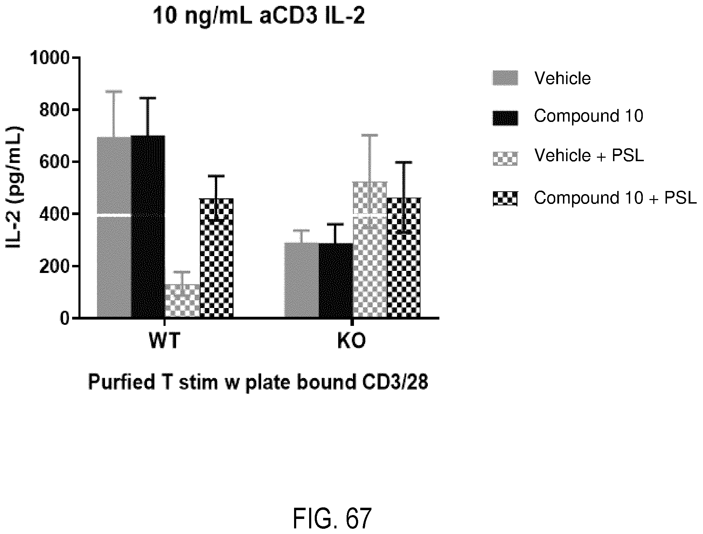

For our extended evaluation of synergistic cytokine induction in PBMCs, we performed a total of 44 individual tests of A2aR inhibitor ZM-241385 and GPR174 inhibitory compound 10 alone and together. We employed PBMCs from 12 healthy donors, wherein individual tests with the same donor ranged from 1 to 8 replicate experiments performed with separate vials of cryopreserved PBMC. For data presentation and analysis of significance, we first derived the average of replicate experiments with the same donor, such that 12 data points from 12 donors were compared for compound effects on cytokine production. Consistent with our initial experiments (described in Examples 15 and 17), ZM-241385 and compound 10 exhibited modest induction of IFN-γ (averaging 2.1 and 2.8-fold, respectively), whereas their combination elicited an average IFN-γ induction of 8.8-fold, ranging up to 25-fold in comparison to vehicle control (see FIGURE 52A). For 75% of donors (9/12), the quantity of IFN-γ induced with the combined inhibitors exceeded the sum of the quantities of IFN-γ induced with each inhibitor alone, indicating that the GPR174 inhibitor and the A2aR inhibitor worked synergistically in the same pathway regulating IFN-γ production in human PBMCs. Similar synergistic effects were observed for induction of IL-2 (FIGURE 52B, 92% of donors), TNF (FIGURE 52C, 67% of donors) and GM-CSF (FIGURE 52D, 89% of donors) wherein synergistic effects were seen in the presence of the combination of ZM-241385 and compound 10 for the majority of donors for each cytokine.

Consistent with the inhibitors promoting Th1 responses, it is noted that additional cytokines characteristic of Th2 and Th17 cells, namely, IL-4, IL-6, IL-10, IL-13 and IL-17, were also measured in some of the experiments described in this Example, all of which were largely unchanged or slightly reduced by compound treatment alone, or in combination (data not shown).

It is also noteworthy that agonists for AdoR (*e.g.*, Ado or NECA) and GPR174 (PS or LysoPS) were not included in the experiments described in this Example. In previous experiments, we found that addition of these agonists did not alter cytokine production, while the AdoR and GPR174 inhibitors increased cytokines to the same degree regardless of agonist supplementation (data not shown), suggesting that natural levels of adenosine and PS/LysoPS present in our high-density PBMC cultures were saturating. This finding also raises the possibility that the immunosuppressive activity of endogenous Ado and PS/LysoPS, both products of cell stress and death that are highly enriched in the tumor microenvironment (Vaupel, P. and Multhoff, G. Front Immunol (8):1887, 2017) may be effectively overcome by combined inhibition of their Gs-coupled GPCRs (*i.e.*, A2aR and GPR174), while inhibition of their individual GPCRs results in suboptimal immune activation. It is noteworthy that, in most experiments, cytokine increases observed with compound 10 alone were greater than those obtained with ZM-241385 alone.

Because we have observed that 0.2 µM ZM-241385 inhibits both A2aR and A2bR (see Table 12 in Example 20) we could not rule out the possibility that both receptors were inhibited for the data shown in this Example. Nevertheless, these findings firmly establish that the combined inhibition of GPR174 with either A2aR, A2bR, or both (*i.e.*, GPR174i plus A2aRi, or GPR174i plus A2bRi, or GPR174i plus A2aRi plus A2bRi) potently and synergistically induce Th1 cytokine responses in total human PBMCs from multiple human donors. Further evaluation of the contribution of A2aR and A2bR to synergy with GPR174 is presented below in Example 20.

### EXAMPLE 20

This Example describes further evaluation of the contribution of A2aRi and A2bRi to synergy with GPR174i.

Because the majority of our experiments employed doses of ZM-241385 that were predicted to inhibit both A2aR and A2bR, we next sought to determine if the observed cytokine increases were due to blockade of A2aR alone or if inhibition of A2bR also played a role. Both receptors signal through the Gs-cAMP pathway and are known to suppress immune responses, and both receptors are considered important targets for cancer immunotherapy, with receptor-specific inhibitors and with inhibitors that target both A2aR and A2bR (see *e.g.*, Vigano S. et al., Front Immunol vol 10: 925, 2019). Determining whether inhibition of both or either receptor achieves the maximal cytokine induction when combined with a GPR174 inhibitor should have important implications for the development of strategies to optimally target both pathways.

To explore the relative contribution of A2aR and A2bR inhibition to synergy with compound 10, we evaluated A2aR-specific doses of PBF-509 (0.1 µM) and SCH-58261 (0.2 µM), with and without the A2bR inhibitor MRS-1754 (1 µM). In addition, we evaluated effects of adenosine deaminase (ADA; 0.75 ug/mL), an enzyme that catabolizes adenosine into inosine and which should promote effects similar to A2aR and A2bR dual inhibition.

### Methods

In an initial experiment, several A2aR and A2bR inhibitors were analyzed for specificity at each receptor and the IC₅₀ values were determined as follows: HEK293 cells were transiently co-transfected with A2aR or A2bR expression constructs (Origene, MD), Cre-luciferase, and TK-renilla as an internal control (Promega) with Lipofectamine 2000 (Invitrogen, CA) according to the manufacturer's directions. The transfected cells were incubated for 4-6 hours at 37°C and then trypsinized and transferred to a 96-well plate. Increasing concentrations of the inhibitors (ZM-241385, SCH-58261, PBF-509 and MRS-1754) were added to the cells and then incubated overnight at 37°C. After overnight incubation, the media was removed, and the cells were lysed. Luciferase and renilla activities were measured with the Dual-luciferase assay system (Promega, WI) according to the manufacturer's directions. Lysis buffer (25 µL) was added to the cells and incubated at room temperature for 20-30 minutes to allow for the cells to lyse. The lysate (10 µL) was measured for luciferase and renilla activity with LARII and Stop&Glo reagent (50 µL each) with a SpectraMax L (Molecular Devices, CA). The results are shown in Table 12 below.

**Table 12: Specificity of Various A2aR and A2bR inhibitors**

| | A2A (IC₅₀ nM) | A2B (IC₅₀ nM) |
|---|---|---|
| ZM-241385 | 4.8 | 72 |
| SCH-58261 | 40 | >2000 |
| PBF-509 | 5.6 | 680 |
| MRS-1754 | ~1000 | 32 |

It is noted that MRS-1754 did not affect GPR174 signaling when tested in signaling assays (data not shown). Similarly, the GPR174 inhibitor did not affect A2bR signaling when assessed in signaling assays (data not shown).

Human PBMC (1 × 10⁶ cells/well in a 96-well flat-bottom plate) obtained from donor 3 and donor 6 were stimulated with 1 µg/mL anti-CD3 antibody (UCHT1) and 0.1 µg/mL anti-CD28 antibody (CD28.2) in the presence of:
GPR174 inhibitory compound 10 (1 µM);
A2aR inhibitory compound ZM-241385 (0.1 µM);
A2aR inhibitory compound PBF-509 (0.1 µM);
A2aR inhibitory compound SCH-58261 (0.2 µM);
A2bR inhibitory compound MRS-1754 (1 µM);
Adenosine deaminase ("ADA") (0.75 µg/mL);
and combinations of these reagents as indicated in Table 13 below.

Supernatants from these cells were collected at 24 hours post-stimulation and the levels of IFN-γ, IL-2, and TNF were determined with the MSD platform (MesoScale Discovery).

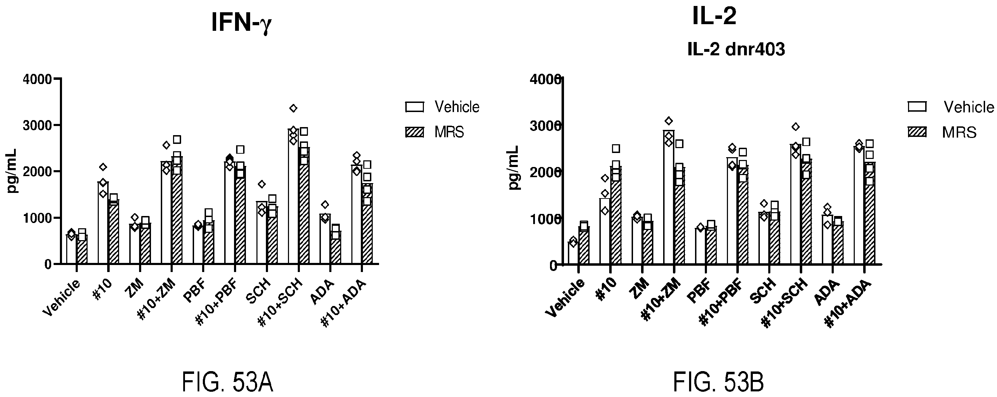

FIGURE 53A graphically illustrates the amount of IFN-γ (pg/mL) in culture supernatants 24 hours post-stimulation of human PBMC cultures from a single donor (donor 3) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 53A and summarized in Table 13, the highest degree of IFN-γ induction was observed when compound 10 was combined with an A2aR inhibitor (ZM, PBF-509, or SCH) or with adenosine deaminase (ADA), resulting in 3.3- to 4.5-fold increases of IFN-γ relative to vehicle control. By itself, compound 10 increased IFN-γ levels 2.8-fold, while the A2aR and A2bR inhibitors and ADA alone had relatively modest effects (1.0- to 2.1-fold increases). Inclusion of the A2bR inhibitor (MRS) did not further enhance IFN-γ levels in any combination. Together, these findings demonstrate that the synergistic activity between GPR174 inhibition and A2aR/A2bR inhibition for IFN-γ production from donor 3 PBMC relied on A2aR inhibition without A2bR involvement.

FIGURE 53B graphically illustrates the amount of IL-2 (pg/mL) in culture supernatants 24 hours post-stimulation of human PBMC cultures from a single donor (donor 3) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 53B and summarized in Table 13, the highest degree of IL-2 induction was observed when compound 10 was combined with an A2aR inhibitor (ZM, PBF-509, or SCH) or with adenosine deaminase (ADA), resulting in 4.7- to 5.9-fold increases relative to vehicle control. By itself, compound 10 increased IL-2 levels 2.9-fold, while the A2aR inhibitors alone and ADA alone had relatively modest effects (1.6- to 2.3-fold increases). Inclusion of the A2bR inhibitor (MRS) with A2aR inhibitors or ADA did not further enhance IL-2 levels; however, in cultures devoid of A2aR inhibitors, MRS alone increased IL-2 levels 1.7-fold, and, with compound 10, 4.3-fold. Together, these findings demonstrate that the synergistic activity between GPR174 inhibition and A2aR/A2bR inhibition for IL-2 production from donor 3 PBMC relied mostly on A2aR inhibition, but that A2bR inhibition could also cooperate with GPR174 inhibition to enhance IL-2 levels.

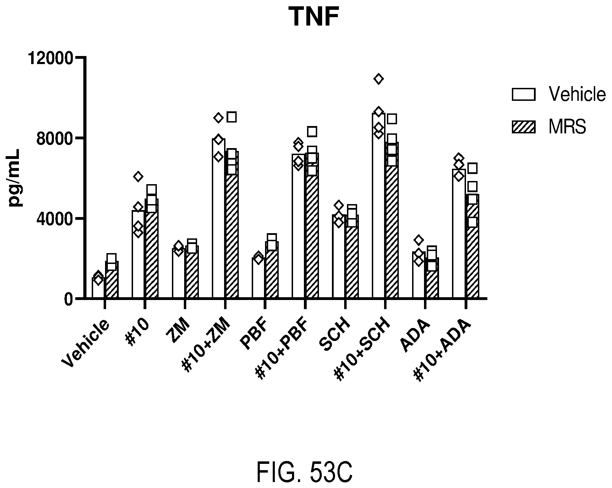

FIGURE 53C graphically illustrates the amount of TNF (pg/mL) in culture supernatants 24 hours post-stimulation of human PBMC cultures from a single donor (donor 3) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 53C and summarized in Table 13, the highest degree of TNF induction was observed when compound 10 was combined with an A2aR inhibitor (ZM, PBF-509, or SCH) or with adenosine deaminase (ADA), resulting in 6.0- to 8.6-fold increases relative to vehicle control. By itself, compound 10 increased TNF levels 4.1-fold, while the A2aR inhibitors alone and ADA alone had relatively modest effects (1.9- to 3.9-fold increases). Inclusion of the A2bR inhibitor (MRS) with A2aR inhibitors or ADA did not further enhance IL-2 levels; however, MRS alone increased IL-2 levels 1.8-fold, and with PBF-509, MRS also slightly increased TNF levels. Together, these findings demonstrate that the synergistic activity between GPR174 inhibition and A2aR/A2bR inhibition for TNF production from donor 3 PBMC relied predominantly on A2aR inhibition.

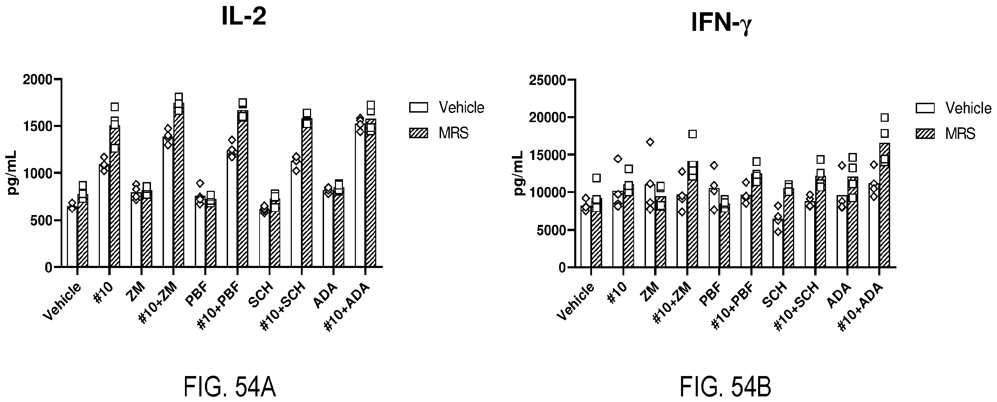

FIGURE 54A graphically illustrates the amount of IFN-γ (pg/mL) in culture supernatants 24 hours post-stimulation of human PBMC cultures from a single donor (donor 6) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 54A and summarized in Table 13, the highest degree of IFN-γ induction was observed when compound 10 was combined with the A2bR inhibitor MRS and an A2aR inhibitor or adenosine deaminase (ADA), resulting in 1.5- to 2.0-fold increases relative to vehicle control. By itself, compound 10 did not significantly increase IFN-γ levels. Similarly, the A2aR and A2bR inhibitors and ADA had no significant effect on their own. The modest effect of all the compounds on IFN-γ levels in this experiment is likely due to the fact that donor 6 responded vigorously to anti-CD3, anti-CD28 stimulation, resulting in very high IFN-γ production (8,000 to 16,000 pg/mL) that could not be further enhanced to a large extent by GPR174 and A2aR/A2bR inhibitors. Nevertheless, an involvement of A2bR inhibition was observable if combined with A2aR and GPR174 inhibitors. Likewise, the only significant increase in IFN-γ levels in the absence of the A2bR inhibitor was observed with the GPR174 inhibitor combined with ADA (1.4-fold), which should have reduced adenosine activity on both A2aR and A2bR. Further enhancement of IFN-γ levels observed when MRS was added to compound 10 and ADA (2.0-fold from vehicle control) may be due to the possibility that ADA was not used at a saturating concentration for this donor, allowing for some activity of adenosine on A2bR to directly or indirectly suppress IFN-γ expression.

FIGURE 54B graphically illustrates the amount of IL-2 (pg/mL) in culture supernatants 24 hours post-stimulation of human PBMC cultures from a single donor (donor 6) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 54B and summarized in Table 13, the highest degree of IL-2 induction was observed when compound 10 was combined with the A2bR inhibitor (MRS) or with adenosine deaminase (ADA), resulting in 2.3- to 2.7-fold increases relative to vehicle control. By itself, compound 10 increased IL-2 levels 1.7-fold, while the A2aR inhibitors alone and ADA alone had no or relatively modest effects (1.0- to 1.3-fold increases). Combination of compound 10 with A2aR inhibitors increased IL-2 to intermediate levels (1.7-to 2.2-fold). In cultures devoid of A2aR inhibitors, MRS alone increased IL-2 levels 1.2-fold, and, with compound 10, 1.4-fold. Together, these findings demonstrate that the synergistic activity between GPR174 inhibition and A2aR/A2bR inhibition for IL-2 production from donor 6 PBMC relied on the combined effects of both A2aR and A2bR inhibition.

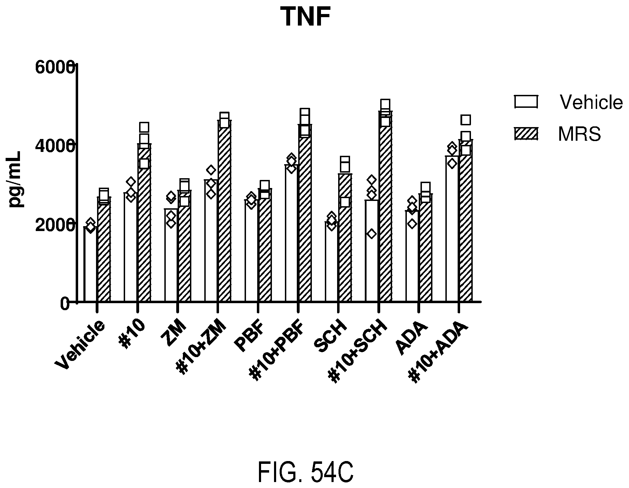

FIGURE 54C graphically illustrates the amount of TNF (pg/mL) in culture supernatants 24 hours post-stimulation of human PBMC cultures from a single donor (donor 6) cultured in the presence of GPR174 inhibitory compound 10 (1 µM); ZM, 0.1 µM); PBF-509 (0.1 µM), SCH (0.2 µM), MRS (1 µM), Adenosine deaminase (ADA, 0.75 µg/mL), alone or in combination with compound 10, or vehicle control.

As shown in FIGURE 54C and summarized in Table 13, the highest degree of TNF induction was observed when compound 10 was combined with the A2bR inhibitor (MRS) or with adenosine deaminase (ADA), resulting in 2.1- to 2.5-fold increases relative to vehicle control. By itself, compound 10 increased TNF levels 1.4-fold, while the A2aR inhibitors alone and ADA alone had no or relatively modest effects (1.1- to 1.4-fold increases). The combination of compound 10 with A2aR inhibitors increased TNF to intermediate levels (1.3- to 1.8-fold). In cultures devoid of A2aR inhibitors, MRS alone increased TNF levels 1.4-fold, and, with compound 10, 2.1-fold. Together, these findings demonstrate that the synergistic activity between GPR174 inhibition and A2aR/A2bR inhibition for TNF production from donor 6 PBMC relied on the combined effects of both A2aR and A2bR inhibition.

**Table 13: Summary of Results Shown in FIGURE 53A-C and 54A-C**

| | | Donor 3 | | | donor 6 | | |
|---|---|---|---|---|---|---|---|
| | | Veh¹ | MRS¹ | MRS/Veh² | Veh¹ | MRS¹ | MRS/Veh² |
| IFN-γ | Vehicle | 1.0 | 1.0 | 1.0 | 1.0 | 1.1 | 1.1 |
| | Cmpd 10 | 2.8* | 2.2* | 0.8* | 1.2 | 1.3* | 1.1 |
| | ZM | 1.4* | 1.4* | 1.0 | 1.3 | 1.2 | 0.9 |
| | Cmpd 10 + ZM | 3.4* | 3.6* | 1.0 | 1.2 | 1.7* | 1.5* |
| | PBF | 1.3* | 1.5* | 1.1 | 1.3 | 1.0 | 0.8 |
| | Cmpd 10 + PBF | 3.4* | 3.3* | 1.0 | 1.2 | 1.5* | 1.3* |
| | SCH | 2.1* | 1.9* | 0.9 | 0.8 | 1.3* | 1.6* |
| | Cmpd 10 + SCH | 4.5* | 3.9* | 0.9 | 1.1 | 1.5* | 1.4* |
| | ADA | 1.7* | 1.1 | 0.7* | 1.2 | 1.5* | 1.3 |
| | Cmpd 10 + ADA | 3.3* | 2.7* | 0.8 | 1.4* | 2.0* | 1.5* |
| | | | | | | | |
| IL-2 | Vehicle | 1.0 | 1.7* | 1.7* | 1.0 | 1.2* | 1.2* |
| | Cmpd 10 | 2.9* | 4.3* | 1.5* | 1.7* | 2.3* | 1.4* |
| | ZM | 2.1* | 1.9* | 0.9 | 1.2* | 1.3* | 1.0 |
| | Cmpd 10 + ZM | 5.9* | 4.3* | 0.7* | 2.2* | 2.7* | 1.3* |
| | PBF | 1.6* | 1.7* | 1.1 | 1.2 | 1.1* | 1.0 |
| | Cmpd 10 + PBF | 4.7* | 4.3* | 0.9 | 1.9* | 2.6* | 1.3* |
| | SCH | 2.3* | 2.3* | 1.0 | 1.0 | 1.1 | 1.2* |
| | Cmpd 10 + SCH | 5.3* | 4.6* | 0.9 | 1.7* | 2.5* | 1.4* |
| | ADA | 2.2* | 1.9* | 0.9 | 1.3* | 1.3* | 1.0 |
| | Cmpd 10 + ADA | 5.2* | 4.5* | 0.9 | 2.4* | 2.5* | 1.0 |
| | | | | | | | |
| TNF | Vehicle | 1.0 | 1.8* | 1.8* | 1.0 | 1.4* | 1.4* |
| | Cmpd 10 | 4.1* | 4.6* | 1.1 | 1.4* | 2.1* | 1.4* |
| | ZM | 2.4* | 2.5* | 1.1 | 1.2* | 1.5* | 1.2 |
| | Cmpd 10 + ZM | 7.4* | 6.9* | 0.9 | 1.6* | 2.4* | 1.5* |
| | PBF | 1.9* | 2.7* | 1.4* | 1.4* | 1.5* | 1.1* |
| | Cmpd 10 + PBF | 6.7* | 6.8* | 1.0 | 1.8* | 2.3* | 1.3* |
| | SCH | 3.9* | 3.9* | 1.0 | 1.1 | 1.7* | 1.6* |
| | Cmpd 10 + SCH | 8.6* | 7.3* | 0.8 | 1.3 | 2.5* | 1.9* |
| | ADA | 2.2* | 1.9* | 0.9 | 1.2* | 1.4* | 1.2* |
| | Cmpd 10 + ADA | 6.0* | 4.9* | 0.8 | 1.9* | 2.1* | 1.1 |

| | | | | | | | |
|---|---|---|---|---|---|---|---|
| ¹ The ratio of cytokine concentration values for the indicated compound combination relative to vehicle control. ²The ratio of cytokine concentration values for each compound combination in the presence vs. the absence of MRS-1754. *, p<0.05, t-test, two-tailed distribution, two-sample equal variance. | | | | | | | |

The findings presented in this example indicate that both A2aR and A2bR inhibition can cooperate with GPR174 inhibition to elicit maximal cytokine induction, and that conditions can exist where inhibition of all 3 GPCRs results in the highest cytokine induction. With donor 3, inhibition of A2aR alone provided the maximal cytokine induction when combined with compound 10, as the inclusion of the A2bR inhibitor (MRS) or the removal of adenosine with ADA did not further increase cytokine levels (with the exception of small increases in IL-2 in the absence of an A2aR inhibitor). With donor 6, addition of the A2bR was necessary to achieve the highest levels of IFN-g, IL-2 and TNF.

Three other cancer immunotherapy targets in the adenosine pathway are CD38, CD39 and CD73, ectonucleotidases that are involved in the production of adenosine. CD39 and CD73 convert ATP into adenosine through phosphate removal. Adenosine may also be produced from NAD+ by an axis centered on the NAD+ metabolizing CD38 generating adenosine diphosphate ribose (ADPR).

Our finding that, in the presence of a GPR174 inhibitor, adenosine depletion with ADA leads to maximal cytokine induction similar to what is achieved with combined A2aR/A2bR inhibition indicates that combination of a GPR174 inhibitor with inhibitors of CD38, CD39 or CD73 should also enhance anti-tumor immune responses.

### EXAMPLE 21

This Example demonstrates that four compounds in two chemical classes of GPR174 inhibitors elicited nearly identical Th1 cytokine profiles from total PBMC stimulated with mitogenic anti-CD3 and anti-CD28 antibodies, and that similar degrees of synergy with the A2aR inhibitor ZM-241385 were obtained for all four GPR174 inhibitors.

As the data presented thus far in the PBMC cytokine release assays made use of a single GPR174 inhibitor (compound 10), it was important to demonstrate that the activity we observed was due to the inhibitor acting on GPR174 rather than an off-target activity unique to a particular compound. To rule out the possibility of compound-specific off-target activity, we tested three additional GPR174 inhibitors: two from the same chemical series as compound 10 (compounds 6 and 11, Group I) and a third compound of a different chemical class (compound 20, Group II). Based on the IC₅₀ values obtained with these compounds in a signaling assay (see Example 16, Figure 47), we tested these compounds at two concentrations: one predicted to efficiently inhibit GPR174 and one that might exhibit partial inhibition in the total PBMC stimulation assay.

Human PBMC (1 × 10⁶ cells/well in a 96-well flat-bottom plate) obtained from donor 7 were stimulated with 1 µg/mL anti-CD3 antibody (UCHT1) and 0.1 µg/mL anti-CD28 antibody (CD28.2) in the presence of:
GPR174 inhibitory compound 10 (1 and 0.3 µM);
GPR174 inhibitory compound 6 (1 and 0.3 µM);
GPR174 inhibitory compound 11 (1 and 0.3 µM);
GPR174 inhibitory compound 20 (10 and 3 µM);
each with and without the A2aR inhibitory compound ZM-241385 (0.1 µM).

Supernatants from these cells were collected at 24 hours post-stimulation and the levels of IFN-γ, IL-2, TNF and GM-CSF were determined with the MSD platform (MesoScale Discovery).

### Results

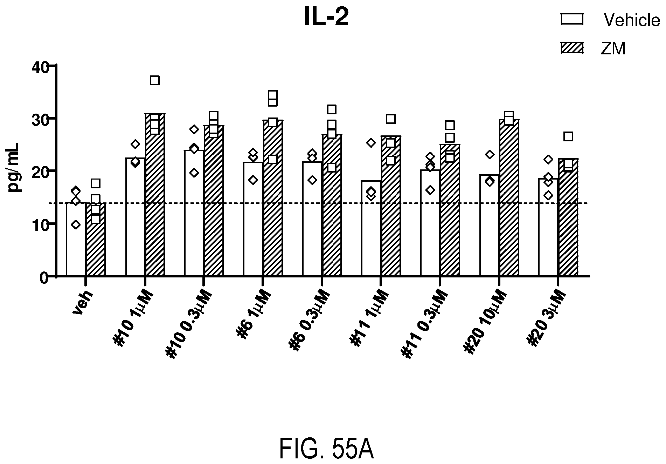

FIGURE 55A graphically illustrates the amount of IFN-γ (pg/mL) in supernatants 24 hours post-stimulation of human PBMC (donor 7) in the presence of GPR174 inhibitory compounds 6, 10, 11 or 20, each in the presence or absence of ZM.

As shown in FIGURE 55A and summarized in Table 14, all four GPR174 inhibitory compounds 6, 10, 11 and 20 synergized with ZM to increase IFN-γ to levels greater than the sum of the increases obtained with each inhibitor alone. Synergy was observed between ZM and both the high and low concentrations of each GPR174 inhibitor.

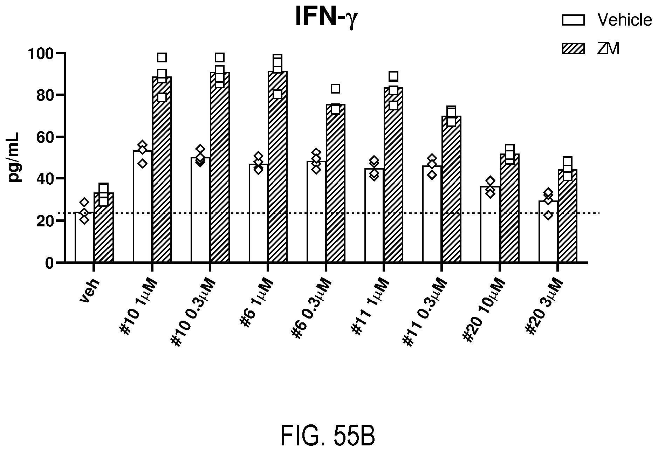

FIGURE 55B graphically illustrates the amount of IL-2 (pg/mL) in supernatants 24 hours post-stimulation of human PBMC (donor 7) in the presence of GPR174 inhibitory compounds 6, 10, 11 or 20, each in the presence or absence of ZM.

As shown in FIGURE 55B and summarized in Table 14, all four GPR174 inhibitory compounds 6, 10, 11 and 20 synergized with ZM to increase IL-2 to levels greater than the sum of the increases obtained with each inhibitor alone. Synergy was observed between ZM and both the high and low concentrations of each GPR174 inhibitor, with the exception of the low dose of compound 20, the least potent of the four compounds (see Example 16, Figure 47).

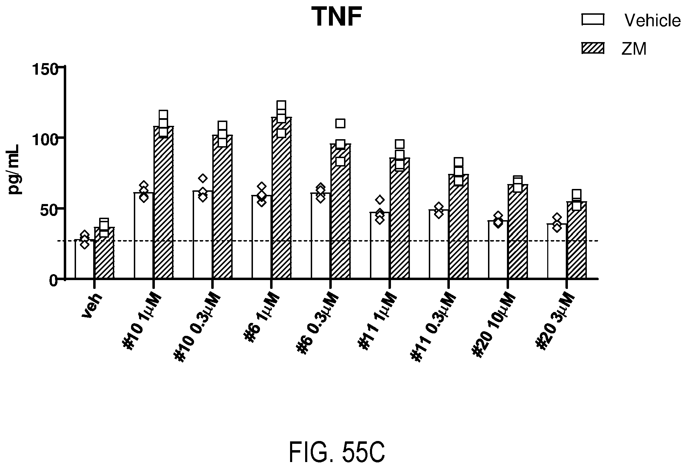

FIGURE 55C graphically illustrates the amount of TNF (pg/mL) in supernatants 24 hours post-stimulation of human PBMC (donor 7) in the presence of GPR174 inhibitory compounds 6, 10, 11 or 20, each in the presence or absence of ZM.

As shown in FIGURE 55C and summarized in Table 14, all four GPR174 inhibitory compounds 6, 10, 11 and 20 synergized with ZM to increase TNF to levels greater than the sum of the increases obtained with each inhibitor alone. Synergy was observed between ZM and both the high and low concentrations of each GPR174 inhibitor, with the exception of the low dose of compound 20, the least potent of the 4 compounds (see Example 16, Figure 47).

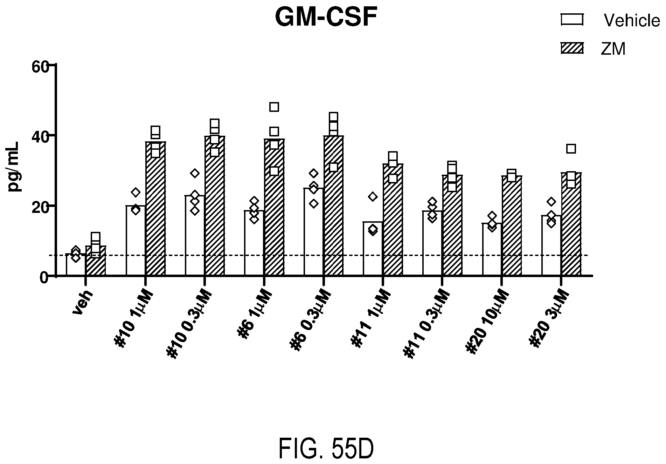

FIGURE 55D graphically illustrates the amount of GM-CSF (pg/mL) in supernatants 24 hours post-stimulation of human PBMC (donor 7) in the presence of GPR174 inhibitory compounds 6, 10, 11 or 20, each in the presence or absence of ZM.

As shown in FIGURE 55D and summarized in Table 14, all four GPR174 inhibitory compounds 6, 10, 11 and 20 synergized with ZM to increase GM-CSF to levels greater than the sum of the increases obtained with each inhibitor alone. Synergy was observed between ZM and both the high and low concentrations of each GPR174 inhibitor, with the exception of the low dose of compound 20, the least potent of the four compounds (see Example 16, Figure 47).

**Table 14: Summary of results shown in Figures 55A-D.**

| | | | Compound 10 | | Compound 6 | | Compound 11 | | Compound 20 | |
|---|---|---|---|---|---|---|---|---|---|---|
| | | Veh | 1 µM¹ | 0.3 µM | 1 µM | 0.3 µM | 1 µM | 0.3 µM | 10 µM | 3 µM |
| IFN-γ | Veh | 1.0 | 2.2* | 2.1* | 1.9* | 2.0* | 1.8* | 1.9* | 1.5* | 1.2 |
| | ZM | 1.4* | 3.7* | 3.7* | 3.8* | 3.1* | 3.4* | 2.9* | 2.1* | 1.8* |
| IL-2 | Veh | 1.0 | 1.6* | 1.7* | 1.5* | 1.5* | 1.3 | 1.4* | 1.4* | 1.3 |
| | ZM | 1.0 | 2.2* | 2.0* | 2.1* | 1.9* | 1.9* | 1.8* | 2.1* | 1.6* |
| TNF | Veh | 1.0 | 2.2* | 2.2* | 2.1* | 2.1* | 1.7* | 1.7* | 1.5* | 1.4* |
| | ZM | 1.3* | 3.8* | 3.6* | 4.0* | 3.4* | 3.0* | 2.6* | 2.4* | 1.9* |
| GM-CSF | Veh | 1.0 | 3.1* | 3.6* | 2.9* | 3.9* | 2.4* | 2.9* | 2.3* | 2.7* |
| | ZM | 1.3 | 5.9* | 6.2* | 6.0* | 6.2* | 4.9* | 4.4* | 4.4* | 4.6* |

| | | | | | | | | | | |
|---|---|---|---|---|---|---|---|---|---|---|
| ¹ The ratio of cytokine concentration values for the indicated compound combination relative to vehicle control (Veh, Veh). *, p<0.05, t-test, two-tailed distribution, two-sample equal variance. | | | | | | | | | | |

In this Example, we demonstrated that four compounds covering two chemical classes of GPR174 inhibitors elicited nearly identical Th1 cytokine profiles from total PBMC stimulated with mitogenic anti-CD3 and anti-CD28 antibodies, and that similar degrees of synergy with the A2aR inhibitor ZM-241385 was obtained for all four GPR174 inhibitors. This finding further confirms that the observed effects are due to each of the four compounds acting on GPR174 rather than other molecular targets.

In summary, our findings demonstrate that combined inhibition of GPR174 and the adenosine/A2aR/A2bR pathway results in synergistic enhancement of immune responses. The synergy observed when GPR174 and A2aR/A2bR inhibitors were combined led to, in some cases, a striking 25-fold increase in IFN-γ and TNF levels. Because the amplified responses are of the Th1 polarity, and because both the adenosine and the GPR174 agonists PS/lysoPS are highly enriched in the tumor microenvironment (see *e.g.*, Vigano S. et al., Front Immunol vol 10: 925, 2019; Li et al., Molecular Cancer Therapeutics vol 17 (1): 169-82, 2017; and Desai T.J. et al., Oncotarget 7:30678-30690, 2016), our findings suggest that combined inhibition of both pathways in cancer patients will be important to effectively resist the immunosuppressive nature of the tumor microenvironment and potentiate tumoricidal immune responses.

### EXAMPLE 22

This Example describes an extended evaluation of synergistic Th1 cytokine induction in which an A2aR inhibitor (ZM-241385), an A2bR inhibitor (MRS1754) and a GPR174 inhibitory (compound 10) were analyzed alone and in combination in PBMCs isolated from 5 healthy donors.

### Methods

The methods were carried out as described in Example 20, with the modification that human PBMCs from 5 healthy donors were stimulated in the presence of an A2aR inhibitor (ZM-24138: 0.2 µM), an A2bR inhibitor (MRS1754: 2 µM) and GPR174 inhibitory compound 10 (1 µM) were analyzed alone and in combination. Supernatants from these cells were collected at 24 hours post-stimulation and the levels of IFN-γ, IL-2 and TNF were determined with the MSD platform (MesoScale Discovery).

### Results

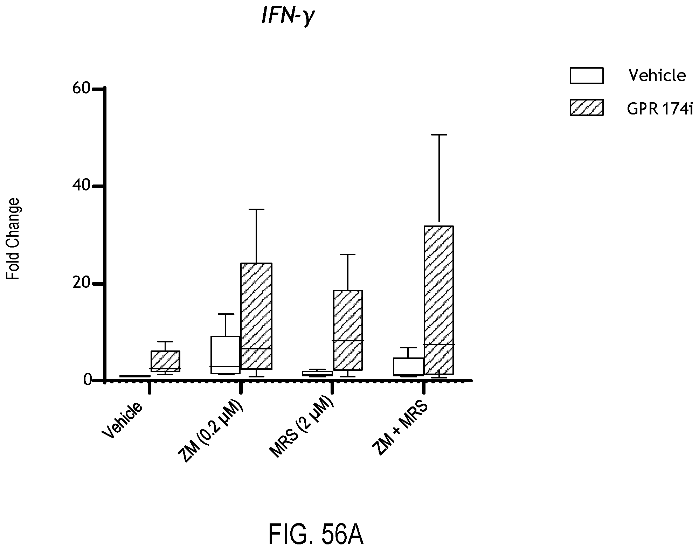

The results showing the effects of combined inhibition of GPR174, A2aR and A2bR in normalized data from 5 human donors are shown in FIGURES 56A-C. Note: GPR174i = Compound 10.

FIGURE 56A graphically illustrates the fold change from vehicle in the amount of IFN-γ in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1 µM) plus 0.2 µM ZM plus 2 µM MRS, or vehicle control (DMSO).

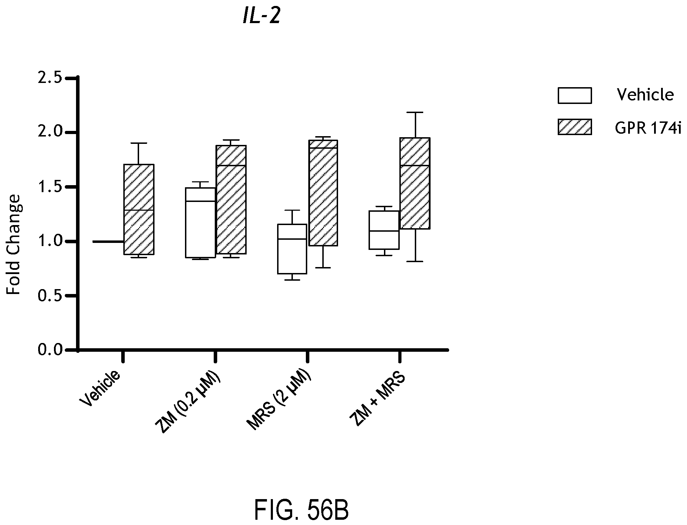

FIGURE 56B graphically illustrates the fold change from vehicle in the amount of IL-2 in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1 µM) plus 0.2 µM ZM plus 2 µM MRS, or vehicle control (DMSO).

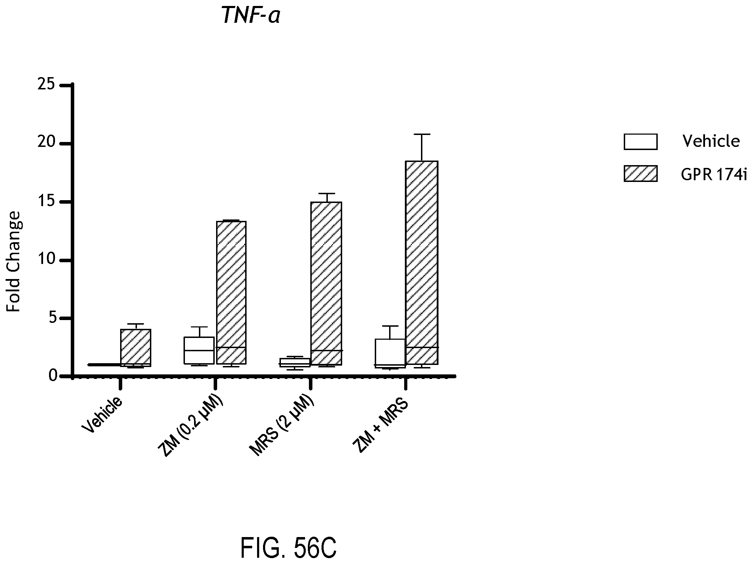

FIGURE 56C graphically illustrates the fold change from vehicle in the amount of TNF in culture supernatants 24 hours post-stimulation in human PBMC cultures from 5 donors cultured in the presence of GPR174 inhibitory compound 10 (1 µM); A2aR inhibitory compound (ZM, 0.2 µM); A2bR inhibitory compound MRS (2 µM), or a combination of compound 10 (1-2 µM) plus 0.2 µM ZM plus 2 µM MRS, or vehicle control (DMSO).

### Discussion

The results shown in this Example further confirm and extend the results shown in Example 20 and indicate that both A2aR and A2bR inhibition can cooperate with GPR174 inhibition to elicit maximal cytokine induction, and that conditions can exist where inhibition of all 3 GPCRs results in the highest cytokine induction.

### EXAMPLE 23

This Example describes a study that was carried out to assess the effect of representative GPR174 inhibitory compound 10 alone or in combination with an A2aR inhibitor (ZM-241385) on the expression of amphiregulin (AREG).

### Methods

The methods were carried out as described in Example 14, modified in that the cells were incubated in the presence of GPR174 inhibitory compound 10 at 1 µM (GPR174i), A2aR inhibitor ZM-241385 at 0.2 µM or the combination of compound 10 (1 µM) plus ZM (0.2 µM).

### Results

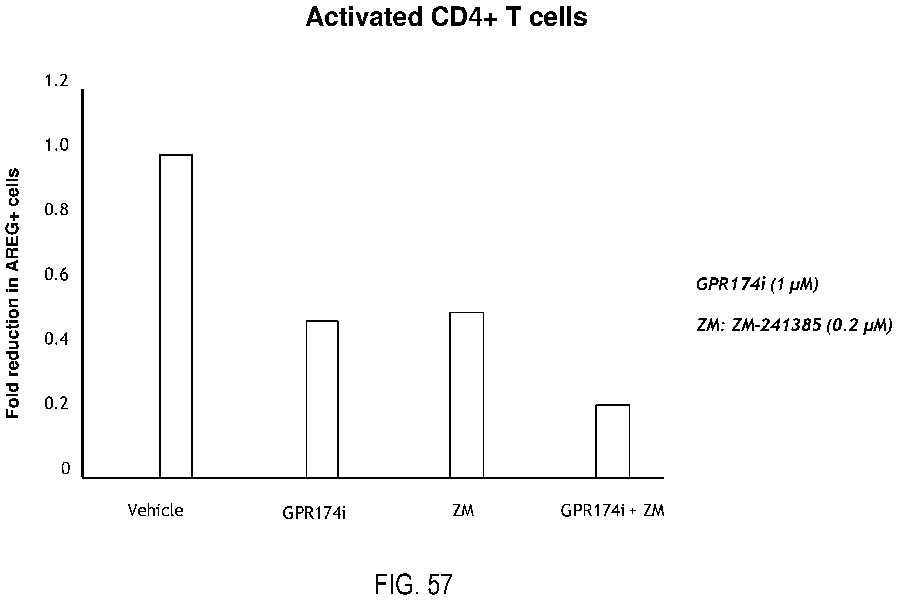

FIGURE 57 graphically illustrates the fold reduction in AREG+ cells in the presence of vehicle; GPR174 inhibitory compound 10 (1 µM); ZM-241385 (0.2 µM); or the combination of compound 10 (1 µM) plus ZM (0.2 µM).

### Discussion

The results shown in this Example are consistent with the results described in Example 14 and demonstrate that the combination of a GPR174 inhibitor and an A2aR inhibitor further reduce expression of AREG in activated CD4+ T-cells as compared to each agent alone.

### Summary

As described and demonstrated herein, GPR174 is an orphan GPCR that is expressed almost exclusively in immune cells (T, B and NK cells), and numerous GPR174 inhibitors have been identified that fall into six different chemical classes, as described herein. As further disclosed herein, the inventors have discovered that phosphatidylserine (PS) is a GPR174 agonist. As further disclosed herein, GPR174 inhibition potentiates the immune system, in particular the Th1 response and suppresses tumor promoters. As further disclosed herein, the combined inhibition of GPR174 and the adenosine pathway synergistically enhances Th1 cytokine production. These findings represent a new therapeutic approach in cancer immunotherapy for all solid tumors. This therapeutic approach includes the use of a GPR174 inhibitor as a single agent or in combination with one or more of an A2aR inhibitor, an A2bR inhibitor, a CD38 inhibitor, a CD39 inhibitor and/or a CD73 inhibitor for the treatment of a solid tumor in a subject in need thereof. This approach is also amenable to combination with check-point inhibitors and cellular therapies.

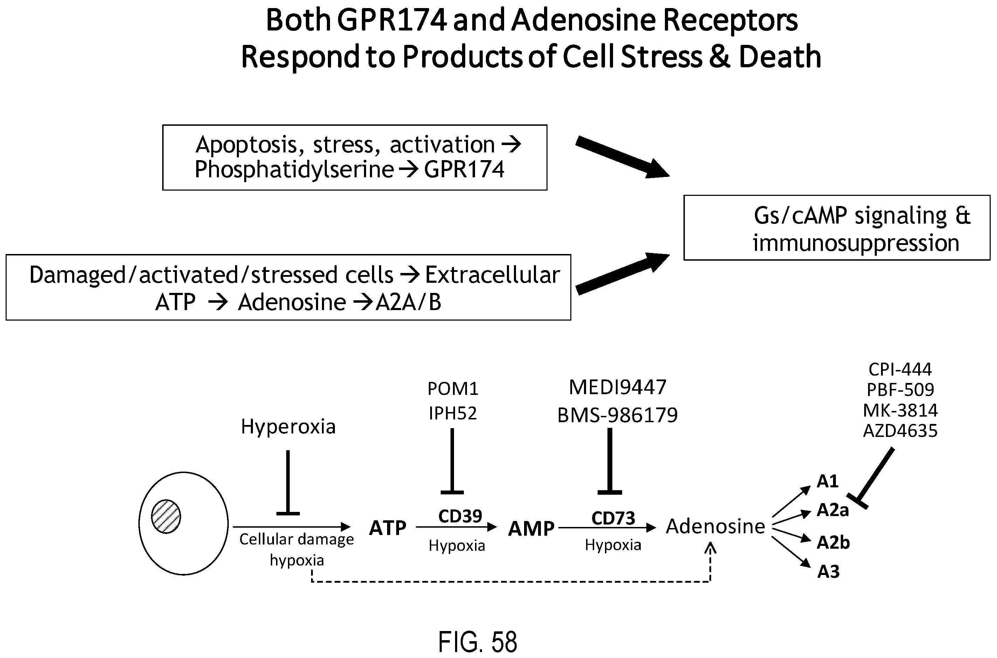

FIGURE 58 is a schematic diagram illustrating how both GPR174 and Adenosine receptors respond to products of cell stress and death. As illustrated in FIGURE 58, both GPR174 and adenosine receptors respond to products of cell stress and death. As shown in FIGURE 58, in the GPR174-mediated pathway, apoptosis and stress lead to phosphatidylserine (PS) which is an agonist of GPR174 and causes Gs/cAMP signaling and results in immunosuppression. As further shown in FIGURE 58, in the adenosine pathway, damaged/activated/stressed cells lead to extracellular ATP which is converted to adenosine, which is an agonist for A2A and A2B receptors which also causes Gs/cAMP signaling and immunosuppression. As further shown in FIGURE 58, the adenosine pathway can be inhibited by CD39 inhibitors (*e.g.*, POM1, IPH52), or by CD73 inhibitors (*e.g.*, MEDI9447, BMS-986179), or by an A2aR inhibitor (*e.g*., CPI-444, PBF-509, MK-3814 or AZD4635) or an A2bR inhibitor. As demonstrated herein, inhibition of both the GPR174-mediated pathway and the adenosine pathway leads to effective cAMP reduction and immune stimulation.

In summary, the results described herein demonstrate (1) cancer-immunity pathways controlled by GPR174; (2) the identification of phosphatidylserine (PS) as a potent natural ligand for GPR174; (3) a collection of novel small-molecule inhibitors of GPR174; and (4) dramatic and synergistic enhancement of "tumor-fighting" cytokine production by T cells following the combined inhibition of both GPR174 and the adenosine pathway, another key metabolic pathway that regulates tumor immunity. Further in this regard, as demonstrated herein, GPR174 inhibitors enhance adenosine pathway inhibitors' tumor-fighting cytokine production by up to 25-fold. The result is a new cancer immunotherapy approach targeting inhibition of GPR174, which can be combined with and significantly improve the tumor killing effects of adenosine pathway inhibitors. GPR174-targeting immunotherapy is expected to be applicable to all solid tumors (*e*.*g*., breast, lung, pancreas, colon, brain, etc.).

Like GPR174, A2A/A2B adenosine receptors are GPCRs. Central components of the adenosine pathway, A2A/A2B receptors are being targeted for cancer immunotherapy by several companies, as shown in Table 15 below. GPR174 and A2A/A2B receptors share certain features. First, each of them increases intracellular cyclic adenosine monophosphate (cAMP), which is well known for suppressing the type of immune response necessary for killing tumor cells. Second, these receptors are activated by molecules (*i.e.*, PS and adenosine, respectively) that are highly enriched in the tumor microenvironment. Strikingly, in experiments with total human peripheral blood mononuclear cells (PBMCs) where PS and adenosine are naturally abundant, GPR174 inhibitors synergized with A2A/A2B inhibitors to increase T-cell responses dramatically. Findings include the following:
A2A/A2B inhibition alone yielded, on average, 2-fold increases in both interferon-gamma (IFN-γ) and tumor necrosis factor (TNF) and smaller increases in granulocyte-macrophage colony stimulating factor (GM-CSF) and interleukin-2 (IL-2);
GPR174 inhibition alone averaged increases of 2.8-fold for IFN- γ, 2.7-fold for TNF, 1.7-fold for IL-2, and 1.4-fold for GM-CSF;
Combining a GPR174 inhibitor with A2A and/or A2B inhibition increased the average IFN-γ and TNF levels 8- to 9-fold and IL-2 and GM-CSF levels nearly 3-fold, with maximum increases reaching 25-fold for IFN- γ and TNF and over 4-fold for IL-2 and GM-CSF
The adenosine pathway has been a focus of immunotherapy drug development efforts across the pharmaceutical industry (see Table 15). The results described herein now shown that a relatively modest boost in cytokine production by T cells observed upon inhibition of the A2A or A2B receptors singly or both together can be prominently augmented when combined with inhibition of the GPR174 pathway. Furthermore, the discovery that PS itself stimulates an immunosuppressive GPCR - namely GPR174 - on T lymphocytes represents a significant advancement in our understanding of how PS may influence tumor immunity.

These findings are also particularly relevant for patients resistant to checkpoint inhibitors, such as anti-PD-1 (*e.g*., Keytruda^{®} and Opdivo^{®}) and anti-CTLA-4 (Yervoy^{®}), and to cellular therapies such at CAR-T cells and adoptive T cell therapy. Checkpoint inhibitors are only effective in a minority of patients, and high levels of adenosine-generating molecules have been observed in non-responding patients. Furthermore, overcoming natural immunosuppression in solid tumors represents a major hurdle for cellular therapies. As PS and adenosine are both products of cell stress and death in solid tumors, it is expected that these patients resistant to checkpoint inhibitors or cellular therapies would benefit greatly from the combined inhibition of the GPR174 and adenosine pathways. Simply put, the inventors have discovered that there are two feet on the cAMP brake pedal restraining tumor immunity and, to enable more effective tumor-killing activity, both GPR174 and the adenosine pathway must be inhibited, as illustrated in FIGURE 58.

**Table 15: Adenosine Pathway Inhibitors in Cancer Immunotherapy Trials¹**

| Drug/Drug Candidate | Target | Stage | Design | Indication | Sponsor |
|---|---|---|---|---|---|
| AZD4635 | A2A | Ph 2 | Single agent, combo w/ Imfinzi^{®} (anti-PD-L1) | Advanced solid malignancies | AstraZeneca |
| CPI-445 | A2A | Ph1b/2 | Single agent, combo w/ Tecentriq^{®} (anti-PD-L1) | Non-small-cell lung cancer (NSCLC) | Hoffman-La Roche |
| NIR178 | A2A | Ph 1b | Combo w/ Keytruda^{®} and LAG525 (anti-LAG3) | Triple-negative breast cancer | Novartis |
| NIR178 | A2A | Ph 2 | Single agent, combo w/ Keytruda^{®} | Advanced solid malignancies | Novartis |
| NIR178 | A2A | Ph 1/1b | Single agent, combo w/ Keytruda^{®} | Non-small-cell lung cancer (NSCLC) | Palobiofarma |
| CPI-444 | A2A | Ph 1/1b | Single agent, combo w/ Tecentriq^{®} | Renal cell carcinoma; NSCLC | Corvus Pharmaceuticals |
| EOS100850 | A2A | Ph 1/1b | Single agent | Solid tumors | iTeos Therapeutics |
| PBF-1129 | A2B | Ph 1 | Single agent | Non-small-cell lung carcinoma (NSCLC) | Palobiofarma |
| CPI-444 | A2A/A2B | Ph 1/1b | Single agent, combo w/ Keytruda^{®} or Tecentriq^{®} | Solid tumors | Corvus Pharmaceuticals |
| Daratumumab | CD38 | Ph 1 | Single agent | Renal carcinoma, bladder cancer | M.D. Anderson |
| TTX-030 | CD39 | Ph 1/1b | Single agent, combo w/ Keytruda^{®} and chemotherapies | Solid tumor, lymphoma | Tizona Therapeutics/ AbbVie |
| Oleclumab | CD73 | Ph 1 | Single agent, combo w/ Imfinzi^{®} (anti-PD-L1) | Advanced solid malignancies | AstraZeneca |
| Oleclumab | CD73 | Ph 1 | Single agent | Advanced solid malignancies | AstraZeneca |
| Oleclumab | CD73 | Ph 1 | Combo w/ Imfinz^{®} (anti-PD-L1) | Muscle-invasive bladder cancer | Dana-Farber Cancer Institute |
| Oleclumab | CD73 | Ph 2 | Combo w/ Imfinzi^{®} (anti-PD-L1) | Relapsed ovarian cancer | Nordic Society of Gynecologic Oncology |
| Oleclumab | CD73 | Ph 2 | Combo w/ Imfinzi^{®} (anti-PD-L1) | PD-1/PD-L1 inhibition-resistant NSCLC | AstraZeneca |
| Oleclumab | CD73 | Ph 1b/2 | Combo w/ Imfinzi^{®} (anti-PD-L1) and chemotherapy | Triple negative breast cancer | AstraZeneca |
| Oleclumab | CD73 | Ph 1b/2 | Combo w/ chemo, combo w/ chemo and Imfinzi^{®} | Metastatic pancreatic cancer | AstraZeneca |
| Oleclumab | CD73 | Ph 1b/2 | Combo w/ AZD4635 (A2Ai), combo w/ osimertinib (EGFR^T790M inhibitor) | Advanced NSCLC | AstraZeneca |
| BMS-986179 | CD73 | Ph 1/2a | Single agent, combo w/ Opdivo^{®} (anti-PD-1) | Advanced solid malignancies | Bristol-Myers Squibb |
| CPI-006 | CD73 | Ph 1/1b | Single agent, combo w/ CPI-444 (A2Ai), combo w Keytruda^{®} | Advanced solid malignancies | Corvus Pharmaceuticals |
| NZV930 | CD73 | Ph 1/1b | Single agent, combo w/ Keytruda^{®}, combo w/ NIR178 (A2Ai) | Advanced solid malignancies | Novartis |

| | | | | | |
|---|---|---|---|---|---|
| ¹Vigano et al., Frontiers in Immunology, vol 10, Article 925, June 2019 | | | | | |

### EXAMPLE 24

This Example describes the effects of GPR174 genetic deficiency on the growth of the CT26.cl25 murine colon carcinoma in mice treated with a regulatory T cell (Treg)-depleting anti-GITR antibody.

Mouse models of tumor growth provide an experimental platform to monitor and manipulate tumor immunity. To evaluate whether a particular gene product is important for directing the nature and intensity of anti-tumor immune responses, a common approach is to inoculate normal or mutant mice lacking the gene of interest with a tumor cell line. The tumor will grow to a volume necessitating euthanasia of the mouse unless an immune response reduces the rate of tumor growth or completely clears the tumor from the mice. In preliminary studies we found that the CT26.cl25 colon carcinoma grew at identical rates in WT mice and mice lacking an intact *GPR174* gene (GPR174-KO mice). Our *in vitro* studies with mouse T cells and mouse splenocytes revealed that GPR174 deficiency or GPR174 inhibition increased IL-2 production more significantly than other cytokines (see Example 7). Because IL-2 is a growth factor for both immunosuppressive Treg and tumor killing Th1 and CD8 T cells, we reasoned that elevated IL-2 in tumor-bearing mice would stimulate both tumor-promoting Treg and tumor-killing Th1 and CD8 T cells, resulting in Treg-mediated neutralization of anti-tumor responses.

Regarding the balance in IL-2 consumption between Treg and Th1/CD8 T cells, there is a fundamental difference between humans and mice that should be taken into account while leveraging and interpreting mouse models of human disease. In unmanipulated laboratory mice, including mice that have just received a tumor cell inoculum, the vast majority of T cells that express the high-affinity IL-2 receptor, CD25, are Treg, with CD25 expression levels that are markedly higher than CD25 on other CD4 T cells. In contrast, CD25 distribution between Treg and other CD4 T cells in humans is reversed, where the majority of CD4 T cells expressing CD25 are effector T cells that express CD25 at levels that largely overlap with Treg (see Churlaud G. et al., Frontiers in Immunology vol 6, article 171, 2015; Akimova T. et al., PLoS ONE, Vol 6, Issue 8, 2011). Effector T cells respond to IL-2 in a proinflammatory manner, including the production of IFN-γ, an essential cytokine for anti-tumor immunity. Thus, in mice, elevated IL-2 levels due to GPR174 deficiency will largely benefit Treg, while in humans, elevated IL-2 following GPR174 inhibitor administration has a greater potential to benefit tumoricidal effector cells, including Th1 cells, CD8 T cells, and NK cells.

To allow for the presumed elevated IL-2 in the GPR174-KO mice to preferentially act on tumoricidal immune cells, we opted to treat mice with an anti-GITR (glucocorticoid-induced TNFR-related gene) antibody (DTA-1), which is known to deplete Treg cells and stimulate anti-tumor responses in other T cells (see Coe D. et al., Cancer Immunol Immunother 59(9):1367-77, 2010). Complete Treg cell depletion with high anti-GITR antibody doses is known to activate potent anti-tumor responses in WT mice, a setting in which we may not observe a difference between WT and GPR174-KO mice. In contrast, partial or transient Treg cell depletion promotes partial anti-tumor immunity that can be enhanced with other immune-stimulating therapies (see Zapposodi R. et al., Nat Med 25(5):759-766, 2019). Because partial Treg depletion allows for IL-2 to be consumed more efficiently by tumoricidal T cells, this approach can be considered as a form of "humanization" of the mouse tumor model. Thus, we compared the growth of a tumor cell line in WT or GPR174-KO mice that were treated with a low dose of anti-GITR DTA-1 antibody on only 2 to 3 select days following tumor inoculation.

### Methods

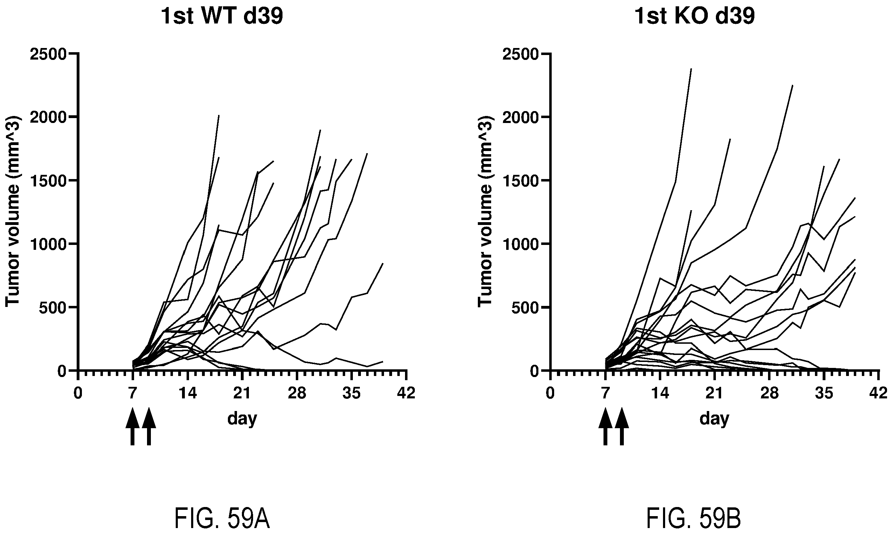

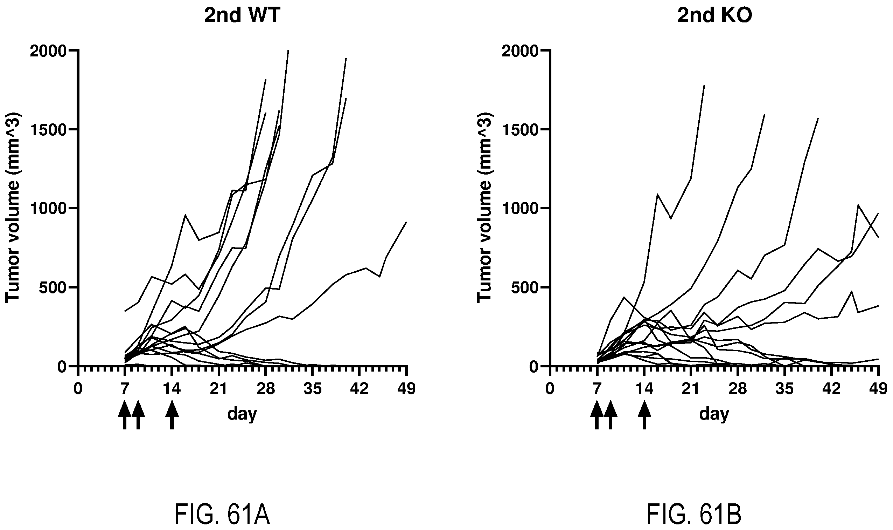

The CT26.cl25 colon carcinoma is of the BALB/c mouse strain. GPR174-KO mice were generated as described in Example 7. Because GPR174 is on the X-chromosome, we were able to use WT and GPR174-KO male mice with a BALB/c-tolerant immune system by crossing 129S GPR174-KO females with BALB/c males. WT or GPR174-KO (n=15) [BALB/c x 129S] F1 male littermates were inoculated subcutaneously with 500,000 CT26.cl25 cells on day 0. In a first experiment, on day 7 and 9 (FIGS 59A and B), or in a second experiment on day 7, 9 and 14 (FIGS 61A and B), all mice received intraperitoneal injections of 200 µg anti-GITR antibody (clone DTA-1; Bio-X-Cell). Tumor volume was calculated by the equation (length x width²)/2), and mice with tumors measuring >1500 mm³ were euthanized.

### Results

FIGURE 59A and FIGURE 59B graphically illustrate the growth of CT26 tumors in individual WT (FIG 59A) and GPR174-KO (FIG 59B) mice inoculated with CT26.cl25 murine colon carcinoma cells on day 0 and treated with anti-GITR antibody on day 7 and 9.

As shown in FIGURE 59B, tumor growth on average was delayed in GPR174-KO mice relative to WT mice (FIG 59A).

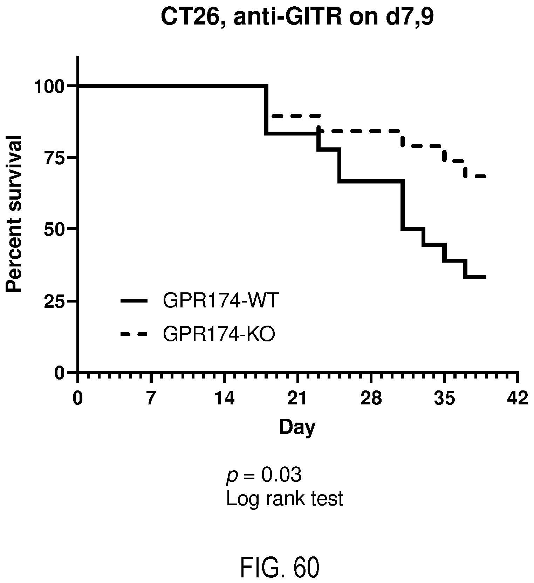

FIGURE 60 graphically illustrates the percent survival of tumor-bearing WT and GPR174-KO mice inoculated with CT26.cl25 murine colon carcinoma cells on day 0 and treated with anti-GITR antibody on day 7 and 9. As shown in FIGURE 60, GPR174-KO mice had a higher percent survival (*i.e.*, were euthanized significantly later) than WT mice (*p* = 0.03, log-rank test).

FIGURE 61A and FIGURE 61B graphically illustrate the growth of tumors in individual WT (FIG 61A) and GPR174-KO mice (FIG 61B) inoculated with CT26.cl25 murine colon carcinoma cells on day 0 and treated with anti-GITR antibody on day 7, 9, and 14.

As shown in FIGURE 61B, tumor growth on average was delayed in GPR174-KO mice relative to WT mice (FIGURE 61A).

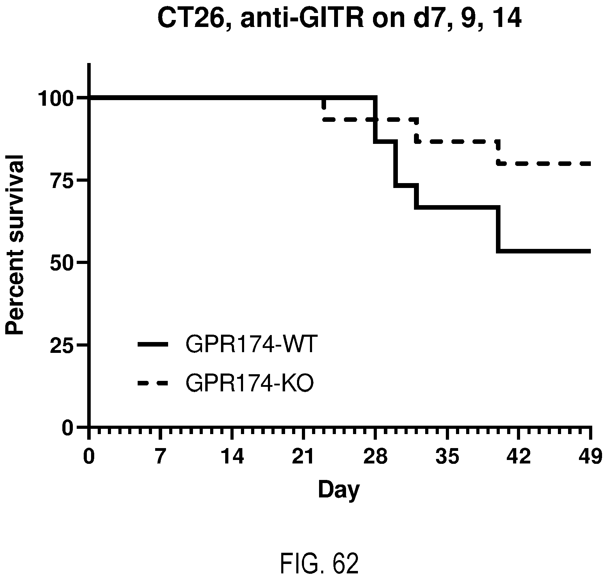

FIGURE 62 graphically illustrates the percent survival of tumor-bearing WT and GPR174-KO mice inoculated with CT26.cl25 murine colon carcinoma cells on day 0 and treated with anti-GITR antibody on days 7, 9 and 14.

As shown in FIGURE 62, GPR174-KO mice had a higher percent survival (*i.e.,* were euthanized significantly later) than WT mice.

### Discussion

In these experiments, our aim was to determine whether GPR174-deficient mice mounted more aggressive anti-tumor immune responses when Treg cells were partially depleted with the anti-GITR DTA-1 antibody. As shown in FIGURES 59A to 62, CT26.cl25 murine colon carcinoma tumor growth was delayed in GPR174-KO mice relative to WT littermates, and survival was significantly improved.

### EXAMPLE 25

This Example described the effects of GPR174 genetic deficiency on the growth of the B16F10 melanoma in mice treated with a regulatory T cell (Treg)-depleting anti-GITR antibody.

In the B16F10 melanoma tumor model, it was previously observed that a single high dose of anti-GITR antibody administered on day 4 resulted in delayed tumor growth but not complete tumor rejection in all mice (Zapposodi R. et al., Nature Medicine 25:759-766, 2019). In view of the results described in Example 24, the following study was carried out to determine whether transient Treg depletion with anti-GITR antibody would delay tumor growth and improve survival of GPR174-KO mice relative to WT mice following inoculation with a more aggressive tumor: B16F10 melanoma.

The B16F10 melanoma is of the C57BL/6 mouse strain. GPR174-KO mice were generated as described in Example 7. Because GPR174 is on the X-chromosome, we were able to use WT and GPR174-KO male mice with a C57BL/6-tolerant immune system by crossing 129S GPR174-KO females with C57BL/6 males. WT or GPR174-KO (n=12) [C57BL/6 x 129S] F1 male littermates were inoculated intradermally with 75,000 B16F10-Kb cells (B16F 10 cells transfected with H-2Kb to increase antigen presentation to CD8 T cells) on day 0. (see Ohta A. et al., PNAS 103(35):13132-13137, 2006). On days 4 and 14, all mice received intraperitoneal injections of 500 µg anti-GITR antibody (clone DTA-1; Bio-X-Cell). Tumor volume was calculated by the equation (length x width²)/2), and mice with tumors measuring >1500 mm³ were euthanized.

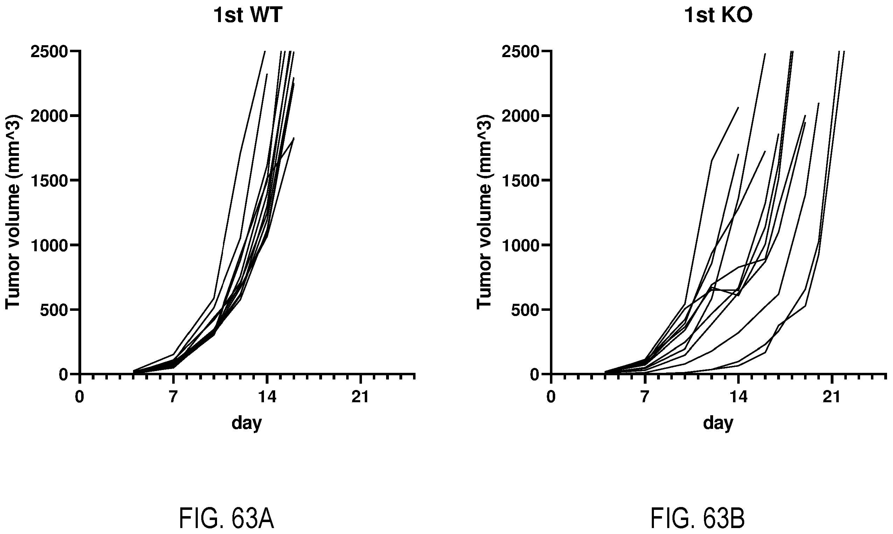

FIGURE 63A and FIGURE 63B graphically illustrate the growth of B16F10-Kb melanoma tumors in individual WT (FIG 63A) and GPR174-KO mice (FIG 63B) inoculated with B16F10-Kb cells on day 0 and treated with anti-GITR antibody on day 4 and 14.

As shown in FIGURE 63B, GPR174-deficiency delayed tumor growth as compared to tumor growth in WT mice (FIGURE 63A). Average tumor sizes were significantly smaller in KO mice on days 14 (p=0.01) and 16 (p=0.00005).

FIGURE 63C graphically illustrates the average tumor volume of B16F10-Kb melanoma tumors in WT and GPR174-KO mice inoculated with B16F10-Kb cells on day 0 and treated with anti-GITR antibody on day 4 and 14. As shown in FIGURE 63C, average tumor sizes were significantly smaller in GPR174 KO mice on days 14 ("*" indicates: p=0.01) and 16 ("***" indicates: p=0.00005).

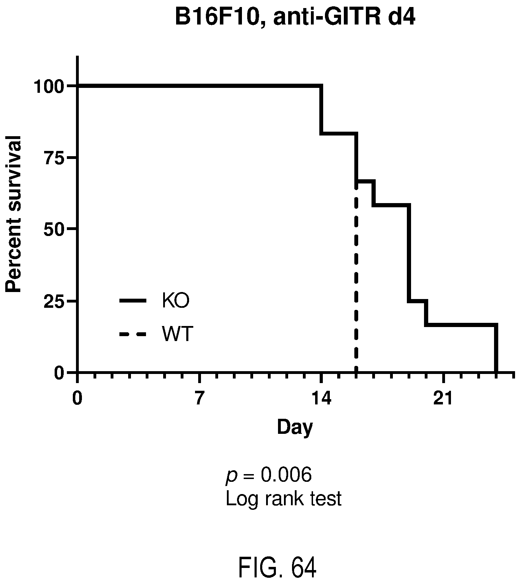

FIGURE 64 graphically illustrates the percent survival of B16F10-Kb melanoma tumor-bearing WT and GPR174-KO mice inoculated with B16F10-Bb cells on day 0 and treated with anti-GITR antibody on day 4 and 14. As shown in FIGURE 64, GPR174-KO mice had a higher percent survival (*i.e.*, were euthanized significantly later) than WT mice (p = 0.006, log-rank test).

### Discussion

As demonstrated in this Example, in the B16F10 melanoma model, GPR174-deficiency resulted in significant attenuation of tumor growth and prolonged survival following anti-GITR antibody treatment. Because Treg-attenuating therapies (*i.e.*, agents that deplete, attenuate or otherwise impair Treg cells) are currently under development as cancer immunotherapies, our data suggest that a GPR174 inhibitor may be more efficacious in the presence of such a co-therapy. Drugs that attenuate Treg or simultaneously attenuate Treg and enhance the tumor-killing activity of other T cells and/or NK cells include antibodies or other molecules that engage one or more of the following: GITR, CTLA-4, CD25, LAG3, TIGIT, NRP1, TGF-β, CCR2, CCR4, CCR8, TNFR2 and/or EZH2, such as described for example, in Tanaka A and Sakaguchi S, European Journal of Immunology vol 49(8):1140-1146, 2019; Yano H. et al., Immunology vol 157(3): 232-247, 2019; Ohue Y and Nishikawa H., Cancer Science vol 110(7):2080-2089, 2019; Han S. et al., Front Oncol vol 9, Article 279, 2019.

Accordingly, in one case of the disclosure, a GPR174 inhibitory agent is used in combination with one or more Treg attenuating agent(s) (*i.e.*, agents that deplete, attenuate or otherwise impair the tumor suppressor function of Treg cells). In some cases, the Treg attenuating agent binds to one or more of GITR, CTLA-4, CD25, LAG3, TIGIT, NRP1, TGF-β, CCR2, CCR4, CCR8, TNFR2 and/or EZH2.

Exemplary Treg attenuating agents for use in combination with GPR174 inhibitors in the compositions, methods and uses described herein are provided below:
GITR binding agents binding agents that can be used in combination with GPR174 inhibitors in the compositions, methods and uses described herein include anti-GITR antibodies and small molecules that bind GITR, such as, for example: TRX518, DTA-1 and MK-4166.

CTLA-4 (cytotoxic T-lymphocyte-associated protein 4) binding agents that can be used in combination with GPR174 inhibitors in the compositions, methods and uses described herein include anti-CTLA-4 antibodies and small molecules that bind CTLA-4, such as, for example, Ipilimumab (Bristol-Myers Squibb); Tremelimumab (MedImmune); AGEN1884 (Agenus); BCD-145 (Biocad); REGN4659 (Regeneron Pharmaceuticals); ADU-1604 (Aduro Biotech); and CS1002 (CStone Pharmaceuticals).

CD25 binding agents that can be used in combination with GPR174 inhibitors in the compositions, methods and uses described herein include anti-CD25 antibodies and small molecules that bind CD25, such as, for example, daclizumab; Basiliximab and CD25-targeted NIR-PIT.

TIGIT (T cell immunoreceptor with Ig and ITIM domains) binding agents that can be used in combination with GPR174 inhibitors in the compositions, methods and uses described herein include anti-TIGIT antibodies and small molecules that bind TIGIT such as, for example, MK-7684 (Merck Sharp & Dohme); AB154 (Arcus Biosciences); MTIG7192A (Genentech); BMS-986207 (Bristol-Myers Squibb); and ASP8374 (Astellas Pharma).

NRP1 (Neuropilin 1) binding agents that can be used in combination with GPR174 inhibitors in the compositions, methods and uses described herein include anti-NRP1 antibodies and small molecules that bind NRP1 such as, for example ASP1948 (Astellas Pharma).

TGF-β binding agents that can be used in combination with GPR174 inhibitors in the compositions, methods and uses described herein include anti-TGF-β antibodies and small molecules that bind TGF-β such as, for example Galunisertib (Eli Lilly); LY320082 (Eli Lilly); PF-06952229 (Pfizer) and Fresolimumab (Cambridge Antibody Technology).

LAG-3 binding agents that can be used in combination with GPR174 inhibitors in the compositions, methods and uses described herein include anti-LAG-3 antibodies and small molecules that bind LAG-3 such as, for example Sym022 (Symphogen); Relatlimab (Bristol-Myers Squibb); REGN3767 (Regeneron Pharmaceuticals); TSR-033 (Tesaro); IMP321 (Immutep); INCAGN02385 (Agenus); LAG525 (Novartis); and MK4280 (Merck Sharp & Dohme).

CCR2 binding agents that can be used in combination with GPR174 inhibitors in the compositions, methods and uses described herein include anti-CCR2 antibodies and small molecules that bind CCR2 such as, for example BMS-813160 (Bristol-Myers Squibb), MLN1202 (Millennium Pharmaceuticals), C775, STI-B0201, STI-B0211, STI-B0221, STI-B0232, carlumab (CNTO 888; Centocor, Inc.), or STI-B0234.

CCR4 binding agents that can be used in combination with GPR174 inhibitors in the compositions, methods and uses described herein include anti-CCR4 antibodies and small molecules that bind CCR4 such as, for example Mogamulizumab (Kyowa Kirin), N-[(3-{[3-{[(5-Chloro-2-thienyl)sulfonyl]amino}-4-(methyloxy)-1H-indazol-1-yl]methyl}phenyl)methyl]-2-hydroxy-2-methylpropanamide which are described in WIPO international publication WO2010/097395 and US Patent Publication No. 20100216860.

CCR8 binding agents that can be used in combination with GPR174 inhibitors in the compositions, methods and uses described herein include anti-CCR8 antibodies and small molecules that bind CCR8 such as, for example in US10,087,259.

TNFR2 binding agents that can be used in combination with GPR174 inhibitors in the compositions, methods and uses described herein include anti-TNFR2 antibodies and small molecules that bind TNFR2 such as, for example in Vanamee E and Faustman D, Trends in Molecular Medicine vol 23, Issue 11:1037-1046, 2017.

EZH2 (Enhancer of zeste homolog 2) binding agents that can be used in combination with GPR174 inhibitors in the compositions, methods and uses described herein include anti-EZH2 antibodies and small molecules that bind EZH2 such as, for example UNC1999, 3-Deazaneplanocin A (DZNcp), EI1, EPZ-5676, EPZ-6438, GSK343, EPZ005687, EPZ011989 and GSK126.

Furthermore, considering our findings with combined A2aR/A2bR and GPR174 inhibition as described in Examples 15 and 18-23, an optimal approach for cancer patients may be to inhibit both the adenosine pathway and GPR174 in the presence of one or more Treg-attenuating agents. Such an approach would ensure that A2aR/A2bR/GPR174 inhibitor-induced IL-2 would act more on tumor-reactive effector T cells than on Treg.

### EXAMPLE 26

This Example describes the effects of PS liposomes and a GPR174 inhibitor on responses of human and mouse T cells in isolation rather than in the presence of total PBMC or splenocytes.

In the Examples presented above, inhibition of GPR174 was performed in cultures of PBMC or mouse splenocytes where cells were plated at a high density (1 million per well of a 96-well flat-bottom plate). In these conditions, PS was considered to be abundant because the GPR174 inhibitors were active in the absence of additional PS liposomes and because supplementation of 1 µM PS in the form of liposomes did not dramatically alter T cell responses or responses to a GPR174 inhibitor. In contrast, in cultures of low numbers of purified T cells, GPR174 may not be exposed to PS to the same degree. Thus, as described in this Example, we explored whether PS liposomes suppressed human and mouse purified T cell responses and whether a GPR174 inhibitor could reverse that suppression. Furthermore, we evaluated the specificity of these effects by comparing responses in WT and GPR174-KO mouse T cells.

In addition to PS liposomes, we tested tumor exosomes for their ability to stimulate GPR174. Tumor exosomes (also referred to as cancer cell-derived exosomes) are small vesicles that are released in high quantities from tumor cells and which are rich in exposed PS (see Kelleher R.J. et al., Cancer Immunology Research Vol 3(11):1269-78, 2015; Birge R.B. et al., Cell Death & Differentiation Vol 23:962-978, 2016). Tumor exosomes are thought to play a role in cancer-related immunosuppression, thus, we hypothesized that they could suppress anti-tumor immunity in part through their activity on GPR174.

Purified human T cells (Astarte Biologics, Inc) were activated in the presence of K562 cells (as artificial antigen presenting cells) presenting anti-CD3 and CD80.Fc via Fc-receptor binding, in 96-well U-bottom plates containing X-vivo-15 media in the following conditions:
50,000 T cells per well
10,000 K562 cells per well
Anti-CD3 (clone UCHT1; 0.5 µg/mL)
CD80.Fc fusion protein (R&D Systems; 2 µg/mL)
PS liposomes (1 µM)
Exosomes from human breast cancer tumor cell line MDA-MB-231 (12 µg/mL) (EXOP-105A-1 from System Biosciences, LLC)
GPR174 inhibitor (compound 10, 0.5 µM or 3 µM)
Cells were cultured for 24 hours and supernatants were evaluated for cytokine concentrations (Legendplex, Biolegend).

### Preparation of purified mouse T cells:

Mouse T cells (Stemcell untouched pan T cell isolation kit) from WT or GPR174-KO mice were cultured in X-vivo-15 media in 96-well flat bottom plates previously coated with 50 µg/mL goat-anti-hamster IgG (Jackson Immunochemicals) in the following conditions:
100,000 T cells per well
Anti-CD3 (clone 2C11; 0.01 µg/mL)
Anti-CD28 (clone 37.51; 0.1 µg/mL)
PS liposomes (1 µM)
GPR174 inhibitor (compound 10, 1 µM)
Cells were cultured for 24 hours and supernatants were evaluated for cytokine concentrations (LegendPlex, Biolegend).

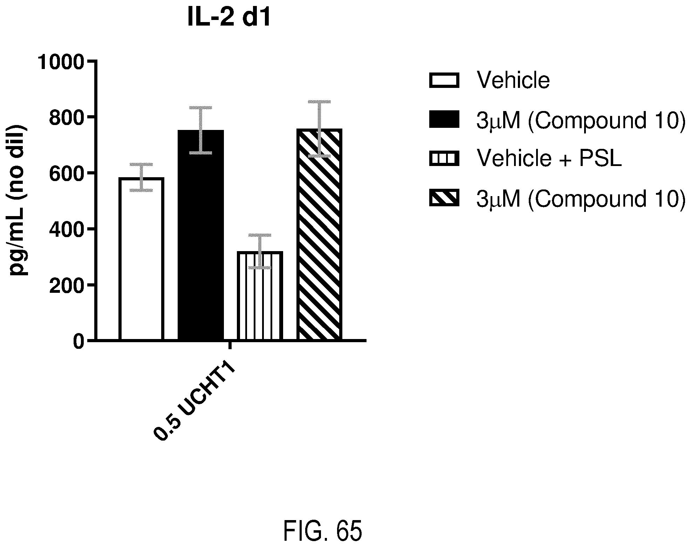

FIGURE 65 graphically illustrates IL-2 concentrations in cultures of stimulated human T cells in the presence or absence of PS liposomes (PSL) or a GPR174 inhibitor (compound 10, 3 µM).

As shown in FIGURE 65, PS liposomes suppress IL-2 production (0.55-fold, p=4 × 10⁻⁴), while GPR174 inhibitory compound 10 reverses this suppression (2.4-fold, p=3 × 10⁻⁴). In contrast, in the absence of PS liposomes, compound 10 only increases IL-2 production 1.3-fold (p=0.01).

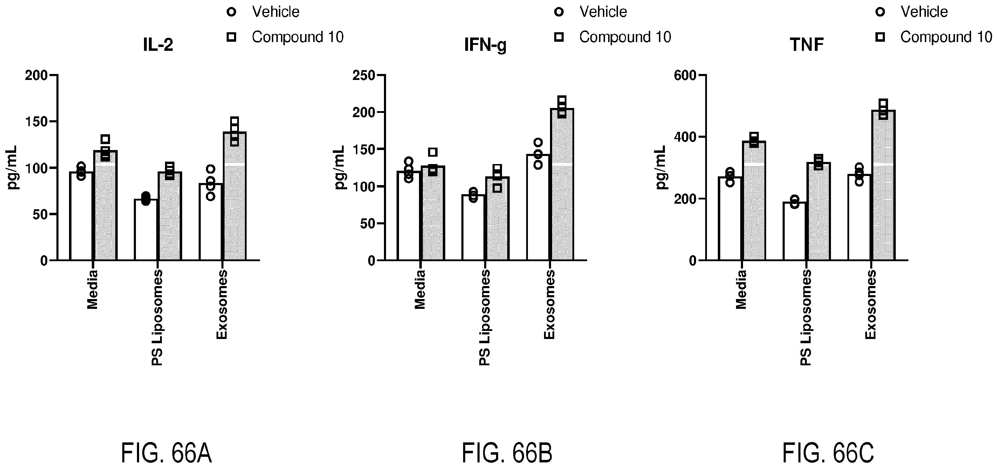

FIGURES 66A, B and C graphically illustrate the concentration of IL-2 (FIG 66A), IFN-γ (FIG 66B) and TNF (FIG 66C) in cultures of stimulated human T cells in the presence or absence of PS liposomes, tumor exosomes, or a GPR174 inhibitory compound (compound 10, 0.5 µM). As shown in FIGURES 66A, B and C, human breast cancer tumor-derived exosomes were more effective than PS liposomes at eliciting cytokine-inducing responses through GPR174 inhibition. Increases of IL-2 (FIG 66A), IFN-γ (FIG 66B) and TNF (FIG 66C) in the presence of media, PS liposomes, or exosomes are summarized below in Table 16. Although exosomes did not suppress cytokine production as did PS liposomes, exosomes may also possess immunostimulatory proteins that counteract their PS-mediated suppression. Thus, the suppressive effects of exosome-associated PS and the hypothesized stimulatory effects of other exosome-associated features together result in negligible net changes in cytokine production, and the addition of a GPR174 inhibitor neutralizes the effects of PS, resulting in a net increase in cytokine production above that which is observed without exosomes.

**Table 16: Summary of IL-2, IFN-γ and TNF production in the presence of media, PS liposomes, or exosomes as shown in FIGS 66A-C**

| Compound 10 vs Vehicle, in the presence of: | IL-2 | | IFN-γ | | TNF | |
|---|---|---|---|---|---|---|
| | FC | *p* value | FC | *p* value | FC | *p* value |
| Media | 1.2 | 3.3E-03 | 1.1 | ns | 1.4 | 1.3E-05 |
| PS Liposomes | 1.4 | 1.6E-05 | 1.3 | 7.3E-03 | 1.7 | 9.0E-07 |
| Exosomes | 1.7 | 4.2E-04 | 1.4 | 1.5E-04 | 1.7 | 3.1E-06 |

| | | | | | | |
|---|---|---|---|---|---|---|
| FC, fold change; p-value, TTEST, 2 tailed, equal variance; ns, not significant | | | | | | |

FIGURE 67 graphically illustrates the IL-2 fold change from vehicle in cultures of stimulated WT or GPR174-KO mouse T cells in the presence or absence of PS liposomes (PSL) or a GPR174 inhibitor (compound 10, 1 µM).

As shown in FIGURE 67, PS liposomes suppress IL-2 production only from GPR174 WT T cells (WT: 0.19-fold, p=8 × 10⁻⁴; KO: 1.8-fold, p=0.04). Furthermore, compound 10 reverses PS liposome-mediated suppression in WT T cells (3.5-fold, p=5 × 10⁻⁴), while having no effect on GPR174-KO T cells (0.9-fold, p=0.6). As noted above, PS liposomes slightly increased IL-2 levels in GPR174-KO T cells. This may be due to PS acting on other lyso-PS receptors that are not coupled to Gαs, such as P2Y10 and GPR34, and which may enhance rather than suppress cytokine production.

The results described in this Example with purified human and mouse T cells establish that PS liposomes suppress T cell responses specifically through GPR174, that GPR174 inhibitory compound 10 reverses this suppression, and that compound 10 has a negligible effect in the absence of PS or GPR174. In addition, the results in this Example indicate that tumor cell-derived exosomes stimulate GPR174, and inhibition of GPR174 in the presence of exosomes enhances Th1 cytokine levels to a greater extent than in the presence of PS liposomes. Thus, reversal of cancer cell-derived exosome-mediated immune suppression with a GPR174 inhibitor may play a role in its anti-tumor efficacy. Furthermore, because tumor exosomes (*i.e.*, cancer cell-derived exosomes) are known to migrate throughout a patient's body and promote tumor metastases (see Wu M et al., Molecular Cancer, 18:53, 2019), a GPR174 inhibitor may amplify anti-tumor immune responses in tumor-draining lymph nodes as well as reduce metastases formation and growth.

### EXAMPLE 27

This Example describes the effects on mouse T cell responses of GPR174 deficiency and GPR174 inhibition when combined with small molecule modulators of A2a/A2b adenosine receptors, employing cultures of total mouse splenocytes.

Both GPR174 and A2a/A2b adenosine receptors signal through the Gαs/cAMP pathway, which suppresses T cell activation. With human PBMC, we observed that inhibition of both axes resulted in synergistic enhancement of Th1 cytokine production as described in Examples 15 and 18-22 herein. As described in this Example, we extended these observations with WT and GPR174-deficient mouse splenocytes. GPR174 and adenosine receptor signaling predominantly suppressed IL-2 production from mouse splenocytes, thus, herein, we present only IL-2 levels for these experiments.

To make single cell suspensions from mouse splenocytes, spleens were cut into 1-2 mm³ pieces with a razor blade and incubated at 37°C with 10 µg/mL DNase and 1 mg/mL Collagenase D with agitation for 90 min. Spleen fragments and cells were then pressed through a nylon screen, and the resulting cell suspension was washed. In 96-well flat-bottom plates, splenocytes were cultured in quadruplicate with the following conditions for 2 days, after which supernatants were tested for cytokine levels with the MSD platform.
1,000,000 splenocytes per well
Anti-CD3 (clone 2C11; 0.1 µg/mL)
Anti-CD28 (clone 37.51; 0.1 µg/mL)
GPR174 inhibitor (compound 10, 1 µM or 0.3 µM, or DMSO vehicle control)
GPR174 inhibitor (compound 6, 0.3 µM, or DMSO vehicle control)
Adenosine receptor agonist (NECA, 0.1 µM)
A2a/A2b adenosine receptor antagonist (ZM-241385, 0.1 µM or 0.2 µM, or DMSO vehicle control)

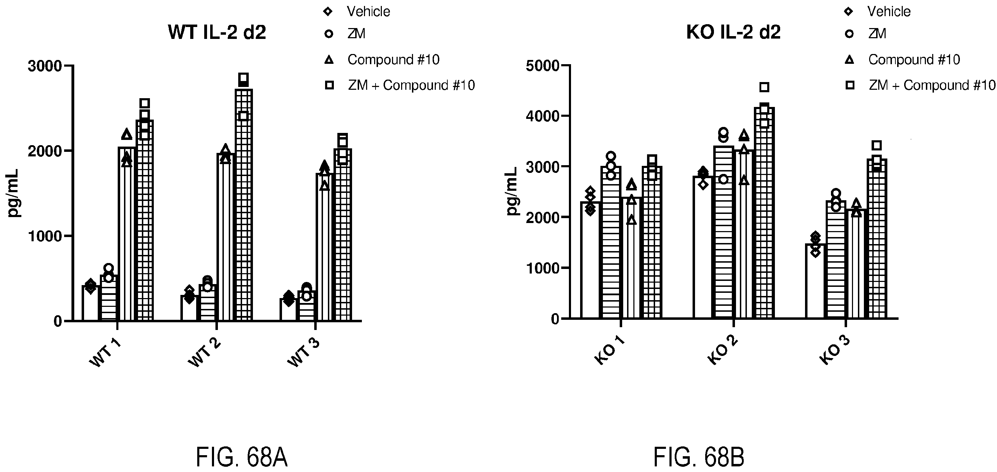

FIGURE 68A and FIGURE 68B graphically illustrate the effects of A2a/A2b adenosine receptor antagonist ZM-241385 (0.1 µM), GPR174 inhibitory Compound 10 (1 µM), or both compounds (ZN-241385 plus compound 10) on IL-2 levels in supernatants of cultured splenocytes isolated from n=3 WT mice (FIG 68A) or n=3 GPR174-KO (FIG 68B) littermate mice following 2 days of stimulation with anti-CD3 and anti-CD28 antibodies.

As shown in FIGURE 68A, Compound 10 increased IL-2 levels greater than 4-fold (p<10⁻⁵) only in WT splenocyte cultures, while vehicle control levels in GPR174-KO cultures (FIGURE 68B) were on average greater than 6-fold higher than those of WT cultures (p<10⁻⁹), demonstrating that this activity of compound 10 is specific for GPR174 and that GPR174 deficiency phenocopies the activity of compound 10. In contrast, ZM-241385 had a modest effect, increasing IL-2 levels an average of 1.3-fold in both WT and GPR174-KO mice (p<0.05). We hypothesized that the poor response to A2a/A2b receptor antagonism was due to low levels of endogenous adenosine in the culture conditions. Thus, as shown below, we repeated this experiment with WT splenocytes in the presence of the adenosine receptor agonist NECA.

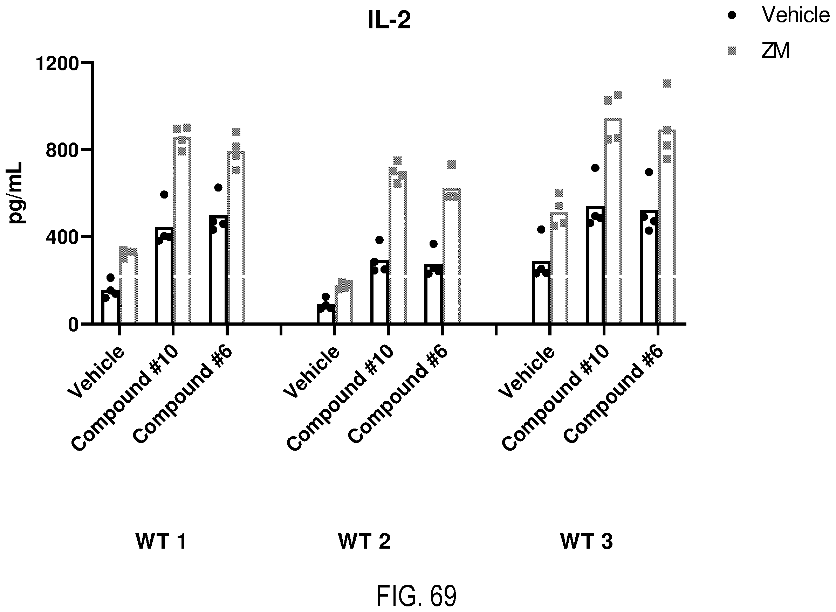

FIGURE 69 graphically illustrates the effects of A2a/A2b adenosine receptor antagonist ZM-241385 (0.2 µM), GPR174 inhibitory compound 10 (0.3 µM) or GPR174 inhibitory compound 6 (0.3 µM), or ZM-241385 plus compound 10 or ZM-241385 plus compound 6 on IL-2 levels in supernatants of cultured splenocytes isolated from 3 WT mice following 2 days of stimulation with anti-CD3 and anti-CD28 antibodies in the presence of the adenosine receptor agonist NECA (0.1 µM).

As shown in FIGURE 69, both GPR174 inhibitory compounds 10 and 6 increased IL-2 levels 2- to 3-fold (p<0.05). When NECA was included in the culture, ZM-241385 increased IL-2 levels on average of 2-fold, both in the absence or presence of GPR174 inhibitory compound 10 or 6 (p<0.01).

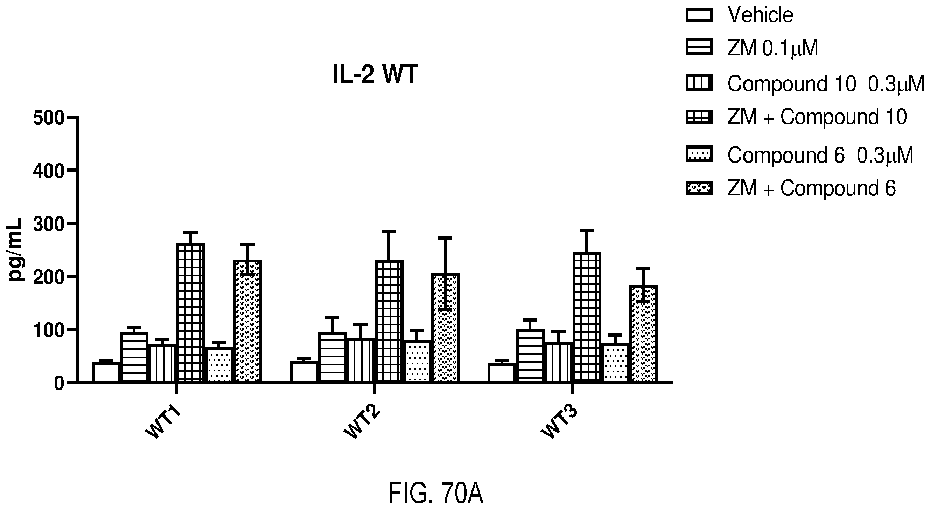

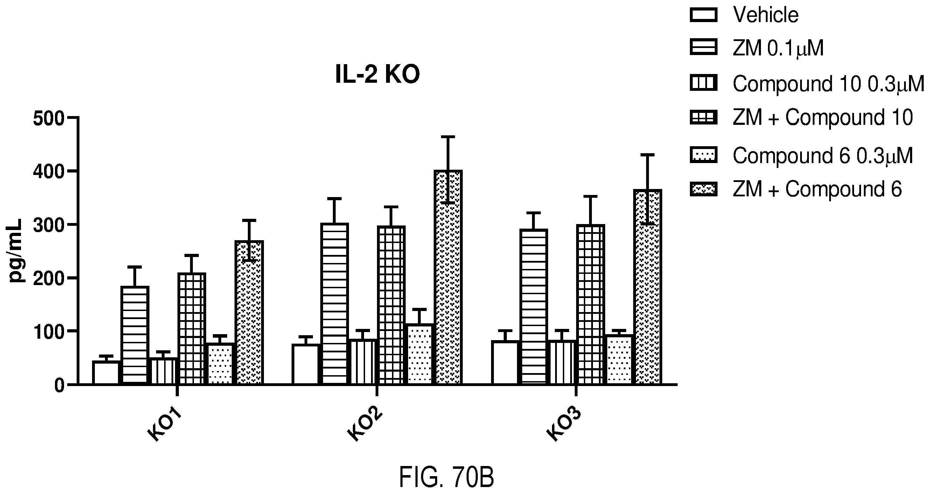

FIGURE 70A and 70B graphically illustrate the effects of A2a/A2b adenosine receptor antagonist ZM-241385 (0.2 µM), GPR174 inhibitory compounds 10 (0.3 µM) or compound 6 (0.3 µM), or ZM-241385 alone or combined with either GPR174 inhibitory compound on IL-2 levels in supernatants of cultured splenocytes isolated from 3 WT mice (FIG 70A) and 3 GPR174-KO mice (FIG 70B) following 2 days of stimulation with anti-CD3 and anti-CD28 antibodies in the presence of the adenosine receptor agonist NECA (0.1 µM).

As shown in FIGURE 70A, in WT splenocytes with ZM-241383 IL-2 levels increased 2.3 to 2.6-fold (p<0.01), and GPR174 inhibitory compounds 10, and 6 increased IL-2 levels 1.7 to 2.0-fold (p<0.02). In contrast, ZM combined with GPR174 inhibitory compound 10 increased IL-2 levels 5.7 to 6.7-fold (p<0.001) and ZM combined with GPR174 inhibitor compound6 increased IL-2 levels 4.9 to 5.9-fold, indicating synergistic enhancement of IL-2 production when both A2aR/A2bR and GPR174 were inhibited in the presence of NECA.

Consistent with this synergy model, as shown in FIGURE 70B, ZM-241385 was more effective with GPR174-KO splenocytes, increasing IL-2 levels 3.5 to 4.1-fold (p<0.001). As expected, GPR174 inhibitory compounds were not active on GPR174-KO splenocytes, with the exception of modest off-target activity with compound 6 in 2 of the 3 splenocyte cultures (KO1 and KO2, p<0.05).

In cultures of mouse splenocytes, both GPR174 inhibition and GPR174 deficiency markedly increased IL-2 production, while GPR174 inhibitors were inactive on GPR174-KO splenocytes. When adenosine receptors were agonized with NECA, inhibition of the two pathways synergized to enhance IL-2 production in that the levels achieved with both inhibitors was greater than the sum of the increases observed with each inhibitor alone. Furthermore, GPR174-deficiency phenocopied the effects of GPR174 inhibitors, both alone and in the presence of the A2aR/A2bR inhibitor, providing further support for the ability of the two pathways to act synergistically to regulate IL-2 production. Because GPR174 inhibitors are reversing the effects of endogenous PS signaling in these splenocyte cultures, small differences in the magnitude of the effects of GPR174 inhibitors may result from inter-experimental differences in PS exposure to GPR174, potentially stemming from small differences in cell compositions and activation states that are difficult to control.

### EXAMPLE 28

This Example describes the combined effects of a GPR174 inhibitor and two adenosine axis inhibitors on induction of IL-2 from cultured human PBMC.

Extracellular adenosine is generated predominantly by enzymes that remove phosphates from extracellular ATP, which is released from activated or dying cells. The ectonucleotidases CD39 and CD73 cleave the first 2 and the 3^{rd} phosphates from ATP, respectively, leaving adenosine to activate adenosine receptors. In this example, we sought to determine if inhibition of CD73 would have the same effect on PBMC cultures as A2aR/A2bR inhibition, and if both CD73 and A2a/A2b inhibition would synergize with GPR174 inhibition in amplifying the T cell response.

Human PBMCs, obtained from a human volunteer donor (donor 8), were dispensed in X-vivo15 media (Lonza) supplemented with Glutamax, penicillin and streptomycin, at density of 1 × 10⁶ cells/well in a 96-well flat-bottom plate and were cultured in quadruplicate for 3 hours in the presence of:
GPR174 inhibitory compound 10 (1 µM);
A2aR/A2bR inhibitory compound ZM-241385 (0.1 µM);
CD73 inhibitory compound: adenosine 5'-(α,β-methylene)diphosphate sodium salt (APCP, Tocris, 10 µM);
Combinations of the above agents;
vehicle control (DMSO).

After which 1 µg/mL anti-CD3 antibody (UCHT1) and 0.1 µg/mL anti-CD28 antibody (CD28.2) were added to activate T cells. Supernatants from these cells were collected at 24 hours post-stimulation and the levels of IL-2 were determined by ELISA.

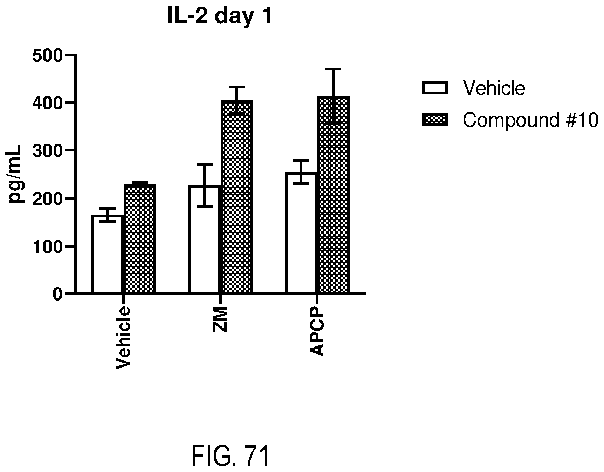

FIGURE 71 graphically illustrates the levels of IL-2 in supernatants of PBMC stimulated with anti-CD3 and anti-CD28 in the presence of the indicated compounds.

As shown in FIGURE 71, GPR174 inhibition with compound 10 increased IL-2 levels 1.4-fold, and adenosine axis inhibition with ZM-241385 or APCP increased IL-2 1.4-and 1.5-fold, respectively (p<0.05 for each). The combination of GPR174 inhibitory compound 10 with either ZM-241385 or APCP increased IL-2 by 2.5-fold (p<0.001).

The results shown in this Example demonstrate that both the A2aR/A2bR inhibitor (ZM-241385) and the CD73 inhibitor (APCP) increased IL-2 levels to the same degree, either alone or in the presence of GPR174 inhibitory compound 10. Furthermore, the degree of IL-2 induction with combined GPR174 and adenosine axis inhibition exceeded the sum of increases obtained with each inhibitor alone, indicating that inhibition of the adenosine axis with either inhibitor synergized with the GPR174 inhibitor to increase IL-2 production.

### EXAMPLE 29

This Example describes suppression of cytokine production from purified human T cells by PS liposomes, adenosine receptor agonist NECA, and both combined.

If, in the tumor microenvironment, immunosuppression from a high degree of exposure to both adenosine and PS cannot be overcome unless both A2aR/A2bR adenosine receptors and GPR174 are inhibited, then it follows that exposure of adenosine analog NECA and PS liposomes together should suppress T cells *in vitro* to a similar extent to the suppression achieved with either inhibitor alone. In this example we test this hypothesis using concentrations of NECA and PS that provide full receptor agonism for their ability to suppress IL-2 production from purified human T cells.

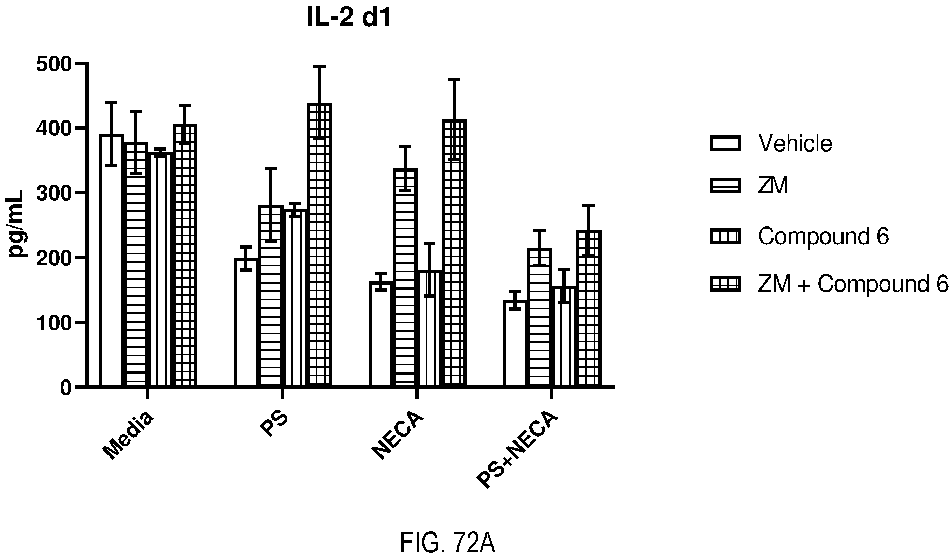

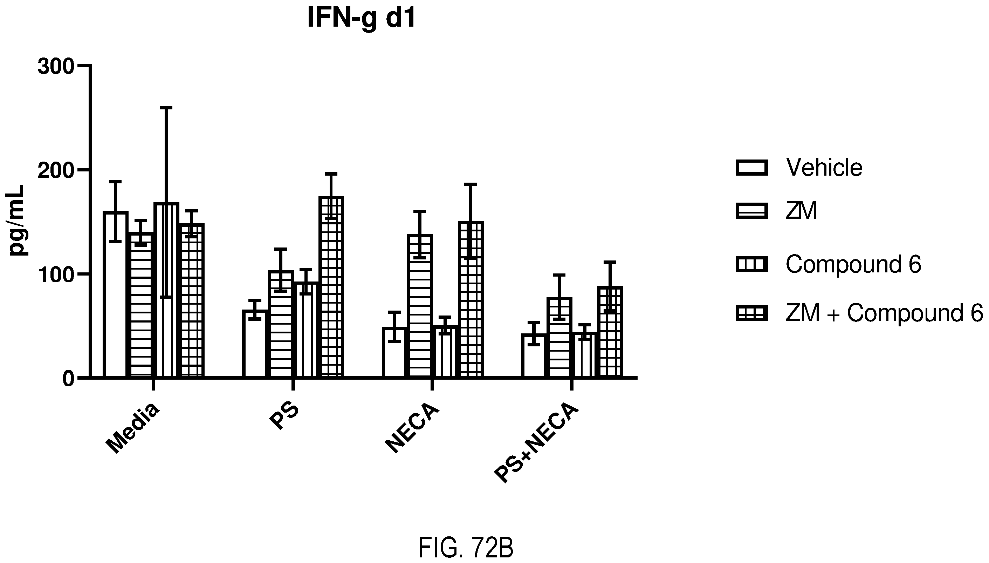

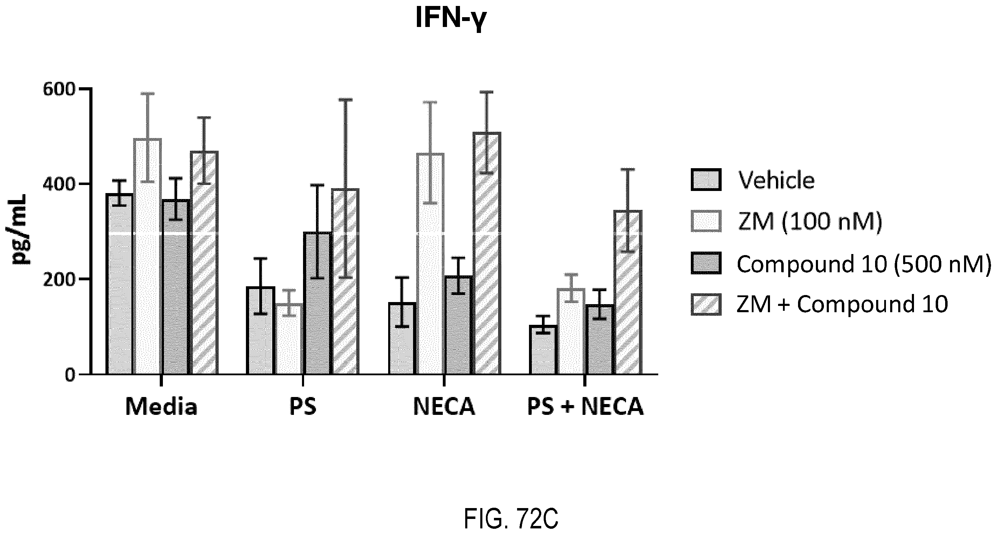

Purified human T cells (untouched total T cells, Astarte Biologics, Inc.; CD3+ T cells in Figures 72A and 72B and CD8+ T cells in FIGURE 72C) were stimulated in quadruplicate at 100,000 cells per well in 96-well flat bottom plates and X-vivo 15 media with the following reagents:
ImmunoCult^{™} Human CD3/CD28 T Cell Activator (StemCell) (1:50 dilution)
PS liposomes (1 µM) or media control
Adenosine receptor agonist (NECA, 0.1 µM or 0.05 µM) or media control
A combination of both reagents.

After 24 hours of culture (44 hours for the results shown in FIGURE 72C), culture media was analyzed for cytokines (MesoScale Discovery).

### Results

FIGURES 72A and 72B graphically illustrate suppression of IL-2 (FIG 72A) and IFN-γ (FIG 72B) production from purified T cells by PS liposomes and NECA, alone or together. For CD8 T cell cultures (FIGURE 72C), the indicated concentrations of ZM-241385 and Compound 10 were included.

As shown in FIGURES 72A, 72B, and 72C, and summarized below in Table 17, PS and NECA both suppress cytokine to nearly the same extent as the suppression achieve with both together.

**Table 17: Summary of IL-2 and IFN-γ production under the various conditions shown in FIGURES 72A and 72B**

| | IL-2 | | IFN-γ | |
|---|---|---|---|---|
| | FC | *p* value | FC | *p* value |
| PS vs Media | 0.51 | 3.0E-04 | 0.41 | 7.8E-04 |
| NECA vs Media | 0.42 | 1.0E-04 | 0.31 | 4.6E-04 |
| PS +NECA vs Media | 0.34 | 5.3E-05 | 0.27 | 2.6E-04 |

| | | | | |
|---|---|---|---|---|
| FC, fold change; p, p-value for TTEST, 2 tailed, equal variance. | | | | |

These findings further support the model that, in the presence of high degrees of GPR174 and A2aR/A2bR agonism, inhibition of both pathways is required to release T cells from cAMP-mediated suppression. This hypothesis is supported by the finding that suppression achieved with NECA or PS liposomes alone was nearly as potent as that observed with both agonists combined. This suggests that both GPR174 and A2aR/A2bR feed the same cAMP compartment; or that their individual cAMP compartments can fully engage cAMP-mediated suppression of cytokine production; and, for this suppression to be effectively neutralized in the presence of PS/lysoPS and adenosine, both GPR174 and A2aR/A2bR must be inhibited.

In summary, as demonstrated herein, GPR174 is an immune system-restricted Gαs-coupled GPCR and PS exposed on liposomes and cellular membranes stimulates GPR174, supporting a model of active GPR174-mediated immune suppression in the tumor microenvironment. Under conditions where both PS/lysoPS and adenosine are present, inhibition of both axes is necessary for effective restoration of T cell function (*e.g.*, it appears that reversal of PS+NECA-mediated suppression of IFN-γ production from human CD8 T cells requires synergistic activity of ZM and Compound 10; see FIGURE 72C). As further demonstrated herein, GPR174-deficiency enhances anti-tumor immune responses in mice.

As described herein, extracellular phosphatidylserine (PS) is a potent modulator of immune responses. Various phospholipid scramblases respond to different cellular processes to expose PS either during apoptosis or during activation of multiple cell types, including platelets, lymphocytes, endothelial cells, and tumor cells. While it is well established that PS exposed during apoptosis suppresses inflammatory responses in phagocytic cells during efferocytosis, whether either form of exposed PS acts directly on T lymphocytes has not been extensively studied. As described herein we show that PS suppresses T cells through GPR174, a Gαs-coupled GPCR previously described as a receptor for lysophosphatidylserine (lysoPS), a soluble catabolite of PS. PS liposomes were found to be 5x more potent than lysoPS in promoting GPR174-dependent cAMP generation, and PS exposed on apoptotic cells, platelets, and activated T cells all induced GPR174 signaling in a reporter cell line. Consistent with the well described immunosuppressive nature of cAMP signaling, PS liposomes suppressed human T cell IL-2 production and mouse IL-2 production from WT but not GPR174-KO T cells. Leveraging a novel GPCR-modulating chemical library screen, we have identified numerous GPR174 inhibitors covering multiple chemical classes, and a GPR174 inhibitor reversed PS liposome-mediated suppression of human and WT mouse T cells while having no effect on GPR174-KO mouse T cells. In a syngeneic mouse tumor model, GPR174-deficiency significantly increased control of tumor growth in the presence of sub-optimal anti-GITR co-therapy. In many respects, GPR174 is similar to the A2A/A2B adenosine receptors in that both suppress T cells through cAMP signaling in response to products of cell stress and death abundant in the tumor microenvironment, and we have found that both pathways work synergistically to restrain T cell responses. In mouse splenocyte cultures containing endogenous levels of adenosine and PS, an A2A inhibitor enhanced T cell responses more effectively in GPR174-knockout cells than WT cells. In similar cultures of human PBMC, GPR174 and A2A/A2B inhibitors, or GPR174 inhibitors and adenosine deaminase, synergistically enhanced IL-2 production. Our findings indicate that inhibition of both GPR174 and the adenosine pathway will be important for effectively overcoming cAMP-mediated immunosuppression in the tumor microenvironment.

Various modifications and variations of the described methods, compositions, and compounds, of the disclosure will be apparent to those skilled in the art.

## Claims

1. A combination of a GPR174 inhibitor and at least one additional agent selected from the group consisting of:
i. an adenosine-A2A (A2A) receptor antagonist;
ii. an adenosine-A2B (A2B) receptor antagonist;
iii. a CD73 inhibitor;
iv. a CD38 inhibitor;
v. a CD39 inhibitor; and
vi. one or more agent selected from the group consisting of an anti-GITR antibody, anti-CTLR-4 antibody, anti-LAG3 antibody, anti-TIGIT antibody, anti-NRP1 antibody, anti-TGF-β antibody, anti-CCR2 antibody, anti-CCR4 antibody, anti-CCR8 antibody, anti-TNFR2 antibody, and an anti-EZH2 antibody,
for use in the treatment of cancer, wherein the GPR174 inhibitor and the at least one additional agent are administered simultaneously, or sequentially in any order, provided that the effects of the first administered inhibitor or antagonist remain present at the time of the second administered inhibitor or antagonist.

2. The combination for use according to claim 1, wherein the additional agent is an anti-GITR, anti-CTLA-4, anti-CD25, anti-LAG3, anti-TIGIT, anti-NRP1, anti-TGF-β, anti-CCR2, anti-CCR4, anti-CCR8, or anti-TNFR2 antibody, or an EZH2 inhibitor.

3. The combination for use according to any one of claims 1-2, wherein the GPR174 inhibitor inhibits PS-dependent or LysoPS-dependent activation of GPR174 signaling in a cell expressing GPR174 by at least 25%.

4. The combination for use according to any one of claims 1-3, wherein the GPR174 inhibitor stimulates immune response in the patient by inhibiting a GPR174 G-alpha-s signaling, and optionally, wherein the stimulated immune response comprises an NK-cell mediated immune response or an anti-tumor cytotoxic T-cell-mediated immune response.

5. An *in vitro*_method of increasing the level of Th1 cytokines in human peripheral blood mononuclear cells (PBMCs), the method comprising contacting *in vitro* human PBMCs with an inhibitor of GPR174 signaling and at least one of an adenosine-A2A (A2A) receptor antagonist, an adenosine-A2B (A2B) receptor antagonist, or a combination thereof.

6. The *in vitro* method of claim 5, wherein the PBMCs comprise T-cells or NK-cells.

7. The *in vitro* method of any one of claims 5-6, wherein the level of at least one of Th1 cytokines IFN-γ, IL-2, TNF, or GM-CSF is increased by at least 20%.

8. A pharmaceutical composition comprising a combination of an inhibitor of GPR174 signaling with at least one of an adenosine-A2A (A2A) receptor antagonist, an adenosine-A2B (A2B) receptor antagonist, a CD73 inhibitor, a CD38 inhibitor, and a CD39 inhibitor and optionally a pharmaceutically acceptable excipient.

9. The pharmaceutical composition of claim 8, wherein the GPR174 inhibitor has a structure according to formula (IV): 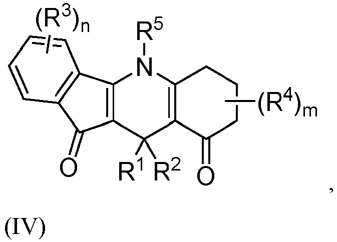
or a stereoisomer thereof, or a tautomer thereof, or a pharmaceutically acceptable salt thereof, wherein
each of R¹ and R² is, independently, H, hydroxy, halo, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfinyl, optionally substituted C₁-C₆ alkylsulfonyl, optionally substituted C₆-C₁₀arylthio, optionally substituted C₆-C₁₀arylsulfinyl, optionally substituted C₆-C₁₀ arylsulfonyl, optionally substituted C₁-C₉ heteroarylthio, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
each of R³ and R⁴ is, independently, H, hydroxy, halo, optionally substituted amino, optionally substituted amido, thiol, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted ester, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ thioalkyl, optionally substituted C₁-C₆ alkylsulfonyl, optionally substituted C₆-C₁₀ arylthio, optionally substituted C₆-C₁₀ arylsulfonyl, optionally substituted C₁-C₉ heteroarylthio, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
R⁵ is H, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, optionally substituted C₁-C₆ alkyloxycarbonyl, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl.
n is 0, 1, 2, 3, or 4; and
m is 0, 1, 2, 3, 4, 5, or 6.

10. The composition of claim 8, wherein the GPR174 inhibitor has a structure according to the following formula (I), 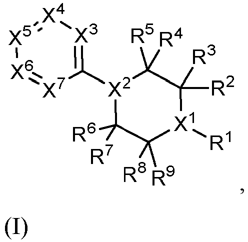
or a stereoisomer thereof, or a pharmaceutically acceptable salt thereof, wherein,
X¹ is N or CR¹⁰;
X² is N or CR¹¹;
X³ is N or CR¹²;
X⁴ is N or CR¹³;
X⁵ is N or CR¹⁴;
X⁶ is N or CR¹⁵;
X⁷ is N or CR¹⁶;
each of R¹, R², R³, R⁴, R⁵, R⁶, R⁷, R⁸, and R⁹ is, independently, H, hydroxy, thiol, optionally substituted amino, optionally substituted amido, cyano, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₉ heterocyclylsulfinyl, optionally substituted C₁-C₉ heterocyclylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl; or
R² and R³ combine to form =O, =S, or =NR¹⁷; or
R⁴ and R⁵ combine to form =O, =S, or =NR¹⁷; or
R⁶ and R⁷ combine to form =O, =S, or =NR¹⁷; or
R⁸ and R⁹ combine to form =O, =S, or =NR¹⁷;
each of R¹⁰, R¹¹, R¹², R¹³, R¹⁴, R¹⁵, and R¹⁶ is, independently, H, hydroxy, halogen, thiol, optionally substituted amino, optionally substituted amido, cyano, nitro, optionally substituted C₁-C₆ alkyl, optionally substituted C₂-C₆ alkenyl, optionally substituted C₂-C₆ alkynyl, optionally substituted C₁-C₆ alkoxy, optionally substituted C₆-C₁₀ aryloxy, optionally substituted C₁-C₉ heteroaryloxy, optionally substituted C₂-C₆ alkanoyl, optionally substituted C₇-C₁₁ aryloyl, optionally substituted C₂-C₁₀ heteroaryloyl, optionally substituted C₂-C₁₀ heterocyclyloyl, hydroxycarbonyl, optionally substituted C₂-C₇ alkoxycarbonyl, optionally substituted carboxamide, optionally substituted C₁-C₆ alkanoyloxy, optionally substituted C₇-C₁₁ aryloyloxy, optionally substituted C₂-C₁₀ heteroaryloyloxy, optionally substituted C₂-C₁₀ heterocyclyloyloxy, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl; or
one of:
(i) R¹² and R¹³, together with the atoms to which each is attached, combine to form an optionally substituted 5-, 6-, or 7-member ring;
(ii) R¹³ and R¹⁴, together with the atoms to which each is attached, combine to form an optionally substituted 5-, 6-, or 7-member ring;
(iii) R¹⁴ and R¹⁵, together with the atoms to which each is attached, combine to form an optionally substituted 5-, 6-, or 7-member ring; and
(iv) R¹⁵ and R¹⁶, together with the atoms to which each is attached, combine to form an optionally substituted 5-, 6-, or 7-member ring;
and
R¹⁷ is H, hydroxyl, cyano, optionally substituted amino, optionally substituted amido, optionally substituted carboxamide, optionally substituted C₁-C₆ alkyl, optionally substituted C₂-C₆ alkenyl, optionally substituted C₂-C₆ alkynyl, optionally substituted C₁-C₆ alkanoyl, optionally substituted C₇-C₁₁ aryloyl, optionally substituted C₂-C₁₀ heterocyclyloyl, optionally substituted C₂-C₁₀ heteroaryloyl, optionally substituted C₁-C₆ alkylsulfinyl, optionally substituted C₁-C₆ alkylsulfonyl, optionally substituted C₆-C₁₀ arylsulfinyl, optionally substituted C₆-C₁₀ arylsulfonyl, optionally substituted C₁-C₉ heteroarylsulfinyl, optionally substituted C₁-C₉ heteroarylsulfonyl, optionally substituted C₁-C₆ heteroalkyl, optionally substituted C₂-C₆ heteroalkenyl, optionally substituted C₂-C₆ heteroalkynyl, optionally substituted C₃-C₁₀ cycloalkyl, optionally substituted C₄-C₁₀ cycloalkenyl, optionally substituted C₈-C₁₀ cycloalkynyl, optionally substituted C₆-C₁₀ aryl, optionally substituted C₆-C₁₀ aryl C₁-C₆ alkyl, optionally substituted C₆-C₁₀ aryl C₂-C₆ alkenyl, optionally substituted C₆-C₁₀ aryl C₂-C₆ alkynyl, optionally substituted C₁-C₉ heteroaryl, optionally substituted C₁-C₉ heteroaryl C₁-C₆ alkyl, optionally substituted C₁-C₉ heteroaryl C₂-C₆ alkenyl, optionally substituted C₁-C₉ heteroaryl C₂-C₆ alkynyl, optionally substituted C₁-C₉ heterocyclyl, optionally substituted C₁-C₉ heterocyclyl C₁-C₆ alkyl, optionally substituted C₁-C₉ heterocyclyl C₂-C₆ alkenyl, or optionally substituted C₁-C₉ heterocyclyl C₂-C₆ alkynyl;
wherein three or fewer of X³, X⁴, X⁵, X⁶, and X⁷ are N; and
at least one of X¹ and X² is N.

11. The composition of claim 8, wherein the composition further comprises one or more agent selected from the group consisting of an anti-GITR antibody, anti-CTLR-4 antibody, anti-LAG3 antibody, anti-TIGIT antibody, anti-NRP1 antibody, anti-TGF-β antibody, anti-CCR2 antibody, anti-CCR4 antibody, anti-CCR8 antibody, anti-TNFR2 antibody, and an anti-EZH2 antibody.

12. A GPR174 inhibitor for use in a method of enhancing an anti-tumor immune response in a subject that is currently undergoing treatment with at least one of an A2aR antagonist, an A2bR antagonist, a CD38 inhibitor, a CD39 inhibitor, a CD73 inhibitor, and an agent selected from the group consisting of an anti-GITR antibody, anti-CTLR-4 antibody, anti-LAG3 antibody, anti-TIGIT antibody, anti-NRP1 antibody, anti-TGF-β antibody, anti-CCR2 antibody, anti-CCR4 antibody, anti-CCR8 antibody, anti-TNFR2 antibody, and an ant-EZH2 antibody.

13. The GPR174 inhibitor for use according to claim 12, wherein the GPR174 inhibitor inhibits PS-dependent or LysoPS-dependent activation of GPR174 signaling in a cell expressing GPR174 by at least 25%.

## Patentansprüche

1. Kombination eines GPR174-Hemmers und mindestens eines zusätzlichen Wirkstoffs, ausgewählt aus der Gruppe bestehend aus:
i. Adenosin-A2A (A2A)-Rezeptorantagonist;
ii. Adenosin-A2B (A2B)-Rezeptorantagonist;
iii. CD73-Hemmer;
iv. CD38-Hemmer;
v. CD39-Hemmer; und
vi. einem oder mehreren Wirkstoffen, ausgewählt aus der Gruppe bestehend aus einem Anti-GITR-Antikörper, Anti-CTLR-4-Antikörper, Anti-LAG3-Antikörper, Anti-TIGIT-Antikörper, Anti-NRP1-Antikörper, Anti-TGF-β Antikörper, Anti-CCR2-Antikörper, Anti-CCR4-Antikörper, Anti-CCR8-Antikörper, Anti-TNFR2-Antikörper und einem Anti-EZH2-Antikörper,
für die Verwendung bei der Behandlung von Krebs, wobei der GPR174-Hemmer und der mindestens eine zusätzliche Wirkstoff gleichzeitig oder in jeglicher Reihenfolge aufeinanderfolgend verabreicht werden, vorausgesetzt, dass die Wirkungen des ersten verabreichten Hemmers oder Antagonisten zum Zeitpunkt des zweiten verabreichten Hemmers oder Antagonisten noch anhalten.

2. Kombination für die Verwendung nach Anspruch 1, wobei der zusätzliche Wirkstoff ein Anti-GITR-, Anti-CTLA-4-, Anti-CD25-, Anti-LAG3-, Anti-TIGIT-, Anti-NRP1, Anti-TGF-β-, Anti-CCR2-, Anti-CCR4-, Anti-CCR8- oder Anti-TNFR2-Antikörper oder ein EZH2-Hemmer ist.

3. Kombination für die Verwendung nach einem der Ansprüche 1 bis 2, wobei der GPR174-Hemmer eine PS-abhängige oder LysoPS-abhängige Aktivierung der GPR174-Signalübertragung in einer GPR174 exprimierenden Zelle um mindestens 25 % hemmt.

4. Kombination für die Verwendung nach einem der Ansprüche 1 bis 3, wobei der GPR174-Hemmer eine Immunantwort im Patienten durch Hemmen einer GPR174 G-alpha-s-Signalübertragung stimuliert, und wobei die stimulierte Immunantwort optional eine NK-Zell-vermittelte Immunantwort oder eine Anti-Tumor-zytotoxische T-Zell-vermittelte Immunantwort umfasst.

5. *In Vitro*-Verfahren zum Erhöhen des Th1-Zytokinspiegels in menschlichen peripheren mononukleären Blutzellen (PBMCs), das Verfahren umfassend das *In Vitro*-Inkontaktbringen menschlicher PBMCs mit einem Hemmer der GPR174-Signalübertragung und mindestens einem aus Adenosin-A2A (A2A)-Rezeptorantagonisten, einem Adenosin-A2B (A2B)-Rezeptorantagonisten oder einer Kombination davon.

6. *In Vitro*-Verfahren nach Anspruch 5, wobei die PBMCs T-Zellen oder NK-Zellen umfassen.

7. *In Vitro*-Verfahren nach einem der Ansprüche 5 bis 6, wobei der Spiegel von mindestens einem aus Th1-Zytokinen IFN-γ, IL-2, TNF oder GM-CSF um mindestens 20 % erhöht wird.

8. Pharmazeutische Zusammensetzung, umfassend eine Kombination eines Hemmers einer GPR174-Signalübertragung mit mindestens einem aus Adenosin-A2A (A2A)-Rezeptorantagonisten, einem Adenosin-A2B (A2B)-Rezeptorantagonisten, einem CD73-Hemmer, einem CD38-Hemmer und einem CD39-Hemmer und optional einem pharmazeutisch zulässigen Hilfsstoff.

9. Pharmazeutische Zusammensetzung nach Anspruch 8, wobei der GPR174-Hemmer eine Struktur nach Formel (IV): 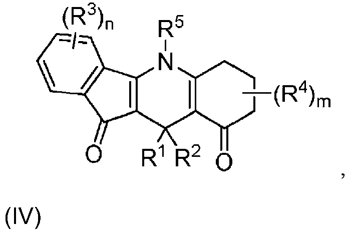
oder ein Stereoisomer davon oder ein Tautomer davon oder ein pharmazeutisch zulässiges Salz davon aufweist, wobei
jedes R¹ und R² unabhängig H, Hydroxy, Halogen, optional substituiertes Amino, optional substituiertes Amido, Thiol, Cyano, optional substituiertes C₁-C₆-Alkyl, optional substituiertes C₂-C₆-Alkenyl, optional substituiertes C₂-C₆-Alkynyl, optional substituiertes C₁-C₆-Alkoxy, optional substituiertes C₆-C₁₀-Aryloxy, optional substituiertes C₁-C₉-Heteroaryloxy, optional substituiertes C₂-C₆-Alkanoyl, optional substituiertes C₇-C₁₁-Aryloyl, optional substituiertes C₂-C₁₀-Heteroaryloyl, optional substituiertes C₂-C₁₀-Heterocyclyloyl, Hydroxycarbonyl, optional substituiertes Ester, optional substituiertes Carboxamid, optional substituiertes C₁-C₆-Alkanoyloxy, optional substituiertes C₇-C₄₁-Aryloyloxy, optional substituiertes C₂-C₁₀-Heteroaryloyloxy, optional substituiertes C₂-C₁₀-Heterocyclyloyloxy, optional substituiertes C₁-C₆-Thioalkyl, optional substituiertes C₁-C₆-Alkylsulfinyl, optional substituiertes C₁-C₆-Alkylsulfonyl, optional substituiertes C₆-C₁₀-Arylthio, optional substituiertes C₆-C₁₀-Arylsulfinyl, optional substituiertes C₆-C₁₀-Arylsulfonyl, optional substituiertes C₁-C₉-Heteroarylthio, optional substituiertes C₁-C₉-Heteroarylsulfinyl, optional substituiertes C₁-C₉-Heteroarylsulfonyl, optional substituiertes C₁-C₉-Heterocyclylsulfinyl, optional substituiertes C₁-C₉-Heterocyclylsulfonyl, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₄₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Akyl, optional substituiertes C₁-C₉-Heterocyclyl C₂-C₆-Alkenyl oder optional substituiertes C₁-C₉-Heterocyclyl C₂-C₆-Alkynyl ist;
jedes R³und R⁴ unabhängig H, Hydroxy, Halogen, optional substituiertes Amino, optional substituiertes Amido, Thiol, Cyano, optional substituiertes C₁-C₆-Alkyl, optional substituiertes C₂-C₆-Alkenyl, optional substituiertes C₂-C₆-Alkynyl, optional substituiertes C₁-C₆-Alkoxy, optional substituiertes C₆-C₁₀-Aryloxy, optional substituiertes C₁-C₉-Heteroaryloxy, optional substituiertes C₂-C₆-Alkanoyl, optional substituiertes C₇-C₁₁-Aryloyl, optional substituiertes C₂-C₁₀-Heteroaryloyl, optional substituiertes C₂-C₁₀-Heterocyclyloyl, Hydroxycarbonyl, optional substituiertes Ester, optional substituiertes Carboxamid, optional substituiertes C₁-C₆-Alkanoyloxy, optional substituiertes C₇-C₁₁-Aryloyloxy, optional substituiertes C₂-C₁₀-Heteroaryloyloxy, optional substituiertes C₂-C₁₀-Heterocyclyloyloxy, optional substituiertes C₁-C₆-Thioalkyl, optional substituiertes C₁-C₆-Alkylsulfonyl, optional substituiertes C₆-C₁₀-Arylthio, optional substituiertes C₆-C₁₀-Arylsulfonyl, optional substituiertes C₁-C₉-Heteroarylthio, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₁₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Alkyl, optional substituiertes C₁-C₉-Heterocyclyl C₂-C₆-Alkenyl oder optional substituiertes C₁-C₉-Heterocyclyl C₂-C₆-Alkynyl ist;
R⁵ H, optional substituiertes C₁-C₆-Alkyl, optional substituiertes C₂-C₆-Alkenyl, optional substituiertes C₂-C₆-Alkynyl, optional substituiertes C₁-C₆-Alkoxy, optional substituiertes C₆-C₁₀-Aryloxy, optional substituiertes C₁-C₉-Heteroaryloxy, optional substituiertes C₂-C₆-Alkanoyl, optional substituiertes C₇-C₁₁-Aryloyl, optional substituiertes C₂-C₁₀-Heteroaryloyl, optional substituiertes C₂-C₁₀-Heterocyclyloyl, optional substituiertes C₁-C₆-Alkyloxycarbonyl, optional substituiertes C₁-C₆-Alkylsulfinyl, optional substituiertes C₁-C₆-Alkylsulfonyl, optional substituiertes C₆-C₁₀-Arylsulfinyl, optional substituiertes C₆-C₁₀-Arylsulfonyl, optional substituiertes C₁-C₉-Heteroarylsulfinyl, optional substituiertes C₁-C₉-Heteroarylsulfonyl, optional substituiertes C₁-C₉-Heterocyclylsulfinyl, optional substituiertes C₁-C₉-Heterocyclylsulfonyl, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₁₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Alkyl, optional substituiertes C₁-C₉-Heterocyclyl C₂-C₆-Alkenyl oder optional substituiertes C₁-C₉-Heterocyclyl C₂-C₆-Alkynyl ist.
n ist 0, 1, 2, 3 oder 4; und
m ist 0, 1, 2, 3, 4, 5 oder 6.

10. Zusammensetzung nach Anspruch 8, wobei der GPR174-Hemmer eine Struktur nach der folgenden Formel (I) aufweist, 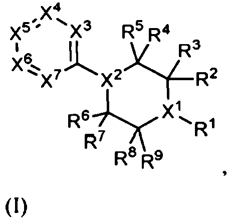
oder ein Stereoisomer davon, oder ein pharmazeutisch zulässiges Salz davon, wobei gilt
X¹ ist N oder CR¹⁰;
X² ist N oder CR¹¹;
X³ ist N oder CR¹²;
X⁴ ist N oder CR¹³;
X⁵ ist N oder CR¹⁴;
X⁶ ist N oder CR¹⁵;
X⁷ ist N oder CR¹⁶;
jedes aus R¹, R², R³, R⁴, R⁵, R⁶, R⁷, R⁸ und R⁹ ist unabhängig H, Hydroxy, Thiol, optional substituiertes Amino, optional substituiertes Amido, Cyano, optional substituiertes C₁-C₆-Alkyl, optional substituiertes C₂-C₆-Alkenyl, optional substituiertes C₂-C₆-Alkynyl, optional substituiertes C₁-C₆ Alkoxy, optional substituiertes C₆-C₁₀-Aryloxy, optional substituiertes C₁-C₉-Heteroaryloxy, optional substituiertes C₂-C₆-Alkanoyl, optional substituiertes C₇-C₁₁-Aryloyl, optional substituiertes C₂-C₁₀-Heteroaryloyl, optional substituiertes C₂-C₁₀-Heterocyclyloyl, Hydroxycarbonyl, optional substituiertes C₂-C₇-Alkoxycarbonyl, optional substituiertes Carboxamid, optional substituiertes C₁-C₆-Alkanoyloxy, optional substituiertes C₇-C₄₁-Aryloyloxy, optional substituiertes C₂-C₁₀-Heteroaryloyloxy, optional substituiertes C₂-C₁₀-Heterocyclyloyloxy, optional substituiertes C₁-C₆-Alkylsulfinyl, optional substituiertes C₁-C₆-Alkylsulfonyl, optional substituiertes C₆-C₁₀-Arylsulfinyl, optional substituiertes C₆-C₁₀-Arylsulfonyl, optional substituiertes C₁-C₉-Heteroarylsulfinyl, optional substituiertes C₁-C₉-Heteroarylsulfonyl, optional substituiertes C₁-C₉-Heterocyclylsulfinyl, optional substituiertes C₁-C₉-Heterocyclylsulfonyl, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₄₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Alkyl, optional substituiertes C₁-C₉-Heterocyclyl C₂-C₆-Alkenyl oder optional substituiertes C₁-C₉-Heterocyclyl C₂-C₆-Alkynyl; oder
R² und R³ werden kombiniert, um =0, =S oder =NR¹⁷ zu bilden; oder
R⁴ und R⁵ werden kombiniert, um =0, =S oder =NR¹⁷ zu bilden; oder
R⁶ und R⁷ werden kombiniert, um =0, =S oder =NR¹⁷ zu bilden; oder
R⁸ und R⁹ werden kombiniert, um =0, =S oder =NR¹⁷ zu bilden;
jedes R¹⁰, R¹¹, R¹², R¹³, R¹⁴, R¹⁵ und R¹⁶ ist unabhängig H, Hydroxy, Halogen, Thiol, optional substituiertes Amino, optional substituiertes Amido, Cyano, Nitro, optional substituiertes C₁-C₆-Alkyl, optional substituiertes C₂-C₆-Alkenyl, optional substituiertes C₂-C₆-Alkynyl, optional substituiertes C₁-C₆-Alkoxy, optional substituiertes C₆-C₁₀-Aryloxy, optional substituiertes C₁-C₉-Heteroaryloxy, optional substituiertes C₂-C₆-Alkanoyl, optional substituiertes C₇-C₄₁-Aryloyl, optional substituiertes C₂-C₁₀-Heteroaryloyl, optional substituiertes C₂-C₁₀-Heterocyclyloyl, Hydroxycarbonyl, optional substituiertes C₂-C₇-Alkoxycarbonyl, optional substituiertes Carboxamid, optional substituiertes C₁-C₆-Alkanoyloxy, optional substituiertes C₇-C₁₁-Aryloyloxy, optional substituiertes C₂-C₁₀-Heteroaryloyloxy, optional substituiertes C₂-C₁₀-Heterocyclyloyloxy, optional substituiertes C₁-C₆-Alkylsulfinyl, optional substituiertes C₁-C₆-Alkylsulfonyl, optional substituiertes C₆-C₁₀-Arylsulfinyl, optional substituiertes C₆-C₁₀-Arylsulfonyl, optional substituiertes C₁-C₉-Heteroarylsulfinyl, optional substituiertes C₁-C₉-Heteroarylsulfonyl, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₁₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Alkyl, optional substituiertes C₁-C₉-Heterocyclyl C₂-C₆-Alkenyl oder optional substituiertes C₁-C₉-Heterocyclyl C₂-C₆-Alkynyl; oder eines aus:
(i) R¹² und R¹³, zusammen mit den Atomen, mit denen sie jeweils verbunden sind, werden kombiniert, um einen optional substituierten 5-, 6- oder 7-gliedrigen Ring zu bilden;
(ii) R¹³ und R¹⁴, zusammen mit den Atomen, mit denen sie jeweils verbunden sind, werden kombiniert, um einen optional substituierten 5-, 6- oder 7-gliedrigen Ring zu bilden;
(iii) R¹⁴ und R¹⁵, zusammen mit den Atomen, mit denen sie jeweils verbunden sind, werden kombiniert, um einen optional substituierten 5-, 6- oder 7-gliedrigen Ring zu bilden; und
(iv) R¹⁵ und R¹⁶, zusammen mit den Atomen, mit denen sie jeweils verbunden sind, werden kombiniert, um einen optional substituierten 5-, 6- oder 7-gliedrigen Ring zu bilden;
und
R¹⁷ ist H, Hydroxyl, Cyano, optional substituiertes Amino, optional substituiertes Amido, optional substituiertes Carboxamid, optional substituiertes C₁-C₆-Alkyl, optional substituiertes C₂-C₆-Alkenyl, optional substituiertes C₂-C₆-Alkynyl, optional substituiertes C₁-C₆-Alkanoyl, optional substituiertes C₇-C₁₁-Aryloyl, optional substituiertes C₂-C₁₀-Heterocyclyloyl, optional substituiertes C₂-C₁₀-Heteroaryloyl, optional substituiertes C₁-C₆-Alkylsulfinyl, optional substituiertes C₁-C₆-Alkylsulfonyl, optional substituiertes C₆-C₁₀-Arylsulfinyl, optional substituiertes C₆-C₁₀-Arylsulfonyl, optional substituiertes C₁-C₉-Heteroarylsulfinyl, optional substituiertes C₁-C₉-Heteroarylsulfonyl, optional substituiertes C₁-C₆-Heteroalkyl, optional substituiertes C₂-C₆-Heteroalkenyl, optional substituiertes C₂-C₆-Heteroalkynyl, optional substituiertes C₃-C₁₀-Cycloalkyl, optional substituiertes C₄-C₁₀-Cycloalkenyl, optional substituiertes C₈-C₁₀-Cycloalkynyl, optional substituiertes C₆-C₁₀-Aryl, optional substituiertes C₆-C₁₀-Aryl C₁-C₆-Alkyl, optional substituiertes C₆-C₁₀-Aryl C₂-C₆-Alkenyl, optional substituiertes C₆-C₁₀-Aryl C₂-C₆-Alkynyl, optional substituiertes C₁-C₉-Heteroaryl, optional substituiertes C₁-C₉-Heteroaryl C₁-C₆-Alkyl, optional substituiertes C₁-C₉-Heteroaryl C₂-C₆-Alkenyl, optional substituiertes C₁-C₉-Heteroaryl C₂-C₆-Alkynyl, optional substituiertes C₁-C₉-Heterocyclyl, optional substituiertes C₁-C₉-Heterocyclyl C₁-C₆-Alkyl, optional substituiertes C₁-C₉-Heterocyclyl C₂-C₆-Alkenyl oder optional substituiertes C₁-C₉-Heterocyclyl C₂-C₆-Alkynyl;
wobei drei oder weniger von X³, X⁴, X⁵, X⁶ und X⁷ N sind; und
mindestens eines aus X¹ und X² N ist.

11. Zusammensetzung nach Anspruch 8, wobei die Zusammensetzung ferner einen oder mehrere Wirkstoffe umfasst, ausgewählt aus der Gruppe bestehend aus einem Anti-GITR-Antikörper, Anti-CTLR-4-Antikörper, Anti-LAG3-Antikörper, Anti-TIGIT-Antikörper , Anti-NRP1-Antikörper, Anti-TGF-β-Antikörper, Anti-CCR2-Antikörper, Anti-CCR4-Antikörper, Anti-CCR8-Antikörper, Anti-TNFR2-Antikörper und einem Anti-EZH2-Antikörper.

12. GPR174-Hemmer für die Verwendung in einem Verfahren zum Verstärken einer Antitumor-Immunreaktion in einem Subjekt, das aktuell einer Behandlung mit mindestens einem aus einem A2aR-Antagonisten, einem A2bR-Antagonisten, einem CD38-Hemmer, einem CD39-Hemmer, einem CD73-Hemmer und einem Wirkstoff ausgewählt aus der Gruppe bestehend aus einem Anti-GITR-Antikörper, Anti-CTLR-4-Antikörper, Anti-LAG3-Antikörper, Anti-TIGIT-Antikörper, Anti-NRP1-Antikörper, Anti-TGF-β-Antikörper, Anti-CCR2-Antikörper, Anti-CCR4-Antikörper, Anti-CCR8-Antikörper, Anti-TNFR2-Antikörper und einem Anti-EZH2-Antikörper unterzogen wird.

13. GPR174-Hemmer für die Verwendung nach Anspruch 12, wobei der GPR174-Hemmer eine PS-abhängige oder LysoPS-abhängige Aktivierung der GPR174-Signalübertragung in einer GPR174 exprimierenden Zelle um mindestens 25 % hemmt.

## Revendications

1. Combinaison d'un inhibiteur de GPR174 et d'au moins un agent supplémentaire sélectionné dans le groupe constitué par :
i. un antagoniste du récepteur de l'adénosine-A2A (A2A) ;
ii. un antagoniste du récepteur de l'adénosine-A2B (A2B) ;
iii. un inhibiteur de CD73 ;
iv. un inhibiteur de CD38 ;
v. un inhibiteur de CD39 ; et
vi. un ou plusieurs agents sélectionnés dans le groupe constitué par un anticorps anti-GITR, un anticorps anti-CTLR-4, un anticorps anti-LAG3, un anticorps anti-TIGIT, un anticorps anti-NRP1, un anticorps anti-TGF-β, un anticorps anti-CCR2, un anticorps anti-CCR4, un anticorps anti-CCR8, un anticorps anti-TNFR2 et un anticorps anti-EZH2,
destinés à être utilisés dans le traitement du cancer, dans laquelle l'inhibiteur de GPR174 et ledit au moins un agent supplémentaire sont administrés simultanément, ou séquentiellement dans n'importe quel ordre, à condition que les effets du premier inhibiteur ou antagoniste administré restent présents au moment de l'administration du second inhibiteur ou antagoniste.

2. Combinaison destinée à être utilisée selon la revendication 1, dans laquelle l'agent supplémentaire est un anticorps anti-GITR, anti-CTLA-4, anti-CD25, anti-LAG3, anti-TIGIT, anti-NRP1, anti-TGF-β, anti-CCR2, anti-CCR4, anti-CCR8 ou anti-TNFR2 ou un inhibiteur de EZH2.

3. Combinaison destinée à être utilisée selon l'une quelconque des revendications 1 et 2, dans laquelle l'inhibiteur de GPR174 inhibe l'activation PS-dépendante ou LysoPS-dépendante de la signalisation de GPR174 dans une cellule exprimant GPR174 par au moins 25 %.

4. Combinaison destinée à être utilisée selon l'une quelconque des revendications 1 à 3, dans laquelle l'inhibiteur de GPR174 stimule la réponse immunitaire chez le patient en inhibant une signalisation G-alpha-s de GPR174 et éventuellement, dans laquelle la réponse immunitaire stimulée comprend une réponse immunitaire médiée par les cellules NK ou une réponse immunitaire médiée par les cellules T cytotoxiques antitumorales.

5. Procédé *in vitro* d'augmentation du niveau de cytokines Th1 dans des cellules mononucléaires du sang périphérique humaines (PBMC), le procédé comprenant la mise en contact *in vitro* des PBMC humaines avec un inhibiteur de la signalisation de GPR174 et au moins un antagoniste du récepteur de l'adénosine-A2A (A2A), un antagoniste du récepteur de l'adénosine-A2B (A2B), ou une combinaison de ceux-ci.

6. Procédé *in vitro* selon la revendication 5, dans lequel les PBMC comprennent des cellules T ou des cellules NK.

7. Procédé *in vitro* selon l'une quelconque des revendications 5 à 6, dans lequel le niveau d'au moins une parmi les cytokines Th1 IFN-γ, IL-2, TNF ou GM-CSF est augmenté d'au moins 20 %.

8. Composition pharmaceutique comprenant une combinaison d'un inhibiteur de la signalisation de GPR174 avec au moins un antagoniste du récepteur de l'adénosine-A2A (A2A), un antagoniste du récepteur de l'adénosine-A2B (A2B), un inhibiteur de CD73, un inhibiteur de CD38 et un inhibiteur de CD39 et éventuellement un excipient pharmaceutiquement acceptable.

9. Composition pharmaceutique selon la revendication 8, dans laquelle l'inhibiteur de GPR174 présente une structure selon la formule (IV) : 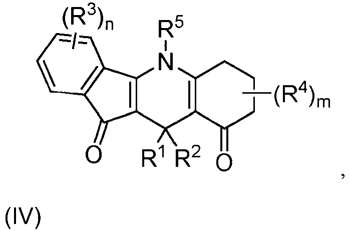
ou un stéréoisomère de celui-ci, ou un tautomère de celui-ci, ou un sel pharmaceutiquement acceptable de celui-ci, dans lequel
chacun parmi R¹ et R² est indépendamment H, hydroxy, halogène, amino éventuellement substitué, amido éventuellement substitué, thiol, cyano, alkyle en C₁-C₆ éventuellement substitué, alcényle en C₂-C₆ éventuellement substitué, alcynyle en C₂-C₆ éventuellement substitué, alcoxy en C₁-C₆ éventuellement substitué, aryloxy en C₆-C₁₀ éventuellement substitué, hétéroaryloxy en C₁-C₉ éventuellement substitué, alcanoyle en C₂-C₆ éventuellement substitué, aryloyle en C₇-C₁₁ éventuellement substitué, hétéroaryloyle en C₂-C₁₀ éventuellement substitué, hétérocyclyloyle en C₂-C₁₀ éventuellement substitué, hydroxycarbonyle, ester éventuellement substitué, carboxamide éventuellement substitué, alcanoyloxy en C₁-C₆ éventuellement substitué, aryloyloxy en C₇-C₁₁ éventuellement substitué, hétéroaryloyloxy en C₂-C₁₀ éventuellement substitué, hétérocyclyloyloxy en C₂-C₁₀ éventuellement substitué, thioalkyle en C₁-C₆ éventuellement substitué, alkylsulfinyle en C₁-C₆ éventuellement substitué, alkylsulfonyle en C₁-C₆ éventuellement substitué, arylthio en C₆-C₁₀ éventuellement substitué, arylsulfinyle en C₆-C₁₀ éventuellement substitué, arylsulfonyle en C₆-C₁₀ éventuellement substitué, hétéroarylthio en C₁-C₉ éventuellement substitué, hétéroarylsulfinyle en C₁-C₉ éventuellement substitué, hétéroarylsulfonyle en C₁-C₉ éventuellement substitué, hétérocyclylsulfinyle en C₁-C₉ éventuellement substitué, hétérocyclylsulfonyle en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₈-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle e n C₂-C₆ éventuellement substitués, ou hétérocyclyle en C₁-C₉ alcynyle en C₂-C₆ éventuellement substitués ;
chacun parmi R³ et R⁴ est indépendamment, H, hydroxy, halogène, amino éventuellement substitué, amido éventuellement substitué, thiol, cyano, alkyle en C₁-C₆ éventuellement substitué, alcényle en C₂-C₆ éventuellement substitué, alcynyle en C₂-C₆ éventuellement substitué, alcoxy en C₁-C₆ éventuellement substitué, aryloxy en C₆-C₁₀ éventuellement substitué, hétéroaryloxy en C₁-C₉ éventuellement substitué, alcanoyle en C₂-C₆ éventuellement substitué, aryloyle en C₇-C₁₁ éventuellement substitué, hétéroaryloyle en C₂-C₁₀ éventuellement substitué, hétérocyclyloyle en C₂-C₁₀ éventuellement substitué, hydroxycarbonyle, ester éventuellement substitué, carboxamide éventuellement substitué, alcanoyloxy en C₁-C₆ éventuellement substitué, aryloyloxy en C₇-C₁₁ éventuellement substitué, hétéroaryloyloxy en C₂-C₁₀ éventuellement substitué, hétérocyclyloyloxy en C₂-C₁₀ éventuellement substitué, thioalkyle en C₁-C₆ éventuellement substitué, alkylsulfonyle en C₁-C₆ éventuellement substitué, arylthio en C₆-C₁₀ éventuellement substitué, arylsulfonyle en C₆-C₁₀ éventuellement substitué, hétéroarylthio en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₈-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle en C₂-C₆ éventuellement substitués, ou hétérocyclyle en C₁-C₉ alcynyle en C₂-C₆ éventuellement substitués ;
R⁵ est H, alkyle en C₁-C₆ éventuellement substitué, alcényle en C₂-C₆ éventuellement substitué, alcynyle en C₂-C₆ éventuellement substitué, alcoxy en C₁-C₆ éventuellement substitué, aryloxy en C₆-C₁₀ éventuellement substitué, hétéroaryloxy en C₁-C₉ éventuellement substitué, alcanoyle en C₂-C₆ éventuellement substitué, aryloyle en C₇-C₁₁ éventuellement substitué, hétéroaryloyle en C₂-C₁₀ éventuellement substitué, hétérocyclyloyle en C₂-C₁₀ éventuellement substitué, alkyloxycarbonyle en C₁-C₆ éventuellement substitué, alkylsulfinyle en C₁-C₆ éventuellement substitué, alkylsulfonyle en C₁-C₆ éventuellement substitué, arylsulfinyle en C₆-C₁₀ éventuellement substitué, arylsulfonyle en C₆-C₁₀ éventuellement substitué, hétéroarylsulfinyle en C₁-C₉ éventuellement substitué, hétéroarylsulfonyle en C₁-C₉ éventuellement substitué, hétérocyclylsulfinyle en C₁-C₉ éventuellement substitué, hétérocyclylsulfonyle en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₈-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle en C₂-C₆ éventuellement substitués, ou hétérocyclyle en C₁-C₉ alcynyle en C₂-C₆ éventuellement substitués.
n est 0, 1, 2, 3 ou 4 ; et
m est 0, 1, 2, 3, 5 ou 6.

10. Composition selon la revendication 8, dans laquelle l'inhibiteur de GPR174 a une structure selon la formule (I) suivante, 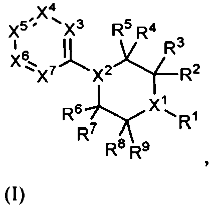
ou un stéréoisomère de celui-ci, ou un sel pharmaceutiquement acceptable de celui-ci, dans lequel
X¹ est N ou CR¹⁰ ;
X² est N ou CR¹¹ ;
X³ est N ou CR¹² ;
X⁴ est N ou CR¹³ ;
X⁵ est N ou CR¹⁴ ;
X⁶ est N ou CR¹⁵ ;
X⁷ est N ou CR¹⁶ ;
chacun parmi R¹, R², R³, R⁴, R⁵, R⁶, R⁷, R⁸ et R⁹ est indépendamment H, hydroxy, thiol, amino éventuellement substitué, amido éventuellement substitué, cyano, alkyle en C₁-C₆ éventuellement substitué, alcényle en C₂-C₆ éventuellement substitué, alcynyle en C₂-C₆ éventuellement substitué, alcoxy en C₁-C₆ éventuellement substitué, aryloxy en C₆-C₁₀ éventuellement substitué, hétéroaryloxy en C₁-C₉ éventuellement substitué, alcanoyle en C₂-C₆ éventuellement substitué, aryloyle en C₇-C₁₁ éventuellement substitué, hétéroaryloyle en C₂-C₁₀ éventuellement substitué, hétérocyclyloyle en C₂-C₁₀ éventuellement substitué, hydroxycarbonyle, alkoxycarbonyle en C₂-C₇ éventuellement substitué, carboxamide éventuellement substitué, alcanoyloxy en C₁-C₆ éventuellement substitué, aryloyloxy en C₇-C₁₁ éventuellement substitué, hétéroaryloyloxy en C₂-C₁₀ éventuellement substitué, hétérocyclyloyloxy en C₂-C₁₀ éventuellement substitué, alkylsulfinyle en C₁-C₆ éventuellement substitué, alkylsulfonyle en C₁-C₆ éventuellement substitué, arylsulfinyle en C₆-C₁₀ éventuellement substitué, arylsulfonyle en C₆-C₁₀ éventuellement substitué, hétéroarylsulfinyle en C₁-C₉ éventuellement substitué, hétéroarylsulfonyle en C₁-C₉ éventuellement substitué, hétérocyclylsulfinyle en C₁-C₉ éventuellement substitué, hétérocyclylsulfonyle en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₈-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle en C₂-C₆ éventuellement substitués, ou hétérocyclyle en C₁-C₉ alcynyle en C₂-C₆ éventuellement substitués ; ou
R² et R³ se combinent pour former =0, =S, ou =NR¹⁷ ; ou
R⁴ et R⁵ se combinent pour former =0, =S, ou =NR¹⁷ ; ou
R⁶ et R⁷ se combinent pour former =0, =S, ou =NR¹⁷ ; ou
R⁸ et R⁹ se combinent pour former =0, =S, ou =NR¹⁷ ;
chacun parmi R¹⁰, R¹¹, R¹², R¹³, R¹⁴, R¹⁵ et R¹⁶ est, indépendamment, H, hydroxy, halogène, thiol, amino éventuellement substitué, amido éventuellement substitué, cyano, nitro, alkyle en C₁-C₆ éventuellement substitué, alcényle en C₂-C₆ éventuellement substitué, alcynyle en C₂-C₆ éventuellement substitué, alcoxy en C₁-C₆ éventuellement substitué, aryloxy en C₆-C₁₀ éventuellement substitué, hétéroaryloxy en C₁-C₉ éventuellement substitué, alcanoyle en C₂-C₆ éventuellement substitué, aryloyle en C₇-C₁₁ éventuellement substitué, hétéroaryloyle en C₂-C₁₀ éventuellement substitué, hétérocyclyloyle en C₂-C₁₀ éventuellement substitué, hydroxycarbonyl, alkoxycarbonyle en C₂-C₇ éventuellement substitué, carboxamide éventuellement substitué, alcanoyloxy en C₁-C₆ éventuellement substitué, aryloyloxy en C₇-C₁₁ éventuellement substitué, hétéroaryloyloxy en C₂-C₁₀ éventuellement substitué, hétérocyclyloyloxy en C₂-C₁₀ éventuellement substitué, alkylsulfinyle en C₁-C₆ éventuellement substitué, alkylsulfonyle en C₁-C₆ éventuellement substitué, arylsulfinyle en C₆-C₁₀ éventuellement substitué, arylsulfonyle en C₆-C₁₀ éventuellement substitué, hétéroarylsulfinyle en C₁-C₉ éventuellement substitué, hétéroarylsulfonyle en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₆-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle en C₂-C₆ éventuellement substitués , ou hétérocyclyle en C₁-C₉ alcynyle en C₂-C₆ éventuellement substitués ; ou un parmi ;
(i) R¹² et R¹³, ainsi que les atomes auxquels chacun est attaché, se combinent pour former un cycle à 5, 6 ou 7 éléments éventuellement substitué ;
(ii) R¹³ et R¹⁴, ainsi que les atomes auxquels chacun est attaché, se combinent pour former un cycle à 5, 6 ou 7 éléments éventuellement substitué ;
(iii) R¹⁴ et R¹⁵, ainsi que les atomes auxquels chacun est attaché, se combinent pour former un cycle à 5, 6 ou 7 éléments éventuellement substitué ; et
(iv) R¹⁵ et R¹⁶, ainsi que les atomes auxquels chacun est attaché, se combinent pour former un cycle à 5, 6 ou 7 éléments éventuellement substitué ;
et
R¹⁷ est H, hydroxyle, cyano, amino éventuellement substitué, amido éventuellement substitué, carboxamide éventuellement substitué, alkyle en C₁-C₆ éventuellement substitué, alcényle en C₂-C₆ éventuellement substitué, alcynyle en C₂-C₆ éventuellement substitué, alcanoyle en C₁-C₆ éventuellement substitué, aryloyle en C₇-C₁₁ éventuellement substitué, hétérocyclyloyle en C₂-C₁₀ éventuellement substitué, hétéroaryloyle en C₂-C₁₀ éventuellement substitué, alkylsulfinyle en C₁-C₆ éventuellement substitué, alkylsulfonyle en C₁-C₆ éventuellement substitué, arylsulfinyle en C₆-C₁₀ éventuellement substitué, arylsulfonyle en C₆-C₁₀ éventuellement substitué, hétéroarylsulfinyle en C₁-C₉ éventuellement substitué, hétéroarylsulfonyle en C₁-C₉ éventuellement substitué, hétéroalkyle en C₁-C₆ éventuellement substitué, hétéroalcényle en C₂-C₆ éventuellement substitué, hétéroalcynyle en C₂-C₆ éventuellement substitué, cycloalkyle en C₃-C₁₀ éventuellement substitué, cycloalcényle en C₄-C₁₀ éventuellement substitué, cycloalcynyle en C₈-C₁₀ éventuellement substitué, aryle en C₆-C₁₀ éventuellement substitué, aryle en C₆-C₁₀ alkyle en C₁-C₆ éventuellement substitués, aryle en C₆-C₁₀ alcényle en C₂-C₆ éventuellement substitués, aryle en C₆-C₁₀ alcynyle en C₂-C₆ éventuellement substitués, hétéroaryle en C₁-C₉ éventuellement substitué, hétéroaryle en C₁-C₉ alkyle en C₁-C₆ éventuellement substitués, hétéroaryle en C₁-C₉ alcényle en C₂-C₆ éventuellement substitués, hétéroaryle en C₁-C₉ alcynyle en C₂-C₆ éventuellement substitués, hétérocyclyle en C₁-C₉ éventuellement substitué, hétérocyclyle en C₁-C₉ alkyle en C₁-C₆ éventuellement substitués, hétérocyclyle en C₁-C₉ alcényle en C₂-C₆ éventuellement substitués, ou hétérocyclyle en C₁-C₉ alcynyle en C₂-C₆ éventuellement substitués.
dans laquelle trois ou moins parmi X³, X⁴, X⁵, X⁶, et X⁷ sont N ; et
au moins un parmi X¹ est X² est N.

11. Composition selon la revendication 8, dans laquelle la composition comprend en outre un ou plusieurs agents sélectionnés dans le groupe constitué par un anticorps anti-GITR, un anticorps anti-CTLR-4, un anticorps anti-LAG3, un anticorps anti-TIGIT, un anticorps anti-NRP1, un anticorps anti-TGF-β, un anticorps anti-CCR2, un anticorps anti-CCR4, un anticorps anti-CCR8, un anticorps anti-TNFR2 et un anticorps anti-EZH2.

12. Inhibiteur de GPR174 destiné à être utilisé dans un procédé d'amélioration d'une réponse immunitaire antitumorale chez un sujet qui subit actuellement un traitement avec au moins un antagoniste de A2aR, un antagoniste de A2bR, un inhibiteur de CD38, un inhibiteur de CD39, un inhibiteur de CD73 et un agent sélectionnés dans le groupe constitué par un anticorps anti-GITR, un anticorps anti-CTLR-4, un anticorps anti-LAG3, un anticorps anti-TIGIT, un anticorps anti-NRP1, un anticorps anti-TGF-β, un anticorps anti-CCR2, un anticorps anti-CCR4, un anticorps anti-CCR8, un anticorps anti-TNFR2 et un anticorps anti-EZH2.

13. Inhibiteur de GPR174 destiné à être utilisé selon la revendication 12, dans lequel l'inhibiteur de GPR174 inhibe l'activation PS-dépendante ou LysoPS-dépendante de la signalisation de GPR174 dans une cellule exprimant GPR174 par au moins 25 %.
